# EUROPEAN PATENT APPLICATION

(11) **EP 3 566 719 A1**
(43) Date of publication of application: **13.11.2019**
(21) Application number: 19165905.1
(22) Date of filing: 18.05.2011
(51) Int. Cl.: A61K 47/61, A61K 47/60, C07B 37/00, C08B 37/16, A61K 47/00, A61P 29/00, A61P 37/00

(54) **COMPOSITIONS AND METHODS FOR TREATMENT OF AUTOIMMUNE AND OTHER DISEASES**

(30) Priority: 18.05.2010 US 34564110 P
(62) Divisional of application: 11784186.6
(71) Applicant: Cerulean Pharma Inc., Cambridge, MA 02139 (US)
(72) Inventor: ELIASOF, Scott D., Lexington, MA 02420 (US)
(74) Representative: Simpson, Tobias Rutger

(57) **Abstract**

Provided are methods relating to the use of CDP-therapeutic agent conjugates for the treatment of autoimmune disease, inflammatory disease, or cancer. Also provided are CDP-therapeutic agent conjugates, particles comprising CDP-therapeutic agent conjugates, and compositions comprising CDP-therapeutic agent conjugates.

## Description

This application claims priority to U.S. Serial Number 61/345,641, filed May 18, 2010. The disclosures of the prior application is considered part of (and is incorporated by reference in) the disclosure of this application.

### Background of the Invention

Drug delivery and dosing of small molecule therapeutic agents, such as camptothecin, can be problematic due to a number issues including half-life, toxicity, distribution etc.

### Summary of the Invention

In one aspect, the invention features a method of treating an autoimmune disease in a subject, e.g., a human subject, comprising administering a CDP-therapeutic agent conjugate, particle or composition to the subject, e.g., a human subject, in an amount effective to treat the disease.

Examples of autoimmune diseases that can be treated according to the methods of the invention include ankylosing spondylitis, arthritis (e.g., rheumatoid arthritis, osteoarthritis, gout), Chagas disease, chronic obstructive pulmonary disease (COPD), dermatomyositis, diabetes mellitus type 1, endometriosis, Goodpasture's syndrome, Graves' disease, Guillain-Barre syndrome (GBS), Hashiomoto's disease, Hidradenitis suppurativa, Kawasaki disease, IgA nephropathy, Idiopathic thrombocytopenic purpura, inflammatory bowel disease (e.g., Crohn's disease, ulcerative colitis, collagenous colitis, lymphocytic colitis, ischemic colitis, diversion colitis, Behcet's syndrome, infective colitis, indeterminate colitisinterstitial cystitis), lupus (e.g., systemic lupus erythematosus, discoid lupus, drug-induced lupus, neonatal lupus), mixed connective tissue disease, morphea, multiple sclerosis, myasthenia gravis, narcolepsy, neuromyotonia, pemphigus vulgaris, pernicious anemia, psoriasis, psoriatic arthritis, polymyositis, primary biliary cirrhosis, relapsing polychondritis, schizophrenia, scleroderma, Sjögren's syndrome, Stiff person syndrome, temporal arteritis (also known as giant cell arteritis), vasculitis, vitiligo, Wegener's granulomatosis. In some embodiments, the method includes inhibiting rejection of a transplanted organ, e.g., rejection of a kidney transplant, e.g., rejection of a lung transplant, e.g., rejection of a liver transplant. In an embodiment, the autoimmune disease is arthritis, e.g., rheumatoid arthritis, osteoarthritis, gout; lupus, e.g., systemic lupus erythematosus, discoid lupus, drug-induced lupus, neonatal lupus; inflammatory bowel disease, e.g., Crohn's disease, ulcerative colitis, collagenous colitis, lymphocytic colitis, ischemic colitis, diversion colitis, Behcet's syndrome, infective colitis, indeterminate colitis; psoriasis; or multiple sclerosis.

In an embodiment, the CDP-therapeutic agent conjugate, particle or composition is a CDP-cytotoxic agent conjugate, particle or composition, e.g., a CDP-topoisomerase inhibitor conjugate, particle or composition, e.g., a CDP-topoisomerase inhibitor I conjugate (e.g., a CDP-camptothecin conjugate, particle or composition, CDP-irinotecan conjugate, particle or composition, or CDP-SN-38 conjugate, particle or composition, a CDP-topotecan conjugate, particle or composition, a CDP-lamellarin D conjugate, particle or composition, a CDP-lurotecan conjugate, particle or composition, a CDP-exatecan conjugate, particle or composition, a CDP-diflomotecan conjugate, particle or composition, or a CDP-topoisomerase I inhibitor conjugate, particle or composition which includes a derivative of camptothecin, irinotecan, SN-38, lamellarin D, lurotecan, exatecan or diflomotecan).

The topoisomerase inhibitor can be a topoisomerase II inhibitor, thus in an embodiment the conjugate, particle or composition is: a CDP-topoisomerase II inhibitor conjugate, particle or composition (e.g., a CDP-etoposide conjugate, particle or composition, a CDP-tenoposide conjugate, particle or composition, a CDP-amsacrine conjugate, particle or composition, or a CDP-topoisomerase II inhibitor conjugate, particle or composition which includes a derivative of etoposide, tenoposide, and amsacrine).

The therapeutic agent can be an anti-metabolite, thus in an embodiment the conjugate, particle or composition is: a CDP-anti-metabolic agent conjugate, particle or composition (e.g., a CDP-antifolate conjugate, particle or composition (e.g., a CDP-pemetrexed conjugate, particle or composition, a CDP-floxuridine conjugate, particle or composition, or a CDP-raltitrexed conjugate, particle or composition); or a CDP-pyrimidine analog conjugate, particle or composition (e.g., a CDP-capecitabine conjugate, particle or composition, a CDP-cytarabine conjugate, particle or composition, a CDP-gemcitabine conjugate, particle or composition, or a CDP-5FU conjugate, particle or composition)).

The therapeutic agent can be an alkylating agent, thus in an embodiment the conjugate, particle or composition is: a CDP-alkylating agent conjugate, particle or composition.

The therapeutic agent can be an anthracycline, thus in an embodiment the conjugate, particle or composition is: a CDP-anthracycline conjugate, particle or composition.

The therapeutic agent can be an anti-tumor antibiotic, thus in an embodiment the conjugate, particle or composition is a CDP-anti-tumor antibiotic conjugate, particle or composition (e.g., a CDP-HSP90 inhibitor conjugate, particle or composition, e.g., a CDP-geldanamycin conjugate, particle or composition, a CDP-tanespimycin conjugate, particle or composition or a CDP-alvespimycin conjugate, particle or composition).

The therapeutic agent can be a platinum based agent, thus in an embodiment the conjugate, particle or composition is a CDP-platinum based agent conjugate, particle or composition (e.g., a CDP-cisplatin conjugate, particle or composition, a CDP-carboplatin conjugate, particle or composition, or a CDP-oxaliplatin conjugate, particle or composition).

The therapeutic agent can be a microtubule inhibitor, thus in an embodiment the conjugate, particle or composition is a CDP-microtubule inhibitor conjugate, particle or composition.

The therapeutic agent can be a kinase inhibitor, thus in an embodiment the conjugate, particle or composition is, a CDP-kinase inhibitor conjugate, particle or composition (e.g., a CDP-seronine/threonine kinase inhibitor conjugate, particle or composition, e.g., a CDP-mTOR inhibitor conjugate, particle or composition, e.g., a CDP-rapamycin conjugate, particle or composition).

The therapeutic agent can be a proteasome inhibitor, thus in an embodiment the conjugate, particle or composition is a CDP-proteasome inhibitor conjugate, particle or composition, e.g., a CDP-bortezomib inhibitor conjugate, particle or composition.

In an embodiment, the CDP-microtubule inhibitor conjugate, particle or composition comprises a CDP-taxane conjugate, particle or composition or a CDP-epothilone conjugate, particle or composition. In an embodiment, the CDP-proteasome inhibitor conjugate, particle or composition is a CDP-boronic acid containing molecule conjugate, particle or composition, e.g., a CDP-bortezomib conjugate, particle or composition.

The therapeutic agent can be an immunomodulator conjugate, thus in an embodiment the conjugate, particle or composposition is a CDP-immunomodulator conjugate, particle or composition, e.g., a CDP-corticosteroid conjugate, particle or composition. In an embodiment, the CDP-immunomodulator conjugate, particle or composition is a CDP-kinase inhibitor conjugate, particle or composition (e.g., a CDP-seronine/threonine kinase inhibitor conjugate, particle or composition, e.g., a CDP-mTOR inhibitor conjugate, particle or composition, e.g., a CDP-rapamycin conjugate, particle or composition).

In an embodiment, the CDP-therapeutic agent conjugate, particle or composition is a CDP-corticosteroid conjugate, particle or composition wherein the corticosteroid is not (or is other than) methylprednisolone. In an embodiment, the CDP-therapeutic agent conjugate, particle or composition is a CDP-corticosteroid conjugate, particle or composition wherein the corticosteroid is a Group B corticosteroid, a Group C corticosteroid, or a Group D corticosteroid. In an embodiment, the CDP-therapeutic agent conjugate, particle or composition is a CDP-corticosteroid conjugate, particle or composition wherein the corticosteroid is hydrocortisone, hydrocortisone acetate, cortisone acetate, tixocortol pivalate, prednisolone, methylprednisolone, or prednisone. In an embodiment, the CDP-therapeutic agent conjugate, particle or composition is a CDP-corticosteroid conjugate, particle or composition wherein the corticosteroid is a Group B corticosteroid, a Group C corticosteroid, a Group D corticosteroid, hydrocortisone, hydrocortisone acetate, cortisone acetate, tixocortol pivalate, prednisolone, methylprednisolone, or prednisone. In an embodiment, the CDP-therapeutic agent conjugate, particle or composition is a CDP-corticosteroid conjugate, particle or composition wherein the corticosteroid is a Group B corticosteroid, a Group C corticosteroid, a Group D corticosteroid, hydrocortisone, hydrocortisone acetate, cortisone acetate, tixocortol pivalate, prednisolone, methylprednisolone, or prednisone. In an embodiment, the CDP-corticosteroid conjugate, e.g., the CDP-methylprednisolone conjugate, includes a linker attaching the corticosteroid to the CDP, wherein the linker is not a glycine. In one embodiment, the linker is one ore more of: alanine, arginine, histidine, lysine, aspartic acid, glutamic acid, serine, threonine, asparganine, glutamine, cysteine, proline, isoleucine, leucine, methionine, phenylalanine, tryptophan, tyrosine and valine. In some embodiments, the linker is a linker described herein. In some embodiments, the linker is not an amino acid (e.g., an alpha amino acid). In some embodiments, the linker is alanine glycolate or amino hexanoate. In some embodiments, the loading of the corticosteroid onto the CDP is at least about 13% by weight of the conjugate (e.g., at least about 14%, 15%, 16%, 17%, 18%, 19%, or 20%). In some embodiments, the loading of the corticosteroid onto the CDP is less than about 12% by weight of the conjugate (e.g., less than about 11%, 10%, 9%, 8%, or 7%).

In an embodiment, the CDP-corticosteroid conjugate, particle or composition is a CDP-corticosteroid conjugate, particle or composition described herein.

In an embodiment, the autoimmune disease is not (or is other than) rheumatoid arthritis. In an embodiment, the autoimmune disease is not (or is other than) rheumatoid arthritis and the CDP-therapeutic agent conjugate, particle or composition is a CDP-corticosteroid conjugate, particle or composition.

In an embodiment, the autoimmune disease is rheumatoid arthritis and the CDP-therapeutic agent conjugate, particle or composition is a CDP-corticosteroid conjugate, particle or composition wherein the corticosteroid is not (or is other than) methylprednisolone. In an embodiment, the autoimmune disease is rheumatoid arthritis and the CDP-therapeutic agent conjugate, particle or composition is a CDP-corticosteroid conjugate, particle or composition wherein the corticosteroid is a Group B corticosteroid, a Group C corticosteroid, or a Group D corticosteroid. In an embodiment, the autoimmune disease is rheumatoid arthritis and the CDP-therapeutic agent conjugate, particle or composition is a CDP-corticosteroid conjugate, particle or composition wherein the corticosteroid is hydrocortisone, hydrocortisone acetate, cortisone acetate, tixocortol pivalate, prednisolone, methylprednisolone, or prednisone. In an embodiment, the autoimmune disease is rheumatoid arthritis and the CDP-therapeutic agent conjugate, particle or composition is a CDP-corticosteroid conjugate, particle or composition wherein the corticosteroid is a Group B corticosteroid, a Group C corticosteroid, or a Group D corticosteroid, hydrocortisone, hydrocortisone acetate, cortisone acetate, tixocortol pivalate, prednisolone, methylprednisolone, or prednisone. In an embodiment, the CDP-corticosteroid conjugate, particle or composition is a CDP-corticosteroid conjugate, particle or composition described herein.

In an embodiment, the autoimmune disease is rheumatoid arthritis, and the CDP-corticosteroid conjugate, particle or composition is a CDP-methylprednisolone conjugate, particle or composition. In an embodiment, the CDP-methylprednisolone conjugate includes a linker attaching the corticosteroid to the CDP, wherein the linker is not a glycine. In one embodiment, the linker is one ore more of: alanine, arginine, histidine, lysine, aspartic acid, glutamic acid, serine, threonine, asparganine, glutamine, cysteine, proline, isoleucine, leucine, methionine, phenylalanine, tryptophan, tyrosine and valine. In some embodiments, the linker is a linker described herein. In some embodiments, the linker is not an amino acid (e.g., an alpha amino acid). In some embodiments, the linker is alanine glycolate or amino hexanoate. In some embodiments, the loading of the methylprednisolone onto the CDP is at least about 13% by weight of the conjugate (e.g., at least about 14%, 15%, 16%, 17%, 18%, 19%, or 20%). In some embodiments, the loading of the methylprednisolone onto the CDP is less than about 12% by weight of the conjugate (e.g., less than about 11%, 10%, 9%, 8%, or 7%).

In an embodiment, the autoimmune disease, e.g., rheumatoid arthritis, and the CDP-therapeutic agent conjugate, particle or composition is administered to the subject in combination with a second therapeutic agent. In an embodiment, e.g., wherein the autoimmune disease is rheumatoid arthritis, the second therapeutic agent is one or more of the following agents: an anti-inflammatory agent, a corticosteroid, a disease modifying antirheumatic drug (DMARD), an immunomodulator, a statin, and/or a bisphosphonate.

In an embodiment, e.g., wherein the autoimmune disease is rheumatoid arthritis, the anti-inflammatory agent is one or more of the following agents: aspirin, acetaminophen, and/or a non-steroidal anti-inflammatory drug.

In an embodiment, e.g., wherein the autoimmune disease is rheumatoid arthritis, the corticosteroid is one of more of the corticosteroids described herein.

In an embodiment, e.g., wherein the autoimmune disease is rheumatoid arthritis, the DMARD is one or more of the following agents; azathioprine, cyclosporine A, D-penicillamine, gold salts, hydroxychloroquine, chloroquine (also called anti-malarial agents herein), leflunomide, methotrexate, minocycline, sulfasalazine, and/or cyclophosphamide.

In an embodiment, e.g., wherein the autoimmune disease is rheumatoid arthritis, the immunomodulator includes one or more of the following agents: TNF inhibitors (e.g. etanercept (Enbrel®), infliximab (Remicade®), adalimumab(Humira®), certolixumab pegol (Cimzia®), and golimumab (Simponi®)), IL-1 inhibitors (e.g. anakinra (Kineret®)), antibodies against B cells (rituxamab (Rituxan®)), T cell comstimulation inhibitors (abatacept (Orencia®)), IL-6 inhibitors (tocilizumab (RoActemra®)), and/or other agents, e.g., biologics, that interfere with immune cell function (e.g., antibodies to other immune system targets, e.g., antibodies to IL-15).

In an embodiment, e.g., wherein the autoimmune disease is rheumatoid arthritis,the statin is one or more of the following agents: atorvastatin (Lipitor®), cerivastatin (Baycol®), fluvastatin (Lescol®), lovastatin (Mevacor®), mevastatin, pitavastatin (Livalo®), pravastatin (Pravachol®), rosuvastatin (Crestor®), and/or simvastatin (Zocor®).

In an embodiment, e.g., wherein the autoimmune disease is rheumatoid arthritis,the bisphosphonate is one or more of the following agents: non-N (nitrogen)-containing bisphosphonates (e.g., etidronate (Didronel®), clodronate (Bonefos®), and tiludronate (Skelid®)) and/or N (nitrogen)-containing bisphosphonates (e.g., pamidronate (Aredia®), neridronate, olpadronate, alendronate (Fosamax®), ibandronate (Boniva®), risedronate (Actonel®), and zoledronate (Zometa®)

In an embodiment, the CDP-therapeutic agent conjugate, particle or composition inhibits rejection of a transplanted organ, e.g., rejection of a kidney transplant, rejection of a lung transplant, rejection of a liver transplant. Tn an embodiment, the CDP-therapeutic agent conjugate, particle or composition inhibits rejection of a kidney transplant and the CDP-immunomodulator conjugate, particle or composition is a CDP-rapamycin conjugate, particle or composition or a CDP-rapamycin analog conjugate, particle or composition.

In an embodiment, the autoimmune disease is an immune response to a transplanted organ, and the CDP-therapeutic agent conjugate, particle or composition is administered to the subject in combination with a second therapeutic agent. In an embodiment, the second therapeutic agent is one or more of the following agents: an anti-inflammatory agent, a corticosteroid, a disease modifying antirheumatic drug (DMARD), an immunomodulator, a statin, and/or a bisphosphonate, e.g., an anti-inflammatory agent, a corticosteroid, a disease modifying antirheumatic drug (DMARD), an immunomodulator, a statin, and/or a bisphosphonate disclosed herein. In an embodiment, the CDP-therapeutic agent conjugate, particle or composition is a CDP-rapamycin conjugate, particle or composition or a CDP-rapamycin analog conjugate, particle or composition, and the CDP-rapamycin conjugate, particle or composition or the CDP-rapamycin analog conjugate, particle or composition is administered to inhibit rejection of a transplanted organ, e.g., rejection of a kidney transplant, in combination with cyclosporine.

In another aspect, the invention features a method of treating lupus e.g., systemic lupus erythematosus, discoid lupus, drug-induced lupus, neonatal lupus, in a subject, e.g., a human subject, comprising administering a CDP-therapeutic agent conjugate, particle or composition to the subject in an amount effective to treat the lupus.

In an embodiment, the CDP-therapeutic agent conjugate, particle or composition is a CDP-topoisomerase inhibitor conjugate, particle or composition, e.g., a CDP-topoisomerase I inhibitor conjugate, particle or composition, e.g., a CDP-camptothecin or camptothecin derivative conjugate, particle or composition, e.g., CRLX101 and is administered to the subject at 30 mg/m² per month or less. In an embodiment, the CDP-topoisomerase I inhibitor conjugate is administered at 30 mg/m² per month or less on a dosing schedule described herein (wherein the dosage is expressed in mg of therapeutic agent, as opposed to mg of conjugate).

In one aspect, the invention features, a method of treating an autoimmune disease in a subject, e.g., a human subject. The method comprises:
providing an initial administration of a CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, to said subject at a dosage of 3 mg/m², 4 mg/m², 5 mg/m², or 6 mg/m² (wherein said dosage is expressed in mg of therapeutic agent, as opposed to mg of conjugate),
optionally, providing one or more subsequent administrations of said CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, at a dosage of 3 mg/m², 4 mg/m², 5 mg/m², or 6 mg/m², wherein each subsequent administration is provided, independently, between 5, 6, 7, 8, 9 days after the previous, e.g., the initial, administration, to thereby treat the autoimmune disease (when a range of individual values for parameter is given herein, the invention also includes a range for the parameter, wherein the upper and lower values for the parameter are selected from the individual values given. E.g., when a range of individual values for a dosage is given herein, the invention also includes a range for the dosage, wherein the upper and lower values for the range are selected from the individual values given. By way of example the individual values of 4 and 6 mg/m² given above provide a range of 4 and 6 mg/m². Similarly, when a range of individual values for a time period is given herein, the invention also includes a range for the time period, wherein the upper and lower values for the range are selected from the individual values given).

In an embodiment, the dosage of at least 2, 3, 4, 5, 6, 7, 8, 9, 10, 12, 15, or 20 administrations is the same.

In an embodiment, the time between at least 2, 3, 4, 5, 6, 7, 8, 9, 10, 12, 15, or 20 administrations is the same.

In an embodiment, each subsequent administration is administered 5-9, e.g., 7, days after the previous administration.

In an embodiment, at least 2, 3, 4, 5, 6, 7, 8, 9, 10, 12, 15, 20, 50 or 100 administrations are administered to the subject.

In one embodiment, the CDP-topoisomerase inhibitor conjugate, particle or composition, e.g., a CDP-camptothecin or camptothecin derivative, a CDP-camptothecin or camptothecin derivative conjugate, particle or composition described herein, e.g., CRLX101, is administered by intravenous administration over a period equal to or less than about 30 minutes, 45 minutes, 60 minutes, 90 minutes, 120 minutes, 150 minutes, or 180 minutes. In one embodiment, the CDP-topoisomerase inhibitor conjugate, particle or composition, e.g., a CDP-camptothecin or camptothecin derivative conjugate, particle or composition, e.g., the CDP-camptothecin or camptothecin derivative conjugate, particle or composition described herein, e.g. CRLX101, is administered at a dosage of 3 mg/m², 4 mg/m², 5 mg/m², or 6 mg/m² by intravenous administration over a period equal to or less than about 30 minutes, 45 minutes, 60 minutes or 90 minutes, e.g., a period equal to or less than 30 minutes, 45 minutes or 60 minutes.

In an embodiment, the method includes an initial administration of CRLX101 to said subject at a dosage of 3 mg/m², 4 mg/m², 5 mg/m², or 6 mg/m² and
one or more subsequent administrations of CRLX101 to said subject, at a dosage of 3 mg/m², 4 mg/m², 5 mg/m², or 6 mg/m², e.g., at the same dosage as the initial dosage, wherein each subsequent administration is administered, independently, 5-9, e.g., 7, days after the previous, e.g., the initial, administration, and the autoimmune disease is arthritis, e.g., rheumatoid arthritis, osteoarthritis, gout; lupus, e.g., systemic lupus erythematosus, discoid lupus, drug-induced lupus, neonatal lupus; inflammatory bowel disease, e.g., Crohn's disease, ulcerative colitis, collagenous colitis, lymphocytic colitis, ischemic colitis, diversion colitis, Behcet's syndrome, infective colitis, indeterminate colitis; psoriasis; or multiple sclerosis. In an embodiment, the autoimmune disease is lupus, e.g., systemic lupus erythematosus, discoid lupus, drug-induced lupus, neonatal lupus.

In one aspect, the invention features, a method of treating an autoimmune disease, e.g., in a subject, e.g., in a human subject. The method comprises:
providing an initial administration of a CDP-topoisomerase inhibitor I conjugate, particle or composition, e.g., a CDP-camptothecin conjugate, particle or composition or camptothecin derivative conjugate, particle or composition, e.g., a CDP-camptothecin conjugate, particle or composition or camptothecin derivative conjugate, particle or composition described herein, e.g., CRLX101, to the subject at a dosage of 6 mg/m², 7 mg/m², 8 mg/m², 9 mg/m², 10 mg/m², 11 mg/m², 12 mg/m², 13 mg/m², 14 mg/m², 15 mg/m², 16 mg/m², 17 mg/m², 18 mg/m², 19 mg/m², 20 mg/m², 21 mg/m², 22 mg/m², 23 mg/m², 24 mg/m², 25 mg/m², 26 mg/m², 27 mg/m², 28 mg/m², 29 mg/m² or 30 mg/m² (wherein said dosage is expressed in mg of therapeutic agent, as opposed to mg of conjugate) and
optionally, providing one or more subsequent administrations of said CDP-topoisomerase inhibitor conjugate, particle or composition, e.g., a CDP-camptothecin conjugate, particle or composition or camptothecin derivative conjugate, particle or composition, e.g., a CDP-camptothecin conjugate, particle or composition or camptothecin derivative conjugate, particle or composition described herein, e.g., CRLX101, at a dosage of 6 mg/m², 7 mg/m², 8 mg/m², 9 mg/m², 10 mg/m², 11 mg/m², 12 mg/m², 13 mg/m², 14 mg/m², 15 mg/m², 16 mg/m², 17 mg/m², 18 mg/m², 19 mg/m², 20 mg/m², 21 mg/m², 22 mg/m², 23 mg/m², 24 mg/m², 25 mg/m², 26 mg/m², 27 mg/m², 28 mg/m², 29 mg/m² or 30 mg/m², wherein each subsequent administration is provided, independently, between 9, 10, 11, 12, 13, 14, 15 or 16 days after the previous, e.g., the initial, administration, to thereby treat the autoimmune disease.

In an embodiment, the dosage of at least 2, 3, 4, 5, 6, 7, 8, 9, 10, 12, 15 or 20 administrations is the same.

In an embodiment, the time between at least 2, 3, 4, 5, 6, 7, 8, 9, 10, 12, 15, or 20 administrations is the same.

In an embodiment, each subsequent administration is administered 12-16, e.g., 14, days after the previous administration.

In an embodiment, at least 2, 3, 4, 5, 6, 7, 8, 9, 10, 12, 15, 20, 50 or 100 administrations are administered to the subject.

In one embodiment, the CDP-topoisomerase I inhibitor conjugate, particle or composition, e.g., a CDP-camptothecin or camptothecin derivative, a CDP-camptothecin or camptothecin derivative conjugate, particle or composition described herein, e.g., CRLX101, is administered by intravenous administration over a period equal to or less than about 30 minutes, 45 minutes, 60 minutes, 90 minutes, 120 minutes, 150 minutes, or 180 minutes. In one embodiment, the CDP-topoisomerase inhibitor conjugate, particle or composition, e.g., a CDP-camptothecin or camptothecin derivative conjugate, particle or composition, e.g., the CDP-camptothecin or camptothecin derivative conjugate, particle or composition described herein, e.g. CRLX101, is administered at a dosage of 9 mg/m², 10 mg/m², 11 mg/m², 12 mg/m², 13 mg/m², 14 mg/m², 15 mg/m², 16 mg/m², 17 mg/m², 18 mg/m², 19 mg/m², 20 mg/m², 21 mg/m², 22 mg/m², 23 mg/m², 24 mg/m², 25 mg/m², 26 mg/m², 27 mg/m², 28 mg/m², 29 mg/m² or 30 mg/m² by intravenous administration over a period equal to or less than about 30 minutes, 45 minutes, 60 minutes or 90 minutes, e.g., a period equal to or less than 30 minutes, 45 minutes or 60 minutes.

In an embodiment, the method includes an initial administration of CRLX101 to said subject at a dosage of 12 mg/m², 13 mg/m², 14 mg/m² or 15 mg/m², and one or more subsequent administrations of CRLX101 to said subject, at a dosage of 12 mg/m², 13 mg/m2, 14 mg/m² or 15 mg/m², e.g., at the same dosage as the initial dosage, wherein each subsequent administration is administered, independently, 12-16, e.g., 14, days after the previous, e.g., the initial, administration, and the autoimmune disease is arthritis, e.g., rheumatoid arthritis, osteoarthritis, gout; lupus, e.g., systemic lupus erythematosus, discoid lupus, drug-induced lupus, neonatal lupus; inflammatory bowel disease, e.g., Crohn's disease, ulcerative colitis, collagenous colitis, lymphocytic colitis, ischemic colitis, diversion colitis, Behcet's syndrome, infective colitis, indeterminate colitis; psoriasis; or multiple sclerosis. In an embodiment, the autoimmune disease is lupus, e.g., systemic lupus erythematosus, discoid lupus, drug-induced lupus, neonatal lupus.

In an embodiment, the method includes an initial administration of CRLX101 to said subject at a dosage of 16 mg/m², 17 mg/m², 18 mg/m², 19 mg/m², 20 mg/m², 21 mg/m², 22 mg/m², 23 mg/m², 24 mg/m², 25 mg/m², 26 mg/m², 27 mg/m², 28 mg/m², 29 mg/m² or 30 mg/m², and one or more subsequent administrations of CRLX101 to said subject, at a dosage of 16 mg/m², 17 mg/m², 18 mg/m², 19 mg/m², 20 mg/m², 21 mg/m², 22 mg/m², 23 mg/m², 24 mg/m², 25 mg/m², 26 mg/m², 27 mg/m², 28 mg/m², 29 mg/m² or 30 mg/m², e.g., at the same dosage as the initial dosage, wherein each subsequent administration is administered, independently, 12-16, e.g., 14, days after the previous, e.g., the initial, administration, and the autoimmune disease is arthritis, e.g., rheumatoid arthritis, osteoarthritis, gout; lupus, e.g., systemic lupus erythematosus, discoid lupus, drug-induced lupus, neonatal lupus; inflammatory bowel disease, e.g., Crohn's disease, ulcerative colitis, collagenous colitis, lymphocytic colitis, ischemic colitis, diversion colitis, Behcet's syndrome, infective colitis, indeterminate colitis; psoriasis; or multiple sclerosis. In an embodiment, the autoimmune disease is lupus, e.g., systemic lupus erythematosus, discoid lupus, drug-induced lupus, neonatal lupus.

In one aspect, the invention features, a method of treating an autoimmune disease, in a subject, e.g., a human subject. The method comprises:
providing an initial administration of a CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, to said subject at a dosage of 9 mg/m², 10 mg/m², 11 mg/m², 12 mg/m², 13 mg/m², 14 mg/m², 15 mg/m², 16 mg/m², 17 mg/m², 18 mg/m², 19 mg/m², 20 mg/m², 21 mg/m², 22 mg/m², 23 mg/m², 24 mg/m², 25 mg/m², 26 mg/m², 27 mg/m², 28 mg/m², 29 mg/m², 30 mg/m², 31 mg/m², 32 mg/m², 33 mg/m², 34 mg/m², 35 mg/m² or 36 mg/m² (wherein said dosage is expressed in mg of therapeutic agent, as opposed to mg of conjugate) and
optionally, providing one or more subsequent administrations of said CDP-topoisomerase inhibitor conjugate, particle or composition, e.g., a CDP-camptothecin or camptothecin derivative conjugate, particle or composition, e.g., a CDP-camptothecin or camptothecin derivative conjugate, particle or composition described herein, e.g., CRLX101, at a dosage of 9 mg/m², 10 mg/m², 11 mg/m², 12 mg/m², 13 mg/m², 14 mg/m², 15 mg/m², 16 mg/m², 17 mg/m², 18 mg/m², 19 mg/m², 20 mg/m², 21 mg/m², 22 mg/m², 23 mg/m², 24 mg/m², 25 mg/m², 26 mg/m², 27 mg/m², 28 mg/m², 29 mg/m², 30 mg/m², 31 mg/m², 32 mg/m², 33 mg/m², 34 mg/m², 35 mg/m² or 36 mg/m², wherein each subsequent administration is provided, independently, between 17, 18, 19, 20, 21, 22, 23, 24, 25, 26, 27, 28, 29, 30, or 31 days after the previous, e.g., the initial, administration, to thereby treat the autoimmune disease.

In an embodiment, the dosage of at least 2, 3, 4, 5, 6, 7, 8, 9, 10, 12, 15 or 20 administrations are the same.

In an embodiment, the time between at least 2, 3, 4, 5, 6, 7, 8, 9, 10, 12 15, or 20 administrations is the same.

In an embodiment, each subsequent administration is administered 19-23, e.g., 21, or 25-29, e.g., 27 or 28 days after the previous administration.

In an embodiment, at least 2, 3, 4, 5, 6, 7, 8, 9, 10, 12, 15, 20, 50 or 100 administrations are administered to the subject.

In one embodiment, the CDP-topoisomerase inhibitor conjugate, particle or composition, e.g., a CDP-camptothecin or camptothecin derivative, a CDP-camptothecin or camptothecin derivative conjugate, particle or composition described herein, e.g., CRLX101, is administered by intravenous administration over a period equal to or less than about 30 minutes, 45 minutes, 60 minutes, 90 minutes, 120 minutes, 150 minutes, or 180 minutes. In one embodiment, the CDP-topoisomerase inhibitor conjugate, particle or composition, e.g., a CDP-camptothecin or camptothecin derivative conjugate, particle or composition, e.g., the CDP-camptothecin or camptothecin derivative conjugate, particle or composition described herein, e.g. CRLX101, is administered at a dosage of 9 mg/m², 10 mg/m², 11 mg/m², 12 mg/m², 13 mg/m², 14 mg/m², 15 mg/m², 16 mg/m², 17 mg/m², 18 mg/m², 19 mg/m², 20 mg/m², 21 mg/m², 22 mg/m², 23 mg/m², 24 mg/m², 25 mg/m², 26 mg/m², 27 mg/m², 28 mg/m², 29 mg/m², 30 mg/m², 31 mg/m², 32 mg/m², 33 mg/m², 34 mg/m², 35 mg/m² or 36 mg/m² by intravenous administration over a period equal to or less than about 30 minutes, 45 minutes, 60 minutes or 90 minutes, e.g., a period equal to or less than 30 minutes, 45 minutes or 60 minutes.

In an embodiment, the method includes an initial administration of CRLX101 to said subject at a dosage of 18 mg/m², 19 mg/m², 20 mg/m², 21 mg/m², 22 mg/m², 23 mg/m², 24 mg/m², 25 mg/m², 26 mg/m², 27 mg/m², 28 mg/m², 29 mg/m², 30 mg/m², 31 mg/m², 32 mg/m², 33 mg/m², 34 mg/m², 35 mg/m² or 36 mg/m², and one or more subsequent administrations of CRLX101 to said subject, at a dosage of 18 mg/m², 19 mg/m2, 20 mg/m², 21 mg/m², 22 mg/m², 23 mg/m², 24 mg/m², 25 mg/m², 26 mg/m², 27 mg/m², 28 mg/m², 29 mg/m², 30 mg/m², 31 mg/m², 32 mg/m², 33 mg/m², 34 mg/m², 35 mg/m² or 36 mg/m², e.g., at the same dosage as the initial dosage, wherein each subsequent administration is administered, independently, 19-22, e.g., 21, days after the previous, e.g., the initial, administration, and the autoimmune disease is arthritis, e.g., rheumatoid arthritis, osteoarthritis, gout; lupus, e.g., systemic lupus erythematosus, discoid lupus, drug-induced lupus, neonatal lupus; inflammatory bowel disease, e.g., Crohn's disease, ulcerative colitis, collagenous colitis, lymphocytic colitis, ischemic colitis, diversion colitis, Behcet's syndrome, infective colitis, indeterminate colitis; psoriasis; or multiple sclerosis. In an embodiment, the autoimmune disease is lupus, e.g., systemic lupus erythematosus, discoid lupus, drug-induced lupus, neonatal lupus.

In one aspect, the invention features a method of treating lupus, e.g., systemic lupus erythematosus, discoid lupus, drug-induced lupus, neonatal lupus in a subject, e.g., a human subject. The method comprises: administering a CDP-therapeutic agent conjugate, particle or composition to the subject in combination with a second therapeutic agent. In one embodiment, the second therapeutic agent is one or more of the following agents: an anti-inflammatory agent, an anti-malarial agent, an immunomodulator, an anti-coagulant, and a hormone.

In one aspect, the invention features, a method of treating an autoimmune disease in a subject, e.g., a human subject. The method comprises:
providing an initial administration of CDP-anti-metabolic agent conjugate, particle or composition, e.g., a CDP-antifolate conjugate, particle or composition, e.g., a CDP-pemetrexed conjugate, particle or composition, e.g., a CDP-pemetrexed conjugate, particle or composition, described herein, or, e.g., a CDP-floxuridine conjugate, particle or composition, e.g., a CDP-floxuridine conjugate, particle or composition, described herein, or, e.g., a CDP-raltitrexed conjugate, particle or composition, e.g., a CDP-raltitrexed conjugate, particle or composition, described herein, to said subject, and, optionally, administering one or more subsequent administrations of said CDP-anti-metabolic agent conjugate, particle or composition, e.g., a CDP-antifolate conjugate, particle or composition, e.g., a CDP-pemetrexed conjugate, particle or composition, e.g., a CDP-pemetrexed conjugate, particle or composition, described herein, or, e.g., a CDP-floxuridine conjugate, particle or composition, e.g., a CDP-floxuridine conjugate, particle or composition, described herein, or, e.g., a CDP-raltitrexed conjugate, particle or composition, e.g., a CDP-raltitrexed conjugate, particle or composition, described herein, is administered, wherein each subsequent administration is provided, independently, between 5, 6, 7, 8, 9, 10, 11, 12, 13, 14, 15, 16, 17, 18, 19, 20, 21, 22, 23, 24, 25, 26, 27, 28 days after the previous, e.g., the initial, administration, to thereby treat the autoimmune disease.

In an embodiment, the dosage of at least 2, 3, 4, 5, 6, 7, 8, 9, 10, 12, 15, or 20 administrations is the same.

In an embodiment, the time between at least 2, 3, 4, 5, 6, 7, 8, 9, 10, 12, 15, or 20 administrations is the same.

In an embodiment, each subsequent administration is administered 18-24, e.g., 21, days after the previous administration.

In an embodiment, at least 2, 3, 4, 5, 6, 7, 8, 9, 10, 12, 15, 20, 50 or 100 administrations are administered to the subject.

In one embodiment, the CDP-anti-metabolic agent conjugate, particle or composition, e.g., a CDP-antifolate conjugate, particle or composition, e.g., a CDP-pemetrexed conjugate, particle or composition, e.g., a CDP-pemetrexed conjugate, particle or composition, described herein, or, e.g., a CDP-floxuridine conjugate, particle or composition, e.g., a CDP-floxuridine conjugate, particle or composition, described herein, or, e.g., a CDP-raltitrexed conjugate, particle or composition, e.g., a CDP-raltitrexed conjugate, particle or composition, described herein, is administered by intravenous administration over a period equal to or less than about 30 minutes, 45 minutes, 60 minutes, 90 minutes, 120 minutes, 150 minutes, or 180 minutes.

In an embodiment, the method includes an initial administration of a CDP-pemetrexed conjugate, particle or composition to said subject at a dosage of 300 mg/m², 320 mg/m², 350 mg/m², 380 mg/m², 400 mg/m², 420 mg/m², 450 mg/m², 480 mg/m², 500 mg/m², 520 mg/m², 550 mg/m², 580 mg/m², 600 mg/m², 620 mg/m², 650 mg/m², 680 mg/m², 700 mg/m², 720 mg/m², or 750 mg/m², (wherein the dosage is expressed in mg of therapeutic agent, as opposed to mg of conjugate), and one or more subsequent administrations of a CDP-pemetrexed conjugate, particle or composition to said subject, at a dosage of 300 mg/m², 320 mg/m², 350 mg/m², 380 mg/m², 400 mg/m², 420 mg/m², 450 mg/m², 480 mg/m², 500 mg/m², 520 mg/m², 550 mg/m², 580 mg/m², 600 mg/m², 620 mg/m², 650 mg/m², 680 mg/m², 700 mg/m², 720 mg/m², or 750 mg/m², e.g., at the same dosage as the initial dosage, wherein each subsequent administration is administered, independently, 18-24, e.g., 21 days after the previous, e.g., the initial, administration. In one embodiment, the autoimmune disease is arthritis, e.g., rheumatoid arthritis, osteoarthritis, gout; lupus, e.g., systemic lupus erythematosus, discoid lupus, drug-induced lupus, neonatal lupus; inflammatory bowel disease, e.g., Crohn's disease, ulcerative colitis, collagenous colitis, lymphocytic colitis, ischemic colitis, diversion colitis, Behcet's syndrome, infective colitis, indeterminate colitis; psoriasis; or multiple sclerosis. In an embodiment, the autoimmune disease is lupus, e.g., systemic lupus erythematosus, discoid lupus, drug-induced lupus, neonatal lupus.

In one aspect, the invention features, a method of treating an autoimmune disease in a subject, e.g., a human subject. The method comprises:
providing an initial administration of CDP-pyrimidine analog conjugate, particle or composition, e.g., a CDP-capecitabine conjugate, particle or composition, e.g., a CDP-capecitabine conjugate, particle or composition, described herein, or, e.g., a CDP-cytarabine conjugate, particle or composition, e.g., a CDP-cytarabine conjugate, particle or composition, described herein, or, e.g., a CDP-gemcitabine conjugate, particle or composition, e.g., a CDP-gemcitabine conjugate, particle or composition, described herein, or, e.g., a CDP-5FU conjugate, particle or composition, e.g., a CDP-5FU conjugate, particle or composition, described herein, to said subject, and, optionally, providing one or more subsequent administrations of said CDP-pyrimidine analog conjugate, particle or composition, e.g., a CDP-antifolate conjugate, particle or composition, e.g., a CDP-capecitabine conjugate, particle or composition, e.g., a CDP-capecitabine conjugate, particle or composition, described herein, or, e.g., a CDP-cytarabine conjugate, particle or composition, e.g., a CDP-cytarabine conjugate, particle or composition, described herein, or, e.g., a CDP-gemcitabine conjugate, particle or composition, e.g., a CDP-gemcitabine conjugate, particle or composition, described herein, or, e.g., a CDP-5FU conjugate, particle or composition, e.g., a CDP-5FU conjugate, particle or composition, described herein, wherein each subsequent administration is provided, independently, between 5, 6, 7, 8, 9, 10, 11, 12, 13, 14, 15, 16, 17, 18, 19, 20, 21, 22, 23, 24, 25, 26, 27, 28 days after the previous, e.g., the initial, administration, to thereby treat the autoimmune disease.

In an embodiment, the dosage of at least 2, 3, 4, 5, 6, 7, 8, 9, 10, 12, 15, or 20 administrations is the same.

In an embodiment, the time between at least 2, 3, 4, 5, 6, 7, 8, 9, 10, 12, 15, or 20 administrations is the same.

In an embodiment, each subsequent administration is administered 5-14 days, e.g., 7 days after the previous administration.

In an embodiment, at least 2, 3, 4, 5, 6, 7, 8, 9, 10, 12, 15, 20, 50 or 100 administrations are administered to the subject.

In one embodiment, the CDP-pyrimidine analog conjugate, particle or composition, e.g., a CDP-antifolate conjugate, particle or composition, e.g., a CDP-capecitabine conjugate, particle or composition, e.g., a CDP-capecitabine conjugate, particle or composition, described herein, or, e.g., a CDP-cytarabine conjugate, particle or composition, e.g., a CDP-cytarabine conjugate, particle or composition, described herein, or, e.g., a CDP-gemcitabine conjugate, particle or composition, e.g., a CDP-gemcitabine conjugate, particle or composition, described herein, or, e.g., a CDP-5FU conjugate, particle or composition, e.g., a CDP-5FU conjugate, particle or composition, described herein, is administered by intravenous administration over a period equal to or less than about 30 minutes, 45 minutes, 60 minutes, 90 minutes, 120 minutes, 150 minutes, or 180 minutes.

In an embodiment, the method includes an initial administration of a CDP-gemcitabine conjugate, particle or composition at a dosage of 600 mg/m², 700 mg/m², 730 mg/m², 750 mg/m², 780 mg/m², 800 mg/m², 830 mg/m², 850 mg/m², 880 mg/m², 900 mg/m², 930 mg/m², 950 mg/m², 980 mg/m², 1000 mg/m², 1030 mg/m², 1050 mg/m², 1080 mg/m², 1100 mg/m², 1130 mg/m², 1150 mg/m², 1180 mg/m², 1200 mg/m², 1230 mg/m², 1250 mg/m², 1280 mg/m², 1300 mg/m², 1330 mg/m², 1350 mg/m², 1380 mg/m², 1400 mg/m², 1430 mg/m², 1450 mg/m², 1480 mg/m², 1500 mg/m², 1530 mg/m², 1580 mg/m2, 1600 mg/m², 1630 mg/m², or 1650 mg/m² (wherein the dosage is expressed in mg of therapeutic agent, as opposed to mg of conjugate), and, optionally, one or more subsequent administrations of a CDP-gemcitabine conjugate, particle or composition at a dosage of 600 mg/m², 700 mg/m², 730 mg/m², 750 mg/m², 780 mg/m², 800 mg/m², 830 mg/m², 850 mg/m², 880 mg/m², 900 mg/m², 930 mg/m², 950 mg/m², 980 mg/m², 1000 mg/m², 1030 mg/m², 1050 mg/m², 1080 mg/m², 1100 mg/m², 1130 mg/m², 1150 mg/m², 1180 mg/m², 1200 mg/m², 1230 mg/m², 1250 mg/m², 1280 mg/m², 1300 mg/m², 1330 mg/m², 1350 mg/m², 1380 mg/m², 1400 mg/m², 1430 mg/m², 1450 mg/m², 1480 mg/m², 1500 mg/m², 1530 mg/m², 1580 mg/m², 1600 mg/m², 1630 mg/m², or 1650 mg/m², e.g., at the same dosage as the initial dosage, wherein each subsequent administration is provided, independently, between 5, 6, 7, 8, 9, 10, 11, 12, 13, 14, 15 or 16 days after the previous, e.g., the initial, administration. In one embodiment, the autoimmune disease is arthritis, e.g., rheumatoid arthritis, osteoarthritis, gout; lupus, e.g., systemic lupus erythematosus, discoid lupus, drug-induced lupus, neonatal lupus; inflammatory bowel disease, e.g., Crohn's disease, ulcerative colitis, collagenous colitis, lymphocytic colitis, ischemic colitis, diversion colitis, Behcet's syndrome, infective colitis, indeterminate colitis; psoriasis; or multiple sclerosis. In an embodiment, the autoimmune disease is lupus, e.g., systemic lupus erythematosus, discoid lupus, drug-induced lupus, neonatal lupus.

In an embodiment, the method includes an initial administration of a CDP-5FU conjugate, particle or composition at a dosage of 1 mg/kg, 2 mg/kg, 3 mg/kg, 4 mg/kg, 5 mg/kg, 6 mg/kg, 7 mg/kg, 8 mg/kg, 9 mg/kg, 10 mg/kg, 11 mg/kg, 12 mg/kg, 13 mg/kg, 14 mg/kg, 15 mg/kg, 16 mg/kg, 17 mg/kg, 18 mg/kg, 19 mg/kg, or 20 mg/kg (wherein the dosage is expressed in mg of therapeutic agent, as opposed to mg of conjugate), and, optionally, one or more subsequent administrations of a CDP-5FU conjugate, particle or composition at a dosage of 1 mg/kg, 2 mg/kg, 3 mg/kg, 4 mg/kg, 5 mg/kg, 6 mg/kg, 7 mg/kg, 8 mg/kg, 9 mg/kg, 10 mg/kg, 11 mg/kg, 12 mg/kg, 13 mg/kg, 14 mg/kg, 15 mg/kg, 16 mg/kg, 17 mg/kg, 18 mg/kg, 19 mg/kg, or 20 mg/kg, e.g., at the same dosage as the initial dosage, wherein each subsequent administration is provided, independently, between 1, 2, 3, 4, 5, 6, 7, 8, 9, 10, 11, 12, 13, 14, day(s) after the previous, e.g., the initial, administration, and the autoimmune disease is arthritis, e.g., rheumatoid arthritis, osteoarthritis, gout; lupus, e.g., systemic lupus erythematosus, discoid lupus, drug-induced lupus, neonatal lupus; inflammatory bowel disease, e.g., Crohn's disease, ulcerative colitis, collagenous colitis, lymphocytic colitis, ischemic colitis, diversion colitis, Behcet's syndrome, infective colitis, indeterminate colitis; psoriasis; or multiple sclerosis. In an embodiment, the autoimmune disease is lupus, e.g., systemic lupus erythematosus, discoid lupus, drug-induced lupus, neonatal lupus. In an embodiments, the CDP-5FU conjugate, particle or composition is administered intravenously once daily for 4 successive days.

In one aspect, the invention features, a method of treating an autoimmune disease in a subject, e.g., a human subject. The method comprises:
providing an initial administration of a CDP-anti-tumor antibiotic conjugate, particle or composition, e.g., a CDP-HSP90 inhibitor conjugate, particle or composition, e.g., a CDP-geldanamycin conjugate, particle or composition, e.g., a CDP-geldanamycin conjugate, particle or composition described herein, to said subject, and, optionally, providing one or more subsequent administrations of said CDP-anti-tumor antibiotic conjugate, particle or composition, e.g., a CDP-HSP90 inhibitor conjugate, particle or composition, e.g., a CDP-geldanamycin conjugate, particle or composition, e.g., a CDP-geldanamycin conjugate, particle or composition described herein, wherein each subsequent administration is provided, independently, between 1, 2, 3, 4, 5, 6, 7, 8, 9, 10, 11, 12, 13, 14, 15, 16, 17, 18, 19, 20, 21 days after the previous, e.g., the initial, administration, to thereby treat the autoimmune disease.

In an embodiment, the dosage of at least 2, 3, 4, 5, 6, 7, 8, 9, 10, 12, 15, or 20 administrations is the same.

In an embodiment, the time between at least 2, 3, 4, 5, 6, 7, 8, 9, 10, 12, 15, or 20 administrations is the same.

In an embodiment, each subsequent administration is administered 1-15, e.g., 3 or 7, days after the previous administration.

In an embodiment, at least 2, 3, 4, 5, 6, 7, 8, 9, 10, 12, 15, 20, 50 or 100 administrations are administered to the subject.

In one embodiment, the CDP-anti-tumor antibiotic conjugate, particle or composition, e.g., a CDP-HSP90 inhibitor conjugate, particle or composition, e.g., a CDP-geldanamycin conjugate, particle or composition, e.g., a CDP-geldanamycin conjugate, particle or composition described herein, is administered by intravenous administration over a period equal to or less than about 30 minutes, 45 minutes, 60 minutes, 90 minutes, 120 minutes, 150 minutes, or 180 minutes.

In an embodiment, the method includes an initial administration of a CDP-geldanamycin conjugate, particle or composition at a dosage of 20 mg/m², 30 mg/m², 40 mg/m², 50 mg/m², 60 mg/m², 70 mg/m², 75 mg/m², 80 mg/m², 85 mg/m², 90 mg/m², 95 mg/m², 100 mg/m², 105 mg/m², 110 mg/m², 115 mg/m², 120 mg/m², 125 mg/m², 130 mg/m², 140 mg/m², 150 mg/m², 160 mg/m², or 170 mg/m² (wherein the dosage is expressed in mg of therapeutic agent, as opposed to mg of conjugate), and, optionally, one or more subsequent administrations of a CDP-geldanamycin conjugate, particle or composition at a dosage of 20 mg/m², 30 mg/m², 40 mg/m², 50 mg/m², 60 mg/m², 70 mg/m², 75 mg/m², 80 mg/m², 85 mg/m², 90 mg/m², 95 mg/m², 100 mg/m², 105 mg/m², 110 mg/m², 115 mg/m², 120 mg/m², 125 mg/m², 130 mg/m², 140 mg/m², 150 mg/m², 160 mg/m², or 170 mg/m², e.g., at the same dosage as the initial dosage, wherein each subsequent administration is provided, independently, between 1, 2, 3, 4, 5, 6, 7, 8, 9, 10, 11, 12, 13, 14, 15, 16, 17, 18, 19, 20, 21 days after the previous, e.g., the initial, administration. In one embodiment, the autoimmune disease is arthritis, e.g., rheumatoid arthritis, osteoarthritis, gout; lupus, e.g., systemic lupus erythematosus, discoid lupus, drug-induced lupus, neonatal lupus; inflammatory bowel disease, e.g., Crohn's disease, ulcerative colitis, collagenous colitis, lymphocytic colitis, ischemic colitis, diversion colitis, Behcet's syndrome, infective colitis, indeterminate colitis; psoriasis; or multiple sclerosis. In an embodiment, the autoimmune disease is lupus, e.g., systemic lupus erythematosus, discoid lupus, drug-induced lupus, neonatal lupus.

In one aspect, the invention features, a method of treating an autoimmune disease in a subject, e.g., a human subject. The method comprises:
providing an initial administration of CDP-platinum based agent conjugate, particle or composition, e.g., a CDP-cisplatin conjugate, particle or composition, e.g., a CDP-cisplatin conjugate, particle or composition, described herein, or, e.g., a CDP-carboplatin conjugate, particle or composition, e.g., a CDP-carboplatin conjugate, particle or composition, described herein, or, e.g., a CDP-oxaliplatin conjugate, particle or composition, e.g., a CDP-oxaliplatin conjugate, particle or composition, described herein, and, optionally, providing one or more subsequent administrations of said CDP-platinum based agent conjugate, particle or composition, e.g., a CDP-cisplatin conjugate, particle or composition, e.g., a CDP-cisplatin conjugate, particle or composition, described herein, or, e.g., a CDP-carboplatin conjugate, particle or composition, e.g., a CDP-carboplatin conjugate, particle or composition, described herein, or, e.g., a CDP-oxaliplatin conjugate, particle or composition, e.g., a CDP-oxaliplatin conjugate, particle or composition, described herein wherein each subsequent administration is provided, independently, between 1, 2, 3, 4, 5, 6, 7, 8, 9, 10, 11, 12, 13, 14, 15, 16, 17, 18, 19, 20, 21, 22, 23, 24, 25, 26, 27, 28, 39, 30, 31 day(s) after the previous, e.g., the initial, administration, to thereby treat the autoimmune disease.

In an embodiment, the dosage of at least 2, 3, 4, 5, 6, 7, 8, 9, 10, 12, 15, or 20 administrations is the same.

In an embodiment, the time between at least 2, 3, 4, 5, 6, 7, 8, 9, 10, 12, 15, or 20 administrations is the same.

In an embodiment, each subsequent administration is administered 17-31 days, e.g., 21 or 28, days after the previous administration. In an embodiment, each subsequent administration is administered 1-5 days, e.g., 1, 3 day(s) after the previous administration.

In an embodiment, at least 2, 3, 4, 5, 6, 7, 8, 9, 10, 12, 15, 20, 50 or 100 administrations are administered to the subject.

In one embodiment, the CDP-platinum based agent conjugate, particle or composition, e.g., a CDP-cisplatin conjugate, particle or composition, e.g., a CDP-cisplatin conjugate, particle or composition, described herein, or, e.g., a CDP-carboplatin conjugate, particle or composition, e.g., a CDP-carboplatin conjugate, particle or composition, described herein, or, e.g., a CDP-oxaliplatin conjugate, particle or composition, e.g., a CDP-oxaliplatin conjugate, particle or composition, described herein, is administered by intravenous administration over a period equal to or less than about 30 minutes, 45 minutes, 60 minutes, 90 minutes, 120 minutes, 150 minutes, or 180 minutes.

In an embodiment, the method includes an initial administration of a CDP-cisplatin conjugate, particle or composition at a dosage of 10 mg/m², 15 mg/m², 20 mg/m², 25 mg/m², 30 mg/m², 40 mg/m², 50 mg/m², 60 mg/m², 70 mg/m², 75 mg/m², 80 mg/m², 85 mg/m², 90 mg/m², 95 mg/m², 100 mg/m², 105 mg/m², 110 mg/m², 115 mg/m², 120 mg/m², 125 mg/m², 130 mg/m², 140 mg/m², 150 mg/m², 160 mg/m², or 170 mg/m² (wherein the dosage is expressed in mg of therapeutic agent, as opposed to mg of conjugate), and, optionally, one or more subsequent administrations of a CDP-cisplatin conjugate, particle or composition at a dosage of 10 mg/m², 15 mg/m², 20 mg/m², 25 mg/m², 30 mg/m², 40 mg/m², 50 mg/m², 60 mg/m², 70 mg/m², 75 mg/m², 80 mg/m², 85 mg/m², 90 mg/m², 95 mg/m², 100 mg/m², 105 mg/m², 110 mg/m², 115 mg/m², 120 mg/m², 125 mg/m², 130 mg/m², 140 mg/m², 150 mg/m², 160 mg/m², or 170 mg/m², e.g., at the same dosage as the initial dosage, wherein each subsequent administration is provided, independently, between 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, or 31 day(s) after the previous, e.g., the initial, administration. In one embodiment, the autoimmune disease is arthritis, e.g., rheumatoid arthritis, osteoarthritis, gout; lupus, e.g., systemic lupus erythematosus, discoid lupus, drug-induced lupus, neonatal lupus; inflammatory bowel disease, e.g., Crohn's disease, ulcerative colitis, collagenous colitis, lymphocytic colitis, ischemic colitis, diversion colitis, Behcet's syndrome, infective colitis, indeterminate colitis; psoriasis; or multiple sclerosis. In an embodiment, the autoimmune disease is lupus, e.g., systemic lupus erythematosus, discoid lupus, drug-induced lupus, neonatal lupus.

In one aspect, the invention features, a method of treating an autoimmune disease in a subject, e.g., a human subject. The method comprises:
providing an initial administration of CDP-kinase inhibitor conjugate, particle or composition, e.g., a CDP-seronine/threonine kinase inhibitor conjugate, particle or composition, e.g., a CDP-mTOR inhibitor conjugate, particle or composition, e.g., a CDP-rapamycin conjugate, particle or composition, e.g., a CDP-rapamycin conjugate, particle or composition, described herein, and, optionally, providing one or more subsequent administrations of said CDP-kinase inhibitor conjugate, particle or composition, e.g., a CDP-seronine/threonine kinase inhibitor conjugate, particle or composition, e.g., a CDP-mTOR inhibitor conjugate, particle or composition, e.g., a CDP-rapamycin conjugate, particle or composition, e.g., a CDP-rapamycin conjugate, particle or composition, described herein, to said subject wherein each subsequent administration is provided, independently, between 1, 2, 3, 4, 5, 6, 7, 8, 9, 10, 11, 12, 13, 14, 15, 16, 17, 18, 19, 20, 21day(s) after the previous, e.g., the initial, administration, to thereby treat the autoimmune disease.

In an embodiment, the dosage of at least 2, 3, 4, 5, 6, 7, 8, 9, 10, 12, 15, or 20 administrations is the same.

In an embodiment, the time between at least 2, 3, 4, 5, 6, 7, 8, 9, 10, 12, 15, or 20 administrations is the same.

In an embodiment, each subsequent administration is administered 1-21 days, e.g., 1, 2, 3, 4 or 5, days after the previous administration. In an embodiment, each subsequent administration is administered 1-5 days, e.g., 1, 3 day(s) after the previous administration.

In an embodiment, at least 2, 3, 4, 5, 6, 7, 8, 9, 10, 12, 15, 20, 50 or 100 administrations are administered to the subject.

In one embodiment, the CDP-kinase inhibitor agent conjugate, particle or composition, e.g., a CDP-rapamycin conjugate, particle or composition, e.g., a CDP-rapamycin conjugate, particle or composition, described herein is administered by intravenous administration over a period equal to or less than about 30 minutes, 45 minutes, 60 minutes, 90 minutes, 120 minutes, 150 minutes, or 180 minutes.

In an embodiment, the method includes an initial administration of a CDP-rapamycin conjugate, particle or composition at a dosage of 2 mg, 3 mg, 4 mg, 5 mg, 6 mg, 7 mg, 8 mg, 9 mg, 10 mg, 12 mg, 15 mg, 20 mg, 25 mg, 30 mg, 35 mg, 40 mg, 45 mg, or 50 mg (wherein the dosage is expressed in mg of therapeutic agent, as opposed to mg of conjugate), and, optionally, one or more subsequent administrations of a CDP-rapamycin conjugate, particle or composition at a dosage of 2 mg, 3 mg, 4 mg, 5 mg, 6 mg, 7 mg, 8 mg, 9 mg, 10 mg, 12 mg, 15 mg, 20 mg, 25 mg, 30 mg, 35 mg, 40 mg, 45 mg, or 50 mg, e.g., at the same dosage as the initial dosage, wherein each subsequent administration is provided, independently, between 1, 2, 3, 4, 5, 6, 7, 8, 9, 10, 11, 12, 13, 14, 15, 16, 17, 18, 19, 20, or 21day(s) after the previous, e.g., the initial, administration. In one embodiment, the autoimmune disease is arthritis, e.g., rheumatoid arthritis, osteoarthritis, gout; lupus, e.g., systemic lupus erythematosus, discoid lupus, drug-induced lupus, neonatal lupus; inflammatory bowel disease, e.g., Crohn's disease, ulcerative colitis, collagenous colitis, lymphocytic colitis, ischemic colitis, diversion colitis, Behcet's syndrome, infective colitis, indeterminate colitis; psoriasis; or multiple sclerosis. In an embodiment, the autoimmune disease is lupus, e.g., systemic lupus erythematosus, discoid lupus, drug-induced lupus, neonatal lupus.

In an embodiment of the aspects provided above, the CDP-therapeutic agent conjugate, particle or composition (e.g., the CDP-cytotoxic agent conjugate, particle or composition) is administered in combination with an anti-inflammatory agent which is one or more of the following agents: aspirin, acetaminophen, a non-steroidal anti-inflammatory drug, and/or a corticosteroid.

In an embodiment of the aspects provided above, the CDP-therapeutic agent conjugate, particle or composition (e.g., the CDP-cytotoxic agent conjugate, particle or composition) is administered in combination with an anti-malarial agent which is one or more of the following agents: hydroxychloroquine and/or chloroquine.

In an embodiment of the aspects provided above, the CDP-therapeutic agent conjugate, particle or composition (e.g., the CDP-cytotoxic agent conjugate, particle or composition) is administered in combination with an immunomodulator which is one or more of the following agents: an immunomodulator with an intracellular target (e.g., a macrolide), an immunomodulator with a cellular receptor target, an immunomodulator with a serum target, and/or other agents that interfere with immune cell function (e.g., thalidomide, mycophenolate mofetil, tacrolimus, pimecrolimus, cyclosporine (e.g., cyclosporine A), rapamycin and rapamycin analogs-some of these agents may also belong to another class of agents described herein).

In an embodiment of the aspects provided above, the CDP-therapeutic agent conjugate, particle or composition (e.g., the CDP-cytotoxic agent conjugate, particle or composition) is administered in combination with an immunomodulator wherein an intracellular target is an anti-metabolite (e.g., an alkylating agent (e.g., cyclophosphamide (e.g., Cytoxan®), a purine synthesis inhibitor (e.g., azathioprine (Imuran®), a pyrimidine synthesis inhibitor (e.g., leflunomide (Arava®), an antifolate (e.g., methotrexate), an IL-2 inhibitor, an mTOR inhibitor, a TNF inhibitor, or a macrolide.

In an embodiment of the aspects provided above, the CDP-therapeutic agent conjugate, particle or composition (e.g., the CDP-cytotoxic agent conjugate, particle or composition) is administered in combination with an immunomodulator wherein the receptor target is an IL-1 receptor inhibitor or an antibody which inhibits the function of the cellular receptor target. Examples of antibodies which inhibit the function of a cellular receptor target include an anti-CD3 antibody, an anti-CD4 antibody, an anti-CD5 antibody, an anti-CD11a antibody, anti-BLyS antibody, an anti-CD20 antibody, an anti-CD22 antibody, an anti-CD23 antibody, an anti-CD40 antibody, an anti-CD62L antibody, an anti-CD80 antibody, an anti-CD147 antibody, an anti-CD154 antibody, an anti-CAT antibody, an anti-integrin antibody, an CTLA4 antibody, an anti-IL6 receptor antibody, an anti-LFAl antibody, an anti-IL2 antibody, and an anti-human T cell antibody.

In an embodiment of the aspects provided above, the CDP-therapeutic agent conjugate, particle or composition (e.g., the CDP-cytotoxic agent conjugate, particle or composition) is administered in combination with an immunomodulator wherein the serum target is an antibody which inhibits the function of the serum target. Examples of antibodies which inhibit the function of a serum target include an anti-BLyS antibody, an anti-IL5 antibody, anti-IL6 antibody, and anti-interferon alpha antibody, an anti-IgE antibody, an anti-C5a antibody, an anti-TNF antibody, anti-ILlO antibody, anti-IL12 antibody, and an anti-IL13 antibody. Other immunomodulators can be soluble forms of the cellular receptor targets described herein. A preferred antibody which inhibits the function of a serum target is an anti-BLyS antibody, e.g., belimumab (Benlysta™).

In an embodiment of the aspects provided above, the CDP-therapeutic agent conjugate, particle or composition (e.g., the CDP-cytotoxic agent conjugate, particle or composition) is administered in combination with an anti-coagulant which is one or more of the following agents: aspirin, heparin, and/or warfarin.

In an embodiment of the aspects provided above, the CDP-therapeutic agent conjugate, particle or composition (e.g., the CDP-cytotoxic agent conjugate, particle or composition) is administered in combination with a hormone which selected from the group consisting of an androgen and/or a gonadotropin-hormone releasing agonist.

In one aspect, the invention features, a method of treating an autoimmune disease, e.g., in a subject. The method comprises administering two or more CDP-therapeutic agent conjugates, wherein one CDP is conjugated to a therapeutic agent and the other CDP is conjugated to a second therapeutic agent, or a composition or particle including one or more of the CDP-therapeutic agent conjugates, to the subject to thereby treat the disease.

In an embodiment, the CDP-therapeutic agent conjugate, particle or composition is a CDP-cytotoxic agent conjugate, particle or composition, e.g., CDP-topoisomerase inhibitor conjugate, particle or composition, e.g., a CDP-topoisomerase inhibitor I conjugate, particle or composition (e.g., a CDP-camptothecin conjugate, particle or composition, CDP-irinotecan conjugate, particle or composition, CDP-SN-38 conjugate, particle or composition, CDP-topotecan conjugate, particle or composition, CDP-lamellarin D conjugate, particle or composition, a CDP-lurotecan conjugate, particle or composition, a CDP-exatecan conjugate, particle or composition, a CDP-diflomotecan conjugate, particle or composition and CDP-topoisomerase I inhibitor conjugates, particles and compositions which include derivatives of camptothecin, irinotecan, SN-38, lamellarin D, lurotecan, exatecan and diflomotecan), a CDP-topoisomerase II inhibitor conjugate, particle or composition (e.g., a CDP-etoposide conjugate, particle, or composition, CDP-tenoposide conjugate, particle or composition, CDP-amsacrine conjugate, particle or composition and CDP-topoisomerase II inhibitor conjugates, particles and compositions which include derivatives of etoposide, tenoposide, and amsacrine), a CDP-anti-metabolic agent conjugate, particle or composition (e.g., a CDP-antifolate conjugate, particle or composition (e.g., a CDP-pemetrexed conjugate, particle or composition, a CDP-floxuridine conjugate, particle or composition, a CDP-raltitrexed conjugate, particle or composition)) or a CDP-pyrimidine analog conjugate, particle or composition (e.g., a CDP-capecitabine conjugate, particle or composition, a CDP-cytarabine conjugate, particle or composition, a CDP-gemcitabine conjugate, particle or composition, a CDP-5FU conjugate, particle or composition)), a CDP-alkylating agent conjugate, particle or composition, a CDP-anthracycline conjugate, particle or composition, a CDP-anti-tumor antibiotic conjugate, particle or composition (e.g., a CDP-HSP90 inhibitor conjugate, particle or composition, e.g., a CDP-geldanamycin conjugate, particle or composition, a CDP-tanespimycin conjugate, particle or composition or a CDP-alvespimycin conjugate, particle or composition), a CDP-platinum based agent conjugate, particle or composition (e.g., a CDP-cisplatin conjugate, particle or composition, a CDP-carboplatin conjugate, particle or composition, a CDP-oxaliplatin conjugate, particle or composition), a CDP-microtubule inhibitor conjugate, particle or composition, a CDP-kinase inhibitor conjugate, particle or composition (e.g., a CDP-seronine/threonine kinase inhibitor conjugate, particle or composition, e.g., a CDP-mTOR inhibitor conjugate, particle or composition, e.g., a CDP-rapamycin conjugate, particle or composition) or a CDP-proteasome inhibitor conjugate, particle or composition.

In an embodiment, the CDP-therapeutic agent conjugate, particle or composition is a CDP-immunomodulator conjugate, particle or composition, e.g., a corticosteroid or a rapamycin analog conjugate, particle or composition.

In another aspect, the invention features, a unit dosage of a CDP-therapeutic agent conjugate described herein, and particles and compositions containing a CDP-therapeutic agent conjugate described herein.

In one aspect, the disclosure features a CDP-therapeutic agent conjugate, particle or composition, e.g., a CDP-therapeutic agent conjugate, particle or composition described herein.

In one embodiment, the CDP-therapeutic agent conjugate, particle or composition is a CDP-cytotoxic agent conjugate, particle or composition, e.g.:
a CDP-topoisomerase inhibitor conjugate, particle or composition, e.g., a CDP-topoisomerase inhibitor I conjugate, particle or composition (e.g., a CDP-camptothecin conjugate, particle or composition, CDP-irinotecan conjugate, particle or composition, CDP-SN-38 conjugate, particle or composition, CDP-topotecan conjugate, particle or composition, CDP-lamellarin D conjugate, particle or composition, a CDP-lurotecan conjugate, particle or composition, a CDP-exatecan conjugate, particle or composition, a CDP-diflomotecan conjugate, particle or composition, and CDP-topoisomerase I inhibitor conjugates, particles and compositions which include derivatives of camptothecin, irinotecan, SN-38, lamellarin D, lurotecan, exatecan, and diflomotecan);
a CDP-topoisomerase II inhibitor conjugate, particle or composition (e.g., a CDP-etoposide conjugate, particle, or composition, CDP-tenoposide conjugate, particle or composition, CDP-amsacrine conjugate, particle or composition and CDP-topoisomerase II inhibitor conjugates, particles and compositions which include derivatives of etoposide, tenoposide, and amsacrine);
a CDP-anti-metabolic agent conjugate, particle or composition (e.g., a CDP-antifolate conjugate, particle or composition (e.g., a CDP-pemetrexed conjugate, particle or composition, a CDP-floxuridine conjugate, particle or composition, a CDP-raltitrexed conjugate, particle or composition) or a CDP-pyrimidine analog conjugate, particle or composition (e.g., a CDP-capecitabine conjugate, particle or composition, a CDP-cytarabine conjugate, particle or composition, a CDP-gemcitabine conjugate, particle or composition, a CDP-5FU conjugate, particle or composition));
a CDP-alkylating agent conjugate, particle or composition, a CDP-anthracycline conjugate, particle or composition;
a CDP-anti-tumor antibiotic conjugate, particle or composition (e.g., a CDP-HSP90 inhibitor conjugate, particle or composition, e.g., a CDP-geldanamycin conjugate, particle or composition, a CDP-tanespimycin conjugate, particle or composition or a CDP-alvespimycin conjugate, particle or composition);
a CDP-platinum based agent conjugate, particle or composition (e.g., a CDP-cisplatin conjugate, particle or composition, a CDP-carboplatin conjugate, particle or composition, a CDP-oxaliplatin conjugate, particle or composition);
a CDP-microtubule inhibitor conjugate, particle or composition;
a CDP-kinase inhibitor conjugate, particle or composition (e.g., a CDP-seronine/threonine kinase inhibitor conjugate, particle or composition, e.g., a CDP-mTOR inhibitor conjugate, particle or composition, e.g., a CDP-rapamycin conjugate, particle or composition);
or a CDP-proteasome inhibitor, e.g., bortezomib, conjugate, particle or composition.

In one embodiment, the CDP-therapeutic agent conjugate, particle or composition is a CDP-immunomodulator conjugate, particle or composition; e.g.,
a CDP-corticosteroid conjugate, particle or composition; or
a CDP-kinase inhibitor conjugate, particle or composition (e.g., a CDP-seronine/threonine kinase inhibitor conjugate, particle or composition, e.g., a CDP-mTOR inhibitor conjugate, particle or composition, e.g., a CDP-rapamycin conjugate, particle or composition).

In an embodiment, the CDP-therapeutic agent conjugate, particle or composition is a CDP-corticosteroid conjugate, particle or composition wherein the corticosteroid is not (or is other than) methylprednisolone. In an embodiment, the CDP-therapeutic agent conjugate, particle or composition is a CDP-corticosteroid conjugate, particle or composition wherein the corticosteroid is a Group B corticosteroid, a Group C corticosteroid, or a Group D corticosteroid. In an embodiment, the CDP-therapeutic agent conjugate, particle or composition is a CDP-corticosteroid conjugate, particle or composition wherein the corticosteroid is hydrocortisone, hydrocortisone acetate, cortisone acetate, tixocortol pivalate, prednisolone, methylprednisolone, or prednisone. In an embodiment, the CDP-therapeutic agent conjugate, particle or composition is a CDP-corticosteroid conjugate, particle or composition wherein the corticosteroid is a Group B corticosteroid, a Group C corticosteroid, a Group D corticosteroid, hydrocortisone, hydrocortisone acetate, cortisone acetate, tixocortol pivalate, prednisolone, methylprednisolone, or prednisone. In an embodiment, the CDP-corticosteroid conjugate, e.g., the CDP-methylprednisolone conjugate, includes a linker attaching the corticosteroid to the CDP, wherein the linker is not a glycine. In one embodiment, the linker is one ore more of: alanine, arginine, histidine, lysine, aspartic acid, glutamic acid, serine, threonine, asparganine, glutamine, cysteine, proline, isoleucine, leucine, methionine, phenylalanine, tryptophan, tyrosine and valine. In some embodiments, the linker is a linker described herein. In some embodiments, the linker is not an amino acid (e.g., an alpha amino acid). In some embodiments, the linker is alanine glycolate or amino hexanoate. In some embodiments, the loading of the corticosteroid onto the CDP is at least about 13% by weight of the conjugate (e.g., at least about 14%, 15%, 16%, 17%, 18%, 19%, or 20%). In some embodiments, the loading of the corticosteroid onto the CDP is less than about 12% by weight of the conjugate (e.g., less than about 11%, 10%, 9%, 8%, or 7%).

Also included are methods of making the CDP-therapeutic agent conjugates, particles and compositions described herein, e.g., a CDP-cytotoxic agent conjugate, particle or composition, e.g., CDP-topoisomerase inhibitor conjugate, particle or composition, e.g., a CDP-topoisomerase inhibitor 1 conjugate, particle or composition (e.g., a CDP-camptothecin conjugate, particle or composition, CDP-irinotecan conjugate, particle or composition, CDP-SN-38 conjugate, particle or composition, CDP-topotecan conjugate, particle or composition, CDP-lamellarin D conjugate, particle or composition, a CDP-lurotecan conjugate, particle or composition, a CDP-exatecan conjugate, particle or composition, a CDP-diflomotecan conjugate, particle or composition, and CDP-topoisomerase I inhibitor conjugates, particles and compositions which include derivatives of camptothecin, irinotecan, SN-38, lamellarin D, lurotecan, exatecan, and diflomotecan), a CDP-topoisomerase II inhibitor conjugate, particle or composition (e.g., a CDP-etoposide conjugate, particle, or composition, CDP-tenoposide conjugate, particle or composition, CDP-amsacrine conjugate, particle or composition and CDP-topoisomerase II inhibitor conjugates, particles and compositions which include derivatives of etoposide, tenoposide, and amsacrine), a CDP-anti-metabolic agent conjugate, particle or composition (e.g., a CDP-antifolate conjugate, particle or composition (e.g., a CDP-pemetrexed conjugate, particle or composition, a CDP-floxuridine conjugate, particle or composition, a CDP-raltitrexed conjugate, particle or composition) or a CDP-pyrimidine analog conjugate, particle or composition (e.g., a CDP-capecitabine conjugate, particle or composition, a CDP-cytarabine conjugate, particle or composition, a CDP-gemcitabine conjugate, particle or composition, a CDP-5FU conjugate, particle or composition)), a CDP-alkylating agent conjugate, particle or composition, a CDP-anthracycline conjugate, particle or composition, a CDP-anti-tumor antibiotic conjugate, particle or composition (e.g., a CDP-HSP90 inhibitor conjugate, particle or composition, e.g., a CDP-geldanamycin conjugate, particle or composition, a CDP-tanespimycin conjugate, particle or composition or a CDP-alvespimycin conjugate, particle or composition), a CDP-platinum based agent conjugate, particle or composition (e.g., a CDP-cisplatin conjugate, particle or composition, a CDP-carboplatin conjugate, particle or composition, a CDP-oxaliplatin conjugate, particle or composition), a CDP-microtubule inhibitor conjugate, particle or composition, a CDP-kinase inhibitor conjugate, particle or composition (e.g., a CDP-seronine/threonine kinase inhibitor conjugate, particle or composition, e.g., a CDP-mTOR inhibitor conjugate, particle or composition, e.g., a CDP-rapamycin conjugate, particle or composition) or a CDP-proteasome inhibitor conjugate, particle or composition.

In one embodiment, the CDP-therapeutic agent conjugate has the following formula: 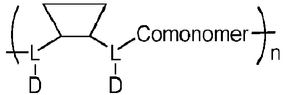 wherein each L is independently a linker, and each D is independently a therapeutic agent, a prodrug derivative thereof, or absent; and each comonomer is independently a comonomer described herein, and n is at least 4, 5, 6, 7, 8, 9, 10, 11, 12, 13, 14, 15, 16, 17, 18, 19 or 20, provided that the polymer comprises at least one therapeutic agent and in some embodiments, at least two therapeutic agents. In some embodiments, the molecular weight of the comonomer is from about 2000 to about 5000 Da (e.g., from about 3000 to about 4000 Da (e.g., about 3400 Da).

In some embodiments, the therapeutic agent is a therapeutic agent described herein (e.g., a cytotoxic agent or an immunomodulator). The therapeutic agent can be attached to the CDP via a functional group such as a hydroxyl group, or where appropriate, an amino group. In some embodiments, one or more of the therapeutic agent in the CDP-therapeutic agent conjugate can be replaced with another therapeutic agent, e.g., another cytotoxic agent or immunomodulator.

In some embodiments, the CDP-therapeutic agent conjugate has the following formula: 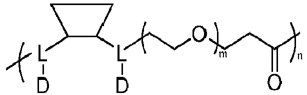 wherein each L is independently a linker, and each D is independently a therapeutic agent, a prodrug derivative thereof, or absent, provided that the polymer comprises at least one therapeutic agent and in some embodiments, at least two therapeutic agent moieties; and wherein the group 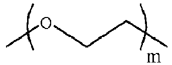 has a Mw of 3400 Da or less and n is at least 4, 5, 6, 7, 8, 9, 10, 11, 12, 13, 14, 15, 16, 17, 18, 19 or 20.

In some embodiments, the therapeutic agent is a therapeutic agent described herein (e.g., a cytotoxic agent or an immunomodulator). The therapeutic agent can be attached to the CDP via a functional group such as a hydroxyl group, or where appropriate, an amino group. In some embodiments, one or more of the therapeutic agent in the CDP-therapeutic agent conjugate can be replaced with another therapeutic agent, e.g., another cytotoxic agent or immunomodulator.

In some embodiments, less than all of the L moieties are attached to D moieties, meaning in some embodiments, at least one D is absent. In some embodiments, the loading of the D moieties on the CDP-therapeutic agent conjugate is from about 1 to about 50% (e.g., from about 1 to about 40%, from about 1 to about 25%, from about 5 to about 20% or from about 5 to about 15%). In some embodiments, each L independently comprises an amino acid or a derivative thereof. In some embodiments, each L independently comprises a plurality of amino acids or derivatives thereof. In some embodiments, each L is independently a dipeptide or derivative thereof. In one embodiment, L is one or more of: alanine, arginine, histidine, lysine, aspartic acid, glutamic acid, serine, threonine, asparganine, glutamine, cysteine, glycine, proline, isoleucine, leucine, methionine, phenylalanine, tryptophan, tyrosine and valine.

In one embodiment, the CDP-therapeutic agent conjugate (e.g., the CDP-cytotoxic agent conjugate) has the following formula: 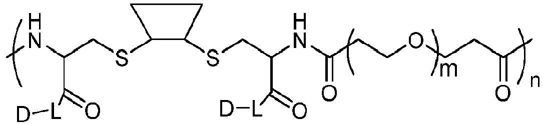 wherein each L is independently a linker or absent and each D is independently a therapeutic agent (e.g., a cytotoxic agent, immunomodulator, a prodrug thereof) or absent, and wherein the group 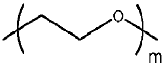 has a Mw of 5,000 Da or less (e.g., 3,400 Da) and n is at least 4, 5, 6, 7, 8, 9, 10, 11, 12, 13, 14, 15, 16, 17, 18, 19 or 20, provided that the polymer comprises at least one therapeutic agent (e.g., at least one cytotoxic agent immunomodulator, a prodrug thereof). In one embodiment, the cytotoxic agent is a cytotoxic agent described herein. In one embodiment, the immunomodulator is an immunomodulator described herein.

In one embodiment, the CDP is not biodegradable. In one embodiment, the CDP is biodegradable. In one embodiment, the CDP is biocompatible. In one embodiment, the conjugate includes a combination of one or more therapeutic agents.

In one embodiment, each L of the CDP-therapeutic agent conjugate (e.g., the CDP-cytotoxic agent conjugate) is independently an amino acid derivative. In one embodiment, at least a portion of the CDP is covalently attached to the therapeutic agent

(e.g., the cytotoxic agent) through a cysteine moiety. Tn one embodiment, the linker comprises a moiety formed using "click chemistry" (e.g., as described in WO 2006/115547). In one embodiment, the linker comprises an amide bond, an ester bond, a disulfide bond, or a triazole. In one embodiment, the linker comprises a bond that is cleavable under physiological conditions. In one embodiment, the linker is hydrolysable under physiologic conditions or the linker is enzymatically cleavable under physiological conditions (e.g., the linker comprises a disulfide bond which can be reduced under physiological conditions). In one embodiment, the linker is not cleavable under physiological conditions. In one embodiment, at least a portion of the CDP is covalently attached to the therapeutic agent (e.g., the cytotoxic agent or immunomodulator) through a carboxy terminal of the therapeutic agent.

In one embodiment, the therapeutic agents (e.g., the cytotoxic agents or immunomodulators) are from about 1 to about 100 weight % of the conjugate, e.g., from 1 to about 80 weight % of the conjugate, e.g., from 1 to about 70 weight % of the conjugate, e.g., from 1 to about 60 weight % of the conjugate, e.g., from 1 to about 50 weight % of the conjugate, e.g., from 1 to about 40 weight % of the conjugate, e.g., from 1 to about 30 weight % of the conjugate, e.g., from 1 to about 20 weight % of the conjugate, e.g., from 1 to about 10 weight % of the conjugate.

In one embodiment, the CDP-therapeutic agent conjugate (e.g., the CDP-cytotoxic agent conjugate or immunomodulator) comprises a subunit of the following formula: 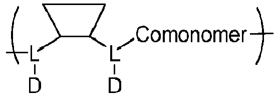 wherein each L is independently a linker, and each D is independently a therapeutic agent, a prodrug derivative thereof, or absent; and each comonomer is independently a comonomer described herein provided that the subunit comprises at least one therapeutic agent.

In one embodiment, the CDP-therapeutic agent conjugate (e.g., the CDP-cytotoxic agent conjugate or immunomodulator) comprises a subunit of the following formula: 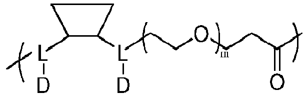 wherein each L is independently a linker, and each D is independently a therapeutic agent, a prodrug derivative thereof, or absent, provided that the subunit comprises at least one therapeutic agent; and
wherein the group 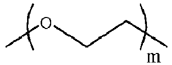 has a Mw of 3400 Da or less.

In one embodiment, the CDP-therapeutic agent conjugate (e.g., the CDP-cytotoxic agent conjugate or immunomodulator) comprises a subunit of the following formula: 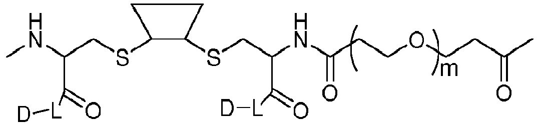 wherein each L is independently a linker and each D is independently a therapeutic agent (e.g., the cytotoxic agent or a prodrug thereof) and wherein the group 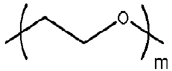 has a Mw of 5,000 Da or less (e.g., 3,400 Da), provided that the subunit comprises at least one therapeutic agent. In one embodiment, the cytotoxic agent is a cytotoxic agent described herein. In one embodiment, the immunomodulator is an immunomodulator described herein.

In one embodiment, the CDP is not biodegradable. In one embodiment, the CDP is biodegradable. In one embodiment, the CDP is biocompatible.

In one embodiment, the CDP-therapeutic agent conjugate, e.g., the CDP-cytotoxic agent conjugate or the CDP-immunomodulator conjugate, e.g., a CDP-cytotoxic agent conjugate or CDP-immunomodulator conjugate described herein, forms an inclusion complex between a therapeutic agent attached or conjugated to the CDP, e.g., via a covalent linkage, and another moiety in the CDP (e.g., a cyclodextrin in the CDP) or a moiety (e.g., a cyclodextrin) in another CDP-therapeutic agent conjugate. In one embodiment, the CDP-therapeutic agent conjugate forms a nanoparticle. A plurality of CDP-therapeutic agent conjugates can form a particle (e.g., where the particle is self-assembled), e.g., through the formation of intramolecular or intermolecular inclusion complexes. In some embodiments, a particle described herein is a nanoparticle. A particle (e.g., a nanoparticle) described herein can include a plurality of CDP-therapeutic agent conjugates (e.g., at least 2, 3, 4, 5, 6, 7, 8, 9, or 10). The nanoparticle can range in size from 10 to 300 nm in diameter, e.g., 15 to 280, 30 to 250, 30 to 200, 20 to 150, 30 to 100, 20 to 80, 30 to 70, 30 to 60 or 30 to 50 nm diameter. In one embodiment, the nanoparticle is 15 to 50 nm in diameter. In one embodiment, the nanoparticle is 30 to 60 nm in diameter. In one embodiment, the composition comprises a population or a plurality of nanoparticles with an average diameter from 10 to 300 nm, e.g., 15 to 280, 30 to 250, 30 to 200, 20 to 150, 30 to 100, 20 to 80, 30 to 70, 30 to 60 or 30 to 50 nm. In one embodiment, the nanoparticle is 15 to 50 nm in diameter. In one embodiment, the average nanoparticle diameter is from 30 to 60 nm. In one embodiment, the surface charge of the molecule is neutral, or slightly negative. In some embodiments, the zeta potential of the particle surface is from about -80 mV to about 50 mV, about -20 mV to about 20 mV, about -20 mV to about -10 mV, or about -10 mV to about 0.

In one embodiment, the therapeutic agent (e.g., a cytotoxic agent, e.g., a topoisomerase inhibitor (e.g., a topoisomerase inhibitor I, a topoisomerase II inhibitor), an anti-metabolic agent (e.g., an antifolate, a pyrimidine analog), an alkylating agent, an anthracycline, a platinum based agent, an anti-tumor antibiotic, a microtubule inhibitor (e.g., a taxane or a epothilone), a kinase inhibitor, or a proteasome inhibitor (a boronic acid containing molecule, e.g., a bortezomib); an immunomodulator (e.g., a corticosteroid or a rapamycin analog) conjugated to the CDP is more soluble when conjugated to the CDP than when not conjugated to the CDP.

In one embodiment, the composition comprises a population, mixture or plurality of CDP-therapeutic agent conjugates or particles comprising CDP-therapeutic agent conjugates (e.g., CDP-cytotoxic agent conjugates, e.g., CDP-topoisomerase inhibitor conjugates (e.g., CDP-topoisomerase inhibitor I conjugates, CDP-topoisomerase II inhibitor conjugates), CDP-anti-metabolic agent conjugates (e.g., CDP-antifolate conjugates, CDP-pyrimidine analog conjugates), CDP-alkylating agent conjugates, CDP-anthracycline conjugates, CDP-platinum based agent conjugates, CDP-anti-tumor antibiotic conjugates, CDP-microtubule inhibitor conjugates (e.g., a CDP-taxane conjugates or CDP-epothilone conjugates), CDP-kinase inhibitor conjugates, CDP-proteasome inhibitor conjugates (CDP-boronic acid containing molecule conjugates, e.g., CDP-bortezomib conjugates); CDP-immunomodulator conjugates (e.g., CDP-corticosteroid conjugates or CDP-rapamycin conjugates). In one embodiment, the population, mixture or plurality of CDP-therapeutic agent conjugates comprises a plurality of different therapeutic agents conjugated to a CDP (e.g., a first therapeutic agent is attached to a first CDP and a different therapeutic agent is attached to a second CDP and both CDP-therapeutic agent conjugates are present in the composition). In one embodiment, the composition comprises a population, mixture or plurality of particles, the particles comprising CDP-therapeutic agent conjugates.

In one aspect, the invention features, a method of treating a proliferative disorder, e.g., cancer, in a subject, e.g., a human subject. The method comprises:
providing an initial administration of a CDP-cytotoxic agent conjugate, particle or composition described herein to said subject, and, optionally, administering one or more subsequent administrations of said CDP-cytotoxic agent conjugate, particle or composition, to said subject.

In one embodiment, the CDP-cytotoxic agent conjugate, particle or composition is administered at a dose and/or dosing schedule described herein.

In one embodiment, the cancer is a bile duct cancer, e.g., a Klatskin tumor.

In one aspect, the invention features, a method of treating cancer in a subject, e.g., a human subject. The method comprises:
providing an initial administration of a CDP-anti-metabolic agent conjugate, particle or composition, e.g., a CDP-antifolate conjugate, particle or composition, e.g., a CDP-pemetrexed conjugate, particle or composition, e.g., a CDP-pemetrexed conjugate, particle or composition, described herein, or, e.g., a CDP-floxuridine conjugate, particle or composition, e.g., a CDP-floxuridine conjugate, particle or composition, described herein, or, e.g., a CDP-raltitrexed conjugate, particle or composition, e.g., a CDP-raltitrexed conjugate, particle or composition, described herein, to said subject, and, optionally, administering one or more subsequent administrations of said CDP-anti-metabolic agent conjugate, particle or composition, e.g., a CDP-antifolate conjugate, particle or composition, e.g., a CDP-pemetrexed conjugate, particle or composition, e.g., a CDP-pemetrexed conjugate, particle or composition, described herein, or, e.g., a CDP-floxuridine conjugate, particle or composition, e.g., a CDP-floxuridine conjugate, particle or composition, described herein, or, e.g., a CDP-raltitrexed conjugate, particle or composition, e.g., a CDP-raltitrexed conjugate, particle or composition, described herein, wherein each subsequent administration is provided, independently, between 5, 6, 7, 8, 9, 10, 11, 12, 13, 14, 15, 16, 17, 18, 19, 20, 21, 22, 23, 24, 25, 26, 27, 28 days after the previous, e.g., the initial, administration, to thereby treat the cancer.

In an embodiment, the dosage of at least 2, 3, 4, 5, 6, 7, 8, 9, 10, 12, 15, or 20 administrations is the same.

In an embodiment, the time between at least 2, 3, 4, 5, 6, 7, 8, 9, 10, 12, 15, or 20 administrations is the same.

In an embodiment, each subsequent administration is administered 18-24, e.g., 21, days after the previous administration.

In an embodiment, at least 2, 3, 4, 5, 6, 7, 8, 9, 10, 12, 15, 20, 50 or 100 administrations are administered to the subject.

In one embodiment, the CDP-anti-metabolic agent conjugate, particle or composition, e.g., a CDP-antifolate conjugate, particle or composition, e.g., a CDP-pemetrexed conjugate, particle or composition, e.g., a CDP-pemetrexed conjugate, particle or composition, described herein, or, e.g., a CDP-floxuridine conjugate, particle or composition, e.g., a CDP-floxuridine conjugate, particle or composition, described herein, or, e.g., a CDP-raltitrexed conjugate, particle or composition, e.g., a CDP-raltitrexed conjugate, particle or composition, described herein, is administered by intravenous administration over a period equal to or less than about 30 minutes, 45 minutes, 60 minutes, 90 minutes, 120 minutes, 150 minutes, or 180 minutes.

In an embodiment, the method includes an initial administration of a CDP-pemetrexed conjugate, particle or composition to said subject at a dosage of 300 mg/m², 320 mg/m², 350 mg/m², 380 mg/m², 400 mg/m², 420 mg/m², 450 mg/m², 480 mg/m², 500 mg/m², 520 mg/m², 550 mg/m², 580 mg/m², 600 mg/m², 620 mg/m², 650 mg/m², 680 mg/m2, 700 mg/m², 720 mg/m², or 750 mg/m² (wherein the dosage is expressed in mg of drug, as opposed to mg of conjugate), and one or more subsequent administrations of a CDP-pemetrexed conjugate, particle or composition to said subject, at a dosage of 300 mg/m², 320 mg/m², 350 mg/m², 380 mg/m², 400 mg/m², 420 mg/m², 450 mg/m², 480 mg/m², 500 mg/m², 520 mg/m², 550 mg/m², 580 mg/m², 600 mg/m², 620 mg/m², 650 mg/m², 680 mg/m², 700 mg/m², 720 mg/m², or 750 mg/m², e.g., at the same dosage as the initial dosage. In one embodiment, each subsequent administration is administered, independently, 18-24, e.g., 21 days after the previous, e.g., the initial, administration.

In an embodiment, the cancer is a cancer described herein. For example, the cancer can be a cancer of the bladder (including accelerated and metastatic bladder cancer), breast (e.g., estrogen receptor positive breast cancer, estrogen receptor negative breast cancer, HER-2 positive breast cancer, HER-2 negative breast cancer, triple negative breast cancer, inflammatory breast cancer), colon (including colorectal cancer), kidney (e.g., renal cell carcinoma), liver, lung (including small cell lung cancer and non-small cell lung cancer (including adenocarcinoma, squamous cell carcinoma, bronchoalveolar carcinoma and large cell carcinoma)), mesothelioma, genitourinary tract, e.g., ovary (including fallopian, endometrial and peritoneal cancers), cervix, prostate and testes, lymphatic system, rectum, larynx, pancreas (including exocrine pancreatic carcinoma), stomach (e.g., gastroesophageal, upper gastric or lower gastric cancer), gastrointestinal cancer (e.g., anal cancer), gall bladder, thyroid, lymphoma (e.g., Burkitt's, Hodgkin's or non-Hodgkin's lymphoma), leukemia (e.g., acute myeloid leukemia), Ewing's sarcoma, nasoesophageal cancer, nasopharyngeal cancer, neural and glial cell cancers (e.g., glioblastoma multiforme), and head and neck. Preferred cancers include breast cancer (e.g., metastatic or locally advanced breast cancer), prostate cancer (e.g., hormone refractory prostate cancer), renal cell carcinoma, lung cancer (e.g., small cell lung cancer and non-small cell lung cancer (including adenocarcinoma, squamous cell carcinoma, bronchoalveolar carcinoma and large cell carcinoma)), mesothelioma, pancreatic cancer, gastric cancer (e.g., gastroesophageal, upper gastric or lower gastric cancer), colorectal cancer, squamous cell cancer of the head and neck, ovarian cancer (e.g., advanced ovarian cancer, platinum-based agent resistant or relapsed ovarian cancer), lymphoma (e.g., Burkitt's, Hodgkin's or non-Hodgkin's lymphoma), leukemia (e.g., acute myeloid leukemia) and gastrointestinal cancer.

In one embodiment, the cancer is lung cancer, e.g., non-small cell lung cancer and/or small cell lung cancer (e.g., squamous cell non-small cell lung cancer, or nonsquamous cell non-small cell lung cancer, or squamous cell small cell lung cancer). In an embodiment, the cancer is lung cancer, e.g., nonsquamous cell non-small cell lung cancer and the CDP-anti-metabolic agent conjugate, particle or composition is a CDP-pemetrexed conjugate, particle or composition. In one embodiment, the lung cancer is metastatic, recurrent or refractory lung cancer. In one embodiment, the lung cancer is KRAS wild-type lung cancer, e.g., KRAS wild-type non-small cell lung cancer.

In an embodiment, the CDP-anti-metabolic agent conjugate, particle or composition, e.g., the CDP-antifolate conjugate, particle or composition, e.g., a CDP-pemetrexed conjugate, particle or composition, is provided at 300-750 mg/m²/month, e.g., 300-600 mg/m²/month or 400-750 mg/m²/month.

In one embodiment, the CDP-anti-metabolic agent conjugate, particle or composition is administered in combination with one or more additional chemotherapeutic agent, e.g., a chemotherapeutic agent (such as an angiogenesis inhibitor) or combination of chemotherapeutic agents described herein. In one embodiment, the conjugate, particle or composition is administered in combination with one or more of: a platinum based agent (e.g., carboplatin, cisplatin, oxaliplatin), a taxane (e.g., paclitaxel, docetaxel, larotaxel, cabazitaxel), a vinca alkaloid (e.g., vinblastine, vincristine, vindesine, vinorelbine), an antimetabolite (e.g., an antifolate (e.g., floxuridine), a pyrimidine analogue (e.g., gemcitabine, 5FU, capecitabine)), an alkylating agent (e.g., cyclophosphamide, decarbazine, melphalan, ifosfamide, temozolomide), a vascular endothelial growth factor (VEGF) pathway inhibitor, a poly ADP-ribose polymerase (PARP) inhibitor and an mTOR inhibitor. In one embodiment, when the CDP-anti-metabolic agent conjugate, particle or composition is administered in combination with an additional chemotherapeutic agent, the dose at which the CDP-anti-metabolic agent conjugate, particle or composition is administered is 1%, 3%, 5%, 10%, 15%, 20%, 25%, 30% less than the doses described herein. In one embodiment, when the CDP-anti-metabolic agent conjugate, particle or composition, e.g., the CDP-antifolate conjugate, particle or composition, e.g., a CDP-pemetrexed conjugate, particle or composition is provided in combination with one or more additional chemotherapeutic agents, e.g., a chemotherapeutic agent described herein, the CDP-anti-metabolic agent conjugate, particle or composition, e.g., the CDP-antifolate conjugate, particle or composition, e.g., a CDP-pemetrexed conjugate, particle or composition, is provided at 100-750 mg/m²/month.

In one embodiment, the CDP-anti-metabolic agent conjugate, particle or composition is administered in combination with an angiogenesis inhibitor, e.g., a VEGF pathway inhibitor, e.g., sorafenib or sunitinib. In one embodiment, the angiogenesis inhibitor, e.g., sorafenib, is administered at a dose of about 400 mg per day or less, daily, e.g., 350 mg per day, 300 mg per day, 250 mg per day, 200 mg per day, or 150 mg per day. In one embodiment, the angiogenesis inhibitor, e.g., sunitinib, is administered daily at a dose of about 50 mg per day or less, daily, e.g., 45 mg per day, 40 mg per day, 38 mg per day, 30 mg per day, 25 mg per day, 20 mg per day, or 15 mg per day. In one embodiment, when the CDP-anti-metabolic agent conjugate, particle or composition is administered in combination with an angiogenesis inhibitor, e.g., sorafenib or sunitinib, the dose at which the CDP-anti-metabolic agent conjugate, particle or composition is administered is 1%, 3%, 5%, 10%, 15%, 20%, 25%, or 30% less than a dose described herein.

In one embodiment, the CDP-anti-metabolic agent conjugate, particle or composition, e.g., a CDP-antifolate conjugate, particle or composition, e.g., a CDP-pemetrexed conjugate, particle or composition, e.g., a CDP-pemetrexed conjugate, particle or composition, described herein is administered at a dosage of 300 mg/m², 320 mg/m², 350 mg/m², 15 mg/m², 380 mg/m², 400 mg/m², 420 mg/m², 450 mg/m², 480 mg/m², 500 mg/m², 520 mg/m², 550 mg/m², 580 mg/m², 600 mg/m², 620 mg/m², 650 mg/m², 680 mg/m², 700 mg/m², 720 mg/m², or 750 mg/m² by intravenous administration over a period equal to or less than about 30 minutes, 45 minutes, 60 minutes or 90 minutes, e.g., a period equal to or less than 30 minutes, 45 minutes or 60 minutes.

In an embodiment, the method includes an initial administration of the CDP-pemetrexed conjugate, particle or composition to the subject at a dosage of 300 mg/m², 320 mg/m², 350 mg/m², 15 mg/m², 380 mg/m², 400 mg/m², 420 mg/m², 450 mg/m², 480 mg/m², 500 mg/m², 520 mg/m², 550 mg/m², 580 mg/m², 600 mg/m², 620 mg/m², 650 mg/m², 680 mg/m², 700 mg/m², 720 mg/m², or 750 mg/m², and
one or more subsequent administrations of the CDP-pemetrexed conjugate, particle or composition to the subject, at a dosage of 300 mg/m², 320 mg/m², 350 mg/m², 15 mg/m², 380 mg/m², 400 mg/m², 420 mg/m², 450 mg/m², 480 mg/m², 500 mg/m², 520 mg/m², 550 mg/m², 580 mg/m², 600 mg/m², 620 mg/m², 650 mg/m², 680 mg/m², 700 mg/m², 720 mg/m², or 750 mg/m², e.g., at the same dosage as the initial dosage, wherein each subsequent administration is administered, independently, 18-24, e.g., 21, days after the previous, e.g., the initial, administration, and the cancer is, e.g., lung cancer, e.g., non-small cell lung cancer or small cell lung cancer (e.g., squamous cell non-small cell lung cancer, squamous cell small cell lung cancer, or nonsquamous cell non-small cell lung cancer), or mesothelioma.

In one embodiment, the subject has not been administered a CDP-anti-metabolic agent conjugate, particle or composition, e.g., a CDP-antifolate conjugate, particle or composition, e.g., a CDP-pemetrexed conjugate, particle or composition, e.g., a CDP-pemetrexed conjugate, particle or composition, described herein, prior to the initial administration.

In an embodiment, the CDP-anti-metabolic agent conjugate, particle or composition is administered as a first line treatment for the cancer.

In an embodiment, the CDP-anti-metabolic agent conjugate, particle or composition is administered as a second, third or fourth line treatment for the cancer. In an embodiment, the cancer is sensitive to one or more chemotherapeutic agents, e.g., a platinum-based agent, a taxane, an alkylating agent, an antimetabolite and/or a vinca alkaloid. In an embodiment, the cancer is a refractory, relapsed or resistant to one or more chemotherapeutic agents, e.g., a platinum-based agent, a taxane, an alkylating agent, an anthracycline (e.g., doxorubicin (e.g., liposomal doxorubicin)), an antimetabolite and/or a vinca alkaloid. In one embodiment, the cancer is, e.g., lung cancer, and the lung cancer is refractory, relapsed or resistant to a taxane (e.g., paclitaxel, docetaxel, larotaxel, cabazitaxel), a platinum-based agent (e.g., carboplatin, cisplatin, oxaliplatin), a vinca alkaloid (e.g., vinblastine, vincristine, vindesine, vinorelbine), a vascular endothelial growth factor (VEGF) pathway inhibitor, and/or an epidermal growth factor (EGF) pathway inhibitor).

In one embodiment, the subject has lung cancer, e.g., nonsquamous non-small cell cancer, or mesothelioma that is refractory, relapsed or resistant to a platinum-based agent, and the subject is administered a CDP-anti-metabolic agent conjugate, particle or composition, e.g., a CDP-antifolate conjugate, particle or composition, e.g., a CDP-pemetrexed conjugate, particle or composition, e.g., a CDP-pemetrexed conjugate, particle or composition, described herein.

In one embodiment, the subject has mesothelioma, and the subject is administered a CDP-anti-metabolic agent conjugate, particle or composition, e.g., a CDP-antifolate conjugate, particle or composition, e.g., a CDP-pemetrexed conjugate, particle or composition, e.g., a CDP-pemetrexed conjugate, particle or composition, described herein, in combination with a platinum based agent (e.g., cisplatin, carboplatin, or oxaliplatin). In one embodiment, the platinum based agent (e.g., cisplatin, carboplatin, or oxaliplatin) is administered at a dose of about 20 mg/m², about 30 mg/m², about 40 mg/m2, 50 mg/m², 60 mg/m², 70 mg/m², 80 mg/m², every 17, 18, 19, 20, 21, 22, 23 or 24 days, e.g., 21 days. In one embodiment, the CDP-anti-metabolic agent conjugate, particle or composition, e.g., a CDP-antifolate conjugate, particle or composition, e.g., a CDP-pemetrexed conjugate, particle or composition, e.g., a CDP-pemetrexed conjugate, particle or composition, described herein is administered at a dose and/or dosing regimen described herein and the platinum-based chemotherapeutic (e.g., cisplatin, carboplatin, or oxaliplatin) is administered at a dose of about 20 mg/m², about 30 mg/m², about 40 mg/m2, 50 mg/m², 60 mg/m², 70 mg/m², 80 mg/m², every 17, 18, 19, 20, 21, 22, 23 or 24 days, e.g., 21 days. In one embodiment, when the CDP-anti-metabolic agent conjugate, particle or composition is administered in combination with platinum-based chemotherapeutic (e.g., cisplatin, carboplatin, or oxaliplatin), the dose at which the CDP-anti-metabolic agent conjugate, particle or composition is administered is 1%, 3%, 5%, 10%, 15%, 20%, 25%, 30% less than a dose described herein.

In one embodiment, the subject has radically resected non-small cell lung cancer, and/or advanced non-squamous KRAS wild type non-squamous cell lung cancer and the subject is administered a CDP-anti-metabolic agent conjugate, particle or composition, e.g., a CDP-antifolate conjugate, particle or composition, e.g., a CDP-pemetrexed conjugate, particle or composition, e.g., a CDP-pemetrexed conjugate, particle or composition, described herein, in combination with a platinum based agent (e.g., cisplatin, carboplatin, or oxaliplatin). In one embodiment, the platinum based agent (e.g., cisplatin, carboplatin, or oxaliplatin) is administered at a dose of about 20 mg/m², about 30 mg/m², about 40 mg/m², 50 mg/m², 60 mg/m², 70 mg/m², 80 mg/m², every 17, 18, 19, 20, 21, 22, 23 or 24 days, e.g., 21 days. In one embodiment, the CDP-anti-metabolic agent conjugate, particle or composition, e.g., a CDP-antifolate conjugate, particle or composition, e.g., a CDP-pemetrexed conjugate, particle or composition, e.g., a CDP-pemetrexed conjugate, particle or composition, described herein is administered at a dose and/or dosing regimen described herein and the platinum-based chemotherapeutic (e.g., cisplatin, carboplatin, or oxaliplatin) is administered at a dose of about 20 mg/m², about 30 mg/m², about 40 mg/m², 50 mg/m², 60 mg/m², 70 mg/m², 80 mg/m², every 17, 18, 19, 20, 21, 22, 23 or 24 days, e.g., 21 days. In one embodiment, when the CDP-anti-metabolic agent conjugate, particle or composition is administered in combination with platinum-based chemotherapeutic (e.g., cisplatin, carboplatin, or oxaliplatin), the dose at which the CDP-anti-metabolic agent conjugate, particle or composition is administered is 1%, 3%, 5%, 10%, 15%, 20%, 25%, 30% less than a dose described herein.

In one aspect, the invention features, a method of treating cancer in a subject, e.g., a human subject. The method comprises:
providing an initial administration of CDP-pyrimidine analog conjugate, particle or composition, e.g., a CDP-capecitabine conjugate, particle or composition, e.g., a CDP-capecitabine conjugate, particle or composition, described herein, or, e.g., a CDP-cytarabine conjugate, particle or composition, e.g., a CDP-cytarabine conjugate, particle or composition, described herein, or, e.g., a CDP-gemcitabine conjugate, particle or composition, e.g., a CDP-gemcitabine conjugate, particle or composition, described herein, or, e.g., a CDP-5FU conjugate, particle or composition, e.g., a CDP-5FU conjugate, particle or composition, described herein, to said subject, and, optionally, providing one or more subsequent administrations of said CDP-pyrimidine analog conjugate, particle or composition, e.g., a CDP-antifolate conjugate, particle or composition, e.g., a CDP-capecitabine conjugate, particle or composition, e.g., a CDP-capecitabine conjugate, particle or composition, described herein, or, e.g., a CDP-cytarabine conjugate, particle or composition, e.g., a CDP-cytarabine conjugate, particle or composition, described herein, or, e.g., a CDP-gemcitabine conjugate, particle or composition, e.g., a CDP-gemcitabine conjugate, particle or composition, described herein, or, e.g., a CDP-5FU conjugate, particle or composition, e.g., a CDP-5FU conjugate, particle or composition, described herein, wherein each subsequent administration is provided, independently, between 5, 6, 7, 8, 9, 10, 11, 12, 13, 14, 15, 16, 17, 18, 19, 20, 21, 22, 23, 24, 25, 26, 27, 28 days after the previous, e.g., the initial, administration, to thereby treat the cancer.

In an embodiment, the dosage of at least 2, 3, 4, 5, 6, 7, 8, 9, 10, 12, 15, or 20 administrations is the same.

In an embodiment, the time between at least 2, 3, 4, 5, 6, 7, 8, 9, 10, 12, 15, or 20 administrations is the same.

In an embodiment, each subsequent administration is administered 20-28, e.g., 24, days after the previous administration.

In an embodiment, at least 2, 3, 4, 5, 6, 7, 8, 9, 10, 12, 15, 20, 50 or 100 administrations are administered to the subject.

In one embodiment, the CDP-pyrimidine analog conjugate, particle or composition, e.g., a CDP-antifolate conjugate, particle or composition, e.g., a CDP-capecitabine conjugate, particle or composition, e.g., a CDP-capecitabine conjugate, particle or composition, described herein, or, e.g., a CDP-cytarabine conjugate, particle or composition, e.g., a CDP-cytarabine conjugate, particle or composition, described herein, or, e.g., a CDP-gemcitabine conjugate, particle or composition, e.g., a CDP-gemcitabine conjugate, particle or composition, described herein, or, e.g., a CDP-5FU conjugate, particle or composition, e.g., a CDP-5FU conjugate, particle or composition, described herein, is administered by intravenous administration over a period equal to or less than about 30 minutes, 45 minutes, 60 minutes, 90 minutes, 120 minutes, 150 minutes, or 180 minutes.

In an embodiment, the method includes an initial administration of a CDP-gemcitabine conjugate, particle or composition at a dosage of 600 mg/m², 700 mg/m², 730 mg/m², 750 mg/m², 780 mg/m², 800 mg/m², 830 mg/m², 850 mg/m², 880 mg/m², 900 mg/m², 930 mg/m², 950 mg/m², 980 mg/m², 1000 mg/m², 1030 mg/m², 1050 mg/m², 1080 mg/m², 1100 mg/m², 1130 mg/m², 1150 mg/m², 1180 mg/m², 1200 mg/m², 1230 mg/m2, 1250 mg/m², 1280 mg/m², 1300 mg/m², 1350 mg/m², 1380 mg/m², 1400 mg/m², 1430 mg/m², 1450 mg/m², 1480 mg/m², 1500 mg/m², 1530 mg/m², 1550 mg/m², 1580 mg/m², 1600 mg/m², 1630 mg/m², or 1650 mg/m² (wherein the dosage is expressed in mg of drug, as opposed to mg of conjugate), and one or more subsequent administrations of a CDP-gemcitabine conjugate, particle or composition at a dosage of 600 mg/m², 700 mg/m², 730 mg/m², 750 mg/m², 780 mg/m², 800 mg/m², 830 mg/m², 850 mg/m², 880 mg/m2, 900 mg/m², 930 mg/m², 950 mg/m², 980 mg/m², 1000 mg/m², 1030 mg/m², 1050 mg/m², 1080 mg/m², 1100 mg/m², 1130 mg/m², 1150 mg/m², 1180 mg/m², 1200 mg/m², 1230 mg/m², 1250 mg/m², 1280 mg/m², 1300 mg/m², 1350 mg/m², 1380 mg/m², 1400 mg/m², 1430 mg/m², 1450 mg/m², 1480 mg/m², 1500 mg/m², 1530 mg/m², 1550 mg/m², 1580 mg/m², 1600 mg/m², 1630 mg/m², or 1650 mg/m², e.g., at the same dosage as the initial dosage. In one embodiment, each subsequent administration is provided, independently, between 5, 6, 7, 8, 9, 10, 11, 12, 13, 14, 15 or 16 days after the previous, e.g., the initial, administration.

In an embodiment, the cancer is a cancer described herein. For example, the cancer can be a cancer of the bladder (including accelerated and metastatic bladder cancer), breast (e.g., estrogen receptor positive breast cancer, estrogen receptor negative breast cancer, HER-2 positive breast cancer, HER-2 negative breast cancer, triple negative breast cancer, inflammatory breast cancer), colon (including colorectal cancer), kidney (e.g., renal cell carcinoma), liver, lung (including small cell lung cancer and non-small cell lung cancer (including adenocarcinoma, squamous cell carcinoma, bronchoalveolar carcinoma and large cell carcinoma), mesothelioma, genitourinary tract, e.g., ovary (including fallopian, endometrial and peritoneal cancers), cervix, prostate and testes, lymphatic system, rectum, larynx, pancreas (including exocrine pancreatic carcinoma), stomach (e.g., gastroesophageal, upper gastric or lower gastric cancer), gastrointestinal cancer (e.g., anal cancer), gall bladder, thyroid, lymphoma (e.g., Burkitt's, Hodgkin's or non-Hodgkin's lymphoma), leukemia (e.g., acute myeloid leukemia), Ewing's sarcoma, nasoesophageal cancer, nasopharyngeal cancer, neural and glial cell cancers (e.g., glioblastoma multiforme), and head and neck. Preferred cancers include breast cancer (e.g., metastatic or locally advanced breast cancer), prostate cancer (e.g., hormone refractory prostate cancer), renal cell carcinoma, lung cancer (e.g., small cell lung cancer and non-small cell lung cancer (including adenocarcinoma, squamous cell carcinoma, bronchoalveolar carcinoma and large cell carcinoma)), pancreatic cancer (e.g., metastatic or locally advanced pancreatic cancer), gastric cancer (e.g., gastroesophageal, upper gastric or lower gastric cancer), colorectal cancer, squamous cell cancer of the head and neck, ovarian cancer (e.g., advanced ovarian cancer, platinum-based agent resistant or relapsed ovarian cancer), lymphoma (e.g., Burkitt's, Hodgkin's or non-Hodgkin's lymphoma), leukemia (e.g., acute myeloid leukemia) and gastrointestinal cancer.

In an embodiment, the CDP-pyrimidine analog conjugate, particle or composition, e.g., a CDP-gemcitabine conjugate, particle or composition, is provided at 1200-4950 mg/m²/month, e.g., 2000-4000 mg/m² /month or 3000-3750 mg/m²/month.

In one embodiment, the CDP-pyrimidine analog conjugate, particle or composition is administered in combination with one or more additional chemotherapeutic agents, e.g., a chemotherapeutic agent (such as an angiogenesis inhibitor) or combination of chemotherapeutic agents described herein. In one embodiment, the conjugate, particle or composition is administered in combination with one or more of: a platinum based agent (e.g., carboplatin, cisplatin, oxaliplatin), a taxane (e.g., paclitaxel, docetaxel, larotaxel, cabazitaxel), a vinca alkaloid (e.g., vinblastine, vincristine, vindesine, vinorelbine), an antimetabolite (e.g., an antifolate (e.g., floxuridine, pemetrexed), a pyrimidine analogue (e.g., 5FU, capecitabine)), an alkylating agent (e.g., cyclophosphamide, decarbazine, melphalan, ifosfamide, temozolomide), a vascular endothelial growth factor (VEGF) pathway inhibitor, a poly ADP-ribose polymerase (PARP) inhibitor and an mTOR inhibitor. In one embodiment, when the CDP-pyrimidine analog conjugate, particle or composition is administered in combination with an additional chemotherapeutic agent, the dose at which the CDP-pyrimidine analog conjugate, particle or composition is administered is 1%, 3%, 5%, 10%, 15%, 20%, 25%, 30% less than the doses described herein. In one embodiment, when the CDP-pyrimidine analog conjugate, particle or composition, e.g., a CDP-gemcitabine conjugate, particle or composition, is provided in combination with one or more additional chemotherapeutic agents, e.g., a chemotherapeutic agent described herein, the CDP-pyrimidine analog conjugate, particle or composition, e.g., a CDP-gemcitabine conjugate, particle or composition, is provided at 1000-4000 mg/m²/month.

In one embodiment, the CDP-pyrimidine analog conjugate, particle or composition, e.g., a CDP-gemcitabine conjugate, particle or composition, e.g., a CDP-gemcitabine conjugate, particle or composition, described herein is administered at a dosage of 600 mg/m², 700 mg/m², 730 mg/m², 750 mg/m², 780 mg/m², 800 mg/m², 830 mg/m², 850 mg/m², 880 mg/m², 900 mg/m², 930 mg/m², 950 mg/m², 980 mg/m², 1000 mg/m², 1030 mg/m², 1050 mg/m², 1080 mg/m², 1100 mg/m², 1130 mg/m², 1150 mg/m², 1180 mg/m², 1200 mg/m², 1230 mg/m², 1250 mg/m², 1280 mg/m², 1300 mg/m², 1350 mg/m², 1380 mg/m², 1400 mg/m², 1430 mg/m², 1450 mg/m², 1480 mg/m², 1500 mg/m², 1530 mg/m², 1550 mg/m², 1580 mg/m², 1600 mg/m², 1630 mg/m², or 1650 mg/m² by intravenous administration over a period equal to or less than about 30 minutes, 45 minutes, 60 minutes or 90 minutes, e.g., a period equal to or less than 30 minutes, 45 minutes or 60 minutes.

In an embodiment, the method includes an initial administration of the CDP-gemcitabine conjugate, particle or composition to the subject at a dosage of 600 mg/m², 700 mg/m², 730 mg/m², 750 mg/m², 780 mg/m², 800 mg/m², 830 mg/m², 850 mg/m², 880 mg/m², 900 mg/m², 930 mg/m², 950 mg/m², 980 mg/m², 1000 mg/m², 1030 mg/m², 1050 mg/m², 1080 mg/m², 1100 mg/m², 1130 mg/m², 1150 mg/m², 1180 mg/m², 1200 mg/m², 1230 mg/m², 1250 mg/m², 1280 mg/m², 1300 mg/m², 1350 mg/m², 1380 mg/m², 1400 mg/m², 1430 mg/m², 1450 mg/m², 1480 mg/m², 1500 mg/m², 1530 mg/m², 1550 mg/m², 1580 mg/m², 1600 mg/m², 1630 mg/m², or 1650 mg/m², and
one or more subsequent administrations of the CDP-gemcitabine conjugate, particle or composition to the subject, at a dosage of 600 mg/m², 700 mg/m², 730 mg/m², 750 mg/m², 780 mg/m², 800 mg/m², 830 mg/m², 850 mg/m², 880 mg/m², 900 mg/m², 930 mg/m², 950 mg/m², 980 mg/m², 1000 mg/m², 1030 mg/m², 1050 mg/m², 1080 mg/m², 1100 mg/m², 1130 mg/m², 1150 mg/m², 1180 mg/m², 1200 mg/m², 1230 mg/m², 1250 mg/m², 1280 mg/m², 1300 mg/m², 1350 mg/m², 1380 mg/m², 1400 mg/m², 1430 mg/m², 1450 mg/m², 1480 mg/m², 1500 mg/m², 1530 mg/m², 1550 mg/m², 1580 mg/m², 1600 mg/m², 1630 mg/m², or 1650 mg/m², e.g., at the same dosage as the initial dosage, wherein each subsequent administration is administered, independently, 5, 6, 7, 8, 9, 10, 11, 12, 13, 14, 15 or 16 days, e.g., 7 or 14, days after the previous, e.g., the initial, administration, and the cancer is, e.g., lung cancer, e.g., non-small cell lung cancer and/or small cell lung cancer (e.g., squamous cell non-small cell lung cancer, squamous cell small cell lung cancer, or nonsquamous cell non-small cell lung cancer). In one embodiment, the lung cancer is locally advanced or metastatic lung cancer, e.g., non-small cell lung cancer and/or small cell lung cancer.

In an embodiment, the method includes an initial administration of the CDP-gemcitabine conjugate, particle or composition to the subject at a dosage of 600 mg/m², 700 mg/m², 730 mg/m², 750 mg/m², 780 mg/m², 800 mg/m², 830 mg/m², 850 mg/m², 880 mg/m2, 900 mg/m², 930 mg/m², 950 mg/m², 980 mg/m², 1000 mg/m², 1030 mg/m², 1050 mg/m², 1080 mg/m², 1100 mg/m², 1130 mg/m², 1150 mg/m², 1180 mg/m², 1200 mg/m², 1230 mg/m², 1250 mg/m², 1280 mg/m², 1300 mg/m², 1350 mg/m², 1380 mg/m², 1400 mg/m², 1430 mg/m², 1450 mg/m², 1480 mg/m², 1500 mg/m², 1530 mg/m², 1550 mg/m², 1580 mg/m², 1600 mg/m², 1630 mg/m², or 1650 mg/m², and
one or more subsequent administrations of the CDP-gemcitabine conjugate, particle or composition to the subject, at a dosage of 600 mg/m², 700 mg/m², 730 mg/m², 750 mg/m², 780 mg/m², 800 mg/m², 830 mg/m², 850 mg/m², 880 mg/m², 900 mg/m², 930 mg/m², 950 mg/m², 980 mg/m², 1000 mg/m², 1030 mg/m², 1050 mg/m², 1080 mg/m², 1100 mg/m², 1130 mg/m², 1150 mg/m², 1180 mg/m², 1200 mg/m², 1230 mg/m², 1250 mg/m², 1280 mg/m², 1300 mg/m², 1350 mg/m², 1380 mg/m², 1400 mg/m², 1430 mg/m², 1450 mg/m², 1480 mg/m², 1500 mg/m², 1530 mg/m², 1550 mg/m², 1580 mg/m², 1600 mg/m², 1630 mg/m², or 1650 mg/m², e.g., at the same dosage as the initial dosage, wherein each subsequent administration is administered, independently, 5, 6, 7, 8, 9, 10, 11, 12, 13, 14, 15, 16, e.g., 7 or 14, days after the previous, e.g., the initial, administration, and the cancer is, e.g., pancreatic cancer, e.g., unresectable or metastatic pancreatic cancer.

In an embodiment, the method includes an initial administration of the CDP-gemcitabine conjugate, particle or composition to the subject at a dosage of 600 mg/m², 700 mg/m², 730 mg/m², 750 mg/m², 780 mg/m², 800 mg/m², 830 mg/m², 850 mg/m², 880 mg/m2, 900 mg/m², 930 mg/m², 950 mg/m², 980 mg/m², 1000 mg/m², 1030 mg/m², 1050 mg/m², 1080 mg/m², 1100 mg/m², 1130 mg/m², 1150 mg/m², 1180 mg/m², 1200 mg/m², 1230 mg/m², 1250 mg/m², 1280 mg/m², 1300 mg/m², 1350 mg/m², 1380 mg/m², 1400 mg/m2, 1430 mg/m², 1450 mg/m², 1480 mg/m², 1500 mg/m², 1530 mg/m², 1550 mg/m², 1580 mg/m², 1600 mg/m², 1630 mg/m², or 1650 mg/m², and
one or more subsequent administrations of the CDP-gemcitabine conjugate, particle or composition to the subject, at a dosage of 600 mg/m², 700 mg/m², 730 mg/m², 750 mg/m², 780 mg/m², 800 mg/m², 830 mg/m², 850 mg/m², 880 mg/m², 900 mg/m², 930 mg/m², 950 mg/m², 980 mg/m², 1000 mg/m², 1030 mg/m², 1050 mg/m², 1080 mg/m², 1100 mg/m², 1130 mg/m², 1150 mg/m², 1180 mg/m², 1200 mg/m², 1230 mg/m², 1250 mg/m2, 1280 mg/m², 1300 mg/m², 1350 mg/m², 1380 mg/m², 1400 mg/m², 1430 mg/m², 1450 mg/m², 1480 mg/m², 1500 mg/m², 1530 mg/m², 1550 mg/m², 1580 mg/m², 1600 mg/m², 1630 mg/m², or 1650 mg/m², e.g., at the same dosage as the initial dosage, wherein each subsequent administration is administered, independently, 5, 6, 7, 8, 9, 10, 11, 12, 13, 14, 15, or 16 days, e.g., 7 or 14, days after the previous, e.g., the initial, administration, and the cancer is, e.g., breast cancer, e.g., estrogen receptor positive breast cancer, estrogen receptor negative breast cancer, HER-2 positive breast cancer, HER-2 negative breast cancer, triple negative breast cancer or inflammatory breast cancer. In one embodiment, the breast cancer is metastatic breast cancer.

In an embodiment, the method includes an initial administration of a CDP-5FU conjugate, particle or composition at a dosage of 1 mg/kg, 2 mg/kg, 3 mg/kg, 4 mg/kg, 5 mg/kg, 6 mg/kg, 7 mg/kg, 8 mg/kg, 9 mg/kg, 10 mg/kg, 11 mg/kg, 12 mg/kg, 13 mg/kg, 14 mg/kg, 15 mg/kg, 16 mg/kg, 17 mg/kg, 18 mg/kg, 19 mg/kg, or 20 mg/kg (wherein the dosage is expressed in mg of drug, as opposed to mg of conjugate), and one or more subsequent administrations of a CDP-5FU conjugate, particle or composition at a dosage of 1 mg/kg, 2 mg/kg, 3 mg/kg, 4 mg/kg, 5 mg/kg, 6 mg/kg, 7 mg/kg, 8 mg/kg, 9 mg/kg, 10 mg/kg, 11 mg/kg, 12 mg/kg, 13 mg/kg, 14 mg/kg, 15 mg/kg, 16 mg/kg, 17 mg/kg, 18 mg/kg, 19 mg/kg, or 20 mg/kg, e.g., at the same dosage as the initial dosage. In some embodiments, each subsequent administration is provided, independently, between 1, 2, 3, 4, 5, 6, 7, 8, 9, 10, 11, 12, 13, 14, day(s) after the previous, e.g., the initial, administration.

In an embodiments, the CDP-5FU conjugate, particle or composition is administered intravenously once daily for 4 successive days.

In an embodiment, the cancer is carcinoma of the colon, rectum, breast, stomach or pancreas, and the CDP-pyrimidine analog conjugate, particle or composition is a CDP-5FU conjugate, particle or composition.

In an embodiment, the cancer is metastatic or refractory colorectal cancer, stage III colorectal cancer, locally advanced squamous cell carcinoma of the head and neck (SCCHN), or gastric adenocarcinoma, and the CDP-pyrimidine analog conjugate, particle or composition is a CDP-5FU conjugate, particle or composition.

In an embodiment, the cancer is superficial basal cell carcinoma or actinic keratosis, and the CDP-pyrimidine analog conjugate, particle or composition is a CDP-5FU conjugate, particle or composition.

In one embodiment, the subject has not been administered a CDP-pyrimidine analog conjugate, particle or composition, e.g., a CDP-gemcitabine conjugate, particle or composition, e.g., a CDP-gemcitabine conjugate, particle or composition, described herein, prior to the initial administration.

In an embodiment, the CDP-pyrimidine analog conjugate, particle or composition is administered as a first line treatment for the cancer.

In an embodiment, the CDP-pyrimidine analog conjugate, particle or composition is administered as a second, third or fourth line treatment for the cancer. In an embodiment, the cancer is sensitive to one or more chemotherapeutic agents, e.g., a platinum-based agent, a taxane, an alkylating agent, an anthracycline, an antimetabolite and/or a vinca alkaloid. In an embodiment, the cancer is a refractory, relapsed or resistant to one or more chemotherapeutic agents, e.g., a platinum-based agent, a taxane, an alkylating agent, an antimetabolite and/or a vinca alkaloid. In one embodiment, the cancer is, e.g., lung cancer, and the cancer is refractory, relapsed or resistant to a taxane (e.g., paclitaxel, docetaxel, larotaxel, cabazitaxel), a platinum-based agent (e.g., carboplatin, cisplatin, oxaliplatin), an anthracycline, a vinca alkaloid (e.g., vinblastine, vincristine, vindesine, vinorelbine), a vascular endothelial growth factor (VEGF) pathway inhibitor, an epidermal growth factor (EGF) pathway inhibitor) and/or an antimetabolite (e.g., an antifolate (e.g., pemetrexed, floxuridine, raltitrexed) and a pyrimidine analogue (e.g., capecitabine, cytarabine, 5FU)). In one embodiment, the cancer is, e.g., breast cancer, and the cancer is refractory, relapsed or resistant to a taxane (e.g., paclitaxel, docetaxel, larotaxel, cabazitaxel), a vascular endothelial growth factor (VEGF) pathway inhibitor, an anthracycline (e.g., daunorubicin, doxorubicin (e.g., liposomal doxorubicin), epirubicin, valrubicin, idarubicin), a platinum-based agent (e.g., carboplatin, cisplatin, oxaliplatin), and/or an antimetabolite (e.g., an antifolate (e.g., pemetrexed, floxuridine, raltitrexed) and a pyrimidine analogue (e.g., capecitabine, cytarabine, 5FU)).

In one embodiment, the subject has breast cancer (e.g., metastatic breast cancer), and the subject is administered a CDP-pyrimidine analog conjugate, particle or composition, e.g., a CDP-gemcitabine conjugate, particle or composition, e.g., a CDP-gemcitabine conjugate, particle or composition, described herein in combination with a taxane. In one embodiment, CDP-pyrimidine analog conjugate, particle or composition is administered in combination with a taxane (e.g., docetaxel, paclitaxel, larotaxel, or cabazitaxel). In one embodiment, the taxane (e.g., docetaxel, paclitaxel, larotaxel, or cabazitaxel) is administered at a dose of about 80 mg/m², 100 mg/m², 125 mg/m², 150 mg/m², 175 mg/m², or about 200 mg/m², every 17, 18, 19, 20, 21, 22, 23, 24, 25, 26, 27 or 28 days, e.g., 21 days. In one embodiment, the CDP-pyrimidine analog conjugate, particle or composition, e.g., a CDP-gemcitabine conjugate, particle or composition, e.g., a CDP-gemcitabine conjugate, particle or composition, described herein is administered at a dose and/or dosing regimen described herein and the taxane (e.g., docetaxel, paclitaxel, larotaxel, or cabazitaxel) is administered at a dose of about 80 mg/m², 100 mg/m², 125 mg/m², 150 mg/m², 175 mg/m², or about 200 mg/m², every 17, 18, 19, 20, 21, 22, 23, 24, 25, 26, 27 or 28 days, e.g., 21 days. In one embodiment, when the CDP-pyrimidine analog conjugate, particle or composition is administered in combination with the taxane (e.g., docetaxel, paclitaxel, larotaxel, or cabazitaxel), the dose at which the CDP-pyrimidine analog conjugate, particle or composition is administered is 1%, 3%, 5%, 10%, 15%, 20%, 25%, 30% less than a dose described herein.

In one embodiment, the subject has non-small cell lung cancer (e.g., locally advanced or metastatic non-small cell lung cancer), and the subject is administered a CDP-pyrimidine analog conjugate, particle or composition, e.g., a CDP-gemcitabine conjugate, particle or composition, e.g., a CDP-gemcitabine conjugate, particle or composition, described herein. In one embodiment, CDP-pyrimidine analog conjugate, particle or composition is administered in combination with a platinum-based chemotherapeutic (e.g., cisplatin, carboplatin, or oxaliplatin). In one embodiment, the platinum-based chemotherapeutic (e.g., cisplatin, carboplatin, or oxaliplatin) is administered at a dose of about 60 mg/m², 80 mg/m², 100 mg/m², 120 mg/m², or 140 mg/m², every 21, 24, 25, 26, 27, 28, 29, 30 or 31 days, e.g., 28 days. In one embodiment, the CDP-pyrimidine analog conjugate, particle or composition, e.g., a CDP-gemcitabine conjugate, particle or composition, e.g., a CDP-gemcitabine conjugate, particle or composition, described herein is administered at a dose and/or dosing regimen described herein and the platinum-based chemotherapeutic (e.g., cisplatin, carboplatin, or oxaliplatin) is administered at a dose of about 60 mg/m², 80 mg/m², 100 mg/m², 120 mg/m², or 140 mg/m², every 21, 24, 25, 26, 27, 28, 29, 30 or 31 days, e.g., 28 days. In one embodiment, when the CDP-pyrimidine analog conjugate, particle or composition is administered in combination with platinum-based chemotherapeutic (e.g., cisplatin, carboplatin, or oxaliplatin), the dose at which the CDP-pyrimidine analog conjugate, particle or composition is administered is 1%, 3%, 5%, 10%, 15%, 20%, 25%, 30% less than a dose described herein.

In one embodiment, the subject has non-small cell lung cancer (e.g., locally advanced or metastatic non-small cell lung cancer), and the CDP-pyrimidine analog conjugate, particle or composition is administered in combination with an angiogenesis inhibitor, e.g., a VEGF pathway inhibitor, e.g., soranenib or sunitinib. In one embodiment, the angiogenesis inhibitor, e.g., sorafenib, is administered at a dose of about 400 mg per day or less, daily, e.g., 350 mg per day, 300 mg per day, 250 mg per day, 200 mg per day, or 150 mg per day. In one embodiment, the angiogenesis inhibitor, e.g., sunitinib, is administered daily at a dose of about 50 mg per day or less, daily, e.g., 45 mg per day, 40 mg per day, 38 mg per day, 30 mg per day, 25 mg per day, 20 mg per day, or 15 mg per day. In one embodiment, when the CDP-pyrimidine analog conjugate, particle or composition is administered in combination with an angiogenesis inhibitor, e.g., sorafenib or sunitinib, the dose at which the CDP-pyrimidine analog conjugate, particle or composition is administered is 1%, 3%, 5%, 10%, 15%, 20%, 25%, or 30% less than a dose described herein.

In one aspect, the invention features, a method of treating cancer in a subject, e.g., a human subject. The method comprises:
providing an initial administration of a CDP-anti-tumor antibiotic conjugate, particle or composition, e.g., a CDP-HSP90 inhibitor conjugate, particle or composition, e.g., a CDP-geldanamycin conjugate, particle or composition, e.g., a CDP-geldanamycin conjugate, particle or composition described herein, to said subject, and, optionally, providing one or more subsequent administrations of said CDP-anti-tumor antibiotic conjugate, particle or composition, e.g., a CDP-HSP90 inhibitor conjugate, particle or composition, e.g., a CDP-geldanamycin conjugate, particle or composition, e.g., a CDP-geldanamycin conjugate, particle or composition described herein, wherein each subsequent administration is provided, independently, between 1, 2, 3, 4, 5, 6, 7, 8, 9, 10, 11, 12, 13, 14, 15, 16, 17, 18, 19, 20, 21 days, e.g., 3 or 7 days after the previous, e.g., the initial, administration, to thereby treat the cancer.

In an embodiment, the dosage of at least 2, 3, 4, 5, 6, 7, 8, 9, 10, 12, 15, or 20 administrations is the same.

In an embodiment, the time between at least 2, 3, 4, 5, 6, 7, 8, 9, 10, 12, 15, or 20 administrations is the same.

In an embodiment, each subsequent administration is administered 1-12, e.g., 3 or 7, days after the previous administration.

In an embodiment, at least 2, 3, 4, 5, 6, 7, 8, 9, 10, 12, 15, 20, 50 or 100 administrations are administered to the subject.

In one embodiment, the CDP-anti-tumor antibiotic conjugate, particle or composition, e.g., a CDP-HSP90 inhibitor conjugate, particle or composition, e.g., a CDP-geldanamycin conjugate, particle or composition, e.g., a CDP-geldanamycin conjugate, particle or composition described herein, is administered by intravenous administration over a period equal to or less than about 30 minutes, 45 minutes, 60 minutes, 90 minutes, 120 minutes, 150 minutes, or 180 minutes.

In an embodiment, the method includes an initial administration of a CDP-geldanamycin conjugate, particle or composition at a dosage of 20 mg/m², 30 mg/m², 40 mg/m², 50 mg/m², 60 mg/m², 70 mg/m², 75 mg/m², 80 mg/m², 85 mg/m², 90 mg/m², 95 mg/m², 100 mg/m², 105 mg/m², 110 mg/m², 115 mg/m², 120 mg/m², 125 mg/m², 130 mg/m², 140 mg/m², 150 mg/m², 160 mg/m², or 170 mg/m², and one or more subsequent administrations of a CDP-geldanamycin conjugate, particle or composition at a dosage of 20 mg/m², 30 mg/m², 40 mg/m², 50 mg/m², 60 mg/m², 70 mg/m², 75 mg/m², 80 mg/m², 85 mg/m², 90 mg/m², 95 mg/m², 100 mg/m², 105 mg/m², 110 mg/m², 115 mg/m², 120 mg/m², 125 mg/m², 130 mg/m², 140 mg/m², 150 mg/m², 160 mg/m², or 170 mg/m², e.g., at the same dosage as the initial dosage. In one embodiment, each subsequent administration is provided, independently, between 1, 2, 3, 4, 5, 6, 7, 8, 9, 10, 11, 12, 13, 14, 15, 16, 17, 18, 19, 20, 21, days after the previous, e.g., the initial, administration.

In an embodiment, the cancer is a cancer described herein. For example, the cancer can be a cancer of the bladder (including accelerated and metastatic bladder cancer), breast (e.g., estrogen receptor positive breast cancer, estrogen receptor negative breast cancer, HER-2 positive breast cancer, HER-2 negative breast cancer, triple negative breast cancer, inflammatory breast cancer), colon (including colorectal cancer), kidney (e.g., renal cell carcinoma), liver, lung (including small cell lung cancer and non-small cell lung cancer (including adenocarcinoma, squamous cell carcinoma, bronchoalveolar carcinoma and large cell carcinoma), mesothelioma, genitourinary tract, e.g., ovary (including fallopian, endometrial and peritoneal cancers), cervix, prostate and testes, lymphatic system, rectum, larynx, pancreas (including exocrine pancreatic carcinoma), stomach (e.g., gastroesophageal, upper gastric or lower gastric cancer), gastrointestinal cancer (e.g., anal cancer), gall bladder, thyroid, lymphoma (e.g., Burkitt's, Hodgkin's or non-Hodgkin's lymphoma (e.g., mantle cell lymphoma or anaplastic large cell lymphoma)), myeloma, leukemia (e.g., acute myeloid leukemia, acute lymphoblastic leukemia, chronic myelogenous leukemia, and chronic lymphoblastic leukemia), Ewing's sarcoma, nasoesophageal cancer, nasopharyngeal cancer, neural and glial cell cancers (e.g., glioblastoma multiforme, neuroblastoma), and head and neck. Preferred cancers include breast cancer (e.g., metastatic or locally advanced breast cancer), prostate cancer (e.g., hormone refractory prostate cancer), renal cell carcinoma, lung cancer (e.g., small cell lung cancer and non-small cell lung cancer (including adenocarcinoma, squamous cell carcinoma, bronchoalveolar carcinoma and large cell carcinoma)), pancreatic cancer (e.g., metastatic or locally advanced pancreatic cancer), gastric cancer (e.g., gastroesophageal, upper gastric or lower gastric cancer), bladder cancer, colorectal cancer, squamous cell cancer of the head and neck, ovarian cancer (e.g., advanced ovarian cancer, platinum-based agent resistant or relapsed ovarian cancer), lymphoma (e.g., Burkitt's, Hodgkin's or non-Hodgkin's lymphoma), leukemia (e.g., acute myeloid leukemia, acute lymphoblastic leukemia, chronic myelogenous leukemia, and chronic lymphoblastic leukemia), myeloma, and gastrointestinal cancer.

In one embodiment, the CDP-geldanamycin conjugate, particle or composition is administered in combination with one or more additional chemotherapeutic agents, e.g., a chemotherapeutic agent (such as an angiogenesis inhibitor) or combination of chemotherapeutic agents described herein. In one embodiment, the conjugate, particle or composition is administered in combination with one or more of: a taxane (e.g., paclitaxel, docetaxel, larotaxel, cabazitaxel), an antimetabolite (e.g., an antifolate (e.g., floxuridine, pemetrexed), a proteasome inhibitor (e.g., a boronic acid containing molecule, e.g., bortezomib), a pyrimidine analogue (e.g., 5FU, cytarabine, capecitabine)), a kinase inhibitor (e.g., imatinib), e.g., a vascular endothelial growth factor (VEGF) pathway inhibitor (e.g., sorafenib), a poly ADP-ribose polymerase (PARP) inhibitor and an mTOR inhibitor. In one embodiment, when the CDP- geldanamycin conjugate, particle or composition is administered in combination with an additional chemotherapeutic agent, the dose at which the CDP- geldanamycin conjugate, particle or composition is administered is 1%, 3%, 5%, 10%, 15%, 20%, 25%, 30% less than the doses described herein. In some embodiments, a CDP-geldanamycin conjugate, particle or composition is administered in combination with bortezomib, gemcitabine, belinostat, cytarabine, paclitaxel, rituximab, sorafenib, imatinib, irinotecan, or docetaxel.

In one aspect, the invention features, a method of treating cancer in a subject, e.g., a human subject. The method comprises:
providing an initial administration of CDP-platinum based agent conjugate, particle or composition, e.g., a CDP-cisplatin conjugate, particle or composition, e.g., a CDP-cisplatin conjugate, particle or composition, described herein, or, e.g., a CDP-carboplatin conjugate, particle or composition, e.g., a CDP-carboplatin conjugate, particle or composition, described herein, or, e.g., a CDP-oxaliplatin conjugate, particle or composition, e.g., a CDP-oxaliplatin conjugate, particle or composition, described herein, and, optionally, providing one or more subsequent administrations of said CDP-platinum based agent conjugate, particle or composition, e.g., a CDP-cisplatin conjugate, particle or composition, e.g., a CDP-cisplatin conjugate, particle or composition, described herein, or, e.g., a CDP-carboplatin conjugate, particle or composition, e.g., a CDP-carboplatin conjugate, particle or composition, described herein, or, e.g., a CDP-oxaliplatin conjugate, particle or composition, e.g., a CDP-oxaliplatin conjugate, particle or composition, described herein wherein each subsequent administration is provided, independently, between 1, 2, 3, 4, 5, 6, 7, 8, 9, 10, 11, 12, 13, 14, 15, 16, 17, 18, 19, 20, 21, 22, 23, 24, 25, 26, 27, 28 day(s) after the previous, e.g., the initial, administration, to thereby treat the cancer.

In an embodiment, the dosage of at least 2, 3, 4, 5, 6, 7, 8, 9, 10, 12, 15, or 20 administrations is the same.

In an embodiment, the time between at least 2, 3, 4, 5, 6, 7, 8, 9, 10, 12, 15, or 20 administrations is the same.

In an embodiment, each subsequent administration is administered 20-28, e.g., 21 or 28, days after the previous administration. In an embodiment, each subsequent administration is administered 1-5, e.g., 1, 3 day(s) after the previous administration.

In an embodiment, at least 2, 3, 4, 5, 6, 7, 8, 9, 10, 12, 15, 20, 50 or 100 administrations are administered to the subject.

In one embodiment, the CDP-platinum based agent conjugate, particle or composition, e.g., a CDP-cisplatin conjugate, particle or composition, e.g., a CDP-cisplatin conjugate, particle or composition, described herein, or, e.g., a CDP-carboplatin conjugate, particle or composition, e.g., a CDP-carboplatin conjugate, particle or composition, described herein, or, e.g., a CDP-oxaliplatin conjugate, particle or composition, e.g., a CDP-oxaliplatin conjugate, particle or composition, described herein, is administered by intravenous administration over a period equal to or less than about 30 minutes, 45 minutes, 60 minutes, 90 minutes, 120 minutes, 150 minutes, or 180 minutes.

In an embodiment, the method includes an initial administration of a CDP-cisplatin conjugate, particle or composition at a dosage of 10 mg/m², 15 mg/m², 20 mg/m², 25 mg/m², 30 mg/m², 40 mg/m², 50 mg/m², 60 mg/m², 70 mg/m², 75 mg/m², 80 mg/m², 85 mg/m², 90 mg/m², 95 mg/m², 100 mg/m², 105 mg/m², 110 mg/m², 115 mg/m², 120 mg/m², 125 mg/m², 130 mg/m², 140 mg/m², 150 mg/m², 160 mg/m², or 170 mg/m², and one or more subsequent administrations of a CDP-cisplatin conjugate, particle or composition at a dosage of 10 mg/m², 15 mg/m², 20 mg/m², 25 mg/m², 30 mg/m², 40 mg/m2, 50 mg/m², 60 mg/m², 70 mg/m², 75 mg/m², 80 mg/m², 85 mg/m², 90 mg/m², 95 mg/m², 100 mg/m², 105 mg/m², 110 mg/m², 115 mg/m², 120 mg/m², 125 mg/m², 130 mg/m², 140 mg/m², 150 mg/m², 160 mg/m², or 170 mg/m², e.g., at the same dosage as the initial dosage. In one embodiment, each subsequent administration is provided, independently, between 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, or 31, day(s) after the previous, e.g., the initial, administration.

In an embodiment, the cancer is a cancer described herein. For example, the cancer can be a cancer of the bladder (including accelerated and metastatic bladder cancer), breast (e.g., estrogen receptor positive breast cancer, estrogen receptor negative breast cancer, HER-2 positive breast cancer, HER-2 negative breast cancer, triple negative breast cancer, inflammatory breast cancer), colon (including colorectal cancer), kidney (e.g., renal cell carcinoma), liver, lung (including small cell lung cancer and non-small cell lung cancer (including adenocarcinoma, squamous cell carcinoma, bronchoalveolar carcinoma and large cell carcinoma), mesothelioma, genitourinary tract, e.g., ovary (including fallopian, endometrial and peritoneal cancers), cervix, prostate and testes (e.g., metastatic testicular cancer), lymphatic system, rectum, larynx, pancreas (including exocrine pancreatic carcinoma), stomach (e.g., gastroesophageal, upper gastric or lower gastric cancer), gastrointestinal cancer (e.g., anal cancer), gall bladder, thyroid, lymphoma (e.g., Burkitt's, Hodgkin's or non-Hodgkin's lymphoma (e.g., mantle cell lymphoma or anaplastic large cell lymphoma)), myeloma, leukemia (e.g., acute myeloid leukemia, acute lymphoblastic leukemia, chronic myelogenous leukemia, and chronic lymphoblastic leukemia), Ewing's sarcoma, nasoesophageal cancer, nasopharyngeal cancer, neural and glial cell cancers (e.g., glioblastoma multiforme, neuroblastoma), and head and neck. Preferred cancers include breast cancer (e.g., metastatic or locally advanced breast cancer), prostate cancer (e.g., hormone refractory prostate cancer) and testicular cancer (e.g., metastatic testicular cancer), renal cell carcinoma, lung cancer (e.g., small cell lung cancer and non-small cell lung cancer (including adenocarcinoma, squamous cell carcinoma, bronchoalveolar carcinoma and large cell carcinoma)), pancreatic cancer (e.g., metastatic or locally advanced pancreatic cancer), gastric cancer (e.g., gastroesophageal, upper gastric or lower gastric cancer), bladder cancer (e.g., advanced bladder cancer), colorectal cancer, squamous cell cancer of the head and neck, ovarian cancer (e.g., advanced ovarian cancer, resistant or relapsed ovarian cancer), lymphoma (e.g., Burkitt's, Hodgkin's or non-Hodgkin's lymphoma), leukemia (e.g., acute myeloid leukemia, acute lymphoblastic leukemia, chronic myelogenous leukemia, and chronic lymphoblastic leukemia), myeloma, and gastrointestinal cancer.

In one embodiment, the method is a method of treating testicular cancer, e.g., metastatic testicular cancer, in a subject and the method includes an initial administration of a CDP- cisplatin conjugate, particle or composition at a dosage of 10 mg/m², 15 mg/m², 20 mg/m², 25 mg/m², 30 mg/m², or 40 mg/m², and, optionally, one or more subsequent administrations of a CDP-cisplatin conjugate, particle or composition at a dosage of 10 mg/m², 15 mg/m², 20 mg/m², 25 mg/m², 30 mg/m², or 40 mg/m², e.g., at the same dosage as the initial dosage. In one embodiment, each subsequent administration is provided, independently, between 1, 2, 3, 4, 5, 6, 7, 8, 9 or 10 day(s) after the previous, e.g., the initial, administration.

In one embodiment, the method is a method of treating ovarian cancer, e.g., metastatic ovarian cancer, in a subject and the method includes an initial administration of a CDP- cisplatin conjugate, particle or composition at a dosage of 40 mg/m², 50 mg/m², 60 mg/m², 70 mg/m², 80 mg/m², 90 mg/m², 100 mg/m², or 110 mg/m², 120 mg/m², or 130 mg/m², and, optionally, one or more subsequent administrations of a CDP-cisplatin conjugate, particle or composition at a dosage of 40 mg/m², 50 mg/m², 60 mg/m², 70 mg/m², 80 mg/m², 90 mg/m², 100 mg/m², or 110 mg/m², 120 mg/m², or 130 mg/m², e.g., at the same dosage as the initial dosage. In one embodiment, each subsequent administration is provided, independently, between 17, 18, 19, 20, 21, 22, 23, 24, 25, 26, 27, 28, 29, 30 or 31 day(s) after the previous, e.g., the initial, administration. In some embodiments, the CDP-cisplatin conjugate, particle or composition is administered in combination with a second therapeutic agent, e.g., cyclophosphamide. In some embodiments, the CDP-cisplatin conjugate, particle or composition is administered in combination with surgical intervention or radiation.

In one embodiment, the method is a method of treating bladder cancer, e.g., advanced bladder cancer, in a subject and the method includes an initial administration of a CDP- cisplatin conjugate, particle or composition at a dosage of 40 mg/m², 50 mg/m², 60 mg/m², 70 mg/m², 80 mg/m², 90 mg/m², 100 mg/m², or 110 mg/m², 120 mg/m², or 130 mg/m², and, optionally, one or more subsequent administrations of a CDP-cisplatin conjugate, particle or composition at a dosage of 40 mg/m², 50 mg/m², 60 mg/m², 70 mg/m², 80 mg/m², 90 mg/m², 100 mg/m², or 110 mg/m², 120 mg/m², or 130 mg/m², e.g., at the same dosage as the initial dosage. In one embodiment, each subsequent administration is provided, independently, between 17, 18, 19, 20, 21, 22, 23, 24, 25, 26, 27, 28, 29, 30 or 31 day(s) after the previous, e.g., the initial, administration. In some embodiments, the CDP-cisplatin conjugate, particle or composition is administered in combination with surgical intervention or radiation.

In one aspect, the invention features, a method of treating cancer in a subject, e.g., a human subject. The method comprises:
providing an initial administration of CDP-kinase inhibitor conjugate, particle or composition, e.g., a CDP-seronine/threonine kinase inhibitor conjugate, particle or composition, e.g., a CDP-mTOR inhibitor conjugate, particle or composition, e.g., a CDP-rapamycin conjugate, particle or composition, e.g., a CDP-rapamycin conjugate, particle or composition, described herein, to said subject, and, optionally, providing one or more subsequent administrations of said CDP-kinase inhibitor conjugate, particle or composition, e.g., a CDP-seronine/threonine kinase inhibitor conjugate, particle or composition, e.g., a CDP-mTOR inhibitor conjugate, particle or composition, e.g., a CDP-rapamycin conjugate, particle or composition, e.g., a CDP-rapamycin conjugate, particle or composition, described herein, wherein each subsequent administration is provided, independently, between 1, 2, 3, 4, 5, 6, 7, 8, 9 day(s) after the previous, e.g., the initial, administration, to thereby treat the cancer.

In an embodiment, the dosage of at least 2, 3, 4, 5, 6, 7, 8, 9, 10, 12, 15, or 20 administrations is the same.

In an embodiment, the time between at least 2, 3, 4, 5, 6, 7, 8, 9, 10, 12, 15, or 20 administrations is the same.

In an embodiment, each subsequent administration is administered 1-9, e.g., 1, 2, 3, 4, days after the previous administration.

In an embodiment, at least 2, 3, 4, 5, 6, 7, 8, 9, 10, 12, 15, 20, 50 or 100 administrations are administered to the subject.

In one embodiment, the CDP-kinase inhibitor conjugate, particle or composition, e.g., a CDP-seronine/threonine kinase inhibitor conjugate, particle or composition, e.g., a CDP-mTOR inhibitor conjugate, particle or composition, e.g., a CDP-rapamycin conjugate, particle or composition, e.g., a CDP-rapamycin conjugate, particle or composition, described herein, is administered by intravenous administration over a period equal to or less than about 30 minutes, 45 minutes, 60 minutes, 90 minutes, 120 minutes, 150 minutes, or 180 minutes.

In one embodiment, the CDP-kinase inhibitor conjugate, particle or composition, e.g., a CDP-seronine/threonine kinase inhibitor conjugate, particle or composition, e.g., a CDP-mTOR inhibitor conjugate, particle or composition, e.g., a CDP-rapamycin conjugate, particle or composition, e.g., a CDP-rapamycin conjugate, particle or composition, described herein, is administered at a dosage of 2 mg, 3 mg, 4 mg, 5 mg, 6 mg, 7 mg, 8 mg, 9 mg, 10 mg, 12 mg, 15 mg, 20 mg, 25 mg, 30 mg, 35 mg, 40 mg, 45 mg, or 50 mg (wherein said dosage is expressed in mg of therapeutic agent, as opposed to mg of conjugate).

In an embodiment, the cancer is a cancer described herein. For example, the cancer can be a cancer of the bladder (including accelerated and metastatic bladder cancer), breast (e.g., estrogen receptor positive breast cancer, estrogen receptor negative breast cancer, HER-2 positive breast cancer, HER-2 negative breast cancer, triple negative breast cancer, inflammatory breast cancer), colon (including colorectal cancer), kidney (e.g., renal cell carcinoma), liver, lung (including small cell lung cancer and non-small cell lung cancer (including adenocarcinoma, squamous cell carcinoma, bronchoalveolar carcinoma and large cell carcinoma), mesothelioma, genitourinary tract, e.g., ovary (including fallopian, endometrial and peritoneal cancers), cervix, prostate and testes (e.g., metastatic testicular cancer), lymphatic system, rectum, larynx, pancreas (including exocrine pancreatic carcinoma), stomach (e.g., gastroesophageal, upper gastric or lower gastric cancer), gastrointestinal cancer (e.g., anal cancer), gall bladder, thyroid, lymphoma (e.g., Burkitt's, Hodgkin's or non-Hodgkin's lymphoma (e.g., mantle cell lymphoma or anaplastic large cell lymphoma)), myeloma, leukemia (e.g., acute myeloid leukemia, acute lymphoblastic leukemia, chronic myelogenous leukemia, and chronic lymphoblastic leukemia), Ewing's sarcoma, nasoesophageal cancer, nasopharyngeal cancer, neural and glial cell cancers (e.g., glioblastoma multiforme, neuroblastoma), and head and neck. Preferred cancers include breast cancer (e.g., metastatic or locally advanced breast cancer), prostate cancer (e.g., hormone refractory prostate cancer) and testicular cancer (e.g., metastatic testicular cancer), renal cell carcinoma, lung cancer (e.g., small cell lung cancer and non-small cell lung cancer (including adenocarcinoma, squamous cell carcinoma, bronchoalveolar carcinoma and large cell carcinoma)), pancreatic cancer (e.g., metastatic or locally advanced pancreatic cancer), gastric cancer (e.g., gastroesophageal, upper gastric or lower gastric cancer), bladder cancer (e.g., advanced bladder cancer), colorectal cancer, squamous cell cancer of the head and neck, ovarian cancer (e.g., advanced ovarian cancer, resistant or relapsed ovarian cancer), lymphoma (e.g., Burkitt's, Hodgkin's or non-Hodgkin's lymphoma), leukemia (e.g., acute myeloid leukemia, acute lymphoblastic leukemia, chronic myelogenous leukemia, and chronic lymphoblastic leukemia), myeloma, and gastrointestinal cancer.

In one embodiment, the method is a method of treating AKT-positive lymphomas in a subject and the method comprises administering a CDP-rapamycin conjugate, particle or composition, e.g., a CDP-rapamycin conjugate, particle or composition, described herein, at a dosage of 2 mg, 3 mg, 4 mg, 5 mg, 6 mg, 7 mg, 8 mg, 9 mg, 10 mg, 12 mg, 15 mg, 20 mg, 25 mg, 30 mg, 35 mg, 40 mg, 45 mg, or 50 mg (wherein said dosage is expressed in mg of therapeutic agent, as opposed to mg of conjugate). In some embodiments, the CDP-rapamycin conjugate, particle or composition, e.g., a CDP-rapamycin conjugate, particle or composition, described herein, is administered in combination with an anthracycline (e.g., doxorubicin (e.g., liposomal doxorubicin)).

In one aspect, the invention features, a method of treating cancer, e.g., in a subject. The method comprises administering two or more CDP-therapeutic agent conjugates, wherein one CDP is conjugated to a therapeutic agent and the other CDP is conjugated to a second therapeutic agent, a composition or particle including one or more of the CDP-therapeutic agent conjugates, to the subject to thereby treat the disease. In an embodiment, the CDP-therapeutic agent conjugate is a CDP-cytotoxic agent conjugate, e.g., CDP-topoisomerase inhibitor conjugate, e.g., a CDP-topoisomerase inhibitor I conjugate (e.g., a CDP-camptothecin conjugate, CDP-irinotecan conjugate, CDP-SN-38 conjugate, CDP-topotecan conjugate, CDP-lamellarin D conjugate, a CDP-lurotecan conjugate, particle or composition, a CDP-exatecan conjugate, particle or composition, a CDP-diflomotecan conjugate, particle or composition, and CDP-topoisomerase I inhibitor conjugates which include derivatives of camptothecin, irinotecan, SN-38, lamellarin D, lurotecan, exatecan, and diflomotecan), a CDP-topoisomerase II inhibitor conjugate (e.g., a CDP-etoposide conjugate, CDP-tenoposide conjugate, CDP-amsacrine conjugate and CDP-topoisomerase II inhibitor conjugates which include derivatives of etoposide, tenoposide, and amsacrine), a CDP-anti-metabolic agent conjugate (e.g., a CDP-antifolate conjugate (e.g., a CDP-pemetrexed conjugate, a CDP-floxuridine conjugate, a CDP-raltitrexed conjugate) or a CDP-pyrimidine analog conjugate (e.g., a CDP-capecitabine conjugate, a CDP-cytarabine conjugate, a CDP-gemcitabine conjugate, a CDP-5FU conjugate)), a CDP-alkylating agent conjugate, a CDP-anthracycline conjugate, a CDP-anti-tumor antibiotic conjugate (e.g., a CDP-HSP90 inhibitor conjugate, e.g., a CDP-geldanamycin conjugate, a CDP-tanespimycin conjugate or a CDP-alvespimycin conjugate), a CDP-platinum based agent conjugate (e.g., a CDP-cisplatin conjugate, a CDP-carboplatin conjugate, a CDP-oxaliplatin conjugate), a CDP-microtubule inhibitor conjugate, a CDP-kinase inhibitor conjugate (e.g., a CDP-seronine/threonine kinase inhibitor conjugate, e.g., a CDP-mTOR inhibitor conjugate, e.g., a CDP-rapamycin conjugate) or a CDP-proteasome inhibitor conjugate (a CDP-boronic acid containing molecule conjugate, e.g., a CDP-bortezomib conjugate); a CDP-immunomodulator conjugate (e.g., a corticosteroid or a rapamycin analog conjugate).

In any of the above aspects or embodiments, the CDP-therapeutic agent conjugate may be administered in the form of a pharmaceutical composition or a particle, e.g., a nanoparticle, e.g., a nanoparticle with an average diameter from 10 to 300 nm, e.g., 15 to 280, 30 to 250, 30 to 200, 20 to 150, 30 to 100, 20 to 80, 30 to 70, 30 to 60 or 30 to 50 nm. In one embodiment, the nanoparticle is 15 to 50 nm in diameter. In one embodiment, the average nanoparticle diameter is from 30 to 60 nm. In one embodiment, the surface charge of the molecule is neutral, or slightly negative. In some embodiments, the zeta potential of the particle surface is from about -80 mV to about 50 mV, about -20 mV to about 20 mV, about -20 mV to about -10 mV, or about -10 mV to about 0.

The details of one or more embodiments of the invention are set forth in the description below. Other features, objects, and advantages of the invention will be apparent from the description and the drawings, and from the claims.

### Brief Description of the Drawings

FIG. 1 depicts exemplary cyclodextrin-containing polymers (CDPs) which may be used for the delivery of therapeutic agents.
FIG. 2 depicts a schematic representation of (β)-cyclodextrin.
FIG. 3 depicts the structure of an exemplary cyclodextrin-containing polymer that may be used for the delivery of therapeutic agents.
FIG. 4 is a table depicting examples of different CDP-taxane conjugates.
FIG. 5 depicts structures of exemplary epothilones that can be used in the CDP-epothilone conjugates.
FIG. 6 is a table depicting examples of different CDP-epothilone conjugates.
FIG. 7 is a table depicting examples of different CDP-proteasome inhibitor conjugates.
FIG. 8 depicts a general strategy for synthesizing linear, branched, or grafted cyclodextrin-containing polymers (CDPs) for loading therapeutic agents, and, optionally, targeting ligands.
FIG. 9 depicts a general scheme for graft CDPs.
FIG. 10 depicts a general scheme of preparing linear CDPs.

### Detailed Description of the Invention

The present invention relates to compositions of therapeutic cyclodextrin-containing polymers (CDP) designed for drug delivery of therapeutic agents described herein. In certain embodiments, these cyclodextrin-containing polymers improve drug stability and/or solubility, and/or reduce toxicity, and/or improve efficacy of the therapeutic agent when used *in vivo.*

Furthermore, by selecting from a variety of linker groups that link or couple CDP to a therapeutic agent described herein, and/or targeting ligands, the rate of drug release from the polymers can be attenuated for controlled delivery. The invention also relates to methods of treating subjects with compositions described herein. The invention further relates to methods for conducting a pharmaceutical business comprising manufacturing, licensing, or distributing kits containing or relating to the CDP-therapeutic agent conjugates, particles and compositions described herein.

More generally, the present invention provides water-soluble, biocompatible polymer conjugates comprising a water-soluble, biocompatible polymer covalently attached to the topoisomerase inhibitor through attachments that are cleaved under biological conditions to release the therapeutic agent.

Polymeric conjugates featured in the methods described herein may be useful to improve solubility and/or stability of a bioactive/therapeutic agent, reduce drug-drug interactions, reduce interactions with blood elements including plasma proteins, reduce or eliminate immunogenicity, protect the agent from metabolism, modulate drug-release kinetics, improve circulation time, improve drug half-life (e.g., in the serum, or in selected tissues, such as tumors), attenuate toxicity, improve efficacy, normalize drug metabolism across subjects of different species, ethnicities, and/or races, and/or provide for targeted delivery into specific cells or tissues.

### Definitions

The term "ambient conditions," as used herein, refers to surrounding conditions at about one atmosphere of pressure, 50% relative humidity and about 25 °C.

The term "attach," as used herein with respect to the relationship of a first moiety to a second moiety, e.g., the attachment of a therapeutic agent to a polymer, refers to the formation of a covalent bond between a first moiety and a second moiety. In the same context, "attachment" refers to the covalent bond. For example, a therapeutic agent attached to a polymer is a therapeutic agent covalently bonded to the polymer (e.g., a hydrophobic polymer described herein). The attachment can be a direct attachment, e.g., through a direct bond of the first moiety to the second moiety, or can be through a linker (e.g., through a covalently linked chain of one or more atoms disposed between the first and second moiety). E.g., where an attachment is through a linker, a first moiety (e.g., a drug) is covalently bonded to a linker, which in turn is covalently bonded to a second moiety (e.g., a hydrophobic polymer described herein).

The term "biodegradable" is art-recognized, and includes polymers, compositions and formulations, such as those described herein, that are intended to degrade during use. Biodegradable polymers typically differ from non-biodegradable polymers in that the former may be degraded during use. In certain embodiments, such use involves *in vivo* use, such as *in vivo* therapy, and in other certain embodiments, such use involves *in vitro* use. In general, degradation attributable to biodegradability involves the degradation of a biodegradable polymer into its component subunits, or digestion, e.g., by a biochemical process, of the polymer into smaller, non-polymeric subunits. In certain embodiments, two different types of biodegradation may generally be identified. For example, one type of biodegradation may involve cleavage of bonds (whether covalent or otherwise) in the polymer backbone. In such biodegradation, monomers and oligomers typically result, and even more typically, such biodegradation occurs by cleavage of a bond connecting one or more of subunits of a polymer. In contrast, another type of biodegradation may involve cleavage of a bond (whether covalent or otherwise) internal to a side chain or that connects a side chain to the polymer backbone. In certain embodiments, one or the other or both general types of biodegradation may occur during use of a polymer.

The term "biodegradation," as used herein, encompasses both general types of biodegradation. The degradation rate of a biodegradable polymer often depends in part on a variety of factors, including the chemical identity of the linkage responsible for any degradation, the molecular weight, crystallinity, biostability, and degree of cross-linking of such polymer, the physical characteristics (e.g., shape and size) of a polymer, assembly of polymers or particle, and the mode and location of administration. For example, a greater molecular weight, a higher degree of crystallinity, and/or a greater biostability, usually lead to slower biodegradation.

The term "carbohydrate," as used herein refers to and encompasses monosaccharides, disaccharides, oligosaccharides and polysaccharides.

The phrase "cleavable under physiological conditions" refers to a bond having a half life of less than about 100 hours, when subjected to physiological conditions. For example, enzymatic degradation can occur over a period of less than about five years, one year, six months, three months, one month, fifteen days, five days, three days, or one day upon exposure to physiological conditions (e.g., an aqueous solution having a pH from about 4 to about 8, and a temperature from about 25°C to about 37°C).

An "effective amount" or "an amount effective" refers to an amount of the CDP-therapeutic agent conjugate which is effective, upon single or multiple dose administrations to a subject, in treating a cell, or curing, alleviating, relieving or improving a symptom of a disorder. An effective amount of the composition may vary according to factors such as the disease state, age, sex, and weight of the individual, and the ability of the compound to elicit a desired response in the individual. An effective amount is also one in which any toxic or detrimental effects of the composition are outweighed by the therapeutically beneficial effects.

"Pharmaceutically acceptable carrier or adjuvant," as used herein, refers to a carrier or adjuvant that may be administered to a patient, together with a CDP-therapeutic agent conjugate described herein, and which does not destroy the pharmacological activity thereof and is nontoxic when administered in doses sufficient to deliver a therapeutic amount of the particle. Some examples of materials which can serve as pharmaceutically acceptable carriers include: (1) sugars, such as lactose, glucose, mannitol and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical compositions.

The term "polymer," as used herein, is given its ordinary meaning as used in the art, i.e., a molecular structure featuring one or more repeat units (monomers), connected by covalent bonds. The repeat units may all be identical, or in some cases, there may be more than one type of repeat unit present within the polymer. In some cases, the polymer is biologically derived, i.e., a biopolymer. Non-limiting examples of biopolymers include peptides or proteins (i.e., polymers of various amino acids), or nucleic acids such as DNA or RNA. In some instances, a polymer may be comprised of subunits, e.g., a subunit described herein, wherein a subunit comprises polymers, e.g., PEG, but the subunit may be repeated within a conjugate. In some embodiments, a conjugate may comprise only one subunit described herein; however conjugates may comprise more than one identical subunit.

As used herein the term "low aqueous solubility" refers to water insoluble compounds having poor solubility in water, that is <5 mg/ml at physiological pH (6.5-7.4). Preferably, their water solubility is <1 mg/ml, more preferably <0.1 mg/ml. It is desirable that the drug is stable in water as a dispersion; otherwise a lyophilized or spraydried solid form may be desirable.

A "hydroxy protecting group" as used herein, is well known in the art and includes those described in detail in Protecting Groups in Organic Synthesis, T. W. Greene and P. G. M. Wuts, 3rd edition, John Wiley & Sons, 1999, the entirety of which is incorporated herein by reference. Suitable hydroxy protecting groups include, for example, acyl (e.g., acetyl), triethylsilyl (TES), *t*-butyldimethylsilyl (TBDMS), 2,2,2-trichloroethoxycarbonyl (Troc), and carbobenzyloxy (Cbz).

"Inert atmosphere," as used herein, refers to an atmosphere composed primarily of an inert gas, which does not chemically react with the CDP-therapeutic agent conjugates, particles, compositions or mixtures described herein. Examples of inert gases are nitrogen (N₂), helium, and argon.

"Linker," as used herein, is a moiety having at least two functional groups. One functional group is capable of reacting with a functional group on a polymer described herein, and a second functional group is capable of reacting with a functional group on agent described herein. In some embodiments the linker has just two functional groups. A linker may have more than two functional groups (e.g., 3, 4, 5, 6, 7, 8, 9, 10 or more functional groups), which may be used, e.g., to link multiple agents to a polymer. Depending on the context, linker can refer to a linker moiety before attachment to either of a first or second moiety (e.g., agent or polymer), after attachment to one moiety but before attachment to a second moiety, or the residue of the linker present after attachment to both the first and second moiety.

The term "lyoprotectant," as used herein refers to a substance present in a lyophilized preparation. Typically it is present prior to the lyophilization process and persists in the resulting lyophilized preparation. It can be used to protect nanoparticles, liposomes, and/or micelles during lyophilization, for example to reduce or prevent aggregation, particle collapse and/or other types of damage. In an embodiment the lyoprotectant is a cryoprotectant. In an embodiment the lyoprotectant is a carbohydrate.

As used herein, the term "prevent" or "preventing" as used in the context of the administration of an agent to a subject, refers to subjecting the subject to a regimen, e.g., the administration of a CDP-therapeutic agent conjugate such that the onset of at least one symptom of the disorder is delayed as compared to what would be seen in the absence of the regimen.

As used herein, the term "subject" is intended to include human and non-human animals. Exemplary human subjects include a human patient having a disorder, e.g., a disorder described herein, or a normal subject. The term "non-human animals" includes all vertebrates, e.g., non-mammals (such as chickens, amphibians, reptiles) and mammals, such as non-human primates, domesticated and/or agriculturally useful animals, e.g., sheep, dog, cat, cow, pig, etc.

The term "therapeutic agent," as used herein, refers to a moiety, wherein upon administration of the moiety to a subject, the subject receives a therapeutic effect (e.g., administration of the therapeutic agent treats a cell, or cures, alleviates, relieves or improves a symptom of a disorder).

As used herein, the term "treat" or "treating" a subject having a disorder refers to subjecting the subject to a regimen, e.g., the administration of a CDP-therapeutic agent conjugate such that at least one symptom of the disorder is cured, healed, alleviated, relieved, altered, remedied, ameliorated, or improved. Treating includes administering an amount effective to alleviate, relieve, alter, remedy, ameliorate, improve or affect the disorder or the symptoms of the disorder. The treatment may inhibit deterioration or worsening of a symptom of a disorder.

The term "acyl" refers to an alkylcarbonyl, cycloalkylcarbonyl, arylcarbonyl, heterocyclylcarbonyl, or heteroarylcarbonyl substituent, any of which may be further substituted (e.g., by one or more substituents). Exemplary acyl groups include acetyl (CH₃C(O)-), benzoyl (C₆H₅C(O)-), and acetylamino acids (e.g., acetylglycine, CH₃C(O)NHCH₂C(O)-.

The term "alkoxy" refers to an alkyl group, as defined below, having an oxygen radical attached thereto. Representative alkoxy groups include methoxy, ethoxy, propyloxy, tert-butoxy and the like.

The term "alkyl" refers to the radical of saturated aliphatic groups, including straight-chain alkyl groups, branched-chain alkyl groups, cycloalkyl (alicyclic) groups, alkyl-substituted cycloalkyl groups, and cycloalkyl-substituted alkyl groups. In preferred embodiments, a straight chain or branched chain alkyl has 30 or fewer carbon atoms in its backbone (e.g., C₁-C₃₀ for straight chains, C₃-C₃₀ for branched chains), and more preferably 20 or fewer, and most preferably 10 or fewer. Likewise, preferred cycloalkyls have from 3-10 carbon atoms in their ring structure, and more preferably have 5, 6 or 7 carbons in the ring structure. The term "alkylenyl" refers to a divalent alkyl, e.g., -CH₂-,-CH₂CH₂-, and -CH₂CH₂CH₂-.

The term "alkenyl" refers to an aliphatic group containing at least one double bond.

The terms "alkoxyl" or "alkoxy" refers to an alkyl group, as defined below, having an oxygen radical attached thereto. Representative alkoxyl groups include methoxy, ethoxy, propyloxy, tert-butoxy and the like. An "ether" is two hydrocarbons covalently linked by an oxygen.

The term "alkyl" refers to the radical of saturated aliphatic groups, including straight-chain alkyl groups, branched-chain alkyl groups, cycloalkyl (alicyclic) groups, alkyl-substituted cycloalkyl groups, and cycloalkyl-substituted alkyl groups. In preferred embodiments, a straight chain or branched chain alkyl has 30 or fewer carbon atoms in its backbone (e.g., C₁-C₃₀ for straight chains, C₃-C₃₀ for branched chains), and more preferably 20 or fewer, and most preferably 10 or fewer. Likewise, preferred cycloalkyls have from 3-10 carbon atoms in their ring structure, and more preferably have 5, 6 or 7 carbons in the ring structure.

The term "alkynyl" refers to an aliphatic group containing at least one triple bond.

The term "aralkyl" or "arylalkyl" refers to an alkyl group substituted with an aryl group (e.g., a phenyl or naphthyl).

The term "aryl" includes 5-14 membered single-ring or bicyclic aromatic groups, for example, benzene, naphthalene, and the like. The aromatic ring can be substituted at one or more ring positions with such substituents as described above, for example, halogen, azide, alkyl, aralkyl, alkenyl, alkynyl, cycloalkyl, polycyclyl, hydroxyl, alkoxyl, amino, nitro, sulfhydryl, imino, amido, phosphate, phosphonate, phosphinate, carbonyl, carboxyl, silyl, ether, alkylthio, sulfonyl, sulfonamido, ketone, aldehyde, ester, heterocyclyl, aromatic or heteroaromatic moieties, -CF₃, -CN, or the like. The term "aryl" also includes polycyclic ring systems having two or more cyclic rings in which two or more carbons are common to two adjoining rings (the rings are "fused rings") wherein at least one of the rings is aromatic, e.g., the other cyclic rings can be cycloalkyls, cycloalkenyls, cycloalkynyls, aryls and/or heterocyclyls. Each ring can contain, e.g., 5-7 members. The term "arylene" refers to a divalent aryl, as defined herein.

The term "arylalkenyl" refers to an alkenyl group substituted with an aryl group. The term "carboxy" refers to a -C(O)OH or salt thereof.

The term "hydroxy" and "hydroxyl" are used interchangeably and refer to -OH. The term "substituents" refers to a group "substituted" on an alkyl, cycloalkyl, alkenyl, alkynyl, heterocyclyl, heterocycloalkenyl, cycloalkenyl, aryl, or heteroaryl group at any atom of that group. Any atom can be substituted. Suitable substituents include, without limitation, alkyl (e.g., C1, C2, C3, C4, C5, C6, C7, C8, C9, C10, C11, C12 straight or branched chain alkyl), cycloalkyl, haloalkyl (e.g., perfluoroalkyl such as CF₃), aryl, heteroaryl, aralkyl, heteroaralkyl, heterocyclyl, alkenyl, alkynyl, cycloalkenyl, heterocycloalkenyl, alkoxy, haloalkoxy (e.g., perfluoroalkoxy such as OCF₃), halo, hydroxy, carboxy, carboxylate, cyano, nitro, amino, alkyl amino, SO₃H, sulfate, phosphate, methylenedioxy (-O-CH₂-O- wherein oxygens are attached to vicinal atoms), ethylenedioxy, oxo, thioxo (e.g., C=S), imino (alkyl, aryl, aralkyl), S(O)ₙalkyl (where n is 0-2), S(O)ₙ aryl (where n is 0-2), S(O)ₙ heteroaryl (where n is 0-2), S(O)ₙ heterocyclyl (where n is 0-2), amine (mono-, di-, alkyl, cycloalkyl, aralkyl, heteroaralkyl, aryl, heteroaryl, and combinations thereof), ester (alkyl, aralkyl, heteroaralkyl, aryl, heteroaryl), amide (mono-, di-, alkyl, aralkyl, heteroaralkyl, aryl, heteroaryl, and combinations thereof), sulfonamide (mono-, di-, alkyl, aralkyl, heteroaralkyl, and combinations thereof). In one aspect, the substituents on a group are independently any one single, or any subset of the aforementioned substituents. In another aspect, a substituent may itself be substituted with any one of the above substituents.

The terms "halo" and "halogen" means halogen and includes chloro, fluoro, bromo, and iodo.

The terms "hetaralkyl", "heteroaralkyl" or "heteroarylalkyl" refers to an alkyl group substituted with a heteroaryl group.

The term "heteroaryl" refers to an aromatic 5-8 membered monocyclic, 8-12 membered bicyclic, or 11-14 membered tricyclic ring system having 1-3 heteroatoms if monocyclic, 1-6 heteroatoms if bicyclic, or 1-9 heteroatoms if tricyclic, said heteroatoms selected from O, N, or S (e.g., carbon atoms and 1-3, 1-6, or 1-9 heteroatoms of N, O, or S if monocyclic, bicyclic, or tricyclic, respectively), wherein 0, 1, 2, 3, or 4 atoms of each ring may be substituted by a substituent. Examples of heteroaryl groups include pyridyl, furyl or furanyl, imidazolyl, benzimidazolyl, pyrimidinyl, thiophenyl or thienyl, quinolinyl, indolyl, thiazolyl, and the like. The term "heteroarylene" refers to a divalent heteroaryl, as defined herein.

The term "heteroarylalkenyl" refers to an alkenyl group substituted with a heteroaryl group.

### CDP-Therapeutic Agent Conjugates, Particles, and Compositions

Described herein are cyclodextrin containing polymer ("CDP")-therapeutic agent conjugates, wherein one or more therapeutic agents are covalently attached to the CDP (e.g., either directly or through a linker). The CDP-therapeutic agent conjugates include linear or branched cyclodextrin-containing polymers and polymers grafted with cyclodextrin. Exemplary cyclodextrin-containing polymers that may be modified as described herein are taught in U.S. Patent Nos. 7,270,808, 6,509,323, 7,091,192, 6,884,789, U.S. Publication Nos. 20040087024, 20040109888 and 20070025952.

The CDP-therapeutic agent conjugate can include a therapeutic agent such that the CDP-therapeutic agent conjugate can be used to treat an autoimmune disease, inflammatory disease, or cancer. Exemplary therapeutic agents that can be used in a conjugate described herein include the following: a topisomerase inhibitor, an anti-metabolic agent, a pyrimide analog, an alkylating agent, an anthracycline an anti-tumor antibiotic, a platinum based agent, a microtubule inhibitor, a proteasome inhibitor, and a corticosteroid.

Accordingly, in one embodiment the CDP-therapeutic agent conjugate is represented by Formula I: 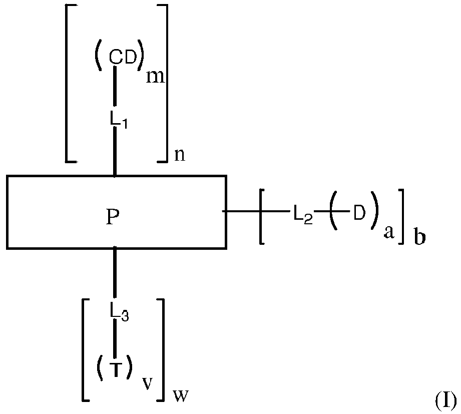 wherein
P represents a linear or branched polymer chain;
CD represents a cyclic moiety such as a cyclodextrin moiety;
L₁, L₂ and L₃, independently for each occurrence, may be absent or represent a linker group;
D, independently for each occurrence, represents a therapeutic agent or a prodrug thereof;
T, independently for each occurrence, represents a targeting ligand or precursor thereof;
a, m, and v, independently for each occurrence, represent integers in the range of 1 to 10 (preferably 1 to 8, 1 to 5, or even 1 to 3);
n and w, independently for each occurrence, represent an integer in the range of 0 to about 30,000 (preferably <25,000, <20,000, <15,000, <10,000, <5,000, <1,000, <500, <100, <50, <25, <10, or even <5); and
b represents an integer in the range of 1 to about 30,000 (preferably <25,000, <20,000, <15,000, <10,000, <5,000, <1,000, <500, <100, <50, <25, <10, or even <5),
wherein either P comprises cyclodextrin moieties or n is at least 1.

In some embodiments, one or more of one type of therapeutic agent in the CDP-therapeutic agent conjugate can be replaced with another, different type of therapeutic agent, e.g., another cytotoxic agent or immunomodulator. Examples of other cytotoxic agents are described herein. Examples of immunomodulators include a steroid, e.g., prednisone, and a NSAID.

In certain embodiments, P contains a plurality of cyclodextrin moieties within the polymer chain as opposed to the cyclodextrin moieties being grafted on to pendant groups off of the polymeric chain. Thus, in certain embodiments, the polymer chain of formula I further comprises n' units of U, wherein n' represents an integer in the range of 1 to about 30,000, e.g., from 4-100, 4-50, 4-25, 4-15, 6-100, 6-50, 6-25, and 6-15 (preferably <25,000, <20,000, <15,000, <10,000, <5,000, <1,000, <500, <100, <50, <25, <20, <15, <10, or even <5); and U is represented by one of the general formulae below: 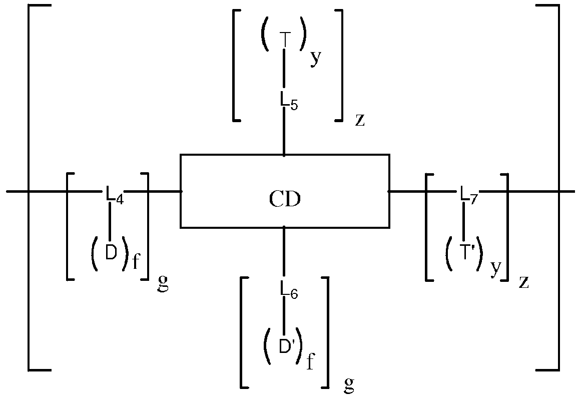 or 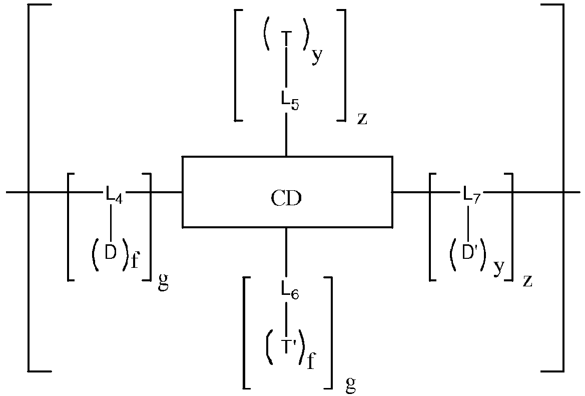 wherein
CD represents a cyclic moiety, such as a cyclodextrin moiety, or derivative thereof;
L₄, L₅, L₆, and L₇, independently for each occurrence, may be absent or represent a linker group;
D and D', independently for each occurrence, represent the same or different therapeutic agent or prodrug forms thereof;
T and T', independently for each occurrence, represent the same or different targeting ligand or precursor thereof;
f and y, independently for each occurrence, represent an integer in the range of 1 and 10; and
g and z, independently for each occurrence, represent an integer in the range of 0 and 10.

In some embodiments, one g is 0 and one g is 1-10. In some embodiments, one z is 0 and one z is 1-10.

Preferably the polymer has a plurality of D or D' moieties. In some embodiments, at least 50% of the U units have at least one D or D'. In some embodiments, one or more of one type of therapeutic agent in the CDP-therapeutic agent conjugate can be replaced with another, different type of therapeutic agent, e.g., another cytotoxic agent or immunomodulator.

In preferred embodiments, L₄ and L₇ represent linker groups.

The CDP may include a polycation, polyanion, or non-ionic polymer. A polycationic or polyanionic polymer has at least one site that bears a positive or negative charge, respectively. In certain such embodiments, at least one of the linker moiety and the cyclic moiety comprises such a charged site, so that every occurrence of that moiety includes a charged site. In some embodiments, the CDP is biocompatible.

In certain embodiments, the CDP may include polysaccharides, and other non-protein biocompatible polymers, and combinations thereof, that contain at least one terminal hydroxyl group, such as polyvinylpyrrollidone, poly(ethylene glycol) (PEG), polysuccinic anhydride, polysebacic acid, PEG-phosphate, polyglutamate, polyethylenimine, maleic anhydride divinylether (DIVMA), cellulose, pullulans, inulin, polyvinyl alcohol (PVA), N-(2-hydroxypropyl)methacrylamide (HPMA), dextran and hydroxyethyl starch (HES), and have optional pendant groups for grafting therapeutic agents, targeting ligands and/or cyclodextrin moieties. In certain embodiments, the polymer may be biodegradable such as poly(lactic acid), poly(glycolic acid), poly(alkyl 2-cyanoacrylates), polyanhydrides, and polyorthoesters, or bioerodible such as polylactide-glycolide copolymers, and derivatives thereof, non-peptide polyaminoacids, polyiminocarbonates, poly alpha-amino acids, polyalkyl-cyano-acrylate, polyphosphazenes or acyloxymethyl poly aspartate and polyglutamate copolymers and mixtures thereof.

In another embodiment the CDP-therapeutic agent conjugate is represented by Formula II: 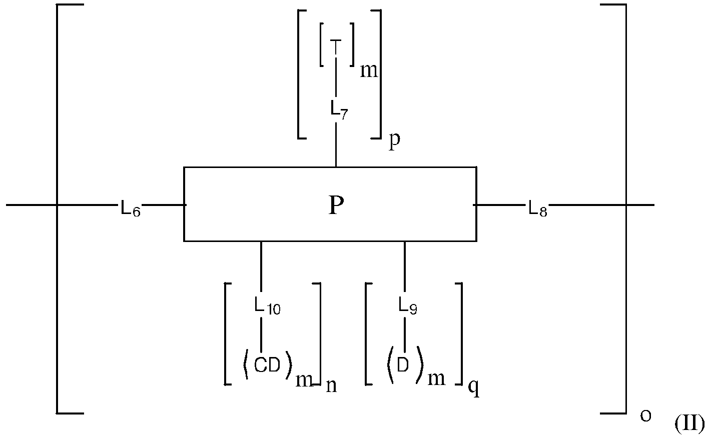 wherein
P represents a monomer unit of a polymer that comprises cyclodextrin moieties;
T, independently for each occurrence, represents a targeting ligand or a precursor thereof;
L₆, L₇, L₈, L₉, and L₁₀, independently for each occurrence, may be absent or represent a linker group;
CD, independently for each occurrence, represents a cyclodextrin moiety or a derivative thereof;
D, independently for each occurrence, represents a therapeutic agent or a prodrug form thereof;
m, independently for each occurrence, represents an integer in the range of 1 to 10 (preferably 1 to 8, 1 to 5, or even 1 to 3);
o represents an integer in the range of 1 to about 30,000 (preferably <25,000, <20,000, <15,000, <10,000, <5,000, <1,000, <500, <100, <50, <25, <10, or even <5); and
p, n, and q, independently for each occurrence, represent an integer in the range of 0 to 10 (preferably 0 to 8, 0 to 5, 0 to 3, or even 0 to about 2),
wherein CD and D are preferably each present at least 1 location (preferably at least 5, 10, 25, or even 50 or 100 locations) in the compound.

In some embodiments, one or more of the therapeutic agents in the CDP-therapeutic agent conjugate can be replaced with another, different therapeutic agent, e.g., another cytotoxic agent or immunomodulator. Examples of cytotoxic agents are described herein. Examples of immunomodulators include a steroid, e.g., prednisone, or a NSAID.

In another embodiment the CDP-therapeutic agent conjugate is represented either of the formulae below: 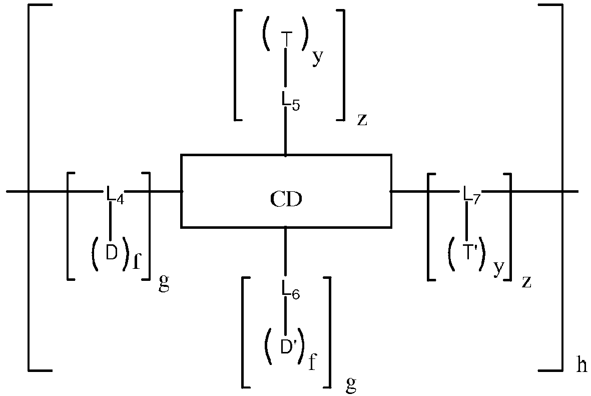 or 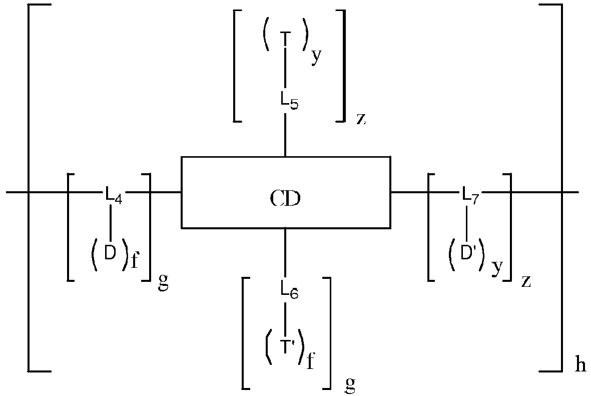 wherein
CD represents a cyclic moiety, such as a cyclodextrin moiety, or derivative thereof;
L₄, L₅, L₆, and L₇, independently for each occurrence, may be absent or represent a linker group;
D and D', independently for each occurrence, represent the same or different therapeutic agent;
T and T', independently for each occurrence, represent the same or different targeting ligand or precursor thereof;
f and y, independently for each occurrence, represent an integer in the range of 1 and 10 (preferably 1 to 8, 1 to 5, or even 1 to 3);
g and z, independently for each occurrence, represent an integer in the range of 0 and 10 (preferably 0 to 8, 0 to 5, 0 to 3, or even 0 to about 2); and
h represents an integer in the range of 1 and 30,000, e.g., from 4-100, 4-50, 4-25, 4-15, 6-100, 6-50, 6-25, and 6-15 (preferably <25,000, <20,000, <15,000, <10,000, <5,000, <1,000, <500, <100, <50, <25, <20, <15, <10, or even <5),
wherein at least one occurrence (and preferably at least 5, 10, or even at least 20, 50, or 100 occurrences) of g represents an integer greater than 0.

In some embodiments, one g is 0 and one g is 1-10. In some embodiments, one z is 0 and one z is 1-10.

Preferably the polymer has a plurality of D or D' moieties. In some embodiments, at least 50% of the polymer repeating units have at least one D or D'. In some embodiments, one or more of the therapeutic agent in the CDP-therapeutic agent conjugate can be replaced with another therapeutic agent, e.g., another cytotoxic agent or immunomodulator.

In preferred embodiments, L4 and L7 represent linker groups.

In certain such embodiments, the CDP comprises cyclic moieties alternating with linker moieties that connect the cyclic structures, e.g., into linear or branched polymers, preferably linear polymers. The cyclic moieties may be any suitable cyclic structures, such as cyclodextrins, crown ethers (e.g., 18-crown-6, 15-crown-5, 12-crown-4, etc.), cyclic oligopeptides (e.g., comprising from 5 to 10 amino acid residues), cryptands or cryptates (e.g., cryptand [2.2.2], cryptand-2,1,1, and complexes thereof), calixarenes, or cavitands, or any combination thereof. Preferably, the cyclic structure is (or is modified to be) water-soluble. In certain embodiments, e.g., for the preparation of a linear polymer, the cyclic structure is selected such that under polymerization conditions, exactly two moieties of each cyclic structure are reactive with the linker moieties, such that the resulting polymer comprises (or consists essentially of) an alternating series of cyclic moieties and linker moieties, such as at least four of each type of moiety. Suitable difunctionalized cyclic moieties include many that are commercially available and/or amenable to preparation using published protocols. In certain embodiments, conjugates are soluble in water to a concentration of at least 0.1 g/mL, preferably at least 0.25 g/mL.

Thus, in certain embodiments, the invention relates to novel compositions of therapeutic cyclodextrin-containing polymeric compounds designed for delivery of a therapeutic agent described herein. In certain embodiments, these CDPs improve drug stability and/or solubility, and/or reduce toxicity, and/or improve efficacy of the therapeutic agent when used *in vivo.* Furthermore, by selecting from a variety of linker groups, and/or targeting ligands, the rate of therapeutic agent release from the CDP can be attenuated for controlled delivery.

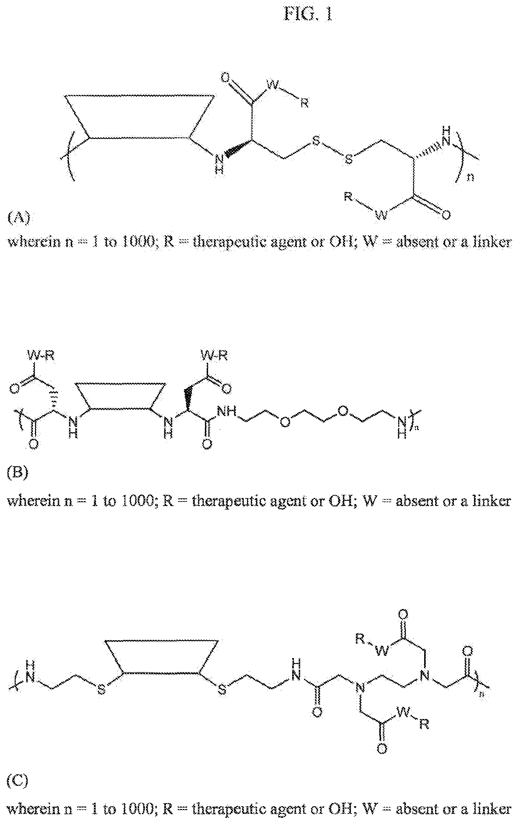

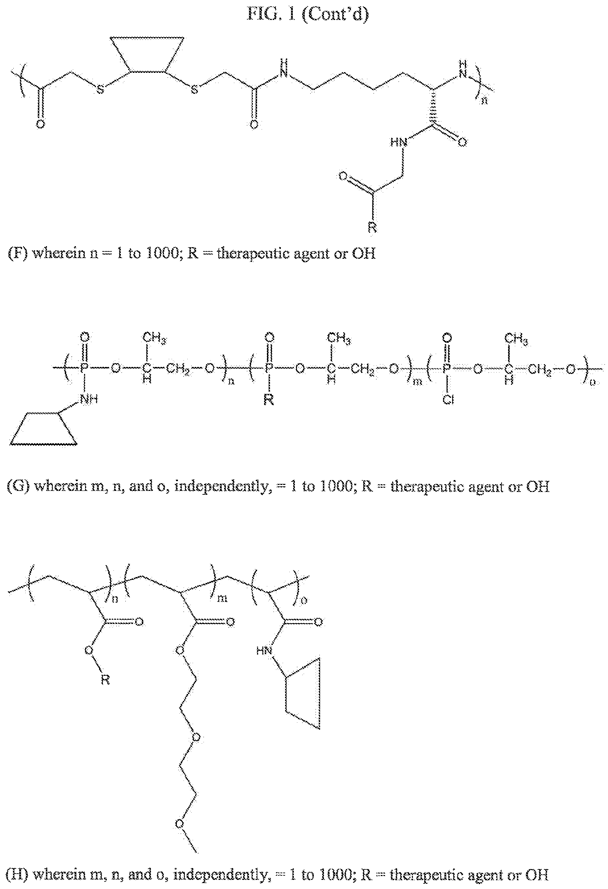

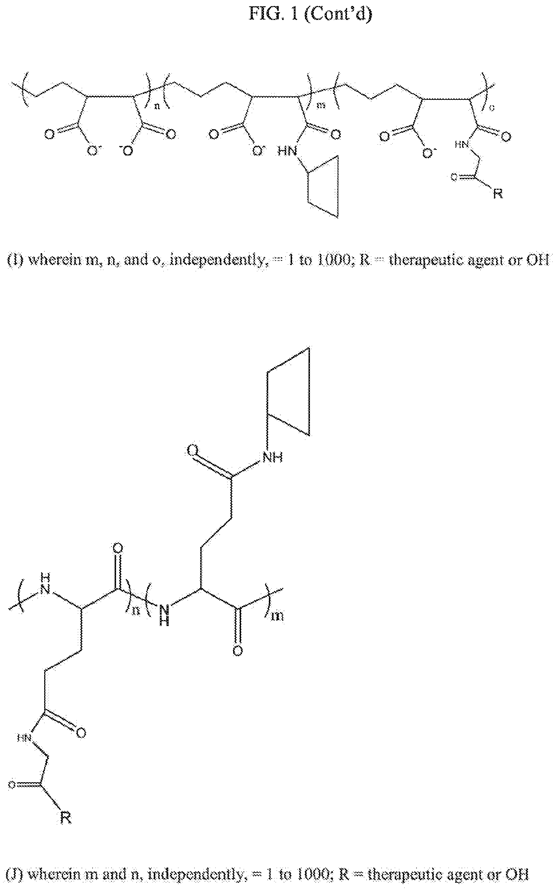

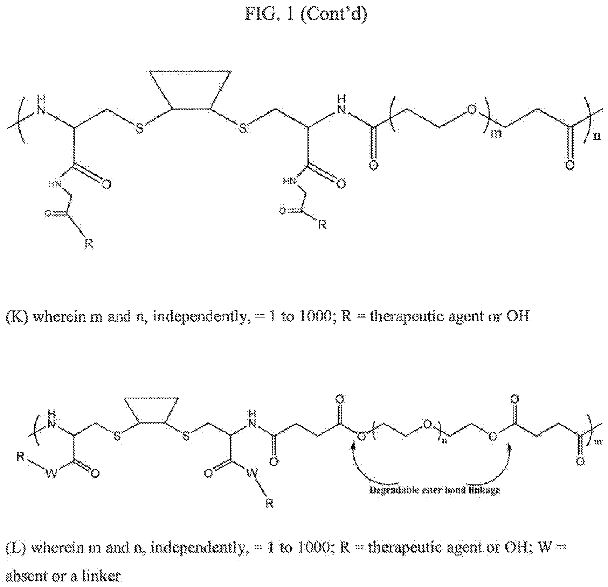

Disclosed herein are various types of linear, branched, or grafted CDPs wherein a therapeutic agent is covalently bound to the polymer. In certain embodiments, the therapeutic agent is covalently linked via a biohydrolyzable bond, for example, an ester, amide, carbamates, or carbonate. General strategies for synthesizing linear, branched or grafted cyclodextrin-containing polymers (CDPs) for loading therapeutic agents, and optional targeting ligands are described in U.S. Patent Nos. 7,270,808, 6,509,323, 7,091,192, 6,884,789, U.S. Publication Nos. 20040087024, 20040109888 and 20070025952, all of which are incorporated by reference in their entireties. As shown in FIG. 1, the general strategies can be used to achieve a variety of different cyclodextrin-containing polymers for the delivery of therapeutic agents, e.g., cytotoxic agents, e.g., topoisomerase inhibitors, e.g., a topoisomerase I inhibitor (e.g., camptothecin, irinotecan, SN-38, topotecan, lamellarin D, lurotecan, exatecan, diflomotecan, or derivatives thereof), or a topoisomerase II inhibitor (e.g., an etoposide, a tenoposide, amsacrine, or derivatives thereof), an anti-metabolic agent (e.g., an antifolate (e.g., pemetrexed, floxuridine, or raltitrexed) or a pyrimidine conjugate (e.g., capecitabine, cytarabine, gemcitabine, or 5FU)), an alkylating agent, an anthracycline, an anti-tumor antibiotic (e.g., a HSP90 inhibitor, e.g., geldanamycin), a platinum based agent (e.g., cisplatin, carboplatin, or oxaliplatin), a microtubule inhibitor, a kinase inhibitor (e.g., a seronine/threonine kinase inhibitor, e.g., a mTOR inhibitor, e.g., rapamycin) or a proteasome inhibitor. The resulting CDPs are shown graphically as polymers (A)-(L) of FIG. 1. Generally, wherein R can be a therapeutic agent or an OH, it is required that at least one R within the polymer be a therapeutic agent, e.g., the loading is not zero. Generally, m, n, and o, if present, are independently from 1 to 1000, e.g., 1 to 500, e.g., 1 to 100, e.g., 1 to 50, e.g., 1 to 25, e.g., 10 to 20, e.g. about 14.

In certain embodiments, the CDP comprises a linear cyclodextrin-containing polymer, e.g., the polymer backbone includes cyclodextrin moieties. For example, the polymer may be a water-soluble, linear cyclodextrin polymer produced by providing at least one cyclodextrin derivative modified to bear one reactive site at each of exactly two positions, and reacting the cyclodextrin derivative with a linker having exactly two reactive moieties capable of forming a covalent bond with the reactive sites under polymerization conditions that promote reaction of the reactive sites with the reactive moieties to form covalent bonds between the linker and the cyclodextrin derivative, whereby a linear polymer comprising alternating units of cyclodextrin derivatives and linkers is produced. Alternatively the polymer may be a water-soluble, linear cyclodextrin polymer having a linear polymer backbone, which polymer comprises a plurality of substituted or unsubstituted cyclodextrin moieties and linker moieties in the linear polymer backbone, wherein each of the cyclodextrin moieties, other than a cyclodextrin moiety at the terminus of a polymer chain, is attached to two of said linker moieties, each linker moiety covalently linking two cyclodextrin moieties. In yet another embodiment, the polymer is a water-soluble, linear cyclodextrin polymer comprising a plurality of cyclodextrin moieties covalently linked together by a plurality of linker moieties, wherein each cyclodextrin moiety, other than a cyclodextrin moiety at the terminus of a polymer chain, is attached to two linker moieties to form a linear cyclodextrin polymer.

In some embodiments, the CDP-therapeutic agent conjugate comprises a water soluble linear polymer conjugate comprising: cyclodextrin moieties; comonomers which do not contain cyclodextrin moieties (comonomers); and a plurality of therapeutic agents; wherein the CDP-therapeutic agent conjugate comprises at least four, five six, seven, eight, etc., cyclodextrin moieties and at least four, five six, seven, eight, etc., comonomers. In some embodiments, the therapeutic agent is a therapeutic gaent described herein, e.g., the CDP-therapeutic agent conjugate is a CDP-cytotoxic agent conjugate, e.g., CDP-topoisomerase inhibitor conjugate, e.g., a CDP-topoisomerase inhibitor I conjugate (e.g., a CDP-camptothecin conjugate, CDP-irinotecan conjugate, CDP-SN-38 conjugate, CDP-topotecan conjugate, CDP-lamellarin D conjugate, a CDP-lurotecan conjugate, particle or composition, a CDP-exatecan conjugate, particle or composition, a CDP-diflomotecan conjugate, particle or composition, and CDP-topoisomerase I inhibitor conjugates which include derivatives of camptothecin, irinotecan, SN-38, lamellarin D, lurotecan, exatecan, and diflomotecan), a CDP-topoisomerase II inhibitor conjugate (e.g., a CDP-eptoposide conjugate, CDP-tenoposide conjugate, CDP-amsacrine conjugate and CDP-topoisomerase II inhibitor conjugates which include derivatives of etoposide, tenoposide, and amsacrine), a CDP-anti-metabolic agent conjugate (e.g., a CDP-antifolate conjugate (e.g., a CDP-pemetrexed conjugate, a CDP-floxuridine conjugate, a CDP-raltitrexed conjugate) or a CDP-pyrimidine analog conjugate (e.g., a CDP-capecitabine conjugate, a CDP-cytarabine conjugate, a CDP-gemcitabine conjugate, a CDP-5FU conjugate)), a CDP-alkylating agent conjugate, a CDP-anthracycline conjugate, a CDP-anti-tumor antibiotic conjugate (e.g., a CDP-HSP90 inhibitor conjugate, e.g., a CDP-geldanamycin conjugate, a CDP-tanespimycin conjugate or a CDP-alvespimycin conjugate), a CDP-platinum based agent conjugate (e.g., a CDP-cisplatin conjugate, a CDP-carboplatin conjugate, a CDP-oxaliplatin conjugate), a CDP-microtubule inhibitor conjugate, a CDP-kinase inhibitor conjugate (e.g., a CDP-seronine/threonine kinase inhibitor conjugate, e.g., a CDP-mTOR inhibitor conjugate, e.g., a CDP-rapamycin conjugate) or a a CDP-proteasome inhibitor conjugate (e.g., CDP-boronic acid containing molecule conjugate, e.g., a CDP-bortezomib conjugate) or a CDP-immunomodulator conjugate (e.g., a CDP-corticosteroid or a CDP-rapamycin analog conjugate).

The therapeutic agent can be attached to the CDP via a functional group such as a hydroxyl group, or where appropriate, an amino group.

In some embodiments, one or more of one type of therapeutic agent in the CDP-therapeutic agent conjugate can be replaced with another, different type of therapeutic agent, e.g., another anticancer agent or anti-inflammatory agent.

In some embodiments, the least four cyclodextrin moieties and at least four comonomers alternate in the CDP-therapeutic agent conjugate. In some embodiments, the therapeutic agents are cleaved from said CDP-therapeutic agent conjugate under biological conditions to release the therapeutic agent. In some embodiments, the cyclodextrin moieties comprise linkers to which therapeutic agents are linked. In some embodiments, the therapeutic agents are attached via linkers.

In some embodiments, the comonomer comprises residues of at least two functional groups through which reaction and linkage of the cyclodextrin monomers was achieved. In some embodiments, the functional groups, which may be the same or different, terminal or internal, of each comonomer comprise an amino, acid, imidazole, hydroxyl, thio, acyl halide, -HC=CH-, -C≡C- group, or derivative thereof. In some embodiments, the two functional groups are the same and are located at termini of the comonomer precursor. In some embodiments, a comonomer contains one or more pendant groups with at least one functional group through which reaction and thus linkage of a therapeutic agent was achieved. In some embodiments, the functional groups, which may be the same or different, terminal or internal, of each comonomer pendant group comprise an amino, acid, imidazole, hydroxyl, thiol, acyl halide, ethylene, ethyne group, or derivative thereof. In some embodiments, the pendant group is a substituted or unsubstituted branched, cyclic or straight chain C1-C10 alkyl, or arylalkyl optionally containing one or more heteroatoms within the chain or ring. In some embodiments, the cyclodextrin moiety comprises an alpha, beta, or gamma cyclodextrin moiety. In some embodiments, the therapeutic agent is at least 5%, 10%, 15%, 20%, 25%, 30%, or 35% by weight of CDP-therapeutic agent conjugate.

In some embodiments, the comonomer comprises polyethylene glycol of molecular weight 3,400 Da, the cyclodextrin moiety comprises beta-cyclodextrin, the theoretical maximum loading of a therapeutic agent such as a topoisomerase inhibitor on a CDP-therapeutic agent conjugate (e.g., a CDP-topoisomerase inhibitor conjugate) is 25% (e.g., 20%, 15%, 13%, or 10%) by weight, and the therapeutic agent (e.g., a topoisomerase inhibitor) is 4-20% by weight (e.g., 6-10% by weight) of CDP-therapeutic agent conjugate (e.g., CDP-topoisomerase inhibitor conjugate). In some embodiments, the therapeutic agent (e.g., a topoisomerase inhibitor) is poorly soluble in water. In some embodiments, the solubility of the therapeutic agent (e.g., a topoisomerase inhibitor) is <5 mg/ml at physiological pH. In some embodiments, the therapeutic agent (e.g., a topoisomerase inhibitor) is a hydrophobic compound with a log P>0.4, >0.6, >0.8, >1, >2, >3, >4, or >5.

In some embodiments, the therapeutic agent is attached to the CDP via a second compound (e.g., a linker).

In some embodiments, administration of the CDP-therapeutic agent conjugate to a subject results in release of the therapeutic agent over a period of at least 6 hours. In some embodiments, administration of the CDP-therapeutic agent conjugate to a subject results in release of the thereapeutic agent over a period of 2 hours, 3 hours, 5 hours, 6 hours, 8 hours, 10 hours, 15 hours, 20 hours, 1 day, 2 days, 3 days, 4 days, 7 days, 10 days, 14 days, 17 days, 20 days, 24 days, 27 days up to a month. In some embodiments, upon administration of the CDP-therapeutic agent conjugate to a subject, the rate of therapeutic agent release is dependent primarily upon the rate of hydrolysis of the therapeutic agent as opposed to enzymatic cleavage.

In some embodiments, the CDP-therapeutic agent conjugate has a molecular weight of 10,000-500,000 Da (e.g., 20,000-300,000, 30,000-200,000, or 40,000-200,000, or 50,000-100,000). In some embodiments, the cyclodextrin moieties make up at least about 2%, 5%, 10%, 20%, 30%, 50% or 80% of the CDP-therapeutic agent conjugate by weight.

In some embodiments, the CDP-therapeutic agent conjugate is made by a method comprising providing cyclodextrin moiety precursors modified to bear one reactive site at each of exactly two positions, and reacting the cyclodextrin moiety precursors with comonomer precursors having exactly two reactive moieties capable of forming a covalent bond with the reactive sites under polymerization conditions that promote reaction of the reactive sites with the reactive moieties to form covalent bonds between the comonomers and the cyclodextrin moieties, whereby a CDP comprising alternating units of a cyclodextrin moiety and a comonomer is produced. In some embodiments, the cyclodextrin moiety precursors are in a composition, the composition being substantially free of cyclodextrin moieties having other than two positions modified to bear a reactive site (e.g., cyclodextrin moieties having 1, 3, 4, 5, 6, or 7 positions modified to bear a reactive site).

In some embodiments, a comonomer of the CDP-therapeutic agent conjugate comprises a moiety selected from the group consisting of: an alkylene chain, polysuccinic anhydride, poly-L-glutamic acid, poly(ethyleneimine), an oligosaccharide, and an amino acid chain. In some embodiments, a CDP-therapeutic agent conjugate comonomer comprises a polyethylene glycol chain. In some embodiments, a comonomer comprises a moiety selected from: polyglycolic acid and polylactic acid chain. In some embodiments, a comonomer comprises a hydrocarbylene group wherein one or more methylene groups is optionally replaced by a group Y (provided that none of the Y groups are adjacent to each other), wherein each Y, independently for each occurrence, is selected from, substituted or unsubstituted aryl, heteroaryl, cycloalkyl, heterocycloalkyl, or -O-, C(=X) (wherein X is NR₁, O or S), -OC(O)-, -C(=O)O, -NR₁-, -NR₁CO-,-C(O)NR₁-, -S(O)ₙ- (wherein n is 0, 1, or 2), -OC(O)-NR₁, -NR₁-C(O)-NR₁-, -NR₁1-C(NR₁)-NR₁-, and -B(OR₁)-; and R₁, independently for each occurrence, represents H or a lower alkyl.

In some embodiments, the CDP-therapeutic agent conjugate is a polymer having attached thereto a plurality of D moieties of the following formula: 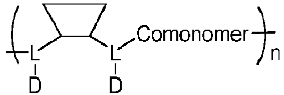 wherein each L is independently a linker, and each D is independently a therapeutic agent, a prodrug derivative thereof, or absent; and each comonomer is independently a comonomer described herein, and n is at least 4, 5, 6, 7, 8, 9, 10, 11, 12, 13, 14, 15, 16, 17, 18, 19 or 20, provided that the polymer comprises at least one therapeutic agent and in some embodiments, at least two therapeutic agent. In some embodiments, the molecular weight of the comonomer is from about 2000 to about 5000 Da (e.g., from about 3000 to about 4000 Da (e.g., about 3400 Da).

In some embodiments, the therapeutic agent is a therapeutic agent described herein. The therapeutic agent can be attached to the CDP via a functional group such as a hydroxyl group, or where appropriate, an amino group. In some embodiments, one or more of the therapeutic agent in the CDP-therapeutic agent conjugate can be replaced with another therapeutic agent, e.g., another cytotoxic agent or immunomodulator.

In some embodiments, the CDP-therapeutic agent conjugate is a polymer having attached thereto a plurality of D moieties of the following formula: 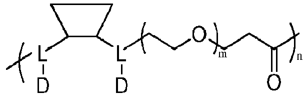 wherein each L is independently a linker, and each D is independently a therapeutic agent, a prodrug derivative thereof, or absent, provided that the polymer comprises at least one therapeutic agent and in some embodiments, at least two therapeutic agent; and
wherein the group 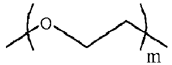 has a Mw of 3400 Da or less and n is at least 4, 5, 6, 7, 8, 9, 10, 11, 12, 13, 14, 15, 16, 17, 18, 19 or 20.

In some embodiments, the therapeutic agent is a therapeutic agent described herein. The therapeutic agent can be attached to the CDP via a functional group such as a hydroxyl group, or where appropriate, an amino group. In some embodiments, one or more of the therapeutic agent in the CDP-therapeutic agent conjugate can be replaced with another therapeutic agent, e.g., another cytotoxic agent or immunomodulator.

In some embodiments, less than all of the L moieties are attached to D moieties, meaning in some embodiments, at least one D is absent. In some embodiments, the loading of the D moieties on the CDP-therapeutic agent conjugate is from about 1 to about 50% (e.g., from about 1 to about 40%, from about 1 to about 25%, from about 5 to about 20% or from about 5 to about 15%). In some embodiments, each L independently comprises an amino acid or a derivative thereof. In some embodiments, each L independently comprises a plurality of amino acids or derivatives thereof. In some embodiments, each L is independently a dipeptide or derivative thereof. In one embodiment, L is one ore more of: alanine, arginine, histidine, lysine, aspartic acid, glutamic acid, serine, threonine, asparganine, glutamine, cysteine, glycine, proline, isoleucine, leucine, methionine, phenylalanine, tryptophan, tyrosine and valine.

In some embodiments, the CDP-therapeutic agent conjugate is a polymer having attached thereto a plurality of L-D moieties of the following formula: 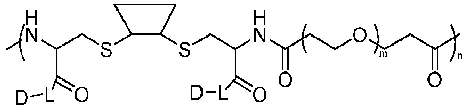 wherein each L is independently a linker or absent and each D is independently a therapeutic agent described herein, a prodrug derivative thereof, or absent and wherein the group 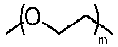 has a Mw of 3400 Da or less and n is at least 4, 5, 6, 7, 8, 9, 10, 11, 12, 13, 14, 15, 16, 17, 18, 19 or 20, provided that the polymer comprises at least one therapeutic agent and in some embodiments, at least two therapeutic agent.

In some embodiments, less than all of the C(=O) moieties are attached to L-D moieties, meaning in some embodiments, at least one L and/or D is absent. In some embodiments, the loading of the L, D and/or L-D moieties on the CDP-therapeutic agent conjugate is from about 1 to about 50% (e.g., from about 1 to about 40%, from about 1 to about 25%, from about 5 to about 20% or from about 5 to about 15%). In some embodiments, each L is independently an amino acid or derivative thereof. In some embodiments, each L is glycine or a derivative thereof.

In some embodiments, one or more of the therapeutic agent in the CDP-therapeutic agent conjugate can be replaced with another therapeutic agent, e.g., another cytotoxic agent or immunomodulator.

In some embodiments, the CDP-therapeutic agent conjugate is a polymer having the following formula: 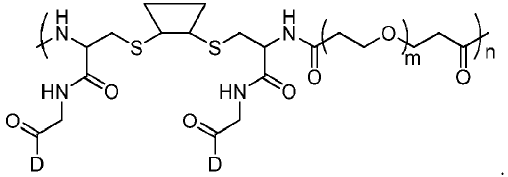 wherein D is independently a therapeutic agent described herein, a prodrug derivative thereof, or absent, the group 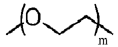 has a Mw of 3400 Da or less and n is at least 4, 5, 6, 7, 8, 9, 10, 11, 12, 13, 14, 15, 16, 17, 18, 19 or 20, provided that the polymer comprises at least one therapeutic agent and in some embodiments, at least two therapeutic agent.

In some embodiments, less than all of the C(=O) moieties are attached to 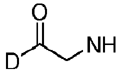 moieties, meaning in some embodiments, 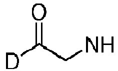 is absent, provided that the polymer comprises at least one therapeutic agent and in some embodiments, at least two therapeutic agent. In some embodiments, the loading of the 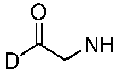 moieties on the CDP-therapeutic agent conjugate is from about 1 to about 50% (e.g., from about 1 to about 40%, from about 1 to about 25%, from about 5 to about 20% or from about 5 to about 15%).

In some embodiments, one or more of the therapeutic agent in the CDP-therapeutic agent conjugate can be replaced with another therapeutic agent, e.g., another cytotoxic agent or immunomodulator.

In some embodiments, the CDP-therapeutic agent conjugate will contain a therapeutic agent and at least one additional therapeutic agent (e.g., a first and second therapeutic agent where the first and second therapeutic agents are different therapeutic agents). For instance, a therapeutic agent described herein and one more different cancer drugs, an immunosuppressant, an antibiotic or an anti-inflammatory agent may be grafted on to the polymer via optional linkers. By selecting different linkers for different drugs, the release of each drug may be attenuated to achieve maximal dosage and efficacy.

### Cyclodextrins

In certain embodiments, the cyclodextrin moieties make up at least about 2%, 5% or 10% by weight, up to 20%, 30%, 50% or even 80% of the CDP by weight. In certain embodiments, the therapeutic agents, or targeting ligands make up at least about 1%, 5%, 10% or 15%, 20%, 25%, 30% or even 35% of the CDP by weight. Number-average molecular weight (Mn) may also vary widely, but generally fall in the range of about 1,000 to about 500,000 daltons, preferably from about 5000 to about 200,000 daltons and, even more preferably, from about 10,000 to about 100,000. Most preferably, Mₙ varies between about 12,000 and 65,000 daltons. In certain other embodiments, Mₙ varies between about 3000 and 150,000 daltons. Within a given sample of a subject polymer, a wide range of molecular weights may be present. For example, molecules within the sample may have molecular weights that differ by a factor of 2, 5, 10, 20, 50, 100, or more, or that differ from the average molecular weight by a factor of 2, 5, 10, 20, 50, 100, or more. Exemplary cyclodextrin moieties include cyclic structures consisting essentially of from 7 to 9 saccharide moieties, such as cyclodextrin and oxidized cyclodextrin. A cyclodextrin moiety optionally comprises a linker moiety that forms a covalent linkage between the cyclic structure and the polymer backbone, preferably having from 1 to 20 atoms in the chain, such as alkyl chains, including dicarboxylic acid derivatives (such as glutaric acid derivatives, succinic acid derivatives, and the like), and heteroalkyl chains, such as oligoethylene glycol chains.

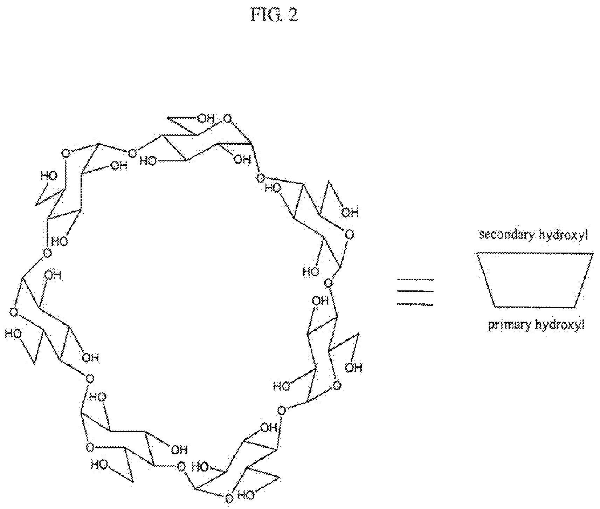

Cyclodextrins are cyclic polysaccharides containing naturally occurring D-(+)-glucopyranose units in an α-(1,4) linkage. The most common cyclodextrins are alpha ((α)-cyclodextrins, beta (β)-cyclodextrins and gamma (y)-cyclodextrins which contain, respectively six, seven, or eight glucopyranose units. Structurally, the cyclic nature of a cyclodextrin forms a torus or donut-like shape having an inner apolar or hydrophobic cavity, the secondary hydroxyl groups situated on one side of the cyclodextrin torus and the primary hydroxyl groups situated on the other. Thus, using (β)-cyclodextrin as an example, a cyclodextrin is often represented schematically as shown in FIG. 2. Attachment on the trapezoid representing the cyclodextrin depicts only whether the moiety is attached through a primary hydroxyl on the cyclodextrin, i.e., by depicting attachment through the base of the trapezoid, or depicting whether the moiety is attached through a secondary hydroxyl on the cyclodextrin, i.e., by depicting attachment through the top of the trapezoid. For example, a trapezoid with two moieties attached at the right and left bottom of the trapezoid does not indicate anything about the relative position of the moieties around the cyclodextrin ring. The attachment of the moieties can be on any glucopyranose in the cyclodextrin ring. Exemplary relative positions of two moieties on a cyclodextrin ring include the following: moieties positioned such that the derivatization on the cyclodextrin is on the A and D glucopyranose moieties, moieties positioned such that the derivatization on the cyclodextrin is on the A and C glucopyranose moieties, moieties positioned such that the derivatization on the cyclodextrin is on the A and F glucopyranose moieties, or moieties positioned such that the derivatization on the cyclodextrin is on the A and E glucopyranose moieties.

The side on which the secondary hydroxyl groups are located has a wider diameter than the side on which the primary hydroxyl groups are located. The present invention contemplates covalent linkages to cyclodextrin moieties on the primary and/or secondary hydroxyl groups. The hydrophobic nature of the cyclodextrin inner cavity allows for host-guest inclusion complexes of a variety of compounds, e.g., adamantane. (Comprehensive Supramolecular Chemistry, Volume 3, J.L. Atwood et al., eds., Pergamon Press (1996); T. Cserhati, Analytical Biochemistry, 225:328-332(1995); Husain et al., Applied Spectroscopy, 46:652-658 (1992); FR 2 665 169). Additional methods for modifying polymers are disclosed in Suh, J. and Noh, Y., Bioorg. Med. Chem. Lett. 1998, 8, 1327-1330.

In certain embodiments, the compounds comprise cyclodextrin moieties and wherein at least one or a plurality of the cyclodextrin moieties of the CDP-therapeutic agent conjugate is oxidized. In certain embodiments, the cyclodextrin moieties of P alternate with linker moieties in the polymer chain.

### Comonomers

In addition to a cyclodextrin moiety, the CDP can also include a comonomer, for example, a comonomer described herein. In some embodiments, a comonomer of the CDP-topoisomerase inhibitor conjugate comprises a moiety selected from the group consisting of: an alkylene chain, polysuccinic anhydride, poly-L-glutamic acid, poly(ethyleneimine), an oligosaccharide, and an amino acid chain. In some embodiments, a CDP-topoisomerase inhibitor conjugate comonomer comprises a polyethylene glycol chain. In some embodiments, a comonomer comprises a moiety selected from: polyglycolic acid and polylactic acid chain. In some embodiments, a comonomer comprises a hydrocarbylene group wherein one or more methylene groups is optionally replaced by a group Y (provided that none of the Y groups are adjacent to each other), wherein each Y, independently for each occurrence, is selected from, substituted or unsubstituted aryl, heteroaryl, cycloalkyl, heterocycloalkyl, or -O-, C(=X) (wherein X is NR₁, O or S), -OC(O)-, -C(=O)O, -NR₁-, -NR₁CO-, -C(O)NR₁-, -S(O)ₙ₋ (wherein n is 0, 1, or 2), -OC(O)-NR₁, -NR₁-C(O)-NR₁-, -NR₁1-C(NR₁)-NR₁-, and -B(OR₁)-; and R₁, independently for each occurrence, represents H or a lower alkyl.

In some embodiments, a comonomer can be and/or can comprise a linker such as a linker described herein.

### Linkers/tethers

The CDPs described herein can include on or more linkers. In some embodiments, a linker can link a therapeutic agent described herein to a CDP. In some embodiments, for example, when referring to a linker that links a therapeutic agent to the CDP, the linker can be referred to as a tether.

In certain embodiments, a plurality of the linker moieties are attached to a therapeutic agent or prodrug thereof and are cleaved under biological conditions.

Described herein are CDP-therapeutic agent conjugates comprising a CDP covalently attached to a therapeutic agent through attachments that are cleaved under biological conditions to release the therapeutic agent. In certain embodiments, a CDP-therapeutic agent conjugate comprises a therapeutic agent covalently attached to a polymer, preferably a biocompatible polymer, through a tether, e.g., a linker, wherein the tether comprises a selectivity-determining moiety and a self-cyclizing moiety which are covalently attached to one another in the tether, e.g., between the polymer and the therapeutic agent.

In some embodiments, such therapeutic agents are covalently attached to CDPs through functional groups comprising one or more heteroatoms, for example, hydroxy, thiol, carboxy, amino, and amide groups. Such groups may be covalently attached to the subject polymers through linker groups as described herein, for example, biocleavable linker groups, and/or through tethers, such as a tether comprising a selectivity-determining moiety and a self-cyclizing moiety which are covalently attached to one another.

In certain embodiments, the CDP-therapeutic agent conjugate comprises a therapeutic agent covalently attached to the CDP through a tether, wherein the tether comprises a self-cyclizing moiety. In some embodiments, the tether further comprises a selectivity-determining moiety. Thus, one aspect of the invention relates to a polymer conjugate comprising a therapeutic agent covalently attached to a polymer, preferably a biocompatible polymer, through a tether, wherein the tether comprises a selectivity-determining moiety and a self-cyclizing moiety which are covalently attached to one another.

In some embodiments, the selectivity-determining moiety is bonded to the self-cyclizing moiety between the self-cyclizing moiety and the CDP.

In certain embodiments, the selectivity-determining moiety is a moiety that promotes selectivity in the cleavage of the bond between the selectivity-determining moiety and the self-cyclizing moiety. Such a moiety may, for example, promote enzymatic cleavage between the selectivity-determining moiety and the self-cyclizing moiety. Alternatively, such a moiety may promote cleavage between the selectivity-determining moiety and the self-cyclizing moiety under acidic conditions or basic conditions.

In certain embodiments, the invention contemplates any combination of the foregoing. Those skilled in the art will recognize that, for example, any therapeutic agent described herein in combination with any linker (e.g., self-cyclizing moiety, any selectivity-determining moiety, and/or any therapeutic agent described herein) are within the scope of the invention.

In certain embodiments, the selectivity-determining moiety is selected such that the bond is cleaved under acidic conditions.

In certain embodiments, where the selectivity-determining moiety is selected such that the bond is cleaved under basic conditions, the selectivity-determining moiety is an aminoalkylcarbonyloxyalkyl moiety. In certain embodiments, the selectivity-determining moiety has a structure

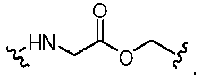

In certain embodiments where the selectivity-determining moiety is selected such that the bond is cleaved enzymatically, it may be selected such that a particular enzyme or class of enzymes cleaves the bond. In certain preferred such embodiments, the selectivity-determining moiety may be selected such that the bond is cleaved by a cathepsin, preferably cathepsin B.

In certain embodiments the selectivity-determining moiety comprises a peptide, preferably a dipeptide, tripeptide, or tetrapeptide. In certain such embodiments, the peptide is a dipeptide is selected from KF and FK, In certain embodiments, the peptide is a tripeptide is selected from GFA, GLA, AVA, GVA, GIA, GVL, GVF, and AVF. In certain embodiments, the peptide is a tetrapeptide selected from GFYA and GFLG, preferably GFLG.

In certain such embodiments, a peptide, such as GFLG, is selected such that the bond between the selectivity-determining moiety and the self-cyclizing moiety is cleaved by a cathepsin, preferably cathepsin B.

In certain embodiments, the selectivity-determining moiety is represented by Formula A: 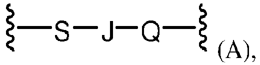 wherein
S a sulfur atom that is part of a disulfide bond;
J is optionally substituted hydrocarbyl; and
Q is O or NR¹³, wherein R¹³ is hydrogen or alkyl.

In certain embodiments, J may be polyethylene glycol, polyethylene, polyester, alkenyl, or alkyl. In certain embodiments, J may represent a hydrocarbylene group comprising one or more methylene groups, wherein one or more methylene groups is optionally replaced by a group Y (provided that none of the Y groups are adjacent to each other), wherein each Y, independently for each occurrence, is selected from, substituted or unsubstituted aryl, heteroaryl, cycloalkyl, heterocycloalkyl, or -O-, C(=X) (wherein X is NR³⁰, O or S), -OC(O)-, -C(=O)O, -NR³⁰-, -NR₁CO-, -C(O)NR³⁰-, -S(O)ₙ- (wherein n is 0, 1, or 2), -OC(O)-NR³⁰, -NR³⁰-C(O)-NR³⁰-, -NR³⁰-C(NR³⁰)-NR³⁰-, and -B(OR³⁰)-; and R³⁰, independently for each occurrence, represents H or a lower alkyl. In certain embodiments, J may be substituted or unsubstituted lower alkylene, such as ethylene. For example, the selectivity-determining moiety may be

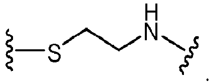

In certain embodiments, the selectivity-determining moiety is represented by Formula B: 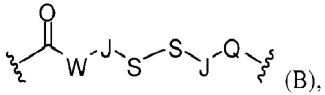 wherein
W is either a direct bond or selected from lower alkyl, NR¹⁴, S, O;
S is sulfur;
J, independently and for each occurrence, is hydrocarbyl or polyethylene glycol;
Q is O or NR¹³, wherein R¹³ is hydrogen or alkyl; and
R¹⁴ is selected from hydrogen and alkyl.

In certain such embodiments, J may be substituted or unsubstituted lower alkyl, such as methylene. In certain such embodiments, J may be an aryl ring. In certain embodiments, the aryl ring is a benzo ring. In certain embodiments W and S are in a 1,2-relationship on the aryl ring. In certain embodiments, the aryl ring may be optionally substituted with alkyl, alkenyl, alkoxy, aralkyl, aryl, heteroaryl, halogen, -CN, azido,-NR^{x}R^{x}, -CO₂OR^{x}, -C(O)-NR^{x}R^{x}, -C(O)-R^{x}, -NR^{x}-C(O)-R^{x}, -NR^{x}SO₂R^{x}, -SR^{x}, -S(O)R^{x},-SO₂R^{x}, -SO₂NR^{x}R^{x}, -(C(R^{x})₂)ₙ-OR^{x}, -(C(R^{x})₂)ₙ-NR^{x}R^{x}, and -(C(R^{x})₂)ₙ-SO₂R^{x}; wherein R^{x} is, independently for each occurrence, H or lower alkyl; and n is, independently for each occurrence, an integer from 0 to 2.

In certain embodiments, the aryl ring is optionally substituted with alkyl, alkenyl, alkoxy, aralkyl, aryl, heteroaryl, halogen, -CN, azido, -NR^{x}R^{x}, -CO₂OR^{x}, -C(O)-NR^{x}R^{x},-C(O)-R^{x}, -NR^{x}-C(O)-R^{x}, -NR^{x}SO₂R^{x}, -SR^{x}, -S(O)R^{x}, -SO₂R^{x}, -SO₂NR^{x}R^{x}, -(C(R^{x})₂)ₙ-OR^{x}, -(C(R^{x})₂)ₙ-NR^{x}R^{x}, and -(C(R^{x})₂)ₙ-SO₂R^{x}; wherein R^{x} is, independently for each occurrence, H or lower alkyl; and n is, independently for each occurrence, an integer from 0 to 2.

In certain embodiments, J, independently and for each occurrence, is polyethylene glycol, polyethylene, polyester, alkenyl, or alkyl.

In certain embodiments, independently and for each occurrence, the linker comprises a hydrocarbylene group comprising one or more methylene groups, wherein one or more methylene groups is optionally replaced by a group Y (provided that none of the Y groups are adjacent to each other), wherein each Y, independently for each occurrence, is selected from, substituted or unsubstituted aryl, heteroaryl, cycloalkyl, heterocycloalkyl, or -O-, C(=X) (wherein X is NR³⁰, O or S), -OC(O)-, -C(=O)O, -NR³⁰-, -NR₁CO-, -C(O)NR³⁰-, -S(O)ₙ- (wherein n is 0, 1, or 2), -OC(O)-NR³⁰, -NR³⁰-C(O)-NR³⁰-, -NR³⁰-C(NR³⁰)-NR³⁰-, and -B(OR³⁰)-; and R³⁰, independently for each occurrence, represents H or a lower alkyl.

In certain embodiments, J, independently and for each occurrence, is substituted or unsubstituted lower alkylene. In certain embodiments, J, independently and for each occurrence, is substituted or unsubstituted ethylene.

In certain embodiments, the selectivity-determining moiety is selected from

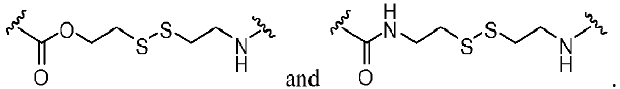

The selectivity-determining moiety may include groups with bonds that are cleavable under certain conditions, such as disulfide groups. In certain embodiments, the selectivity-determining moiety comprises a disulfide-containing moiety, for example, comprising aryl and/or alkyl group(s) bonded to a disulfide group. In certain embodiments, the selectivity-determining moiety has a structure 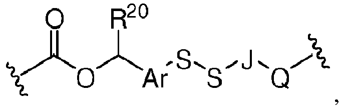 wherein
Ar is a substituted or unsubstituted benzo ring;
J is optionally substituted hydrocarbyl; and
Q is O or NR¹³,
wherein R¹³ is hydrogen or alkyl.

In certain embodiments, Ar is unsubstituted. In certain embodiments, Ar is a 1,2-benzo ring. For example, suitable moieties within Formula B include:

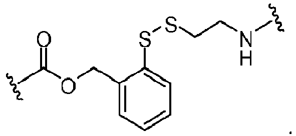

In certain embodiments, the self-cyclizing moiety is selected such that upon cleavage of the bond between the selectivity-determining moiety and the self-cyclizing moiety, cyclization occurs thereby releasing the therapeutic agent. Such a cleavage-cyclization-release cascade may occur sequentially in discrete steps or substantially simultaneously. Thus, in certain embodiments, there may be a temporal and/or spatial difference between the cleavage and the self-cyclization. The rate of the self-cyclization cascade may depend on pH, e.g., a basic pH may increase the rate of self-cyclization after cleavage. Self-cyclization may have a half-life after introduction *in vivo* of 24 hours, 18 hours, 14 hours, 10 hours, 6 hours, 3 hours, 2 hours, 1 hour, 30 minutes, 10 minutes, 5 minutes, or 1 minute.

In certain such embodiments, the self-cyclizing moiety may be selected such that, upon cyclization, a five- or six-membered ring is formed, preferably a five-membered ring. In certain such embodiments, the five- or six-membered ring comprises at least one heteroatom selected from oxygen, nitrogen, or sulfur, preferably at least two, wherein the heteroatoms may be the same or different. In certain such embodiments, the heterocyclic ring contains at least one nitrogen, preferably two. In certain such embodiments, the self-cyclizing moiety cyclizes to form an imidazolidone.

In certain embodiments, the self-cyclizing moiety has a structure 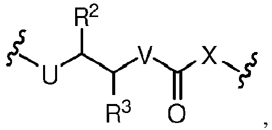 wherein
U is selected from NR¹ and S;
X is selected from O, NR⁵, and S, preferably O or S;
V is selected from O, S and NR⁴, preferably O or NR⁴;
R² and R³ are independently selected from hydrogen, alkyl, and alkoxy; or R² and R³ together with the carbon atoms to which they are attached form a ring; and
R¹, R⁴, and R⁵ are independently selected from hydrogen and alkyl.

In certain embodiments, U is NR¹ and/or V is NR⁴, and R¹ and R⁴ are independently selected from methyl, ethyl, propyl, and isopropyl. In certain embodiments, both R¹ and R⁴ are methyl. On certain embodiments, both R² and R³ are hydrogen. In certain embodiments R² and R³ are independently alkyl, preferably lower alkyl. In certain embodiments, R² and R³ together are -(CH₂)ₙ- wherein n is 3 or 4, thereby forming a cyclopentyl or cyclohexyl ring. In certain embodiments, the nature of R² and R³ may affect the rate of cyclization of the self-cyclizing moiety. In certain such embodiments, it would be expected that the rate of cyclization would be greater when R² and R³ together with the carbon atoms to which they are attached form a ring than the rate when R² and R³ are independently selected from hydrogen, alkyl, and alkoxy. In certain embodiments, U is bonded to the self-cyclizing moiety.

In certain embodiments, the self-cyclizing moiety is selected from 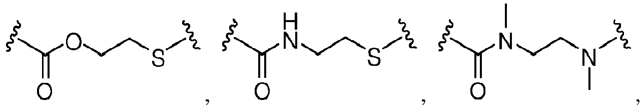 and

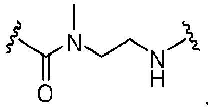

In certain embodiments, the selectivity-determining moiety may connect to the self-cyclizing moiety through carbonyl-heteroatom bonds, e.g., amide, carbamate, carbonate, ester, thioester, and urea bonds.

In certain embodiments, a therapeutic agent is covalently attached to a polymer through a tether, wherein the tether comprises a selectivity-determining moiety and a self-cyclizing moiety which are covalently attached to one another. In certain embodiments, the self-cyclizing moiety is selected such that after cleavage of the bond between the selectivity-determining moiety and the self-cyclizing moiety, cyclization of the self-cyclizing moiety occurs, thereby releasing the therapeutic agent. As an illustration, ABC may be a selectivity-determining moiety, and DEFGH maybe be a self-cyclizing moiety, and ABC may be selected such that enzyme Y cleaves between C and D. Once cleavage of the bond between C and D progresses to a certain point, D will cyclize onto H, thereby releasing therapeutic agent X, or a prodrug thereof.

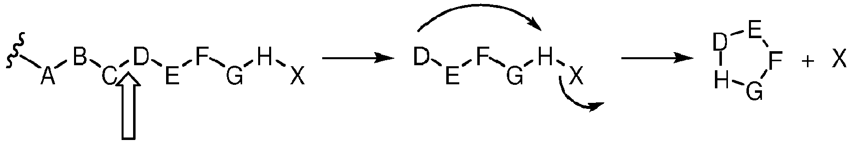

In certain embodiments, the conjugate may further comprise additional intervening components, including, but not limited to another self-cyclizing moiety or a leaving group linker, such as CO₂ or methoxymethyl, that spontaneously dissociates from the remainder of the molecule after cleavage occurs.

In some embodiments, a linker may be and/or comprise an alkylene chain, a polyethylene glycol (PEG) chain, polysuccinic anhydride, poly-L-glutamic acid, poly(ethyleneimine), an oligosaccharide, an amino acid (e.g., glycine or cysteine), an amino acid chain, or any other suitable linkage. In certain embodiments, the linker group itself can be stable under physiological conditions, such as an alkylene chain, or it can be cleavable under physiological conditions, such as by an enzyme (e.g., the linkage contains a peptide sequence that is a substrate for a peptidase), or by hydrolysis (e.g., the linkage contains a hydrolyzable group, such as an ester or thioester). The linker groups can be biologically inactive, such as a PEG, polyglycolic acid, or polylactic acid chain, or can be biologically active, such as an oligo- or polypeptide that, when cleaved from the moieties, binds a receptor, deactivates an enzyme, etc. Various oligomeric linker groups that are biologically compatible and/or bioerodible are known in the art, and the selection of the linkage may influence the ultimate properties of the material, such as whether it is durable when implanted, whether it gradually deforms or shrinks after implantation, or whether it gradually degrades and is absorbed by the body. The linker group may be attached to the moieties by any suitable bond or functional group, including carbon-carbon bonds, esters, ethers, amides, amines, carbonates, carbamates, sulfonamides, etc.

In certain embodiments, the linker group(s) of the present invention represent a hydrocarbylene group wherein one or more methylene groups is optionally replaced by a group Y (provided that none of the Y groups are adjacent to each other), wherein each Y, independently for each occurrence, is selected from, substituted or unsubstituted aryl, heteroaryl, cycloalkyl, heterocycloalkyl, or -O-, C(=X) (wherein X is NR₁, O or S), -OC(O)-, -C(=O)O, -NR₁-, -NR₁CO-, -C(O)NR₁-, -S(O)ₙ- (wherein n is 0, 1, or 2), -OC(O)-NR₁, -NR₁-C(O)-NR₁-, -NR₁-C(NR₁)-NR₁-, and -B(OR₁)-; and R₁, independently for each occurrence, represents H or a lower alkyl.

In certain embodiments, the linker group represents a derivatized or non-derivatized amino acid (e.g., glycine or cysteine). In certain embodiments, linker groups with one or more terminal carboxyl groups may be conjugated to the polymer. In certain embodiments, one or more of these terminal carboxyl groups may be capped by covalently attaching them to a therapeutic agent, a targeting moiety, or a cyclodextrin moiety via an (thio)ester or amide bond. In still other embodiments, linker groups with one or more terminal hydroxyl, thiol, or amino groups may be incorporated into the polymer. In preferred embodiments, one or more of these terminal hydroxyl groups may be capped by covalently attaching them to a therapeutic agent, a targeting moiety, or a cyclodextrin moiety via an (thio)ester, amide, carbonate, carbamate, thiocarbonate, or thiocarbamate bond. In certain embodiments, these (thio)ester, amide, (thio)carbonate or (thio)carbamates bonds may be biohydrolyzable, i.e., capable of being hydrolyzed under biological conditions.

In certain embodiments, a linker group represents a hydrocarbylene group wherein one or more methylene groups is optionally replaced by a group Y (provided that none of the Y groups are adjacent to each other), wherein each Y, independently for each occurrence, is selected from, substituted or unsubstituted aryl, heteroaryl, cycloalkyl, heterocycloalkyl, or -O-, C(=X) (wherein X is NR₁, O or S), -OC(O)-, -C(=O)O, -NR₁-, -NR₁CO-, -C(O)NR₁-, -S(O)ₙ- (wherein n is 0, 1, or 2), -OC(O)-NR₁, -NR₁-C(O)-NR₁-, -NR₁-C(NR₁)-NR₁-, and -B(OR₁)-; and R₁, independently for each occurrence, represents H or a lower alkyl.

In certain embodiments, a linker group, e.g., between a therapeutic agent described herein and the CDP, comprises a self-cyclizing moiety. In certain embodiments, a linker group, e.g., between a therapeutic agent described herein and the CDP, comprises a selectivity-determining moiety.

In certain embodiments as disclosed herein, a linker group, e.g., between a therapeutic agent and the CDP, comprises a self-cyclizing moiety and a selectivity-determining moiety.

In certain embodiments as disclosed herein, the therapeutic agent or targeting ligand is covalently bonded to the linker group via a biohydrolyzable bond (e.g., an ester, amide, carbonate, carbamate, or a phosphate).

In certain embodiments as disclosed herein, the CDP comprises cyclodextrin moieties that alternate with linker moieties in the polymer chain.

In certain embodiments, the linker moieties are attached to therapeutic agents or prodrugs thereof that are cleaved under biological conditions.

In certain embodiments, at least one linker that connects the therapeutic agent or prodrug thereof to the polymer comprises a group represented by the formula 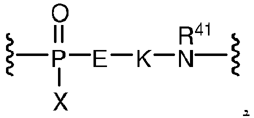 wherein
P is phosphorus;
O is oxygen;
E represents oxygen or NR⁴⁰;
K represents hydrocarbyl;
X is selected from OR⁴² or NR⁴³R⁴⁴; and
R⁴⁰, R⁴¹, R⁴², R⁴³, and R⁴⁴ independently represent hydrogen or optionally substituted alkyl.

In certain embodiments, E is NR⁴⁰ and R⁴⁰ is hydrogen.

In certain embodiments, K is lower alkylene (e.g., ethylene).

In certain embodiments, at least one linker comprises a group selected from

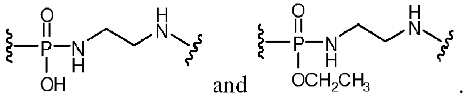

In certain embodiments, X is OR⁴².

In certain embodiments, the linker group comprises an amino acid or peptide, or derivative thereof (e.g., a glycine or cysteine).

In certain embodiments as disclosed herein, the linker is connected to the therapeutic agent through a hydroxyl group. In certain embodiments as disclosed herein, the linker is connected to the therapeutic agent through an amino group.

In certain embodiments, the linker group that connects to the therapeutic agent may comprise a self-cyclizing moiety, or a selectivity-determining moiety, or both. In certain embodiments, the selectivity-determining moiety is a moiety that promotes selectivity in the cleavage of the bond between the selectivity-determining moiety and the self-cyclizing moiety. Such a moiety may, for example, promote enzymatic cleavage between the selectivity-determining moiety and the self-cyclizing moiety. Alternatively, such a moiety may promote cleavage between the selectivity-determining moiety and the self-cyclizing moiety under acidic conditions or basic conditions.

In certain embodiments, any of the linker groups may comprise a self-cyclizing moiety or a selectivity-determining moiety, or both. In certain embodiments, the selectivity-determining moiety may be bonded to the self-cyclizing moiety between the self-cyclizing moiety and the polymer.

In certain embodiments, any of the linker groups may independently be or include an alkyl chain, a polyethylene glycol (PEG) chain, polysuccinic anhydride, poly-L-glutamic acid, poly(ethyleneimine), an oligosaccharide, an amino acid chain, or any other suitable linkage. In certain embodiments, the linker group itself can be stable under physiological conditions, such as an alkyl chain, or it can be cleavable under physiological conditions, such as by an enzyme (e.g., the linkage contains a peptide sequence that is a substrate for a peptidase), or by hydrolysis (e.g., the linkage contains a hydrolyzable group, such as an ester or thioester). The linker groups can be biologically inactive, such as a PEG, polyglycolic acid, or polylactic acid chain, or can be biologically active, such as an oligo- or polypeptide that, when cleaved from the moieties, binds a receptor, deactivates an enzyme, etc. Various oligomeric linker groups that are biologically compatible and/or bioerodible are known in the art, and the selection of the linkage may influence the ultimate properties of the material, such as whether it is durable when implanted, whether it gradually deforms or shrinks after implantation, or whether it gradually degrades and is absorbed by the body. The linker group may be attached to the moieties by any suitable bond or functional group, including carbon-carbon bonds, esters, ethers, amides, amines, carbonates, carbamates, sulfonamides, etc.

In certain embodiments, any of the linker groups may independently be an alkyl group wherein one or more methylene groups is optionally replaced by a group Y (provided that none of the Y groups are adjacent to each other), wherein each Y, independently for each occurrence, is selected from aryl, heteroaryl, carbocyclyl, heterocyclyl, or -O-, C(=X) (wherein X is NR¹, O or S), -OC(O)-, -C(=O)O-, -NR¹-, -NR¹CO-, -C(O)NR¹-, -S(O)ₙ- (wherein n is 0, 1, or 2), -OC(O)-NR¹-, -NR¹-C(O)-NR¹-, -NR¹-C(NR¹)-NR¹-, and -B(OR¹)-; and R¹, independently for each occurrence, is H or lower alkyl.

In certain embodiments, the present invention contemplates a CDP, wherein a plurality of therapeutic agents are covalently attached to the polymer through attachments that are cleaved under biological conditions to release the therapeutic agents as discussed above, wherein administration of the polymer to a subject results in release of the therapeutic agent over a period of at least 2, 3, 5, 6, 8, 10, 15, 20, 24, 36, 48 or even 72 hours.

In some embodiments, the conjugation of the therapeutic agent to the CDP improves the aqueous solubility of the therapeutic agent and hence the bioavailability. Accordingly, in one embodiment of the invention, the therapeutic agent has a log P >0.4, >0.6, >0.8, >1, >2, >3, >4, or even >5.

The CDP-therapeutic agent conjugate of the present invention preferably has a molecular weight in the range of 10,000 to 500,000; 30,000 to 200,000; or even 70,000 to 150,000 Da.

In certain embodiments, the present invention contemplates attenuating the rate of release of the therapeutic agent by introducing various tether and/or linking groups between the therapeutic agent and the polymer. Thus, in certain embodiments, the CDP-therapeutic agent conjugates of the present invention are compositions for controlled delivery of the therapeutic agent.

### Characteristics of CDP-therapeutic agent conjugates, particles or compositions

In some embodiments, the CDP and/or CDP-therapeutic agent conjugate, particle or composition as described herein have polydispersities less than about 3, or even less than about 2 (e.g., 1.5, 1.25, or less).

One embodiment of the present invention provides an improved delivery of certain therapeutic agents by covalently attaching one or more therapeutic agents to a CDP. Such conjugation can improve the aqueous solubility and hence the bioavailability of the therapeutic agent.

In certain embodiments as disclosed herein, the CDP-therapeutic agent conjugate has a number average (Mₙ) molecular weight between 1,000-500,000 Da, or between 5,000-200,000 Da, or between 10,000-100,000 Da. One method to determine molecular weight is by gel permeation chromatography ("GPC"), e.g., mixed bed columns, CH₂Cl₂ or HFIP (hexafluoroisopropanol) solvent, light scattering detector, and off-line dn/dc. Other methods are known in the art.

In certain embodiments as disclosed herein, the CDP-therapeutic agent conjugate, particle or composition is biodegradable or bioerodable.

In certain embodiments as disclosed herein, the therapeutic agent makes up at least 3% (e.g., at least about 5%) by weight of the CDP-therapeutic agent conjugate or particle. In certain embodiments, the therapeutic agent makes up at least 20% by weight of the CDP-therapeutic agent conjugate. In certain embodiments, the therapeutic agent makees up at least 5%, 10%, 15%, or at least 20% by weight of the CDP-therapeutic agent conjugate or particle.

In one embodiment, the CDP-therapeutic agent conjugate forms a particle, e.g., a nanoparticle. The particle can comprise multiple CDP-therapeutic agent conjugates, e.g., a plurality of CDP-therapeutic agent conjugates, e.g., CDP-therapeutic agent conjugates having the same therapeutic agents or different therapeutic agents. The nanoparticle ranges in size from 10 to 300 nm in diameter, e.g., 15 to 280, 30 to 250, 40 to 200, 20 to 150, 30 to 100, 20 to 80, 30 to 70, 40 to 60 or 40 to 50 nm diameter. In one embodiment, the particle is 50 to 60 nm, 20 to 60 nm, 30 to 60 nm, 35 to 55 nm, 35 to 50 nm or 35 to 45 nm in diameter.

In one embodiment, the CDP-therapeutic agent conjugate forms an inclusion complex. In one embodiment, the CDP-therapeutic agent conjugate containing the inclusion complex forms a particle, e.g., a nanoparticle. The nanoparticle ranges in size from 10 to 300 nm in diameter, e.g., 15 to 280, 30 to 250, 40 to 200, 20 to 150, 30 to 100, 20 to 80, 30 to 70, 40 to 60 or 40 to 50 nm diameter. In one embodiment, the particle is 50 to 60 nm, 20 to 60 nm, 30 to 60 nm, 35 to 55 nm, 35 to 50 nm or 35 to 45 nm in diameter.

In one embodiment, the surface charge of the molecule is neutral, or slightly negative. In some embodiments, the zeta potential of the particle surface is from about - 80 mV to about 50 mV, about -20 mV to about 20 mV, about -20 mV to about -10 mV, or about -10 mV to about 0.

CDP-therapeutic agent conjugates, particles and compositions of the present invention may be useful to improve solubility and/or stability of the therapeutic agent, reduce drug-drug interactions, reduce interactions with blood elements including plasma proteins, reduce or eliminate immunogenicity, protect the therapeutic agent from metabolism, modulate drug-release kinetics, improve circulation time, improve therapeutic agent half-life (e.g., in the serum, or in selected tissues, such as tumors), attenuate toxicity, improve efficacy, normalize therapeutic agent metabolism across subjects of different species, ethnicities, and/or races, and/or provide for targeted delivery into specific cells or tissues.

In other embodiments, the CDP-therapeutic agent conjugate, particle or composition may be a flexible or flowable material. When the CDP used is itself flowable, the CDP composition of the invention, even when viscous, need not include a biocompatible solvent to be flowable, although trace or residual amounts of biocompatible solvents may still be present.

While it is possible that the biodegradable polymer or the biologically active agent may be dissolved in a small quantity of a solvent that is non-toxic to more efficiently produce an amorphous, monolithic distribution or a fine dispersion of the biologically active agent in the flexible or flowable composition, it is an advantage of the invention that, in a preferred embodiment, no solvent is needed to form a flowable composition. Moreover, the use of solvents is preferably avoided because, once a polymer composition containing solvent is placed totally or partially within the body, the solvent dissipates or diffuses away from the polymer and must be processed and eliminated by the body, placing an extra burden on the body's clearance ability at a time when the illness (and/or other treatments for the illness) may have already deleteriously affected it.

However, when a solvent is used to facilitate mixing or to maintain the flowability of the CDP-therapeutic agent conjugate, particle or composition, it should be non-toxic, otherwise biocompatible, and should be used in relatively small amounts. Solvents that are toxic should not be used in any material to be placed even partially within a living body. Such a solvent also must not cause substantial tissue irritation or necrosis at the site of administration.

Examples of suitable biocompatible solvents, when used, include N-methyl-2-pyrrolidone, 2-pyrrolidone, ethanol, propylene glycol, acetone, methyl acetate, ethyl acetate, methyl ethyl ketone, dimethylformamide, dimethylsulfoxide, tetrahydrofuran, caprolactam, oleic acid, or 1-dodecylazacylcoheptanone. Preferred solvents include N-methylpyrrolidone, 2-pyrrolidone, dimethylsulfoxide, and acetone because of their solvating ability and their biocompatibility.

In certain embodiments, the CDP-therapeutic agent conjugates, particles and compositions are soluble in one or more common organic solvents for ease of fabrication and processing. Common organic solvents include such solvents as chloroform, dichloromethane, dichloroethane, 2-butanone, butyl acetate, ethyl butyrate, acetone, ethyl acetate, dimethylacetamide, N-methylpyrrolidone, dimethylformamide, and dimethylsulfoxide.

In certain embodiments, the CDP-therapeutic agent conjugates, particles and compositions described herein, upon contact with body fluids, undergo gradual degradation. The life of a biodegradable polymer in vivo depends upon, among other things, its molecular weight, crystallinity, biostability, and the degree of crosslinking. In general, the greater the molecular weight, the higher the degree of crystallinity, and the greater the biostability, the slower biodegradation will be.

If a subject composition is formulated with a therapeutic agent or other material, release of the therapeutic agent or other material for a sustained or extended period as compared to the release from an isotonic saline solution generally results. Such release profile may result in prolonged delivery (over, say 1 to about 2,000 hours, or alternatively about 2 to about 800 hours) of effective amounts (e.g., about 0.0001 mg/kg/hour to about 10 mg/kg/hour, e.g., 0.001 mg/kg/hour, 0.01 mg/kg/hour, 0.1 mg/kg/hour, 1.0 mg/kg/hour) of the therapeutic agent or any other material associated with the polymer.

A variety of factors may affect the desired rate of hydrolysis of CDP-therapeutic agent conjugates, particles and compositions, the desired softness and flexibility of the resulting solid matrix, rate and extent of bioactive material release. Some of such factors include the selection/identity of the various subunits, the enantiomeric or diastereomeric purity of the monomeric subunits, homogeneity of subunits found in the polymer, and the length of the polymer. For instance, the present invention contemplates heteropolymers with varying linkages, and/or the inclusion of other monomeric elements in the polymer, in order to control, for example, the rate of biodegradation of the matrix.

To illustrate further, a wide range of degradation rates may be obtained by adjusting the hydrophobicities of the backbones or side chains of the polymers while still maintaining sufficient biodegradability for the use intended for any such polymer. Such a result may be achieved by varying the various functional groups of the polymer. For example, the combination of a hydrophobic backbone and a hydrophilic linkage produces heterogeneous degradation because cleavage is encouraged whereas water penetration is resisted.

One protocol generally accepted in the field that may be used to determine the release rate of a therapeutic agent or other material loaded in the CDP-therapeutic agent conjugates, particles or compositions of the present invention involves degradation of any such matrix in a 0.1 M PBS solution (pH 7.4) at 37 °C, an assay known in the art. For purposes of the present invention, the term "PBS protocol" is used herein to refer to such protocol.

In certain instances, the release rates of different CDP-therapeutic agent conjugates, particles and compositions of the present invention may be compared by subjecting them to such a protocol. In certain instances, it may be necessary to process polymeric systems in the same fashion to allow direct and relatively accurate comparisons of different systems to be made. For example, the present invention teaches several different methods of formulating the CDP-therapeutic agent conjugates, particles and compositions. Such comparisons may indicate that any one CDP-therapeutic agent conjugate, particle or composition releases incorporated material at a rate from about 2 or less to about 1000 or more times faster than another polymeric system.

Alternatively, a comparison may reveal a rate difference of about 3, 5, 7, 10, 25, 50, 100, 250, 500 or 750 times. Even higher rate differences are contemplated by the present invention and release rate protocols.

In certain embodiments, when formulated in a certain manner, the release rate for CDP-therapeutic agent conjugates, particles and compositions of the present invention may present as mono- or bi-phasic.

Release of any material incorporated into the polymer matrix, which is often provided as a microsphere, may be characterized in certain instances by an initial increased release rate, which may release from about 5 to about 50% or more of any incorporated material, or alternatively about 10, 15, 20, 25, 30 or 40%, followed by a release rate of lesser magnitude.

The release rate of any incorporated material may also be characterized by the amount of such material released per day per mg of polymer matrix. For example, in certain embodiments, the release rate may vary from about 1 ng or less of any incorporated material per day per mg of polymeric system to about 500 or more ng/day/mg. Alternatively, the release rate may be about 0.05, 0.5, 5, 10, 25, 50, 75, 100, 125, 150, 175, 200, 250, 300, 350, 400, 450, or 500 ng/day/mg. In still other embodiments, the release rate of any incorporated material may be 10,000 ng/day/mg, or even higher. In certain instances, materials incorporated and characterized by such release rate protocols may include therapeutic agents, fillers, and other substances.

In another aspect, the rate of release of any material from any CDP-therapeutic agent conjugate, particle or composition of the present invention may be presented as the half-life of such material in the matrix.

In addition to the embodiment involving protocols for in vitro determination of release rates, in vivo protocols, whereby in certain instances release rates for polymeric systems may be determined in vivo, are also contemplated by the present invention. Other assays useful for determining the release of any material from the polymers of the present system are known in the art.

### Physical Structures of the CDP-therapeutic agent conjugates, particles and compositions

The CDP-therapeutic agent conjugates, particles and compositions may be formed in a variety of shapes. For example, in certain embodiments, CDP-therapeutic agent conjugates may be presented in the form of microparticles or nanoparticles. Microspheres typically comprise a biodegradable polymer matrix incorporating a drug. Microspheres can be formed by a wide variety of techniques known to those of skill in the art. Examples of microsphere forming techniques include, but are not limited to, (a) phase separation by emulsification and subsequent organic solvent evaporation (including complex emulsion methods such as oil in water emulsions, water in oil emulsions and water-oil-water emulsions); (b) coacervation-phase separation; (c) melt dispersion; (d) interfacial deposition; (e) in situ polymerization; (f) spray drying and spray congealing; (g) air suspension coating; and (h) pan and spray coating. These methods, as well as properties and characteristics of microspheres are disclosed in, for example, U.S. Patent No. 4,438,253; U.S. Patent No. 4,652,441; U.S. Patent No. 5,100,669; U.S. Patent No. 5,330,768; U.S. Patent No. 4,526,938; U.S. Patent No. 5,889,110; U.S. Patent No. 6,034,175; and European Patent 0258780, the entire disclosures of which are incorporated by reference herein in their entireties.

To prepare microspheres, several methods can be employed depending upon the desired application of the delivery vehicles. Suitable methods include, but are not limited to, spray drying, freeze drying, air drying, vacuum drying, fluidized-bed drying, milling, co-precipitation and critical fluid extraction. In the case of spray drying, freeze drying, air drying, vacuum drying, fluidized-bed drying and critical fluid extraction; the components (stabilizing polyol, bioactive material, buffers, etc.) are first dissolved or suspended in aqueous conditions. In the case of milling, the components are mixed in the dried form and milled by any method known in the art. In the case of co-precipitation, the components are mixed in organic conditions and processed as described below. Spray drying can be used to load the stabilizing polyol with the bioactive material. The components are mixed under aqueous conditions and dried using precision nozzles to produce extremely uniform droplets in a drying chamber. Suitable spray drying machines include, but are not limited to, Buchi, NIRO, APV and Lab-plant spray driers used according to the manufacturer's instructions.

The shape of microparticles and nanoparticles may be determined by scanning electron microscopy. Spherically shaped nanoparticles are used in certain embodiments, for circulation through the bloodstream. If desired, the particles may be fabricated using known techniques into other shapes that are more useful for a specific application.

In addition to intracellular delivery of a therapeutic agent, it also possible that particles of the CDP-therapeutic agent conjugates, such as microparticles or nanoparticles, may undergo endocytosis, thereby obtaining access to the cell. The frequency of such an endocytosis process will likely depend on the size of any particle. In one embodiment, the surface charge of the particle is neutral, or slightly negative. In some embodiments, the zeta potential of the particle surface is from about -80 mV to about 50 mV, e.g., from about -40 mV to about 30 mV, e.g., from about -20 mV to about 30 mV.

### Exemplary CDP-Therapeutic Agent Conjugates

Described herein are cyclodextrin containing polymer ("CDP")-therapeutic agent conjugates, wherein one or more therapeutic agents are covalently attached to the CDP (e.g., either directly or through a linker). These cyclodextrin containing polymer ("CDP")-therapeutic agent conjugates are useful as carriers for delivery of a therapeutic agent and may improve therapeutic agent stability and solubility when used *in vivo.* The CDP-therapeutic agent conjugate can include a therapeutic agent such that the CDP-therapeutic agent conjugate can be used to treat an autoimmune disease or cancer. In an embodiment, the therapeutic agent in the CDP-therapeutic agent conjugate is a cytotoxic agent or immunomodulator. In an embodiment, the CDP-therapeutic agent conjugate is a CDP-cytotoxic agent conjugate, e.g., CDP-topoisomerase inhibitor conjugate, e.g., a CDP-topoisomerase inhibitor I conjugate (e.g., a CDP-camptothecin conjugate, CDP-irinotecan conjugate, CDP-SN-38 conjugate, CDP-topotecan conjugate, CDP-lamellarin D conjugate, a CDP-lurotecan conjugate, particle or composition, a CDP-exatecan conjugate, particle or composition, a CDP-diflomotecan conjugate, particle or composition, and CDP-topoisomerase I inhibitor conjugates which include derivatives of camptothecin, irinotecan, SN-38, lamellarin D, lurotecan, exatecan, and diflomotecan), a CDP-topoisomerase II inhibitor conjugate (e.g., a CDP-etoposide conjugate, CDP-tenoposide conjugate, CDP-amsacrine conjugate and CDP-topoisomerase II inhibitor conjugates which include derivatives of etoposide, tenoposide, and amsacrine), a CDP-anti-metabolic agent conjugate (e.g., a CDP-antifolate conjugate (e.g., a CDP-pemetrexed conjugate, a CDP-floxuridine conjugate, a CDP-raltitrexed conjugate) or a CDP-pyrimidine analog conjugate (e.g., a CDP-capecitabine conjugate, a CDP-cytarabine conjugate, a CDP-gemcitabine conjugate, a CDP-5FU conjugate)), a CDP-alkylating agent conjugate, a CDP-anthracycline conjugate, a CDP-anti-tumor antibiotic conjugate (e.g., a CDP-HSP90 inhibitor conjugate, e.g., a CDP-geldanamycin conjugate, a CDP-tanespimycin conjugate or a CDP-alvespimycin conjugate), a CDP-platinum based agent conjugate (e.g., a CDP-cisplatin conjugate, a CDP-carboplatin conjugate, a CDP-oxaliplatin conjugate), a CDP-microtubule inhibitor conjugate, a CDP-kinase inhibitor conjugate (e.g., a CDP-seronine/threonine kinase inhibitor conjugate, e.g., a CDP-mTOR inhibitor conjugate, e.g., a CDP-rapamycin conjugate) or a CDP-proteasome inhibitor conjugate.

In one embodiment, the cytotoxic agents include topoisomerase inhibitors, e.g., a topoisomerase I inhibitor (e.g., camptothecin, irinotecan, SN-38, topotecan, lamellarin D, lurotecan, exatecan, diflomotecan, and derivatives thereof), a topoisomerase II inhibitor (e.g., etoposide, tenoposide, amsacrine and derivatives thereof).

In an embodiment, the topoisomerase inhibitor in the CDP-topoisomerase inhibitor conjugate, particle or composition is camptothecin or a camptothecin derivative. For example, camptothecin derivatives can have the following structure: 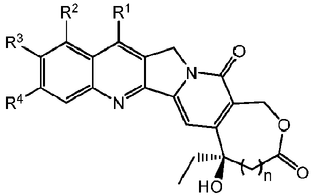 wherein,
R¹ is H, OH, optionally substituted alkyl (e.g., optionally substituted with NR^{a}₂ or ORₐ, or SiR^{a}₃), or SiR^{a}₃; or R¹ and R² may be taken together to form an optionally substituted 5- to 8-membered ring (e.g., optionally substituted with NR^{a}₂ or OR^{a});
R² is H, OH, NH₂, halo, nitro, optionally substituted alkyl (e.g., optionally substituted with NR^{a}₂ or OR^{a}, NR^{a}₂, OC(=O)NR^{a}₂, or OC(=O)OR^{a});
R³ is H, OH, NH₂, halo, nitro, NR^{a}₂, OC(=O)NR^{a}₂, or OC(=O)OR^{a};
R⁴ is H, OH, NH₂, halo, CN, or NR^{a}₂; or R³ and R⁴ taken together with the atoms to which they are attached form a 5- or 6-membered ring (e.g. forming a ring including-OCH₂O- or -OCH₂CH₂O-);
each R^{a} is independently H or alkyl; or two R^{a}s, taken together with the atom to which they are attached, form a 4- to 8-membered ring (e.g., optionally containing an O or NR^{b});
R^{b} is H or optionally substituted alkyl (e.g., optionally substituted with OR^{c} or NR^{c}₂);
R^{c} is H or alkyl; or, two R^{c}s, taken together with the atom to which they are attached, form a 4- to 8-membered ring; and
n = 0 or 1.

In one embodiment, R¹, R², R³ and R⁴ of the camptothecin derivative are each H, and n is 0.

In one embodiment, R¹, R², R³ and R⁴ of the camptothecin derivative are each H, and n is 1.

In some embodiments, the camptothecin or camptothecin derivative is the compound as provided below.

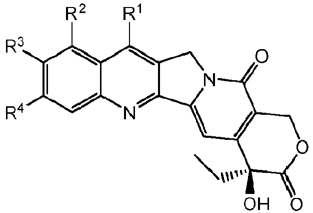

In one embodiment, R¹ of the camptothecin derivative is H, R² is -CH₂N(CH₃)₂, R³ is -OH, R⁴ is H; and n is 0.

In one embodiment, R¹ of the camptothecin derivative is -CH₂CH₃, R² is H, R³ is:

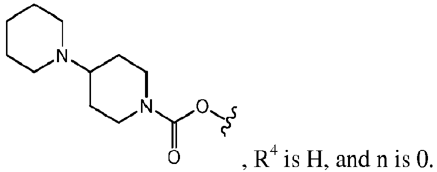

In one embodiment, R¹ of the camptothecin derivative is -CH₂CH₃, R² is H, R³ is -OH, R⁴ is H, and n is 0.

In one embodiment, R¹ of the camptothecin derivative is *tert*-butyldimethylsilyl, R² is H, R³ is -OH and R⁴ is H, and n is 0.

In one embodiment, R¹ of the camptothecin derivative is *tert*-butyldimethylsilyl, R² is hydrogen, R³ is -OH and R⁴ is hydrogen, and n is 1.

In one embodiment, R¹ of the camptothecin derivative is *tert*-butyldimethylsilyl, R², R³ and R⁴ are each H, and n is 0.

In one embodiment, R¹ of the camptothecin derivative is *tert*-butyldimethylsilyl, R², R³ and R⁴ are each H, and n is 1.

In one embodiment, R¹ of the camptothecin derivative is -CH₂CH₂Si(CH₃)₃ and R², R³ and R⁴ are each H.

In one embodiment, R¹ and R² of the camptothecin derivative are taken together with the carbons to which they are attached to form an optionally substituted ring. In one embodiment, R¹ and R² of the camptothecin derivative are taken together with the carbons to which they are attached to form a substituted 6-membered ring. In one embodiment, the camptothecin derivative has the following formula: 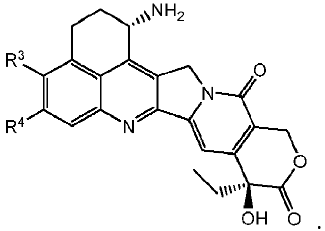 In one embodiment, R³ is methyl and R⁴ is fluoro.

In one embodiment, R³ and R⁴ are taken together with the carbons to which they are attached to form an optionally substituted ring. In one embodiment, R³ and R⁴ are taken together with the carbons to which they are attached to form a 6-membered heterocyclic ring. In one embodiment, the camptothecin derivative has the following formula:

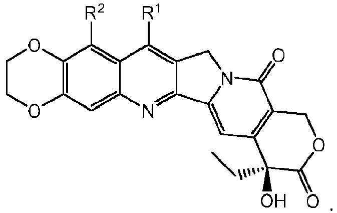

In one embodiment, R¹ is: 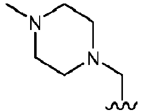 and R² is hydrogen.

In one embodiment, the camptothecin derivative has the following formula: 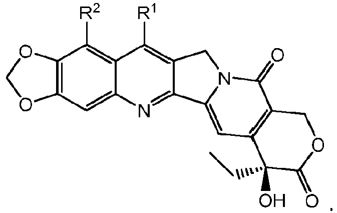 In one embodiment, R¹ is: 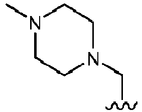 and R² is hydrogen.

In one embodiment, R¹ is: 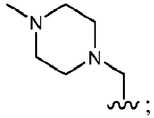 R² is H, R³ is methyl, R⁴ is chloro; and n is 1.

In one embodiment, R¹ is -CH=NOC(CH₃)₃, R², R³ and R⁴ are each H, and n is 0.

In one embodiment, R¹ is -CH₂CH₂NHCH(CH₃)₂, R², R³ and R⁴ are each H; and n is 0.

In one embodiment, R¹ and R² are H, R³ and R⁴ are fluoro, and n is 1.

In one embodiment, each of R¹, R³, and R⁴ is H, R² is NH₂, and n is 0.

In one embodiment, each of R¹, R³, and R⁴ is H, R² is NO₂, and n is 0.

In one embodiment, the CDP-topoisomerase I inhibitor conjugate is a CDP-camptothecin conjugate, e.g., as shown below, 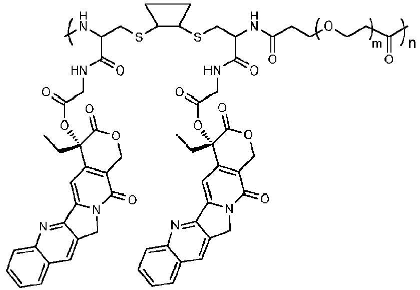 wherein 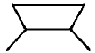 represents a cyclodextrin; n is an integer from 1 to 100 (e.g., n is an integer from 4 to 80, from 4 to 50, from 4 to 30 or from 4 to 20, or n is 4, 5, 6, 7, 8, 9, 10, 11, 12, 13, 14, 15, 16, 17, 18, 19 or 20); m is an integer from 1 to 1000 (e.g., m is an integer from 1 to 200, from 1 to 100, from 1 to 80, from 2 to 80, from 5 to 70, from 10 to 50, or from 20 to 40). In some embodiments, the CDP-topoisomerase I inhibitor conjugate, e.g., the CDP-camptothecin conjugate, does not have complete loading, e.g., one or more binding sites, e.g., cysteine residues, are not bound to a topoisomerase I inhibitor, e.g., a camptothecin moiety, e.g., a glycine-linkage bound camptothecin, e.g., the CDP-camptothecin conjugate comprises one or more subunits having the formulae provided below 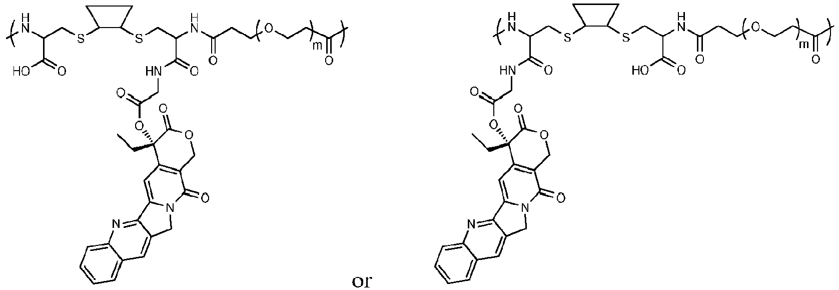 wherein 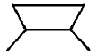 represents a cyclodextrin; m is an integer from 1 to 1000 (e.g., m is an integer from 1 to 200, from 1 to 100, from 1 to 80, from 2 to 80, from 5 to 70, from 10 to 50, or from 20 to 40). In some embodiments, the CDP-topoisomerase I inhibitor conjugate, particle or composition e.g., the CDP-camptothecin conjugate, particle or composition, comprises a mixture of fully-loaded and partially-loaded CDP-topoisomerase I inhibitor subunits within the conjugates, e.g., CDP-camptothecin conjugates.

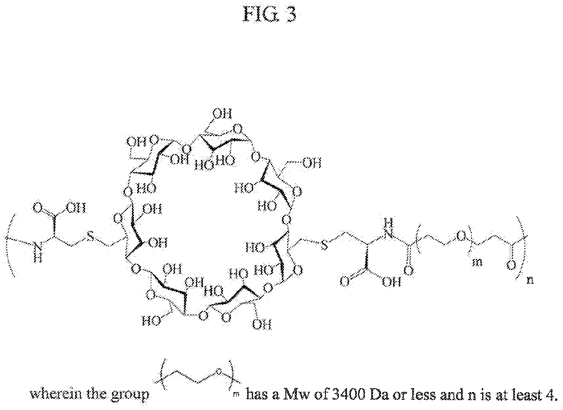

In one embodiment, the CDP is the cyclodextrin-containing polymer shown below (as well as in FIG. 3): 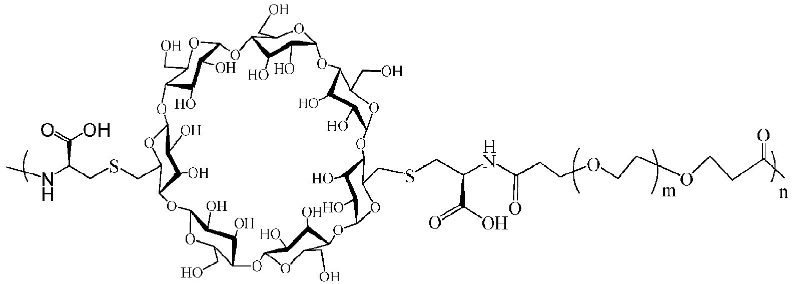 wherein the group 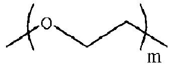 has a Mw of 3400 Da or less and n is at least 4, 5, 6, 7, 8, 9, 10, 11, 12, 13, 14, 15, 16, 17, 18, 19 or 20. Note that the taxane is conjugated to the CDP through the carboxylic acid moieties of the polymer as provided above. Full loading of the taxane onto the CDP is not required. In some embodiments, at least one, e.g., at least 2, 3, 4, 5, 6 or 7, of the carboxylic acid moieties remains unreacted with the taxane after conjugation (e.g., a plurality of the carboxylic acid moieties remain unreacted).

In one embodiment, the CDP-topoisomerase I inhibitor conjugate comprises a subunit of 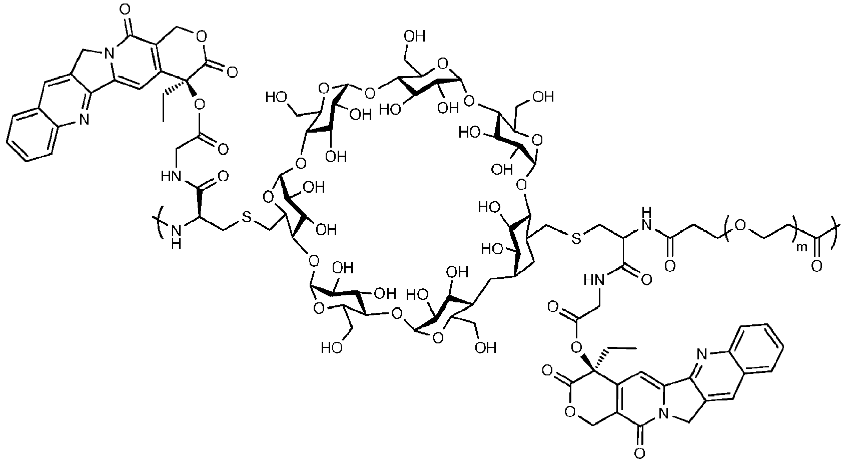 wherein m is an integer from 1 to 1000 (e.g., m is an integer from 1 to 200, from 1 to 100, from 1 to 80, from 2 to 80, from 5 to 70, from 10 to 50, or from 20 to 40).

In some embodiments, the CDP-topoisomerase inhibitor conjugate is a polymer having the following formula: 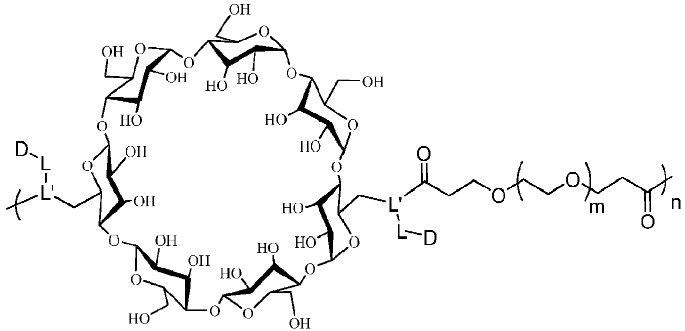 wherein L and L' independently for each occurence, is a linker, a bond, or -OH and D, independently for each occurence, is a topoisomerase inhibitor such as camptothecin ("CPT"), a camptothecin derivative or absent, and wherein the group 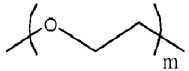 has a Mw of 3400 Da or less and n is at least 4, 5, 6, 7, 8, 9, 10, 11, 12, 13, 14, 15, 16, 17, 18, 19 or 20, provided that at least one D is CPT or a camptothecin derivative. In some embodiments, at least 2 D moieties are CPT and/or a camptothecin derivative.

In some embodiments, each L', for each occurence, is a cysteine. In some embodiments, the cysteine is attached to the cyclodextrin via a sulfide bond. In some embodiments, the cysteine is attached to the PEG containing portion of the polymer via an amide bond.

In some embodiments, the L is a linker (e.g., an amino acid such as glycine). In some embodiments, L is absent. In some embodiments, D-L together form

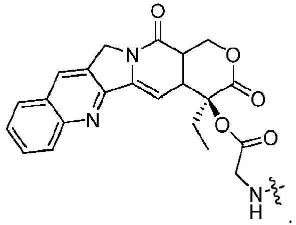

In some embodiments, a plurality of D moieties are absent and at the same position on the polymer, the corresponding L is -OH.

In some embodiments, less than all of the C(=O) moieties of the cysteine residue in the polymer backbone are attached to 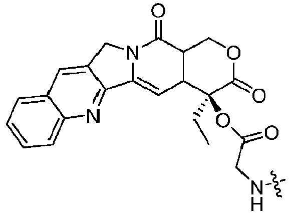 moieties, meaning in some embodiments, 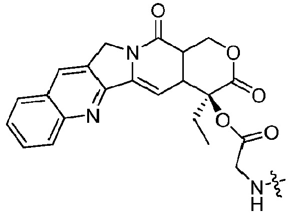 is absent in one or more positions of the polymer backbone, provided that the polymer comprises at least one 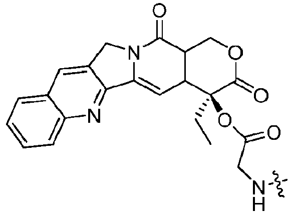 and in some embodiments, at least two 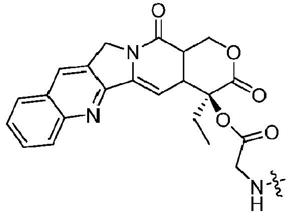 moieties. In some embodiments, the loading of the 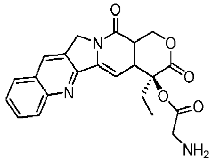 moieties on the CDP-topoisomerase inhibitor conjugate is from about 1 to about 50% (e.g., from about 1 to about 40%, from about 1 to about 25%, from about 5 to about 20% or from about 5 to about 15%, e.g., from about 6 to about 10%). In some embodiments, the loading of 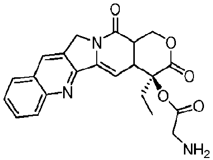 on the CDP is from about 6% to about 10% by weight of the total polymer.

In some embodiments, the CDP-topoisomerase inhibitor conjugate is a polymer having the following formula: 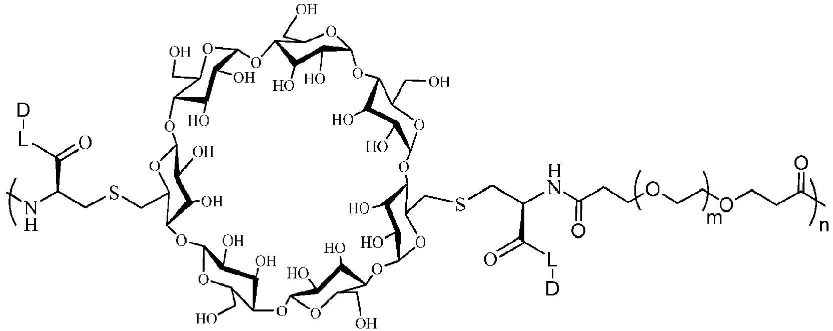 wherein L, independently for each occurrence, is a linker, a bond, or -OH and D, independently for each occurrence, is camptothecin ("CPT"), a camptothecin derivative or absent, and wherein the group 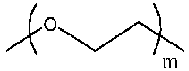 has a Mw of 3400 Da or less and n is at least 4, 5, 6, 7, 8, 9, 10, 11, 12, 13, 14, 15, 16, 17, 18, 19 or 20, provided that at least one D is CPT or a camptothecin derivative. In some embodiments, at least 2 D moieties are CPT and/or a camptothecin derivative.

In some embodiments, the CDP-camptothecin conjugate is as shown below, which is referred to herein as "CRLX101." In some embodiments, a CDP-camptothecin conjugate may have one or more binding sites, e.g., a cysteine residue, not bound to the CDP, e.g., as described below:

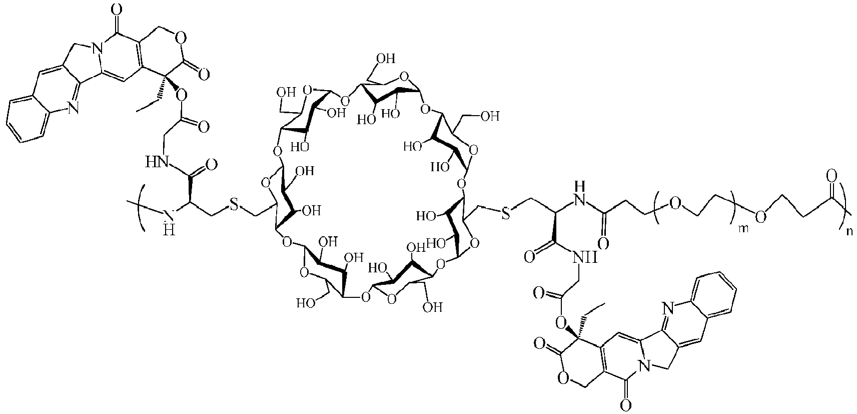

In the above structure:
m = about 77 or the molecular weight of the PEG moiety is from about 3060 to about 3740 (e.g., about 3400) Da;
n = is from about 10 to about 18 (e.g., about 14);
the molecular weight of the polymer backbone (i.e., the polymer minus the CPT-gly, which results in the cysteine moieties having a free -C(O)OH) is from about 48 to about 8500 Da;
   the polydispersity of the polymer backbone is less than about 2.2; and
the loading of the CPT onto the polymer backbone is from about 6 to about 13% by weight, wherein 13% is theoretical maximum, meaning, in some instances, one or more of the cysteine residues has a free -C(O)OH (i.e., it lacks the CPT-gly).

In some embodiments, the polydispersity of the PEG component in the above structure is less than about 1.1.

In some embodiments, a CDP-camptothecin conjugate described herein has a terminal amine and/or a terminal carboxylic acid.

In an embodiment, the topoisomerase inhibitor of the CDP-topoisomerase inhibitor conjugate, particle, or composition is a topoisomerase II inhibitor, e.g., etoposide (Toposar® or VePesid®), teniposide (Vumon®), amsacrine and derivatives thereof.

In an embodiment, the therapeutic agent in the CDP-therapeutic agent conjugate is a cytotoxic agent such as an anti-metabolic agent. In some embodiments, the anti-metabolic agent in the CDP-anti-metabolic agent conjugate, particle or composition is an anti-metabolic agent including, without limitation, folic acid antagonists (also referred to herein as antifolates), pyrimidine analogs, purine analogs and adenosine deaminase inhibitors): methotrexate (Rheumatrex®, Trexall®), 5-fluorouracil (Adrucil®, Efudex®, or Fluoroplex®), floxuridine (FUDF®), cytarabine (Cytosar-U® or Tarabine PFS), 6-mercaptopurine (Puri-Nethol®)), 6-thioguanine (Thioguanine Tabloid®), fludarabine phosphate (Fludara®), pentostatin (Nipent®), pemetrexed (Alimta®), raltitrexed (Tomudex®), cladribine (Leustatin®), clofarabine (Clofarex® or Clolar®), mercaptopurine (Puri-Nethol®), capecitabine (Xeloda®), nelarabine (Arranon®), azacitidine (Vidaza®) and gemcitabine (Gemzar®). Preferred anti-metabolites include, e.g., 5-fluorouracil (5FU) (Adrucil®, Efudex®, or Fluoroplex®), floxuridine (FUDF®), capecitabine (Xeloda®), pemetrexed (Alimta®), raltitrexed (Tomudex®) and gemcitabine (Gemzar®).

In an embodiment, the anti-metabolic agent in the CDP-anti-metabolic agent conjugate, particle or composition is an antifolate, e.g., a CDP-antifolate conjugate, particle or composition. In preferred embodiments, the antifolate in the CDP-antifolate conjugate, particle or composition is pemetrexed or a pemetrexed derivative. For example, pemetrexed has the following structure:

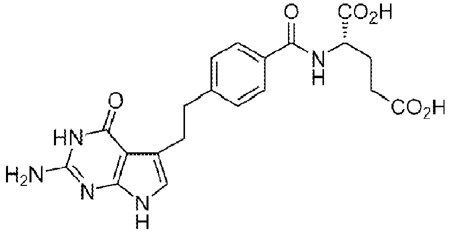

In one embodiment, the CDP-antifolate conjugate is a CDP-pemetrexed conjugate, e.g., 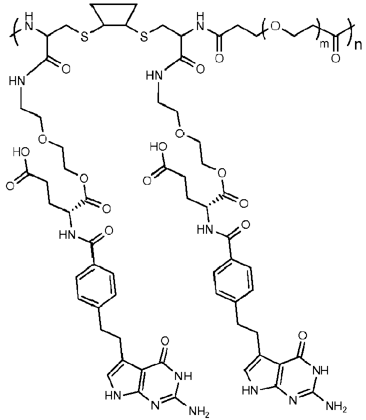 wherein 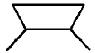 represents a cyclodextrin; n is an integer from 1 to 100 (e.g., n is an integer from 4 to 80, from 4 to 50, from 4 to 30 or from 4 to 20, or n is 4, 5, 6, 7, 8, 9, 10, 11, 12, 13, 14, 15, 16, 17, 18, 19 or 20); m is an integer from 1 to 1000 (e.g., m is an integer from 1 to 200, from 1 to 100, from 1 to 80, from 2 to 80, from 5 to 70, from 10 to 50, or from 20 to 40). In some embodiments, the CDP-antifolate conjugate, e.g., the CDP-pemetrexed conjugate, does not have complete loading, e.g., one or more binding sites, e.g., cysteine residues, are not bound to an antifolate, e.g., a pemetrexed moiety, e.g., an amine-linkage bound pemetrexed, e.g., the CDP-pemetrexed conjugate comprises one or more subunits having the formulae provided below: 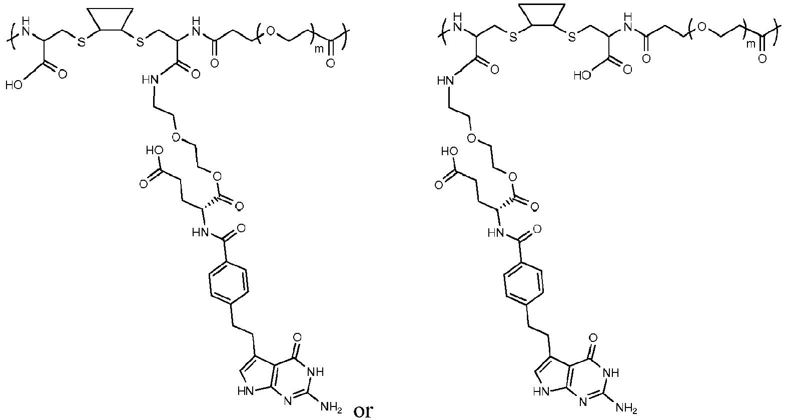 wherein 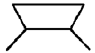 represents a cyclodextrin and m is an integer from 1 to 1000 (e.g., m is an integer from 1 to 200, from 1 to 100, from 1 to 80, from 2 to 80, from 5 to 70, from 10 to 50, or from 20 to 40). In some embodiments, the CDP-antifolate conjugate, particle or composition e.g., the CDP-pemetrexed conjugate, particle or composition, comprises a mixture of fully-loaded and partially-loaded CDP-antifolate analog conjugates, e.g., CDP-pemetrexed conjugates.

In one embodiment, the CDP-pemetrexed conjugate comprises a subunit of 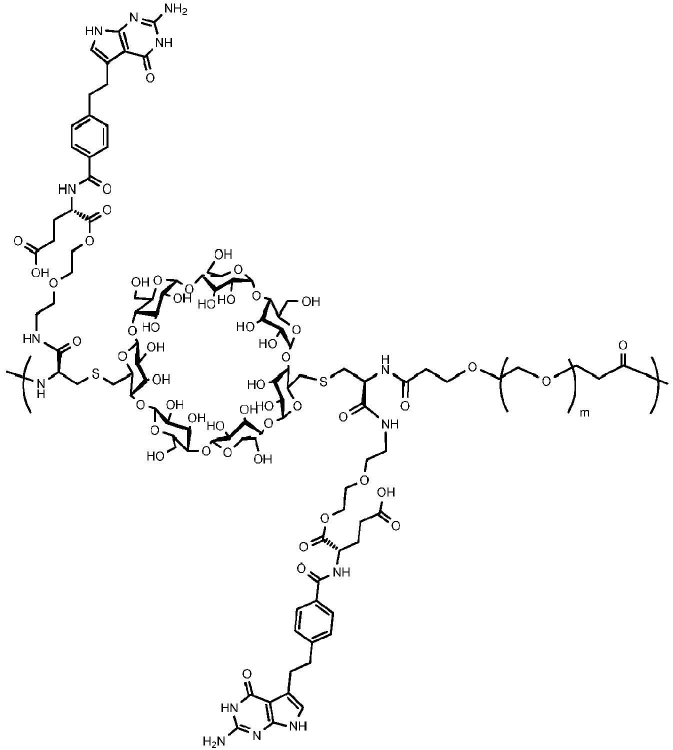 wherein m is an integer from 1 to 1000 (e.g., m is an integer from 1 to 200, from 1 to 100, from 1 to 80, from 2 to 80, from 5 to 70, from 10 to 50, or from 20 to 40).

In one embodiment, the CDP-antifolate conjugate is a CDP-pemetrexed conjugate, e.g., 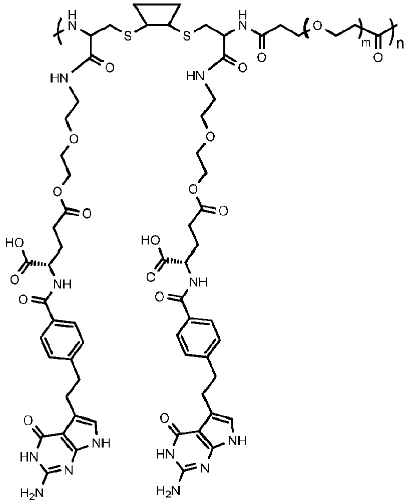 wherein 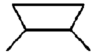 represents a cyclodextrin; n is an integer from 1 to 100 (e.g., n is an integer from 4 to 80, from 4 to 50, from 4 to 30 or from 4 to 20, or n is 4, 5, 6, 7, 8, 9, 10, 11, 12, 13, 14, 15, 16, 17, 18, 19 or 20); m is an integer from 1 to 1000 (e.g., m is an integer from 1 to 200, from 1 to 100, from 1 to 80, from 2 to 80, from 5 to 70, from 10 to 50, or from 20 to 40). In some embodiments, the CDP-antifolate conjugate, e.g., the CDP-pemetrexed conjugate, does not have complete loading, e.g., one or more binding sites, e.g., cysteine residues, are not bound to an antifolate, e.g., a pemetrexed moiety, e.g., an amine-linkage bound pemetrexed, e.g., the CDP-pemetrexed conjugate comprises one or more subunits having the formulae provided below: 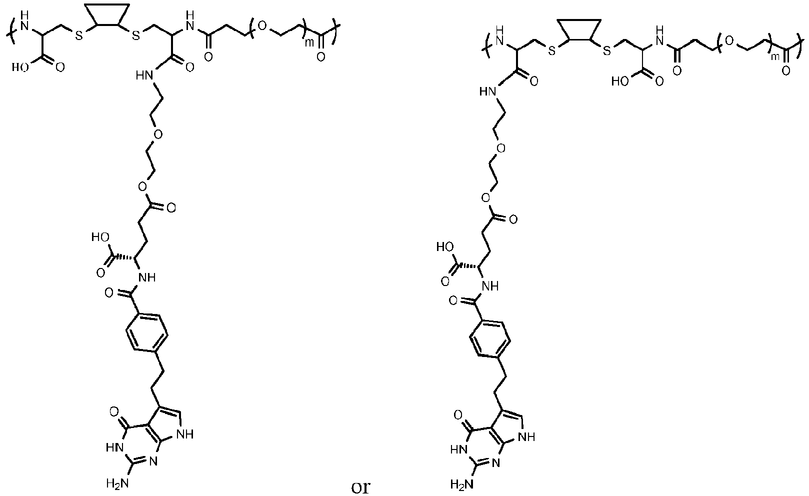 wherein 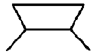 represents a cyclodextrin and m is an integer from 1 to 1000 (e.g., m is an integer from 1 to 200, from 1 to 100, from 1 to 80, from 2 to 80, from 5 to 70, from 10 to 50, or from 20 to 40). In some embodiments, the CDP-antifolate conjugate, particle or composition e.g., the CDP-pemetrexed conjugate, particle or composition, comprises a mixture of fully-loaded and partially-loaded CDP-antifolate analog conjugates, e.g., CDP-pemetrexed conjugates.

In one embodiment, the CDP-pemetrexed conjugate comprises a subunit of 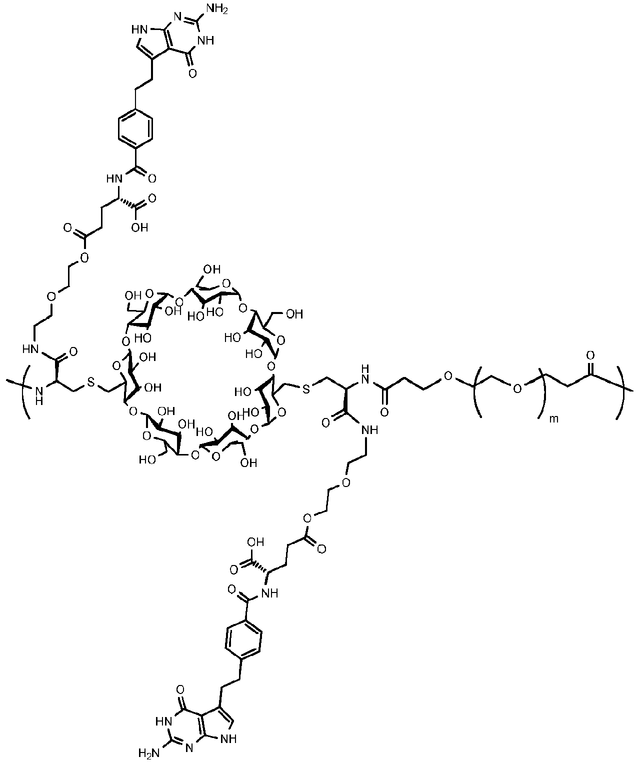 wherein m is an integer from 1 to 1000 (e.g., m is an integer from 1 to 200, from 1 to 100, from 1 to 80, from 2 to 80, from 5 to 70, from 10 to 50, or from 20 to 40).CDP-pemetrexed conjugates can be made using many different combinations of components described herein. For example, various combinations of cyclodextrins (e.g., beta-cyclodextrin), comonomers (e.g., PEG containing comonomers), linkers linking the cyclodextrins and comonomers, and/or linkers tethering the pemetrexed to the CDP are described herein.

In one embodiment, the CDP-pemetrexed conjugate forms a particle, e.g., a nanoparticle. The compositions described herein comprise a CDP-pemetrexed conjugate or a plurality of CDP-pemetrexed conjugates. The composition can also comprise a particle or a plurality of particles described herein.

In one embodiment, the CDP-pemetrexed conjugate forms a particle, e.g., a nanoparticle. The nanoparticle ranges in size from 10 to 300 nm in diameter, e.g., 15 to 280, 30 to 250, 40 to 200, 20 to 150, 30 to 100, 20 to 80, 30 to 70, 40 to 60 or 40 to 50 nm diameter. In one embodiment, the particle is 50 to 60 nm, 20 to 60 nm, 30 to 60 nm, 35 to 55 nm, 35 to 50 nm or 35 to 45 nm in diameter.

In one embodiment, the surface charge of the molecule is neutral, or slightly negative. In some embodiments, the zeta potential of the particle surface is from about - 80 mV to about 50 mV, about -20 mV to about 20 mV, about -20 mV to about -10 mV, or about -10 mV to about 0.

In some embodiments, the CDP-pemetrexed conjugate is a polymer having the formula: 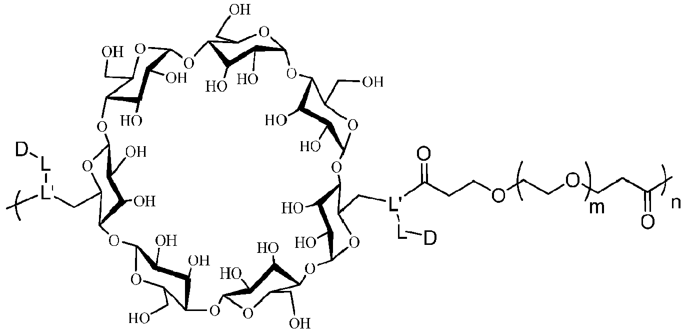 wherein L and L' independently for each occurence, is a linker, a bond, or -OH and D, independently for each occurence, is a pemetrexed, a pemetrexed derivative or absent, and wherein the group 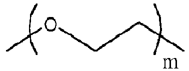 has a Mw of 3400 Da or less and n is at least 4, 5, 6, 7, 8, 9, 10, 11, 12, 13, 14, 15, 16, 17, 18, 19 or 20, provided that at least one D is pemetrexed or a pemetrexed derivative. In some embodiments, at least 2 D moieties are pemetrexed and/or a pemetrexed derivative.

In some embodiments, each L', for each occurence, is a cysteine. In some embodiments, the cysteine is attached to the cyclodextrin via a sulfide bond. In some embodiments, the cysteine is attached to the PEG containing portion of the polymer via an amide bond.

In some embodiments, the L is a linker (e.g., an amine linkage). In some embodiments, L is absent. In some embodiments, D-L together form

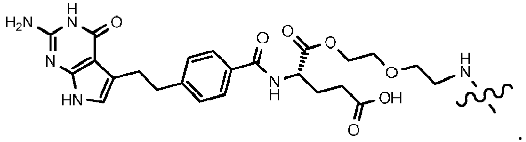

In some embodiments, a plurality of D moieties are absent and at the same position on the polymer, the corresponding L is -OH.

In some embodiments, less than all of the C(=O) moieties of the cysteine residue in the polymer backbone are attached to 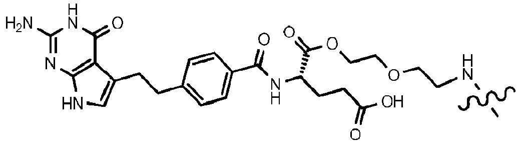 moieties, meaning in some embodiments, 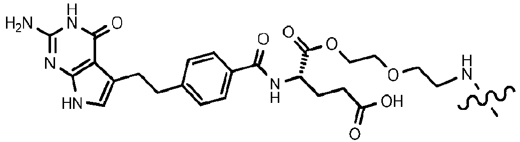 is absent in one or more positions of the polymer backbone, provided that the polymer comprises at least one 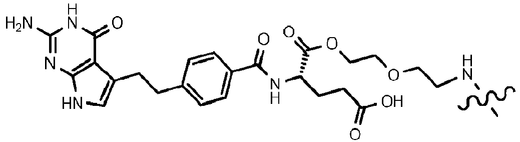 and in some embodiments, at least two 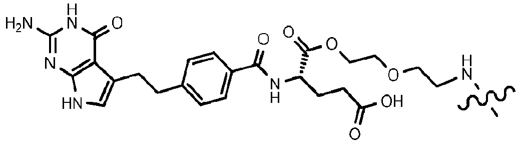 moieties. In some embodiments, the loading of the 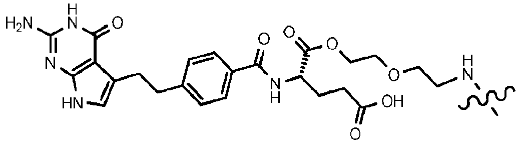 moieties on the CDP-pemetrexed conjugate is from about 1 to about 50% (e.g., from about 1 to about 40%, from about 1 to about 25%, from about 5 to about 20% or from about 5 to about 15%, e.g., from about 6 to about 10%). In some embodiments, the loading of 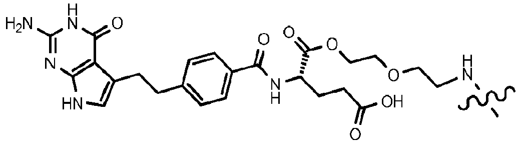 on the CDP is from about 6% to about 10% by weight of the total polymer.

In some embodiments, the L is a linker (e.g., an amine linkage). In some embodiments, L is absent. In some embodiments, D-L together form

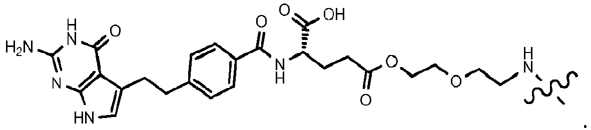

In some embodiments, a plurality of D moieties are absent and at the same position on the polymer, the corresponding L is -OH.

In some embodiments, less than all of the C(=O) moieties of the cysteine residue in the polymer backbone are attached to 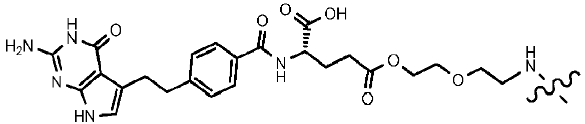 moieties, meaning in some embodiments, 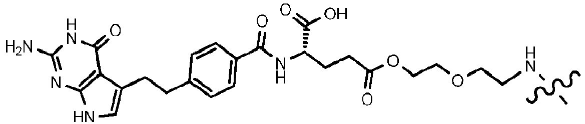 is absent in one or more positions of the polymer backbone, provided that the polymer comprises at least one 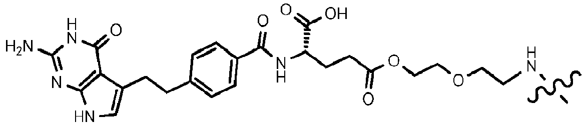 and in some embodiments, at least two 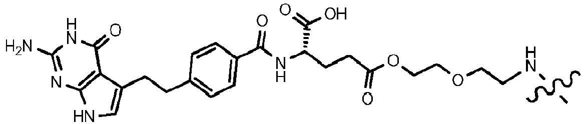 moieties. In some embodiments, the loading of the 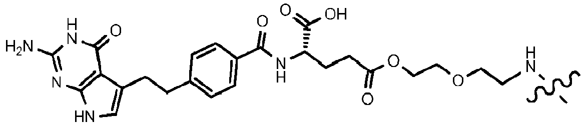 moieties on the CDP-pemetrexed conjugate is from about 1 to about 50% (e.g., from about 1 to about 40%, from about 1 to about 25%, from about 5 to about 20% or from about 5 to about 15%, e.g., from about 6 to about 10%). In some embodiments, the loading of 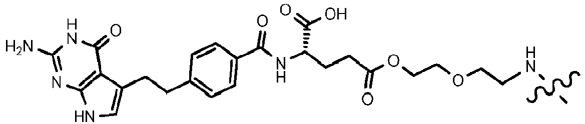 on the CDP is from about 6% to about 10% by weight of the total polymer.

In some embodiments, the CDP-pemetrexed conjugate is a polymer of the formula: 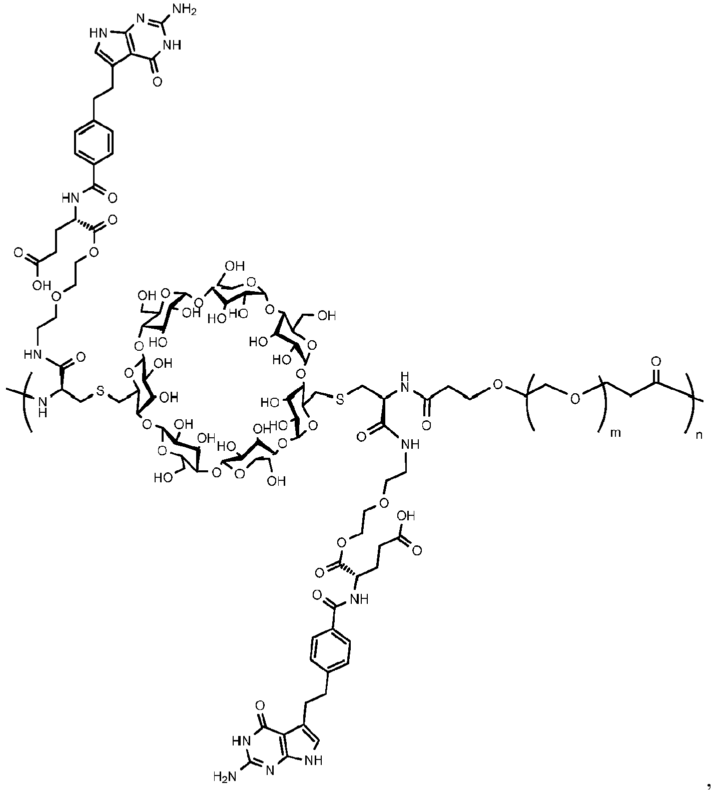 wherein m and n are as defined above, and wherein less than all of the C(=O) sites of the cysteine of the polymer backbone are occupied as indicated above with the pemetrexed-ester, but instead are free acids, meaning, the theoretical loading of the polymer is less than 100%.In some embodiments, the CDP-pemetrexed conjugate is a polymer of the formula: 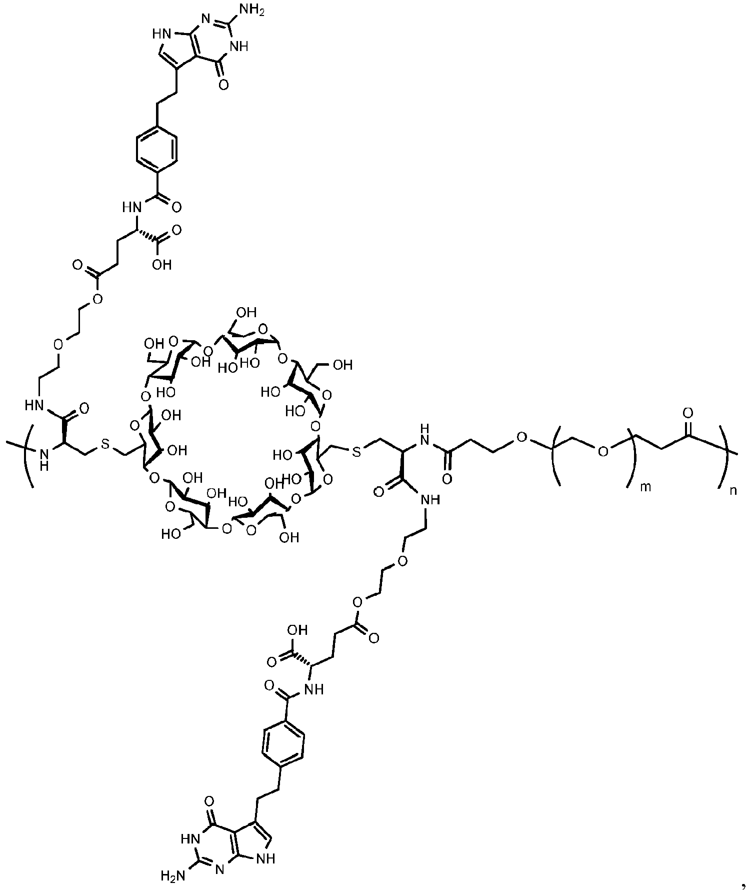 wherein m and n are as defined above, and wherein less than all of the C(=O) sites of the cysteine of the polymer backbone are occupied as indicated above with the pemetrexed-ester, but instead are free acids, meaning, the theoretical loading of the polymer is less than 100%.

In an embodiment, the anti-metabolic agent in the CDP-anti-metabolic agent conjugate, particle or composition is pyrimidine analog, e.g., a CDP-pyrimidine analog conjugate, particle or composition. In preferred embodiments, the pyrimidine analog agent in the CDP-pyrimidine analog conjugate, particle or composition comprises gemcitabine or a gemcitabine derivative. For example, gemcitabine can have the following structure:

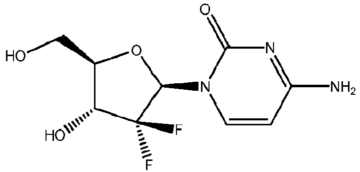

In one embodiment, the CDP-pyrimidine analog conjugate is a CDP-gemcitabine conjugate, e.g., 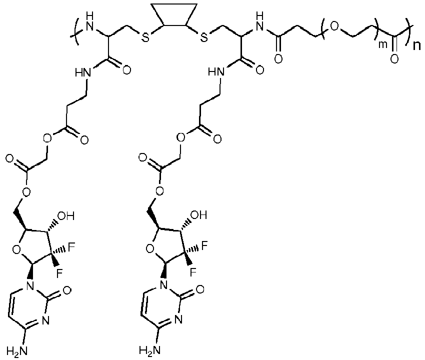 wherein 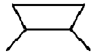 represents a cyclodextrin; n is an integer from 1 to 100 (e.g., n is an integer from 4 to 80, from 4 to 50, from 4 to 30 or from 4 to 20, or n is 4, 5, 6, 7, 8, 9, 10, 11, 12, 13, 14, 15, 16, 17, 18, 19 or 20); m is an integer from 1 to 1000 (e.g., m is an integer from 1 to 200, from 1 to 100, from 1 to 80, from 2 to 80, from 5 to 70, from 10 to 50, or from 20 to 40). In some embodiments, the CDP-pyrimidine analog conjugate, e.g., the CDP-gemcitabine conjugate, does not have complete loading, e.g., one or more binding sites, e.g., cysteine residues, are not bound to a pyrimidine analog, e.g., a gemcitabine moiety, e.g., an ester-linkage bound gemcitabine, e.g., the CDP-gemcitabine conjugate comprises one or more subunits having the formulae provided below: 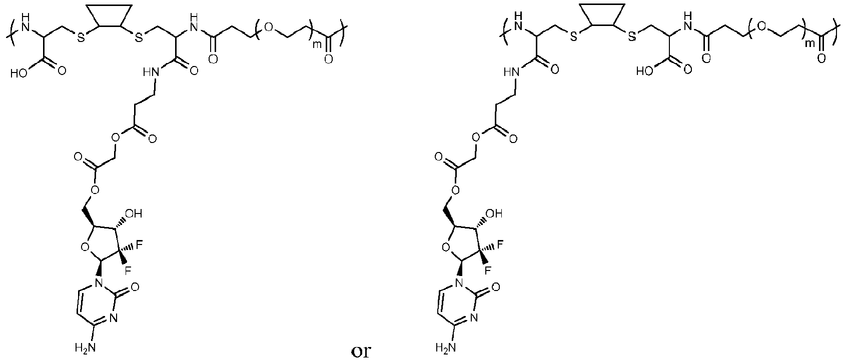 wherein 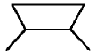 represents a cyclodextrin and m is an integer from 1 to 1000 (e.g., m is an integer from 1 to 200, from 1 to 100, from 1 to 80, from 2 to 80, from 5 to 70, from 10 to 50, or from 20 to 40). In some embodiments, the CDP-pyrimidine analog conjugate, particle or composition e.g., the CDP-gemcitabine conjugate, particle or composition, comprises a mixture of fully-loaded and partially-loaded CDP-pyrimidine analog conjugates, e.g., CDP-gemcitabine conjugates.

In one embodiment, the CDP-pyrimidine analog conjugate comprises a subunit of 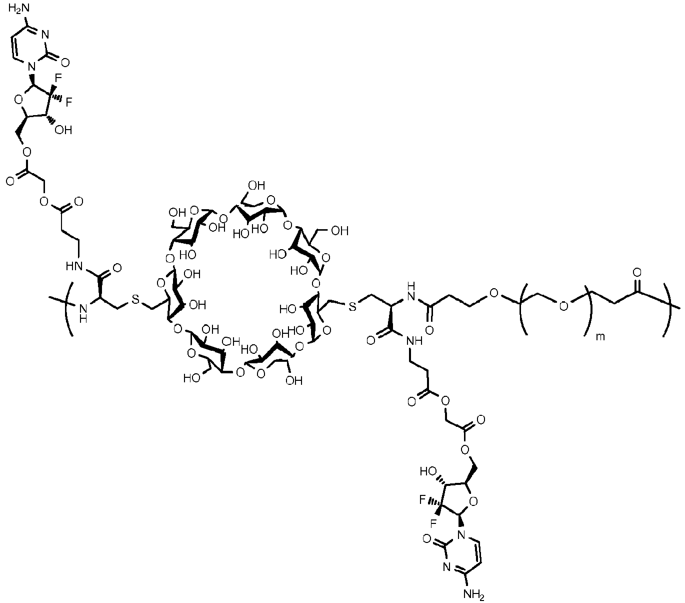 wherein m is an integer from 1 to 1000 (e.g., m is an integer from 1 to 200, from 1 to 100, from 1 to 80, from 2 to 80, from 5 to 70, from 10 to 50, or from 20 to 40).

In one embodiment, the CDP-pyrimidine analog conjugate is a CDP-gemcitabine conjugate, e.g., 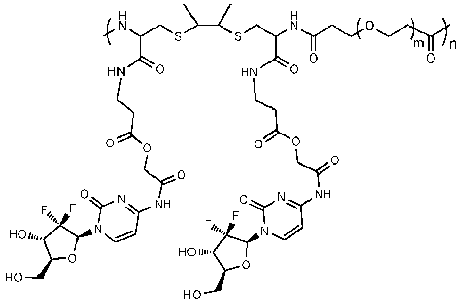 wherein 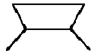 represents a cyclodextrin; n is an integer from 1 to 100 (e.g., n is an integer from 4 to 80, from 4 to 50, from 4 to 30 or from 4 to 20, or n is 4, 5, 6, 7, 8, 9, 10, 11, 12, 13, 14, 15, 16, 17, 18, 19 or 20); m is an integer from 1 to 1000 (e.g., m is an integer from 1 to 200, from 1 to 100, from 1 to 80, from 2 to 80, from 5 to 70, from 10 to 50, or from 20 to 40). In some embodiments, the CDP-pyrimidine analog conjugate, e.g., the CDP-gemcitabine conjugate, does not have complete loading, e.g., one or more binding sites, e.g., cysteine residues, are not bound to a pyrimidine analog, e.g., a gemcitabine moiety, e.g., an ester-linkage bound gemcitabine, e.g., the CDP-gemcitabine conjugate comprises one or more subunits having the formulae provided below: 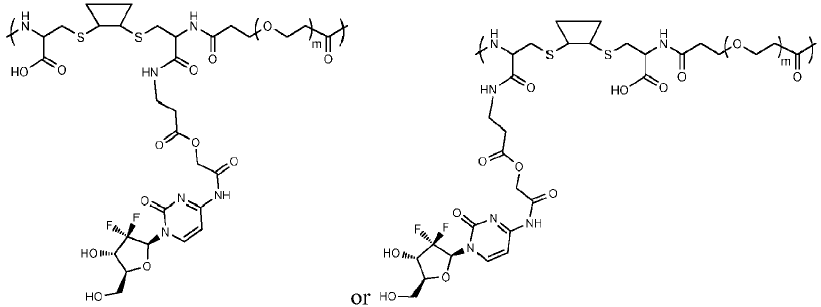 wherein 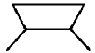 represents a cyclodextrin and m is an integer from 1 to 1000 (e.g., m is an integer from 1 to 200, from 1 to 100, from 1 to 80, from 2 to 80, from 5 to 70, from 10 to 50, or from 20 to 40). In some embodiments, the CDP-pyrimidine analog conjugate, particle or composition e.g., the CDP-gemcitabine conjugate, particle or composition, comprises a mixture of fully-loaded and partially-loaded CDP-pyrimidine analog conjugates, e.g., CDP-gemcitabine conjugates.

In one embodiment, the CDP-pyrimidine analog conjugate comprises a subunit of 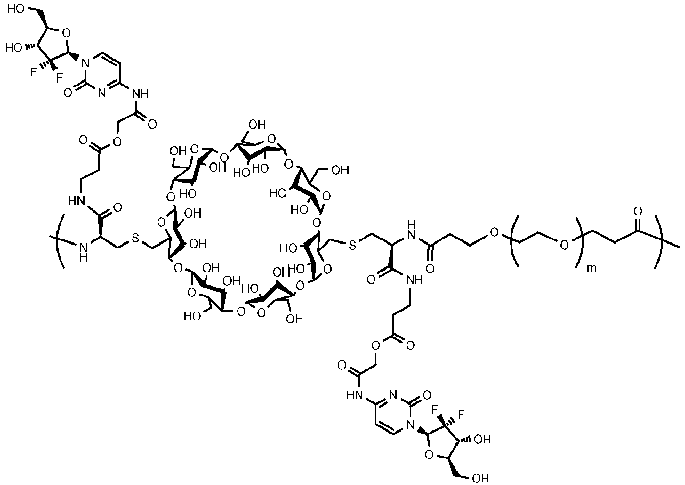 wherein m is an integer from 1 to 1000 (e.g., m is an integer from 1 to 200, from 1 to 100, from 1 to 80, from 2 to 80, from 5 to 70, from 10 to 50, or from 20 to 40).

In one embodiment, the CDP-pyrimidine analog conjugate is a CDP-gemcitabine derivative conjugate, e.g., 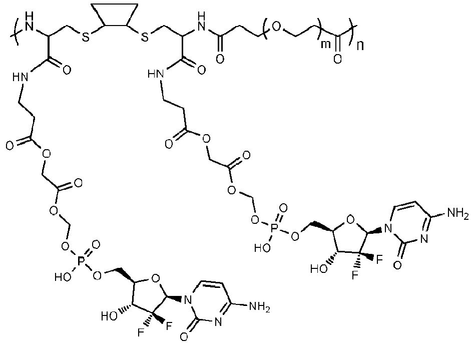 wherein 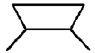 represents a cyclodextrin; n is an integer from 1 to 100 (e.g., n is an integer from 4 to 80, from 4 to 50, from 4 to 30 or from 4 to 20, or n is 4, 5, 6, 7, 8, 9, 10, 11, 12, 13, 14, 15, 16, 17, 18, 19 or 20); m is an integer from 1 to 1000 (e.g., m is an integer from 1 to 200, from 1 to 100, from 1 to 80, from 2 to 80, from 5 to 70, from 10 to 50, or from 20 to 40). In some embodiments, the CDP-pyrimidine analog conjugate, e.g., the CDP-gemcitabine derivative conjugate, does not have complete loading, e.g., one or more binding sites, e.g., cysteine residues, are not bound to a pyrimidine analog, e.g., a gemcitabine derivative, e.g., an ester-linkage bound gemcitabine derivative, e.g., the CDP-gemcitabine derivative conjugate comprises one or more subunits having the formulae provided below: 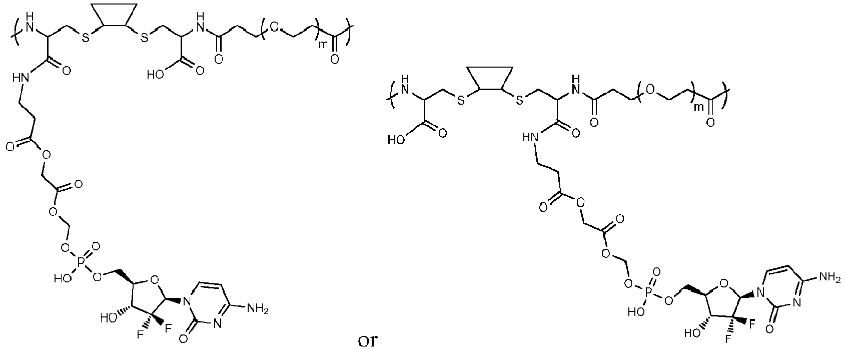 wherein 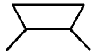 represents a cyclodextrin; n is an integer from 1 to 100 (e.g., n is an integer from 4 to 80, from 4 to 50, from 4 to 30 or from 4 to 20, or n is 4, 5, 6, 7, 8, 9, 10, 11, 12, 13, 14, 15, 16, 17, 18, 19 or 20); m is an integer from 1 to 1000 (e.g., m is an integer from 1 to 200, from 1 to 100, from 1 to 80, from 2 to 80, from 5 to 70, from 10 to 50, or from 20 to 40). In some embodiments, the CDP-pyrimidine analog conjugate, particle or composition e.g., the CDP-gemcitabine derivative conjugate, particle or composition, comprises a mixture of fully-loaded and partially-loaded CDP-pyrimidine analog conjugates, e.g., CDP-gemcitabine derivative conjugates.

In one embodiment, the CDP-pyrimidine analog conjugate comprises a subunit of 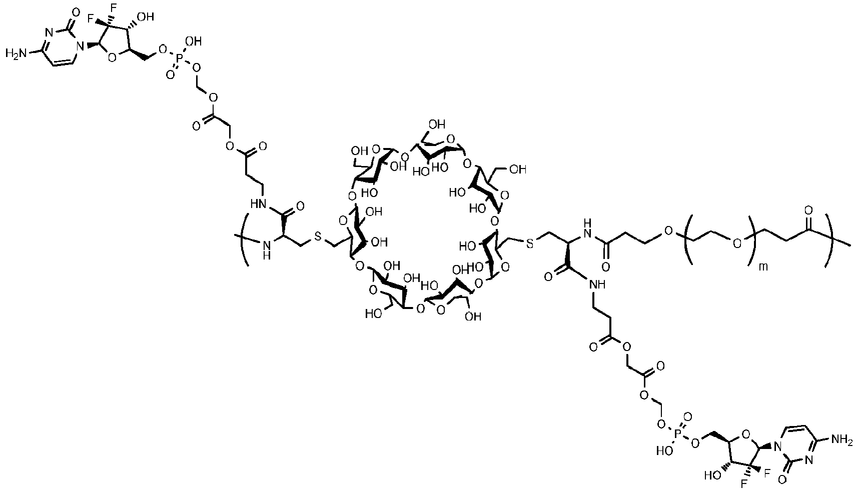 wherein m is an integer from 1 to 1000 (e.g., m is an integer from 1 to 200, from 1 to 100, from 1 to 80, from 2 to 80, from 5 to 70, from 10 to 50, or from 20 to 40).

In one embodiment, the CDP-pyrimidine analog conjugate is a CDP-gemcitabine derivative conjugate, e.g., 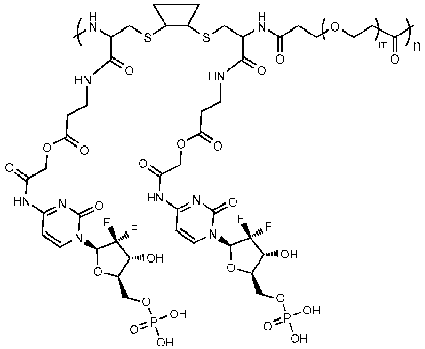 wherein 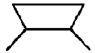 represents a cyclodextrin; n is an integer from 1 to 100 (e.g., n is an integer from 4 to 80, from 4 to 50, from 4 to 30 or from 4 to 20, or n is 4, 5, 6, 7, 8, 9, 10, 11, 12, 13, 14, 15, 16, 17, 18, 19 or 20); m is an integer from 1 to 1000 (e.g., m is an integer from 1 to 200, from 1 to 100, from 1 to 80, from 2 to 80, from 5 to 70, from 10 to 50, or from 20 to 40). In some embodiments, the CDP-pyrimidine analog conjugate, e.g., the CDP-gemcitabine derivative conjugate, does not have complete loading, e.g., one or more binding sites, e.g., cysteine residues, are not bound to a pyrimidine analog, e.g., a gemcitabine derivative, e.g., an ester-linkage bound gemcitabine derivative, e.g., the CDP-gemcitabine derivative conjugate comprises one or more subunits having the formulae provided below: 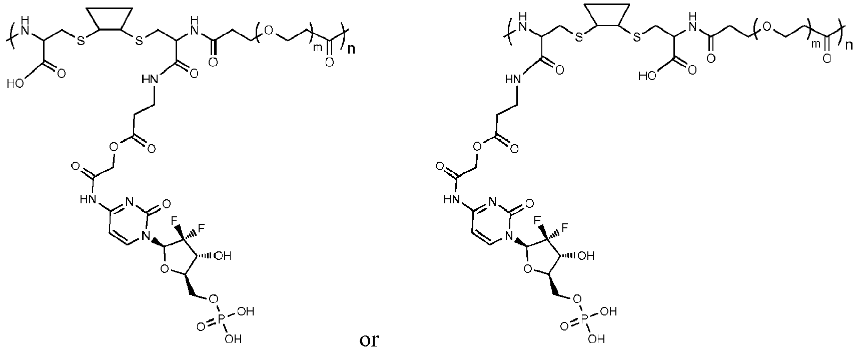 wherein 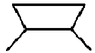 represents a cyclodextrin and m is an integer from 1 to 1000 (e.g., m is an integer from 1 to 200, from 1 to 100, from 1 to 80, from 2 to 80, from 5 to 70, from 10 to 50, or from 20 to 40). In some embodiments, the CDP-pyrimidine analog conjugate, particle or composition e.g., the CDP-gemcitabine derivative conjugate, particle or composition, comprises a mixture of fully-loaded and partially-loaded CDP-pyrimidine analog conjugates, e.g., CDP-gemcitabine derivative conjugates.

In one embodiment, the CDP-pyrimidine analog conjugate comprises a subunit of 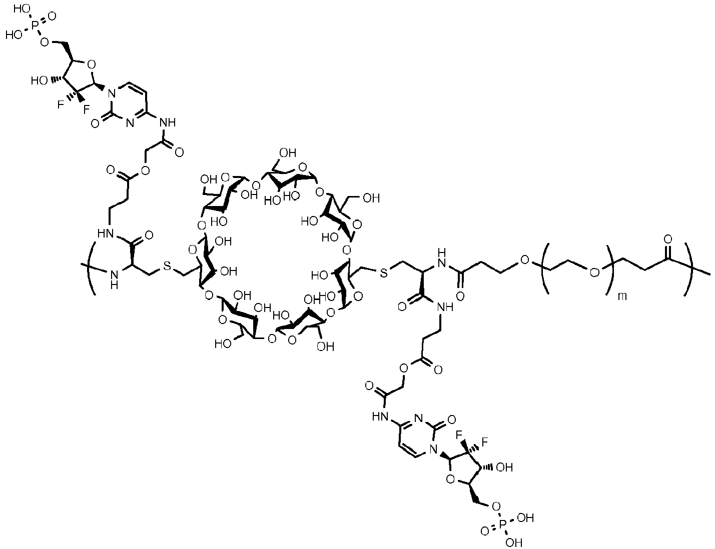 wherein m is an integer from 1 to 1000 (e.g., m is an integer from 1 to 200, from 1 to 100, from 1 to 80, from 2 to 80, from 5 to 70, from 10 to 50, or from 20 to 40).

CDP-gemcitabine conjugates and CDP-gemcitabine derivative conjugates can be made using many different combinations of components described herein. For example, various combinations of cyclodextrins (e.g., beta-cyclodextrin), comonomers (e.g., PEG containing comonomers), linkers linking the cyclodextrins and comonomers, and/or linkers tethering the gemcitabine to the CDP are described herein.

In one embodiment, the CDP-gemcitabine conjugate forms a particle, e.g., a nanoparticle. The particle can comprise a CDP-gemcitabine conjugate, e.g., a plurality of CDP-gemcitabine conjugates, e.g., CDP-gemcitabine conjugates having the same gemcitabine or different gemcitabines. The compositions described herein comprise a CDP-gemcitabine conjugate or a plurality of CDP-gemcitabine conjugates. The composition can also comprise a particle or a plurality of particles described herein.

In one embodiment, the CDP-gemcitabine conjugate containing the inclusion complex forms a particle, e.g., a nanoparticle. The nanoparticle ranges in size from 10 to 300 nm in diameter, e.g., 15 to 280, 30 to 250, 40 to 200, 20 to 150, 30 to 100, 20 to 80, 30 to 70, 40 to 60 or 40 to 50 nm diameter. In one embodiment, the particle is 50 to 60 nm, 20 to 60 nm, 30 to 60 nm, 35 to 55 nm, 35 to 50 nm or 35 to 45 nm in diameter.

In one embodiment, the surface charge of the molecule is neutral, or slightly negative. In some embodiments, the zeta potential of the particle surface is from about - 80 mV to about 50 mV, about -20 mV to about 20 mV, about -20 mV to about -10 mV, or about -10 mV to about 0.

In some embodiments, the CDP-gemcitabine conjugate or CDP-gemcitabine derivative conjugate is a polymer having a formula: 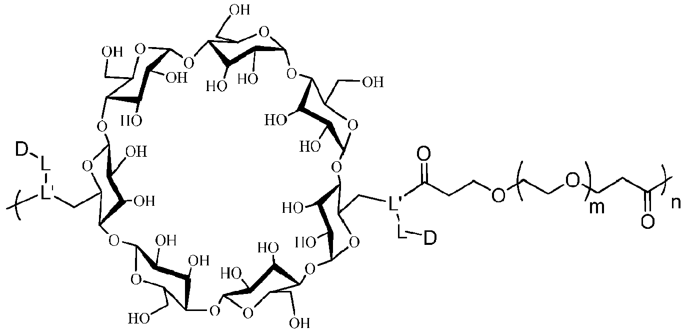 wherein L and L' independently for each occurence, is a linker, a bond, or -OH and D, independently for each occurence, is a gemcitabine, a gemcitabine derivative or absent, and wherein the group 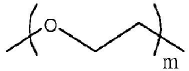 has a Mw of 3400 Da or less and n is at least 4, 5, 6, 7, 8, 9, 10, 11, 12, 13, 14, 15, 16, 17, 18, 19 or 20, provided that at least one D is gemcitabine or a gemcitabine derivative. In some embodiments, at least 2 D moieties are gemcitabine and/or a gemcitabine derivative.

In some embodiments, each L', for each occurence, is a cysteine. In some embodiments, the cysteine is attached to the cyclodextrin via a sulfide bond. In some embodiments, the cysteine is attached to the PEG containing portion of the polymer via an amide bond.

In some embodiments, the L is a linker (e.g., an ester linkage). In some embodiments, L is absent. In some embodiments, D-L together form

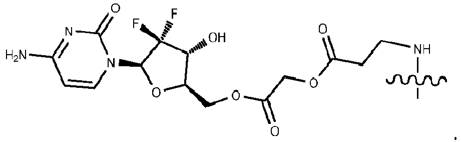

In some embodiments, a plurality of D moieties are absent and at the same position on the polymer, the corresponding L is -OH.

In some embodiments, less than all of the C(=O) moieties of the cysteine residue in the polymer backbone are attached to 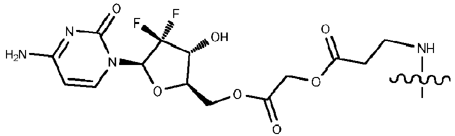 moieties, meaning in some embodiments, 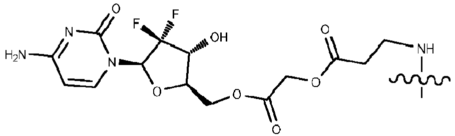 is absent in one or more positions of the polymer backbone, provided that the polymer comprises at least one 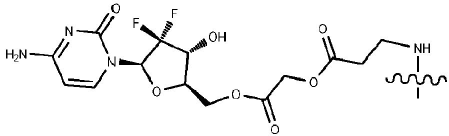 and in some embodiments, at least two 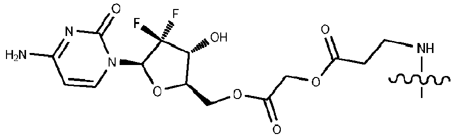 moieties. In some embodiments, the loading of the 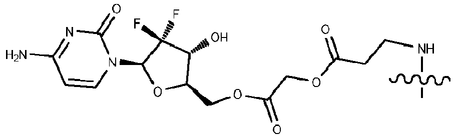 moieties on the CDP-gemcitabine conjugate is from about 1 to about 50% (e.g., from about 1 to about 40%, from about 1 to about 25%, from about 5 to about 20% or from about 5 to about 15%, e.g., from about 6 to about 10%). In some embodiments, the loading of 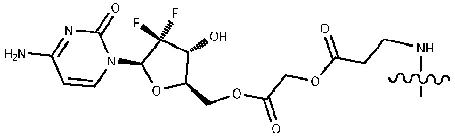 on the CDP is from about 6% to about 10% by weight of the total polymer.

In some embodiments, the L is a linker (e.g., an ester linkage). In some embodiments, L is absent. In some embodiments, D-L together form

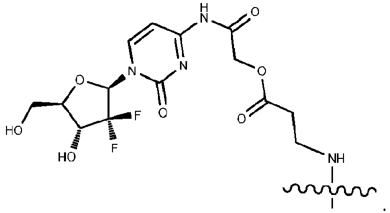

In some embodiments, a plurality of D moieties are absent and at the same position on the polymer, the corresponding L is -OH.

In some embodiments, less than all of the C(=O) moieties of the cysteine residue in the polymer backbone are attached to 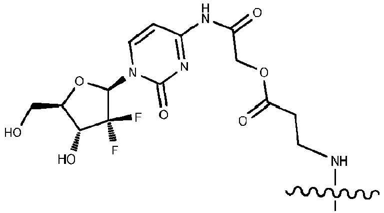 moieties, meaning in some embodiments, 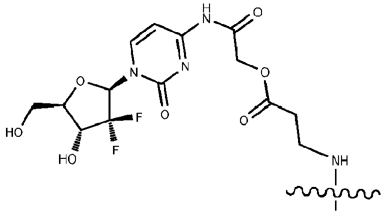 is absent in one or more positions of the polymer backbone, provided that the polymer comprises at least one 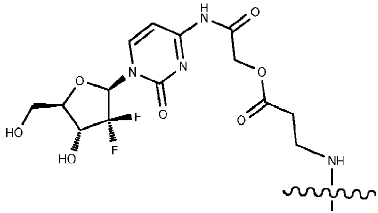 and in some embodiments, at least two 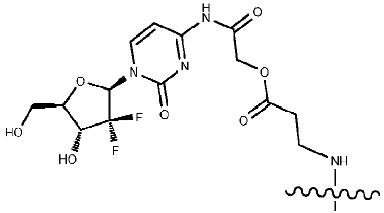 moieties. In some embodiments, the loading of the 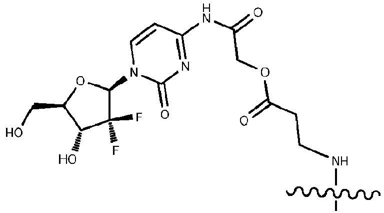 moieties on the CDP-gemcitabine conjugate is from about 1 to about 50% (e.g., from about 1 to about 40%, from about 1 to about 25%, from about 5 to about 20% or from about 5 to about 15%, e.g., from about 6 to about 10%). Tn some embodiments, the loading of 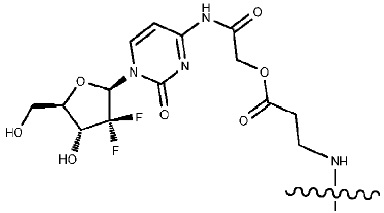 on the CDP is from about 6% to about 10% by weight of the total polymer.

In some embodiments, the L is a linker (e.g., an ester linkage). In some embodiments, L is absent. In some embodiments, D-L together form

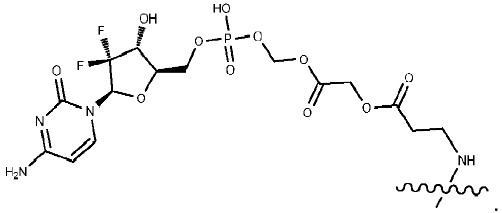

In some embodiments, a plurality of D moieties are absent and at the same position on the polymer, the corresponding L is -OH.

In some embodiments, less than all of the C(=O) moieties of the cysteine residue in the polymer backbone are attached to 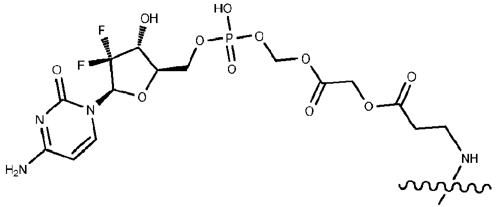 moieties, meaning in some embodiments, 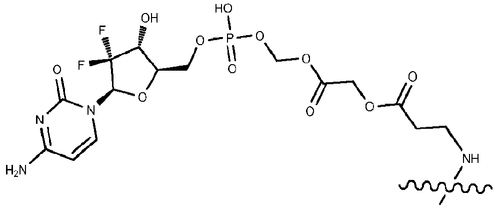 is absent in one or more positions of the polymer backbone, provided that the polymer comprises at least one 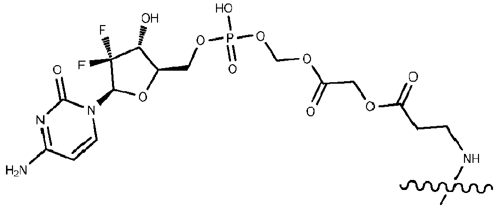 and in some embodiments, at least two 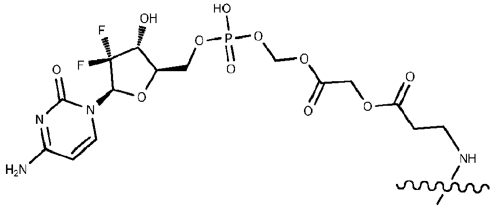 moieties. In some embodiments, the loading of the 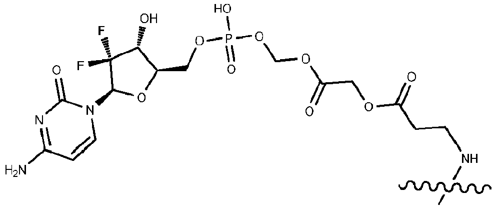 moieties on the CDP-gemcitabine conjugate is from about 1 to about 50% (e.g., from about 1 to about 40%, from about 1 to about 25%, from about 5 to about 20% or from about 5 to about 15%, e.g., from about 6 to about 10%). In some embodiments, the loading of 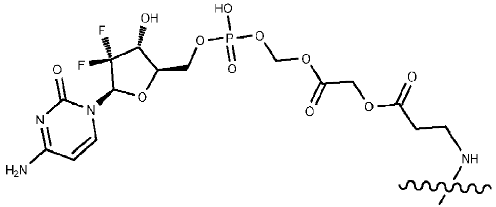 on the CDP is from about 6% to about 10% by weight of the total polymer.

In some embodiments, the L is a linker (e.g., an ester linkage). In some embodiments, L is absent. In some embodiments, D-L together form

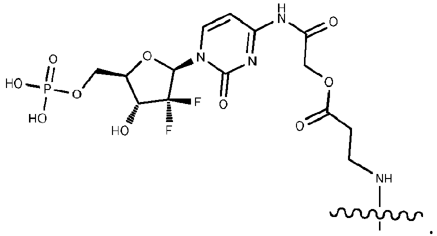

In some embodiments, a plurality of D moieties are absent and at the same position on the polymer, the corresponding L is -OH.

In some embodiments, less than all of the C(=O) moieties of the cysteine residue in the polymer backbone are attached to 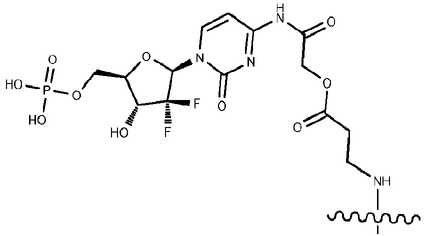 moieties, meaning in some embodiments, 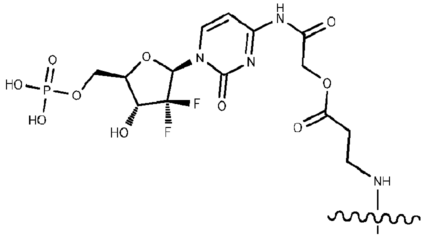 is absent in one or more positions of the polymer backbone, provided that the polymer comprises at least one 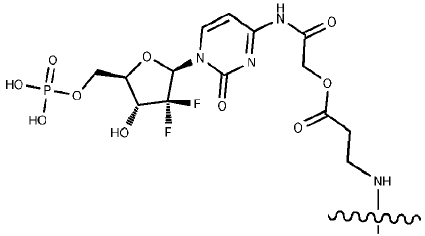 and in some embodiments, at least two 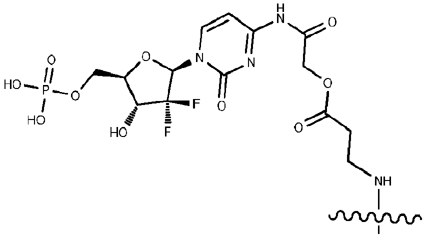 moieties. In some embodiments, the loading of the 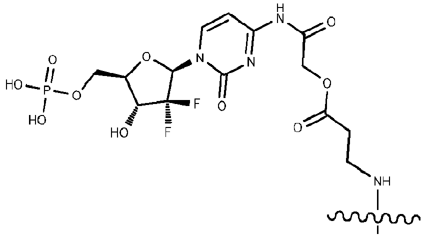 moieties on the CDP-gemcitabine conjugate is from about 1 to about 50% (e.g., from about 1 to about 40%, from about 1 to about 25%, from about 5 to about 20% or from about 5 to about 15%, e.g., from about 6 to about 10%). In some embodiments, the loading of 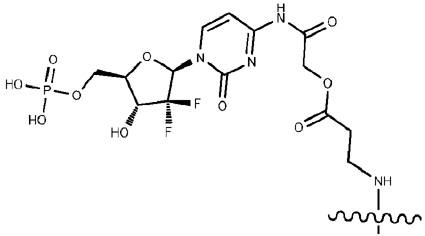 on the CDP is from about 6% to about 10% by weight of the total polymer.

In some embodiments, the CDP-gemcitabine conjugate of formula C is a polymer of formula: 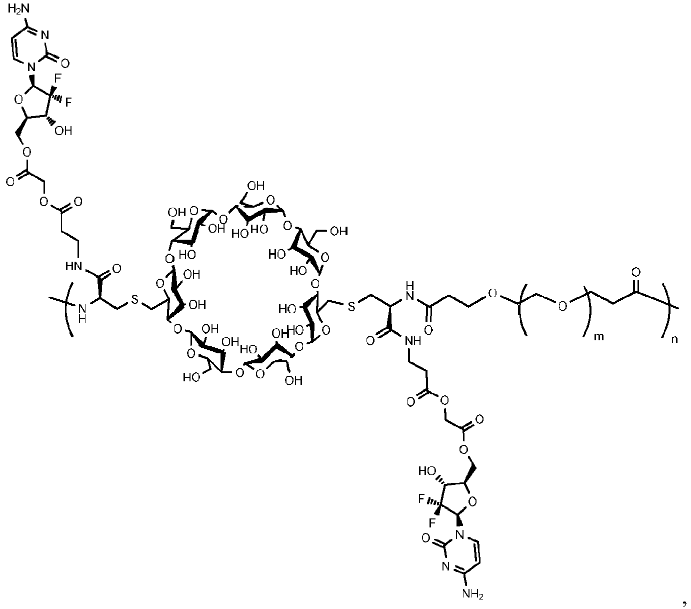 wherein m and n are as defined above, and wherein less than all of the C(=O) sites of the cysteine of the polymer backbone are occupied as indicated above with the gemcitabine-ester, but instead are free acids, meaning, the theoretical loading of the polymer is less than 100%.

In some embodiments, the CDP-gemcitabine conjugate is a polymer of formula: 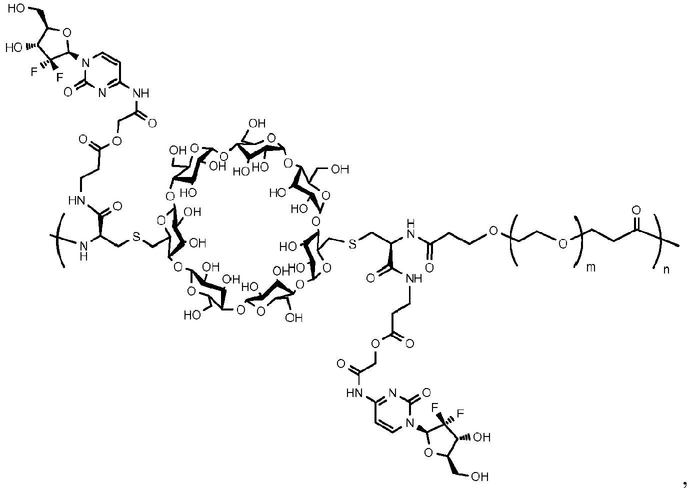 wherein m and n are as defined above, and wherein less than all of the C(=O) sites of the cysteine of the polymer backbone are occupied as indicated above with the gemcitabine-ester, but instead are free acids, meaning, the theoretical loading of the polymer is less than 100%.

In some embodiments, the CDP-gemcitabine conjugate is a polymer of the formula: 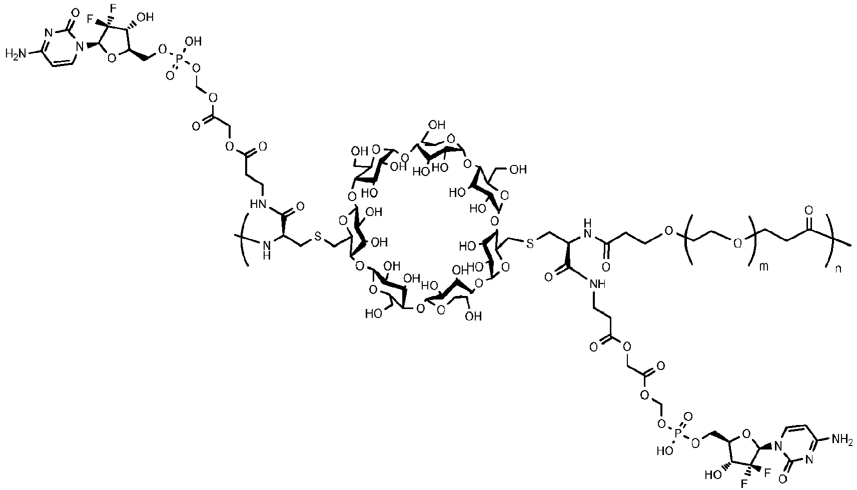 wherein m and n are as defined above, and wherein less than all of the C(=O) sites of the cysteine of the polymer backbone are occupied as indicated above with the gemcitabine-ester, but instead are free acids, meaning, the theoretical loading of the polymer is less than 100%.

In some embodiments, the CDP-gemcitabine conjugate is a polymer of the formula: 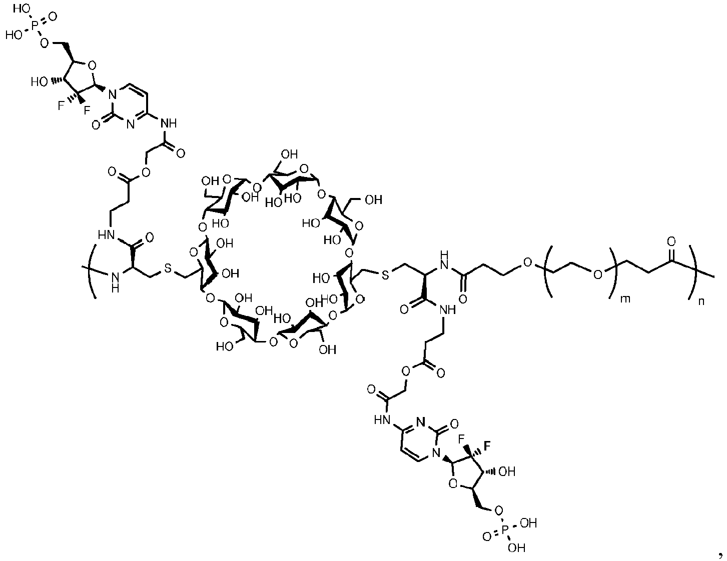 wherein m and n are as defined above, and wherein less than all of the C(=O) sites of the cysteine of the polymer backbone are occupied as indicated above with the gemcitabine-ester, but instead are free acids, meaning, the theoretical loading of the polymer is less than 100%.

In an embodiment, the therapeutic agent in the CDP-therapeutic agent conjugate is a cytotoxic agent such as an alkylating agent. In some embodiments, the alkylating agent in the CDP-alkylating agent conjugate, particle or composition is an alkylating agent including alkylating agents (including, without limitation, nitrogen mustards, ethylenimine derivatives, alkyl sulfonates, nitrosoureas and triazenes): uracil mustard (Aminouracil Mustard®, Chlorethaminacil®, Demethyldopan®, Desmethyldopan®, Haemanthamine®, Nordopan®, Uracil nitrogen mustard®, Uracillost®, Uracilmostaza®, UrDastin®, UrDastine®), chlormethine (Mustargen®), cyclophosphamide (Cytoxan®, Neosar®, Clafen®, Endoxan®, Procytox®, Revimmune™), ifosfamide (Mitoxana®), melphalan (Alkeran®), Chlorambucil (Leukeran®), pipobroman (Amedel®, Vercyte®), triethylenemelamine (Hemel®, Hexalen®, Hexastat®), triethylenethiophosphoramine, Temozolomide (Temodar®), thiotepa (Thioplex®), busulfan (Busilvex®, Myleran®), carmustine (BiCNU®), lomustine (CeeNU®), streptozocin (Zanosar®), and Dacarbazine (DTIC-Dome®)

In an embodiment, the therapeutic agent in the CDP-therapeutic agent conjugate is a cytotoxic agent such as an anthracycline agent. In some embodiments, the anthracycline in the CDP-anthracycline conjugate, particle or composition is an anthracycline including, without limitation, daunorubicin (Cerubidine® or Rubidomycin®), doxorubicin (Adriamycin®), epirubicin (Ellence®), idarubicin (Idamycin®), mitoxantrone (Novantrone®), and valrubicin (Valstar®). Preferred anthracyclines include daunorubicin (Cerubidine® or Rubidomycin®) and doxorubicin (Adriamycin®).

In an embodiment, the therapeutic agent in the CDP-therapeutic agent conjugate is a cytotoxic agent such as an anti-tumor-antibiotic agent. In some embodiments, the anti-tumor-antibiotic agent in the CDP-anti-tumor-antibiotic agent conjugate, particle or composition is an anti-tumor-antibiotic agent including, without limitation, a HSP90 inhibitor, e.g., geldanamycin, a CDP-tanespimycin conjugate or a CDP-alvespimycin conjugate.

In an embodiment, the therapeutic agent in the CDP-therapeutic agent conjugate is a cytotoxic agent such as platinum based agent. In some embodiments, the platinum based agent in the CDP-platinum based agent conjugate, particle or composition is a platinum based agent including, without limitation, cisplatin (Platinol® or Platinol-AQ®) carboplatin (Paraplatin® or Paraplatin-AQ®), and oxaliplatin (Eloxatin®).

In an embodiment, the therapeutic agent in the CDP-therapeutic agent conjugate is a cytotoxic agent such as microtubule inhibitor. In some embodiments, the microtubule inhibitor in the CDP-microtubule inhibitor conjugate is a taxane. In some embodiments, the taxane in the CDP-taxane conjugate, particle or composition is a taxane including, without limitation, paclitaxel (Taxol®), docetaxel (Taxotere®), larotaxel, and cabazitaxel.

### Taxanes

The term "taxane," as used herein, refers to any naturally occurring, synthetic, or semi-synthetic taxane structure, for example, known in the art. Exemplary taxanes include those compounds shown below, including, for example, formula (X), (XIIa), and (XIIb).

In one embodiment, a taxane is a compound of the following formula (X): 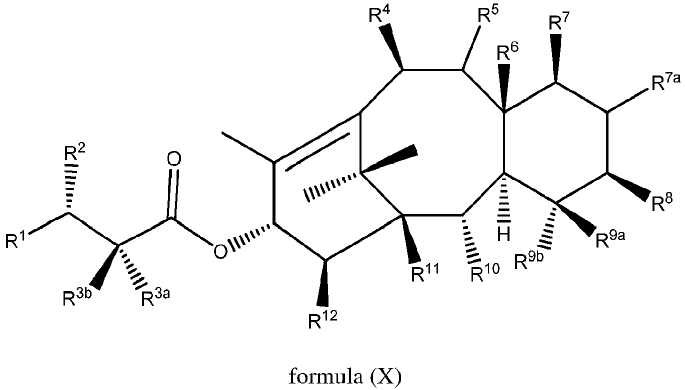 wherein;
R¹ is aryl (e.g., phenyl), heteroaryl (e.g., furanyl, thiophenyl, or pyridyl), alkyl (e.g., butyl such as isobutyl or tert-butyl), cycloalyl (e.g., cyclopropyl), heterocycloalkyl (epoxyl), or R¹, when taken together with one of R^{3b}, R^{9b}, or R¹⁰ and the carbons to which they are attached, forms a mono- or bi-cyclic ring system; wherein R¹ is optionally substituted with 1-3 R^{1a};
R² is NR^{2a}R^{2b} or OR^{2c};
R^{3a} is H, OH, O-polymer, OC(O)alkyl, or OC(O)alkenyl;
R^{3b} is H or OH; or together with R¹ and the carbon to which it is attached, forms a mono- or bi-cyclic ring system;
R⁴ is OH, alkoxy (e.g., methoxy), OC(O)alkyl (e.g., Oacyl), OC(O)cycloalkyl, heterocycloalkylalkyl; or R⁴ together with R⁵ and the carbons to which they are attached, form an optionally substituted ring; or R⁴, together with the carbon to which it is attached, forms a ring (forming a spirocyclic ring) or an oxo;
R⁵ is OH, OC(O)alkyl (e.g., Oacyl); or R⁵ together with R⁴ or R⁷ and the carbons to which they are attached, form an optionally substituted ring; or R⁵, together with the carbon to which it is attached, forms a ring (forming a spirocyclic ring) or an oxo;
R⁶ is alkyl (e.g., methyl); or R⁶ together with R⁷ and the carbons to which they are attached, form an optionally substituted ring (e.g., a cyclopropyl ring);
R⁷ is H, OH, alkoxy (e.g., methoxy), OC(O)Oalkyl, OalkylSalkyl (e.g., OCH₂SMe), or OalkylOalkyl (e.g., OCH₂OMe), thioalkyl, SalkylOalkyl (e.g., SCH₂OMe); or R⁷ together with R⁵ or R⁶ and the carbons to which they are attached, form an optionally substituted ring (e.g., a cyclopropyl ring);
R^{7a} H or OH;
R⁸ is OH or a leaving group (e.g., a mesylate, or halo); or R⁸ taken together with R^{9a} and the carbons to which they are attached form a ring;
R^{9a} is an activated alkyl (e.g.CH₂I); or R^{9a} taken together with R⁸ and the carbons to which they are attached form a ring; or R^{9a}, together with R^{9b} and the carbon to which it is attached, forms a ring (forming a spirocyclic ring);
R^{9b} is OH, OC(O)alkyl (e.g., Oacyl), OC(O)Oalkyl (e.g., OC(O)OMe), or OC(O)cycloalkyl; or R^{9b}, taken together with R¹ and the carbons to which they are attached, form a ring; or R^{9b}, together with R^{9a} and the carbon to which it is attached, forms a ring (forming a spirocyclic ring);
R¹⁰ is OH, OC(O)aryl (e.g., wherein aryl is optionally substituted for example with halo, alkoxy, or N₃) or OC(O)alkyl; or R¹⁰ taken together with R¹ or R¹¹ and the carbons to which they are attached, forms a ring;
R¹¹ H or OH; or R¹¹ taken together with R¹⁰ or R¹² and the carbons to which they are attached, forms a ring;
R¹² is H, or OH; or R¹² taken together with R¹¹ and the carbons to which they are attached, forms a ring;
each R^{1a} is independently halo (e.g., fluro), alkyl (e.g., methyl)
each R^{2a} and R^{2b} is independently H, C(O)aryl (e.g, C(O)phenyl), C(O)alkyl (e.g., acyl), C(O)H, C(O)Oalkyl; wherein C(O)aryl (e.g, C(O)phenyl), C(O)alkyl (e.g., acyl), and C(O)Oalkyl is each optionally further substituted, for example, with a substituent as descdribed in R^{1a}; and
R^{2c} is H or C(O)NHalkyl.

In some embodiments, R¹ is phenyl (e.g., optionally substituted for example with halo such as fluoro). In some embodiments, R¹ is heteroaryl, for example, furanyl, thiophenyl, or pyridyl (e.g., an optionally substituted pyridyl).

In some embodiments, R¹ is alkyl, e.g., butyl such as isobutyl or tert-butyl.

In some embodiments, R¹ is heterocyclcoalkyl (e.g., epoxyl optionally substituted, for example, with one or more alkyl groups such as methyl).

In some embodiments, R¹, taken together with R^{3b} and the carbons to which they are attached form a bicyclic ring system (e.g.,

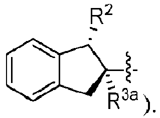

In some embodiments, R¹, taken together with R¹⁰ and the carbons to which they are attached, form a ring, e.g., a mono- or bi-cyclic ring system).

In some embodiments, R¹, taken together with R^{9b} and the carbons to which they are attached, form a ring, e.g., a mono- or bi-cyclic ring system).

In some embodiments, R² is NR^{2a}R^{2b}. In some embodiments, at least one of R^{2a} or R^{2b} is H. In some embodiments, R^{2a} is H and R^{2b} is C(O)aryl (e.g, C(O)phenyl), C(O)alkyl (e.g., acyl), C(O)H, or C(O)Oalkyl. In some embodiments, R² is NHC(O)aryl or NHC(O)Oalkyl.

In some embodiments, R^{3a} is OH. In some embodiments, R^{3a} is Opolymer. In some embodiments, polymer is polyglutamic acid. In some embodiments, R^{3a} is OC(O)C₂₁alkenyl.

In some embodiments, one of R^{3a} or R^{3b} is H and the other of R^{3a} or R^{3b} is OH. In some embodiments, R⁴ is OAcyl. In some embodiments, R⁴ is OH. In some embodiments, R⁴ is methoxy. In some embodiments, R⁴ together with R⁵ and the carbons to which they are attached forms 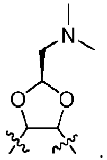 In some embodiments, R⁴, together with the carbon to which it is attached, forms 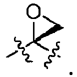 In some embodiments, R⁴, together with the carbon to which it is attached, forms an oxo. In some embodiments, R⁴ is heterocycloalkylalkyl (e.g.,

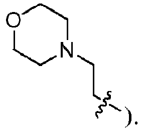

In some embodiments, R⁵, together with the carbon to which it is attached, forms an oxo. In some embodiments, R⁵ together with R⁷ and the carbons to which they are attached forms

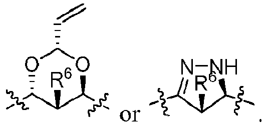

In some embodiments, R⁶ is methyl. In some embodiments, R⁶ together with R⁷ and the carbons to which they are attached form a ring (e.g., cyclopropyl).

In some embodiments, R⁷ is OH. In some embodiments, R⁷ is H. In some embodiments, when R⁷ is H, R^{7a} is OH.

In some embodiments, R^{7a} is H. In some embodiments, R^{7a} is OH. In some embobodiments, R⁸ together with R^{9a} and the carbons to which they are attached form 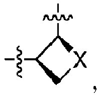 wherein X is O, S, Se, or NR^{8a} (e.g., O), wherein R^{8a} is H, alkyl, arylalkyl (e.g., benzyl), C(O)alkyl, or C(O)H. In some embobodiments, R⁸ together with R^{9a} and the carbons to which they are attached form a cyclopropyl ring.

In some embodiments, R^{9b} is OAc.

In some embodiments, R¹⁰ is OC(O)phenyl. In some embodiments, R¹⁰ taken together with R¹¹ and the carbon to which it is attached, forms a ring such as 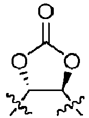 or

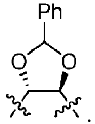

In some embodiments, R¹¹ is OH. In some embodiments, R¹¹ taken together with R¹² and the carbon to which it is attached, forms a ring such as

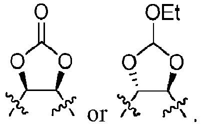

In some embodiments, R¹² is H.

In some embodiments, the variables defined above are chosen so as to form docetaxel, paclitaxel, larotaxel, or cabazitaxel or a structural analogue therof.

In some embodiments, the taxane is a compound of formula (Xa):

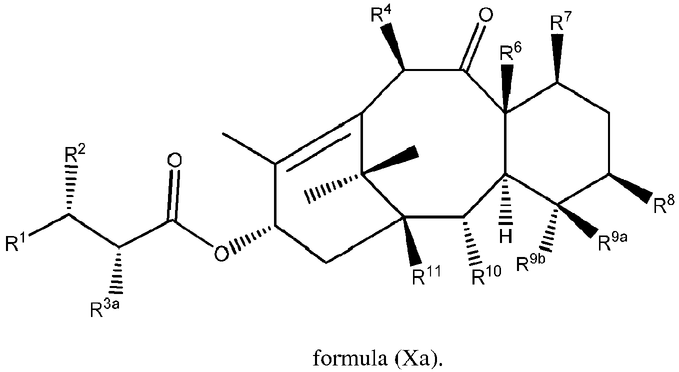

In some embodiments, the taxane is a compound of formula (Xb):

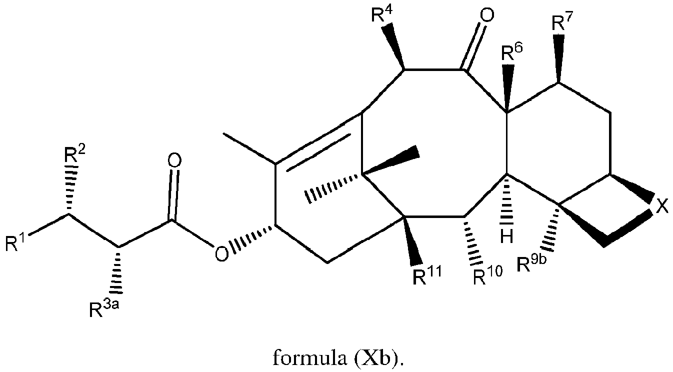

In some embodiments, the compound is a compound of formula Xc:

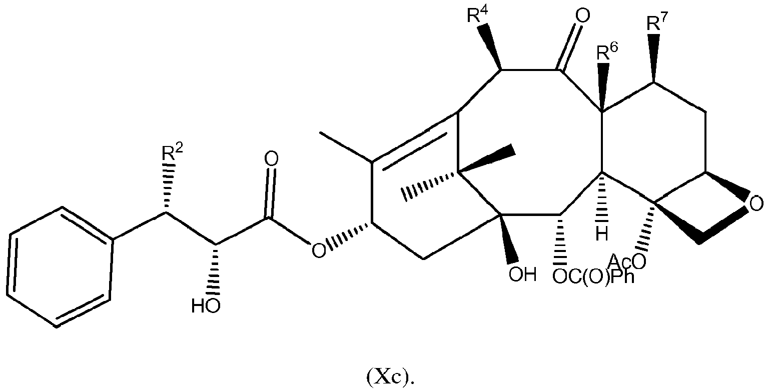

In some embodiments, R² is NHC(O)aryl or NHC(O)Oalkyl.

In some embodiments, R⁴ is OH or OAc.

In some embodiments, R⁶ is methyl.

In some embodiments, R⁷ is OH or OMe.

In some embodiments, R⁶ and R⁷, together with the carbons to which they are attached, form a ring.

In some embodiments, the variables defined above are chosen so as to form docetaxel, paclitaxel, larotaxel, or cabazitaxel or a structural analogue therof.

In one embodiment, the taxane is a compound of formula (XI): 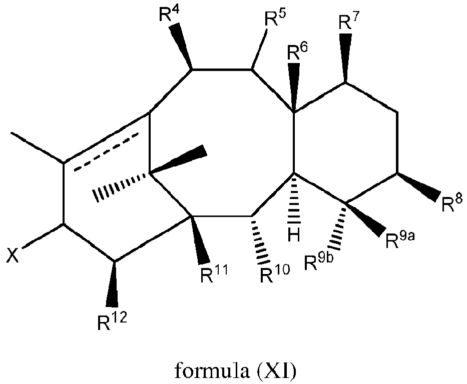 wherein,
X is OH, oxo (i.e., when forming a double bond with the carbon to which it is attached), alkoxy, OC(O)alkyl (e.g., Oacyl), or OPg;
R⁴ is OH, alkoxy (e.g., methoxy), OC(O)alkyl (e.g., Oacyl), OC(O)cycloalkyl, OPg, heterocycloalkylalkyl; or R⁴ together with R⁵ and the carbons to which they are attached, form an optionally substituted ring; or R⁴, together with the carbon to which it is attached, forms a ring (forming a spirocyclic ring) or an oxo;
R⁵ is OH, OC(O)alkyl (e.g., Oacyl), or OPg; or R⁵ together with R⁴ and the carbons to which they are attached, form an optionally substituted ring; or R⁵, together with the carbon to which it is attached, forms an oxo;
R⁶ is alkyl (e.g., methyl);
R⁷ is H, OH, alkoxy (e.g., methoxy), OC(O)alkyl (e.g., OAc); OPg (e.g., OTES or OTroc), or OC(O)alkenyl (wherein alkenyl is substituted, e.g., with aryl (e.g., napthyl) (e.g., OC(O)CHCHnapthyl), or R⁷, together with the carbon to which it is attached, forms an oxo;
R⁸ is OH, optionally substituted OC(O)arylalkyl (e.g., OC(O)CHCHphenyl), OC(O)(CH₂)₁₋₃aryl (e.g., OC(O)CH₂CH₂phenyl), or a leaving group (e.g., a mesylate, or halo); or R⁸ taken together with R^{9a} and the carbons to which they are attached form a ring;
R^{9a} is an activated alkyl (e.g. CH₂I); or R^{9a} taken together with R⁸ and the carbons to which they are attached form a ring; or R^{9a}, together with R^{9b} and the carbon to which it is attached, forms a ring (forming a spirocyclic ring)or R^{9a} taken together with R^{9b} and the carbon to which they are attached form an alylenyl;
R^{9b} is OH, alkoxy, OC(O)alkyl (e.g., Oacyl), OC(O)Oalkyl (e.g., OC(O)OMe), OC(O)cycloalkyl, or OPg; or R^{9b}, together with R^{9a} and the carbon to which it is attached, forms a ring (forming a spirocyclic ring); or R^{9b} taken together with R^{9a} and the carbon to which they are attached form an alylenyl;
R¹⁰ is OH, OC(O)aryl (e.g., wherein aryl is optionally substituted for example with halo, alkoxy, or N₃) or OC(O)alkyl; or R¹⁰ taken together with R¹¹ and the carbons to which they are attached, forms a ring;
R¹¹ H, OH; or R¹¹ taken together with R¹⁰ or R¹² and the carbons to which they are attached, forms a ring;
R¹² is H, OH, or OC(O)alkyl, wherein alkyl is substituted with 1-4 substituents; or R¹² taken together with R¹¹ and the carbons to which they are attached, forms a ring;
Pg is a protecting group for a heteroatom such as O or N (e.g., Bn, Bz, TES, TMS, DMS, Troc, or Ac); and
is a single or double bond

In some embodiments, X is OH. In some embodiments, X is oxo. In some embodiments, X is OAc.

In some embodiments, is a single bond.

In some embodiments, R⁴ is OAcyl. In some embodiments, R⁴ is OH. In some embodiments, R⁴ is methoxy. In some embodiments, R⁴ is OPg (e.g., OTroc or OAc). In some embodiments, R⁴ together with R⁵ and the carbons to which they are attached forms a ring.

In some embodiments, R⁵, together with the carbon to which it is attached, forms
an oxo. In some embodiments, R⁵ is OH or OPg. In some embodiments, R⁶ is methyl.

In some embodiments, R⁷ is H. In some embodiments, R⁷ is OH or OPg. In some embodiments, R⁷, together with the carbon to which it is attached, forms an oxo. In some embodiments, R⁸ is 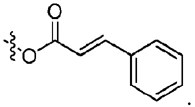 In some embodiments, R⁸ together with R^{9a} and the carbons to which they are attached form 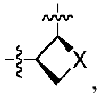 wherein X is O, S, Se, or NR^{8a} (e.g., O), wherein R^{8a} is H, alkyl, arylalkyl (e.g., benzyl), C(O)alkyl, Pg, or C(O)H. In some embodiments, R⁸ together with R^{9a} and the carbons to which they are attached form a cyclopropyl ring. In some embodiments,

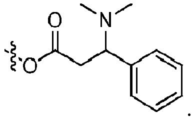

In some embodiments, R^{9a} and R^{9b}, together with the carbon to which they are attached form

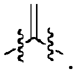

In some embodiments, R^{9b} is OAc.

In some embodiments, R¹⁰ is OC(O)phenyl. In some embodiments, R¹⁰ taken together with R¹¹ and the carbon to which it is attached, forms a ring such as 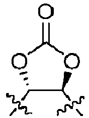 or

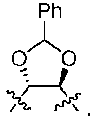

In some embodiments, R¹¹ is H. In some embodiments, R¹¹ is OH.

In some embodiments, R¹² is H. In some embodiments, R¹² is OH. In some embodiments, R¹² is

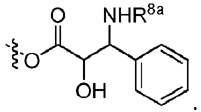

In one embodiment, the taxane is a compound of formula (XIIa): 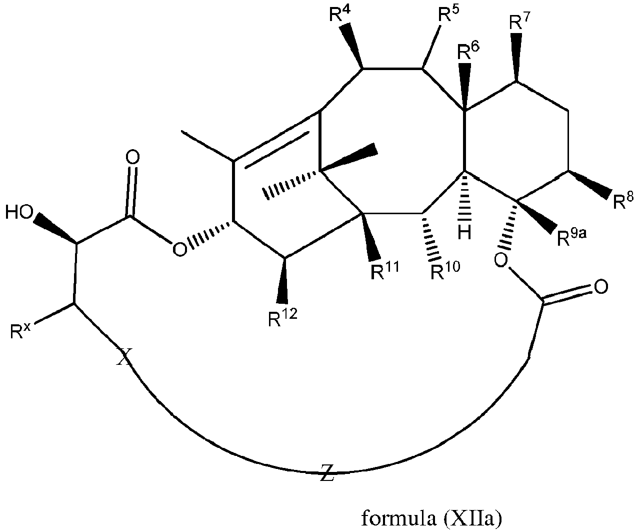 wherein,
Z forms a ring by linking O with the atom X attached to -CHR^{x};
R⁴ is OH, alkoxy (e.g., methoxy), OC(O)alkyl (e.g., Oacyl), OC(O)cycloalkyl, heterocycloalkylalkyl; or R⁴ together with R⁵ and the carbons to which they are attached, form an optionally substituted ring; or R⁴, together with the carbon to which it is attached, forms a ring (forming a spirocyclic ring) or an oxo;
R⁵ is OH, OC(O)alkyl (e.g., Oacyl); or R⁵ together with R⁴ or R⁷ and the carbons to which they are attached, form an optionally substituted ring; or R⁵, together with the carbon to which it is attached, forms a ring (forming a spirocyclic ring) or an oxo;
R⁶ is alkyl (e.g., methyl); or R⁶ together with R⁷ and the carbons to which they are attached, form an optionally substituted ring (e.g., a cyclopropyl ring);
R⁷ is H, OH, alkoxy (e.g., methoxy), OC(O)Oalkyl, OalkylSalkyl (e.g., OCH₂SMe), or OalkylOalkyl (e.g., OCH₂OMe), thioalkyl, SalkylOalkyl (e.g., SCH₂OMe); or R⁷ together with R⁵ or R⁶ and the carbons to which they are attached, form an optionally substituted ring (e.g., a cyclopropyl ring);
R^{7a} H or OH;
R⁸ is OH or a leaving group (e.g., a mesylate, or halo); or R⁸ taken together with R^{9a} and the carbons to which they are attached form a ring;
R^{9a} is an activated alkyl (e.g.CH₂I); or R^{9a} taken together with R⁸ and the carbons to which they are attached form a ring;
R¹⁰ is OH, OC(O)aryl (e.g., wherein aryl is optionally substituted for example with halo, alkoxy, or N₃) or OC(O)alkyl; or R¹⁰ taken together with R¹ or R¹¹ and the carbons to which they are attached, forms a ring;
R¹¹ H or OH; or R¹¹ taken together with R¹⁰ or R¹² and the carbons to which they are attached, forms a ring;
R¹² is H, or OH; or R¹² taken together with R¹¹ and the carbons to which they are attached, forms a ring;
R^{x} is NHPg or aryl;
X is C or N; and
Pg is a protecting group for a heteroatom such as O or N (e.g., Bn, Bz, TES, TMS, DMS, Troc, Boc or Ac).

In some embodiments, Z includes one or more phenyl rings.

In some embodiments, Z includes one or more double bonds.

In some embodiments, Z includes one or more heteroatoms.

In some embodiments, Z is 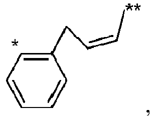 wherein * indicates the atom X attached to CHR^{x} and ** indicates the carbon attached to C(O). In some embodiments, Z is 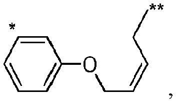 wherein * indicates the atom X attached to CHR^{x} and ** indicates the carbon attached to C(O). In some embodiments, Z is 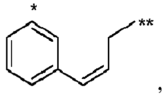 wherein * indicates the atom X attached to CRR^{x} and ** indicates the carbon attached to C(O).

In some embodiments, the taxane is a compound of formula (XIIb): 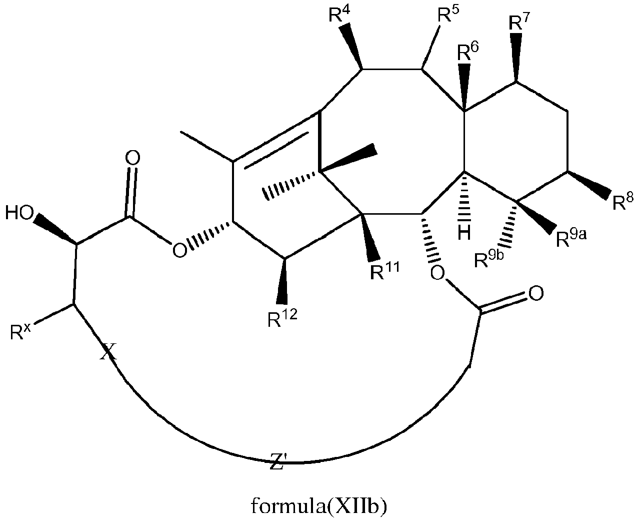 wherein,
Z' forms a ring by linking O with the atom X, which is attached to -CHR^{x};
R⁴ is OH, alkoxy (e.g., methoxy), OC(O)alkyl (e.g., Oacyl), OC(O)cycloalkyl, heterocycloalkylalkyl; or R⁴ together with R⁵ and the carbons to which they are attached, form an optionally substituted ring; or R⁴, together with the carbon to which it is attached, forms a ring (forming a spirocyclic ring) or an oxo;
R⁵ is OH, OC(O)alkyl (e.g., Oacyl); or R⁵ together with R⁴ or R⁷ and the carbons to which they are attached, form an optionally substituted ring; or R⁵, together with the carbon to which it is attached, forms a ring (forming a spirocyclic ring) or an oxo;
R⁶ is alkyl (e.g., methyl); or R⁶ together with R⁷ and the carbons to which they are attached, form an optionally substituted ring (e.g., a cyclopropyl ring);
R⁷ is H, OH, alkoxy (e.g., methoxy), OC(O)Oalkyl, OalkylSalkyl (e.g., OCH₂SMe), or OalkylOalkyl (e.g., OCH₂OMe), thioalkyl, SalkylOalkyl (e.g., SCH₂OMe); or R⁷ together with R⁵ or R⁶ and the carbons to which they are attached, form an optionally substituted ring (e.g., a cyclopropyl ring);
R^{7a} H or OH;
R⁸ is OH or a leaving group (e.g., a mesylate, or halo); or R⁸ taken together with R^{9a} and the carbons to which they are attached form a ring;
R^{9a} is an activated alkyl (e.g.CH₂I); or R^{9a} taken together with R⁸ and the carbons to which they are attached form a ring; or R^{9a}, together with R^{9b} and the carbon to which it is attached, forms a ring (forming a spirocyclic ring);
R^{9b} is OH, OC(O)alkyl (e.g., Oacyl), OC(O)Oalkyl (e.g., OC(O)OMe), or OC(O)cycloalkyl; or R^{9b}, together with R^{9a} and the carbon to which it is attached, forms a ring (forming a spirocyclic ring);
R¹¹ H or OH; or R¹¹ taken together with R¹⁰ or R¹² and the carbons to which they are attached, forms a ring;
R¹² is H, or OH; or R¹² taken together with R¹¹ and the carbons to which they are attached, forms a ring;
R^{x} is NHPg or aryl;
X is C or N; and
Pg is a protecting group for a heteroatom such as O or N (e.g., Bn, Bz, TES, TMS, DMS, Troc, Boc or Ac).

In some embodiments, Z' includes one or more phenyl rings.

In some embodiments, Z' includes one or more double bonds.

In some embodiments, Z' includes one or more heteroatoms.

In some embodiments, Z' is 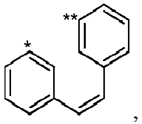 wherein * indicates the atom X attached to CHR^{x} and ** indicates the carbon attached to C(O). In some embodiments, Z' is 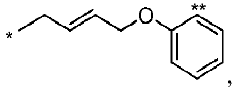 wherein * indicates the atom X attached to CHR^{x} and ** indicates the carbon attached to C(O). In some embodiments, Z' is 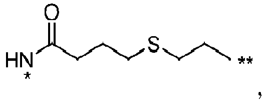 wherein * indicates the atom X attached to CHR^{x} and ** indicates the carbon attached to C(O).

In some embodiments, the taxane is a compound of formula (XIII): 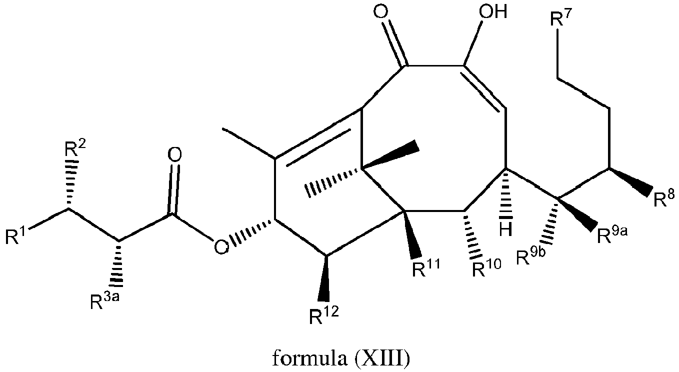 wherein,
R¹ is aryl (e.g., phenyl), heteroaryl (e.g., furanyl, thiophenyl, or pyridyl), alkyl (e.g., butyl such as isobutyl or tert-butyl), cycloalyl (e.g., cyclopropyl), heterocycloalkyl (epoxyl), or R¹, when taken together with one of R^{3b}, R^{9b}, or R¹⁰ and the carbons to which they are attached, forms a mono- or bi-cyclic ring system; wherein R¹ is optionally substituted with 1-3 R^{1a};
R² is NR^{2a}R^{2b} or OR^{2c};
R^{3a} is H, OH, Opolymer, OC(O)alkyl, or OC(O)alkenyl;
R⁷ is OH, alkoxy (e.g., methoxy), OC(O)Oalkyl;
R⁸ is OH or a leaving group (e.g., a mesylate, or halo); or R⁸ taken together with R^{9a} and the carbons to which they are attached form a ring;
R^{9a} is an activated alkyl (e.g.CH₂I); or R^{9a} taken together with R⁸ and the carbons to which they are attached form a ring; or R^{9a}, together with R^{9b} and the carbon to which it is attached, forms a ring (forming a spirocyclic ring)
R^{9b} is OH, OC(O)alkyl (e.g., Oacyl), OC(O)Oalkyl (e.g., OC(O)OMe), or OC(O)cycloalkyl; or R^{9b}, taken together with R¹ and the carbons to which they are attached, form a ring; or R^{9b}, together with R^{9a} and the carbon to which it is attached, forms a ring (forming a spirocyclic ring);
R¹⁰ is OH, OC(O)aryl (e.g., wherein aryl is optionally substituted for example with halo, alkoxy, or N₃) or OC(O)alkyl; or R¹⁰ taken together with R¹ or R¹¹ and the carbons to which they are attached, forms a ring;
R¹¹ H or OH; or R¹¹ taken together with R¹⁰ or R¹² and the carbons to which they are attached, forms a ring;
R¹² is H, or OH; or R¹² taken together with R¹¹ and the carbons to which they are attached, forms a ring;
each R^{1a} is independently halo (e.g., fluro), alkyl (e.g., methyl)
each R^{2a} and R^{2b} is independently H, C(O)aryl (e.g, C(O)phenyl), C(O)alkyl (e.g., acyl), C(O)H, C(O)Oalkyl; wherein C(O)aryl (e.g, C(O)phenyl), C(O)alkyl (e.g., acyl), and C(O)Oalkyl is each optionally further substituted, for example, with a substituent as descdribed in R^{1a};
R^{2c} is H or C(O)NHalkyl; and
R^{8a} is H, alkyl, arylalkyl (e.g., benzyl), C(O)alkyl, or C(O)H.

In some embodiments, R⁷ is OH.

In some preferred embodiments, the taxane is docetaxel, larotaxel, milataxel, TPI-287, TL-310, BMS-275183, BMS-184476, BMS-188797, ortataxel, tesetaxel, or cabazitaxel. Additional taxanes are provided in Fan, Mini-Reviews in Medicinal Chemistry, 2005, 5, 1-12; Gueritte, Current Pharmaceutical Design, 2001, 7, 1229-1249; Kingston, J. Nat. Prod., 2009, 72, 507-515; and Ferlini, Exper Opin. Invest. Drugs, 2008, 17, 3, 335-347; the contents of each of which is incorporated herein by reference in its entirety.

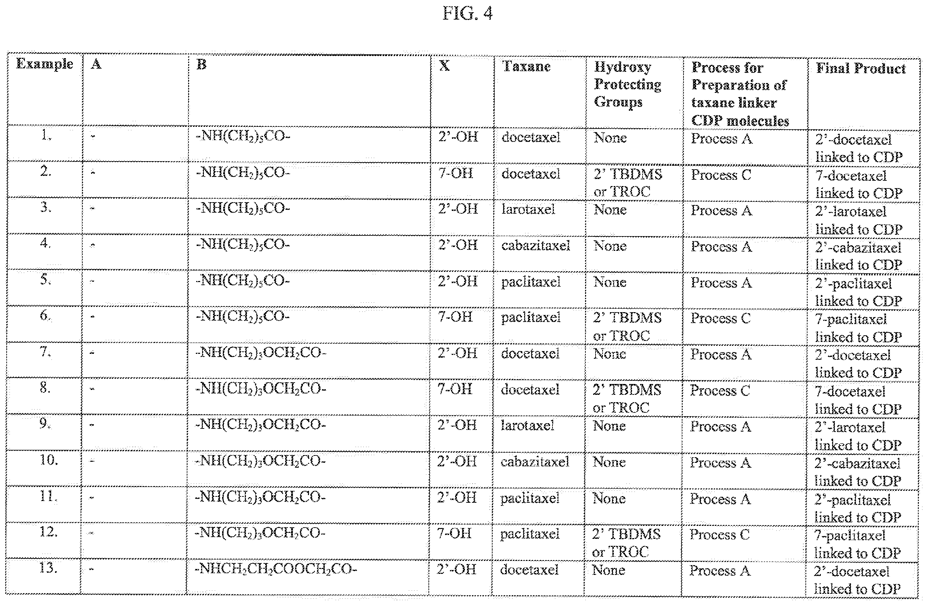

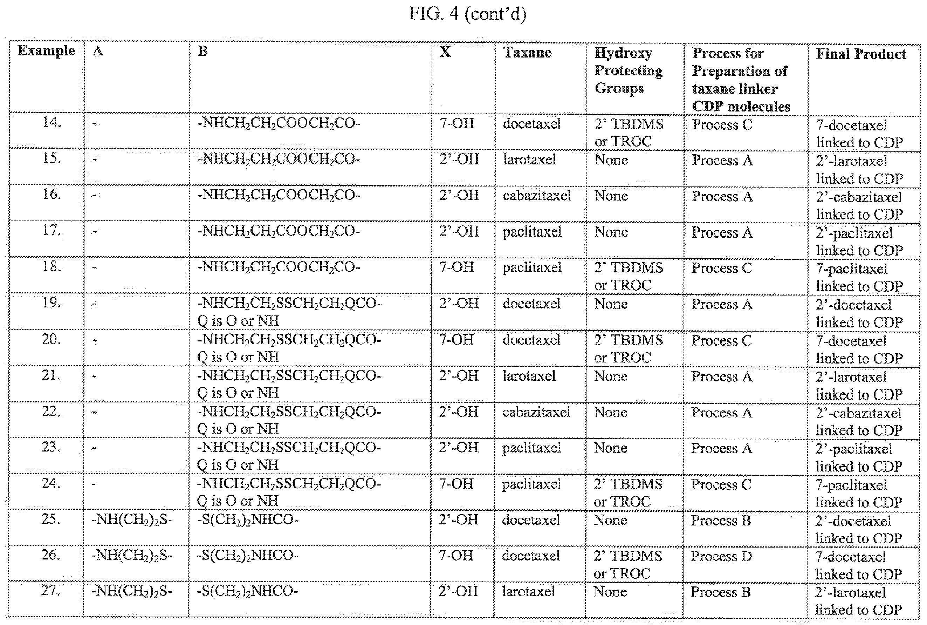

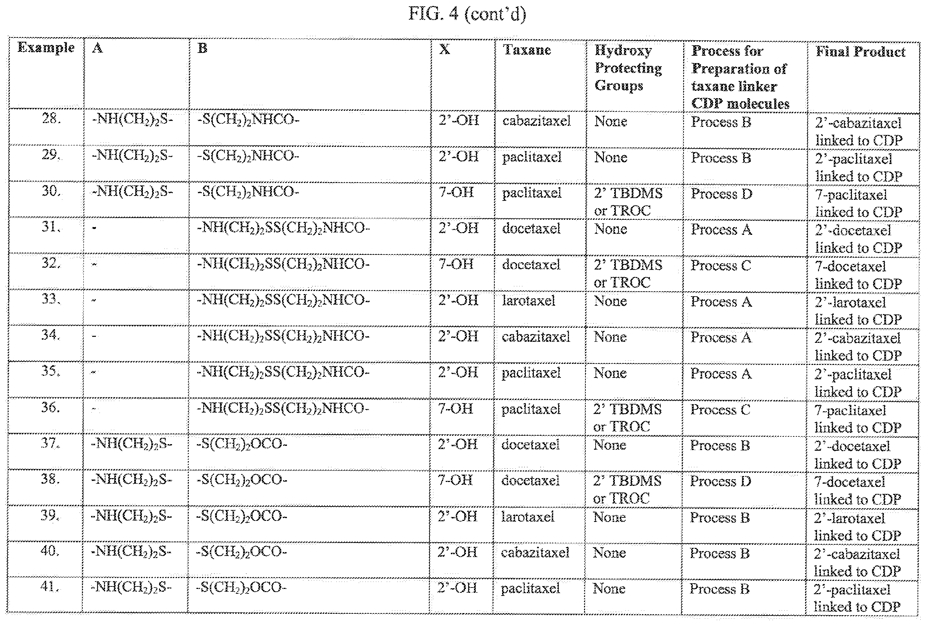

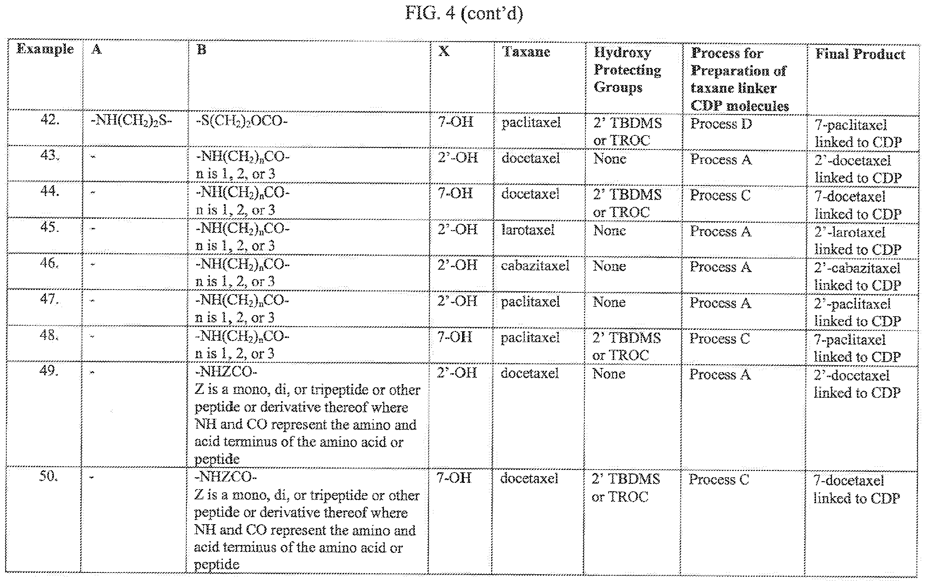

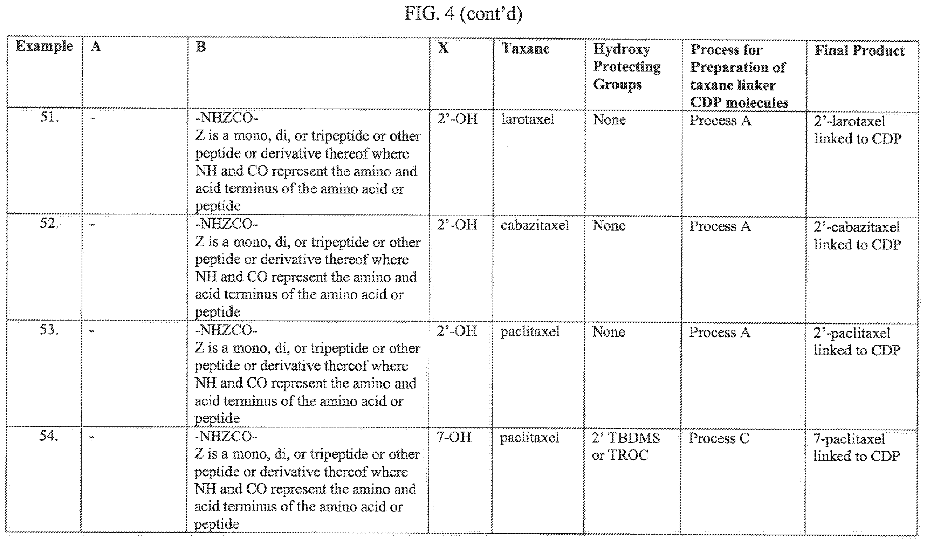

In one embodiment, the CDP-microtubule inhibitor conjugate is a CDP-taxane conjugate, e.g., 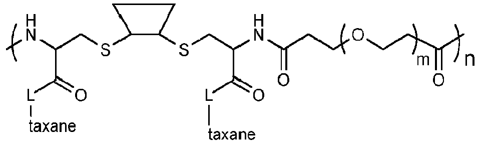 wherein 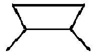 represents a cyclodextrin; n is an integer from 1 to 100 (e.g., n is an integer from 4 to 80, from 4 to 50, from 4 to 30 or from 4 to 20, or n is 4, 5, 6, 7, 8, 9, 10, 11, 12, 13, 14, 15, 16, 17, 18, 19 or 20); m is an integer from 1 to 1000 (e.g., m is an integer from 1 to 200, from 1 to 100, from 1 to 80, from 2 to 80, from 5 to 70, from 10 to 50, or from 20 to 40); L is a linker, e.g., a linker described herein; and "taxane" is a taxane, e.g., a taxane described herein, e.g., a taxane shown in FIG. 4. In some embodiments, the CDP-microtubule inhibitor conjugate, e.g., the CDP-taxane conjugate, does not have complete loading, e.g., one or more binding sites, e.g., cysteine residues, are not bound to a microtubule inhibitor, e.g., a taxane moiety, e.g., e.g., a taxane described herein, bound with a linker described herein, e.g., the CDP-taxane conjugate comprises one or more subunits having the formulae provided below: 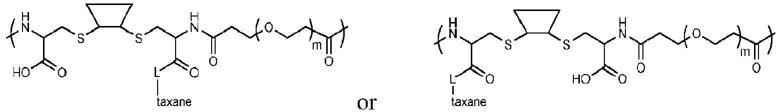 wherein 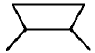 represents a cyclodextrin; m is an integer from 1 to 1000 (e.g., m is an integer from 1 to 200, from 1 to 100, from 1 to 80, from 2 to 80, from 5 to 70, from 10 to 50, or from 20 to 40); L is a linker, e.g., a linker described herein; and "taxane" is a taxane, e.g., a taxane described herein, e.g., a taxane shown in FIG. 4. In some embodiments, the CDP-microtubule inhibitor conjugate, particle or composition e.g., the CDP-taxane conjugate, particle or composition, comprises a mixture of fully-loaded and partially-loaded CDP-microtubule inhibitor conjugates, e.g., CDP-taxane conjugates.

In one embodiment, the CDP-microtubule inhibitor conjugate comprises a subunit of 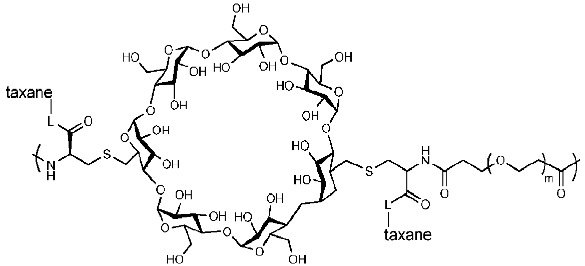 wherein m is an integer from 1 to 1000 (e.g., m is an integer from 1 to 200, from 1 to 100, from 1 to 80, from 2 to 80, from 5 to 70, from 10 to 50, or from 20 to 40); L is a linker, e.g., a linker described herein; and "taxane" is a taxane, e.g., a taxane described herein, e.g., a taxane shown in FIG. 4.

FIG. 4 is a table depicting examples of different CDP-taxane conjugates. The CDP-taxane conjugates in FIG. 4 are represented by the following formula:

CDP-CO-ABX-Taxane

In this formula, CDP is the cyclodextrin-containing polymer shown below (as well as in FIG. 3): 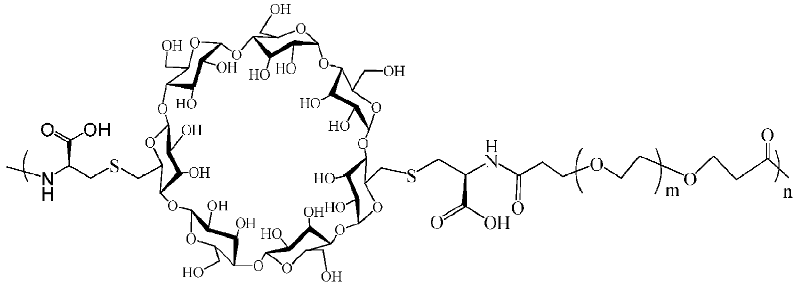 wherein the group 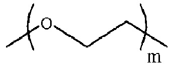 has a Mw of 3400 Da or less and n is at least 4, 5, 6, 7, 8, 9, 10. 11, 12, 13, 14, 15, 16, 17, 18, 19 or 20. Note that the taxane is conjugated to the CDP through the carboxylic acid moieties of the polymer as provided above. Full loading of the taxane onto the CDP is not required. In some embodiments, at least one, e.g., at least 2, 3, 4, 5, 6 or 7, of the carboxylic acid moieties remains unreacted with the taxane after conjugation (e.g., a plurality of the carboxylic acid moieties remain unreacted).

CO represents the carbonyl group of the cysteine residue of the CDP;

A and B represent the link between the CDP and the taxane. Position A is either a bond between linker B and the cysteine acid carbonyl of CDP (represented as a "-" in FIG. 4), a bond between the taxane and the cysteine acid carbonyl of CDP (represented as a "-"in FIG. 4) or depicts a portion of the linker that is attached via a bond to the cysteine acid carbonyl of the CDP. Position B is either not occupied (represented by "-" in FIG. 4) or represents the linker or the portion of the linker that is attached via a bond to the taxane; and

X represents the heteroatom to which the linker is coupled on the taxane.

As provided in FIG. 4, the column with the heading "Taxane" indicates which taxane is included in the CDP-taxane conjugate.

The three columns on the right of the table in FIG. 4 indicate respectively, what, if any, protecting groups are used to protect the indicated position of the taxane, the process for producing the CDP-taxane conjugate, and the final product of the process for producing the CDP-taxane conjugate.

The processes referred to in FIG. 4 are given a letter representation, e.g., Process A, Process B, etc. as seen in the second column from the right. The steps for each these processes respectively are provided below.

Process A: Couple the protected linker of position B to the taxane, deprotect the linker and couple to CDP via the carboxylic acid group of the CDP to afford the 2'-taxane linked to CDP.

Process B: Couple the activated linker of position B to the 2'-hydroxyl of taxane, and couple to CDP containing linker of position A via the linker of A to afford the 2'-taxane linked to CDP.

Process C: Protect the C2' hydroxy group of the taxane, couple the protected linker of position B to the taxane, deprotect the linker and the C2' hydroxy group, and couple to CDP via the carboxylic acid group of the CDP to afford the 7-taxane linked to CDP.

Process D: Protect the C2' hydroxy group of the taxane, couple the activated linker of position B to the 7-hydroxyl of the taxane, deprotect the C2' hydroxy group and couple to CDP containing linker of position A via the linker of A to afford afford the 7-taxane linked to CDP.

As shown specifically in FIG. 4, the CDP-taxane conjugates can be prepared using a variety of methods known in the art, including those described herein. In some embodiments, the CDP-taxane conjugates can be prepared using no protecting groups on the taxane. For taxanes having hydroxyl groups at both the 2' and the 7-positions, one of skill in the art will understand that the 2'-position is more reactive, and therefore when using no protecting groups, the major product of the reaction(s) will be that which is linked via the 2' position.

One or more protecting groups can be used in the processes described above to make the CDP-taxane conjugates described herein. A protecting group can be used to control the point of attachment of the taxane and/or taxane linker to position A. In some embodiments, the protecting group is removed and, in other embodiments, the protecting group is not removed. If a protecting group is not removed, then it can be selected so that it is removed *in vivo* (e.g., acting as a prodrug). An example is hexanoic acid which has been shown to be removed by lipases *in vivo* if used to protect a hydroxyl group in doxorubicin. Protecting groups are generally selected for both the reactive groups of the taxane and the reactive groups of the linker that are not targeted to be part of the coupling reaction. The protecting group should be removable under conditions which will not degrade the taxane and/or linker material. Examples include t-butyldimethylsilyl ("TBDMS") and TROC (derived from 2,2,2-trichloroethoxy chloroformate). Carboxybenzyl ("CBz") can also be used in place of TROC if there is selectivity seen for removal over olefin reduction. This can be addressed by using a group which is more readily removed by hydrogenation such as -methoxybenzyl OCO-. Other protecting groups may also be acceptable. One of skill in the art can select suitable protecting groups for the products and methods described herein.

In some embodiments, the microtubule inhibitor in the CDP-microtubule inhibitor conjugate is an epothilone. In some embodiments, the epothilone in the CDP-epothilone conjugate, particle or composition is an epothilone including, without limitation, ixabepilone, epothilone B, epothilone D, BMS310705, dehydelone, and ZK-Epothilone (ZK-EPO). Other epothilones described herein can also be included in the CDP-epothilone conjugates.

### Epothilones

The term "epothilone," as used herein, refers to any naturally occurring, synthetic, or semi-synthetic epothilone structure, for example, known in the art. The term epothilone also includes structures falling within the generic formulae XX, XXI, XXII, XXIII, XXIV, XXV, and XXVI as provided herein.

Exemplary epothilones include those described generically and specifically herein. In some embodiments, the epothilone is epothilone B, ixabepilone, BMS310705, epothilone D, dehydelone, or sagopilone. The structures of all of these epothilones are provided below:

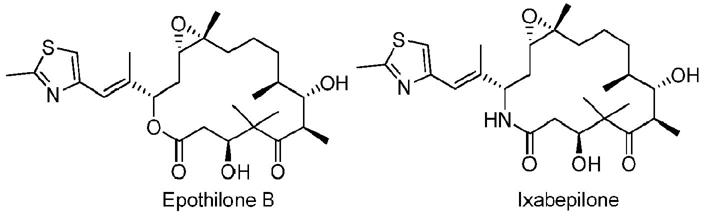

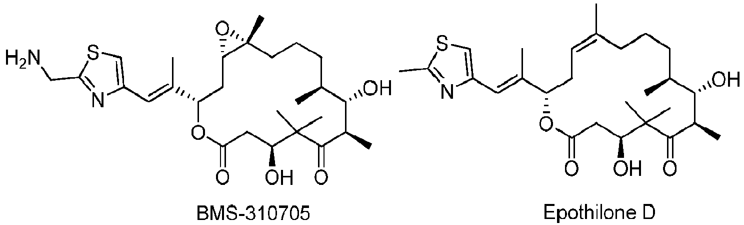

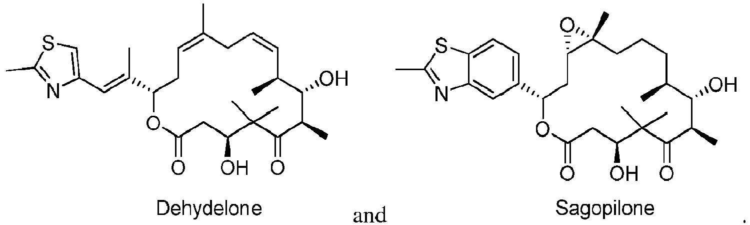

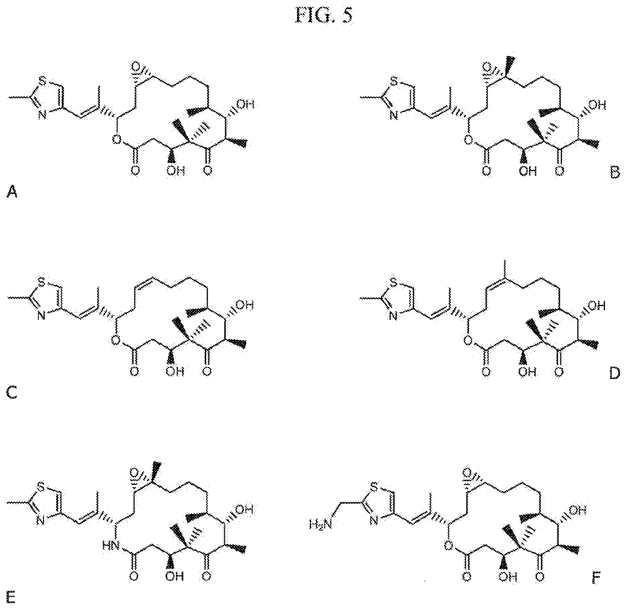

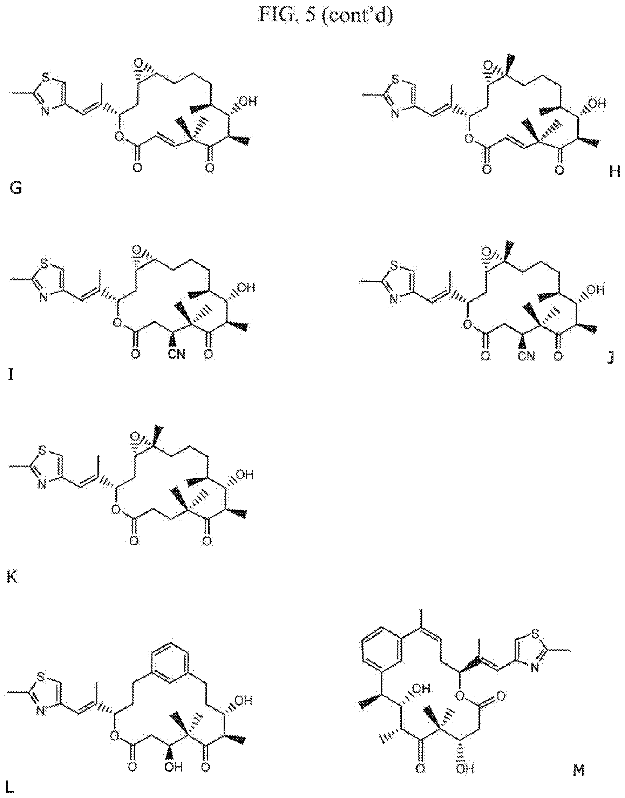

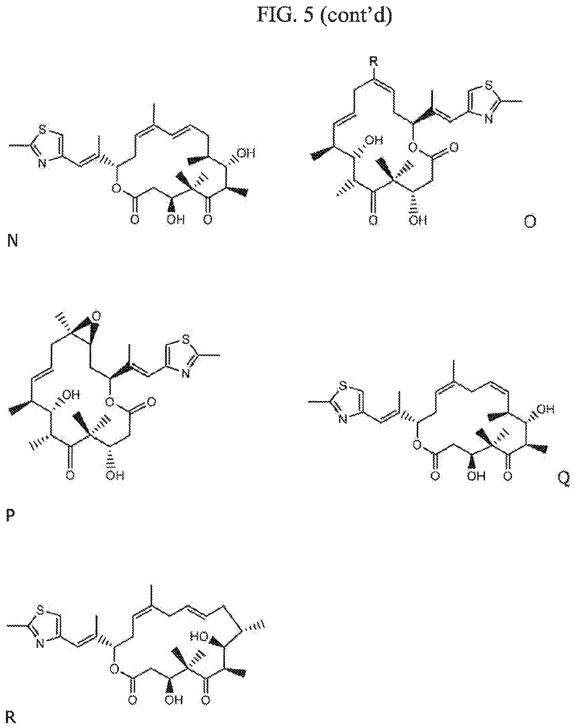

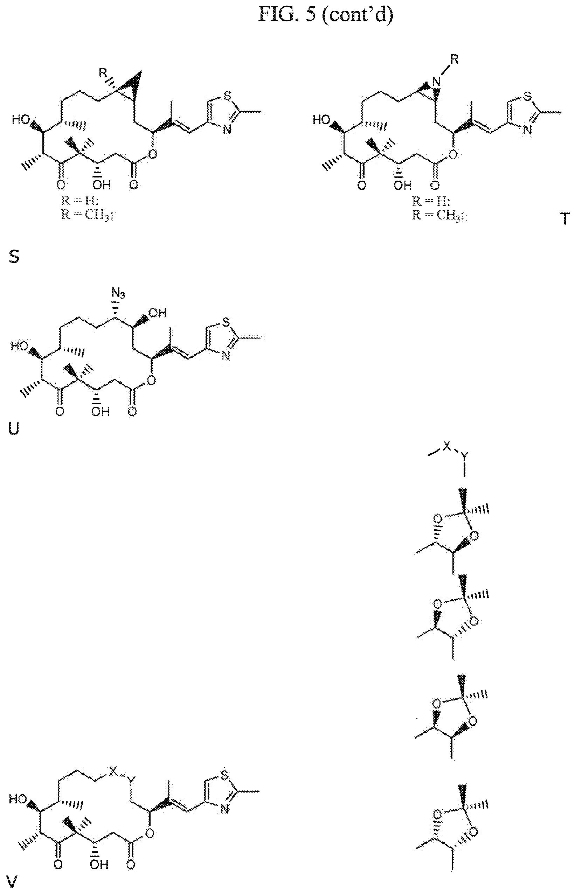

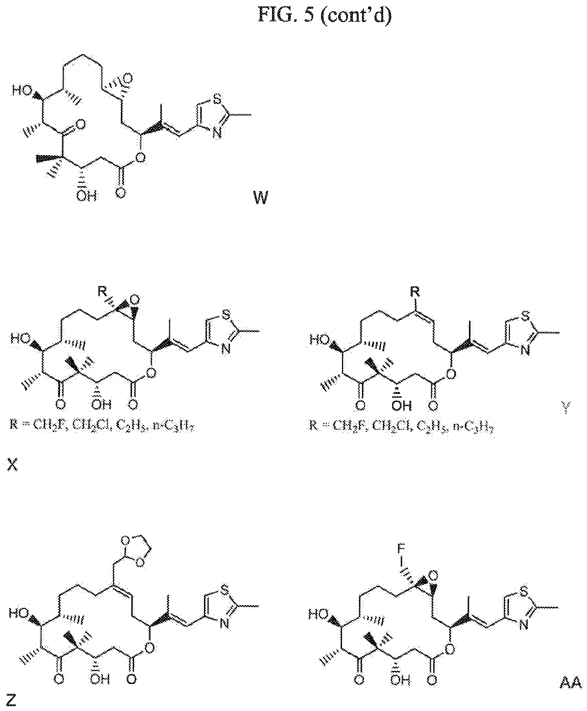

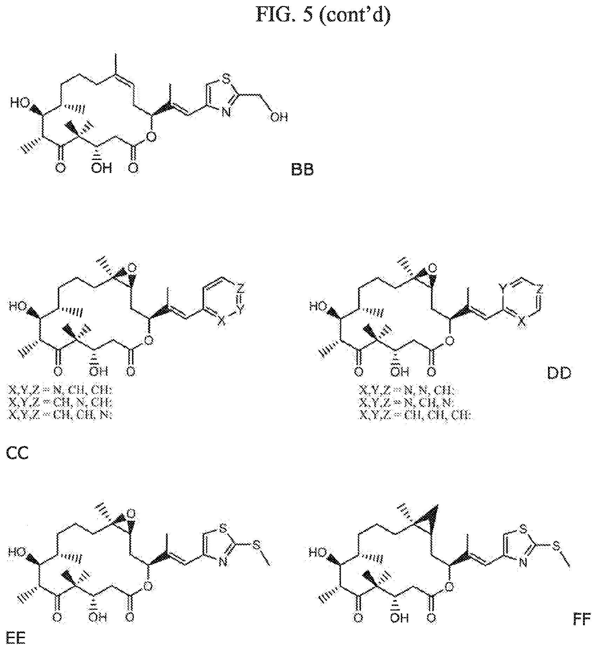

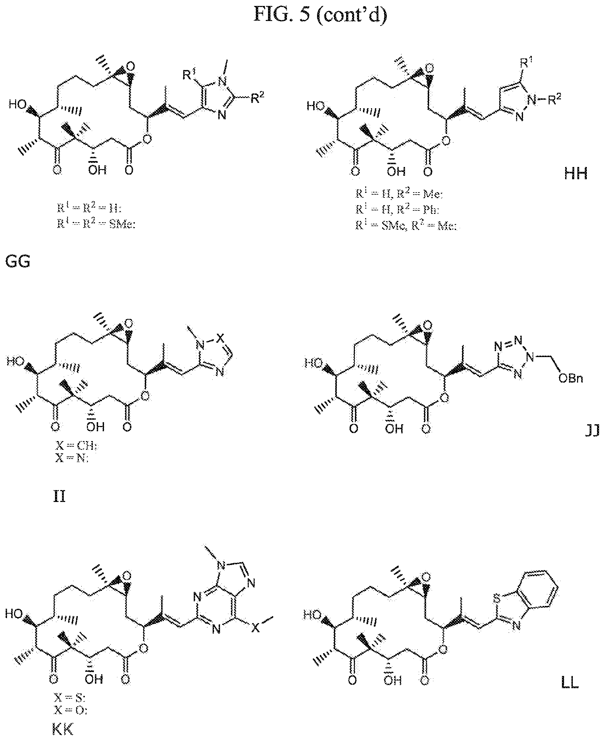

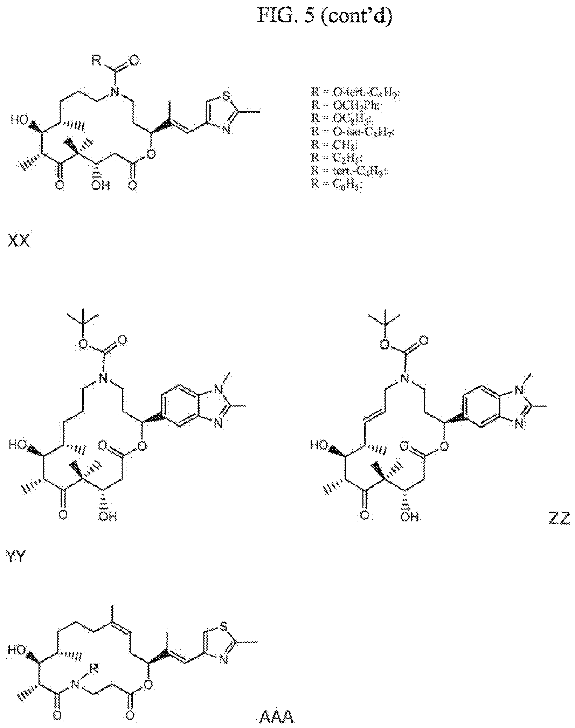

Other exemplary epothilones are also provided in FIG. 5 and disclosed in Altmann et al. "Epothilones as Lead Structures for New Anticancer Drugs-Pharmacology, Fermentation, and Structure-activity-relationships;" Progress in Drug Research (2008) Vol. 66, page 274-334, which is incorporated herein by reference. Additionally, epothilones may be found, for example, in US 7,317,100; US 6,946,561; US 6,350,878; US 6,302,838; US 7,030,147; US 6,387,927; US 6,346,404; US 2004/0038324; US 2009/0041715; US 2007/0129411; US 2005/0271669; US 2008/0139587; US 2004/0235796; US 2005/0282873; US 2006/0089327; WO 2008/071404; WO 2008/019820; WO 2007/121088; WO 1998/08849; EP 1198225; EP 1420780; EP 1385522; EP 1539768; EP 1485090; and EP 1463504, the contents of these references are incorporated herein in their entireties.

Further epothilones may be found, for example, in US 6,410,301; US 7,091,193; US 7,402,421; US 7,067,286; US 6,489,314; US 6,589,968; US 6,893,859; US 7,176,235; US 7,220,560; US 6,280,999; US 7,070,964; US 2005/0148543; US 2005/0215604; US 2003/0134883; US 2008/0319211; US 2005/0277682; US 2005/0020558; US 2005/0203174; US 20020045609, US 2004/0167097; US 2004/0072882; US 2002/0137152; WO 2009/064800; and WO 2002/012534, the contents of these references are incorporated herein in their entireties.
Further epothilones may be found, for example, in US 6,537,988; US 7,312,237; US 7,022,330; US 6,670,384; US 6,605,599; US 7,125,899; US 6,399,638; US 7,053,069; US 6,936,628; US 7,211,593; US 6,686,380; US 6,727,276; US 6,291,684; US 6,780,620; US 6,719,540; US 2009/0004277; US 2007/0276018; WO 2004/078978; and EP 1157023, the contents of these references are incorporated herein in their entireties. Further epothilones may be found, for example, in US 2008/0146626; US 2009/0076098; WO 2009/003706 and WO 2009/074274, the contents of these references are incorporated herein in their entireties.

Further epothilones may be found, for example, in US 7,169,930; US 6,294,374; US 6,380,394; and US 6,441,186, the contents of these references are incorporated herein in their entireties.

Further epothilones may be found, for example, in US 7,119,071; and German Application Serials Nos. DE 197 13 970.1, DE 100 51 136.8, DE 101 34 172.5, and DE 102 32 094.2, the contents of these references are incorporated herein in their entireties. In some embodiments, the epothilone is attached to a targeting moiety such as a folate moiety. In some embodiments, the targeting moiety (e.g., a folate) is attached to a functional group on the epothilone such as a hydroxyl group or an amino group where appropriate. In some embodiments, the folate is attached to the epothilone directly. In some embodiments, the folate is attached to the epothilone via a linker. Epofolate (BMS-753493) is an example an epothilone attached to a folate, see, for example, U.S. 7,033,594, which is incorporated herein by reference.

In one embodiment, the epothilone is a compound of formula (XX) 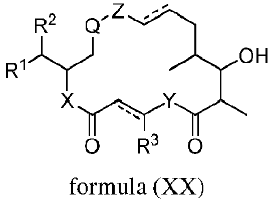 wherein
R¹ is aryl, heteroaryl, arylalkenyl or heteroarylalkenyl; each of which is optionally substituted with 1-3 R⁸;
R² is H or alkyl (e.g., a methyl); or
R¹ and R², when taken together with the carbon to which they are attached, form an aryl or a heteroaryl moiety optionally substituted with 1-3 R⁸;
R³ is H, OH, NH₂, or CN;
X is O or NR⁴;
R⁴ is H, alkyl, -C(O)Oalkyl, -C(O)Oarylalkyl, -C(O)NR⁵alkyl, -C(O)NR⁵arylalkyl,-C(O)alkyl, -C(O)aryl or arylalkyl;
Y is CR⁵R⁶, O or NR⁷;
each of R⁵ and R⁶ is independently H or alkyl (e.g., methyl);
R⁷ is H, alkyl, -C(O)Oalkyl, -C(O)Oarylalkyl, -C(O)NR⁵alkyl, -C(O) NR⁵arylalkyl,-C(O)alkyl, -C(O)aryl or arylalkyl;
each R⁸, for each occurrence, is independently alkyl, aminoalkyl, hydroxyalkyl, alkylthiol, aryl, arylalkyloxyalkyl or alkoxy;
Q-Z, when taken together, form 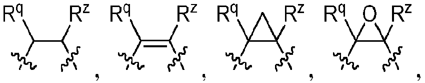 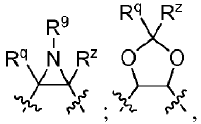 heteroarylenyl, C(O)NR⁴, NR⁴C(O), CR⁵R⁶NR⁴, or NR⁴CR⁵R⁶;
R^{q} is H, alkyl (e.g., methyl) or hydroxy;
R^{z} is H, alkyl (e.g., methyl), haloalkyl (e.g., CF₃), heterocyclylalkyl or N₃;
R⁹ is H, alkyl, -C(O)Oalkyl, -C(O)Oarylalkyl, -C(O)NR⁵alkyl, -C(O) NR⁵arylalkyl,-C(O)alkyl, -C(O)aryl or arylalkyl; and
each 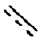 , for each occurrence, is independently a single or double bond.

In some embodiments, R¹ is 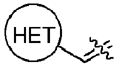 optionally substituted with 1-3 R⁸.

In some embodiments, HET is a five membered ring heteroaryl optionally substituted with 1-3 R⁸.

In some embodiments, HET is a thiazolyl optionally substituted with 1-3 R⁸. In some embodiments, HET is substituted with alkyl (e.g., methyl), aminoalkyl (e.g., aminomethyl), alkylthiol (e.g., methylthiol), hydroxyalkyl (e.g., hydroxymethyl), alkoxy (e.g., methoxy) or aryl (e.g., phenyl).

In some embodiments, HET is substituted with alkyl (e.g., methyl) or amino alkyl.

In some embodiments, HET is 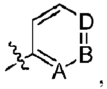 wherein each of A, B and D is independently CH or N. In some embodiments, A is N, B is CH and D is CH. In some embodiments, A is CH, B is N and D is CH. In some embodiments, A is CH, B is CH and D is N.

In some embodiments, HET is 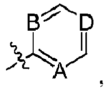 wherein each of A, B and D is independently CH or N. In some embodiments, A is N, B is N and D is CH. In some embodiments, A is N, B is CH and D is N. Tn some embodiments, A is CH, B is CH and D is CH.

In some embodiments, HET is 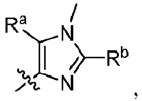 wherein each R^{a} and R^{b} is independently H or -SMe.

In some embodiments, HET is 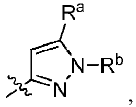 wherein each R^{a} is H, alkyl or -Salkyl; and R^{b} is H, alkyl (e.g., methyl) or aryl (e.g., phenyl).

In some embodiments, HET is 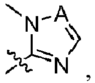 wherein A is CH or N.

In some embodiments, HET is

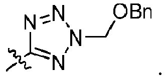

In some embodiments, HET is 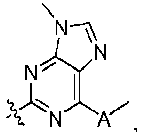 wherein A is S or O.

In some embodiments, HET is

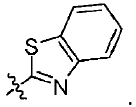

In some embodiments R² is H.

In some embodiments, R² is alkyl (e.g., methyl).

In some embodiments, R¹ and R², when taken together with the carbon to which they are attached, form an aryl or a heteroaryl moiety optionally substituted with 1-3 R⁸.

In some embodiments, R¹ and R², when taken together with the carbon to which they are attached, form a heteroaryl moiety optionally substituted with 1-3 R⁸.

In some embodiments, the heteroaryl moiety is a bicyclic heteroaryl moiety.

In some embodiments, R¹ and R², when taken together with the carbon to which they are attached, are

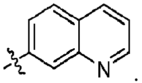

In some embodiments, R¹ and R², when taken together with the carbon to which they are attached, are

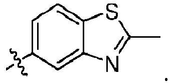

In some embodiments, R¹ and R², when taken together with the carbon to which they are attached, are

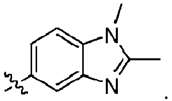

In some embodiments, R¹ and R², when taken together with the carbon to which they are attached, are 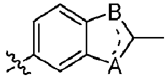 , wherein A is N and B is S or wherein A is S and B is N.

In some embodiments, R¹ and R², when taken together with the carbon to which they are attached, are 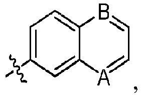 wherein A is N and B is CH or wherein A is CH and B is N.

In some embodiments, 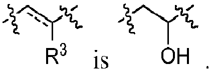 In some embodiments, 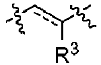 is

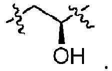

In some embodiments, 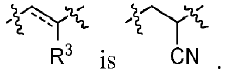 In some embodiments, 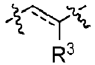 is

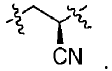

In some embodiments,

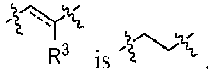

In some embodiments,

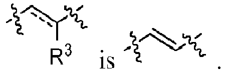

In some embodiments, X is O.

In some embodiments, X is NR⁴ (e.g., NH).

In some embodiments, Y is CR⁵R⁶. In some embodiments, Y is 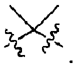 In some embodiments, Y is CH₂.

In some embodiments, Y is NR⁷ (e.g., NH or NMe).

In some embodiments, Q-Z, when taken together, form 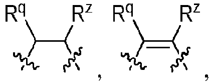 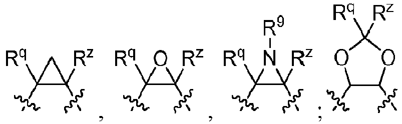 or heteroarylenyl.

In some embodiments, Q-Z, when taken together, form 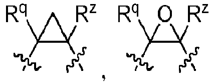 or

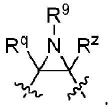

In some embodiments, Q-Z, when taken together, form 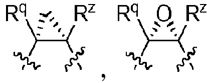 or

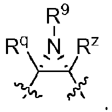

In some embodiments, Q-Z, when taken together, form 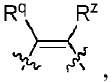 wherein R^{q} is H and R^{z} is H or alkyl (e.g., methyl).

In some embodiments, Q-Z, when taken together, form 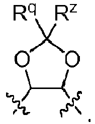 In some embodiments, both R^{q} and R^{z} are methyl. In some embodiments, 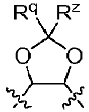 is selected from 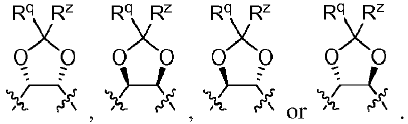 In some embodiments, both R^{q} and R^{z} are methyl.

In some embodiments, Q-Z, when taken together, form a heteroarylenyl. In some embodiments, Q-Z, when taken together, form

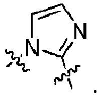

In some embodiments, Q-Z, when taken together, form C(O)NR⁴. In some embodiments, R⁴ is H or alkyl (e.g., methyl or ethyl).

In some embodiments, Q-Z, when taken together, form NR⁴C(O). In some embodiments, R⁴ is H or alkyl (e.g., methyl or ethyl).

Tn some embodiments, Q-Z, when taken together, form CH₂NR⁴. In some embodiments, R⁴ is H, alkyl, -C(O)Oalkyl, -C(O)Oarylalkyl, -C(O)alkyl, -C(O)aryl or arylalkyl. In some embodiments, R⁴ is -C(O)Oalkyl, -C(O)Oarylalkyl, -C(O)alkyl, -C(O)aryl or arylalkyl.

In some embodiments, Q-Z, when taken together, form NR⁴CH₂. In some embodiments, R⁴ is H, alkyl, -C(O)Oalkyl, -C(O)Oarylalkyl, -C(O)alkyl, -C(O)aryl or arylalkyl. In some embodiments, R⁴ is -C(O)Oalkyl, -C(O)Oarylalkyl, -C(O)alkyl, -C(O)aryl or arylalkyl.

In some embodiments, the compound of formula (XX) is a compound of formula (XXa)

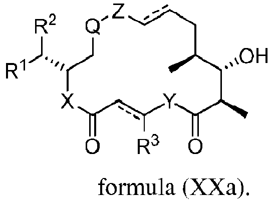

In some embodiments, the compound of formula (XX) is a compound of formula (XXb)

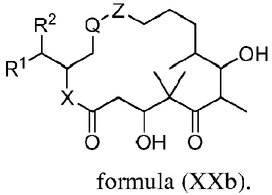

In some embodiments, the compound of formula (XX) is a compound of formula (XXc) 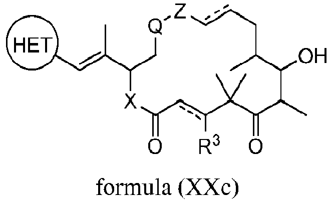 wherein HET is an optionally substituted heteroaryl.

In some embodiments, HET is an optionally substituted 5 membered ring.

In some embodiments, the compound of formula (XX) is a compound of formula (XXd)

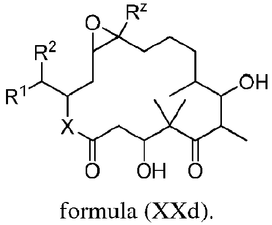

In some embodiments, the compound of formula (XX) is a compound of formula (XXe)

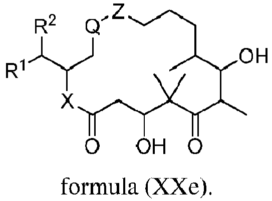

In some embodiments, the compound of formula (XX) is a compound of formula (XXf)

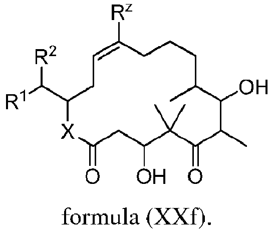

In some embodiments, the compound of formula (XX) is a compound of formula (XXg)

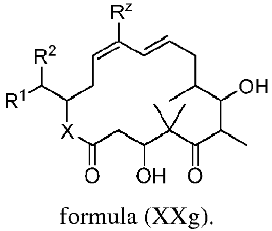

In one embodiment, the epothilone is a compound of formula (XXI) 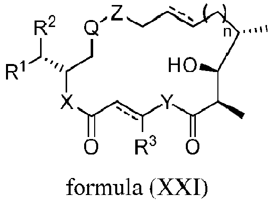 wherein
R¹ is aryl, heteroaryl, arylalkenyl, or heteroarylalkenyl; each of which is optionally substituted with 1-3 R⁸;
R² is H or alkyl (e.g., methyl); or
R¹ and R², when taken together with the carbon to which they are attached, form an aryl or a heteroaryl moiety optionally substituted with 1-3 R⁸;
R³ is H, OH, NH₂ or CN;
X is O or NR⁴;
R⁴ is H, alkyl, -C(O)Oalkyl, -C(O)Oarylalkyl, -C(O)NR⁵alkyl, -C(O) NR⁵arylalkyl,-C(O)alkyl, -C(O)aryl or arylalkyl;
Y is CR⁵R⁶, O or NR⁷;
each of R⁵ and R⁶ is independently H or alkyl (e.g., methyl);
R⁷ is H, alkyl, -C(O)Oalkyl, -C(O)Oarylalkyl, -C(O)NR⁵alkyl, -C(O) NR⁵arylalkyl,-C(O)alkyl, -C(O)aryl or arylalkyl;
each R⁸, for each occurrence, is independently alkyl, aminoalkyl, hydroxyalkyl, alkylthiol, aryl, arylalkyloxyalkyl or alkoxy;
Q-Z, when taken together, form 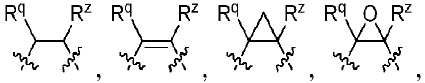 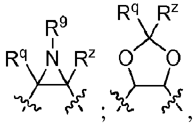 heteroarylenyl, C(O)NR⁴, NR⁴C(O), CR⁵R⁶NR⁴, or NR⁴CR⁵R⁶NR⁴;
R^{q} is H, alkyl (e.g., methyl) or hydroxy;
R^{z} is H, alkyl (e.g., methyl), haloalkyl (e.g., CF₃), heterocyclylalkyl or N₃;
R⁹ is H, alkyl, -C(O)Oalkyl, -C(O)Oarylalkyl, -C(O)NR⁵alkyl, -C(O) NR⁵arylalkyl,-C(O)alkyl, -C(O)aryl or arylalkyl;
each 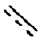 , for each occurrence, is independently a single or double bond; and n is 0, 1 or 2.

In some embodiments, R¹ is 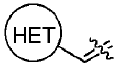 optionally substituted with 1-3 R⁸. In some embodiments, HET is a five membered ring heteroaryl optionally substituted with 1-3 R⁸. In some embodiments, HET is a thiazolyl optionally substituted with 1-3 R⁸. In some embodiments, HET is substituted with alkyl (e.g., a methyl), aminoalkyl (e.g., aminomethyl), alkylthiol (e.g., methylthiol), hydroxyalkyl (e.g., hydroxymethyl), alkoxy (e.g., methoxy) or aryl (e.g., phenyl). In some embodiments, HET is substituted with alkyl (e.g., methyl) or aminoalkyl.

In some embodiments, HET is 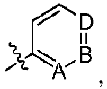 wherein each of A, B and D is independently CH or N. In some embodiments, A is N, B is CH and D is CH. In some embodiments, A is CH, B is N and D is CH. In some embodiments, A is CH, B is CH and D is N.

In some embodiments, HET is 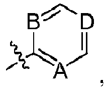 wherein each of A, B and D is independently CH or N. In some embodiments, A is N, B is N and D is CH. In some embodiments, A is N, B is CH and D is N. In some embodiments, A is CH, B is CH and D is CH.

In some embodiments, HET is 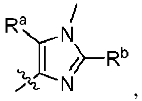 wherein each R^{a} and R^{b} is independently -H or -SMe.

In some embodiments, HET is 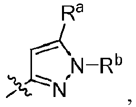 wherein each R^{a} is H, alkyl or -Salkyl; and R^{b} is H, alkyl (e.g., methyl) or aryl (e.g., phenyl).

In some embodiments, HET is 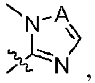 wherein A is CH or N.

In some embodiments, HET is

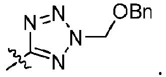

In some embodiments, HET is 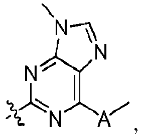 wherein A is S or O.

In some embodiments, HET is

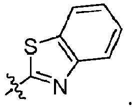

In some embodiments R² is H.

In some embodiments, R² is alkyl (e.g., methyl).

In some embodiments, R¹ and R², when taken together with the carbon to which they are attached, form an aryl or a heteroaryl moiety optionally substituted with 1-3 R⁸. In some embodiments, the heteroaryl moiety is a bicyclic heteroaryl moiety.

In some embodiments, R¹ and R², when taken together with the carbon to which they are attached, are

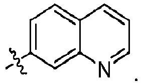

In some embodiments, R¹ and R², when taken together with the carbon to which they are attached, are

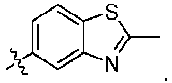

In some embodiments, R¹ and R², when taken together with the carbon to which they are attached, are

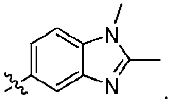

In some embodiments, R¹ and R², when taken together with the carbon to which they are attached, are 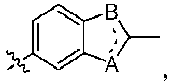 wherein A is N and B is S or wherein A is S and B is N.

In some embodiments, R¹ and R², when taken together with the carbon to which they are attached, are 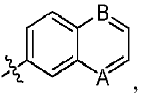 wherein A is N and B is CH or wherein A is CH and B is N.

In some embodiments, 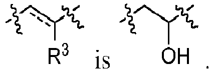 In some embodiments, 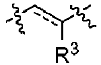 is

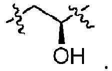

In some embodiments, 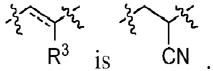 In some embodiments, 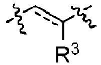 is

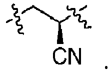

In some embodiments,

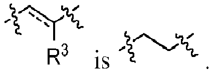

In some embodiments,

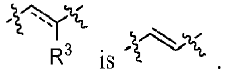

In some embodiments, X is O.

In some embodiments, X is NR⁴ (e.g., NH).

In some embodiments, Y is CR⁵R⁶.

In some embodiments, Y is

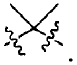

In some embodiments, Y is CH₂.

In some embodiments, Y is NR⁷ (e.g., NH or NMe).

In some embodiments, Q-Z, when taken together, form 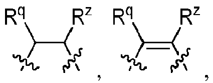 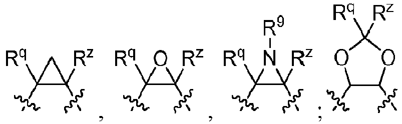 or heteroarylenyl.

In some embodiments, Q-Z, when taken together, form 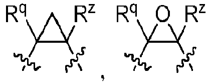 or 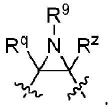 In some embodiments, Q-Z, when taken together, form 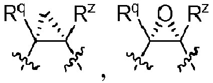 or

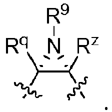

In some embodiments, Q-Z, when taken together, form 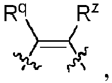 wherein R^{q} is H and R^{z} is H or alkyl (e.g., methyl).

In some embodiments, Q-Z, when taken together, form 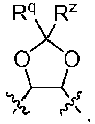 In some embodiments, both R^{q} and R^{z} are methyl.

In some embodiments, 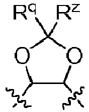 is selected from 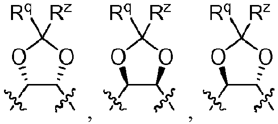 or 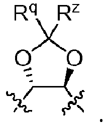 In some embodiments, both R^{q} and R^{z} are methyl.

In some embodiments, Q-Z, when taken together, form a heteroarylenyl. In some embodiments, Q-Z, when taken together, form

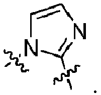

In some embodiments, Q-Z, when taken together, form C(O)NR⁴. In some embodiments, R⁴ is H or alkyl (e.g., methyl or ethyl).

In some embodiments, Q-Z, when taken together, form NR⁴C(O). In some embodiments, R⁴ is H or alkyl (e.g., methyl or ethyl).

Tn some embodiments, Q-Z, when taken together, form CH₂NR⁴. In some embodiments, R⁴ is H, alkyl, -C(O)Oalkyl, -C(O)Oarylalkyl, -C(O)alkyl, -C(O)aryl or arylalkyl. In some embodiments, R⁴ is -C(O)Oalkyl, -C(O)Oarylalkyl, -C(O)alkyl, -C(O)aryl or arylalkyl.

In some embodiments, Q-Z, when taken together, form NR⁴CH₂. In some embodiments, R⁴ is H, alkyl, -C(O)Oalkyl, -C(O)Oarylalkyl, -C(O)alkyl, -C(O)aryl or arylalkyl. In some embodiments, R⁴ is -C(O)Oalkyl, -C(O)Oarylalkyl, -C(O)alkyl, -C(O)aryl or arylalkyl.

In some embodiments, n is 0.

In some embodiments, n is 1.

In some embodiments, the compound of formula (XXI) is a compound of formula (XXIa)

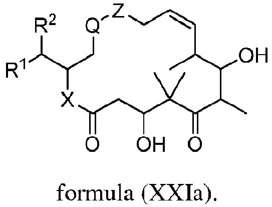

In some embodiments, the compound of formula (XXI) is a compound of formula (XXIb)

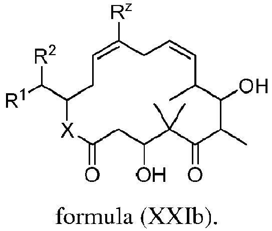

In some embodiments, the compound of formula (XXI) is a compound of formula (XXIc)

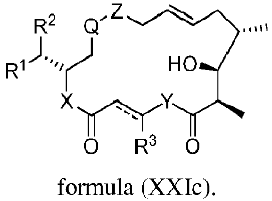

In some embodiments, the epothilone is a compound of formula (XXII) 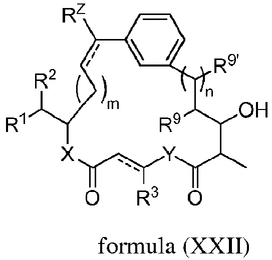 wherein,
R¹ is aryl, heteroaryl, arylalkenyl or heteroarylalkenyl; each of which is optionally substituted with 1-3 R⁸;
R² is H or alkyl (e.g., methyl); or
R¹ and R², when taken together with the carbon to which they are attached, form an aryl or a heteroaryl moiety optionally substituted with 1-3 R⁸;
R³ is H, OH, NH₂, or CN;
X is O or NR⁴;
R⁴ is H,alkyl, -C(O)Oalkyl, -C(O)Oarylalkyl, -C(O)NR⁵alkyl, -C(O) NR⁵arylalkyl,-C(O)alkyl, -C(O)aryl or arylalkyl;
Y is CR⁵R⁶, O or NR⁷;
each of R⁵ and R⁶ is independently H or alkyl (e.g., methyl);
R⁷ is H, alkyl, -C(O)Oalkyl, -C(O)Oarylalkyl, -C(O)NR⁵alkyl, -C(O) NR⁵arylalkyl,-C(O)alkyl, -C(O)aryl or arylalkyl;
each R⁸, for each occurrence, is independently alkyl, aminoalkyl or hydroxyalkyl;
each R⁹ and R⁹, is independently H or alkyl (e.g., methyl);
R^{z} is H, alkyl (e.g., methyl), haloalkyl (e.g., CF₃), heterocyclylalkyl or N₃;
each 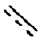 , for each occurrence, is independently a single or double bond;
m is 0, 1 or 2; and
n is 0, 1 or 2.

In some embodiments, R¹ is 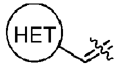 optionally substituted with 1-3 R⁸. In some embodiments, HET is a five membered ring heteroaryl optionally substituted with 1-3 R⁸. In some embodiments, HET is thiazolyl optionally substituted with 1-3 R⁸. In some embodiments, HET is substituted with alkyl (e.g., methyl), aminoalkyl (e.g., aminomethyl), alkylthiol (e.g., methylthiol), hydroxyalkyl (e.g., hydroxymethyl), alkoxy (e.g., methoxy) or aryl (e.g., phenyl). In some embodiments, HET is substituted with alkyl (e.g., methyl) or amino alkyl.

In some embodiments, HET is 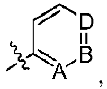 wherein each of A, B and D is independently CH or N. In some embodiments, A is N, B is CH and D is CH. In some embodiments, A is CH, B is N and D is CH. In some embodiments, A is CH, B is CH and D is N.

In some embodiments, HET is 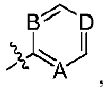 wherein each of A, B and D is independently CH or N. In some embodiments, A is N, B is N and D is CH. In some embodiments, A is N, B is CH and D is N. In some embodiments, A is CH, B is CH and D is CH.

In some embodiments, HET is 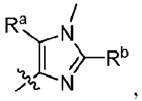 wherein each R^{a} and R^{b} is independently H or -SMe.

In some embodiments, HET is 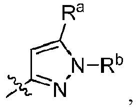 wherein each R^{a} is H, an alkyl or -Salkyl; and R^{b} is H, alkyl (e.g., methyl) or aryl (e.g., phenyl).

In some embodiments, HET is 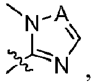 wherein A is CH or N.

In some embodiments, HET is

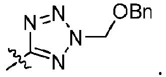

In some embodiments, HET is 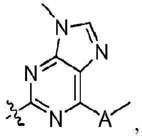 wherein A is S or O.

In some embodiments, HET is

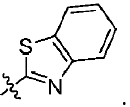

In some embodiments R² is H.

In some embodiments, R² is alkyl (e.g., methyl).

In some embodiments, R¹ and R², when taken together with the carbon to which they are attached, form an aryl or a heteroaryl moiety optionally substituted with 1-3 R⁸.

In some embodiments, the heteroaryl moiety is a bicyclic heteroaryl moiety. In some embodiments, R¹ and R², when taken together with the carbon to which they are attached, are

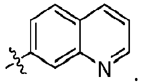

In some embodiments, R¹ and R², when taken together with the carbon to which they are attached, are

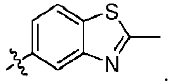

In some embodiments, R¹ and R², when taken together with the carbon to which they are attached, are

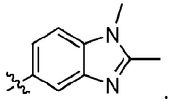

In some embodiments, R¹ and R², when taken together with the carbon to which they are attached, are 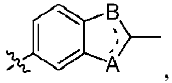 wherein A is N and B is S or wherein A is S and B is N.

In some embodiments, R¹ and R², when taken together with the carbon to which they are attached, are 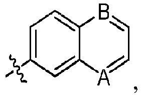 wherein A is N and B is CH or wherein A is CH and B is N.

In some embodiments, 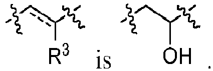 In some embodiments, 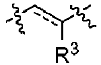 is

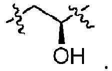

In some embodiments, 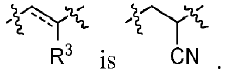 In some embodiments, 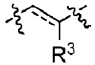 is

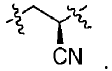

In some embodiments,

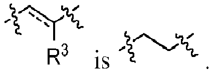

In some embodiments,

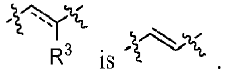

In some embodiments, X is O.

In some embodiments, X is NR⁴ (e.g., NH).

In some embodiments, Y is CR⁵R⁶. In some embodiments, Y is 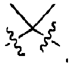 In some embodiments, Y is CH₂.

In some embodiments, Y is NR⁷ (e.g., NH or NMe).

In some embodiments, R⁹ is H.

In some embodiments, R⁹ is Me.

In some embodiments, 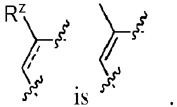 In some embodiments, m is 1.

In some embodiments, 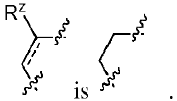 In some embodiments, m is 0.

In some embodiments, n is 0.

In some embodiments,

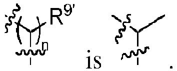

In some embodiments, compound of formula (XXII) is a compound of formula (XXIIa)

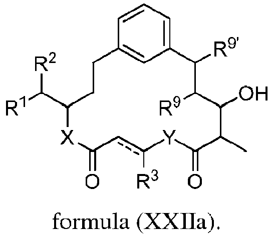

In some embodiments, compound of formula (XXII) is a compound of formula (XXIIb)

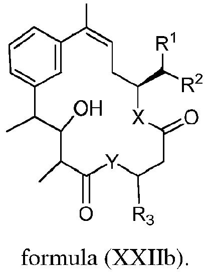

In some embodiments, the epothilone is a compound of formula (XXIII): 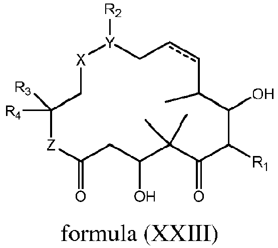 wherein
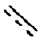 represents a single or double bond;
R₁ is C₁₋₆alkyl, C₂₋₆alkynyl or C₂₋₆alkenyl radical;
R₂ is H or C₁₋₆alkyl radical;
X-Y is selected from the following groups: 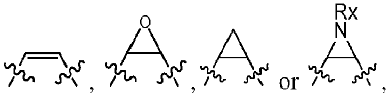 preferably 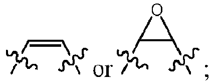
Z is O or NR_{X}, wherein R_{X} is hydrogen, alkyl, alkenyl, alkynyl, heteroalkyl, aryl, heteroaryl, cycloalkyl, alkylcycloalkyl, heteroalkylcycloalkyl, heterocycloalkyl, aralkyl or heteroaralkyl group;
R₃ is halogen atom or C₁₋₆alkyl, C₂₋₆alkenyl or C₁₋₆-heteroalkyl radical;
R₄ is bicycloaryl, bicycloheteroaryl or a group of formula -C(R₅)=CHR₆;
R₅ is H or methyl; and
R₆ is an optionally substituted aryl or a heteroaryl group.

In certain embodiments, R₄ is

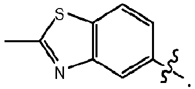

In some embodiments, Z is O. In some embodiments, Z is NH.

In certain embodiments, the compound of formula (XXIII) can be represented by the following structures:

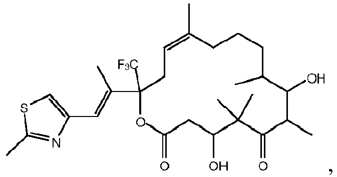

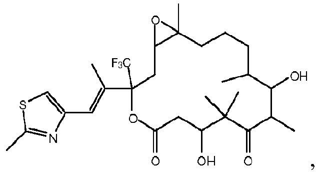

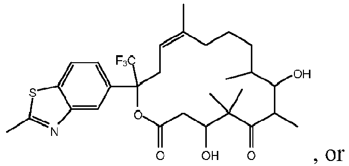

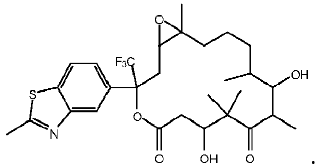

In some embodiments, the epothilone is a compound of formula (XXIV): 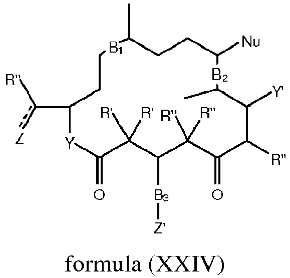 wherein
B₁, B₂, B₃ are selected from single bonds; double bonds in the E(trans) form, the Z(cis) form or as an E/Z mixture; epoxide rings in the E(trans) form, the Z(cis) form or an E/Z mixture; aziridine rings in the E(trans) form, the Z(cis) form or an E/Z mixture; cyclopropane rings in the E(trans) form, the Z(cis) form or an E/Z mixture; and/or combinations thereof; and being preferably selected from single and double bonds; and particularly preferably being selected from B₁ as Z double bonds or epoxide and B₂ and B₃ as single bond;
R is selected from H, alkyl, aryl, aralkyl (such as -CH₂-aryl, -C₂ H4-aryl and the like), alkenyl (such as vinyl), cycloalkyl (preferably a 3- to 7-membered cycloalkyl), CHₙF₃₋ₙ wherein n=0 to 3, oxacycloalkyl (preferably a 3- to 7-membered oxacycloalkyl) and/or combinations thereof. Preferably R is selected from H, methyl, ethyl, phenyl, benzyl and combinations thereof, and more preferably R is selected from H, methyl, ethyl and combinations thereof;
R' is selected from the same group as R, and is preferably H;
R" is selected from the same group as R, and is preferably methyl;
Y is selected from S, NH, N-PG, NR and O; preferably Y is selected from NH, N-PG, NR and O, and more preferably Y is O;
Y' is selected from H, OH, OR, O-PG, NH₂, NR₂, N(PG)₂, SR and SH; preferably Y' is O-PG and/or OH;
Nu is selected from R, O-PG, OR, N(PG)₂, NR₂, S-PG, SR, SeR, CN, N₃, aryl and heteroaryl; Nu is preferably selected from R, O-PG, OR, N(PG)₂ and NR₂, and more preferably Nu is H;
Z is selected from -OH, -O-PG, -OR, =O, =N-Nu, =CH-heteroaryl, =CH-aryl and =PR₃, where all previously mentioned double bound groups may be present in the E(trans) form, the Z(cis) form or as an E/Z mixture; preferably Z is =CH-heteroaryl; and more preferably Z is selected from =O, (E)-(2-methylthiazol-4-yl)-CH= and (E)-(2-methyloxazol-4-yl)-CH=;
Z' is selected from O, OH, OR, O-PG, N(H)₁₋₂, N(R)₁₋₂, N(PG)₁₋₂, SR, S-PG and R; preferably Z' is O, O-PG and/or OR;
B₃ is selected from single or double bonds in the E(trans) form, the Z(cis) form or as an E/Z mixture; preferably B₃ is selected from single and double bonds with heteroatoms such as O, S and N; and more preferably B₃ is a single bond to O-PG and/or OH;
PG, as referred to herein, is a protecting group, and is preferably selected from allyl, methyl, t-butyl (preferably with electron withdrawing group), benzyl, silyl, acyl and activated methylene derivative (e.g., methoxymethyl), alkoxyalkyl or 2-oxacycloalkyl. Exemplary protecting groups for alcohol and amines include trimethylsilyl, triethylsilyl, dimethyl-tert-butylsilyl, acetyl, propionyl, benzoyl, or a tetrahydropyranyl protecting group. Protecting groups can also be used to protect two neighboring groups (e.g.,-CH(OH)-CH(OH)-), or bivalent groups (PG₂). Such protecting groups can form a ring such as a 5- to 7-membered ring. Exemplary protecting groups include succinyl, phthalyl, methylene, ethylene, propylene, 2,2-dimethylpropa-1,3-diyl, and acetonide. Any combination of protecting groups described herein can be used as determined by one of skill in the art.

In some embodiments, the epothilone is a compound of formula (XXV): 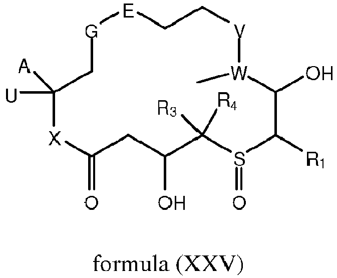 wherein
A is heteroalkyl, heterocycloalkyl, heteroalkylcycloalkyl, heteroaryl, heteroaralkenyl or heteroaralkyl group;
U is hydrogen, halogen, alkyl, heteroalkyl, heterocycloalkyl, heteroalkylcycloalkyl, heteroaryl or heteroaralkyl;
G-E is selected from the following groups, 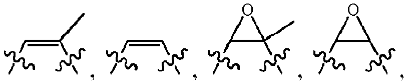 or is part of an optionally substituted phenyl ring;
R₁ is C₁-C₄-alkyl, C₂-C₄-alkenyl, C₂-C₄-alkynyl, or C₃-C₄-cycloalkyl group;
V-W is selected from the group consisting of CH₂CH or CH=C;
X is oxygen or a group of the formula NR₂, wherein R₂ is hydrogen, alkyl, alkenyl, alkynyl, heteroalkyl, aryl, heteroaryl, cycloalkyl, alkylcycloalkyl, heteroalkylcycloalkyl, heterocycloalkyl, aralkyl, or heteroaralkyl; and
each of R₃ and R₄ independently from each other, is hydrogen, C₁-C₄-alkyl or R₃ and R₄ together are part of a cycloalkyl group with 3 or 4 ring atoms.

In certain embodiments of formula (XXV), A is a group of Formula (XXVII) or (XXVIII), 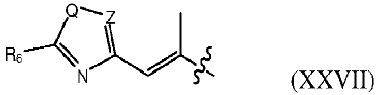 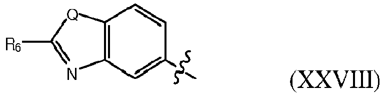 wherein
Q is sulfur, oxygen or NR₇ (preferably oxygen or sulfur), wherein R₇ is hydrogen, C₁-C₄ alkyl or C₁-C₄ heteroalkyl;
Z is nitrogen or CH (preferably CH); and
R₆ is OR₈, NHR₈, C₁-C₄ alkyl, C₁-C₄ alkenyl, C₁-C₄ alkynyl or C₁-C₆ heteroalkyl (preferably methyl, CH₂OR₈ or CH₂NHR₈), wherein R₈ is hydrogen, C₁-C₄ alkyl or C₁-C₄ heteroalkyl (preferably hydrogen).

In some embodiments, the epothilone is a compound of formula (XXVI): 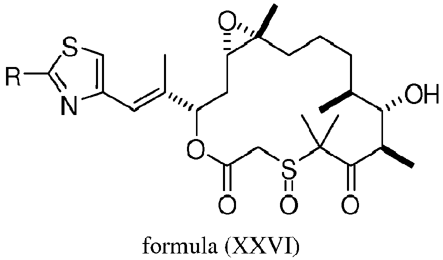 wherein R is selected from OR¹, NHR¹, alkyl, alkenyl, alkynyl and heteroalkyl (e.g., CH₂OR¹ or CH₂NHR¹) and R¹ is selected from hydrogen, C₁₋₄ alkyl and C₁₋₄ heteroalkyl (preferably hydrogen).

In certain embodiments, R is selected from methyl, CH₂OH and CH₂NH₂. Preparation of naturally occurring and semi-synthetic epothilones and corresponding derivatives is known in the art. Epothilones A & B were first extracted from *Sorangium cellulosum* So ce90 which exists at the German Collection of Microorganisms as DMS 6773 and DSM 11999. It has been reported that DSM 6773 allegedly displays increased production of epothilones A and B over the wild type strain. Representative fermentation conditions for Sorangium are described, for example, in U.S. Patent 6,194,181 and various international PCT publications including WO 98/10121, WO 97/19086, WO 98/22461 and WO 99/42602. Methods of preparing epothilones are also described in WO 93/10121.

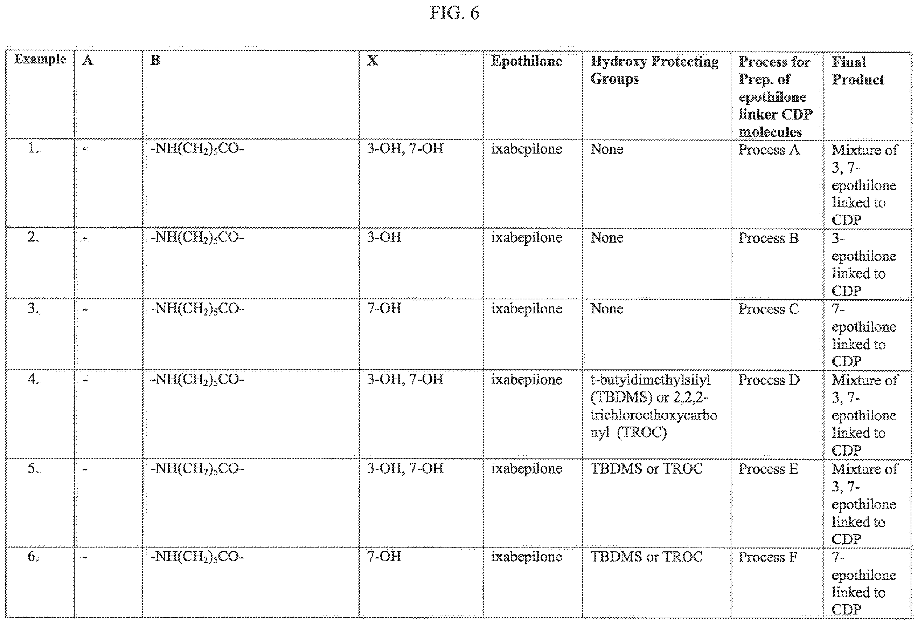

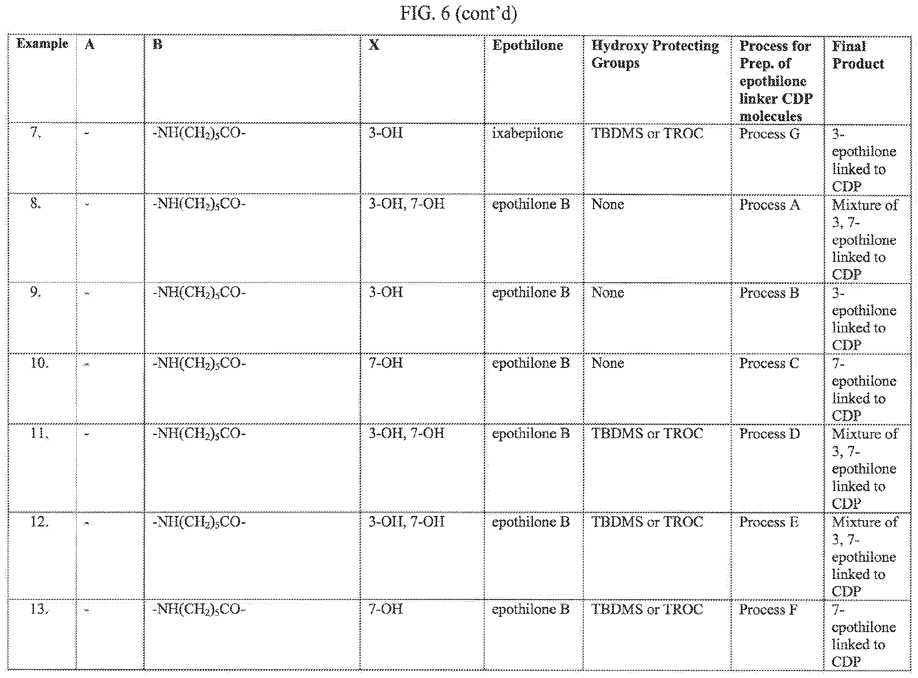

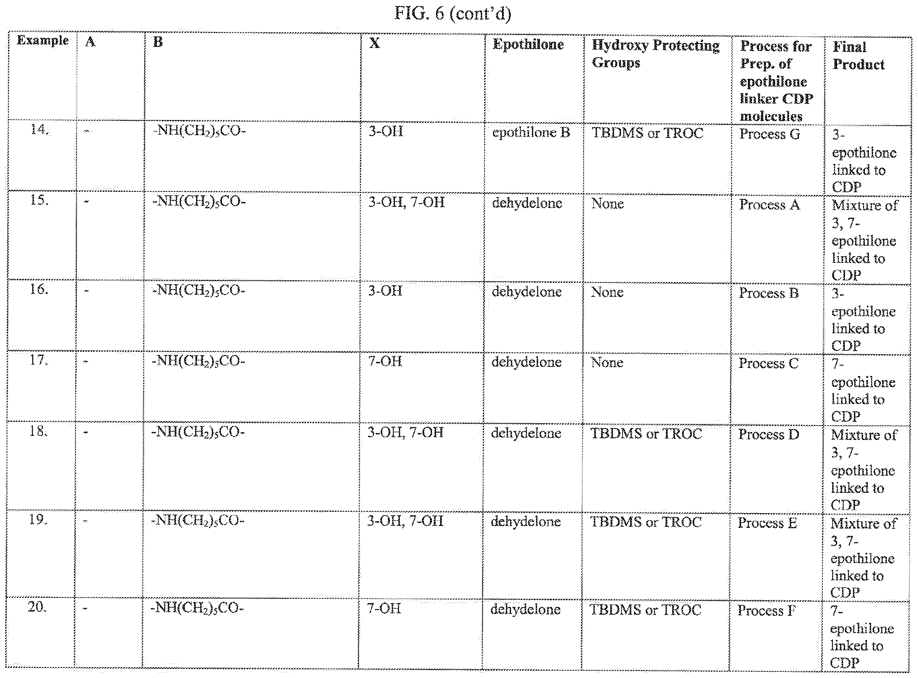

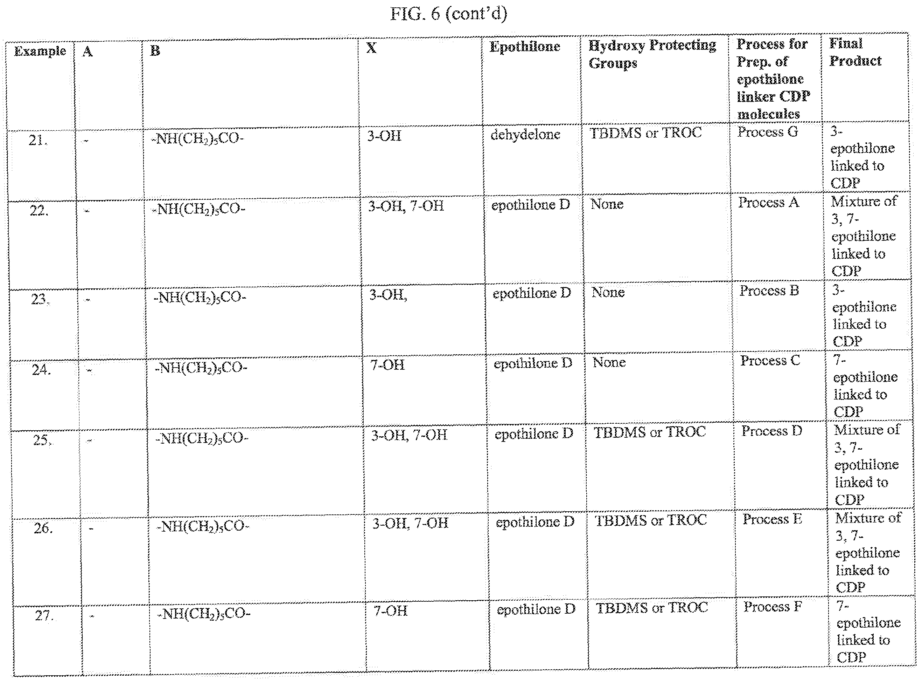

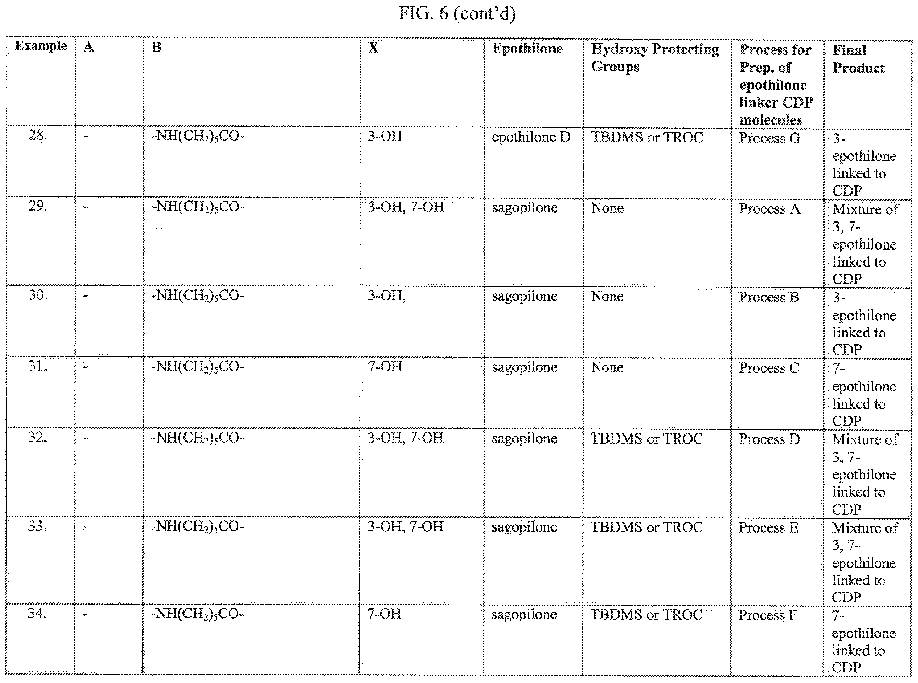

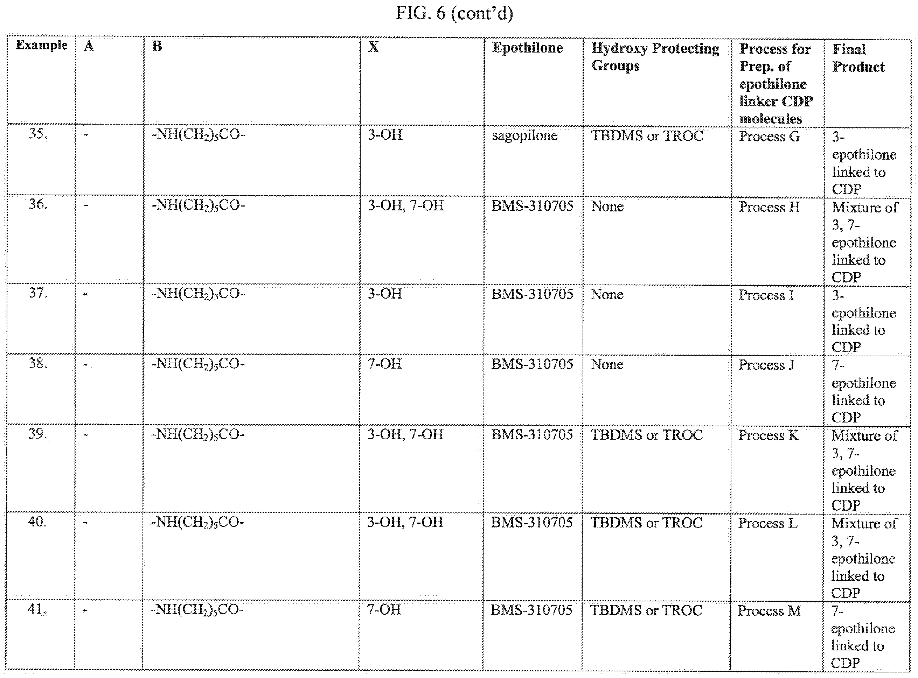

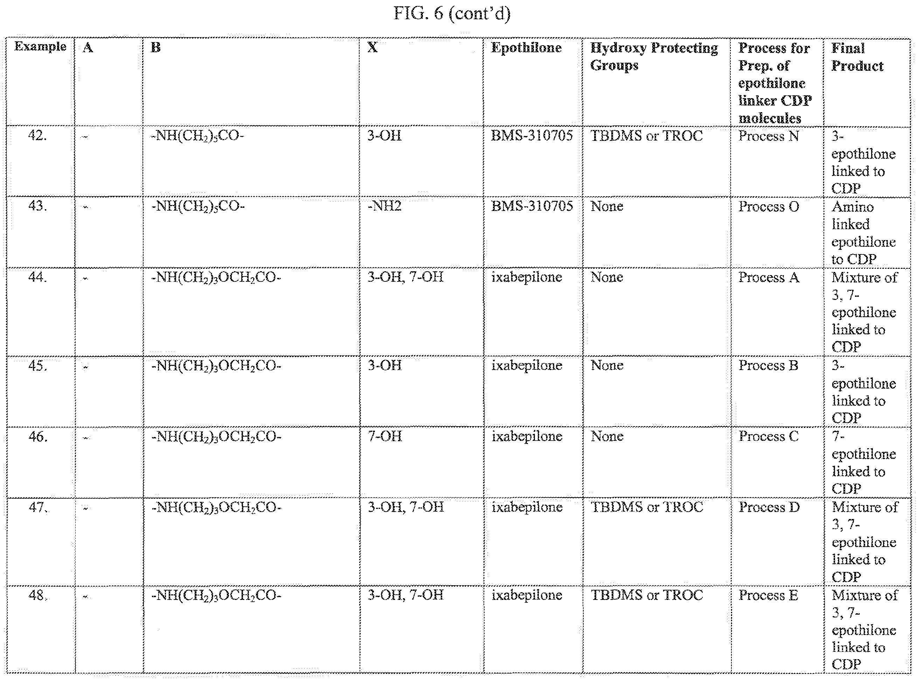

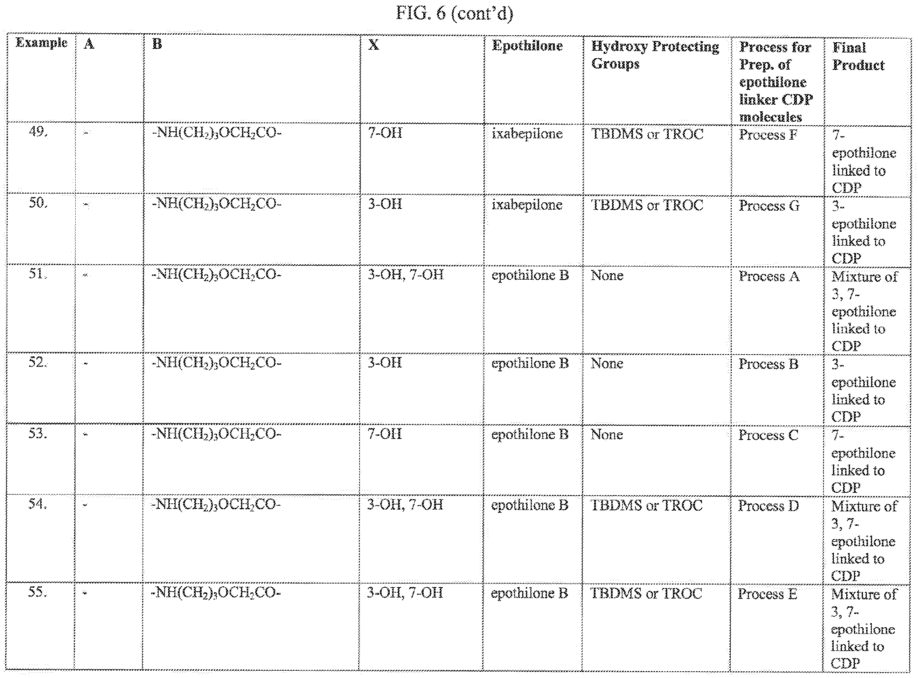

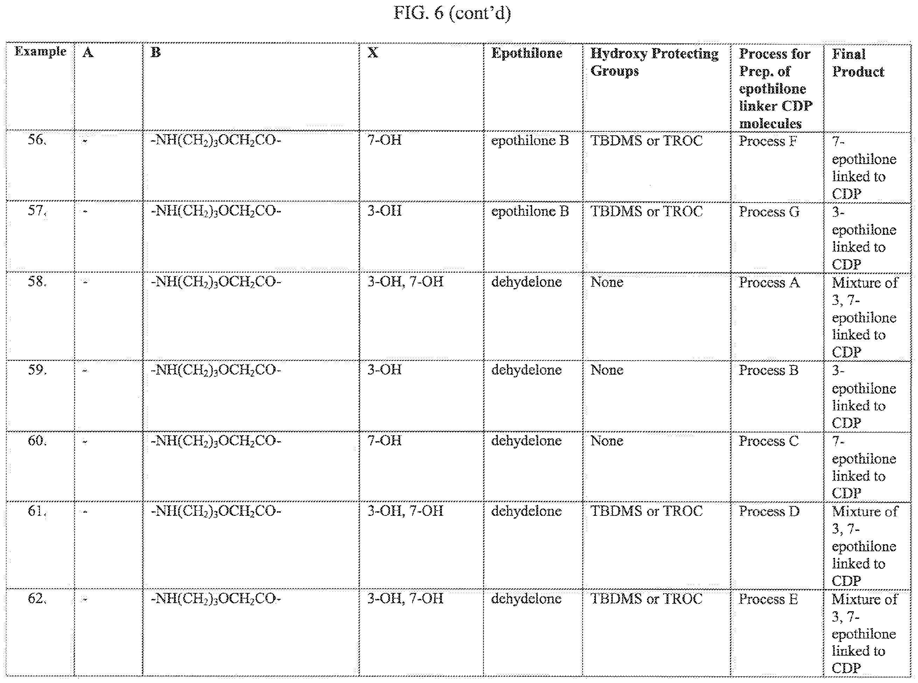

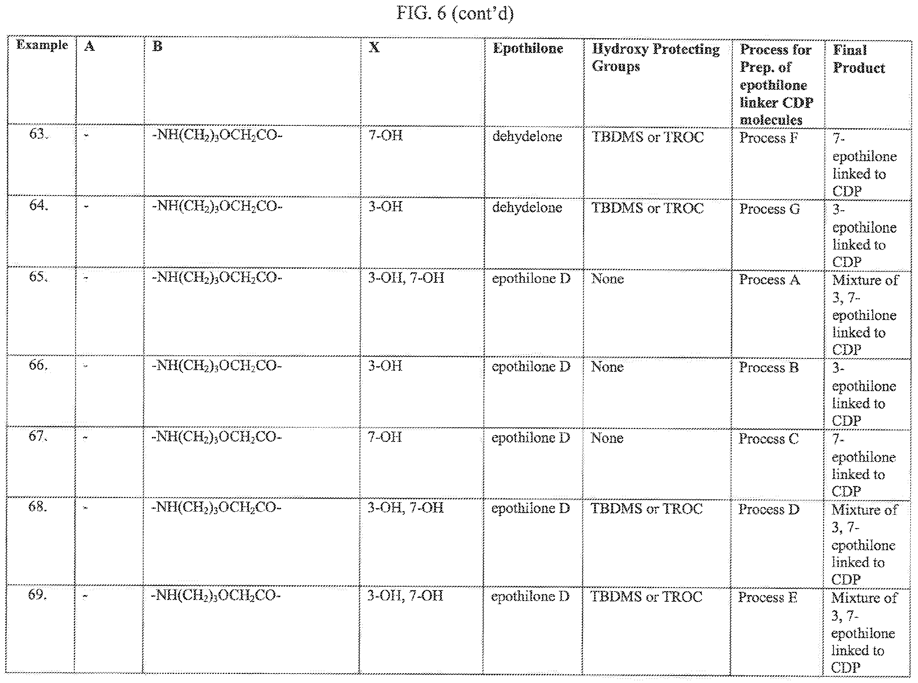

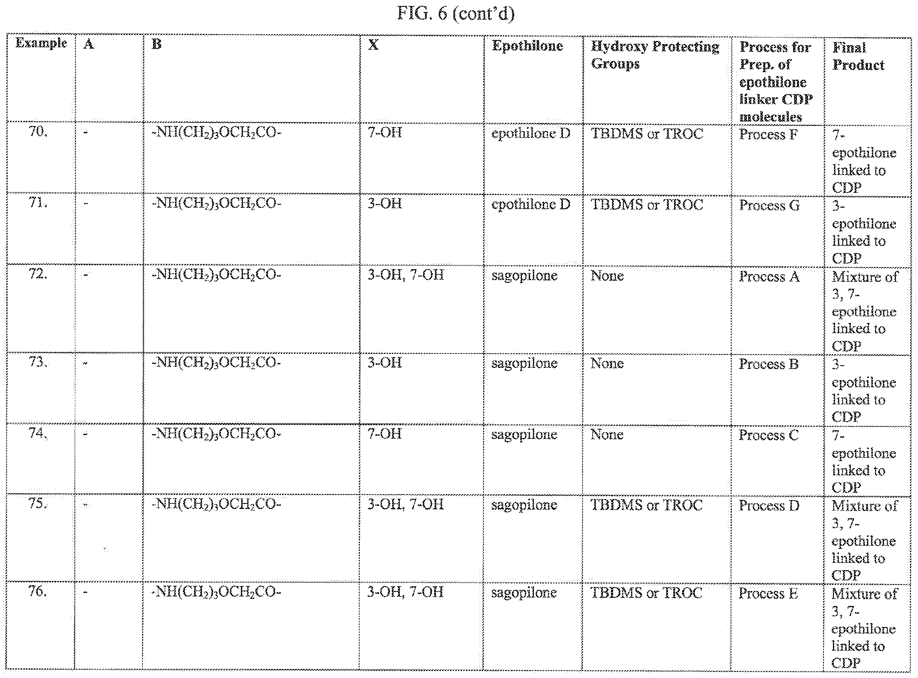

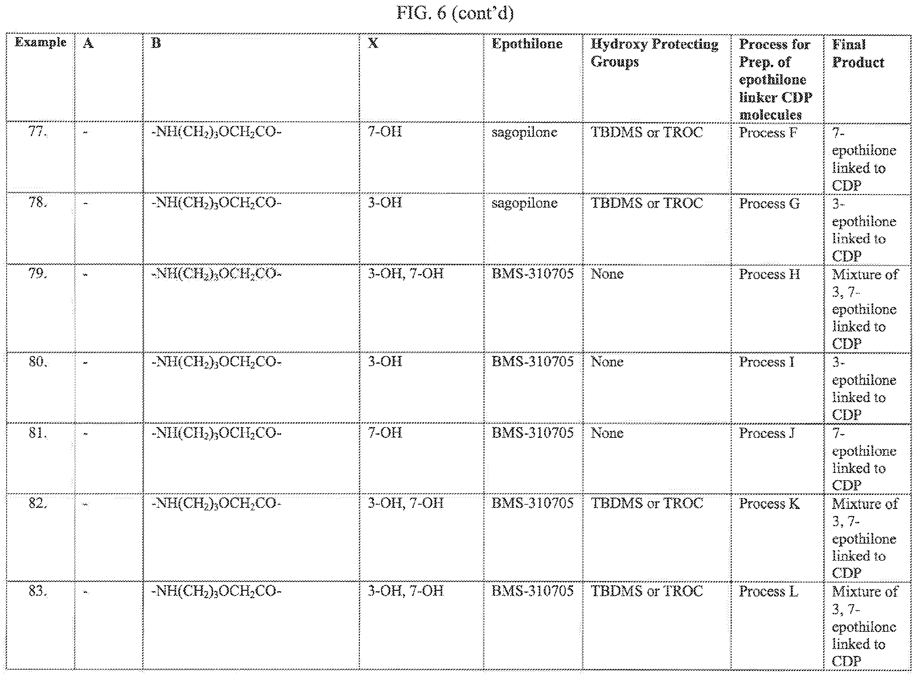

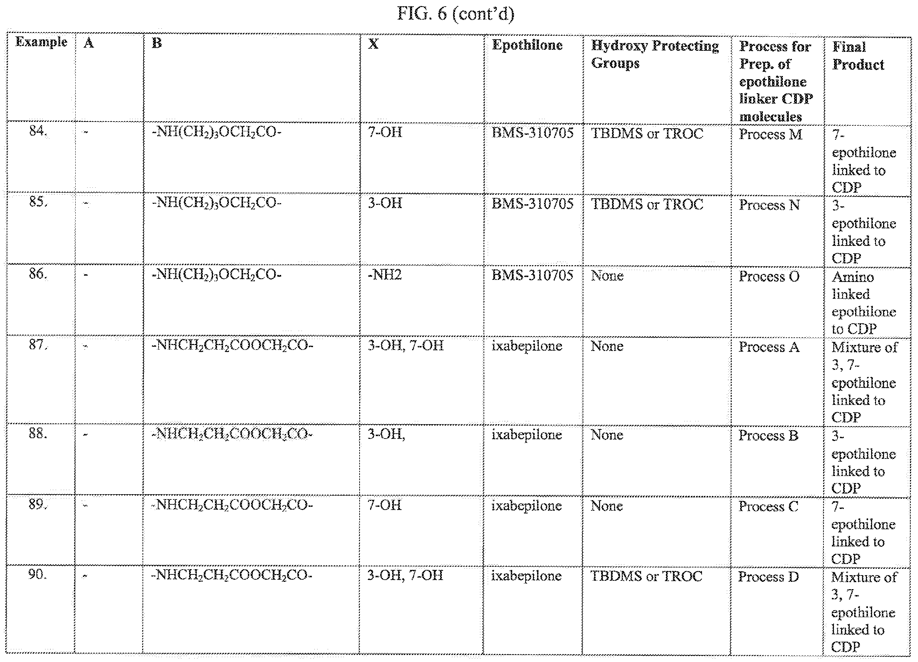

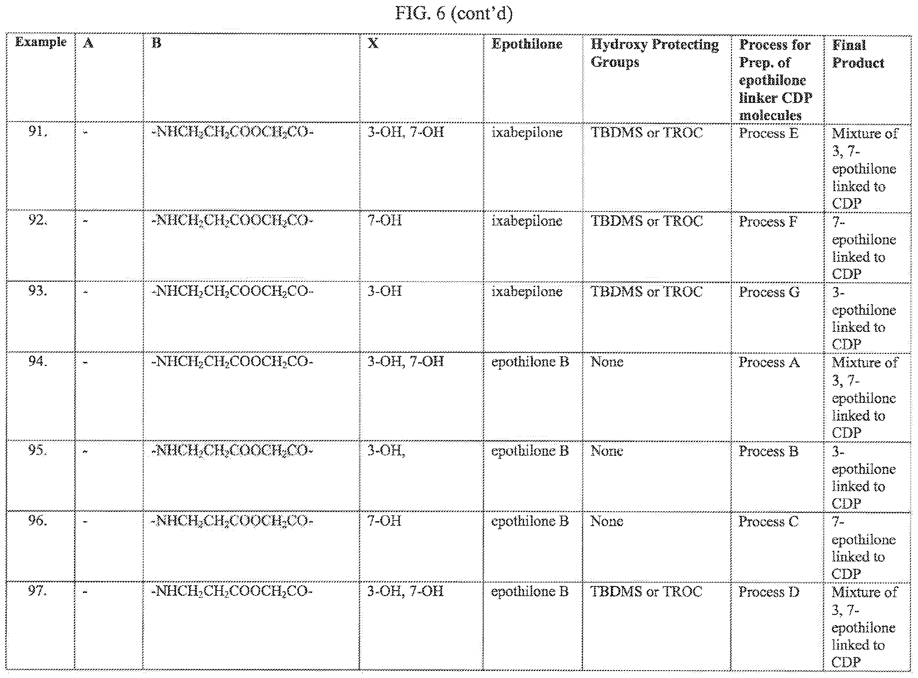

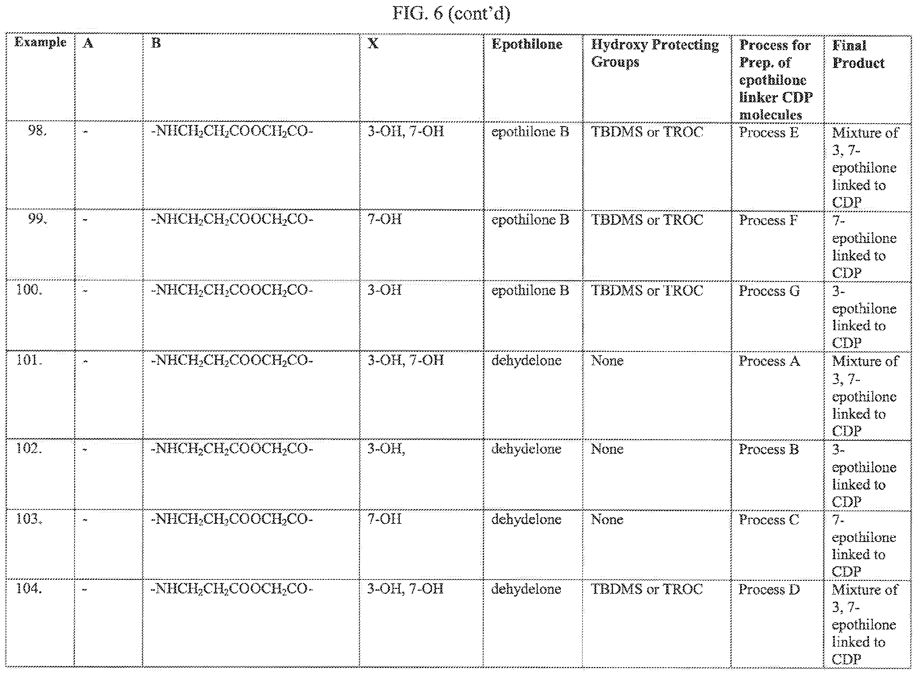

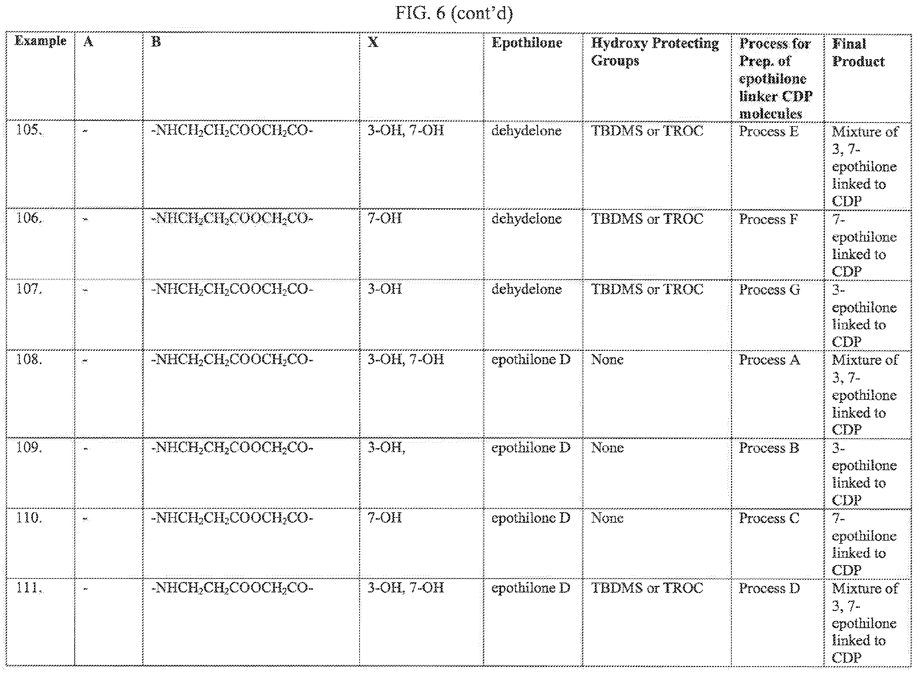

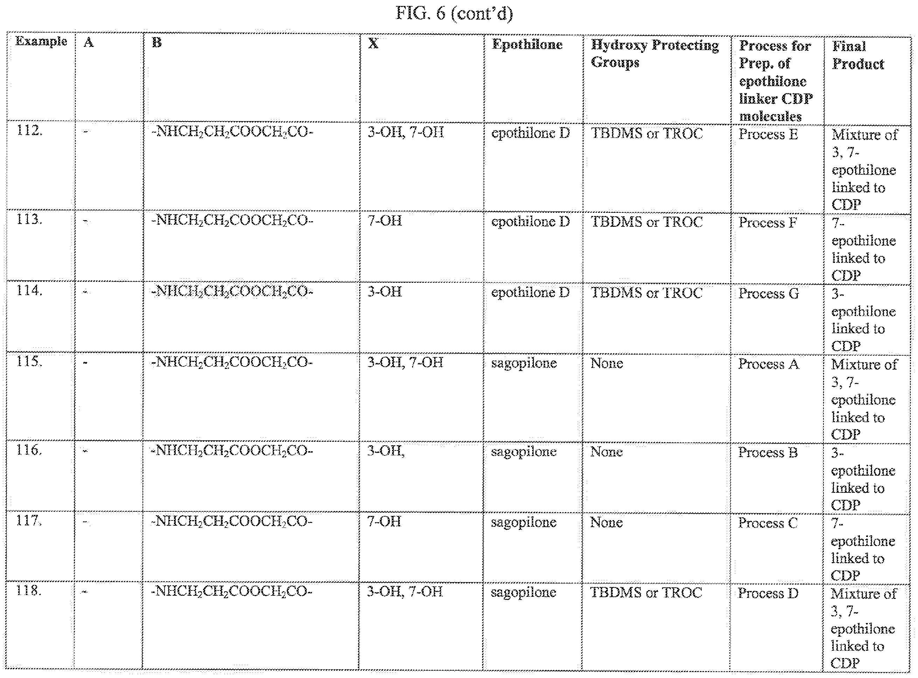

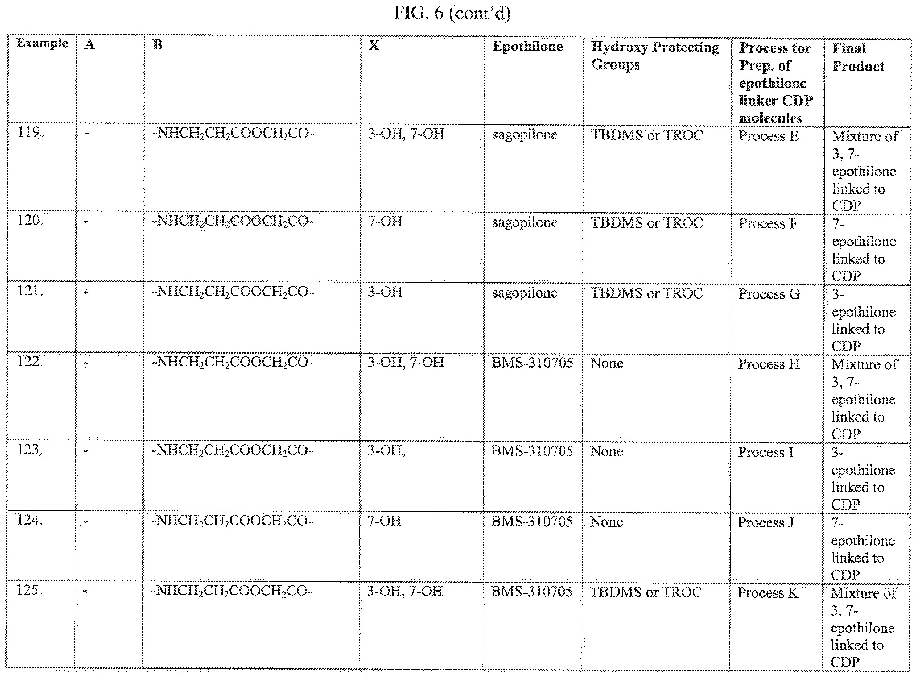

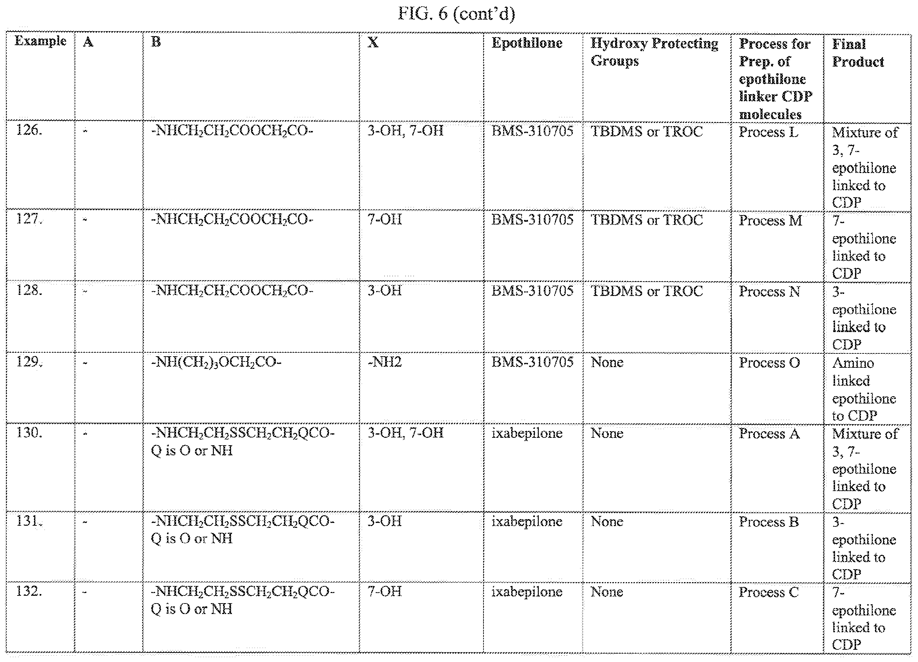

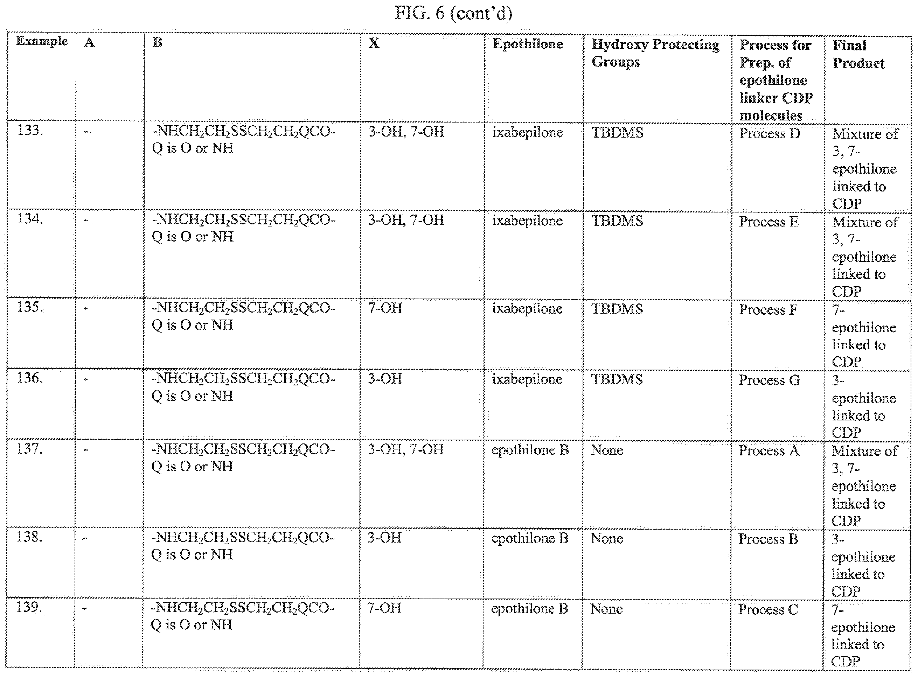

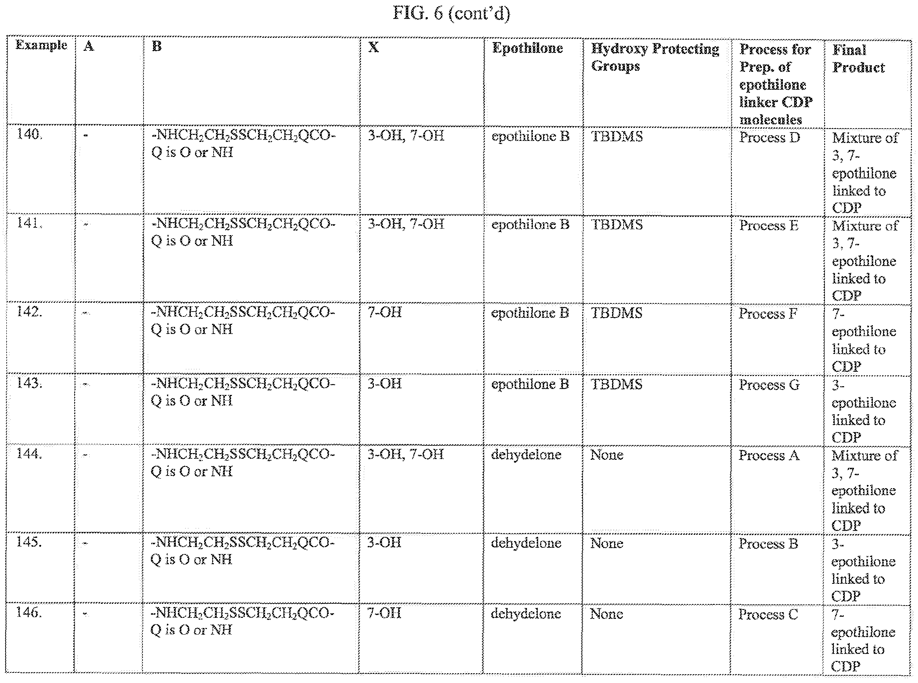

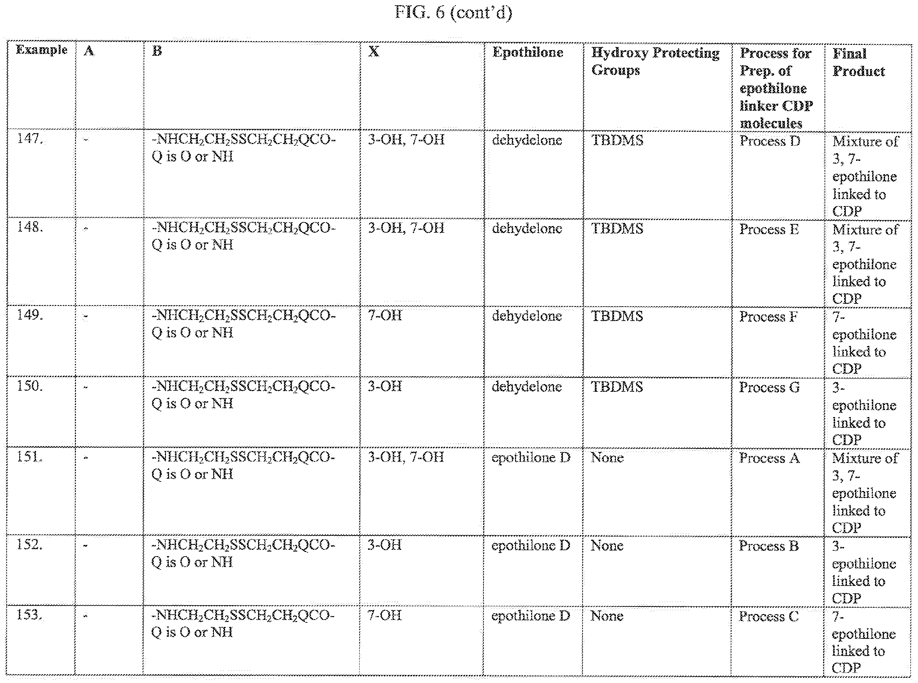

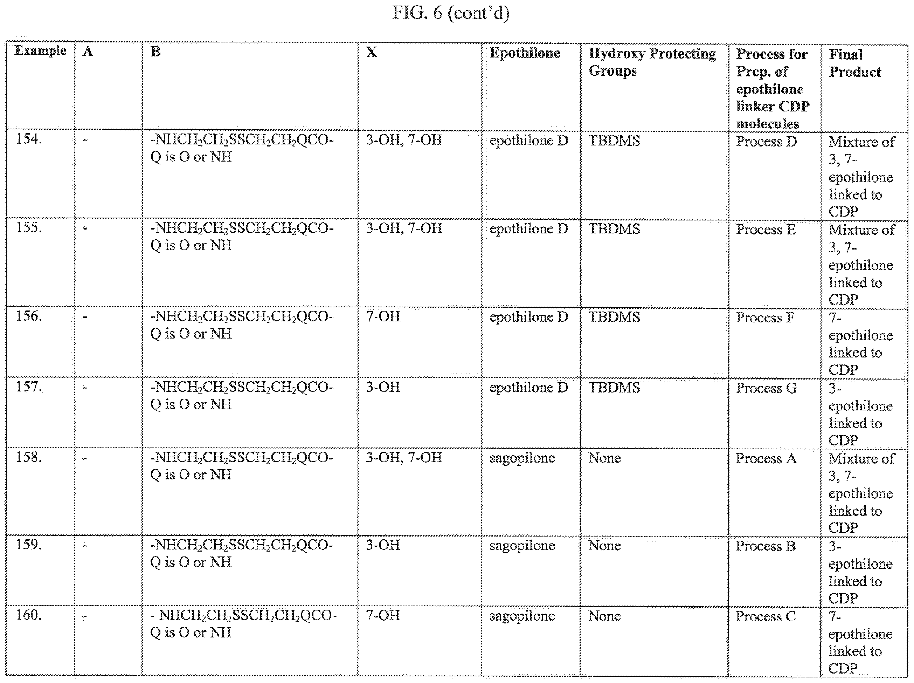

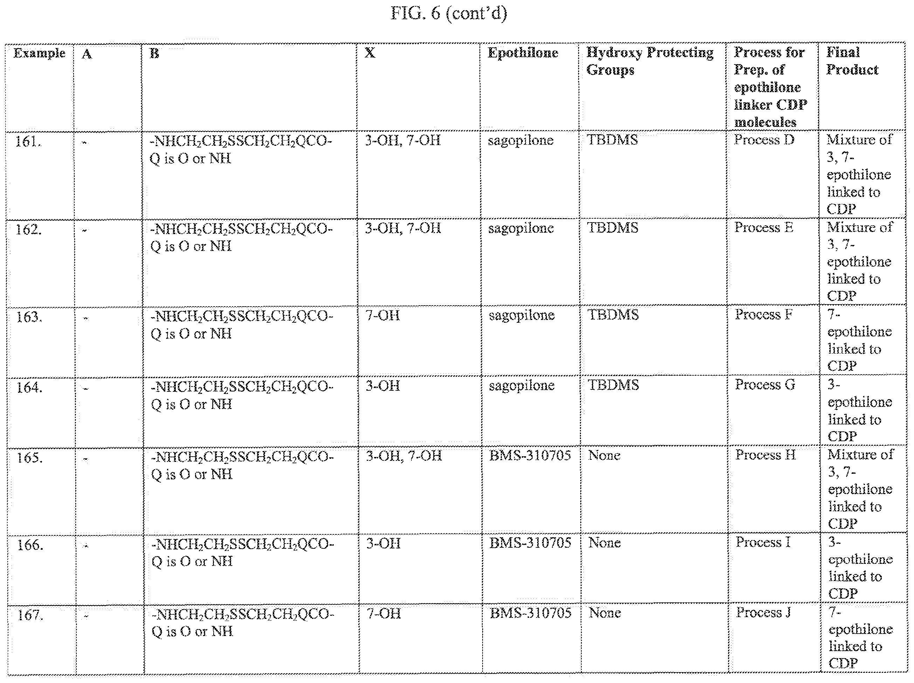

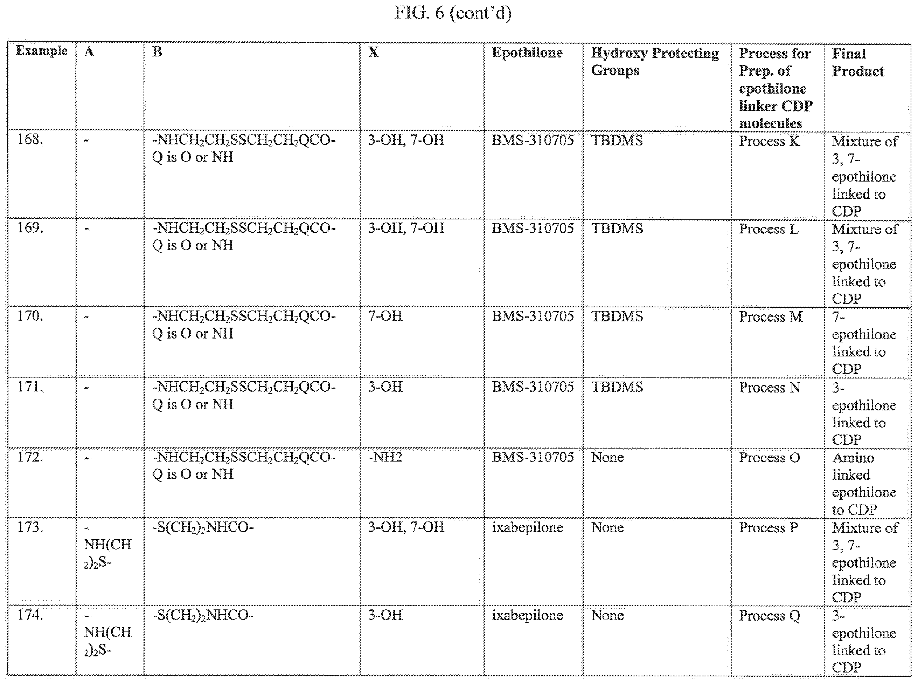

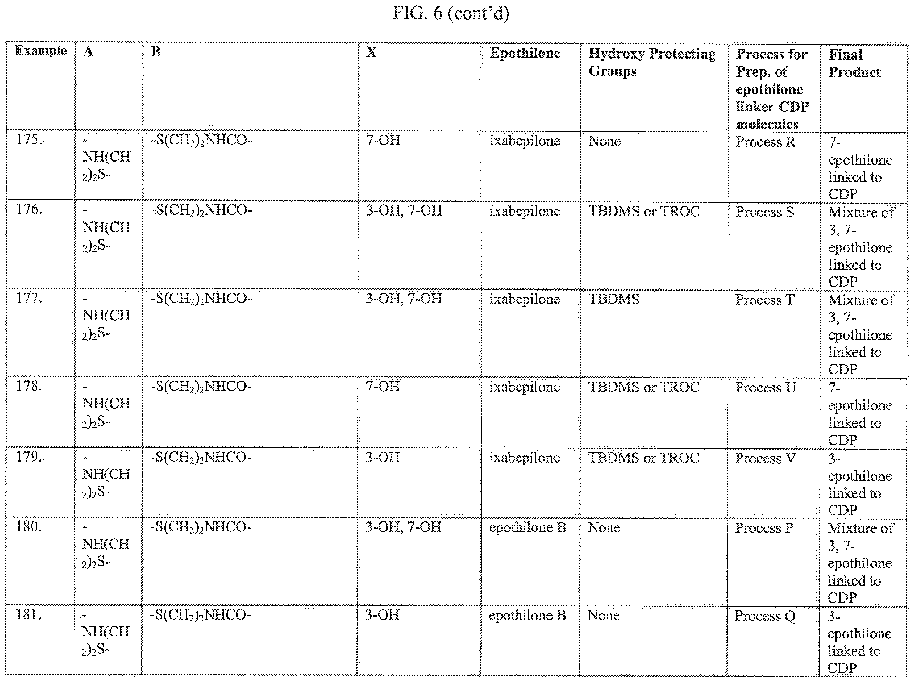

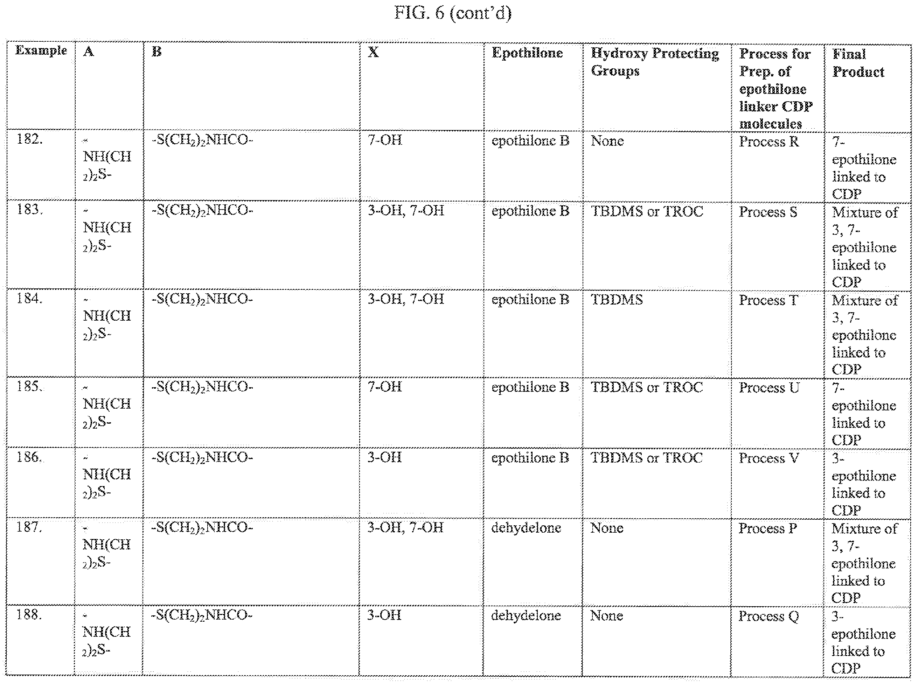

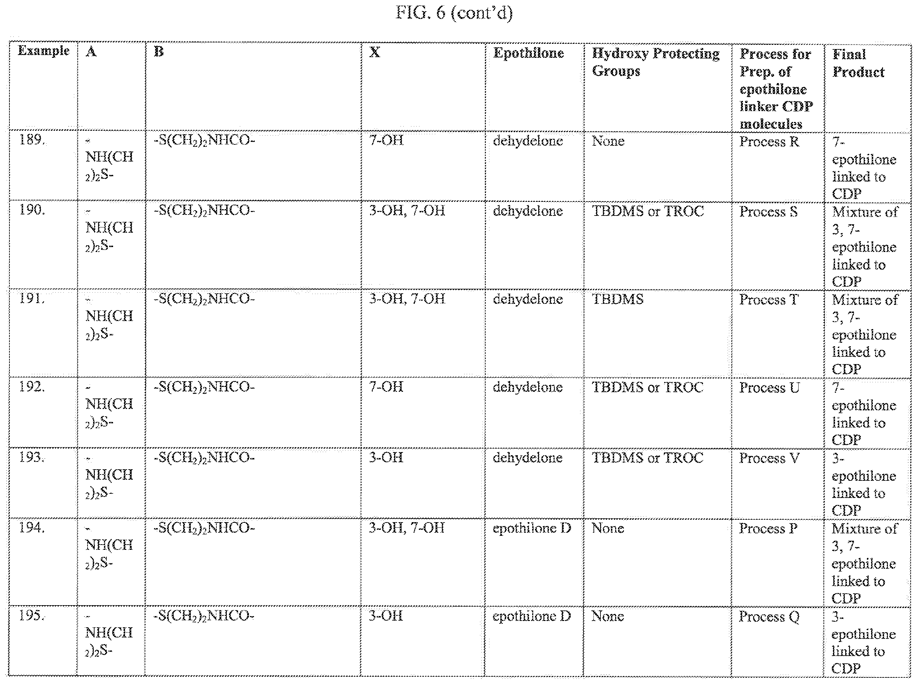

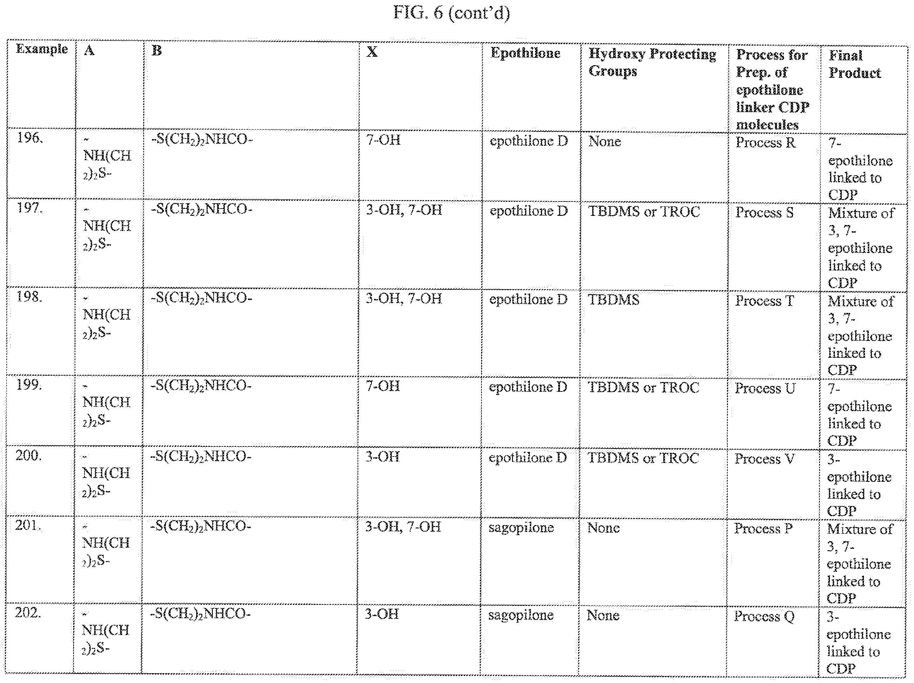

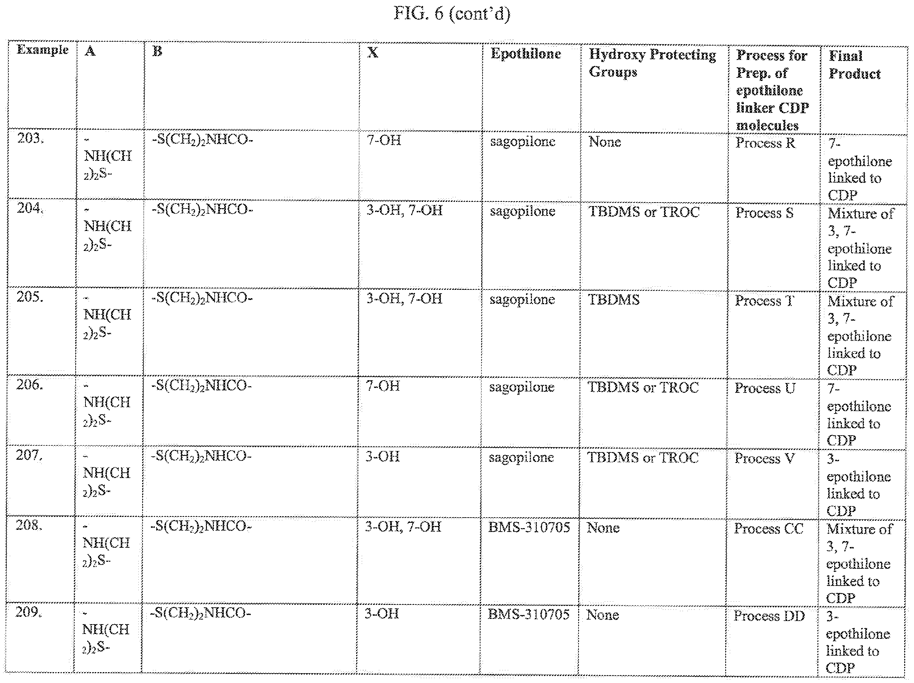

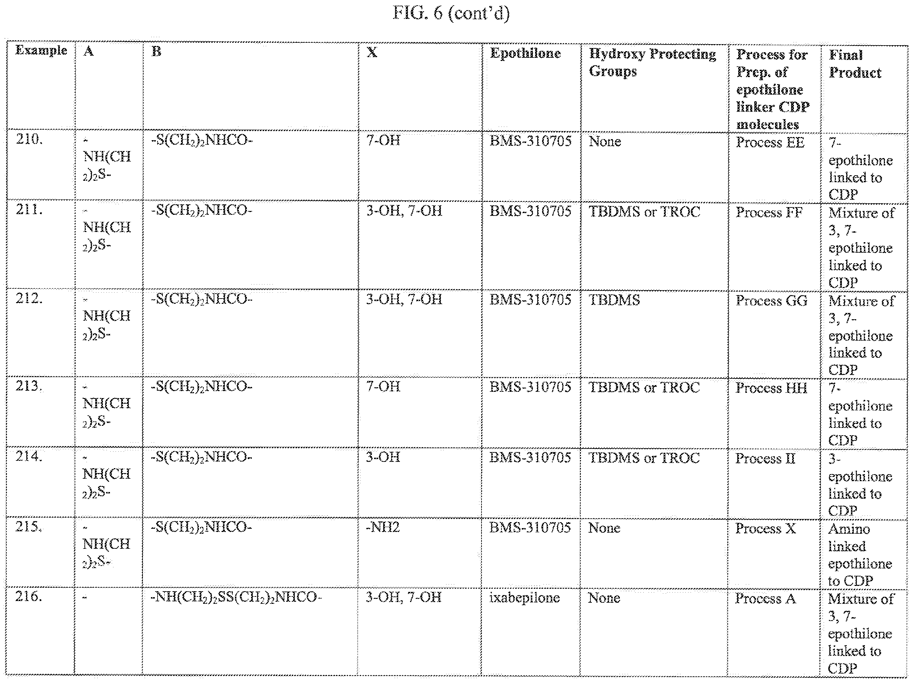

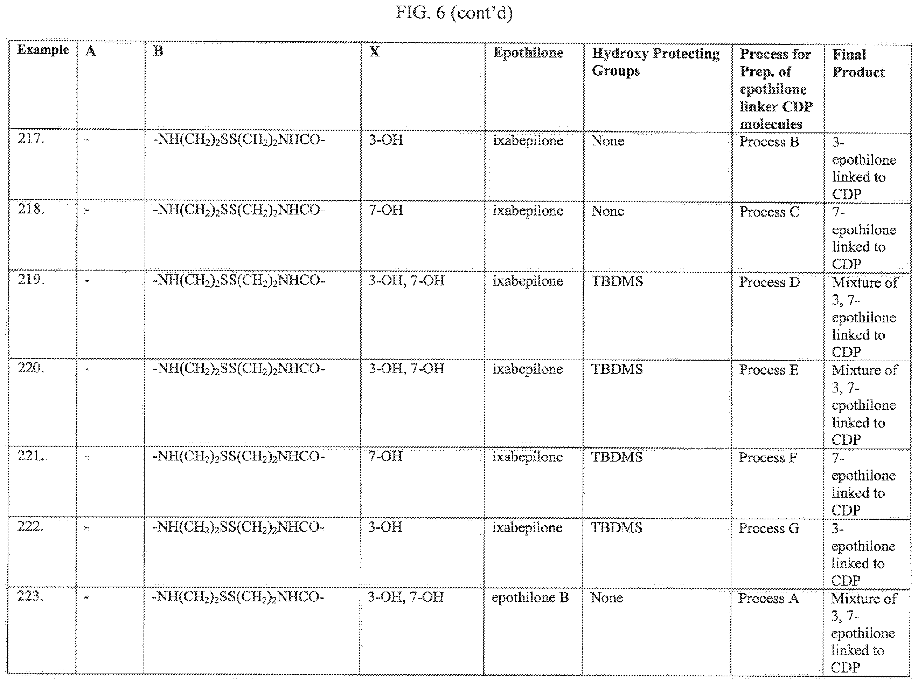

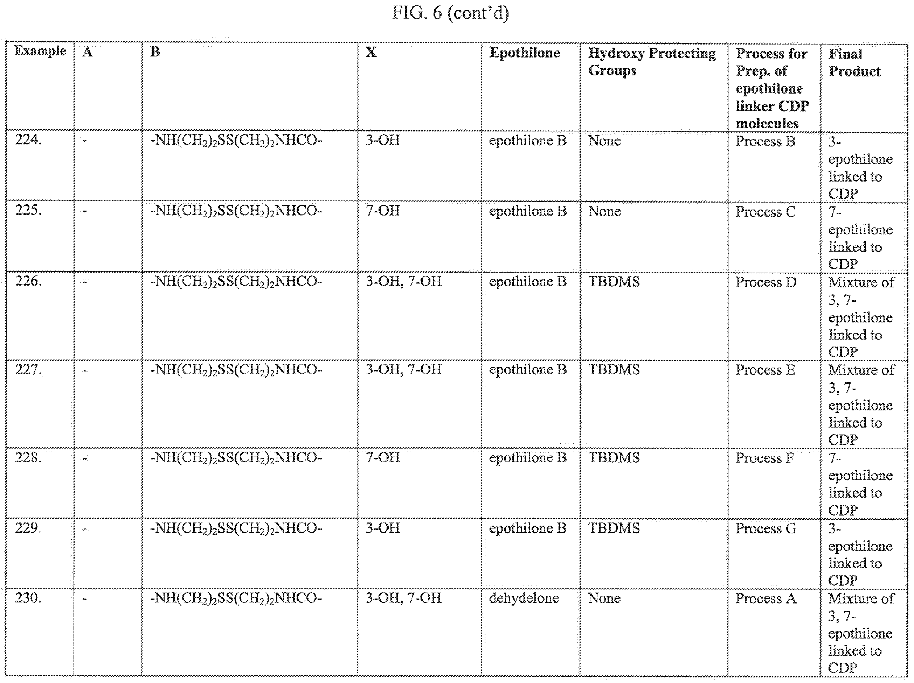

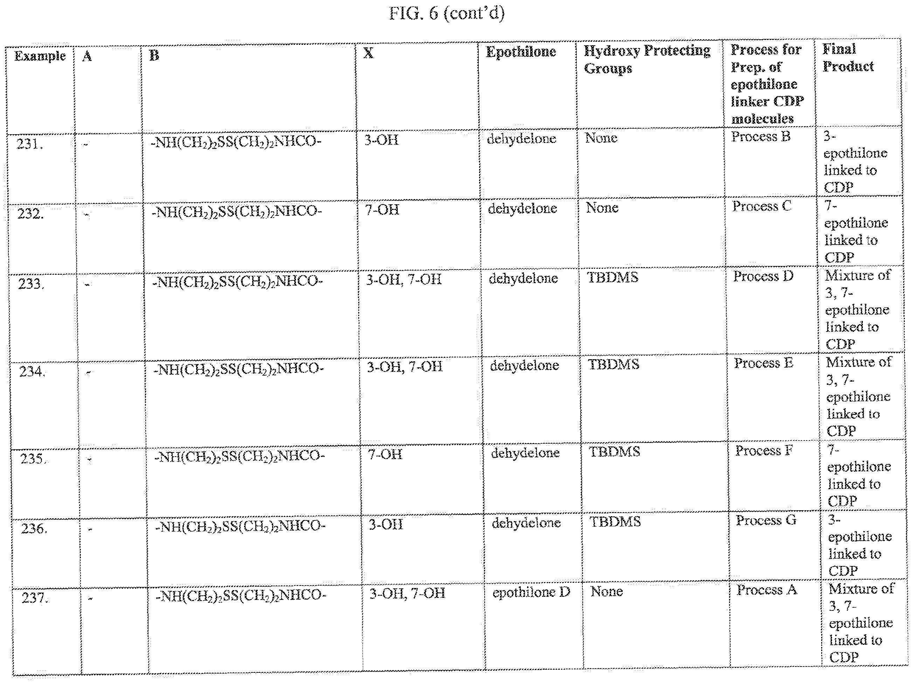

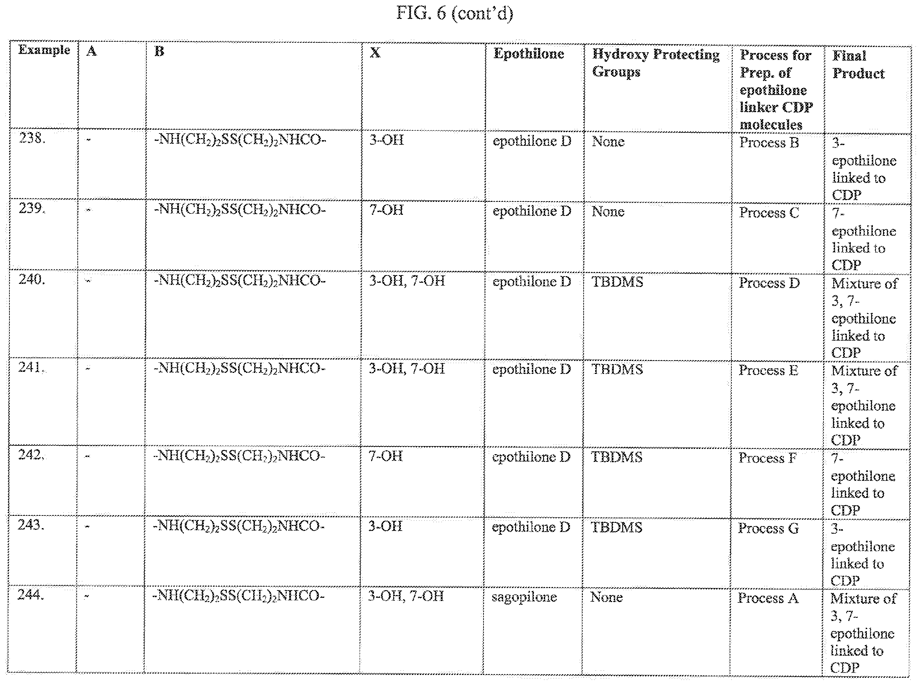

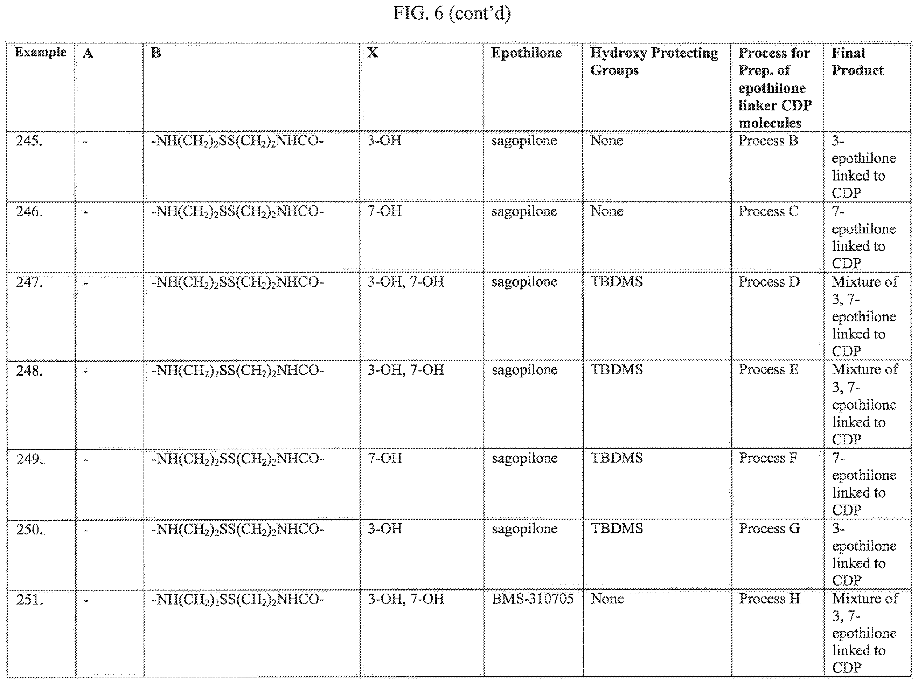

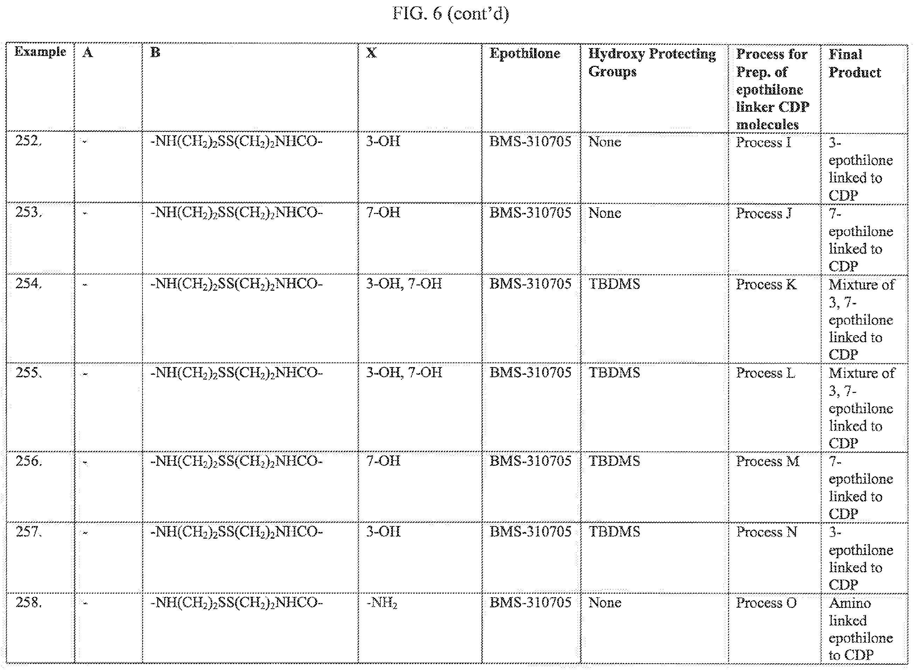

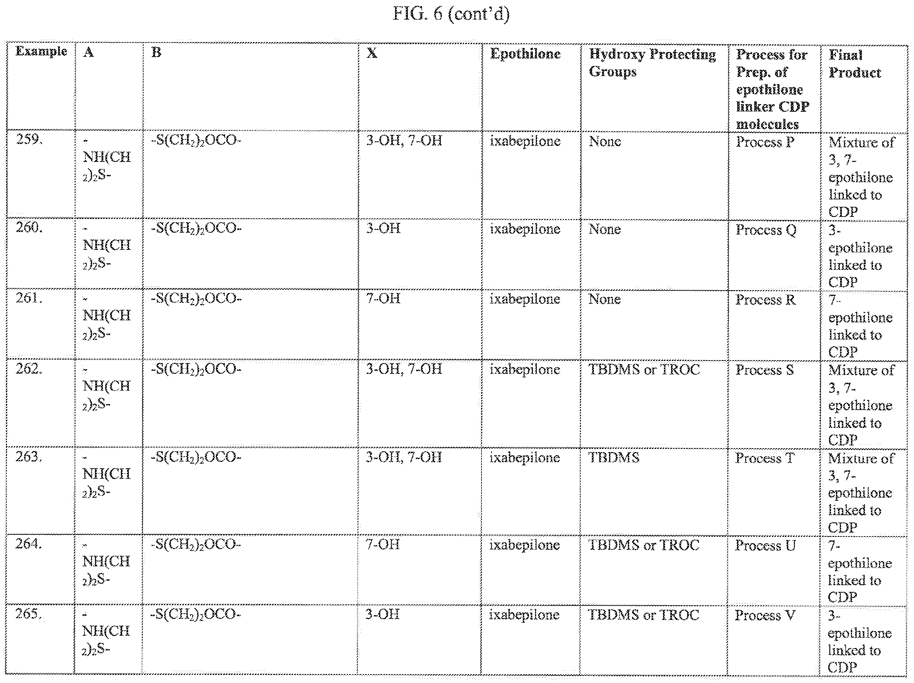

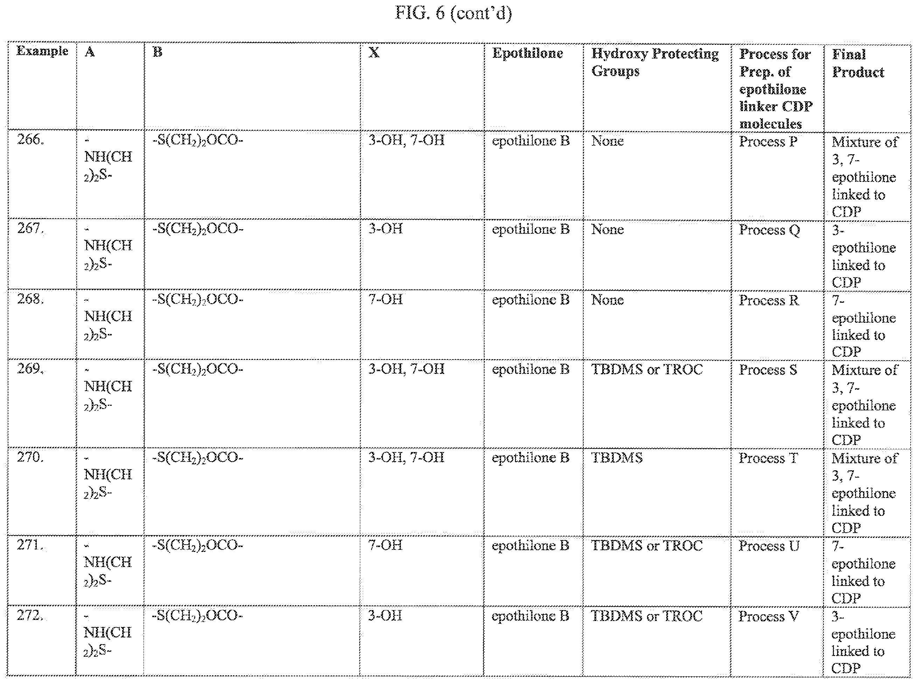

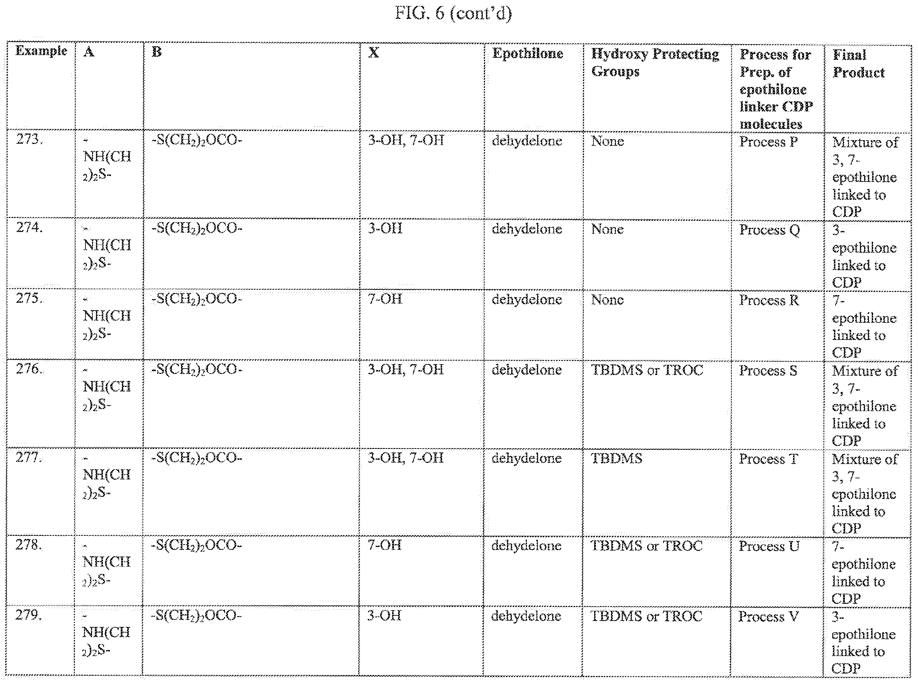

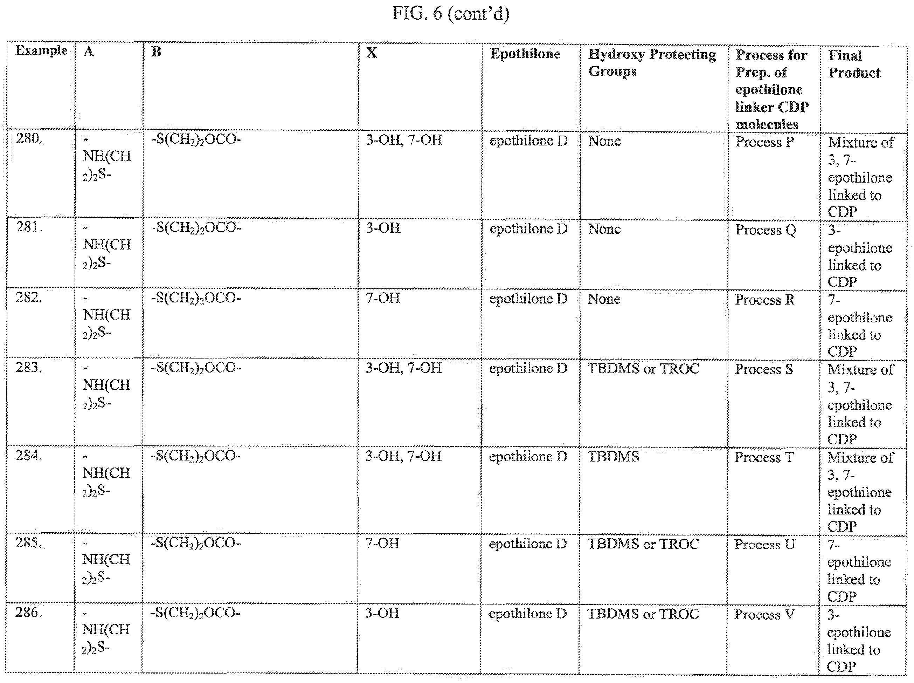

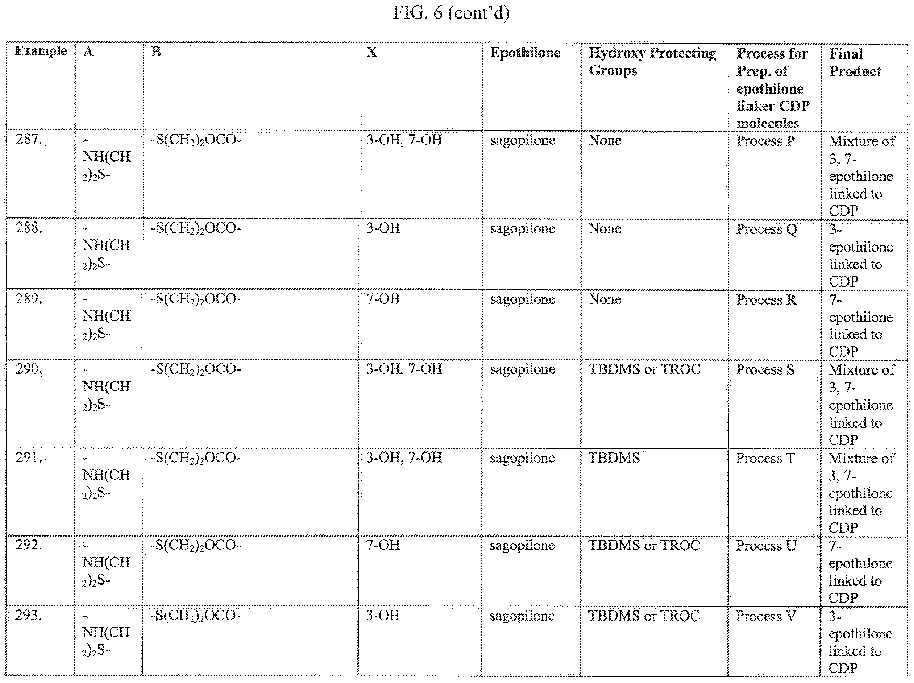

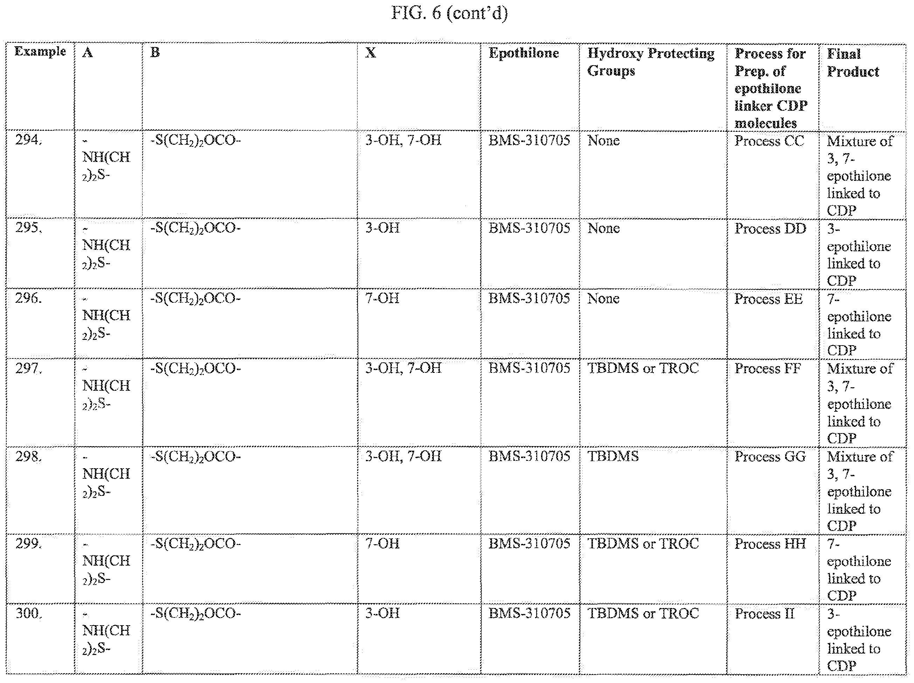

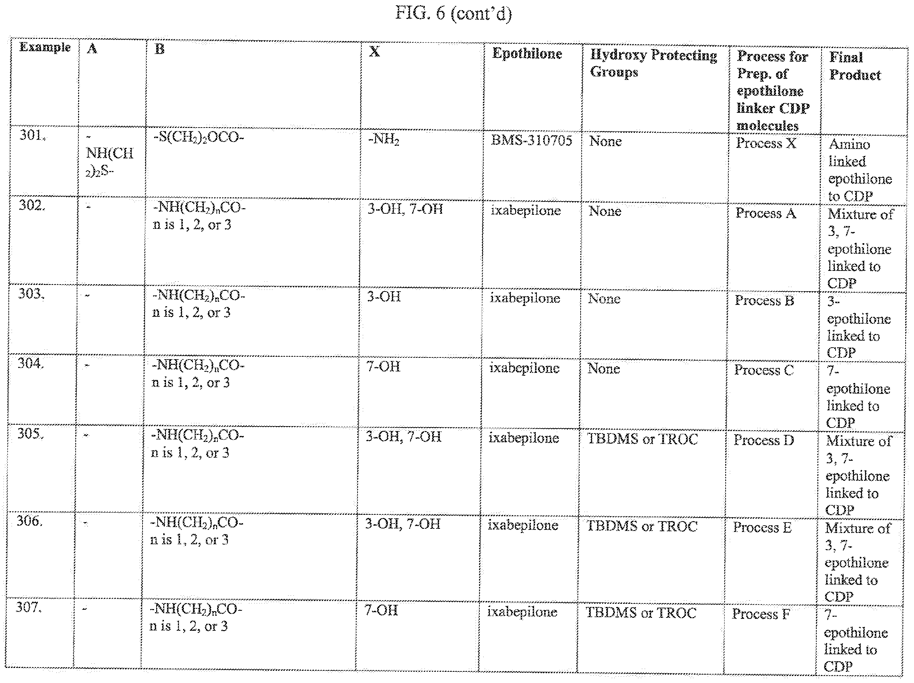

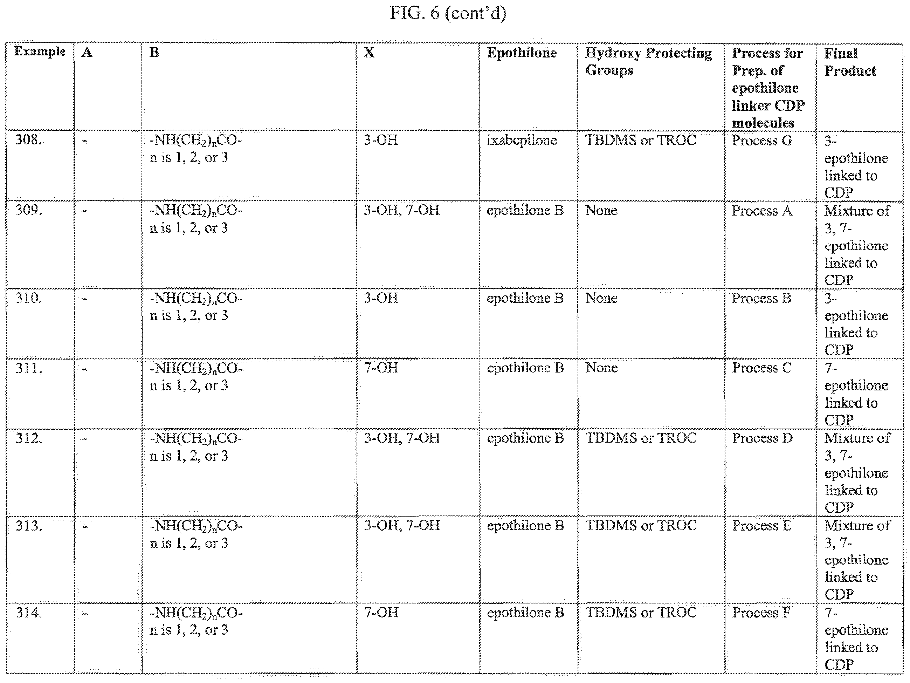

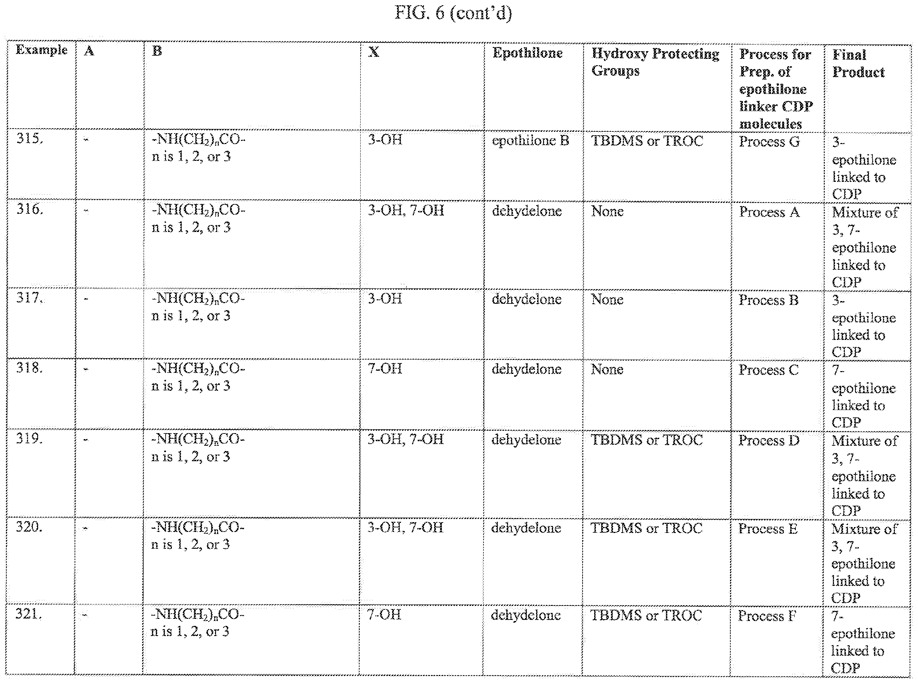

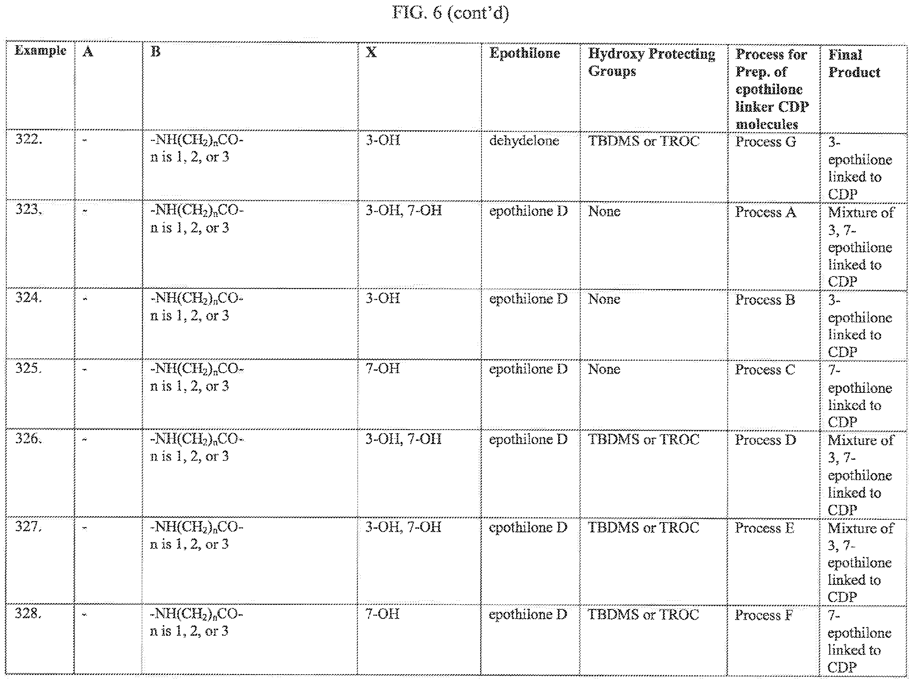

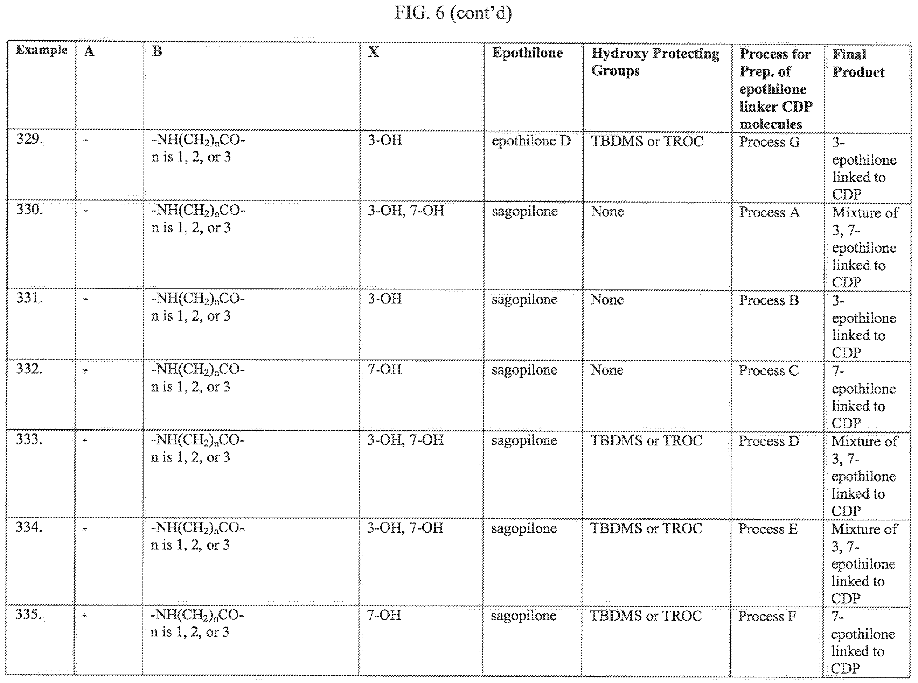

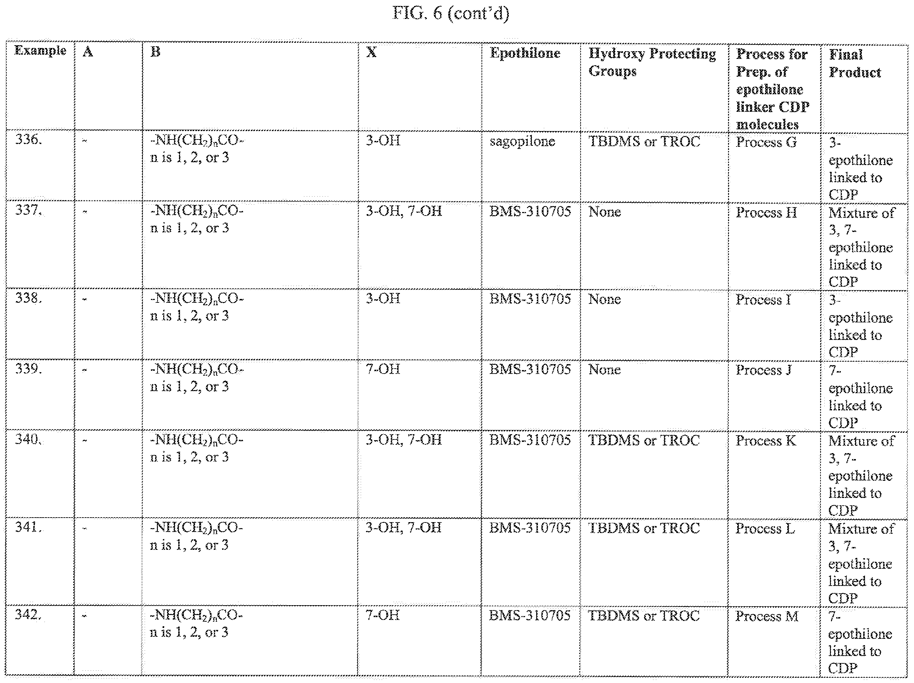

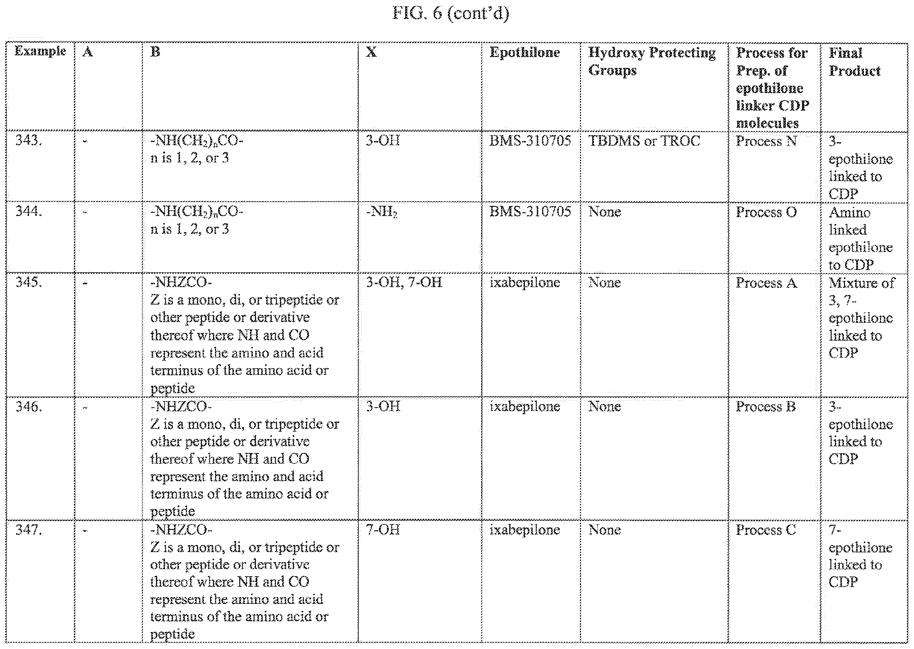

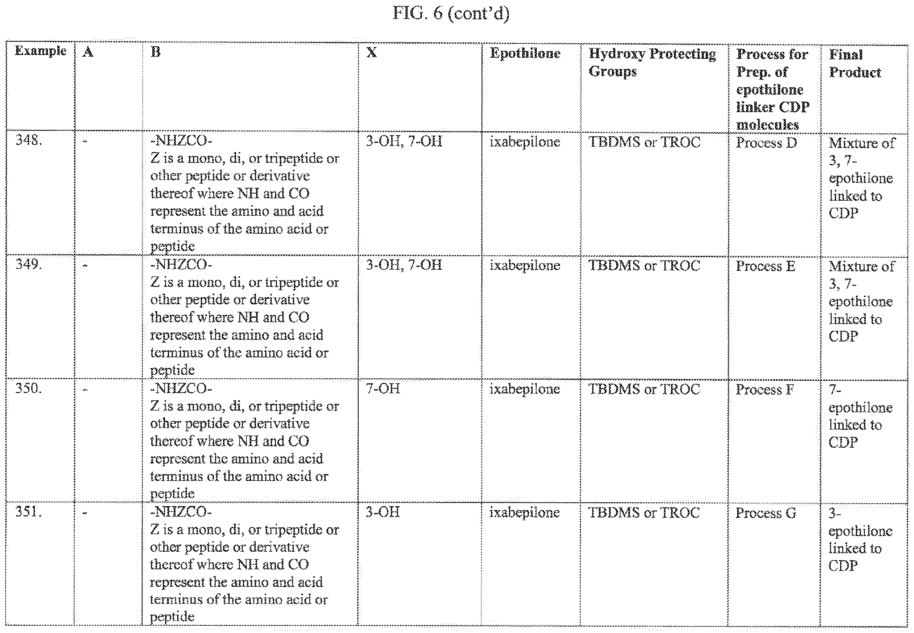

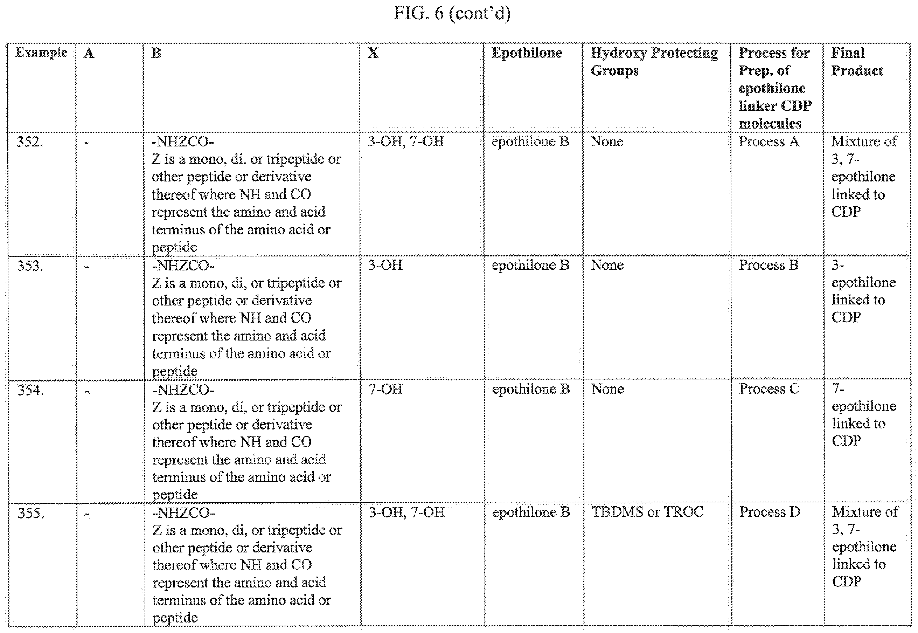

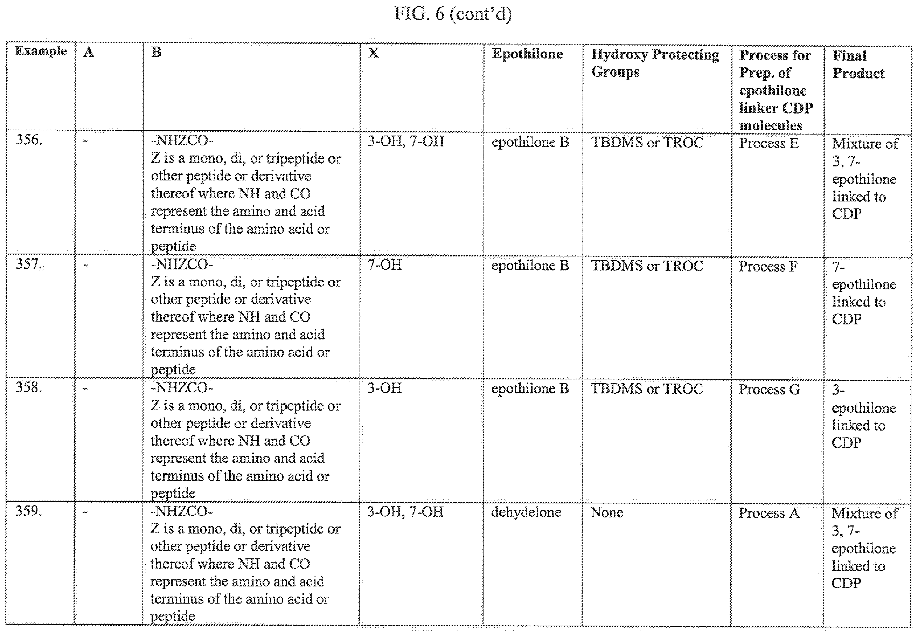

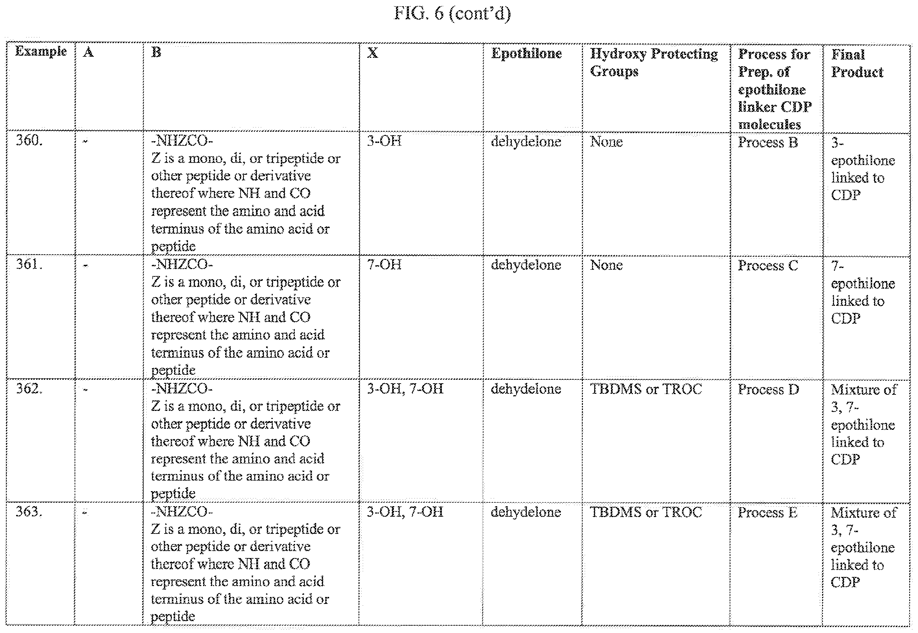

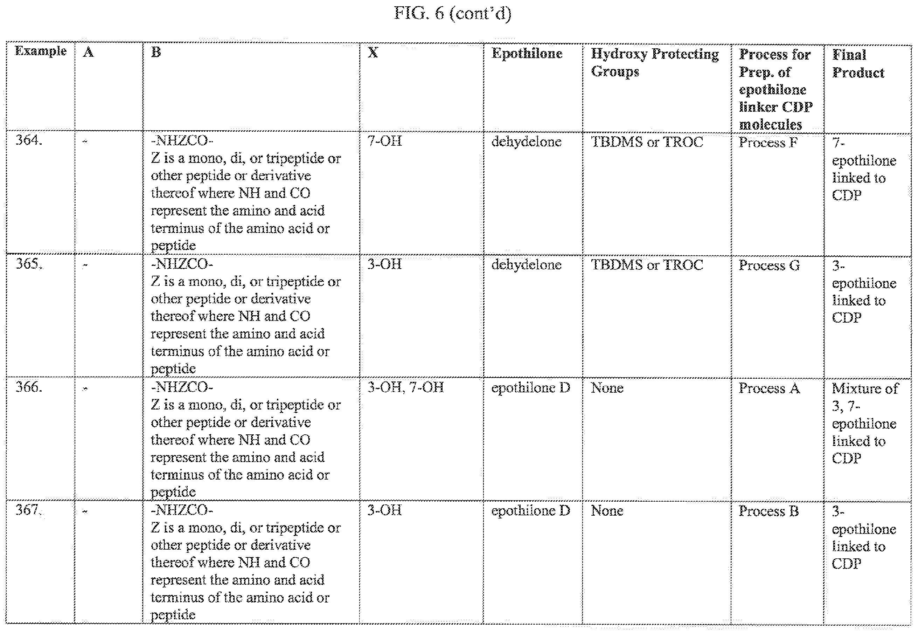

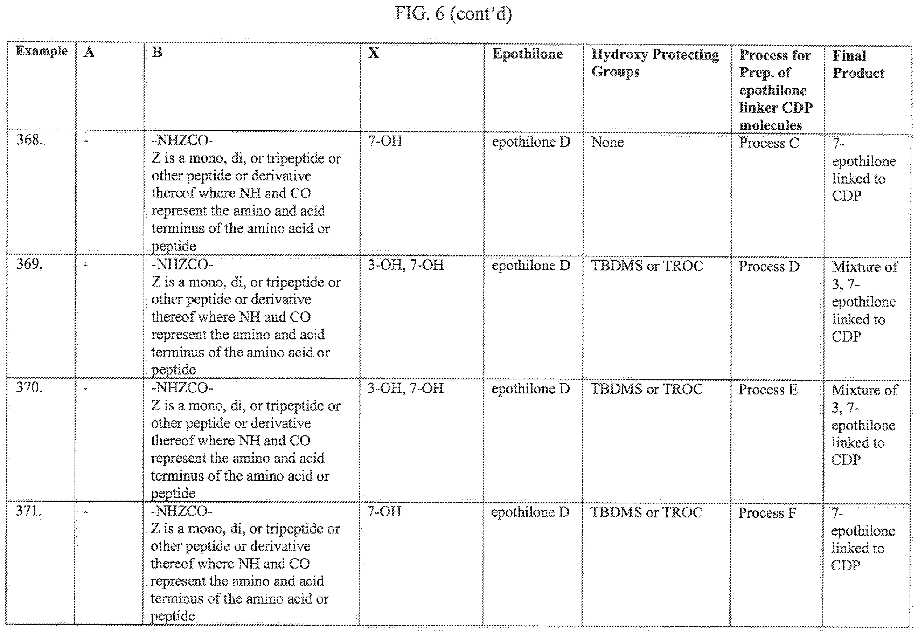

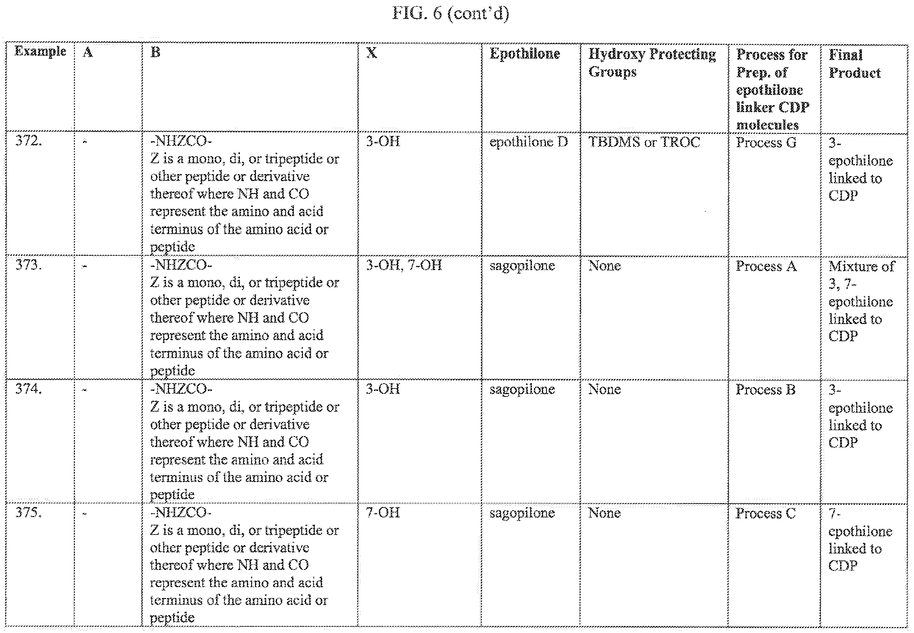

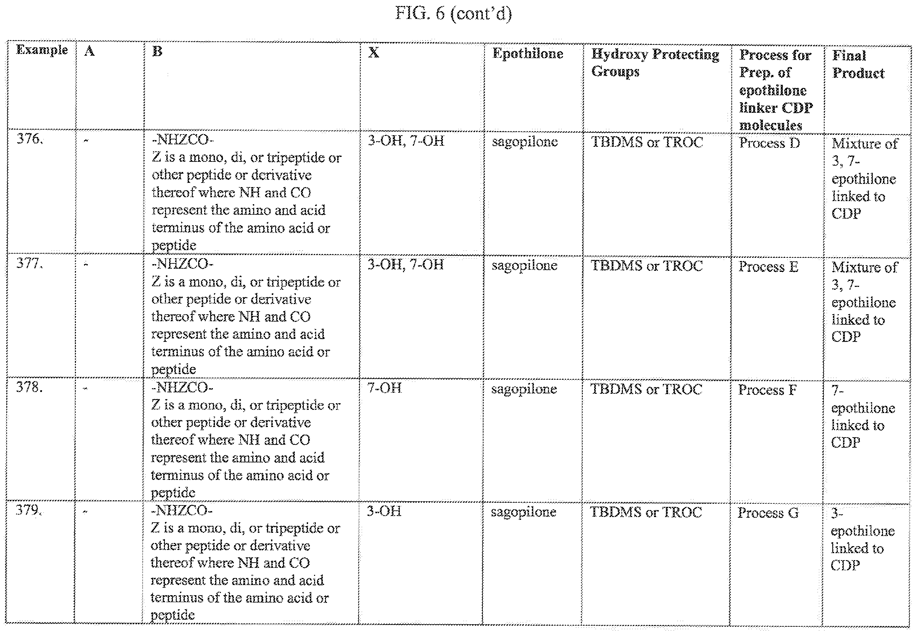

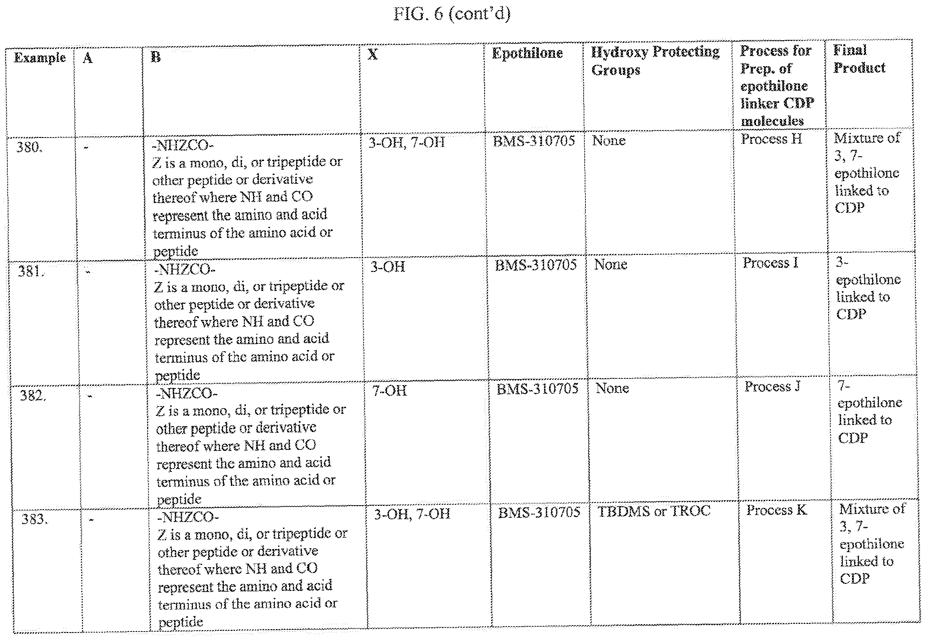

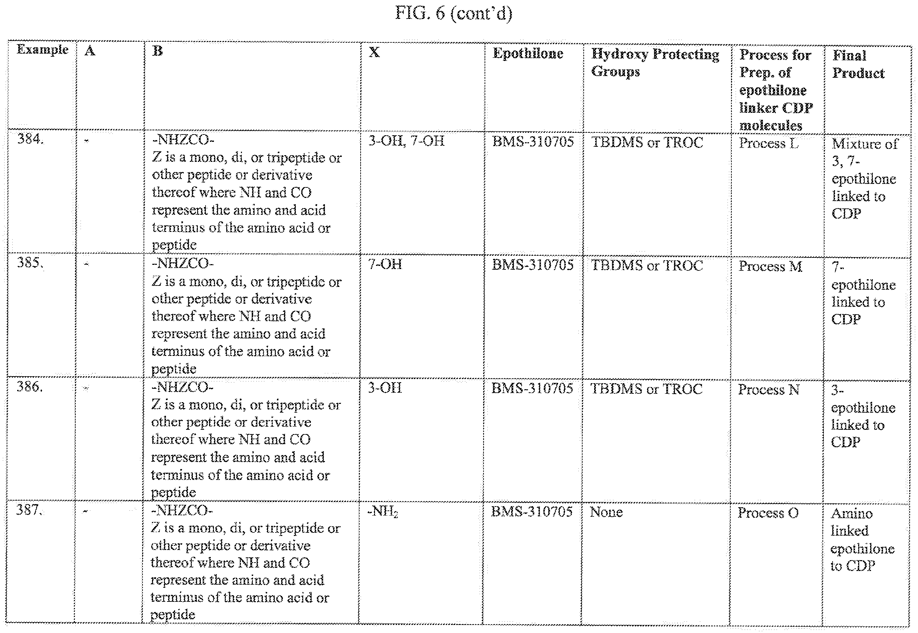

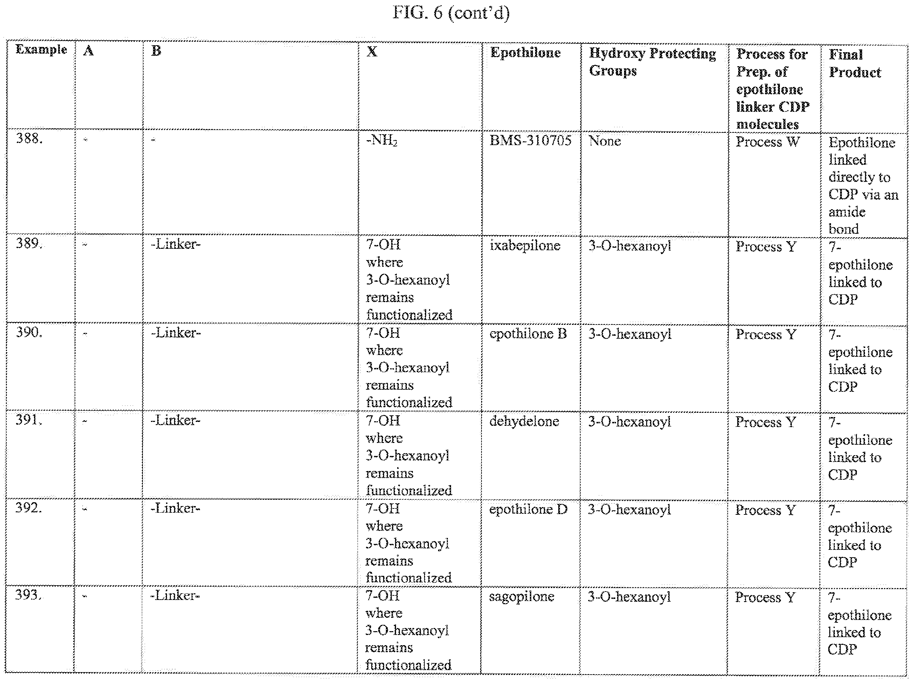

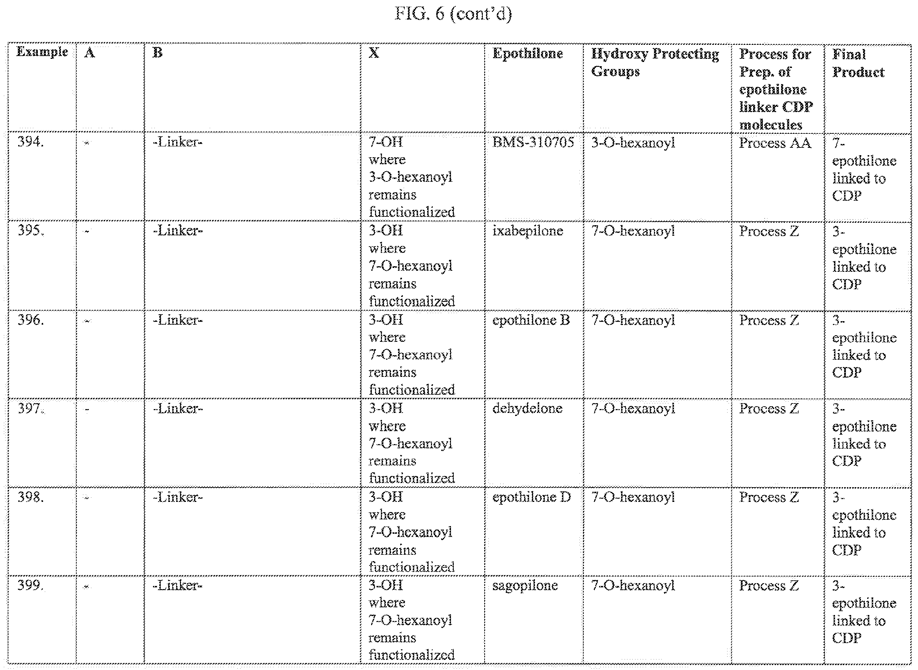

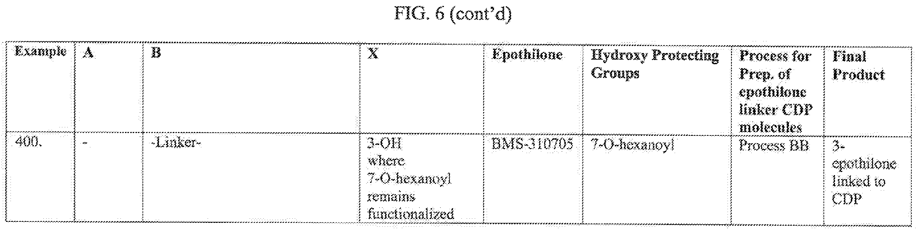

In addition, epothilones can be obtained via de novo synthesis. The total synthesis of epothilones A and B have been reported by a number of research groups including Danishefsky, Schinzer and Nicolaou. These total syntheses are described, for example, in U.S. Patents 6,156,905, 6,043,372, and 5,969,145 and in international PCT publications WO 98/08849, WO 98/25929, and WO 99/01124. Additional synthetic methods for making epothilone compounds are also described in PCT publications WO 97/19086, WO 98/38192, WO 99/02514, WO 99/07692, WO 99/27890, WO 99/28324, WO 99/43653, WO 99/54318, WO 99/54319, WO 99/54330, WO 99/58534, WO 59985, WO 99/67252, WO 99/67253, WO 00/00485, WO 00/23452, WO 00/37473, WO 00/47584, WO 00/50423, WO 00/57874, WO 00/58254, WO 00/66589, WO 00/71521, WO 01/07439 and WO 01/27308. In preferred embodiments, the microtubule inhibitor in the CDP-microtubule inhibitor conjugate, particle or composition comprises an epothilone, e.g., an epothilone described herein, e.g., an epothilone shown in FIG. 5 or FIG. 6.

In one embodiment, the CDP-microtubule inhibitor conjugate is a CDP-epothilone conjugate, e.g., 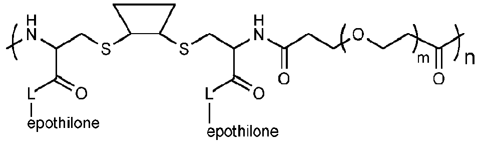 wherein 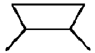 represents a cyclodextrin; n is an integer from 1 to 100 (e.g., n is an integer from 4 to 80, from 4 to 50, from 4 to 30 or from 4 to 20, or n is 4, 5, 6, 7, 8, 9, 10, 11, 12, 13, 14, 15, 16, 17, 18, 19 or 20); m is an integer from 1 to 1000 (e.g., m is an integer from 1 to 200, from 1 to 100, from 1 to 80, from 2 to 80, from 5 to 70, from 10 to 50, or from 20 to 40); L is a linker, e.g., a linker described herein; and "epothilone" is an epothilone, e.g., an epothilone described herein, e.g., an epothilone shown in FIG. 5 or FIG. 6. In some embodiments, the CDP-microtubule inhibitor conjugate, e.g., the CDP-epothilone conjugate, does not have complete loading, e.g., one or more binding sites, e.g., cysteine residues, are not bound to a microtubule inhibitor, e.g., an epothilone moiety, e.g., e.g., an epothilone described herein, bound with a linker described herein, e.g., the CDP-epothilone conjugate comprises one or more subunits having the formulae provided below: 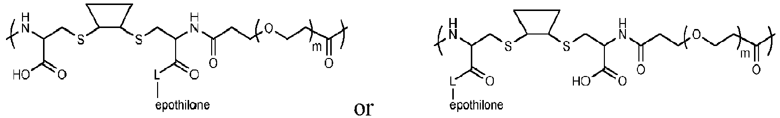 wherein 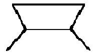 represents a cyclodextrin; m is an integer from 1 to 1000 (e.g., m is an integer from 1 to 200, from 1 to 100, from 1 to 80, from 2 to 80, from 5 to 70, from 10 to 50, or from 20 to 40); L is a linker, e.g., a linker described herein; and "epothilone" is an epothilone, e.g., an epothilone described herein, e.g., an epothilone shown in FIG. 5 or FIG. 6. In some embodiments, the CDP-microtubule inhibitor conjugate, particle or composition e.g., the CDP-epothilone conjugate, particle or composition, comprises a mixture of fully-loaded and partially-loaded CDP-microtubule inhibitor conjugates, e.g., CDP-epothilone conjugates.

In one embodiment, the CDP-microtubule inhibitor conjugate comprises a subunit of 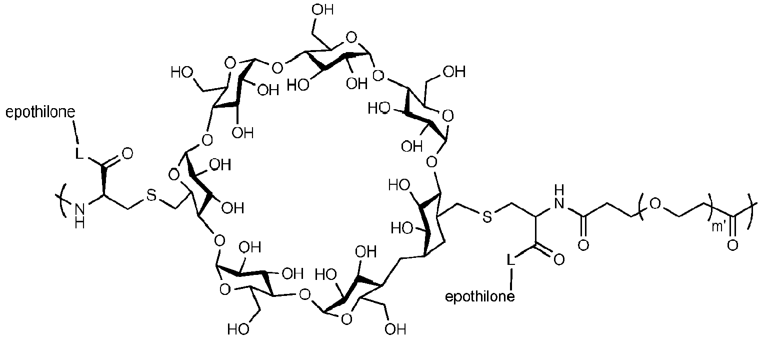 wherein m is an integer from 1 to 1000 (e.g., m is an integer from 1 to 200, from 1 to 100, from 1 to 80, from 2 to 80, from 5 to 70, from 10 to 50, or from 20 to 40); L is a linker, e.g., a linker described herein; and "epothilone" is an epothilone, e.g., an epothilone described herein, e.g., an epothilone shown in FIG. 5 or FIG. 6.

CDP-epothilone conjugates can be made using many different combinations of components described herein. For example, various combinations of cyclodextrins (e.g., beta-cyclodextrin), comonomers (e.g., PEG containing comonomers), linkers linking the cyclodextrins and comonomers, and/or linkers tethering the epothilone to the CDP are described herein.

FIG. 6 is a table depicting examples of different CDP-epothilone conjugates. The CDP-epothilone conjugates in FIG. 6 are represented by the following formula:

CDP-COABX-Epothilone

In this formula,
CDP is the cyclodextrin-containing polymer shown below (as well as in FIG. 3): 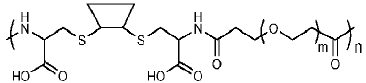 or 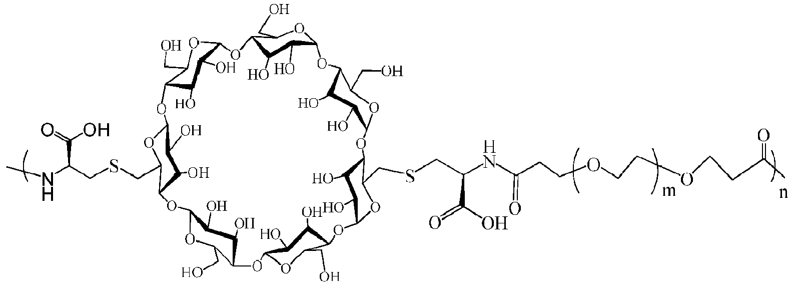 wherein for each example above, the group 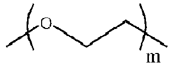 has a Mw of 3400 Da or less and n is at least 4, 5, 6, 7, 8, 9, 10, 11, 12, 13, 14, 15, 16, 17, 18, 19 or 20. Note that the epothilone is conjugated to the CDP through the carboxylic acid moieties of the polymer as provided above. Full loading of the epothilone onto the CDP is not required. In some embodiments, at least one, e.g., at least 2, 3, 4, 5, 6 or 7, of the carboxylic acid moieties remains unreacted with the epothilone after conjugation (e.g., a plurality of the carboxylic acid moieties remain unreacted).

CO represents the carbonyl group of the cysteine residue of the CDP;

A and B represent the link between the CDP and the epothilone. Position A is either a bond between linker B and the cysteine acid carbonyl of CDP (represented as a "-" in FIG. 6), a bond between the epothilone and the cysteine acid carbonyl of CDP (represented as a "-" in FIG. 6) or depicts a portion of the linker that is attached via a bond to the cysteine acid carbonyl of the CDP. Position B is either not occupied (represented by "-" in FIG. 6) or represents the linker or the portion of the linker that is attached via a bond to the epothilone; and

X represents the heteroatom to which the linker is coupled on the epothilone.

As provided in FIG. 6, the column with the heading "Epothilone" indicates which epothilone is included in the CDP-epothilone conjugate.

The three columns on the right of the table in FIG. 6 indicate respectively, what, if any, protecting groups are used to protect the X on the epothilone, the process for producing the CDP-epothilone conjugate, and the final product of the process for producing the CDP-epothilone conjugate.

The processes referred to in FIG. 6 are given a letter representation, e.g., Process A, Process B, Process C, etc. as seen in the second column from the right. The steps for each these processes respectively are provided below.
Process A: Couple the protected linker of position B to the epothilone, deprotect the linker and couple to CDP via the carboxylic acid group of the CDP to afford a mixture of 3- and 7-linked epothilone to CDP.
Process B: Couple the protected linker of position B to the epothilone, isolate 3-linked epothilone, and deprotect the linker and couple to CDP via the carboxylic acid group of the CDP to afford a 3-linked epothilone to CDP.
Process C: Couple the protected linker of position B to the epothilone, isolate 7-linked epothilone, deprotect the linker and couple to CDP via the carboxylic acid group of the CDP to afford a 7-linked epothilone to CDP.
Process D: Protect the epothilone, couple the protected linker of position B to an unprotected hydroxyl group of the epothilone, deprotect the linker and the epothilone hydroxyl protecting group, and couple to CDP via the carboxylic acid group of the CDP to afford a mixture of 3- and 7-linked epothilone to CDP.
Process E: Protect the epothilone, couple the protected linker of position B to an unprotected hydroxyl group of the epothilone, deprotect the linker protecting group, couple the linker to CDP via the carboxylic acid group of the CDP, and deprotect the hydroxyl protecting group to afford a mixture of 3- and 7-linked epothilone to CDP.
Process F: Protect the epothilone, isolate the 3-protected epothilone, couple the 3-protected epothilone to the protected linker of position B, deprotect linker and hydroxyl protecting group of the epothilone, and couple to CDP via the carboxylic acid group of the CDP to afford a 7-linked epothilone to CDP.
Process G: Protect the epothilone, isolate the 7-protected epothilone, couple to the protected linker of position B, deprotect linker and hydroxyl protecting group of the epothilone, and couple to CDP via the carboxylic acid group of the CDP to afford 3-linked epothilone to CDP.
Process H: Protect an amino group of the epothilone, couple the protected linker of position B to the epothilone, deprotect linker, couple to CDP via the carboxylic acid group of the CDP to afford a mixture of 3- and 7-linked epothilone to CDP, and deprotect the amino group of the epothilone.
Process I: Protect an amino group of the epothilone, couple the protected linker of position B to the epothilone, isolate the 3-linked epothilone, deprotect the linker, couple to CDP via the carboxylic acid group of the CDP to afford 3-linked epothilone to CDP, and deprotect the amino group of the epothilone.
Process J: Protect an amino group of the epothilone, couple the protected linker of position B to the epothilone, isolate the 7-linked epothilone, deprotect the linker, couple to CDP via the carboxylic acid group of the CDP to afford 7-linked epothilone to CDP, and deprotect the amino group of the epothilone.
Process K: Protect an amino group and a hydroxyl group of the epothilone, couple the protected linker of position B to an unprotected hydroxyl group of the epothilone, deprotect the linker and the hydroxyl group of the epothilone, couple to CDP via the carboxylic acid group of the CDP to afford a mixture of 3- and 7-linked epothilone to CDP, and deprotect the amino group of the epothilone.
Process L: Protect epothilone amino group and hydroxyl group, couple the protected linker of position B to unprotected hydroxyl group, deprotect linker protecting group, couple to CDP, deprotect hydroxyl protecting group to afford a mixture of 3- and 7-linked epothilone to CDP, and deprotect the amino group of the epothilone.
Process M: Protect an amino group and a hydroxyl group of the epothilone, isolate 3-protected epothilone, couple the epothilone to the linker of position B, deprotect the linker and the hydroxyl group of the epothilone, couple to CDP via the carboxylic acid group of the CDP to afford 7-linked epothilone to CDP, and deprotect the amino group of the epothilone.
Process N: Protect an amino group and a hydroxyl group of the epothilone, isolate 7-protected epothilone, couple the epothilone to the linker of position B, deprotect the linker and the hydroxyl group of the epothilone, couple to CDP via the carboxylic acid group of the CDP to afford 3-linked epothilone to CDP, and deprotect the amino group of the epothilone.
Process O: Couple the protected linker of position B to an amino group of epothilone, deprotect the linker, and couple to CDP via the carboxylic acid group to afford NH-linked epothilone to CDP.
Process P: Couple the activated linker of position B to the epothilone, and couple to CDP containing linker of position A via the linker of A to afford a mixture of 3- and 7-linked epothilone to CDP.
Process Q: Couple the activated linker of position B to the epothilone, isolate the 3-linked epothilone, and couple to the CDP containing linker of position A via the linker of A to afford the 3-linked epothilone to CDP.
Process R: Couple the activated linker of position B, isolate the 7-linked epothilone, and couple to the CDP containing linker of position A via the linker of A to afford 7-linked epothilone to CDP.
Process S: Protect the epothilone, couple the activated linker of position B to an unprotected hydroxyl group of the epothilone, deprotect the hydroxyl group of the epothilone, and couple to the CDP containing linker of position A via the linker of A to afford a mixture of 3- and 7-linked epothilone to CDP.
Process T: Protect the epothilone, couple the activated linker of position B to an unprotected hydroxyl group of the epothilone, couple to the CDP containing linker of position A via the linker of A, and deprotect hydroxyl group of the epothilone to afford a mixture of 3- and 7-linked epothilone to CDP.
Process U: Protect the epothilone, isolate the 3-protected epothilone, couple the epothilone to the activated linker of position B, deprotect the hydroxyl protecting group of the epothilone, and couple to the CDP containing linker of position A to afford the 7-linked epothilone to CDP.
Process V: Protect the epothilone, isolate the 7-protected epothilone, couple to the activated linker of position B, deprotect the hydroxyl group of the epothilone, and couple to CDP containing linker of position A via the linker of A to afford the 3-linked epothilone to CDP.
Process W: Couple the epothilone directly to CDP via the free amino group of the epothilone to the carboxylic acid group of the CDP to form NH-linked epothilone to CDP.
Process X: Couple the activated linker of position B to an amino group of epothilone, and couple to CDP containing linker of position A via the linker of A to form NH-linked epothilone to CDP.
Process Y: Protect the epothilone, isolate the 3-protected epothilone, couple the epothilone to the linker of position B, deprotect the linker, and couple to CDP via the carboxylic acid group of CDP to afford the 7-linked epothilone to CDP.
Process Z: Protect the epothilone, isolate the 7-protected epothilone, couple to the protected linker of position B, deprotect linker, and couple to CDP via the carboxylic acid group of CDP to afford the 3-linked epothilone to CDP.
Process AA: Protect the amino and hydroxyl groups of the epothilone, isolate 3-protected epothilone, couple to the protected linker of position B, deprotect the linker, and couple to CDP via the carboxylic acid group of CDP to afford 7-linked epothilone to CDP.
Process BB: Protect the amino and hydroxyl groups of the epothilone, isolate 7-protected epothilone, couple to the protected linker of position B, deprotect the linker, and couple to CDP via the carboxylic acid group of the CDP to afford 3-linked epothilone to CDP.
Process CC: Protect an amino group of the epothilone, couple the activated linker of position B to the epothilone, couple to CDP containing linker of position A via the linker of A to afford a mixture of 3- and 7-linked epothilone to CDP, and deprotect the amino group of the epothilone.
Process DD: Protect an amino group of the epothilone, couple the activated linker of position B to the epothilone, isolate the 3-linked epothilone, couple to the CDP containing linker of position A via the linker of A to afford the 3-linked epothilone to CDP, and deprotect the amino group of the epothilone.
Process EE: Protect an amino group of the epothilone, couple the activated linker of position B, isolate the 7-linked epothilone, couple to the CDP containing linker of position A via the linker of A to afford 7-linked epothilone to CDP, and deprotect the amino group of the epothilone.
Process FF: Protect an amino group and a hydroxyl group of the epothilone, couple the activated linker of position B to an unprotected hydroxyl group of the epothilone, deprotect the hydroxyl group of the epothilone, couple to the CDP containing linker of position A via the linker of A to afford a mixture of 3- and 7-linked epothilone to CDP, and deprotect the amino group of the epothilone.
Process GG: Protect an amino group and a hydroxyl group of the epothilone, couple the activated linker of position B to an unprotected hydroxyl group of the epothilone, couple to the CDP containing linker of position A via the linker of A, deprotect hydroxyl group of the epothilone to afford a mixture of 3- and 7-linked epothilone to CDP, and deprotect the amino group of the epothilone.
Process HH: Protect an amino group and a hydroxyl group of the epothilone, isolate the 3-protected epothilone, couple the epothilone to the activated linker of position B, deprotect the hydroxyl protecting group of the epothilone, couple to the CDP containing linker of position A to afford the 7-linked epothilone to CDP, and deprotect the amino group of the epothilone.
Process II: Protect an amino group and a hydroxyl group of the epothilone, isolate the 7-protected epothilone, couple to the activated linker of position B, deprotect the hydroxyl group of the epothilone, couple to CDP containing linker of position A via the linker of A to afford the 3-linked epothilone to CDP, and deprotect the amino group of the epothilone.

As shown specifically in FIG. 6, the CDP-epothilone conjugates can be prepared using a variety of methods known in the art, including those described herein. In some embodiments, the CDP-epothilone conjugates can be prepared using no protecting groups on the epothilone. For example, the CDP-epothilone conjugates can be prepared as a mixture (e.g., where there are two free hydroxyl groups on the epothilone) at the time the epothilone is coupled to the CDP or the linker. The mixture can be coupled with a linker, e.g., a linker of position A, which is attached to the cysteine acid carbonyl of the CDP. The mixture may also be directly coupled with the CDP, i.e., the cysteine acid carbonyl of the CDP.

In some embodiments, the CDP-epothilone conjugates can be prepared using a protecting group on a hydroxyl group of the epothilone that is not used as a point of attachment to the CDP. When a linker is present, e.g., a linker of position B, the linker can be coupled to the epothilone at an unprotected point of attachment, e.g., at an unprotected hydroxyl group of the epothilone. In one embodiment, the epothilone can be deprotected and a linker of position B can be coupled to CDP via linker of position A. When a linker of position A is present, it can be attached to cysteine acid carbonyl of the CDP. Position A may also be a bond, and therefore, the coupling of the epothilone and/or epothilone linker B may be directly with the CDP, i.e., the cysteine acid carbonyl of the CDP.

In some embodiments, the CDP-epothilone conjugates can be prepared using a prodrug protecting group on a hydroxyl group of the epothilone that is not used as a point of attachment to the CDP. When linker of position B is present, the linker can be coupled to the epothilone without deprotecting the epothilone. For example, the prodrug can be an ester group that remains on a hydroxyl group of the epothilone and a different hydroxyl group of the epothilone can be used as the point of attachment to the CDP (see, e.g., examples 289-400 of FIG. 6). In some embodiments, the protected epothilone can be coupled to the CDP via a linker of position A. When position A includes a linker, the linker at position A is attached to the cysteine acid carbonyl of the CDP. Position A may also be a bond, and therefore, the coupling may be directly with the CDP, i.e., the cysteine acid carbonyl of the CDP.

One or more protecting groups can be used in the processes described above to make the CDP-epothilone conjugates described herein. A protecting group can be used to control the point of attachment of the epothilone and/or epothilone linker to position A. In some embodiments, the protecting group is removed and, in other embodiments, the protecting group is not removed. If a protecting group is not removed, then it can be selected so that it is removed *in vivo* (e.g., acting as a prodrug). An example is hexanoic acid which has been shown to be removed by lipases *in vivo* if used to protect a hydroxyl group in doxorubicin. Protecting groups are generally selected for both the reactive groups of the epothilone and the reactive groups of the linker that are not targeted to be part of the coupling reaction. The protecting group should be removable under conditions which will not degrade the epothilone and/or linker material. Examples include t-butyldimethylsilyl ("TBDMS") and TROC (derived from 2,2,2-trichloroethoxy chloroformate). Carboxybenzyl ("CBz") can also be used in place of TROC if there is selectivity seen for removal over olefin reduction. This can be addressed by using a group which is more readily removed by hydrogentation such as -methoxybenzyl OCO-. Other protecting groups may also be acceptable. One of skill in the art can select suitable protecting groups for the products and methods described herein.

Although the products in FIG. 6 corresponding to processes E, L, T, and FF result in a mixture of 3- and 7-linked epothilone to CDP. These processes can be readily modified to produce a product having an epothilone linked by a single group, e.g., linked either through the 3- position only or 7- position only. For example, a 3- linked epothilone to CDP can be produced in methods E, L, T, and FF by separating and isoloating a pure isomer of the 7-protected epothilone prior to coupling of the epothilone to the CDP; and a 7- linked epothilone to CDP can be produced in methods E, L, T, and FF by separating and isoloating a pure isomer of the 3-protected epothilone prior to coupling of the epothilone to the CDP.

In some embodiments, microtubule inhibitor in the CDP-microtubule inhibitor conjugate is an a vinca alkaloid, e.g., vinblastine (Velban® or Velsar®), vincristine (Vincasar® or Oncovin®), vindesine (Eldisine®), vinorelbine (Navelbine®).

In some embodiments, the anti-tumor antibiotic in the CDP-anti-tumor antibiotic conjugate, particle or composition is an antibiotic including, without limitation, actinomycin (Cosmegen®), bleomycin (Blenoxane®), hydroxyurea (Droxia® or Hydrea®), mitomycin (Mitozytrex® or Mutamycin®).

In an embodiment, the therapeutic agent in the CDP-therapeutic agent conjugate is a cytotoxic agent such as a kinase inhibitor. In some embodiments, the kinase inhibitor in the CDP-kinase inhibitor conjugate, particle or composition is a kinase inhibitor including, without limitation, a seronine/threonine kinase inhibitor conjugate, e.g., an mTOR inhibitor, e.g., rapamycin (RapDane®).

In an embodiment, the therapeutic agent in the CDP-therapeutic agent conjugate is a cytotoxic agent such as a proteasome inhibitor. In some embodiments, the proteasome inhibitor in the CDP-proteasome inhibitor conjugate, particle or composition is a boronic acid containing molecule or a boronic acid derivative, e.g., bortezomib (Velcade®). Other proteasome inhibitors described herein can also be included in the CDP-proteasome inhibitor conjugates.

As used herein, a boronic acid derivative is represented by R-B(Y)₂, wherein each Y is a group that is readily displaced by an amine or alcohol group on a liker L to form a covalent bond (e.g., conjugating the therapeutic agent (e.g., a proteasome inhibitor containing a boronic acid or derivative thereof to the CDP)). Examples of boronic acid derivatives include boronic ester (e.g., RB(O-alkyl)₂), boronic amides (e.g., RB(N(alkyl)₂)₂), alkoxyboranamine (e.g., RB(O-alkyl)(N(alkyl)₂); and boronic acid anhydride. Mixed boronic acid derivatives are also included, such as RB(O-alkyl)(N(alkyl)₂).

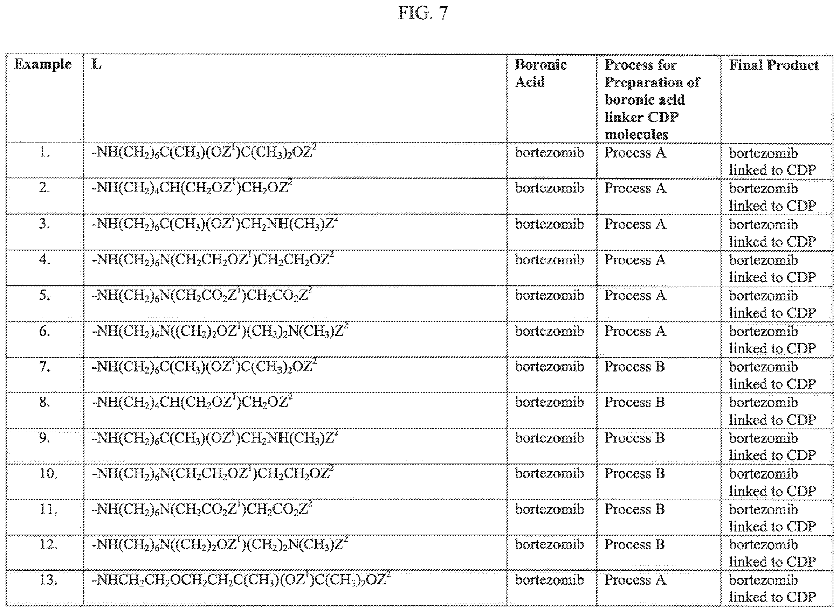

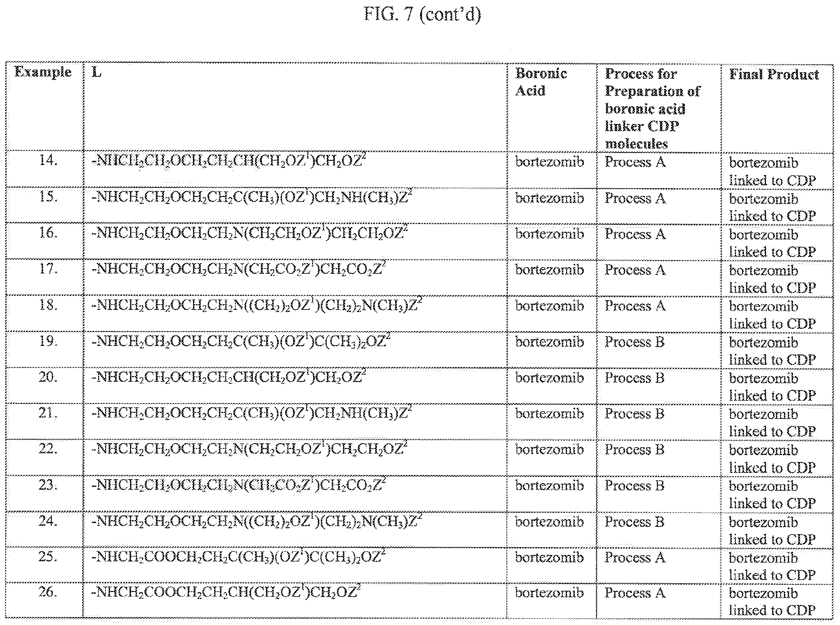

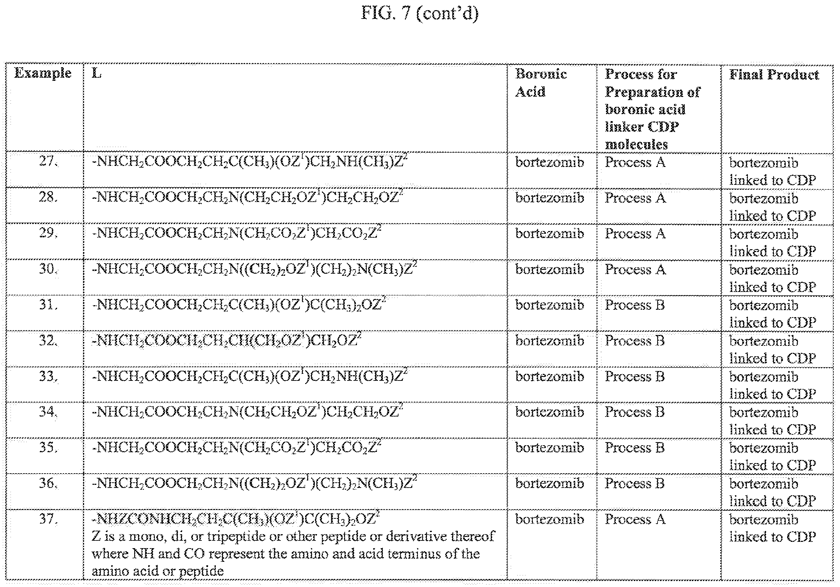

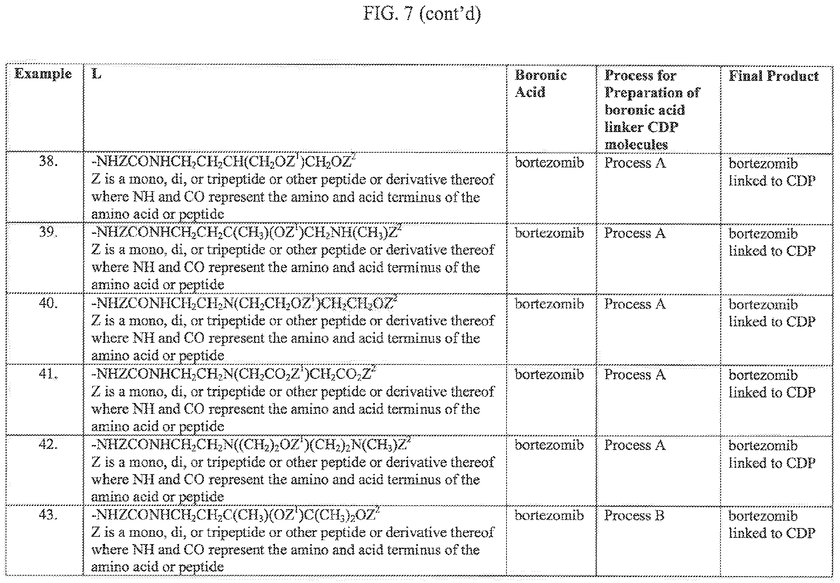

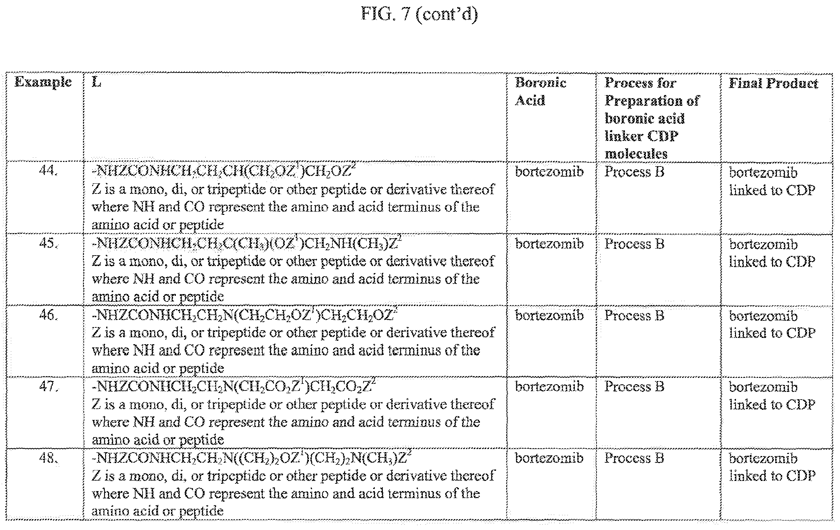

A number of CDP-L-boronic acid structures are shown in FIG. 7, wherein the structures for the CDP-proteasome inhibitor are represented by CDP-L-boronic acid, wherein Z¹ and Z² each represent bonds to the boron atom of the conjugated drug. For example, the CDP-bortezomib conjugate is represented by CDP-L-B-(*L*)-CH(CH₂CH(CH₃)₂)NH-(*L*)-Phe-CO-pyrazine. In FIG. 7 Z¹ and Z² each represents a bond to the boron atom of the boronic acid. Process A comprises: 1) couple linker, optionally protected, to CDP, 2) deprotect linker if protected, 3) conjugate to boronic acid. Process B comprises: 1) conjugate linker, optionally protected, to boronic acid, 2) deprotect linker if protected, 3) couple to CDP.

In one embodiment, for the CDP-proteasome inhibitor conjugates described in any one of 1^{st} to 15^{th} embodiments (below) wherein the proteasome inhibitor contains a boronic acid or derivative thereof, RB(OH)₂ or RB(Y)₂ is represented by formula (1a) below: 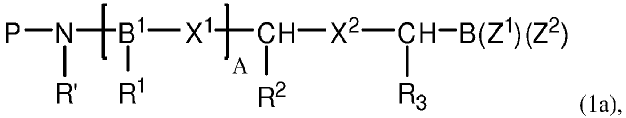 or a pharmaceutically acceptable salts thereof, wherein:
P is hydrogen or an amino-group-protecting moiety;
B¹, at each occurrence, is independently one of N or CH;
X¹, at each occurrence, is independently one of -C(O)-NH-, -CH₂-NH-, -CH(OH)-CH₂-, -CH(OH)-CH(OH)-, -CH(OH)-CH₂-NH-, -CH=CH-, -C(O)CH₂-, -SO₂-NH-, -SO₂-CH₂- or -CH(OH)-CH₂-C(O)-NH-, provided that when B¹ is N, then the X¹ attached to said B¹ is -C(O)-NH-;
X² is one of -C(O)-NH-, -CH(OH)-CH₂-, -CH(OH)-CH(OH)-, -C(O)-CH₂-, -SO₂-NH-, -SO₂ -CH₂- or -CH(OH)-CH₂-C(O)-NH-;
R' is hydrogen or alkyl, or R forms together with the adjacent R¹, or when A is zero, forms together with the adjacent R², a nitrogen-containing mono-, bi- or tri-cyclic, saturated or partially saturated ring system having 4-14 ring members, that can be optionally substituted by one or two of keto, hydroxy, alkyl, aryl, aralkyl, alkoxy or aryloxy;
R¹, at each occurrence, is independently one of hydrogen, alkyl, cycloalkyl, aryl, a 5-10 membered saturated, partially unsaturated or aromatic heterocycle or -CH₂ -R⁵, where the ring portion of any of said aryl, aralkyl, alkaryl or heterocycle can be optionally substituted;
R² is one of hydrogen, alkyl, cycloalkyl, aryl, a 5-10 membered saturated, partially unsaturated or aromatic heterocycle or -CH-R⁵, where the ring portion of any of said aryl, aralkyl, alkaryl or heterocycle can be optionally substituted;
R³ is one of hydrogen, alkyl, cycloalkyl, aryl, a 5-10 membered saturated, partially unsaturated or aromatic heterocycle or -CH₂-R⁵, where the ring portion of any of said aryl, aralkyl, alkaryl or heterocycle can be optionally substituted;
R⁵, in each instance, is one of aryl, aralkyl, alkaryl, cycloalkyl, a 5-10 membered saturated, partially unsaturated or aromatic heterocycle or -W-R⁶, where W is a chalcogen and R⁶ is alkyl, where the ring portion of any of said aryl, aralkyl, alkaryl or heterocycle can be optionally substituted;
Z¹ and Z² are independently one of alkyl, hydroxy, alkoxy, or aryloxy, or together Z¹ and Z² form a moiety derived from a dihydroxy compound having at least two hydroxy groups separated by at least two connecting atoms in a chain or ring, said chain or ring comprising carbon atoms, and optionally, a heteroatom or heteroatoms which can be N, S, or O; and A is 0, 1, or 2.

In one embodiment, for formula (1a):
P is R' or R⁷-C(=O)- or R⁷-SO₂-, wherein R⁷ selected from the group consisting of 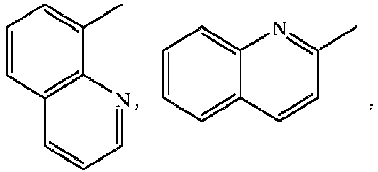 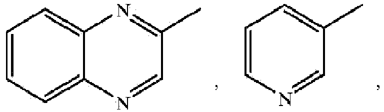 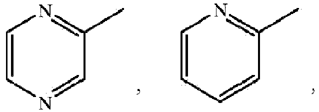 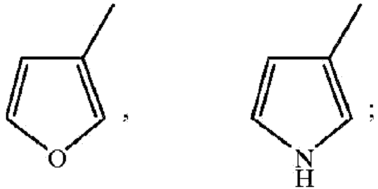
or P is 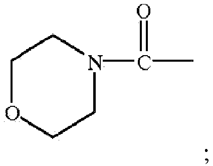
X₂ is selected from the group consisting of 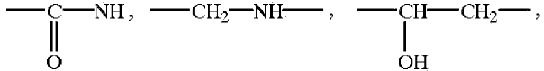 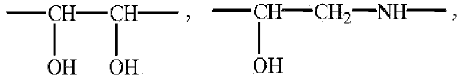 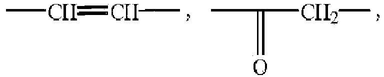 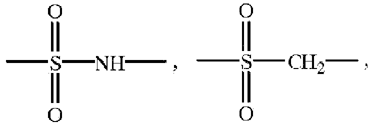 and 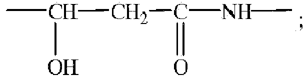
R' is hydrogen or alkyl;
R₂ and R₃ are independently selected from the group consisting of hydrogen, alkyl, cycloalkyl, aryl, heterocycle and -CH₂-R₅, where R₅ is aryl, aralkyl, alkaryl, cycloalkyl, heterocycle or -Y-R₆,
where Y is a chalcogen, and R₆ is alkyl;
Z₁ and Z₂ are independently alkyl, hydroxy, alkoxy, aryloxy, or together form a dihydroxy compound having at least two hydroxy groups separated by at least two connecting atoms in a chain or ring, said chain or ring comprising carbon atoms, and optionally, a heteroatom or heteroatoms which can be N, S, or O; and A is 0.

In another embodiment, for structural formula (1a):
P is R₇-C(O)- or P₇-SO₂-, where R₇ is pyrazinyl;
X₂ is -C(O)-NH-;
R' is hydrogen or alkyl;
R₂ and R₃ are independently hydrogen, alkyl, cycloalkyl, aryl, or -CH₂-R₅;
R₅ in each instance, is one of aryl, aralkyl, alkaryl, cycloalkyl, or -W-R₆, where W is a chalcogen and R₆ is alkyl;
where the ring portion of any of said aryl, aralkyl, or alkaryl in R₂, R₃ and R₅ can be optionally substituted by one or two substituents independently selected from the group consisting of C₁₋₆ alkyl, C₃₋₈ cycloalkyl, C₁₋₆ alkyl(C₃₋₈)cycloalkyl, C₂₋₈ alkenyl, C₂₋₈ alkynyl, cyano, amino, C₁₋₆ alkylamino, di(C₁₋₆)alkylamino, benzylamino, dibenzylamino, nitro, carboxy, carbo(C₁₋₆)alkoxy, trifluoromethyl, halogen, C₁₋₆ alkoxy, C₆₋₁₀ aryl, C₆₋₁₀ aryl(C₁₋₆)alkyl, C₆₋₁₀ aryl(C₁₋₆)alkoxy, hydroxy, C₁₋₆ alkylthio, C₁₋₆ alkylsulfinyl, C₁₋₆ alkylsulfonyl, C₆₋₁₀ arylthio, C₆₋₁₀ arylsulfinyl, C₆₋₁₀ arylsulfonyl, C₆₋₁₀ aryl, C₁₋₆alkyl(C₆₋₁₀) aryl, and halo(C₆₋₁₀)aryl;
Z₁ and Z₂ are independently one of hydroxy, alkoxy, or aryloxy, or together Z₁ and Z₂ form a moiety derived from a dihydroxy compound having at least two hydroxy groups separated by at least two connecting atoms in a chain or ring, said chain or ring comprising carbon atoms, and optionally, a heteroatom or heteroatoms which can be N, S, or O; and
A is zero.

In one embodiment, for CDP-proteasome inhibitor conjugates described in any one of the 1^{st} to 15^{th} embodiments (below) wherein the proteasome inhibitor contains a boronic acid or derivative thereof, RB(OH)₂ or its analog is represented by formula 2a below 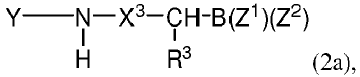 or a pharmaceutically acceptable salts thereof, wherein:
Y is one of R⁸ -C(O)-, R⁸-SO₂ -, R⁸-NH-C(O)- or R⁸-O-C(O)-, where R⁸ is one of alkyl, aryl, alkaryl, aralkyl, any of which can be optionally substituted, or when Y is R⁸ -C(O)- or R⁸-SO₂-, then R⁸ can also be an optionally substituted 5-10 membered, saturated, partially unsaturated or aromatic heterocycle;
X³ is a covalent bond or -C(O)-CH₂-;
R³ is one of hydrogen, alkyl, cycloalkyl, aryl, a 5-10 membered saturated, partially unsaturated or aromatic heterocycle or -CH₂-R⁵, where the ring portion of any of said aryl, aralkyl, alkaryl or heterocycle can be optionally substituted;
R⁵, in each instance, is one of aryl, aralkyl, alkaryl, cycloalkyl, a 5-10 membered saturated, partially unsaturated or aromatic heterocycle or -W-R⁶, where W is a chalcogen and R⁶ is alkyl, where the ring portion of any of said aryl, aralkyl, alkaryl or heterocycle can be optionally substituted; and
Z¹ and Z² are independently alkyl, hydroxy, alkoxy, aryloxy, or together form a moiety derived from dihydroxy compound having at least two hydroxy groups separated by at least two connecting atoms in a chain or ring, said chain or ring comprising carbon atoms, and optionally, a heteroatom or heteroatoms which can be N, S, or O;
provided that when Y is R⁸-C(O)-, R⁸ is other than phenyl, benzyl or C₁-C₃ alkyl. Alternatively, the group Y in formula (2a) above, can be as provided in formula 3a below: 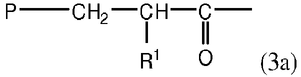
P is one of R⁷-C(O)-, R⁷-SO₂-, R⁷-NH-C(O)- or R⁷-O-C(O)-;
R⁷ is one of alkyl, aryl, alkaryl, aralkyl, any of which can be optionally substituted, or when Y is R⁷-C(O)- or R⁷-SO₂-, R⁷ can also be an optionally substituted 5-10 membered saturated, partially unsaturated or aromatic heterocycle; and
R¹ is defined above as for formula (1a).

In one embodiments, compounds of formula (1a) or (2a) described above are compounds depicted in Table 1.

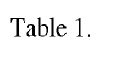

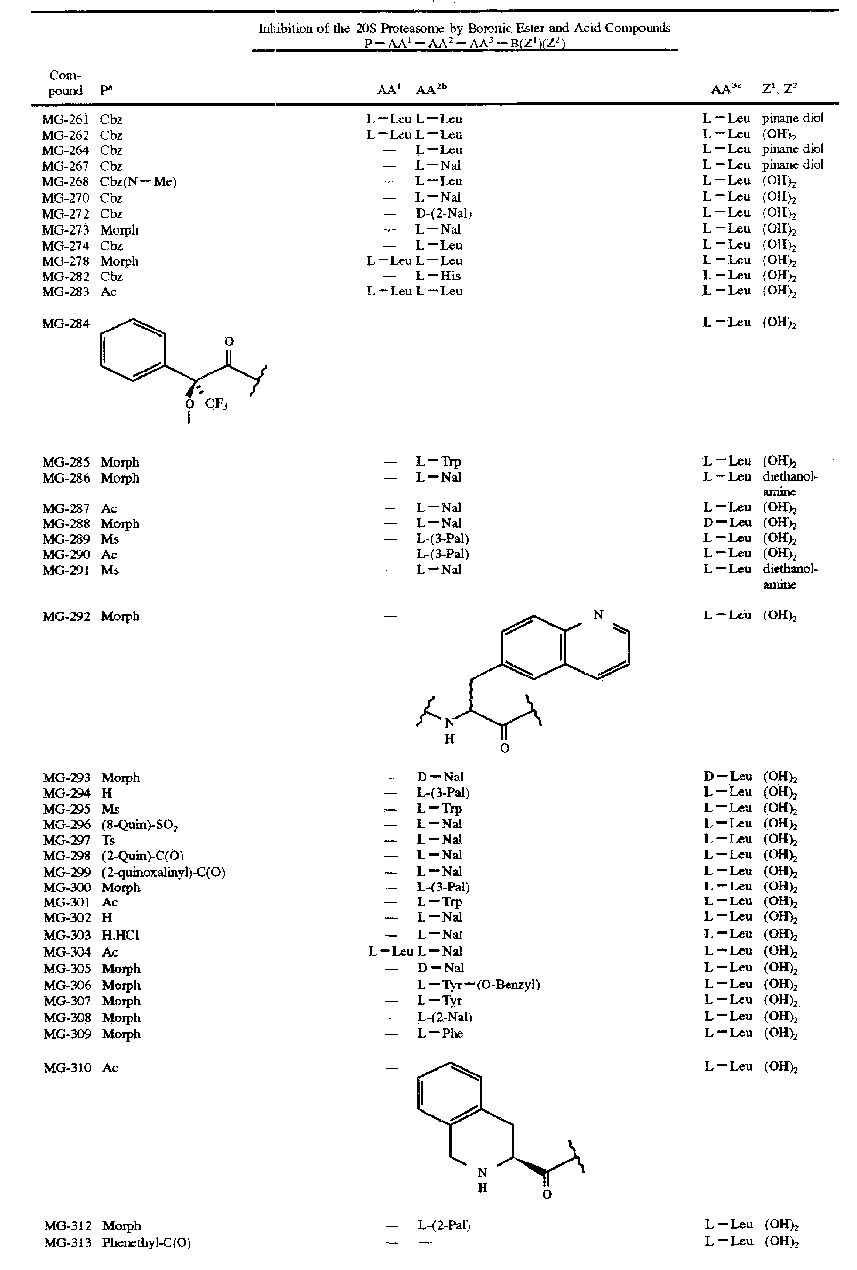

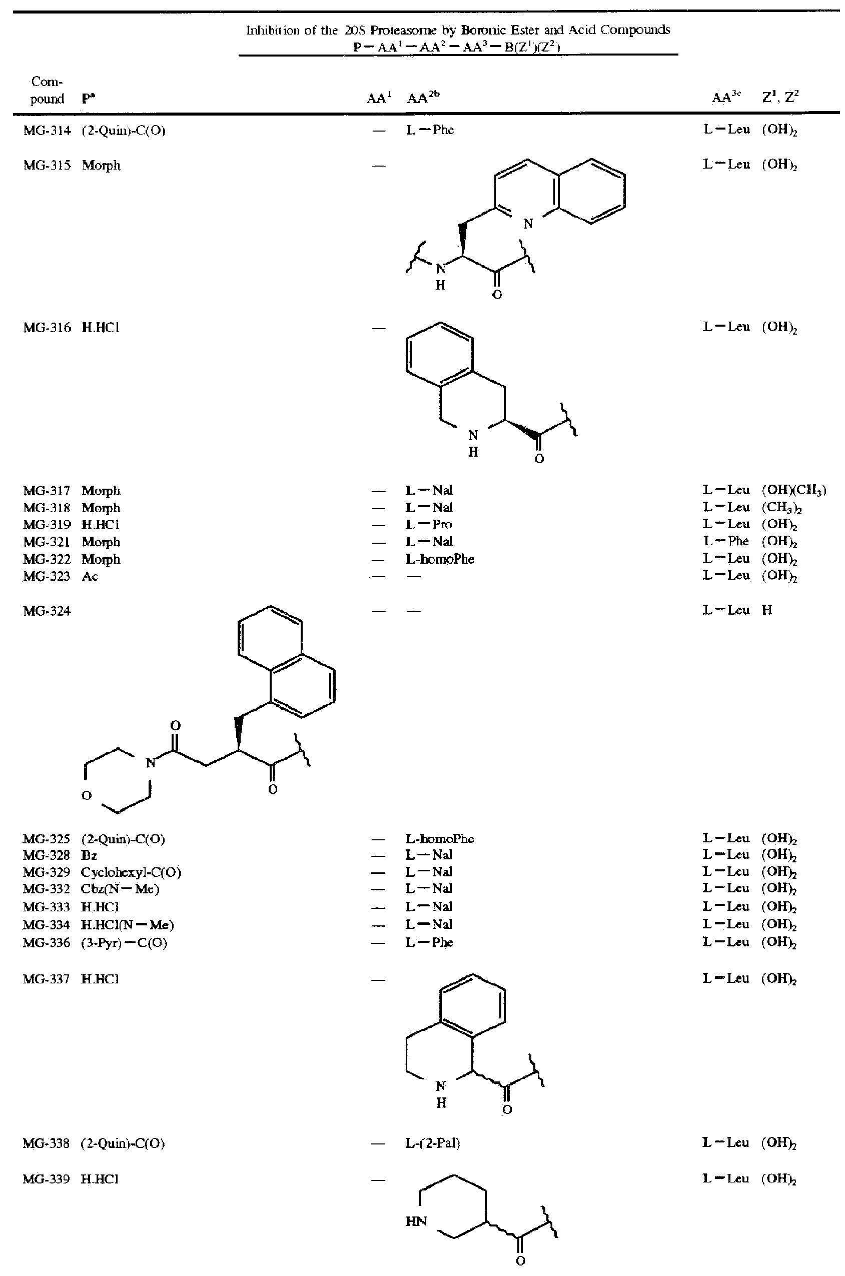

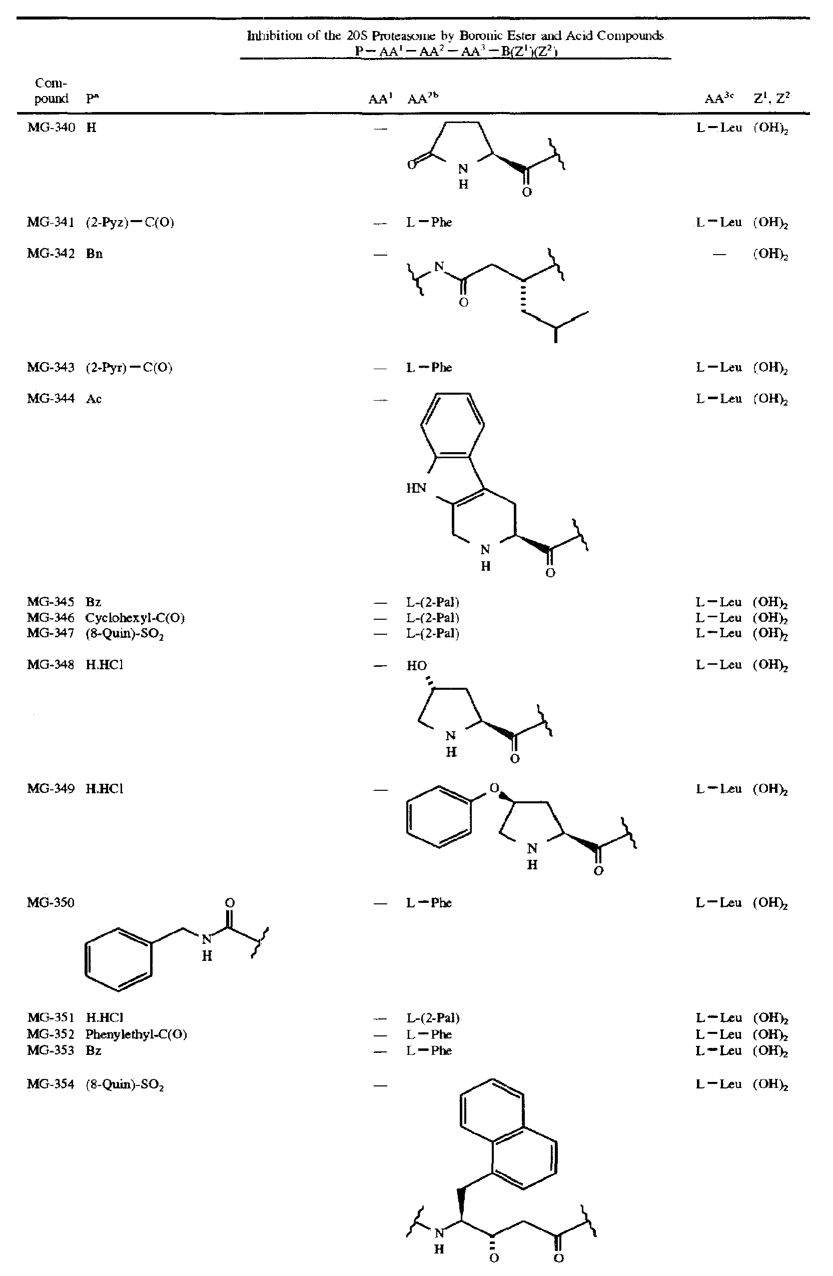

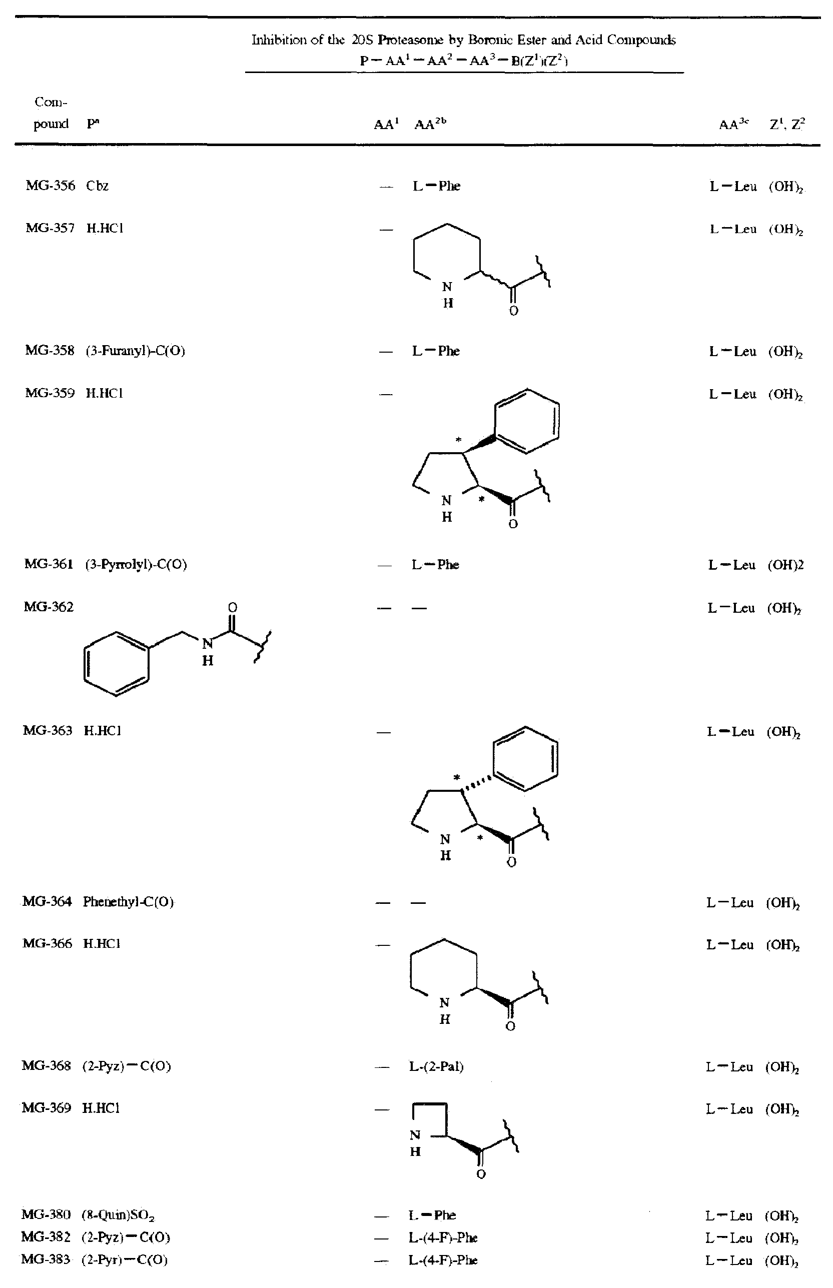

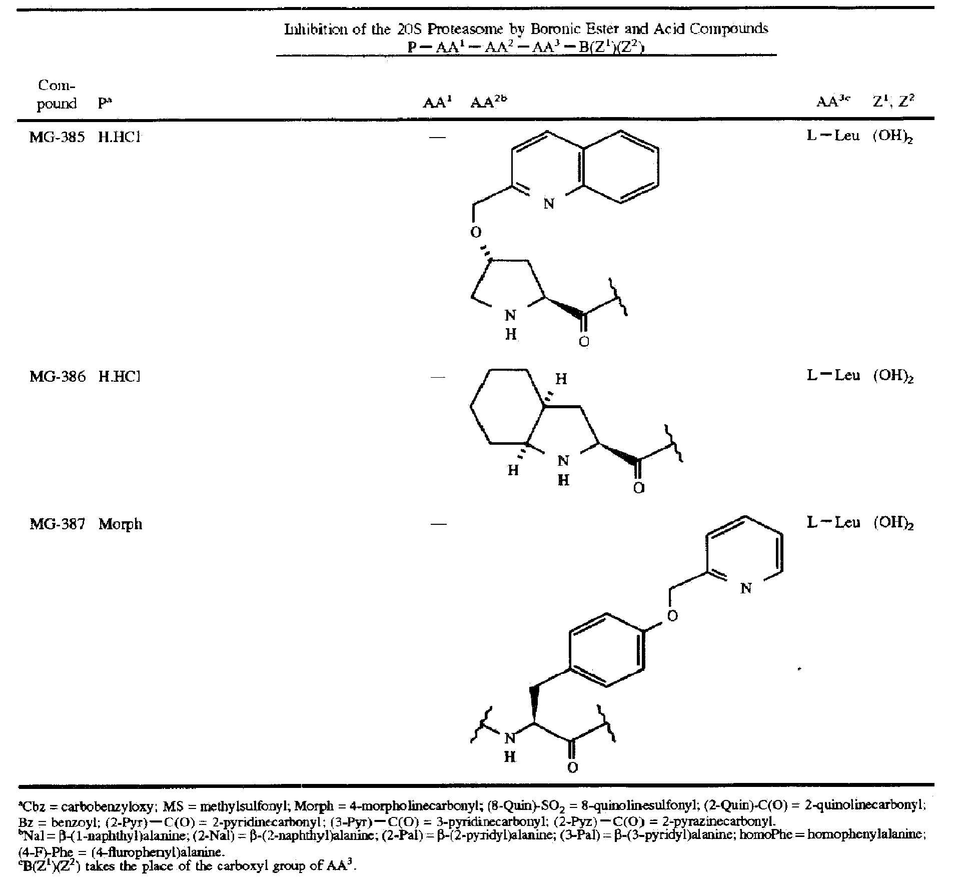

In another embodiment, compounds of formula (1a) or (2a) described above are selected from the following compounds as well as pharmaceutically acceptable salts and boronate esters thereof:
N-(4-morpholine)carbonyl-β-(1-naphthyl)-L-alanine-L-leucine boronic acid,
   N-(8-quinoline)sulfonyl-β-(1-naphthyl)-L-alanine-L-leucine boronic acid,
   N-(2-pyrazine)carbonyl-L-phenylalanine-L-leucine boronic acid,
   L-proline-L-leucine boronic acid,
   N-(2-quinoline)carbonyl-L-homophenylalanine-L-leucine boronic acid,
   N-(3-pyridine)carbonyl-L-phenylalanine-L-leucine boronic acid,
   N-(3-phenylpropionyl)-L-phenylalanine-L-leucine boronic acid,
   N-(4-morpholine)carbonyl-L-phenylalanine-L-leucine boronic acid,
   N-(4-morpholine)carbonyl-(O-benzyl)-L-tyrosine-L-leucine boronic acid,
N-(4-morpholine)carbonyl-L-tyrosine-L-leucine boronic acid, and
   N-(4-morpholine)carbonyl-[O-(2-pyridylmethyl)]-L-tyrosine-L-leucine boronic acid.

In one embodiment, for the CDP-proteasome inhibitor conjugates described in any one of 1^{st} to 15^{th} embodiments wherein the proteasome inhibitor contains a boronic acid or derivative thereof, RB(OH)₂ or RB(Y)₂ is represented by the formula (3b): 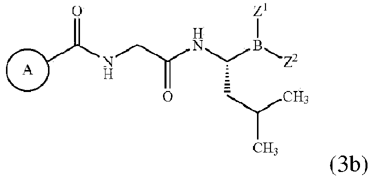 or a pharmaceutically acceptable salt or boronic acid anhydride thereof, wherein: Z¹ and Z² are each independently hydroxy, alkoxy, aryloxy, or aralkoxy; or Z¹ and Z² together form a moiety derived from a boronic acid completing agent; and
Ring A is selected from the group consisting of: 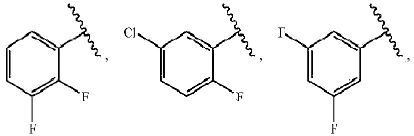 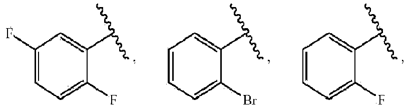 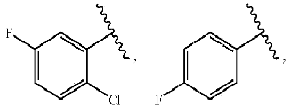 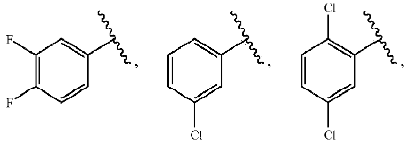 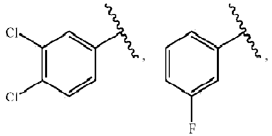 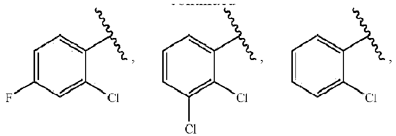 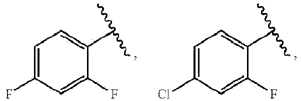 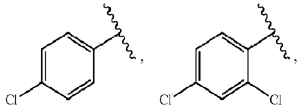 and 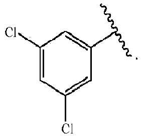 More specifically, compounds of formula (3b) are referred to by the following chemical names:
I-1 [(1R)-1-({[(2,3-difluorobenzoyl)amino]acetyl}amino)-3-methylbutyl]boronic acid
T-2 [(1R)-1-({[(5-chloro-2-fluorobenzoyl)amino]acetyl}amino)-3-methylbutyl]boronic acid
I-3 [(1R)-1-({[(3,5-difluorobenzoyl)amino]acetyl}amino)-3-methylbutyl]boronic acid
I-4 [(1R)-1-({[(2,5-difluorobenzoyl)amino]acetyl}amino)-3-methylbutyl]boronic acid
I-5 [(1R)-1-({[(2-bromobenzoyl)amino]acetyl}amino)-3-methylbutyl]boronic acid
I-6 [(1R)-1-({[(2-fluorobenzoyl)amino]acetyl}amino)-3-methylbutyl]boronic acid
I-7 [(1R)-1-({[(2-chloro-5-fluorobenzoyl)amino]acetyl}amino)-3-methylbutyl]boronic acid
I-8 [(1R)-1-({[(4-fluorobenzoyl)amino]acetyl}amino)-3-methylbutyl]boronic acid
I-9 [(1R)-1-({[(3,4-difluorobenzoyl)amino]acetyl}amino)-3-methylbutyl]boronic acid
I-10 [(1R)-1-({[(3-chlorobenzoyl)amino]acetyl}amino)-3-methylbutyl]boronic acid
I-11 [(1R)-1-({[(2,5-dichlorobenzoyl)amino]acetyl}amino)-3-methylbutyl]boronic acid
I-12 [(1R)-1-({[(3,4-dichlorobenzoyl)amino]acetyl}amino)-3-methylbutyl]boronic acid
I-13 [(1R)-1-({[(3-fluorobenzoyl)amino]acetyl}amino)-3-methylbutyl]boronic acid
I-14 [(1R)-1-({[(2-chloro-4-fluorobenzoyl)amino]acetyl}amino)-3-methylbutyl]boronic acid
I-15 [(1R)-1-({[(2,3-dichlorobenzoyl)amino]acetyl}amino)-3-methylbutyl]boronic acid
I-16 [(1R)-1-({[(2-chlorobenzoyl)amino]acetyl}amino)-3-methylbutyl]boronic acid
I-17 [(1R)-1-({[(2,4-difluorobenzoyl)amino]acetyl}amino)-3-methylbutyl]boronic acid
I-18 [(1R)-1-({[(4-chloro-2-fluorobenzoyl)amino]acetyl}amino)-3-methylbutyl]boronic acid
I-19 [(1R)-1-({[(4-chlorobenzoyl)amino]acetyl}amino)-3-methylbutyl]boronic acid
I-20 [(1R)-1-({[(2,4-dichlorobenzoyl)amino]acetyl}amino)-3-methylbutyl]boronic acid
I-21 [(1R)-1-({[(3,5-dichlorobenzoyl)amino]acetyl}amino)-3-methylbutyl]boronic acid.

In another embodiment, for the CDP-proteasome inhibitor conjugates described in any one of the 1^{st} to 15^{th} embodiments (below) wherein the proteasome inhibitor contains a boronic acid or derivative thereof, RB(OH)₂ or RB(Y)₂ is represented by formula (4a): 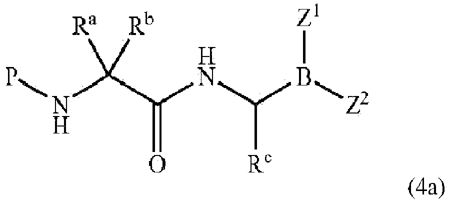 or a pharmaceutically acceptable salt or boronic acid anhydride thereof, wherein:
P is hydrogen or an amino-group-blocking moiety;
R^{a} is a C₁₋₄ aliphatic or C₁₋₄ fluoroaliphatic group that is substituted with 0-1 R^{A}; or R^{a} and R^{b} taken together with the carbon atom to which they are attached, form a substituted or unsubstituted 3- to 6-membered cycloaliphatic group;
   R^{A} is a substituted or unsubstituted aromatic or cycloaliphatic ring;
R^{b} is a C₁₋₄ aliphatic or C₁₋₄ fluoroaliphatic group; or R^{a} and R^{b}, taken together with the carbon atom to which they are attached, form a substituted or unsubstituted 3- to 6-membered cycloaliphatic group;
R^{c} is a C₁₋₄ aliphatic or C₁₋₄ fluoroaliphatic group that is substituted with 0-1 R^{C};
   R^{C} is a substituted or unsubstituted aromatic or cycloaliphatic ring; and
Z¹ and Z² are each independently hydroxy, alkoxy, aryloxy, or aralkoxy; or Z¹ and Z² together form a moiety derived from a boronic acid complexing agent. Representative examples of compounds of formula (4a), wherein Z¹ and Z² are each -OH are shown as the following:

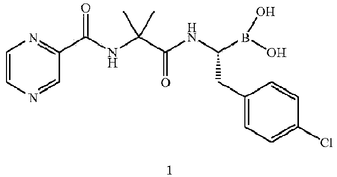

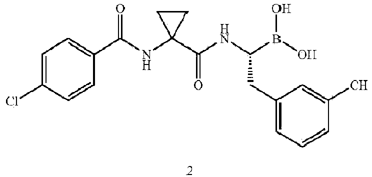

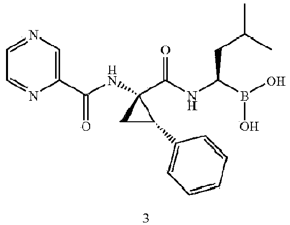

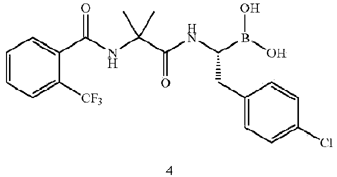

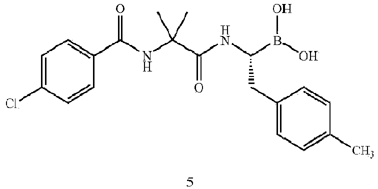

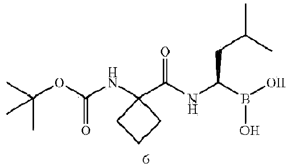

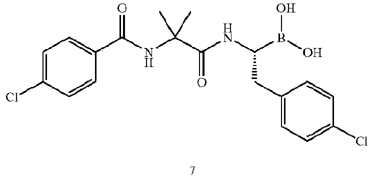

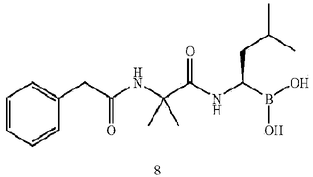

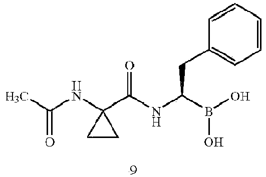

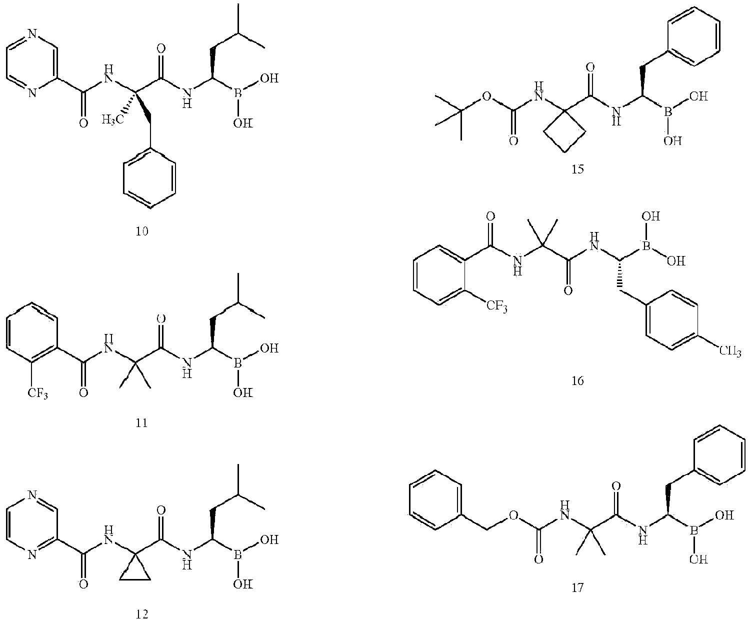

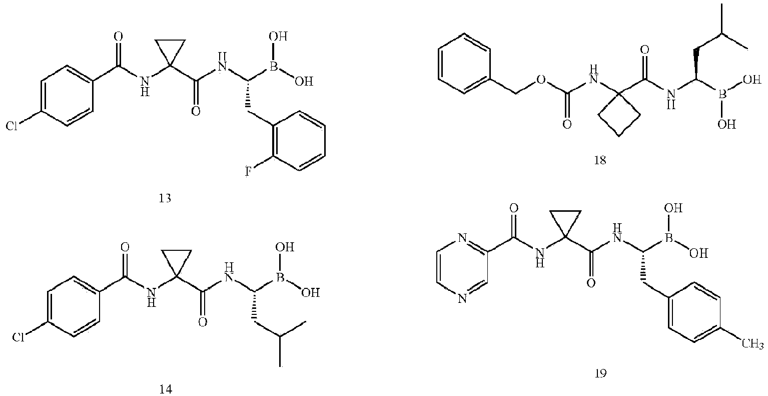

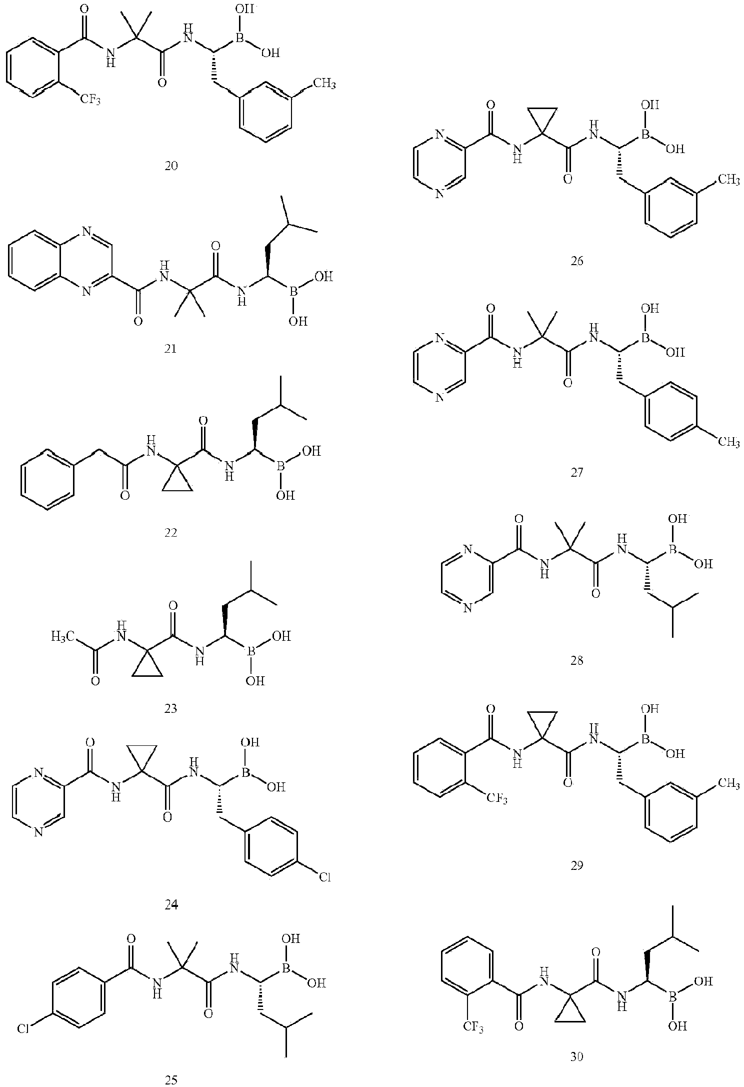

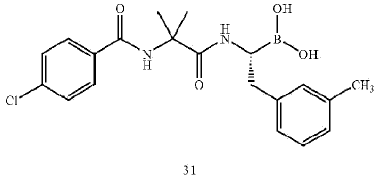

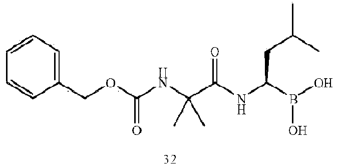

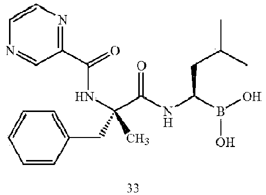

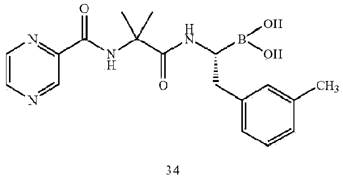

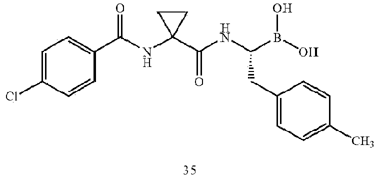

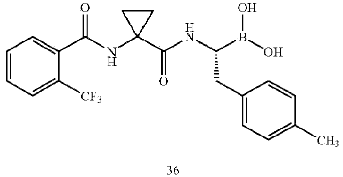

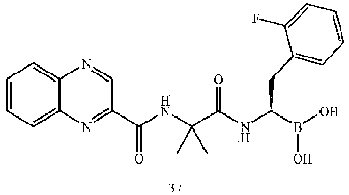

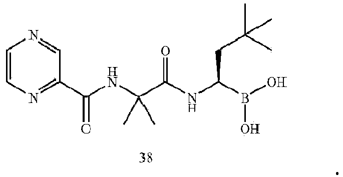

In preferred embodiments, the proteasome inhibitor in the CDP-proteasome inhibitor conjugate, particle or composition comprises a boronic acid containing molecule, e.g., a boronic acid containing molecule described herein, e.g., bortezomib;

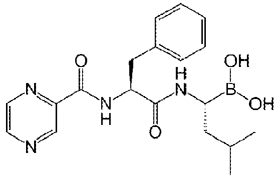

In one embodiment, the CDP-proteasome inhibitor conjugate is a CDP-bortezomib conjugate, e.g., 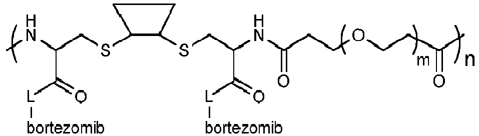 wherein 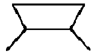 represents a cyclodextrin; n is an integer from 1 to 100 (e.g., n is an integer from 4 to 80, from 4 to 50, from 4 to 30 or from 4 to 20, or n is 4, 5, 6, 7, 8, 9, 10, 11, 12, 13, 14, 15, 16, 17, 18, 19 or 20); m is an integer from 1 to 1000 (e.g., m is an integer from 1 to 200, from 1 to 100, from 1 to 80, from 2 to 80, from 5 to 70, from 10 to 50, or from 20 to 40); and "L-bortezemib" is a bortezemib-linker moiety, e.g., a bortezemib-linker moiety described herein, e.g., a bortezemib-linker moiety described in FIG. 7. In some embodiments, the CDP-proteasome inhibitor conjugate, e.g., the CDP-bortezomib conjugate, does not have complete loading, e.g., one or more binding sites, e.g., cysteine residues, are not bound to a proteasome inhibitor, e.g., a bortezomib moiety, bound with a linker described herein, e.g., the CDP-bortezemib conjugate comprises one or more subunits having the formulae provided below: 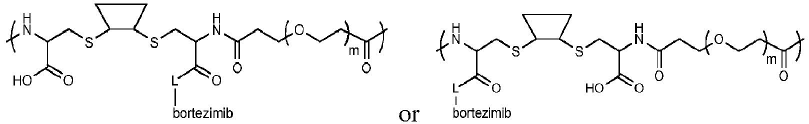 wherein 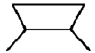 represents a cyclodextrin; m is an integer from 1 to 1000 (e.g., m is an integer from 1 to 200, from 1 to 100, from 1 to 80, from 2 to 80, from 5 to 70, from 10 to 50, or from 20 to 40); and "L-bortezemib" is a bortezemib-linker moiety, e.g., a bortezemib-linker moiety described herein, e.g., a bortezemib-linker moiety described in FIG. 7. In some embodiments, the CDP-proteasome inhibitor conjugate, particle or composition e.g., the CDP-bortezomib conjugate, particle or composition, comprises a mixture of fully-loaded and partially-loaded CDP-proteasome inhibitor conjugates, e.g., CDP-bortezomib conjugates.

In one embodiment, the CDP-proteasome inhibitor conjugate comprises a subunit of 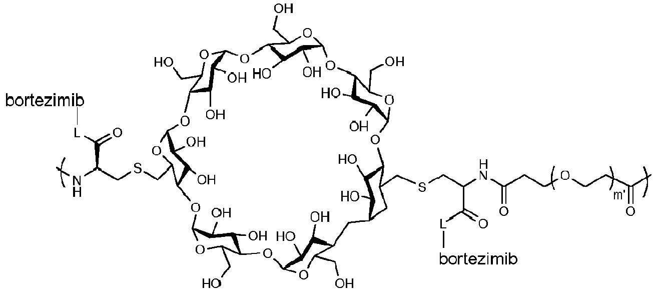 wherein m is an integer from 1 to 1000 (e.g., m is an integer from 1 to 200, from 1 to 100, from 1 to 80, from 2 to 80, from 5 to 70, from 10 to 50, or from 20 to 40); and "L-bortezemib" is a bortezemib-linker moiety, e.g., a bortezemib-linker moiety described herein, e.g., a bortezemib-linker moiety described in FIG. 7.

The CDP-proteasome inhibitor conjugate (such as a boronic acid containing proteasome inhibitor) of the invention comprises a proteasome inhibitor (such as a boronic acid containing proteasome inhibitor, e.g., bortezomib) covalently linked to a CDP described herein. In one embodiment, the proteasome inhibitor is a pharmaceutically active agent, preferably comprises a boronic acid moiety or a boronic acid derivative described herein.

In the 1^{st} embodiment, the CDP-proteasome inhibitor conjugate is formula (K) below: 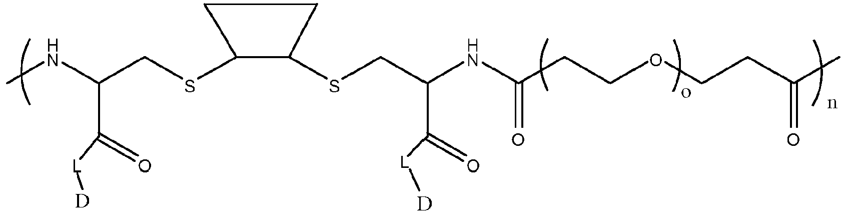 wherein:
n is an integer from 1 to 100;
o is an integer from 1 to 1000;
L is a linker described in Formulas (I)-(VIII); and
D is -B-R, wherein R is as described in RB(OH)₂ or RB(Y)₂ described above.

In another embodiment, the L-D moiety in formula (K) is represented by the following formula: 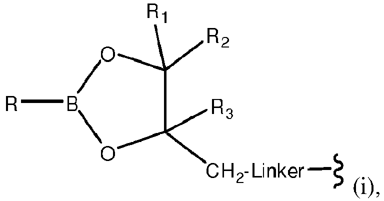 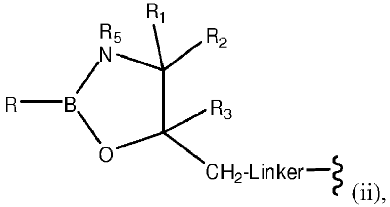 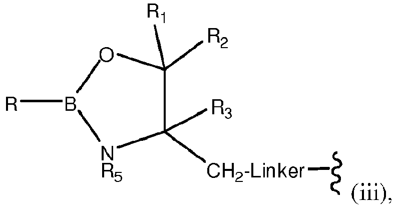 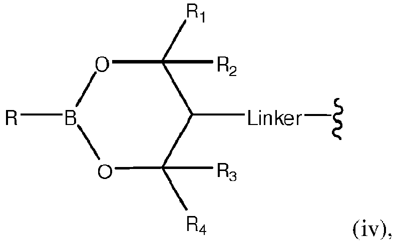 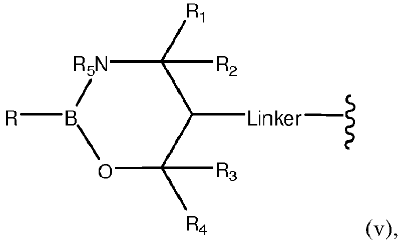 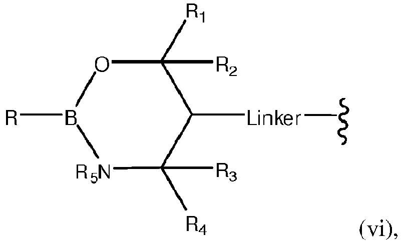 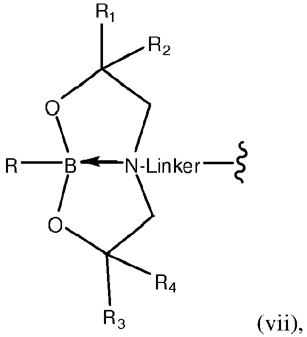 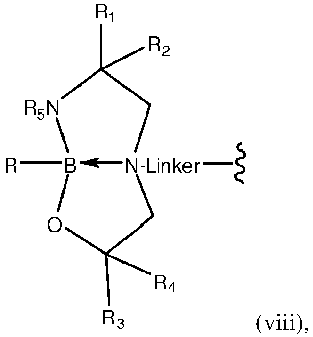 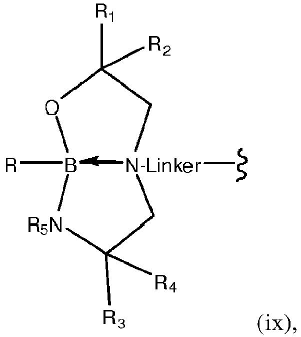 or 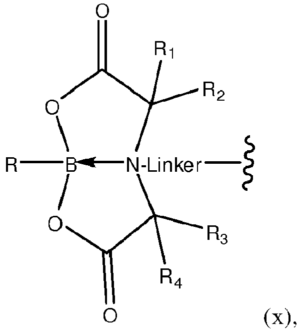 wherein:
R is the non-boronic acid moiety in R-B(OH)₂ or R is as described in a boronic acid derivative RB(Y)₂ described herein;
RB(OH)₂ is a pharmaceutically active agent, preferably a proteasome inhibitor comprising a boronic acid moiety, such as bortezomib;
RB(Y)₂ is a pharmaceutically active agent, preferably a proteasome inhibitor such as a proteosome inhibitor comprising a boronic acid derivative;
R₁, R₂, R₃, R₄ and R₅ are each independently -H or a (C₁-C₅)alkyl;
Linker is a linker group comprising an amino terminal group.

In a 2^{nd} embodiment, for CDP-proteasome inhibitor conjugate represented by formulas (K), the L-D moiety is represented by a formula selected from: 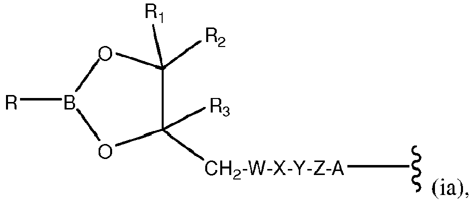 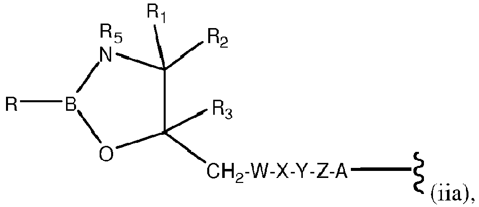 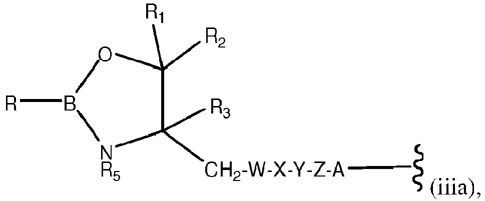 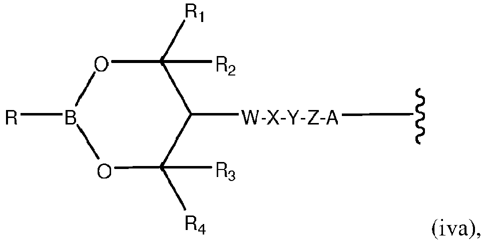 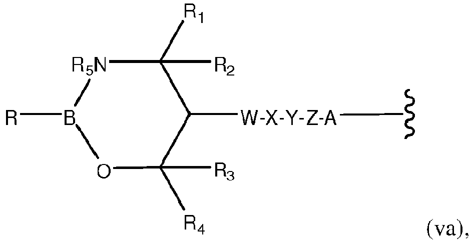 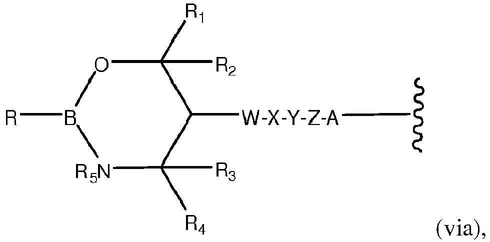 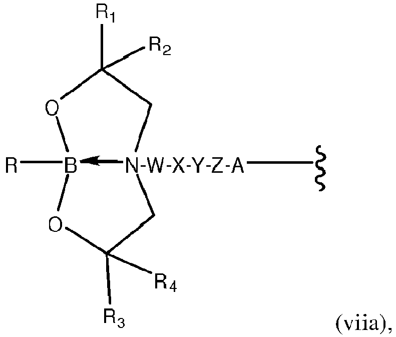 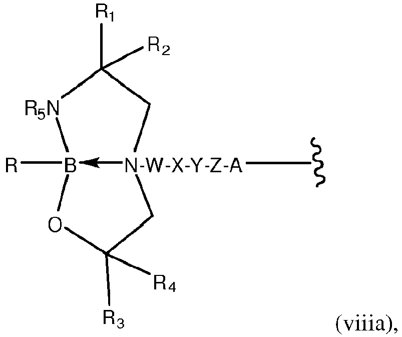 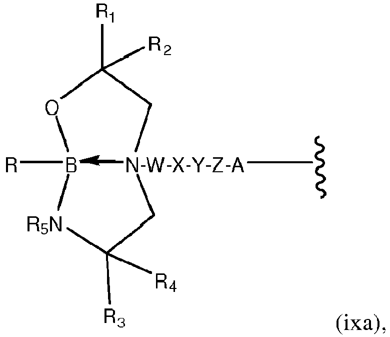 and 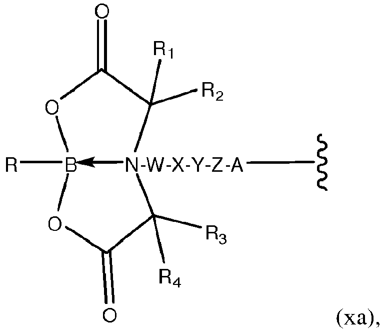 wherein:
R₁, R₂, R₃, R₄ and R₅ are each independently -H or a (C₁-C₅)alkyl;
R is as described in RB(OH)₂ or RB(Y)₂ described above;
W is -(CH₂)ₘ-, -O- or -N(R₅')-, when the polymer-agent conjugate is represented by structural formulas (ia)-(via); or
W is -(CH₂)ₘ-, when the polymer-agent conjugate is represented by structural formulas (viia)-(xa);
X is a bond when W is -(CH₂)ₘ- and X is -C(=O)- when W is -O-, or -N(R₅');
Y is a bond, -O-, or -N(R₅')-;
Z is represented by the following structural formula:

   -(CH₂)ₚ-Q-(CH₂)_{q}-E-;
E is a bond, aryl (e.g., phenyl) or heteroaryl (e.g., pyridyl, furyl or furanyl, imidazolyl, benzimidazolyl, pyrimidinyl, thiophenyl or thienyl, quinolinyl, indolyl and thiazolyl);
Q is a bond, -O-, -N(R₅')-, -N(R₅')-C(=O)-O-, -O-C(=O)-N(R₅')-, -OC(=O)-, -C(=O)-O-, -S-S-, -(O-CH₂-CH₂)ₙ- or 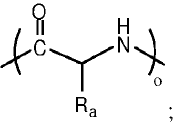
R^{a} is a side chain of a naturally occurring amino acid or an analog thereof;
A is -N(R₅')-, or A is a bond when Q is 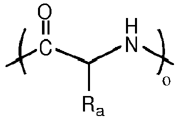 and q is 0;
R₅' is -H or (C₁-C₆)alkyl;
m, p, q are each an integer from 0 to 10;
n is an integer from 1 to 10; and
o is an integer from 1 to 10, provided when Y is -O- or -N(R₅')- and Q is -O-, -N(R₅')-, -(O-CH₂-CH₂)ₙ-, -N(R₅')-C(=O)-O-, -O-C(=O)-N(R₅')-, -OC(=O)- or -S-S-, then p is an integer from 2 to 10; when Q is -O-, -N(R₅')-, -N(R₅')-C(=O)-O-, -O-C(=O)-N(R₅')-, -OC(=O)-, -C(=O)-O-, or -S-S- and E is a bond, then q is an integer from 2 to 10; when Y is -O- or -N(R₅')-, Q and E are both a bond, then p+q ≥ 2; when W is -O- or -N(R₅')-, Y, Q and E are all bond, then p+q ≥ 1; and when W is -O- or -N(R₅')-, Y is a bond, and Q is -N(R₅')-C(=O)-O-, -O-C(=O)-N(R₅')-, -OC(=O)-, -C(=O)-O-, -S-S- or -(O-CH₂-CH₂)ₙ-, then p is an integer from 2 to 10.

In one embodiment, Z is a bond or -(CH₂)ᵣ-, wherein r is an integer from 1 to 10.

In a 3^{rd} embodiment, for CDP-proteasome inhibitor conjugate described in the 2^{nd} embodiment, the linker (i.e. -W-X-Y-Z-A) is represented by any one of the following formula: 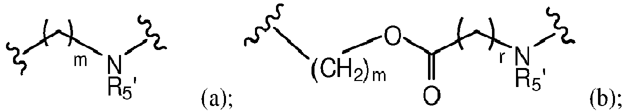 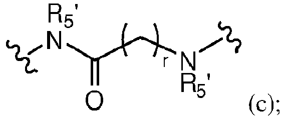 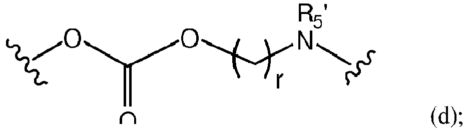 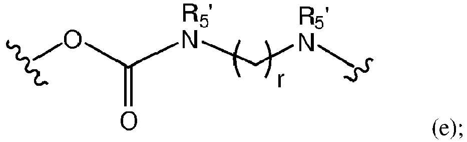 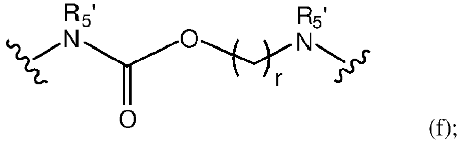 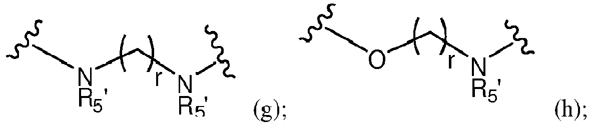 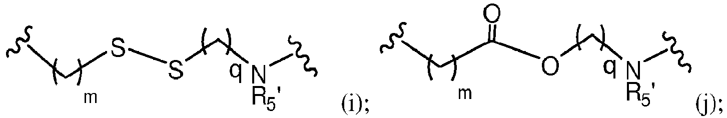 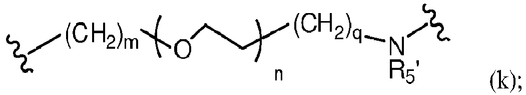 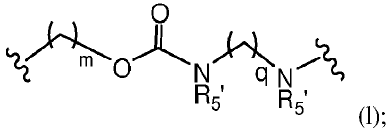 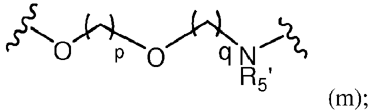 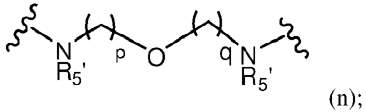 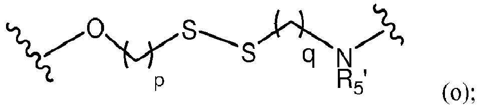 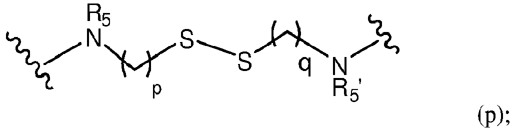 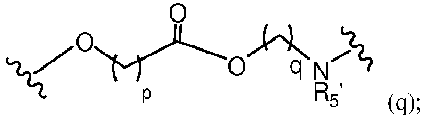 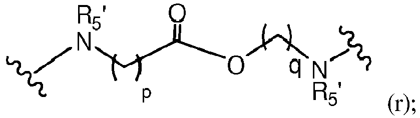 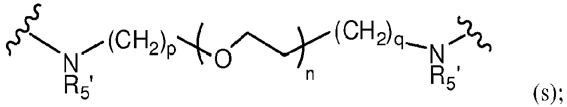 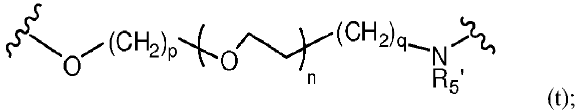 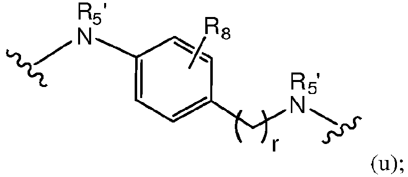 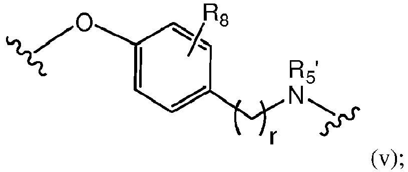 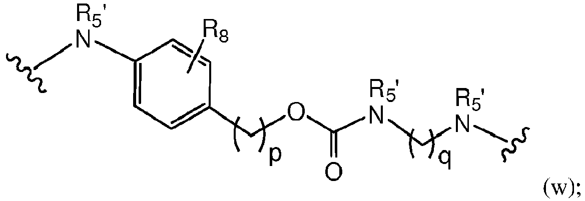 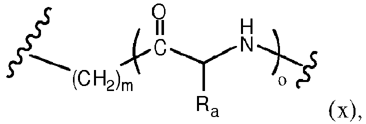 wherein R₅' is -H or (C₁-C₆)alkyl; Rₐ is a side chain of a naturally occurring amino acid or an analog thereof; R₈ is a substituent; n is an integer from 1 to 10; r is an integer from 1 to 10; m, p and q are each an integer from 0 to 10; and o is an integer from 1 to 10. For formulas (d)-(h), r is an integer from 2 to 10. For formulas (i), (j) and (1), q is an integer from 2 to 10. For formulas (m)-(p), p and q are each an integer from 2 to 10. For formulas (q) and (r), p is an integer from 1 to 10 and q is an integer from 2 to 10. For formulas (s) and (t), p is an integer from 2 to 10. For formula (w), q is an integer from 2 to 10. More specifically, R₈ is selected from H, halo, -CN, -NO₂, -OH, (C₁-C₆)alkyl, halo(C₁-C₆)alkyl, hydroxy(C₁-C₆)alkyl, (C₁-C₆)alkoxy, halo(C₁-C₆)alkoxy, (C₁-C₃)alkoxy(C₁-C₃)alkyl and -NR₉R₁₀; wherein R₉ and R₁₀ are each independently H, (C₁-C₆)alkyl, halo(C₁-C₆)alkyl, (C₁-C₆)alkoxy, halo(C₁-C₆)alkoxy, (C₁-C₃)alkoxy(C₁-C₃)alkyl.

In a 4^{th} embodiment, for CDP-proteasome inhibitor conjugate described in the 3^{rd} embodiment, the linker (i.e., -W-X-Y-Z-A) is represented by any one of the following formulas: 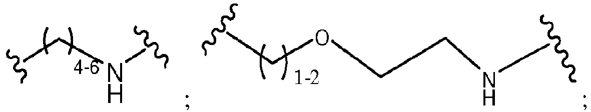 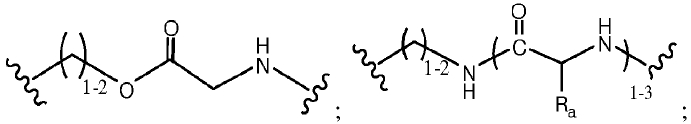 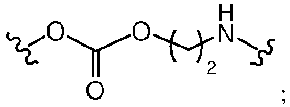 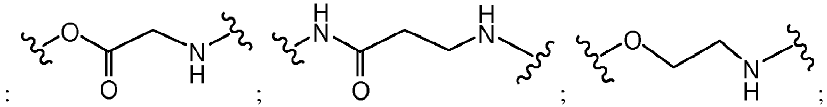 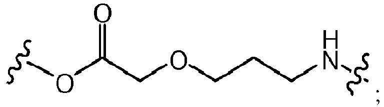 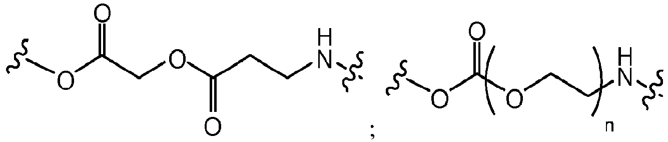 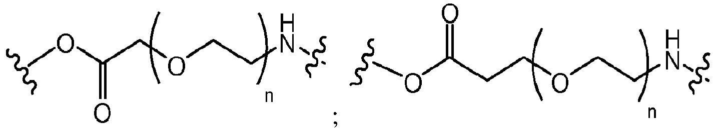 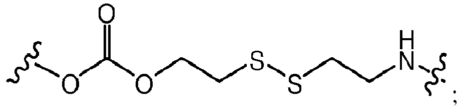 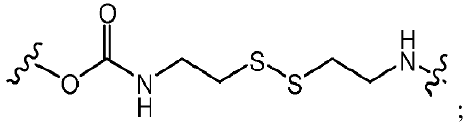 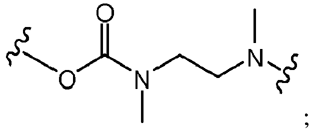 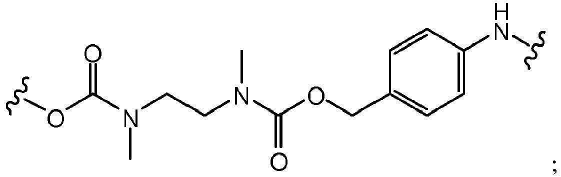 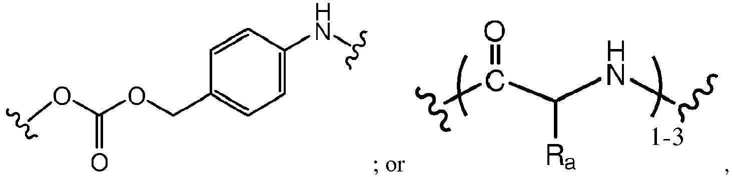 wherein n is an integer from 2 to 5; and Rₐ is a side chain of a naturally occurring amino acid or an analog thereof.

In a 5^{th} embodiment, for the CDP-proteasome inhibitor conjugate described in the 1^{st} embodiment, the linker is represented by formulas (AA1), (BB1) or (CC1):

-(CH₂)ₘ-O-CH₂-O-(CH₂)_{q}-N(R₅)- (AA1),

-(CH₂)ₘ-O-(CH₂)ₚ-O-CH₂-N(R₅)- (BB1)

-(CH₂)ₘ-(CH₂)ₚ-O-CH₂-N(R₅)- (CC1)

wherein m is an integer from 0 to 10; q is an integer from 2 to 10; p is an integer from 0 to 10 for structural formula (CC1) and p is an integer from 2 to 10 for structural formula (BB1).

In a 6^{th} embodiment, for CDP-proteasome inhibitor conjugate of formula (K) described in the 1^{st} embodiment, the L-D moiety is as described in FIG. 7.

In a 7^{th} embodiment, the CDP-proteasome inhibitor conjugate is represented by the following formula: 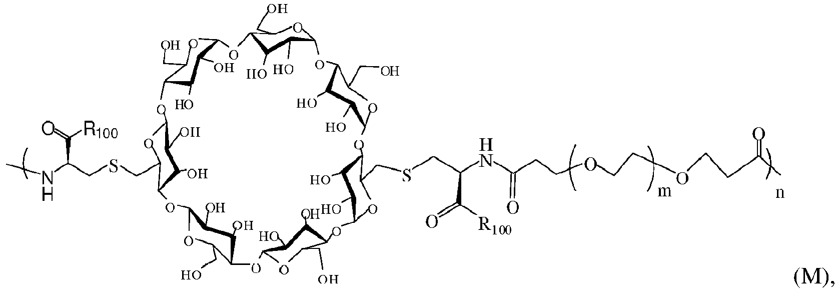 wherein n is an integer from 1 to 100 (e.g., n is an integer from 4 to 80, from 4 to 50, from 4 to 30 or from 4 to 20, or n is 4, 5, 6, 7, 8, 9, 10, 11, 12, 13, 14, 15, 16, 17, 18, 19 or 20); m is an integer from 1 to 1000 (e.g., m is an integer from 1 to 200, from 1 to 100, from 1 to 80, from 2 to 80, from 5 to 70, from 10 to 50, or from 20 to 40); and R₁₀₀ is - OH or a group comprising a -B-R moiety, wherein R is as described in RB(OH)₂ or RB(Y)₂ described above. At least one R₁₀₀ in the conjugate is a group comprising a -B-R moiety. Alternatively, the conjugate represented by formula (M) comprises at least 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9 or 2.0 R₁₀₀ groups represented by a group comprising a -B-R moiety per repeat unit. In one embodiment, at least one R₁₀₀ in the conjugate is a group comprising a -B-R moiety and R is represented by the following structural formula:

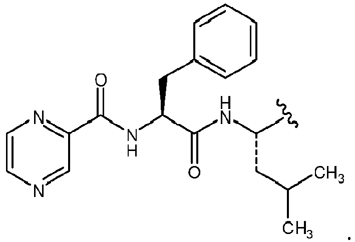

Alternatively, the conjugate represented by formula (M) comprises at least 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9 or 2.0 R₁₀₀ groups represented by a group comprising a -B-R moiety per repeat unit and R is represented by the following structural formula:

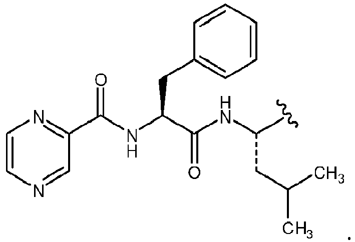

In a 8^{th} embodiment, the CDP-proteasome inhibitor conjugate is represented by formula (M): 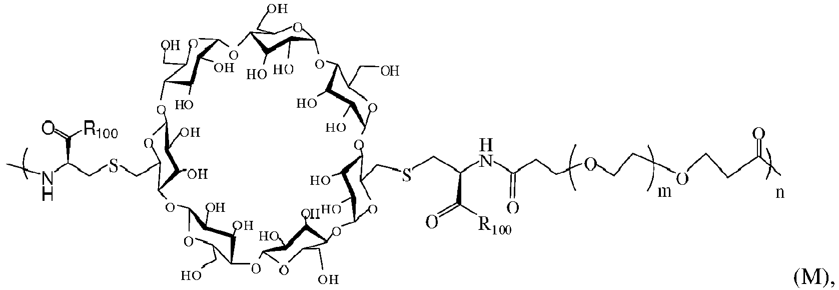 wherein n is an integer from 1 to 100 (e.g., n is an integer from 4 to 80, from 4 to 50, from 4 to 30 or from 4 to 20, or n is 4, 5, 6, 7, 8, 9, 10, 11, 12, 13, 14, 15, 16, 17, 18, 19 or 20); m is an integer from 1 to 1000 (e.g., m is an integer from 1 to 200, from 1 to 100, from 1 to 80, from 2 to 80, from 5 to 70, from 10 to 50, or from 20 to 40); R₁₀₀ is -OH or a group represented by a formula selected from formulas (i)-(x). At least one R₁₀₀ group in the conjugate is a group represented by a formula selected from formulas (i)-(x). Alternatively, the conjugate represented by formula (M) comprises at least 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9 or 2.0 R₁₀₀ groups represented by a formula selected from formulas (i)-(x) per repeat unit.

In a 9^{th} embodiment, for the CDP-proteasome inhibitor conjugate represented by formula (M), n is an integer from 1 to 100 (e.g., n is an integer from 4 to 80, from 4 to 50, from 4 to 30 or from 4 to 20, or n is 4, 5, 6, 7, 8, 9, 10, 11, 12, 13, 14, 15, 16, 17, 18, 19 or 20); m is an integer from 1 to 1000 (e.g., m is an integer from 1 to 200, from 1 to 100, from 1 to 80, from 2 to 80, from 5 to 70, from 10 to 50, or from 20 to 40); R₁₀₀ is -OH or a group represented by a formula selected from formulas (i)-(x). At least one R₁₀₀ group in the conjugate is a group represented by a formula selected from formulas (i)-(x); and R in formulas (i)-(x) is as described in RB(OH)₂ or RB(Y)₂ described above. More specifically, at least one R₁₀₀ group in the conjugate is a group represented by a formula selected from formulas (i)-(x); and R in formulas (i)-(x) is represented by the following structural formula:

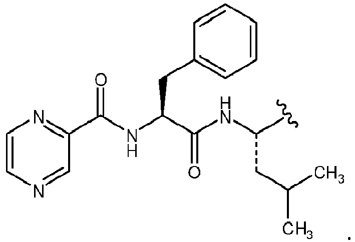

Alternatively, the CDP-proteasome inhibitor conjugate represented by formula (M) comprises at least 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9 or 2.0 R₁₀₀ groups represented by a formula selected from formulas (i)-(x) per repeat unit; and R in formulas (i)-(x) is as described in RB(OH)₂ or RB(Y)₂ described above. More specifically, the CDP-proteasome inhibitor conjugate represented by formula (M) comprises at least 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9 or 2.0 R₁₀₀ groups represented by a formula selected from formulas (i)-(x) per repeat unit; and R in formulas (i)-(x) is represented by the following structural formula:

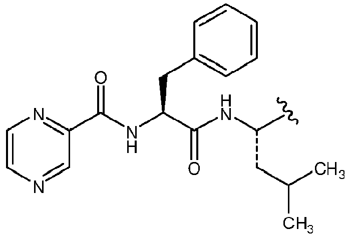

In a 10^{th} embodiment, for the CDP-proteasome inhibitor conjugate represented by formula (M), n is an integer from 1 to 100 (e.g., n is an integer from 4 to 80, from 4 to 50, from 4 to 30 or from 4 to 20, or n is 4, 5, 6, 7, 8, 9, 10, 11, 12, 13, 14, 15, 16, 17, 18, 19 or 20); m is an integer from 1 to 1000 (e.g., m is an integer from 1 to 200, from 1 to 100, from 1 to 80, from 2 to 80, from 5 to 70, from 10 to 50, or from 20 to 40); R₁₀₀ is -OH or a group represented by a formula selected from formulas (ia)-(xa). At least one R₁₀₀ group in the conjugate is a group represented by a formula selected from formulas (ia)-(xa). Alternatively, the conjugate represented by formula (M) comprises at least 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9 or 2.0 R₁₀₀ groups represented by a formula selected from formulas (ia)-(xa) per repeat unit.

In a 11^{th} embodiment, for the CDP-proteasome inhibitor conjugate represented by formula (M), n is an integer from 1 to 100 (e.g., n is an integer from 4 to 80, from 4 to 50, from 4 to 30 or from 4 to 20, or n is 4, 5, 6, 7, 8, 9, 10, 11, 12, 13, 14, 15, 16, 17, 18, 19 or 20); m is an integer from 1 to 1000 (e.g., m is an integer from 1 to 200, from 1 to 100, from 1 to 80, from 2 to 80, from 5 to 70, from 10 to 50, or from 20 to 40); R₁₀₀ is -OH or a group represented by a formula selected from formulas (ia)-(xa). At least one R₁₀₀ group in the conjugate is a group represented by a formula selected from formulas (ia)-(xa); and R in formulas (ia)-(xa) is as described in RB(OH)₂ or RB(Y)₂ described above. More specifically, at least one R₁₀₀ group in the conjugate is a group represented by a formula selected from formulas (ia)-(xa); and R in formulas (i)-(x) is represented by the following structural formula:

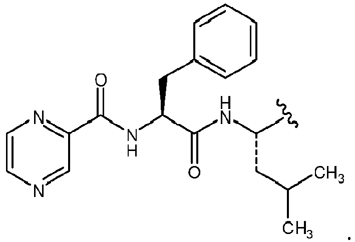

Alternatively, the CDP-proteasome inhibitor conjugate represented by formula (M) comprises at least 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9 or 2.0 R₁₀₀ groups represented by a formula selected from formulas (ia)-(xa) per repeat unit; and R in formulas (ia)-(xa) is as described in RB(OH)₂ or RB(Y)₂ described above. More specifically, the CDP-proteasome inhibitor conjugate represented by formula (M) comprises at least 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9 or 2.0 R₁₀₀ groups represented by a formula selected from formulas (ia)-(xa) per repeat unit; and R in formulas (ia)-(xa) is represented by the following structural formula:

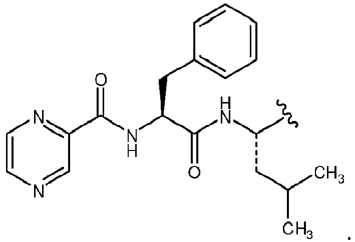

In a 12^{th} embodiment, for the CDP-proteasome inhibitor conjugate represented by formula (M), n is an integer from 1 to 100 (e.g., n is an integer from 4 to 80, from 4 to 50, from 4 to 30 or from 4 to 20, or n is 4, 5, 6, 7, 8, 9, 10, 11, 12, 13, 14, 15, 16, 17, 18, 19 or 20); m is an integer from 1 to 1000 (e.g., m is an integer from 1 to 200, from 1 to 100, from 1 to 80, from 2 to 80, from 5 to 70, from 10 to 50, or from 20 to 40); R₁₀₀ is -OH or a group represented by formula (ia). At least one R₁₀₀ group in the conjugate is a group represented by formula (1a) and the group -W-X-Y-Z-A in R₁₀₀ represented by formula (ia) is represented by a formula selected from formulas (a)-(x) described in the 3^{rd} embodiment and formulas (AA1), (BB1) and (CC1) described in the 5^{th} embodiment. Alternatively, the CDP-proteasome inhibitor conjugate represented by formula (M) comprises at least 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9 or 2.0 R₁₀₀ groups represented by formula (ia) per repeat unit; and the group -W-X-Y-Z-A in R₁₀₀ represented by formula (ia) is represented by a formula selected from formulas (a)-(x) described in the 3^{rd} embodiment and formulas (AA1), (BB1) and (CC1) described in the 5^{th} embodiment.

Alternatively, in the 12^{th} embodiment described above, R₁₀₀ is represented by formula (iia) instead of formula (ia). Alternatively, in the 12^{th} embodiment described above, R₁₀₀ is represented by formula (iiia) instead of formula (ia). Alternatively, in the 12^{th} embodiment above, R₁₀₀ is represented by formula (iva) instead of formula (ia). Alternatively, in the 12^{th} embodiment described above, R₁₀₀ is represented by formula (va) instead of formula (ia). Alternatively, in the 12^{th} embodiment described above, R₁₀₀ is represented by formula (via) instead of formula (ia). Alternatively, in the 12^{th} embodiment described above, R₁₀₀ is represented by formula (viia) instead of formula (ia). Alternatively, in the 12^{th} embodiment described above, R₁₀₀ is represented by formula (viiia) instead of formula (ia). Alternatively, in the 12^{th} embodiment described above, R₁₀₀ is represented by formula (ixa) instead of formula (ia). Alternatively, in the 12^{th} embodiment described above, R₁₀₀ is represented by formula (xa) instead of formula (ia).

In a 13^{th} embodiment, for the CDP-proteasome inhibitor conjugate represented by formula (M), n is an integer from 1 to 100 (e.g., n is an integer from 4 to 80, from 4 to 50, from 4 to 30 or from 4 to 20, or n is 4, 5, 6, 7, 8, 9, 10, 11, 12, 13, 14, 15, 16, 17, 18, 19 or 20); m is an integer from 1 to 1000 (e.g., m is an integer from 1 to 200, from 1 to 100, from 1 to 80, from 2 to 80, from 5 to 70, from 10 to 50, or from 20 to 40); R₁₀₀ is -OH or a group represented by (ia). At least one R₁₀₀ group in the conjugate is a group represented by (ia); the group -W-X-Y-Z-A in formula (ia) is represented by a formula selected from formulas (a)-(x) described in the 3^{rd} embodiment and formulas (AA1), (BB1) and (CC1) described in the 5^{th} embodiment; and R in R₁₀₀ represented by formula (ia) is as describe in RB(OH)₂ or RB(Y)₂ described above. More specifically, at least one R₁₀₀ group in the conjugate is a group represented by formula (ia); the group -W-X-Y-Z-A in R₁₀₀ represented by formula (ia) is represented by a formula selected from formulas (a)-(x) described in the 3^{rd} embodiment and formulas (AA1), (BB1) and (CC1) described in the 5^{th} embodiment; and R in R₁₀₀ represented by formula (ia) is represented by the following structural formula:

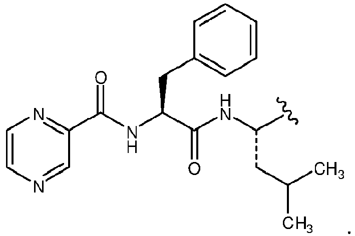

Alternatively, the CDP-proteasome inhibitor conjugate represented by formula (M) comprises at least 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9 or 2.0 R₁₀₀ groups represented by formula (ia) per repeat unit; the group -W-X-Y-Z-A in R₁₀₀ represented by formula (ia) is represented by a formula selected from formulas (a)-(x) described in the 3^{rd} embodiment and formulas (AA1), (BB1) and (CC1) described in the 5^{th} embodiment; and R in R₁₀₀ represented by formula (ia) is as described in RB(OH)₂ or RB(Y)₂ described above. More specifically, the CDP-proteasome inhibitor conjugate represented by formula (M) comprises at least 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9 or 2.0 R₁₀₀ groups represented by formula (ia) per repeat unit; the group -W-X-Y-Z-A in R₁₀₀ represented by formula (ia) is represented by a formula selected from formulas (a)-(x) described in the 3^{rd} embodiment and formulas (AA1), (BB1) and (CC1) described in the 5^{th} embodiment; and R in R₁₀₀ represented by formula (ia) is represented by the following structural formula:

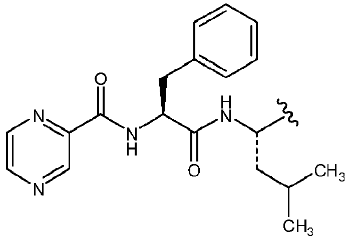

Alternatively, in the 13^{th} embodiment described above, R₁₀₀ is represented by formula (iia) instead of formula (ia). Alternatively, in the 13^{th} embodiment described above, R₁₀₀ is represented by formula (iiia) instead of formula (ia). Alternatively, in the 13^{th} embodiment above, R₁₀₀ is represented by formula (iva) instead of formula (ia). Alternatively, in the 13^{th} embodiment described above, R₁₀₀ is represented by formula (va) instead of formula (ia). Alternatively, in the 13^{th} embodiment described above, R₁₀₀ is represented by formula (via) instead of formula (ia). Alternatively, in the 13^{th} embodiment described above, R₁₀₀ is represented by formula (viia) instead of formula (ia). Alternatively, in the 13^{th} embodiment described above, R₁₀₀ is represented by formula (viiia) instead of formula (ia). Alternatively, in the 13^{th} embodiment described above, R₁₀₀ is represented by formula (ixa) instead of formula (ia). Alternatively, in the 13^{th} embodiment described above, R₁₀₀ is represented by formula (xa) instead of formula (ia).

In a 14^{th} embodiment, for the CDP-proteasome inhibitor conjugate represented by formula (M), n is an integer from 1 to 100 (e.g., n is an integer from 4 to 80, from 4 to 50, from 4 to 30 or from 4 to 20, or n is 4, 5, 6, 7, 8, 9, 10, 11, 12, 13, 14, 15, 16, 17, 18, 19 or 20); m is an integer from 1 to 1000 (e.g., m is an integer from 1 to 200, from 1 to 100, from 1 to 80, from 2 to 80, from 5 to 70, from 10 to 50, or from 20 to 40); R₁₀₀ is -OH or a group represented by formula (ia). At least one R₁₀₀ group in the conjugate is a group represented by formula (ia) and the group -W-X-Y-Z-A in R₁₀₀ represented by formula (ia) is represented by a formula selected from the formulas described in the 4^{th} embodiment. Alternatively, the CDP-proteasome inhibitor conjugate represented by formula (M) comprises at least 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9 or 2.0 R₁₀₀ groups represented by formula (ia) per repeat unit; and the group -W-X-Y-Z-A in R₁₀₀ represented by formula (ia) is represented by a formula selected from the formulas described in the 4^{th} embodiment.

Alternatively, in the 14^{th} embodiment described above, R₁₀₀ is represented by formula (iia) instead of formula (ia). Alternatively, in the 14^{th} embodiment described above, R₁₀₀ is represented by formula (iiia) instead of formula (ia). Alternatively, in the 14^{th} embodiment above, R₁₀₀ is represented by formula (iva) instead of formula (ia). Alternatively, in the 14^{th} embodiment described above, R₁₀₀ is represented by formula (va) instead of formula (ia). Alternatively, in the 14^{th} embodiment described above, R₁₀₀ is represented by formula (via) instead of formula (ia). Alternatively, in the 14^{th} embodiment described above, R₁₀₀ is represented by formula (viia) instead of formula (ia). Alternatively, in the 14^{th} embodiment described above, R₁₀₀ is represented by formula (viiia) instead of formula (ia). Alternatively, in the 14^{th} embodiment described above, R₁₀₀ is represented by formula (ixa) instead of formula (ia). Alternatively, in the 14^{th} embodiment described above, R₁₀₀ is represented by formula (xa) instead of formula (ia).

In a 15^{th} embodiment, for the CDP-proteasome inhibitor conjugate represented by formula (M), n is an integer from 1 to 100 (e.g., n is an integer from 4 to 80, from 4 to 50, from 4 to 30 or from 4 to 20, or n is 4, 5, 6, 7, 8, 9, 10, 11, 12, 13, 14, 15, 16, 17, 18, 19 or 20); m is an integer from 1 to 1000 (e.g., m is an integer from 1 to 200, from 1 to 100, from 1 to 80, from 2 to 80, from 5 to 70, from 10 to 50, or from 20 to 40); R₁₀₀ is -OH or a group represented by formula (ia). At least one R₁₀₀ group in the conjugate is a group represented by formula (ia); the group -W-X-Y-Z-A in R₁₀₀ represented by formula (ia) is represented by a formula selected from the formulas described in the 4^{th} embodiment; and R in R₁₀₀ represented by formula (ia) is as described in RB(OH)₂ or RB(Y)₂ described above. More specifically, at least one R₁₀₀ group in the conjugate is a group represented by formula (ia); the group -W-X-Y-Z-A in R₁₀₀ represented by formula (ia) is represented by a formula selected from the formulas described in the 4^{th} embodiment; and R in R₁₀₀ represented by formulas (ia) is represented by the following structural formula:

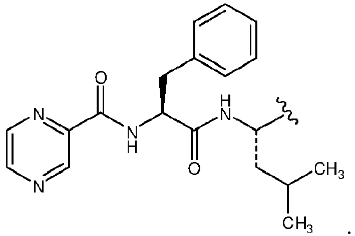

Alternatively, the CDP-proteasome inhibitor conjugate represented by formula (M) comprises at least 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9 or 2.0 R₁₀₀ groups represented by formula (ia) per repeat unit; the group -W-X-Y-Z-A in R₁₀₀ represented by formula (ia) is represented by a formula selected from the formulas described in the 4^{th} embodiment; and R in R₁₀₀ represented by formula (ia) is as described in RB(OH)₂ or RB(Y)₂ described above. More specifically, the CDP-proteasome inhibitor conjugate represented by formula (M) comprises at least 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9 or 2.0 R₁₀₀ groups represented by formula (ia) per repeat unit; the group -W-X-Y-Z-A in R₁₀₀ represented by formula (ia) is represented by a formula selected from the formulas described in the 4^{th} embodiment; and R in R₁₀₀ represented by formula (ia) is represented by the following structural formula:

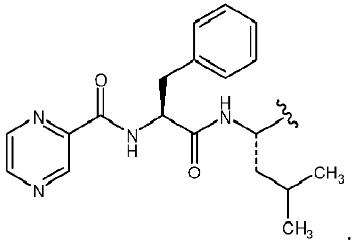

Alternatively, in the 15^{th} embodiment described above, R₁₀₀ is represented by formula (iia) instead of formula (ia). Alternatively, in the 15^{th} embodiment described above, R₁₀₀ is represented by formula (iiia) instead of formula (ia). Alternatively, in the 15^{th} embodiment above, R₁₀₀ is represented by formula (iva) instead of formula (ia). Alternatively, in the 15^{th} embodiment described above, R₁₀₀ is represented by formula (va) instead of formula (ia). Alternatively, in the 15^{th} embodiment described above, R₁₀₀ is represented by formula (via) instead of formula (ia). Alternatively, in the 15^{th} embodiment described above, R₁₀₀ is represented by formula (viia) instead of formula (ia). Alternatively, in the 15^{th} embodiment described above, R₁₀₀ is represented by formula (viiia) instead of formula (ia). Alternatively, in the 15^{th} embodiment described above, R₁₀₀ is represented by formula (ixa) instead of formula (ia). Alternatively, in the 15^{th} embodiment described above, R₁₀₀ is represented by formula (xa) instead of formula (ia).

In the 7^{th} through the 15^{th} embodiment, n is preferably an integer from 4 to 20 and m is an integer from 1 to 1000; n is an integer from 4 to 80 and m is an integer from 1 to 200; n is an integer from 4 to 50 and m is an integer from 1 to 100; n is an integer from 4 to 30 and m is an integer from 1 to 80; n is an integer from 4 to 20 and m is an integer from 2 to 80; n is an integer from 4 to 20 and m is an integer from 5 to 70; n is an integer from 4 to 20 and and m is an integer from 10 to 50; or n is an integer from 4 to 20 and and m is an integer from 20-40.

In one embodiment, for the CDP-proteasome inhibitor conjugate described in any one of 1^{st} to 15^{th} embodiments, R in formulas (i)-(x) and (ia)-(xa) is represented by the following structural formula:

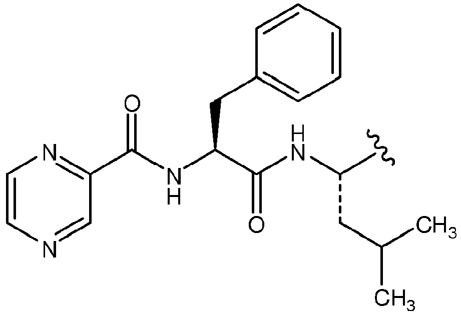

In one embodiment, for the CDP-proteasome inhibitor conjugate described in any one of 1^{st} to 15^{th} embodiments, RB(OH)₂ or RB(Y)₂ is as described in WO 91/13904, U.S. Patent Nos 5,780,454, 6,066,730, 6,083,903, 6,297,217, 6,465,433, 6,548,668, 6,617,317, 6,699,835, 6,713,446, 6,747,150, 6,958,319, 7,109,323, 7,119,080, 7,442,830, 7,531,526 and U.S. Published Applications 2009/0247731, 2009/099132, 2009/0042836, 2008/0132678, 2007/0282100, 2006/0122390, 2005/0282742, 2005/0240047, 2004/0167332, 2004/0138411, 2003/0199561, 2002/0188100 and 2002/0173488. Each of these patent documents is incorporated by reference in its entirety.

CDP-proteasome inhibitor (such as a boronic acid containing proteasome inhibitor, e.g., bortezomib) conjugates can be made using many different combinations of components described herein. For example, various combinations of cyclodextrins (e.g., beta-cyclodextrin), comonomers (e.g., PEG containing comonomers), linkers linking the cyclodextrins and comonomers, and/or linkers tethering the proteasome inhibitor (such as a boronic acid containing proteasome inhibitor, e.g., bortezomib) to the CDP are described herein.

FIG. 7 is a table depicting examples of different CDP-proteasome inhibitor conjugates. The CDP-proteasome inhibitor conjugates in FIG. 7 are represented by the following formula:

CDP-CO-L-D

In this formula, CDP is the cyclodextrin-containing polymer shown below (as well as in FIG. 3): 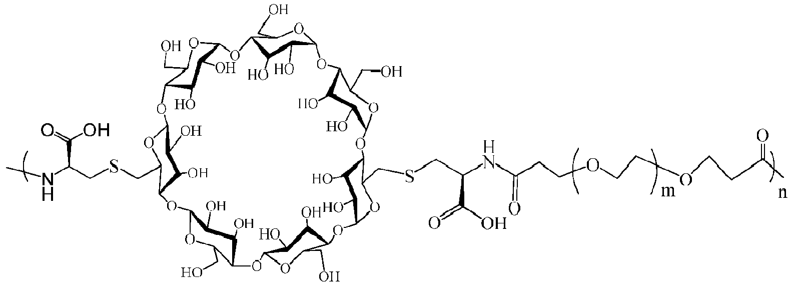 wherein the group 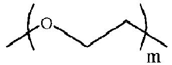 has a Mw of 3400 Da or less and n is at least 4, 5, 6, 7, 8, 9, 10, 11, 12, 13, 14, 15, 16, 17, 18, 19 or 20; and D is -B-R, wherein R is the non-boronic acid moiety in bortezomib. Note that the proteasome inhibitor (such as a boronic acid containing proteasome inhibitor, e.g., bortezomib) is conjugated to the CDP through the carboxylic acid moieties of the polymer as provided above. Full loading of the proteasome inhibitor (such as a boronic acid containing proteasome inhibitor, e.g., bortezomib) onto the CDP is not required. In some embodiments, at least one, e.g., at least 2, 3, 4, 5, 6 or 7, of the carboxylic acid moieties remains unreacted with the proteasome inhibitor (such as a boronic acid containing proteasome inhibitor, e.g., bortezomib) after conjugation (e.g., a plurality of the carboxylic acid moieties remain unreacted).

CO represents the carbonyl group of the cysteine residue of the CDP;
L represents a linker group between the CDP and the boronic acid. L has a terminal amino group that is bonded to the cysteine acid carbonyl of CDP. The other terminal of L comprises two functional groups that bind to the boron atom in bortezomib and upon binding to bortezomib, the two functional groups displace the two -OH groups in bortezomib that are bonded to the boron atom.

As provided in FIG. 7, the column with the heading "Boronic Acid" indicates which pharmaceutically active agent, preferably a proteasome inhibitor, comprising a boronic acid that is included in the CDP-proteasome inhibitor conjugate.

The two columns on the right of the table in FIG. 7 indicate respectively, the process for producing the CDP-proteasome inhibitor conjugate, and the final product of the process for producing the CDP-proteasome inhibitor conjugate.

The processes referred to in FIG. 7 are given a letter representation, e.g., Process A and Process B, as seen in the second column from the right. The steps for each these processes respectively are provided below.
Process A: Couple the optionally protected L to CDP; deprotect L-CDP if protected; and conjugate the boronic acid.
Process B: Conjugate the optionally protected L to boronic acid; deprotect L-boronic acid; and couple L-boronic acid to CDP.

As shown specifically in FIG. 7, the CDP-proteasome inhibitor conjugates can be prepared using a variety of methods known in the art, including those described herein..

One or more protecting groups can be used in the processes described above to make the CDP-proteasome inhibitor conjugates described herein. In some embodiments, the protecting group is removed and, in other embodiments, the protecting group is not removed. If a protecting group is not removed, then it can be selected so that it is removed *in vivo* (e.g., acting as a prodrug). An example is hexanoic acid which has been shown to be removed by lipases *in vivo* if used to protect a hydroxyl group in doxorubicin. Protecting groups are generally selected for both the reactive groups of the proteasome inhibitor and the reactive groups of the linker that are not targeted to be part of the coupling reaction. The protecting group should be removable under conditions which will not degrade the proteasome inhibitor and/or linker material. Examples include t-butyldimethylsilyl ("TBDMS"), TROC (derived from 2,2,2-trichloroethoxy chloroformate), carboxybenzyl ("CBz") and *tert*-butyloxycarbonyl ("Boc"). Carboxybenzyl ("CBz") can also be used in place of TROC if there is selectivity seen for removal over olefin reduction. This can be addressed by using a group which is more readily removed by hydrogenation such as -methoxybenzyl OCO-. Other protecting groups may also be acceptable. One of skill in the art can select suitable protecting groups for the products and methods described herein.

In an embodiment, the therapeutic agent in the CDP-therapeutic agent conjugate is a cytotoxic agent such as an immunomodulator. In some embodiments, the immunomodulator in the CDP-immunomodulator conjugate, particle, or composition is a corticosteroid, rapamycin, or a rapamycin analog.

In some embodiments, the immunomodulator is a corticosteroid (e.g., prednisone). In some embodiments, the corticosteroid can have the following structure: 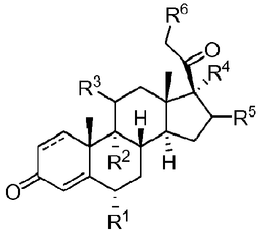
R¹ is H, C₁-C₆ alkyl (e.g., CH₃) or halo (e.g., F);
R² is H or halo (e.g., F or Cl);
R³ is OH, or taken together with the carbon to which it is attached forms and oxo;
R⁴ is H, OH, OC(O)R^{a}, or OR^{b};
R⁵ is H, OH, C₁-C₆ alkyl (e.g., CH₃), C₁-C₆ alkenyl (e.g., where the alkenyl includes a double bond with the carbon to which it is attached), or OR^{c};
R⁶ is OH, halo, OC(O)R^{e}, SR^{e}
R^{a} is C₁-C₆ alkyl, C₁-C₆ alkoxy, aryl or heteroaryl;
OR^{b} and OR^{c}, when taken together with the carbons to which they are attached, form a ring, optionally substituted with 1 or 2 R^{d};
each R^{d} is independently C₁-C₆ alkyl; or two R^{d}, taken together with the carbon to which they are attached, form a cycloalkyl;
R^{e} is OC₁-C₆alkyl or C₁-C₆alkyl; and
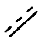 denotes a double or single bond.

In some embodiments, R¹ is H or halo (e.g., F). In some embodiments, R¹ is methyl.

In some embodiments, R² is H. In some embodiments, R² is F.

In some embodiments, R³ is OH.

In some embodiments, R⁴ is OH or OC(O)R^{a} (e.g., wherein R^{a} is C₁-C₆ alkyl heteroaryl).

In some embodiments, R⁵ is H. In some embodiments, R⁵ is or methyl. In some embodiments, R⁵, together with the carbon to which it is attached forms C₂ alkenyl.

In some embodiments, R⁴ and R⁵, are OR^{b} and OR^{c} repsectively, and OR^{b} and OR^{c}, together with the carbons to which they are attached form the following structure 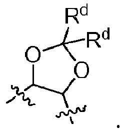 In some embodiments, each R^{d} is independently C₁-C₆ alkyl. In some embodiments, two R^{d}, taken together with the carbon to which they are attached, form a cyclyoalkyl (e.g., C₄-C₈ cycloalkyl such as C₅ cycloalkyl).

In some embodiments, R⁴ is OH or OC(O)R^{a}; and R⁵ is H.

In some embodiments, R⁴ is H or OC(O)R^{a}; and R⁵ is methyl.

In some embodiments, R⁶ is OH. In some embodiments, R⁶ is halo (e.g., Cl). In some embodiments, R⁶ is OC(O)R^{e}, e.g., wherein R^{e} is C₁-C₆alkyl.

In some embodiments, the compound is not methylprednisolone.

In some embodiments, the compound is a compound of the following formula 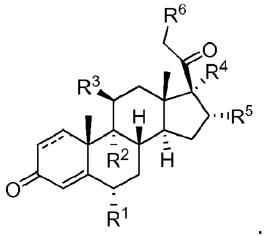 In some embodiments, 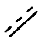 denotes a double bond. In some embodiments, R³ is OH.

In some embodiments, the compound is a compound of the following formula 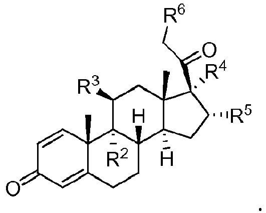 In some embodiments, R⁴ is OH and R⁵ is H. In some embodiments, R⁴ and R⁵, are OR^{b} and OR^{c} repsectively, and OR^{b} and OR^{c}, together with the carbons to which they are attached form the following structure 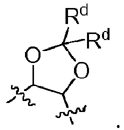 In some embodiments, R³ is OH.

In some embodiments, the compound is a compound of the following formula 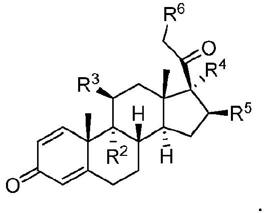 In some embodiments, R³ is OH.

Exemplary corticosteroids that can be conjugated to CDP include the corticosteroids shown below.

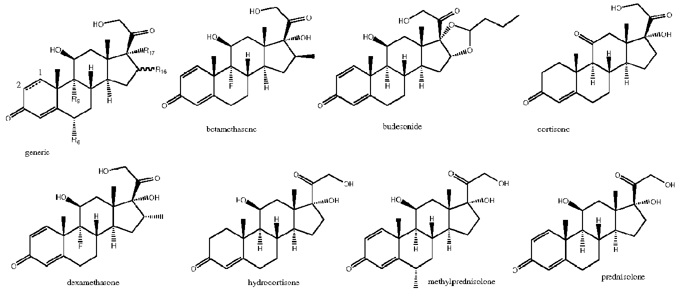

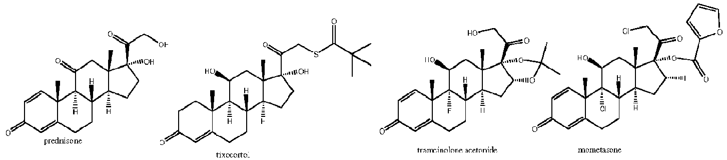

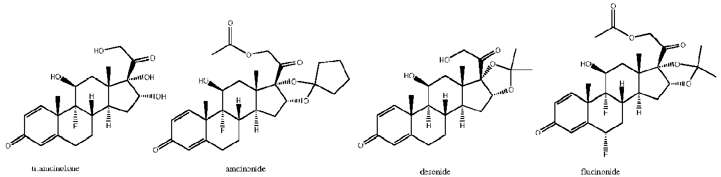

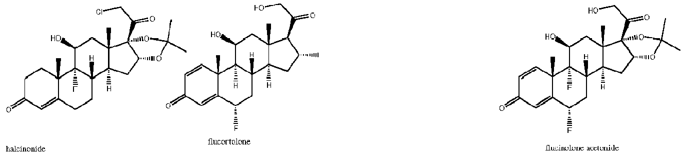

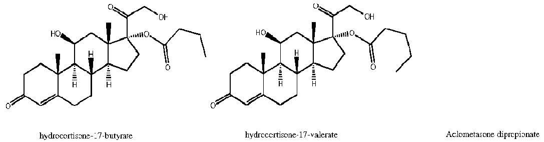

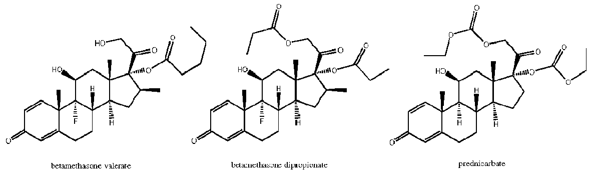

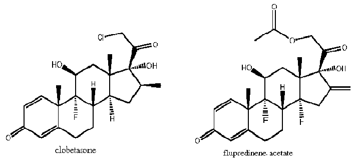

A corticosteroid described herein can be linked to a CDP. For example, a corticosteroid described herein can be linked to the CDP through a free OH group on the corticosteroid. The corticosteroid can be directly linked to the CDP for example, through a covalent bond or through a linker. Exemplary linkers are described herein and include amino acids and other linkers which can react with a free OH group to form a bond such as an ester bond.

In preferred embodiments, the corticosteroid in the CDP-corticosteroid conjugate, particle or composition comprises prednisone or a prednisone derivative. For example, prednisone can have the following structure:

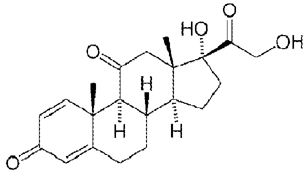

In one embodiment, the CDP-corticosteroid conjugate is a CDP-prednisone conjugate, e.g., 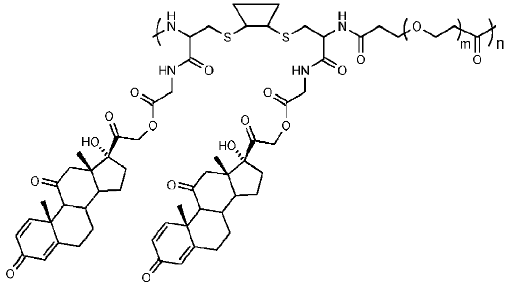 wherein 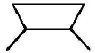 represents a cyclodextrin; n is an integer from 1 to 100 (e.g., n is an integer from 4 to 80, from 4 to 50, from 4 to 30 or from 4 to 20, or n is 4, 5, 6, 7, 8, 9, 10, 11, 12, 13, 14, 15, 16, 17, 18, 19 or 20); m is an integer from 1 to 1000 (e.g., m is an integer from 1 to 200, from 1 to 100, from 1 to 80, from 2 to 80, from 5 to 70, from 10 to 50, or from 20 to 40). In some embodiments, the CDP-corticosteroid conjugate, e.g., the CDP-prednisone conjugate, does not have complete loading, e.g., one or more binding sites, e.g., cysteine residues, are not bound to a corticosteroid, e.g., a prednisone moiety, e.g., a glycine-linkage bound prednisone, e.g., the CDP-prednisone conjugate comprises one or more subunits having the formulae provided below: 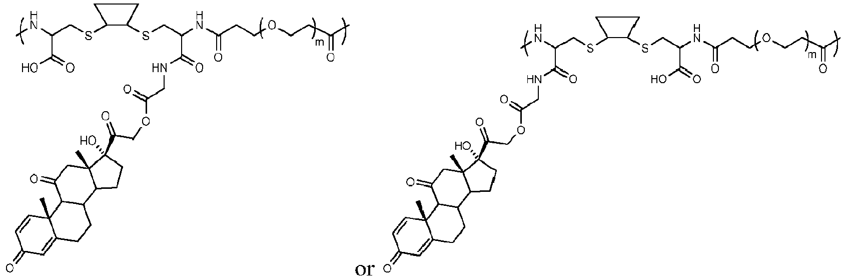 wherein 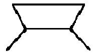 represents a cyclodextrin and m is an integer from 1 to 1000 (e.g., m is an integer from 1 to 200, from 1 to 100, from 1 to 80, from 2 to 80, from 5 to 70, from 10 to 50, or from 20 to 40). In some embodiments, the CDP-corticosteroid conjugate, particle or composition e.g., the CDP-prednisone conjugate, particle or composition, comprises a mixture of fully-loaded and partially-loaded CDP-corticosteroid conjugates, e.g., CDP-prednisone conjugates.

In one embodiment, the CDP-corticosteroid conjugate comprises a subunit of 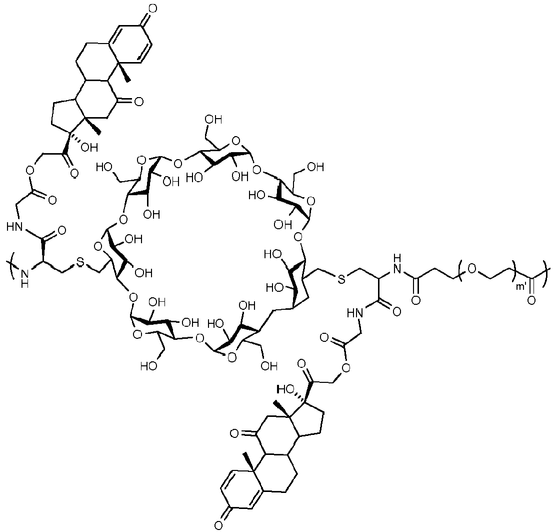 wherein m is an integer from 1 to 1000 (e.g., m is an integer from 1 to 200, from 1 to 100, from 1 to 80, from 2 to 80, from 5 to 70, from 10 to 50, or from 20 to 40).

In some embodiments, the corticosteroid is a short to medium acting glucocorticoid. In some embodiments, the corticosteroid is a Group A corticosteroid. Examples of Group A corticosterodis include hydrocortisone, hydrocortisone acetate, cortisone acetate, tixocortol pivalate, prednisolone, methylprednisolone and prednisone.

In some embodiments, the corticosteroid is a Group B corticosteroid. Examples of Group B corticosteroids include triamcinolone acetonide, triamcinolone alcohol, mometasone, amcinonide, budesonide, desonide, fluocinonide, fluocinolone acetonide, and halcinonide.

In some embodiments, the corticosteroid is a Group C corticosteroid. Examples of Group C corticosteroids include betamethasone, betamethasone sodium phosphate, dexamethasone, dexamethasone sodium phosphate, and fluocortolone.

In some embodiments, the corticosteroid is a Group D corticosteroid. Examples of Group D corticosteroids include hydrocortisone- 17-butyrate, hydrocortisone-17-valerate, aclometasone diproprionate, betamethasone valerate, betamethasone diproprionate, prednicarbate, clobetasone-17-butyrate, clobetasol-17-propionate, fluocortolone caproate, fluocortolone pivalate, and fluprednidene acetate.

An amount of a CDP-therapeutic agent conjugate, particle or composition effective to prevent a disorder, or "a prophylactically effective amount" of the conjugate, particle or composition as used in the context of the administration of an agent to a subject, refers to subjecting the subject to a regimen, e.g., the administration of a CDP-therapeutic agent conjugate, particle or composition such that the onset of at least one symptom of the disorder is delayed as compared to what would be seen in the absence of the regimen.

### CDPs, methods of making same, and methods of conjugating CDPs to therapeutic agents

Generally, the CDP-therapeutic agent conjugates described herein can be prepared in one of two ways: monomers bearing therapeutic agents, targeting ligands, and/or cyclodextrin moieties can be polymerized; or polymer backbones can be derivatized with therapeutic agents, targeting ligands, and/or cyclodextrin moieties. Therapeutic agents may include cytotoxic agents, e.g., topoisomerase inhibitors, e.g., a topoisomerase I inhibitor (e.g., camptothecin, irinotecan, SN-38, topotecan, lamellarin D, lurotecan, exatecan, diflomotecan, or derivatives thereof), or a topoisomerase II inhibitor (e.g., an etoposide, a tenoposide, doxorubicin, or derivatives thereof), an anti-metabolic agent (e.g., an antifolate (e.g., pemetrexed, floxuridine, or raltitrexed) or a pyrimidine conjugate (e.g., capecitabine, cytarabine, gemcitabine, or 5FU)), an alkylating agent, an anthracycline, an anti-tumor antibiotic (e.g., a HSP90 inhibitor, e.g., geldanamycin), a platinum based agent (e.g., cisplatin, carboplatin, or oxaliplatin), a microtubule inhibitor, a kinase inhibitor (e.g., a seronine/threonine kinase inhibitor, e.g., a mTOR inhibitor, e.g., rapamycin) or a proteasome inhibitor.

In one embodiment, the synthesis of the CDP-therapeutic agent conjugates can be accomplished by reacting monomers M-L-CD and M-L-D (and, optionally, M-L-T), wherein

CD represents a cyclic moiety, such as a cyclodextrin molecule, or derivative thereof;
L, independently for each occurrence, may be absent or represents a linker group;
D, independently for each occurrence, represents the same or different therapeutic agent or prodrug thereof;
T, independently for each occurrence, represents the same or different targeting ligand or precursor thereof; and
M represents a monomer subunit bearing one or more reactive moieties capable of undergoing a polymerization reaction with one or more other M in the monomers in the reaction mixture, under conditions that cause polymerization of the monomers to take place.

In some embodiments, one or more of the therapeutic agents in the CDP-therapeutic agent conjugate can be replaced with another therapeutic agent, e.g., another cytotoxic agent or immunomodulator.

In certain embodiments, the reaction mixture may further comprise monomers that do not bear CD, T, or D moieties, e.g., to space the derivatized monomer units throughout the polymer.

In an alternative embodiment, the invention contemplates synthesizing a CDP-therapeutic agent conjugate by reacting a polymer P (the polymer bearing a plurality of reactive groups, such as carboxylic acids, alcohols, thiols, amines, epoxides, etc.) with grafting agents X-L-CD and/or Y-L-D (and, optionally, Z-L-T), wherein
CD represents a cyclic moiety, such as a cyclodextrin molecule, or derivative thereof;
L, independently for each occurrence, may be absent or represents a linker group;
D, independently for each occurrence, represents the same or different therapeutic agent or prodrug thereof;
T, independently for each occurrence, represents the same or different targeting ligand or precursor thereof;
X, independently for each occurrence, represents a reactive group, such as carboxylic acids, alcohols, thiols, amines, epoxides, etc., capable of forming a covalent bond with a reactive group of the polymer; and
Y and Z, independently for each occurrence, represent inclusion hosts or reactive groups, such as carboxylic acids, alcohols, thiols, amines, epoxides, etc., capable of forming a covalent bond with a reactive group of the polymer or inclusion complexes with CD moieties grafted to the polymer, under conditions that cause the grafting agents to form covalent bonds and/or inclusion complexes, as appropriate, with the polymer or moieties grafted to the polymer.

In some embodiments, one or more of the therapeutic agents in the CDP-taxane conjugate can be replaced with another therapeutic agent, e.g., another cytotoxic agent or immunomodulator.

For example, if the CDP includes alcohols, thiols, or amines as reactive groups, the grafting agents may include reactive groups that react with them, such as isocyanates, isothiocyanates, acid chlorides, acid anhydrides, epoxides, ketenes, sulfonyl chlorides, activated carboxylic acids (e.g., carboxylic acids treated with an activating agent such as PyBrOP, carbonyldiimidazole, or another reagent that reacts with a carboxylic acid to form a moiety susceptible to nucleophilic attack), or other electrophilic moieties known to those of skill in the art. In certain embodiments, a catalyst may be needed to cause the reaction to take place (e.g., a Lewis acid, a transition metal catalyst, an amine base, etc.) as will be understood by those of skill in the art.

In certain embodiments, the different grafting agents are reacted with the polymer simultaneously or substantially simultaneously (e.g., in a one-pot reaction), or are reacted sequentially with the polymer (optionally with a purification and/or wash step between reactions).

Another aspect of the present invention is a method for manufacturing the linear or branched CDPs and CDP-therapeutic agent conjugates as described herein. While the discussion below focuses on the preparation of linear cyclodextrin molecules, one skilled in the art would readily recognize that the methods described can be adapted for producing branched polymers by choosing an appropriate comonomer precursor.

Accordingly, one embodiment of the invention is a method of preparing a linear CDP. According to the invention, a linear CDP may be prepared by copolymerizing a cyclodextrin monomer precursor disubstituted with one or more appropriate leaving groups with a comonomer precursor capable of displacing the leaving groups. The leaving group, which may be the same or different, may be any leaving group known in the art which may be displaced upon copolymerization with a comonomer precursor. In a preferred embodiment, a linear CDP may be prepared by iodinating a cyclodextrin monomer precursor to form a diiodinated cyclodextrin monomer precursor and copolymerizing the diiodinated cyclodextrin monomer precursor with a comonomer precursor to form a linear CDP having a repeating unit of formula I or II, provided in the section entitles "CDP-Therapeutic agent conjugates" or a combination thereof, each as described above. In some embodiments, the cyclodextrin moiety precursors are in a composition, the composition being substantially free of cyclodextrin moieties having other than two positions modified to bear a reactive site (e.g., 1, 3, 4, 5, 6, or 7). While examples presented below discuss iodinated cyclodextrin moieties, one skilled in the art would readily recognize that the present invention contemplates and encompasses cyclodextrin moieties wherein other leaving groups such as alkyl and aryl sulfonate may be present instead of iodo groups. In a preferred embodiment, a method of preparing a linear cyclodextrin copolymer of the invention by iodinating a cyclodextrin monomer precursor as described above to form a diiodinated cyclodextrin monomer precursor of formula XXXIVa, XXXIVb, XXXIVc or a mixture thereof:

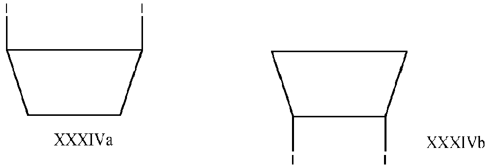

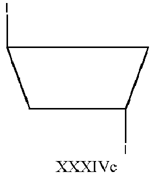

In some embodiments, the iodine moieties as shown on the cyclodextrin moieties are positioned such that the derivatization on the cyclodextrin is on the A and D glucopyranose moieties. In some embodiments, the iodine moieties as shown on the cyclodextrin moieties are positioned in such that the derivatization on the cyclodextrin is on the A and C glucopyranose moieties. In some embodiments, the iodine moieties as shown on the cyclodextrin moieties are positioned in such that the derivatization on the cyclodextrin is on the A and F glucopyranose moieties. In some embodiments, the iodine moieties as shown on the cyclodextrin moieties are positioned in such that the derivatization on the cyclodextrin is on the A and E glucopyranose moieties.

The diiodinated cyclodextrin may be prepared by any means known in the art. (Tabushi et al. J. Am. Chem. 106, 5267-5270 (1984); Tabushi et al. J. Am. Chem. 106, 4580-4584 (1984)). For example, β-cyclodextrin may be reacted with biphenyl-4,4'-disulfonyl chloride in the presence of anhydrous pyridine to form a biphenyl-4,4'-disulfonyl chloride capped β-cyclodextrin which may then be reacted with potassium iodide to produce diiodo-β-cyclodextrin. The cyclodextrin monomer precursor is iodinated at only two positions. By copolymerizing the diiodinated cyclodextrin monomer precursor with a comonomer precursor, as described above, a linear cyclodextrin polymer having a repeating unit of Formula la, Ib, or a combination thereof, also as described above, may be prepared. If appropriate, the iodine or iodo groups may be replaced with other known leaving groups.

Also according to the invention, the iodo groups or other appropriate leaving group may be displaced with a group that permits reaction with a comonomer precursor, as described above. For example, a diiodinated cyclodextrin monomer precursor of formula XXXIVa, XXXIVb, XXXIVc or a mixture thereof may be aminated to form a diaminated cyclodextrin monomer precursor of formula XXXVa, XXXVb, XXXVc or a mixture thereof:

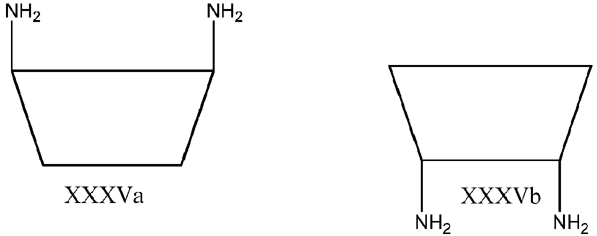

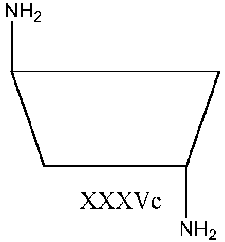

In some embodiments, the amino moieties as shown on the cyclodextrin moieties are positioned such that the derivatization on the cyclodextrin is on the A and D glucopyranose moieties. In some embodiments, the amino moieties as shown on the cyclodextrin moieties are positioned in such that the derivatization on the cyclodextrin is on the A and C glucopyranose moieties. In some embodiments, the amino moieties as shown on the cyclodextrin moieties are positioned in such that the derivatization on the cyclodextrin is on the A and F glucopyranose moieties. In some embodiments, the amino moieties as shown on the cyclodextrin moieties are positioned in such that the derivatization on the cyclodextrin is on the A and E glucopyranose moieties.

The diaminated cyclodextrin monomer precursor may be prepared by any means known in the art. (Tabushi et al. Tetrahedron Lett. 18:11527-1530 (1977); Mungall et al., J. Org. Chem. 16591662 (1975)). For example, a diiodo-β-cyclodextrin may be reacted with sodium azide and then reduced to form a diamino-β-cyclodextrin). The cyclodextrin monomer precursor is aminated at only two positions. The diaminated cyclodextrin monomer precursor may then be copolymerized with a comonomer precursor, as described above, to produce a linear cyclodextrin copolymer having a repeating unit. However, the amino functionality of a diaminated cyclodextrin monomer precursor need not be directly attached to the cyclodextrin moiety. Alternatively, the amino functionality or another nucleophilic functionality may be introduced by displacement of the iodo or other appropriate leaving groups of a cyclodextrin monomer precursor with amino group containing moieties such as, for example, HSCH₂CH₂NH₂ (or a di-nucleophilic molecule more generally represented by HW-(CR₁R₂)ₙ-WH wherein W, independently for each occurrence, represents O, S, or NR₁; R₁ and R₂, independently for each occurrence, represent H, (un)substituted alkyl, (un)substituted aryl, (un)substituted heteroalkyl, (un)substituted heteroaryl) with an appropriate base such as a metal hydride, alkali or alkaline carbonate, or tertiary amine to form a diaminated cyclodextrin monomer precursor of formula XXXVd, XXXVe, XXXVf or a mixture thereof:

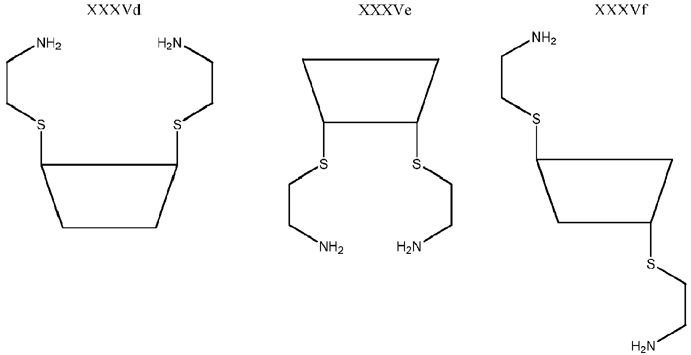

In some embodiments, the -SCH₂CH₂NH₂ moieties as shown on the cyclodextrin moieties are positioned such that the derivatization on the cyclodextrin is on the A and D glucopyranose moieties. In some embodiments, the -SCH₂CH₂NH₂ moieties as shown on the cyclodextrin moieties are positioned in such that the derivatization on the cyclodextrin is on the A and C glucopyranose moieties. In some embodiments, the - SCH₂CH₂NH₂ moieties as shown on the cyclodextrin moieties are positioned in such that the derivatization on the cyclodextrin is on the A and F glucopyranose moieties. In some embodiments, the -SCH₂CH₂NH₂ moieties as shown on the cyclodextrin moieties are positioned in such that the derivatization on the cyclodextrin is on the A and E glucopyranose moieties.

A linear oxidized CDP may also be prepared by oxidizing a reduced linear cyclodextrin-containing copolymer as described below. This method may be performed as long as the comonomer does not contain an oxidation sensitive moiety or group such as, for example, a thiol.

A linear CDP of the invention may be oxidized so as to introduce at least one oxidized cyclodextrin monomer into the copolymer such that the oxidized cyclodextrin monomer is an integral part of the polymer backbone. A linear CDP which contains at least one oxidized cyclodextrin monomer is defined as a linear oxidized cyclodextrin copolymer or a linear oxidized cyclodextrin-containing polymer. The cyclodextrin monomer may be oxidized on either the secondary or primary hydroxyl side of the cyclodextrin moiety. If more than one oxidized cyclodextrin monomer is present in a linear oxidized cyclodextrin copolymer of the invention, the same or different cyclodextrin monomers oxidized on either the primary hydroxyl side, the secondary hydroxyl side, or both may be present. For illustration purposes, a linear oxidized cyclodextrin copolymer with oxidized secondary hydroxyl groups has, for example, at least one unit of formula XXXVIa or XXXVIb:

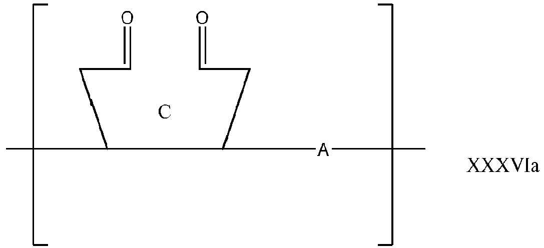

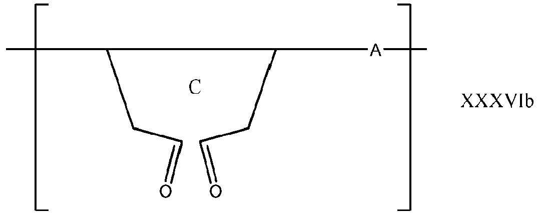

In formulae XXXVIa and XXXVIb, C is a substituted or unsubstituted oxidized cyclodextrin monomer and the comonomer (i.e., shown herein as A) is a comonomer bound, i.e., covalently bound, to the oxidized cyclodextrin C. Also in formulae XXXVIa and XXXVIb, oxidation of the secondary hydroxyl groups leads to ring opening of the cyclodextrin moiety and the formation of aldehyde groups.

A linear oxidized CDP copolymer may be prepared by oxidation of a linear cyclodextrin copolymer as discussed above. Oxidation of a linear cyclodextrin copolymer of the invention may be accomplished by oxidation techniques known in the art. (Hisamatsu et al., Starch 44:188-191 (1992)). Preferably, an oxidant such as, for example, sodium periodate is used. It would be understood by one of ordinary skill in the art that under standard oxidation conditions that the degree of oxidation may vary or be varied per copolymer. Thus in one embodiment of the invention, a CDP may contain one oxidized cyclodextrin monomer. In another embodiment, substantially all cyclodextrin monomers of the copolymer would be oxidized.

Another method of preparing a linear oxidized CDP involves the oxidation of a diiodinated or diaminated cyclodextrin monomer precursor, as described above, to form an oxidized diiodinated or diaminated cyclodextrin monomer precursor and copolymerization of the oxidized diiodinated or diaminated cyclodextrin monomer precursor with a comonomer precursor. In a preferred embodiment, an oxidized diiodinated cyclodextrin monomer precursor of formula XXXVIIa, XXXVIIb, XXXVIIc, or a mixture thereof: 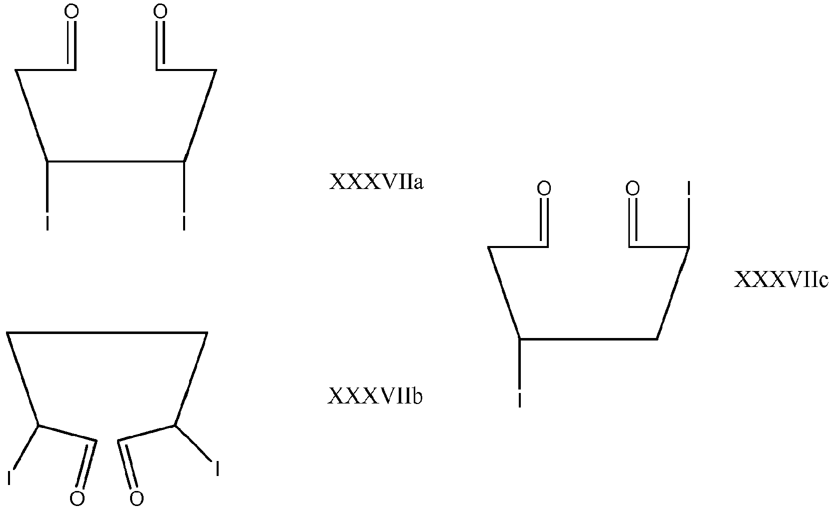 may be prepared by oxidation of a diiodinated cyclodextrin monomer precursor of formulae XXXIVa, XXXIVb, XXXIVc, or a mixture thereof, as described above. In another preferred embodiment, an oxidized diaminated cyclodextrin monomer precursor of formula XXXVIIIa, XXXVIIIb, XXXVIIIc or a mixture thereof: 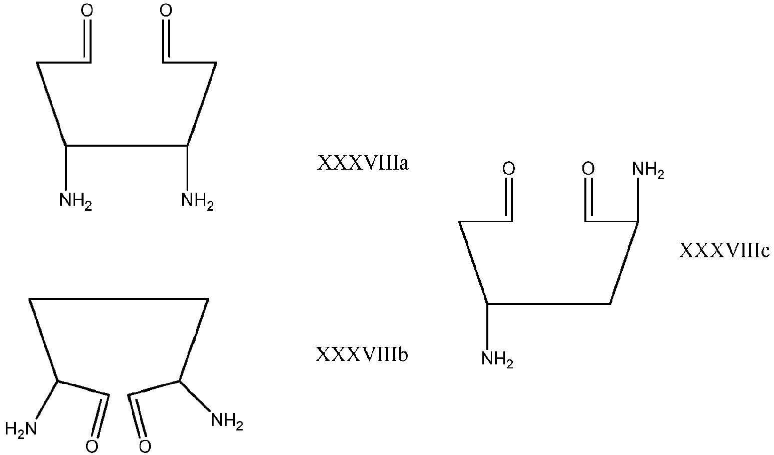 may be prepared by amination of an oxidized diiodinated cyclodextrin monomer precursor of formulae XXXVIIa, XXXVIIb, XXXVIIc, or a mixture thereof, as described above. In still another preferred embodiment, an oxidized diaminated cyclodextrin monomer precursor of formula XXXIXa, XXXIXb, XXXIXc or a mixture thereof: 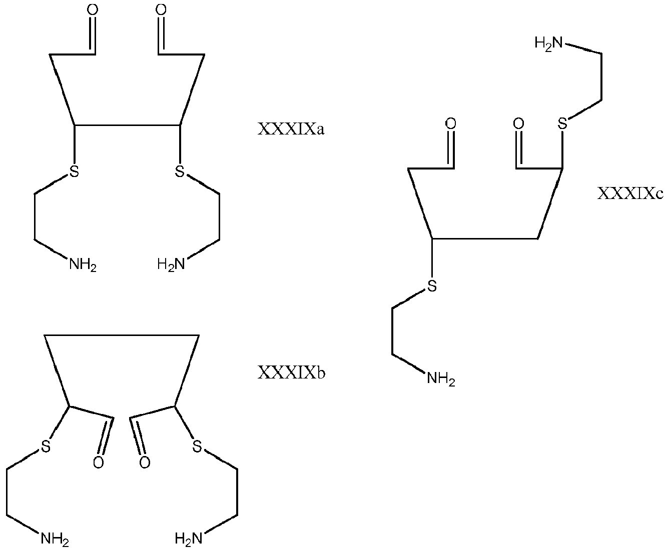 may be prepared by displacement of the iodo or other appropriate leaving groups of an oxidized cyclodextrin monomer precursor disubstituted with an iodo or other appropriate leaving group with the amino or other nucleophilic group containing moiety such as, e.g. HSCH₂CH₂NH₂ (or a di-nucleophilic molecule more generally represented by HW-(CR₁R₂)ₙ-WH wherein W, independently for each occurrence, represents O, S, or NR₁; R₁ and R₂, independently for each occurrence, represent H, (un)substituted alkyl, (un)substituted aryl, (un)substituted heteroalkyl, (un)substituted heteroaryl) with an appropriate base such as a metal hydride, alkali or alkaline carbonate, or tertiary amine.

Alternatively, an oxidized diiodinated or diaminated cyclodextrin monomer precursor, as described above, may be prepared by oxidizing a cyclodextrin monomer precursor to form an oxidized cyclodextrin monomer precursor and then diiodinating and/or diaminating the oxidized cyclodextrin monomer, as described above. As discussed above, the cyclodextrin moiety may be modified with other leaving groups other than iodo groups and other amino group containing functionalities. The oxidized diiodinated or diaminated cyclodextrin monomer precursor may then be copolymerized with a comonomer precursor, as described above, to form a linear oxidized cyclodextrin copolymer of the invention.

A linear oxidized CDP may also be further modified by attachment of at least one ligand to the copolymer. The ligand is as described above. In some embodiments, a CDP comprises: cyclodextrin moieties, and comonomers which do not contain cyclodextrin moieties (comonomers), and wherein the CDP comprises at least four, five six, seven, eight, nine, ten, eleven, twelve, thirteen, fourteen, fifteen, sixteen, seventeen, eighteen, nineteen or twenty cyclodextrin moieties and at least four, five six, seven, eight, nine, ten, eleven, twelve, thirteen, fourteen, fifteen, sixteen, seventeen, eighteen, nineteen or twenty comonomers.

In some embodiments, the at least four, five six, seven, eight, etc., cyclodextrin moieties and at least four, five six, seven, eight, nine, ten, eleven, twelve, thirteen, fourteen, fifteen, sixteen, seventeen, eighteen, nineteen or twenty comonomers alternate in the water soluble linear polymer.

In some embodiments, the cyclodextrin moieties comprise linkers to which therapeutic agents may be further linked. In some embodiments, the comonomer is a compound containing residues of least two functional groups through which reaction and thus linkage of the cyclodextrin monomers is achieved. In some embodiments, the functional groups, which may be the same or different, terminal or internal, of each comonomer comprise an amino, acid, imidazole, hydroxyl, thio, acyl halide, -HC=CH-, -C≡C- group, or derivative thereof. In some embodiments, the residues of the two functional groups are the same and are located at termini of the comonomer. In some embodiments, a comonomer contains one or more pendant groups with at least one functional group through which reaction and thus linkage of a therapeutic agent can be achieved. In some embodiments, the functional groups, which may be the same or different, terminal or internal, of each comonomer pendant group comprise an amino, acid, imidazole, hydroxyl, thiol, acyl halide, ethylene, ethyne group, or derivative thereof. In some embodiments, the pendant group is a substituted or unsubstituted branched, cyclic or straight chain C₁-C₁₀ alkyl, or arylalkyl optionally containing one or more heteroatoms within the chain or ring.

In some embodiments, the cyclodextrin moiety comprises an alpha, beta, or gamma cyclodextrin moiety.

In some embodiments, the CDP is suitable for the attachment of sufficient therapeutic agent such that up to at least 5%, 10%, 15%, 20%, 25%, 30%, or even 35% by weight of the water soluble linear polymer, when conjugated, is therapeutic agent. In some embodiments, the molecular weight of the CDP is 10,000-500,000 Da, e.g., about 30,000 to about 100,000 Da.

In some embodiments, the cyclodextrin moieties make up at least about 2%, 5%, 10%, 11%, 12%, 13%, 14%, 15%, 16%, 17%, 18%, 19%, 20%, 30%, 50% or 80% of the polymer by weight.

In some embodiments, the CDP is made by a method comprising providing cyclodextrin moiety precursors modified to bear one reactive site at each of exactly two positions, and reacting the cyclodextrin moiety with comonomer precursors having exactly two reactive moieties capable of forming a covalent bond with the reactive sites under polymerization conditions that promote reaction of the reactive sites with the reactive moieties to form covalent bonds between the comonomers and the cyclodextrin moieties, whereby a CDP comprising alternating units of a cyclodextrin moiety and comonomer is produced.

In some embodiments, the CDP comprises a comonomer selected from the group consisting of: an alkylene chain, polysuccinic anhydride, poly-L-glutamic acid, poly(ethyleneimine), an oligosaccharide, and an amino acid chain. In some embodiments, a comonomer comprises a polyethylene glycol chain. In some embodiments, the CDP comprises a comonomer selected from the group consisting of: polyglycolic acid and polylactic acid chain.

In some embodiments, a comonomer comprises a hydrocarbylene group wherein one or more methylene groups is optionally replaced by a group Y (provided that none of the Y groups are adjacent to each other), wherein each Y, independently for each occurrence, is selected from, substituted or unsubstituted aryl, heteroaryl, cycloalkyl, heterocycloalkyl, or -O-, C(=X) (wherein X is NR₁, O or S), -OC(O)-, -C(=O)O, -NR₁-, -NR₁CO-,-C(O)NR₁-, -S(O)ₙ- (wherein n is 0, 1, or 2), -OC(O)-NR₁, -NR₁-C(O)-NR₁-, -NR₁1-C(NR₁)-NR₁-, and -B(OR₁)-; and R₁, independently for each occurrence, represents H or a lower alkyl.

In some embodiments, the CDP is a polymer of the following formula: 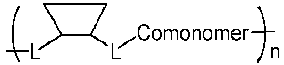 wherein each L is independently a linker, each comonomer is independently a comonomer described herein, and n is at least 4, 5, 6, 7, 8, 9, 10, 11, 12, 13, 14, 15, 16, 17, 18, 19 or 20. In some embodiments, the molecular weight of the comonomer is from about 2000 to about 5000 Da (e.g., from about 3000 to about 4000 Da (e.g., about 3400 Da).

In some embodiments, the CDP is a polymer of the following formula: 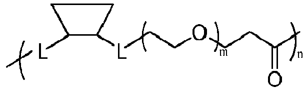 wherein each L is independently a linker,
wherein the group 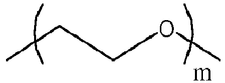 has a Mw of 3400 Da or less and n is at least 4, 5, 6, 7, 8, 9, 10, 11, 12, 13, 14, 15, 16, 17, 18, 19 or 20.

In some embodiments, 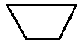 is alpha, beta or gamma cyclodextrin, e.g., beta cyclodextrin.

In some embodiments, each L independently comprises an amino acid or a derivative thereof. In some embodiments, at least one L comprises cysteine or a derivative thereof. In some embodiments, each L comprises cysteine. In some embodiments, each L is cysteine and the cysteine is connected to the CD by way of a thiol linkage.

In some embodiments, the CDP is a polymer of the following formula: 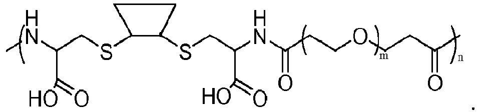 wherein the group 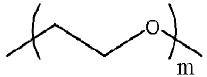 has a Mw of 3400 Da or less and n is at least 4, 5, 6, 7, 8, 9, 10, 11, 12, 13, 14, 15, 16, 17, 18, 19 or 20.

In some embodiments, 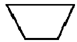 is alpha, beta or gamma cyclodextrin, e.g., beta cyclodextrin.

In some embodiments, the CDP is a polymer of the following formula: 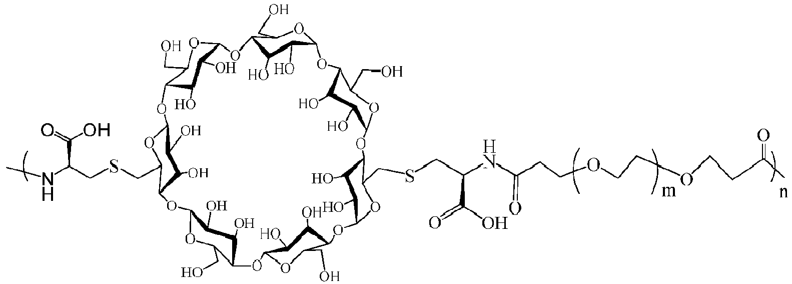 wherein the group 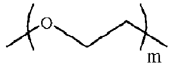 has a Mw of 3400 Da or less and n is at least 4, 5, 6, 7, 8, 9, 10, 11, 12, 13, 14, 15, 16, 17, 18, 19 or 20.

In some embodiments, the group 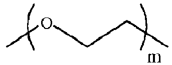 has a Mw of 3400 Da and the Mw of the compound as a whole is from 27,000 Da to 99,600 Da.

The CDPs described herein can be made using a variety of methods including those described herein. In some embodiments, a CDP can be made by: providing cyclodextrin moiety precursors; providing comonomer precursors which do not contain cyclodextrin moieties (comonomer precursors); and copolymerizing the said cyclodextrin moiety precursors and comonomer precursors to thereby make a CDP wherein CDP comprises at least four, five six, seven, eight, or more, cyclodextrin moieties and at least four, five six, seven, eight, or more, comonomers.

In some embodiments, the at least four, five, six, seven, eight, or more cyclodextrin moieties and at least four, five, six, seven, eight, or more comonomers alternate in the water soluble linear polymer. In some embodiments, the method includes providing cyclodextrin moiety precursors modified to bear one reactive site at each of exactly two positions, and reacting the cyclodextrin moiety precursors with comonomer precursors having exactly two reactive moieties capable of forming a covalent bond with the reactive sites under polymerization conditions that promote reaction of the reactive sites with the reactive moieties to form covalent bonds between the comonomers and the cyclodextrin moieties, whereby a CDP comprising alternating units of a cyclodextrin moiety and a comonomer is produced.

In some embodiments, the cyclodextrin comonomers comprise linkers to which therapeutic agents may be further linked. In some embodiments, the therapeutic agents are linked via second linkers.

In some embodiments, the comonomer precursor is a compound containing at least two functional groups through which reaction and thus linkage of the cyclodextrin moieties is achieved. In some embodiments, the functional groups, which may be the same or different, terminal or internal, of each comonomer precursor comprise an amino, acid, imidazole, hydroxyl, thio, acyl halide, -HC=CH-, -C≡C- group, or derivative thereof. In some embodiments, the two functional groups are the same and are located at termini of the comonomer precursor. In some embodiments, a comonomer contains one or more pendant groups with at least one functional group through which reaction and thus linkage of a therapeutic agent can be achieved. In some embodiments, the functional groups, which may be the same or different, terminal or internal, of each comonomer pendant group comprise an amino, acid, imidazole, hydroxyl, thiol, acyl halide, ethylene, ethyne group, or derivative thereof. In some embodiments, the pendant group is a substituted or unsubstituted branched, cyclic or straight chain C₁-C₁₀ alkyl, or arylalkyl optionally containing one or more heteroatoms within the chain or ring.

In some embodiments, the cyclodextrin moiety comprises an alpha, beta, or gamma cyclodextrin moiety.

In some embodiments, the CDP is suitable for the attachment of sufficient therapeutic agent such that up to at least 3%, 5%, 10%, 15%, 20%, 25%, 30%, or even 35% by weight of the CDP, when conjugated, is therapeutic agent.

In some embodiments, the CDP has a molecular weight of 10,000-500,000 Da. In some embodiments, the cyclodextrin moieties make up at least about 2%, 5%, 10%, 20%, 30%, 50% or 80% of the CDP by weight.

In some embodiments, the CDP comprises a comonomer selected from the group consisting of: an alkylene chain, polysuccinic anhydride, poly-L-glutamic acid, poly(ethyleneimine), an oligosaccharide, and an amino acid chain. In some embodiments, a comonomer comprises a polyethylene glycol chain. In some embodiments, the CDP comprises a comonomer selected from the group consisting of: polyglycolic acid and polylactic acid chain. In some embodiments, the CDP comprises a comonomer selected from the group consisting of a comonomer comprises a hydrocarbylene group wherein one or more methylene groups is optionally replaced by a group Y (provided that none of the Y groups are adjacent to each other), wherein each Y, independently for each occurrence, is selected from, substituted or unsubstituted aryl, heteroaryl, cycloalkyl, heterocycloalkyl, or -O-, C(=X) (wherein X is NR₁, O or S),-OC(O)-, -C(=O)O, -NR₁-, -NR₁CO-, -C(O)NR₁-, -S(O)ₙ- (wherein n is 0, 1, or 2),-OC(O)-NR₁, -NR₁-C(O)-NR₁-, -NR₁-C(NR₁)-NR₁-, and -B(OR₁)-; and R_{1,} independently for each occurrence, represents H or a lower alkyl.

In some embodiments, a CDP of the following formula can be made by the scheme below: 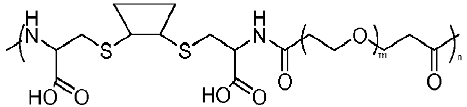 providing a compound of formula AA and formula BB: 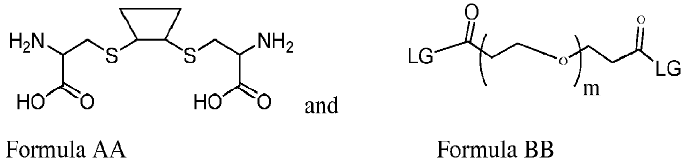 wherein LG is a leaving group;
and contacting the compounds under conditions that allow for the formation of a covalent bond between the compounds of formula AA and BB, to form a polymer of the following formula: 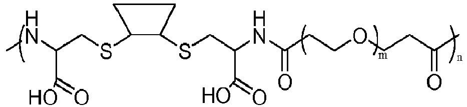 wherein the group 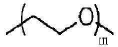 has a Mw of 3400 Da or less and n is at least 4, 5, 6, 7, 8, 9, 10, 11, 12, 13, 14, 15, 16, 17, 18, 19 or 20.

In some embodiments, Formula BB is

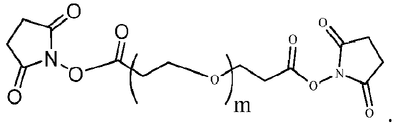

In some embodiments, the group 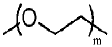 has a Mw of 3400 Da and the Mw of the compound is from 27,000 Da to 99,600 Da.

In some embodiments, the compounds of formula AA and formula BB are contacted in the presence of a base. In some embodiments, the base is an amine containing base. In some embodiments, the base is DEA.

In some embodiments, a CDP of the following formula can be made by the scheme below: 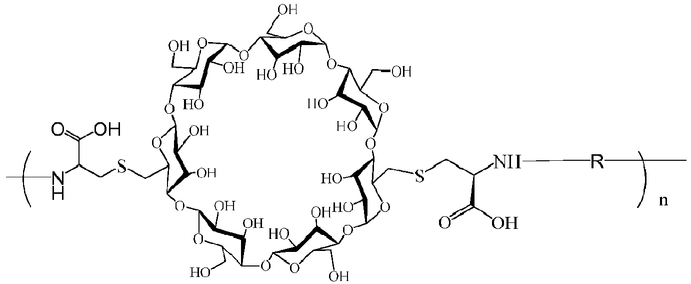 wherein R is of the form: 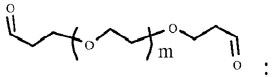 comprising the steps of:
reacting a compound of the formula below: 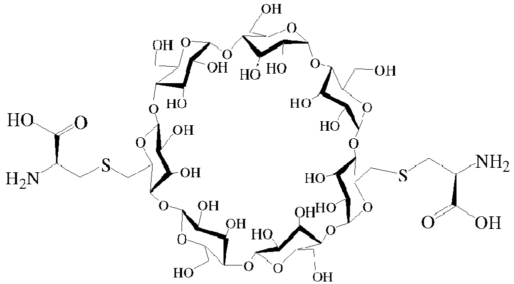 with a compound of the formula below: 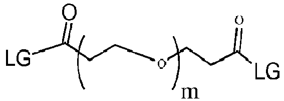 wherein the group 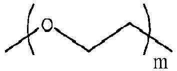 has a Mw of 3400 Da or less and n is at least 4, 5, 6, 7, 8, 9, 10, 11, 12, 13, 14, 15, 16, 17, 18, 19 or 20,

in the presence of a non-nucleophilic organic base in a solvent.

In some embodiments, 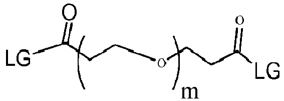 is

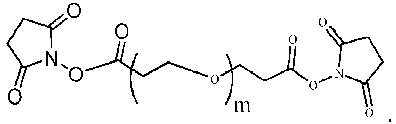

In some embodiments, the solvent is a polar aprotic solvent. In some embodiments, the solvent is DMSO.

In some embodiments, the method also includes the steps of dialysis; and lyophylization.

In some embodiments, a CDP provided below can be made by the following scheme: 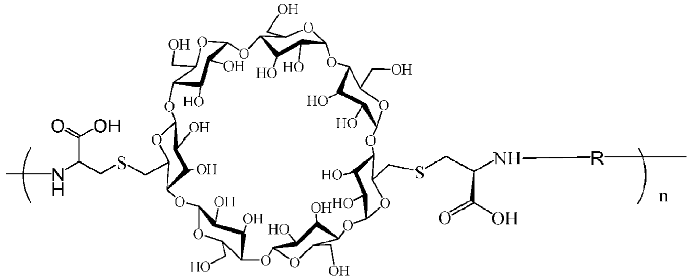 wherein R is of the form: 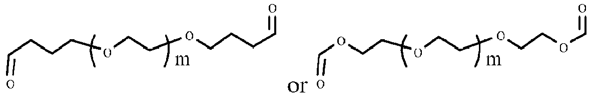 comprising the steps of:
reacting a compound of the formula below: 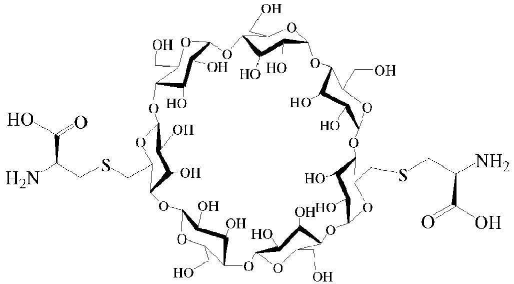 with a compound of the formula below: 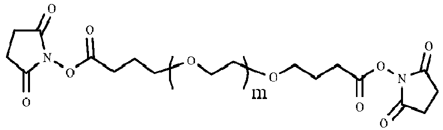 wherein the group 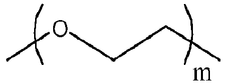 has a Mw of 3400 Da or less and n is at least 4, 5, 6, 7, 8, 9, 10, 11, 12, 13, 14, 15, 16, 17, 18, 19 or 20, or with a compound provided below: 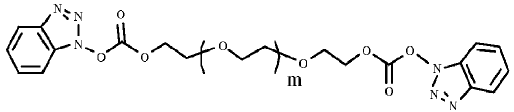 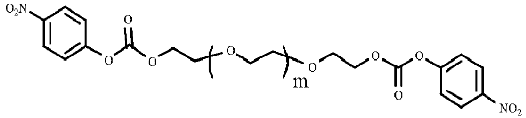 wherein the group 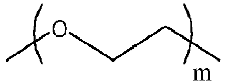 has a Mw of 3400 Da;
in the presence of a non-nucleophilic organic base in DMSO;
and dialyzing and lyophilizing the following polymer

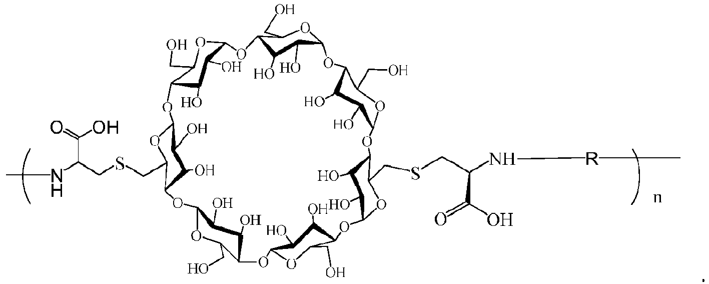

A CDP described herein may be attached to or grafted onto a substrate. The substrate may be any substrate as recognized by those of ordinary skill in the art. In another preferred embodiment of the invention, a CDP may be crosslinked to a polymer to form, respectively, a crosslinked cyclodextrin copolymer or a crosslinked oxidized cyclodextrin copolymer. The polymer may be any polymer capable of crosslinking with a CDP (e.g., polyethylene glycol (PEG) polymer, polyethylene polymer). The polymer may also be the same or different CDP. Thus, for example, a linear CDP may be crosslinked to any polymer including, but not limited to, itself, another linear CDP, and a linear oxidized CDP. A crosslinked linear CDP may be prepared by reacting a linear CDP with a polymer in the presence of a crosslinking agent. A crosslinked linear oxidized CDP may be prepared by reacting a linear oxidized CDP with a polymer in the presence of an appropriate crosslinking agent. The crosslinking agent may be any crosslinking agent known in the art. Examples of crosslinking agents include dihydrazides and disulfides. In a preferred embodiment, the crosslinking agent is a labile group such that a crosslinked copolymer may be uncrosslinked if desired.

A linear CDP and a linear oxidized CDP may be characterized by any means known in the art. Such characterization methods or techniques include, but are not limited to, gel permeation chromatography (GPC), matrix assisted laser desorption ionization-time of flight mass spectrometry (MALDT-TOF Mass spec), ¹H and ¹³C NMR, light scattering and titration.

The invention also provides a cyclodextrin composition containing at least one linear CDP and at least one linear oxidized CDP as described above. Accordingly, either or both of the linear CDP and linear oxidized CDP may be crosslinked to another polymer and/or bound to a ligand as described above. Therapeutic compositions according to the invention contain a therapeutic agent and a linear CDP or a linear oxidized CDP, including crosslinked copolymers. A linear CDP, a linear oxidized CDP and their crosslinked derivatives are as described above. The therapeutic agent may be any synthetic, semi-synthetic or naturally occurring biologically active therapeutic agent, including those known in the art.

One aspect of the present invention contemplates attaching a therapeutic agent to a CDP for delivery of a therapeutic agent. The present invention discloses various types of linear, branched, or grafted CDPs wherein a therapeutic agent is covalently bound to the polymer. In certain embodiments, the therapeutic agent is covalently linked via a biohydrolyzable bond, for example, an ester, amide, carbamates, or carbonate. An exemplary synthetic scheme for covalently bonding a derivatized CD to a therapeutic agent (T.A.) is shown in Scheme I.

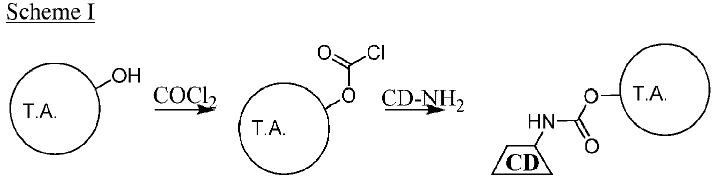

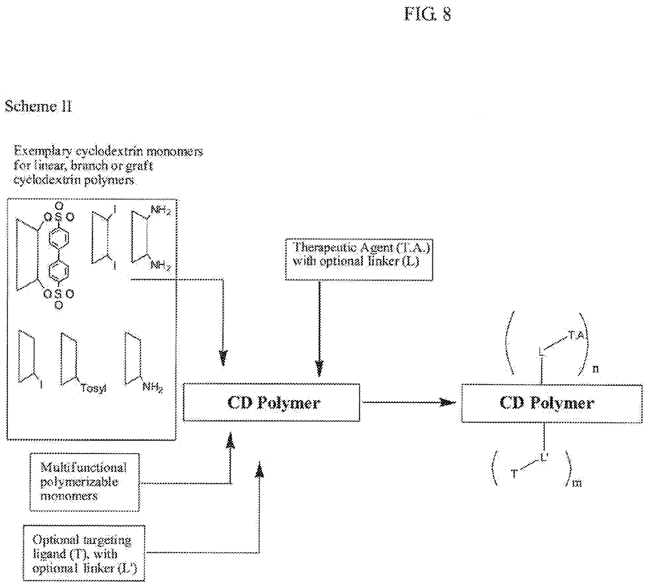

A general strategy for synthesizing linear, branched or grafted cyclodextrin-containing polymers (CDPs) for loading a therapeutic agent, and an optional targeting ligand is shown in FIG. 8. As described below in Schemes II-XIV, this general strategy can be used to achieve a variety of different cyclodextrin-containing polymers for the delivery of therapeutic agents, e.g., cytotoxic agents, e.g., topoisomerase inhibitors, e.g., a topoisomerase I inhibitor (e.g., camptothecin, irinotecan, SN-38, topotecan, lamellarin D, lurotecan, exatecan, diflomotecan, or derivatives thereof), or a topoisomerase II inhibitor (e.g., an etoposide, a tenoposide, doxorubicin, or derivatives thereof), an anti-metabolic agent (e.g., an antifolate (e.g., pemetrexed, floxuridine, or raltitrexed) or a pyrimidine conjugate (e.g., capecitabine, cytarabine, gemcitabine, or 5FU)), an alkylating agent, an anthracycline, an anti-tumor antibiotic (e.g., a HSP90 inhibitor, e.g., geldanamycin), a platinum based agent (e.g., cisplatin, carboplatin, or oxaliplatin), a microtubule inhibitor, a kinase inhibitor (e.g., a seronine/threonine kinase inhibitor, e.g., a mTOR inhibitor, e.g., rapamycin) or a proteasome inhibitor. The resulting CDPs are shown graphically as polymers (A)-(L) of FIG. 1.

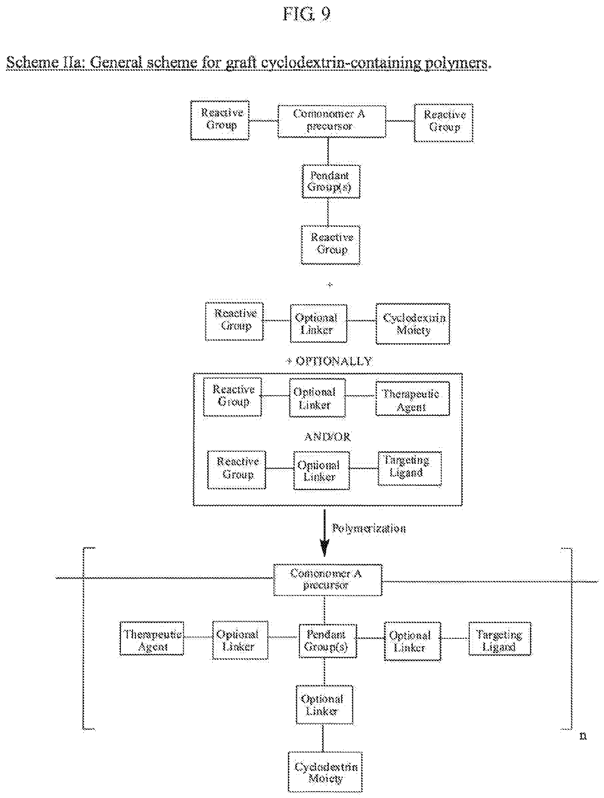

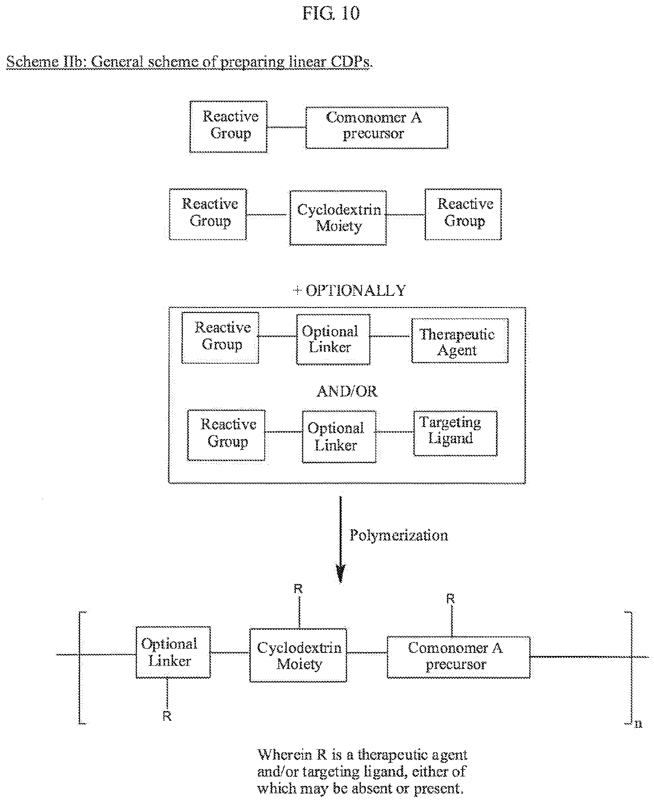

For example, comonomer precursors (shown in FIG. 9 as A), cyclodextrin moieties, therapeutic agents, and/or targeting ligands may be assembled as shown in FIGS. 9 and 10. Note that in FIGS. 9 and 10, in any given reaction there may be more than one comonomer precursor, cyclodextrin moiety, therapeutic agent or targeting ligand that is of the same type or different. Furthermore, prior to polymerization, one or more comonomer precursor, cyclodextrin moiety, therapeutic agent or targeting ligand may be covalently linked with each other in one or more separate step. The scheme as provided above includes embodiments, where not all available positions for attachment of the therapeutic agent are occupied on the CDP. For example, in some embodiments, less than all of the available points of attachments are reacted, leaving less than 100% yield of the therapeutic agent onto the polymer. Accordingly, the loading of the therapeutic agent onto the polymer can vary. This is also the case regarding a targeting agent when a targeting agent is included.

FIG. 9: Scheme IIa: General scheme for graft CDPs. The comonomer A precursor, cyclodextrin moiety, therapeutic agent and optional targeting ligand are as defined in FIG. 9. Furthermore, one skilled in the art may choose from a variety of reactive groups, e.g., hydroxyls, carboxyls, halides, amines, and activated ethenes, ethynes, or aromatic groups in order achieve polymerization. For further examples of reactive groups are disclosed in Advanced Organic Chemistry: Reactions, Mechanisms, and Structure, 5th Edition, 2000.

In some embodiments, one or more of the therapeutic agent moieties in the CDP-therapeutic agent conjugate can be replaced with another therapeutic agent, e.g., another cytotoxic agent or immunomodulator.

FIG. 10: Scheme IIb: General scheme of preparing linear CDPs. One skilled in the art would recognize that by choosing a comonomer A precursor that has multiple reactive groups polymer branching can be achieved.

In some embodiments, one or more of the therapeutic agent moieties in the CDP-therapeutic agent conjugate can be replaced with another therapeutic agent, e.g., another cytotoxic agent or immunomodulator.

Examples of different ways of synthesizing CDP-therapeutic agent conjugates are shown in Schemes III-VIII below. In each of Schemes III-VIII, one or more of the therapeutic agent moieties in the CDP-therapeutic agent conjugate can be replaced with another therapeutic agent, e.g., another cytotoxic agent or immunomodulator.

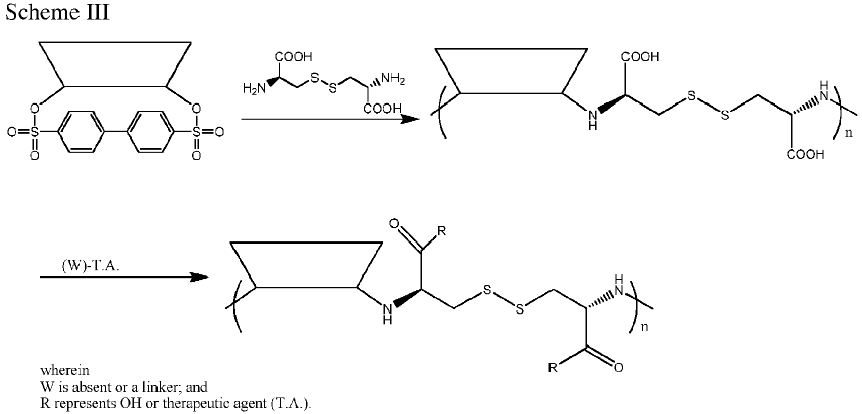

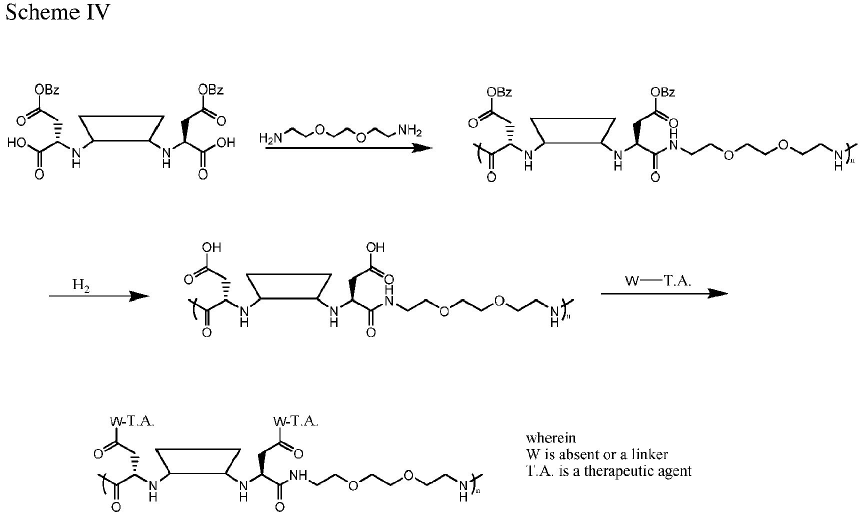

Scheme IV, as provided above, includes embodiments where W-therapeutic agent is absent in one or more positions as provided above. This can be achieved, for example, when less than 100% yield is achieved when coupling the therapeutic agent to the polymer and/or when less than an equivalent amount of therapeutic agent is used in the reaction. Accordingly, the loading of the therapeutic agent, by weight of the polymer, can vary.

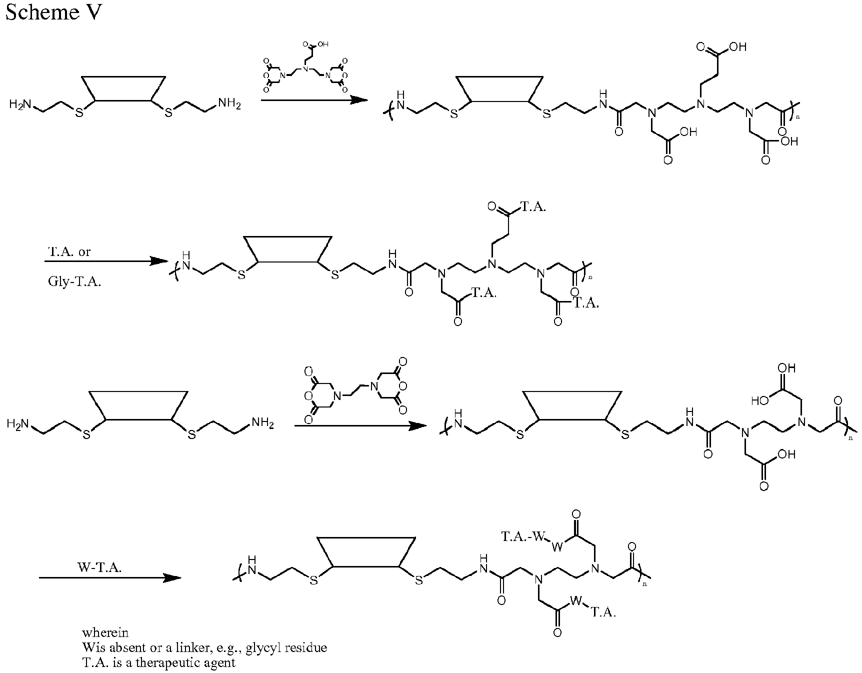

Scheme V, as provided above, includes embodiments where W-therapeutic agent is absent in one or more positions as provided above. This can be achieved, for example, when less than 100% yield is achieved when coupling the therapeutic agent to the polymer and/or when less than an equivalent amount of therapeutic agent is used in the reaction. Accordingly, the loading of the therapeutic agent, by weight of the polymer, can vary.

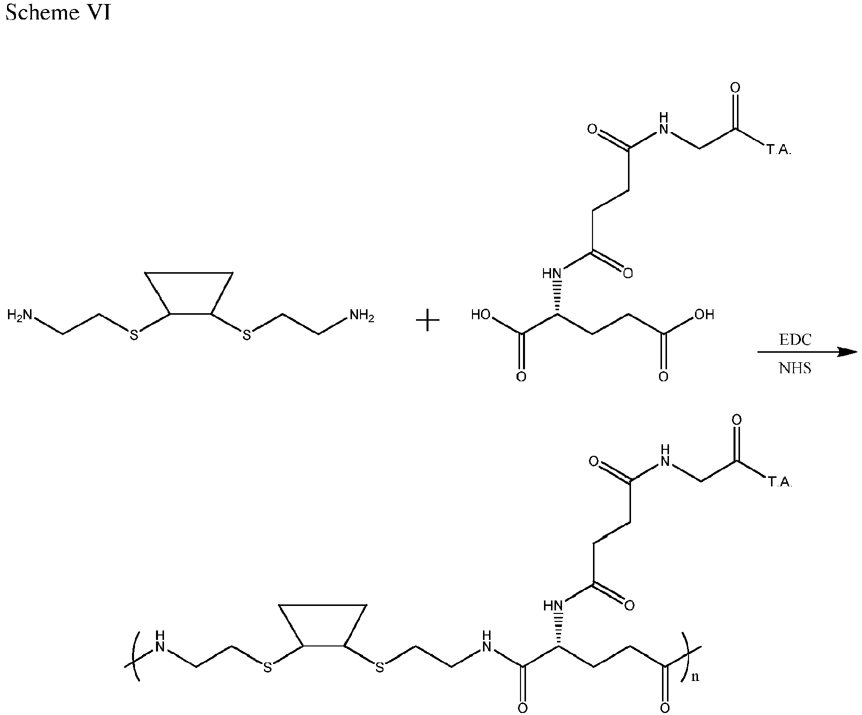

Scheme VI, as provided above, includes embodiments where therapeutic agent is absent in one or more positions as provided above. This can be achieved, for example, when less than 100% yield is achieved when coupling the therapeutic agent to the polymer and/or when less than an equivalent amount of therapeutic agent is used in the reaction. Accordingly, the loading of the therapeutic agent, by weight of the polymer, can vary.

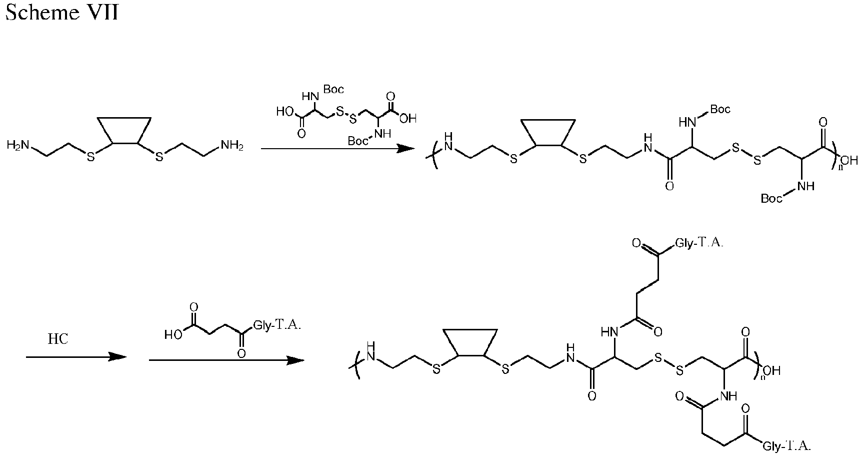

Scheme VII, as provided above, includes embodiments where gly-therapeutic agent is absent in one or more positions as provided above. This can be achieved, for example, when less than 100% yield is achieved when coupling the therapeutic agent to the polymer and/or when less than an equivalent amount of therapeutic agent is used in the reaction. Accordingly, the loading of the therapeutic agent, by weight of the polymer, can vary.

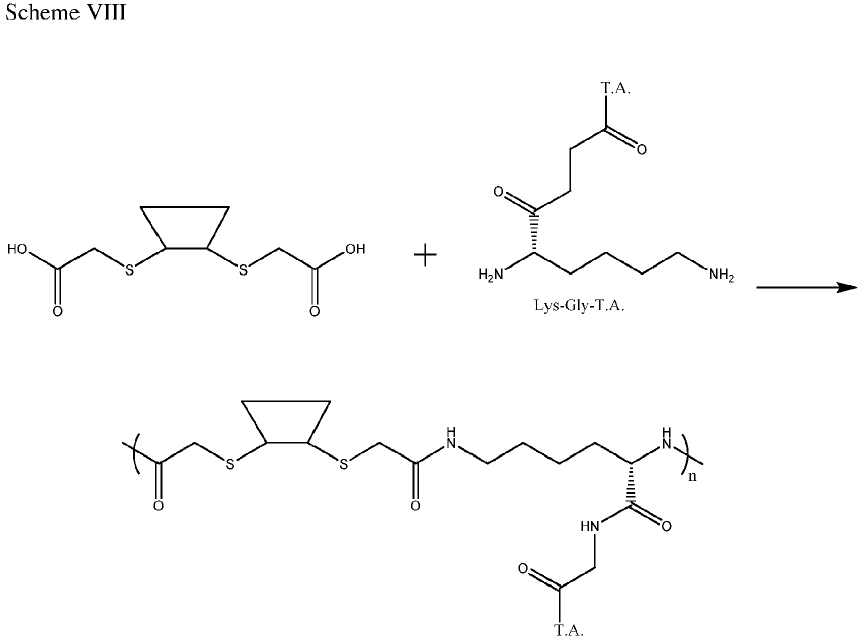

Scheme VIII, as provided above, includes embodiments where therapeutic agent is absent in one or more positions as provided above. This can be achieved, for example, when less than 100% yield is achieved when coupling the therapeutic agent to the polymer and/or when less than an equivalent amount of therapeutic agent is used in the reaction. Accordingly, the loading of the therapeutic agent, by weight of the polymer, can vary.

Additional examples of methods of synthesizing CDP-therapeutic agent conjugates are shown in Schemes IX-XIV below. In each of Schemes IX-XIV, one or more of the therapeutic agent moieties in the CDP-therapeutic agent conjugate can be replaced with another therapeutic agent, e.g., another cytotoxic agent or immunomodulator.

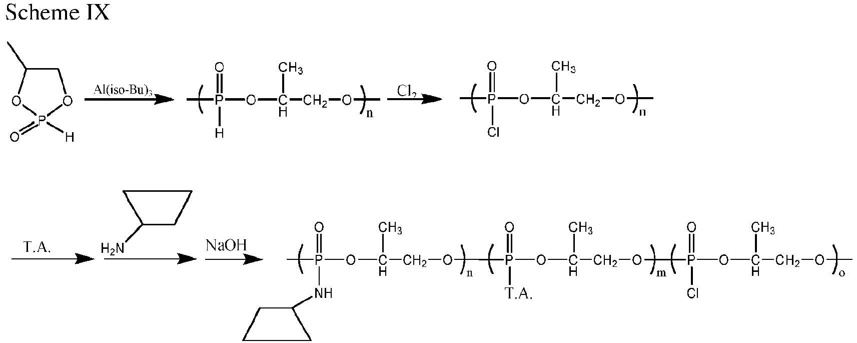

Scheme IX, as provided above, includes embodiments where therapeutic agent is absent in one or more positions as provided above. This can be achieved, for example, when less than 100% yield is achieved when coupling the therapeutic agent to the polymer and/or when less than an equivalent amount of therapeutic agent is used in the reaction. Accordingly, the loading of the therapeutic agent, by weight of the polymer, can vary. 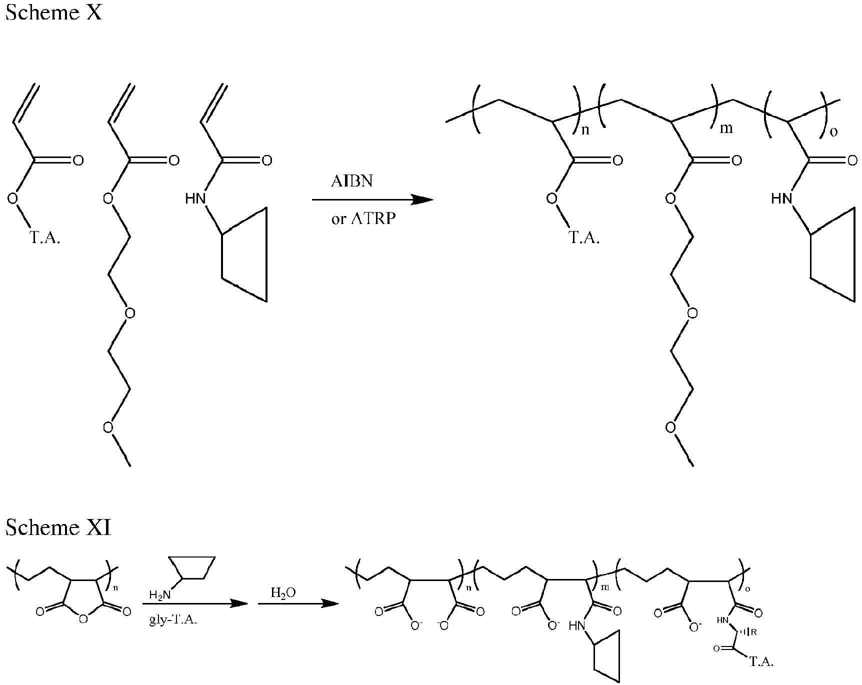 Scheme XI, as provided above, includes embodiments where gly-therapeutic agent is absent in one or more positions as provided above. This can be achieved, for example, when less than 100% yield is achieved when coupling the therapeutic agent to the polymer and/or when less than an equivalent amount of therapeutic agent is used in the reaction. Accordingly, the loading of the therapeutic agent, by weight of the polymer, can vary.

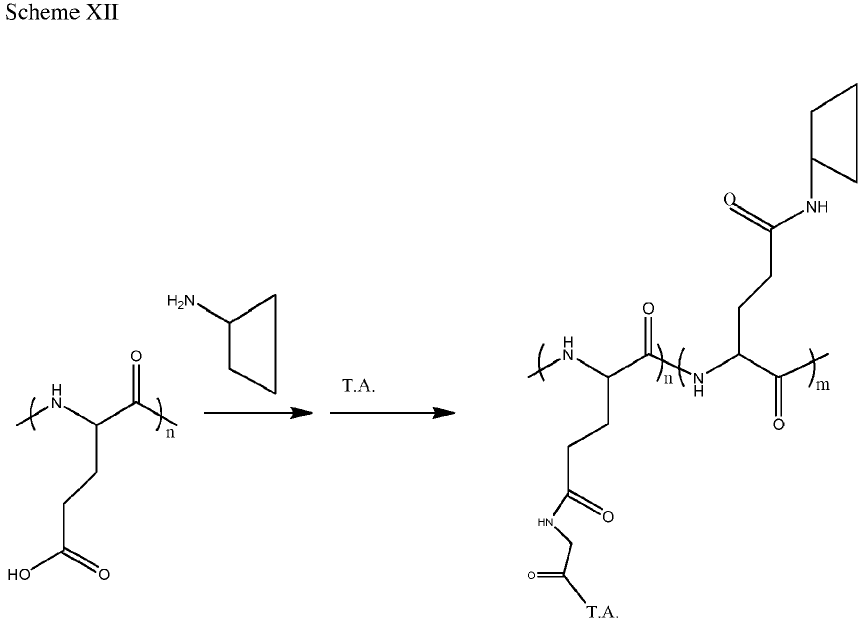

Scheme XII, as provided above, includes embodiments where therapeutic agent is absent in one or more positions as provided above. This can be achieved, for example, when less than 100% yield is achieved when coupling the therapeutic agent to the polymer and/or when less than an equivalent amount of therapeutic agent is used in the reaction. Accordingly, the loading of the therapeutic agent, by weight of the polymer, can vary.

The present invention further contemplates CDPs and CDP-conjugates synthesized using CD-biscysteine monomer and a di-NHS ester such as PEG-DiSPA or PEG-BTC as shown in Schemes XIII-XIV below.

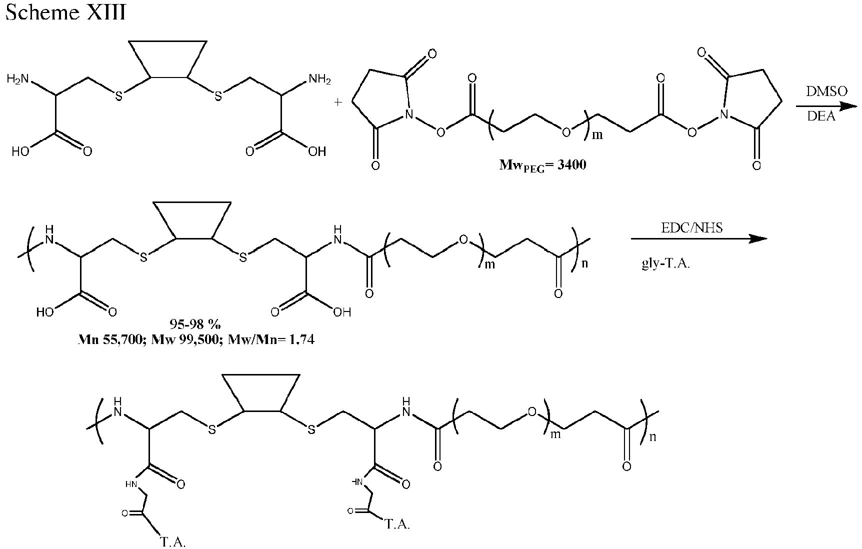

Scheme XIII, as provided above, includes embodiments where gly-therapeutic agent is absent in one or more positions as provided above. This can be achieved, for example, when less than 100% yield is achieved when coupling the therapeutic agent to the polymer and/or when less than an equivalent amount of therapeutic agent is used in the reaction. Accordingly, the loading of the therapeutic agent, by weight of the polymer, can vary.

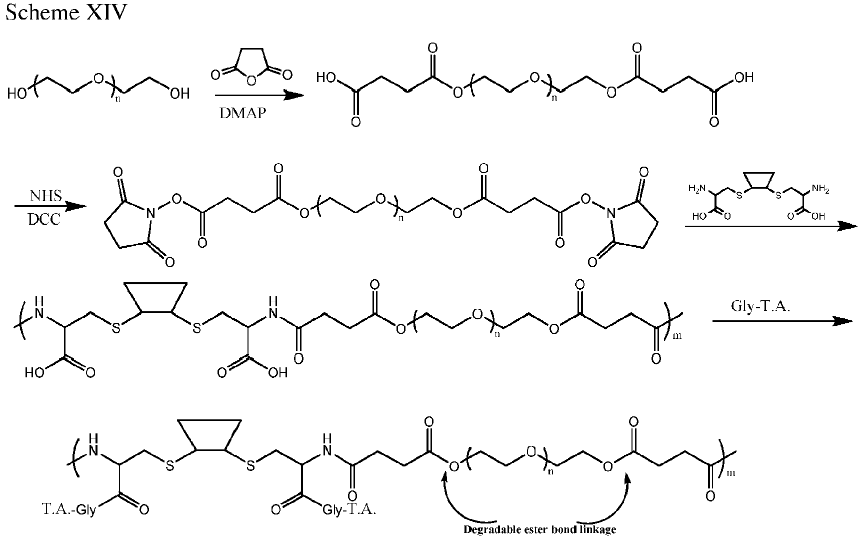

Scheme XIV, as provided above, includes embodiments where gly-therapeutic agent is absent in one or more positions as provided above. This can be achieved, for example, when less than 100% yield is achieved when coupling the therapeutic agent to the polymer and/or when less than an equivalent amount of therapeutic agent is used in the reaction. Accordingly, the loading of the therapeutic agent, by weight of the polymer, can vary.

In some embodiments, a CDP-therapeutic agent conjugate can be made by providing a CDP comprising cyclodextrin moieties and comonomers which do not contain cyclodextrin moieties (comonomers), wherein the cyclodextrin moieties and comonomers alternate in the CDP and wherein the CDP comprises at least four, five, six, seven, eight, etc. cyclodextrin moieties and at least four, five, six, seven, eight, etc. comonomers; and attaching a therapeutic agent to the CDP.

In some embodiments, one or more of the therapeutic agent moieties in the CDP-therapeutic agent conjugate can be replaced with another therapeutic agent, e.g., another cytotoxic agent or immunomodulator.

In some embodiments, the therapeutic agent is attached via a linker. In some embodiments, the therapeutic agent is attached to the water soluble linear polymer through an attachment that is cleaved under biological conditions to release the therapeutic agent. In some embodiments, the therapeutic agent is attached to the water soluble linear polymer at a cyclodextrin moiety or a comonomer. In some embodiments, the therapeutic agent is attached to the water soluble linear polymer via an optional linker to a cyclodextrin moiety or a comonomer.

In some embodiments, the cyclodextrin moieties comprise linkers to which therapeutic agents are linked. In some embodiments, the cyclodextrin moieties comprise linkers to which therapeutic agents are linked via a second linker. In some embodiments, the CDP is made by a process comprising: providing cyclodextrin moiety precursors, providing comonomer precursors, and copolymerizing said cyclodextrin moiety precursors and comonomer precursors to thereby make a CDP comprising cyclodextrin moieties and comonomers. In some embodiments, the CDP is conjugated with a therapeutic agent to provide a CDP-therapeutic agent conjugate. In some embodiments, the method includes providing cyclodextrin moiety precursors modified to bear one reactive site at each of exactly two positions, and reacting the cyclodextrin moiety precursors with comonomer precursors having exactly two reactive moieties capable of forming a covalent bond with the reactive sites under polymerization conditions that promote reaction of the reactive sites with the reactive moieties to form covalent bonds between the comonomers and the cyclodextrin moieties, whereby a CDP comprising alternating units of a cyclodextrin moiety and a comonomer is produced.

In some embodiments, the therapeutic agent is attached to the CDP via a linker. In some embodiments, the linker is cleaved under biological conditions.

In some embodiments, the therapeutic agent makes up at least 5%, 10%, 15%, 20%, 25%, 30%, or even 35% by weight of the CDP-therapeutic agent conjugate. In some embodiments, at least about 50% of available positions on the CDP are reacted with a therapeutic agent and/or a linker therapeutic agent (e.g., at least about 55%, 60%, 65%, 70%, 75%, 80%, 85%, 90%, or 95%).

In some embodiments, the comonomer comprises polyethylene glycol of molecular weight 3,400 Da, the cyclodextrin moiety comprises beta-cyclodextrin, the theoretical maximum loading of therapeutic agent on the CDP-therapeutic agent conjugate is 19%, and therapeutic agent is 17-21 % by weight of the CDP-therapeutic agent conjugate. In some embodiments, about 80-90% of available positions on the CDP are reacted with a therapeutic agent and/or a linker therapeutic agent.

In some embodiments, the comonomer precursor is a compound containing at least two functional groups through which reaction and thus linkage of the cyclodextrin moieties is achieved. In some embodiments, the functional groups, which may be the same or different, terminal or internal, of each comonomer precursor comprise an amino, acid, imidazole, hydroxyl, thio, acyl halide, -HC=CH-, -C≡C- group, or derivative thereof. In some embodiments, the two functional groups are the same and are located at termini of the comonomer precursor. In some embodiments, a comonomer contains one or more pendant groups with at least one functional group through which reaction and thus linkage of a therapeutic agent is achieved. In some embodiments, the functional groups, which may be the same or different, terminal or internal, of each comonomer pendant group comprise an amino, acid, imidazole, hydroxyl, thiol, acyl halide, ethylene, ethyne group, or derivative thereof. In some embodiments, the pendant group is a substituted or unsubstituted branched, cyclic or straight chain C1-C10 alkyl, or arylalkyl optionally containing one or more heteroatoms within the chain or ring.

In some embodiments, the cyclodextrin moiety comprises an alpha, beta, or gamma cyclodextrin moiety.

In some embodiments, the therapeutic agent is poorly soluble in water.

In some embodiments, the solubility of the therapeutic agent is <5 mg/ml at physiological pH.

In some embodiments, the therapeutic agent is a hydrophobic compound with a log P>0.4, >0.6, >0.8, >1, >2, >3, >4, or >5. In some embodiments, the therapeutic agent is hydrophobic and is attached via a second compound.

In some embodiments, administration of the CDP-therapeutic agent conjugate to a subject results in release of the therapeutic agent over a period of at least 6 hours. In some embodiments, administration of the CDP-therapeutic agent conjugate to a subject results in release of the therapeutic agent over a period of 6 hours to a month. In some embodiments, upon administration of the CDP-therapeutic agent conjugate to a subject the rate of therapeutic agent release is dependent primarily upon the rate of hydrolysis as opposed to enzymatic cleavage.

In some embodiments, the CDP-therapeutic agent conjugate has a molecular weight of 10,000-500,000 Da.

In some embodiments, the cyclodextrin moieties make up at least about 2%, 5%, 10%, 20%, 30%, 50% or 80% of the polymer by weight.

In some embodiments, the CDP includes a comonomer selected from the group consisting of: an alkylene chain, polysuccinic anhydride, poly-L-glutamic acid, poly(ethyleneimine), an oligosaccharide, and an amino acid chain. In some embodiments, a comonomer comprises a polyethylene glycol chain. In some embodiments, a comonomer comprises a polyglycolic acid or polylactic acid chain. In some embodiments, a comonomer comprises a hydrocarbylene group wherein one or more methylene groups is optionally replaced by a group Y (provided that none of the Y groups are adjacent to each other), wherein each Y, independently for each occurrence, is selected from, substituted or unsubstituted aryl, heteroaryl, cycloalkyl, heterocycloalkyl, or -O-, C(=X) (wherein X is NR₁, O or S), -OC(O)-, -C(=O)O, -NR₁-, -NR₁CO-,-C(O)NR₁-, -S(O)ₙ- (wherein n is 0, 1, or 2), -OC(O)-NR₁, -NR₁-C(O)-NR₁-, -NR₁-C(NR₁)-NR₁-, and -B(OR₁)-; and R₁, independently for each occurrence, represents H or a lower alkyl.

In some embodiments, a CDP-polymer conjugate of the following formula can be made as follows: 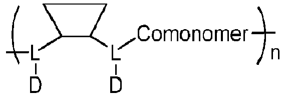 providing a polymer of the formula below: 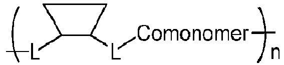 and coupling the polymer with a plurality of D moieties, wherein each D is independently absent or a therapeutic agent, to provide: 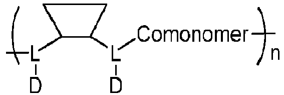 wherein the comonomer has a Mw of 2000 to 5000 Da (e.g., 3000 to 4000 Da, e.g., 3200 Da to about 3800 Da, e.g., about 3400 Da) and n is at least 4, 5, 6, 7, 8, 9, 10, 11, 12, 13, 14, 15, 16, 17, 18, 19 or 20.

In some embodiments, one or more of the therapeutic agent moieties in the CDP-therapeutic agent conjugate can be replaced with another therapeutic agent, e.g., another cytotoxic agent or immunomodulator.

In some embodiments, a CDP-polymer conjugate of the following formula can be made as follows: 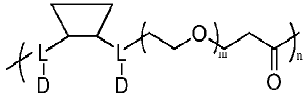 providing a polymer of the formula below: 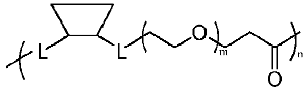 and coupling the polymer with a plurality of D moieties, wherein each D is independently absent or a therapeutic agent, to provide: 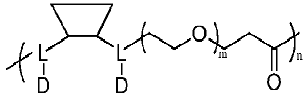 wherein the group 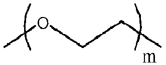 has a Mw of 4000 Da or less, e.g., 3200 to 3800 Da, e.g., 3400 Da and n is at least 4, 5, 6, 7, 8, 9, 10, 11, 12, 13, 14, 15, 16, 17, 18, 19 or 20.

In some embodiments, one or more of the therapeutic agent moieties in the CDP-therapeutic agent conjugate can be replaced with another therapeutic agent, e.g., another cytotoxic agent or immunomodulator.

The reaction scheme as provided above includes embodiments where D is absent in one or more positions as provided above. This can be achieved, for example, when less than 100% yield is achieved when coupling the therapeutic agent to the polymer (e.g., 80-90%) and/or when less than an equivalent amount of therapeutic agent is used in the reaction. Accordingly, the loading of the therapeutic agent, by weight of the polymer, can vary, for example, the loading of the therapeutic agent can be at least about 3% by weight, e.g., at least about 5%, at least about 8%, at least about 10%, at least about 13%, at least about 15%, or at least about 20%.

In some embodiments, a CDP-polymer conjugate of the following formula can be made as follows: 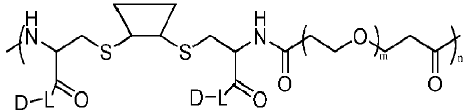 providing a polymer below: 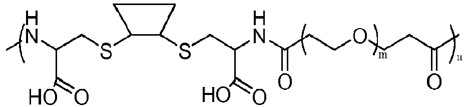 and coupling the polymer with a plurality of L-D moieties, wherein L is a linker or absent and D is a therapeutic agent, to provide: 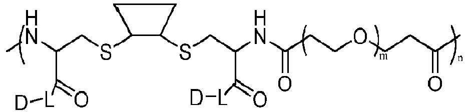 wherein the group 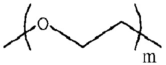 has a Mw of 4000 Da or less, e.g., 3200 to 3800 Da, e.g., 3400 Da and n is at least 4, 5, 6, 7, 8, 9, 10, 11, 12, 13, 14, 15, 16, 17, 18, 19 or 20.

In some embodiments, one or more of the therapeutic agent moieties in the CDP-therapeutic agent conjugate can be replaced with another therapeutic agent, e.g., another cytotoxic agent or immunomodulator.

The reaction scheme as provided above includes embodiments where L-D is absent in one or more positions as provided above. This can be achieved, for example, when less than 100% yield is achieved when coupling the therapeutic agent-linker to the polymer (e.g., 80-90%) and/or when less than an equivalent amount of therapeutic agent-linker is used in the reaction. Accordingly, the loading of the therapeutic agent, by weight of the polymer, can vary, for example, the loading of the therapeutic agent can be at least about 3% by weight, e.g., at least about 5%, at least about 8%, at least about 10%, at least about 13%, at least about 15%, or at least about 20%.

In some embodiments, at least a portion of the L moieties of L-D is absent. In some embodiments, each L is independently an amino acid or derivative thereof (e.g., glycine).

In some embodiments, the coupling of the polymer with the plurality of L-D moieties results in the formation of a plurality of amide bonds.

In certain instances, the CDPs are random copolymers, in which the different subunits and/or other monomeric units are distributed randomly throughout the polymer chain. Thus, where the formula Xₘ-Yₙ-Zₒ appears, wherein X, Y and Z are polymer subunits, these subunits may be randomly interspersed throughout the polymer backbone. In part, the term "random" is intended to refer to the situation in which the particular distribution or incorporation of monomeric units in a polymer that has more than one type of monomeric units is not directed or controlled directly by the synthetic protocol, but instead results from features inherent to the polymer system, such as the reactivity, amounts of subunits and other characteristics of the synthetic reaction or other methods of manufacture, processing, or treatment.

In some embodiments, one or more of the therapeutic agent (e.g., cytotoxic agent or immunomodulator) in the CDP-therapeutic agent conjugate (e.g., CDP-cytotoxic agent conjugate or CDP-immunomodulator conjugate) can be replaced with another therapeutic agent, e.g., a cytotoxic agent or immunomodulator such as another anticancer agent or anti-inflammatory agent.

The reaction scheme as provided above includes embodiments where L-D is absent in one or more positions as provided above. This can be achieved, for example, when less than 100% yield is achieved when coupling the therapeutic agent (e.g., topoisomerase inhibitor)-linker to the polymer and/or when less than an equivalent amount of therapeutic agent (e.g., topoisomerase inhibitor)-linker is used in the reaction. Accordingly, the loading of the therapeutic agent (e.g., topoisomerase inhibitor), by weight of the polymer, can vary, for example, the loading of the therapeutic agent (e.g., topoisomerase inhibitor) can be at least about 3% by weight, e.g., at least about 5%, at least about 8%, at least about 10%, at least about 11%, at least about 12%, at least about 13%, at least about 14%, at least about 15%, at least about 20%, at least about 25%, at least about 30%, at least about 35%, at least about 40%, at least about 45%, or at least about 50%.

In some embodiments, at least a portion of the L moieties of L-D is absent. In some embodiments, each L is independently an amino acid or derivative thereof (e.g., glycine).

In some embodiments, the coupling of the polymer with the plurality of L-D moieties results in the formation of a plurality of amide bonds.

### Pharmaceutical Compositions

In another aspect, the present invention provides a composition, e.g., a pharmaceutical composition, comprising a CDP-therapeutic agent conjugate or particle and a pharmaceutically acceptable carrier or adjuvant. The compositions described herein may also comprise a plurality of CDP-therapeutic agent conjugates. The composition can also comprise a plurality of particles described herein.

In some embodiments, a pharmaceutical composition may include a pharmaceutically acceptable salt of a compound described herein, e.g., a CDP-therapeutic agent conjugate, particle or composition. Pharmaceutically acceptable salts of the compounds described herein include those derived from pharmaceutically acceptable inorganic and organic acids and bases. Examples of suitable acid salts include acetate, adipate, benzoate, benzenesulfonate, butyrate, citrate, digluconate, dodecylsulfate, formate, fumarate, glycolate, hemisulfate, heptanoate, hexanoate, hydrochloride, hydrobromide, hydroiodide, lactate, maleate, malonate, methanesulfonate, 2-naphthalenesulfonate, nicotinate, nitrate, palmoate, phosphate, picrate, pivalate, propionate, salicylate, succinate, sulfate, tartrate, tosylate and undecanoate. Salts derived from appropriate bases include alkali metal (e.g., sodium), alkaline earth metal (e.g., magnesium), ammonium and N-(alkyl)₄⁺ salts. This invention also envisions the quaternization of any basic nitrogen-containing groups of the compounds described herein. Water or oil-soluble or dispersible products may be obtained by such quaternization.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gailate, aipha-tocopherol, and the like; and (3) metal chelating agents, such as citric acid, ethylenediamine tetraacetic acid (EDTA), sorbitol, tartaric acid, phosphoric acid, and the like.

A composition may include a liquid used for suspending a CDP-therapeutic agent conjugate, particle or composition, which may be any liquid solution compatible with the CDP-therapeutic agent conjugate, particle or composition, which is also suitable to be used in pharmaceutical compositions, such as a pharmaceutically acceptable nontoxic liquid. Suitable suspending liquids including but are not limited to suspending liquids selected from the group consisting of water, aqueous sucrose syrups, corn syrups, sorbitol, polyethylene glycol, propylene glycol, and mixtures thereof.

A composition described herein may also include another component, such as an antioxidant, antibacterial, buffer, bulking agent, chelating agent, an inert gas, a tonicity agent and/or a viscosity agent.

In one embodiment, the CDP-therapeutic agent conjugate, particle or composition is provided in lyophilized form and is reconstituted prior to administration to a subject. The lyophilized CDP-therapeutic agent conjugate, particle or composition can be reconstituted by a diluent solution, such as a salt or saline solution, e.g., a sodium chloride solution having a pH between 6 and 9, lactated Ringer's injection solution, or a commercially available diluent, such as PLASMA-LYTE A Injection pH 7.4® (Baxter, Deerfield, IL).

In one embodiment, a lyophilized formulation includes a lyoprotectant or stabilizer to maintain physical and chemical stability by protecting the CDP-therapeutic agent conjugate, particle or composition from damage from crystal formation and the fusion process during freeze-drying. The lyoprotectant or stabilizer can be one or more of polyethylene glycol (PEG), a PEG lipid conjugate (e.g., PEG-ceramide or D-alpha-tocopheryl polyethylene glycol 1000 succinate), poly(vinyl alcohol) (PVA), poly(vinylpyrrolidone) (PVP), polyoxyethylene esters, poloxomers, Tweens, lecithins, saccharides, oligosaccharides, polysaccharides and polyols (e.g., trehalose, mannitol, sorbitol, lactose, sucrose, glucose and dextran), salts and crown ethers. In one embodiment, the lyoprotectant is mannitol.

In some embodiments, the lyophilized CDP-therapeutic agent conjugate, particle or composition is reconstituted with a mixture of equal parts by volume of Dehydrated Alcohol, USP and a nonionic surfactant, such as a polyoxyethylated castor oil surfactant available from GAF Corporation, Mount Olive, N.J., under the trademark, Cremophor EL. In some embodiments, the lyophilized CDP-therapeutic agent conjugate, particle or composition is reconstituted in water for infusion. The lyophilized product and vehicle for reconstitution can be packaged separately in appropriately light-protected vials, e.g., amber or other colored vials. To minimize the amount of surfactant in the reconstituted solution, only a sufficient amount of the vehicle may be provided to form a solution having a concentration of about 2 mg/mL to about 4 mg/mL of the CDP-therapeutic agent conjugate, particle or composition. Once dissolution of the drug is achieved, the resulting solution is further diluted prior to injection with a suitable parenteral diluent. Such diluents are well known to those of ordinary skill in the art. These diluents are generally available in clinical facilities. It is, however, within the scope of the present invention to package the subject CDP-therapeutic agent conjugate, particle or composition with a third vial containing sufficient parenteral diluent to prepare the final concentration for administration. A typical diluent is Lactated Ringer's Injection.

The final dilution of the reconstituted CDP-therapeutic agent conjugate, particle or composition may be carried out with other preparations having similar utility, for example, 5% Dextrose Injection, Lactated Ringer's and Dextrose for Injection (D5W), Sterile Water for Injection, and the like. However, because of its narrow pH range, pH 6.0 to 7.5, Lactated Ringer's Injection is most typical. Per 100 mL, Lactated Ringer's Injection contains Sodium Chloride USP 0.6 g, Sodium Lactate 0.31 g, Potassium chloride USP 0.03 g and Calcium Chloride2H2O USP 0.02 g. The osmolarity is 275 mOsmol/L, which is very close to isotonicity.

The compositions may conveniently be presented in unit dosage form and may be prepared by any methods well known in the art of pharmacy. The dosage form can be, e.g., in a bag, e.g., a bag for infusion. The amount of active ingredient which can be combined with a carrier material to produce a single dosage form will vary depending upon the host being treated, the particular mode of administration. The amount of active ingredient which can be combined with a carrier material to produce a single dosage form will generally be that amount of the compound which produces a therapeutic effect. Generally, out of one hundred percent, this amount will range from about 1 percent to about ninety-nine percent of active ingredient, preferably from about 5 percent to about 70 percent, most preferably from about 10 percent to about 30 percent.

### Routes of Administration

The pharmaceutical compositions described herein may be administered orally, parenterally (e.g., via intravenous, subcutaneous, intracutaneous, intrDascular, intraarticular, intraarterial, intraperitoneal, intrasynovial, intrasternal, intrathecal, intralesional or intracranial injection), topically, mucosally (e.g., rectally or vaginally), nasally, buccally, ophthalmically, via inhalation spray (e.g., delivered via nebulzation, propellant or a dry powder device) or via an implanted reservoir. Typically, the compositions are in the form of injectable or infusible solutions. The preferred mode of administration is, e.g., intravenous, subcutaneous, intraperitoneal, intrDascular.

Pharmaceutical compositions suitable for parenteral administration comprise one or more CDP-therapeutic agent conjugate(s), particle(s) or composition(s) in combination with one or more pharmaceutically acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

Examples of suitable aqueous and nonaqueous carriers which may be employed in the pharmaceutical compositions include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

In some cases, in order to prolong the effect of a drug, it is desirable to slow the absorption of the agent from subcutaneous or intrDascular injection. This may be accomplished by the use of a liquid suspension of crystalline or amorphous material having poor water solubility. The rate of absorption of the CDP-therapeutic agent conjugate, particle or composition then depends upon its rate of dissolution which, in turn, may depend upon crystal size and crystalline form. Alternatively, delayed absorption of a parenterally administered drug form is accomplished by dissolving or suspending the CDP-therapeutic agent conjugate, particle or composition in an oil vehicle.

Pharmaceutical compositions suitable for oral administration may be in the form of capsules, cachets, pills, tablets, gums, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouthwashes and the like, each containing a predetermined amount of an agent as an active ingredient. A compound may also be administered as a bolus, electuary or paste.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered peptide or peptidomimetic moistened with an inert liquid diluent.

Tablets, and other solid dosage forms, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter, or by incorporating sterilizing agents in the form of sterile solid compositions which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions which can be used include polymeric substances and waxes. The active ingredient can also be in micro-encapsulated form, if appropriate, with one or more of the above-described excipients.

Liquid dosage forms for oral administration include pharmaceutically acceptable emulsions, microemulsions, solutions, suspensions, syrups and elixirs. In addition to the CDP-therapeutic agent conjugate, particle or composition, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, coloring, perfuming and preservative agents.

Suspensions, in addition to the CDP-therapeutic agent conjugate, particle or composition may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Pharmaceutical compositions suitable for topical administration are useful when the desired treatment involves areas or organs readily accessible by topical application. For application topically to the skin, the pharmaceutical composition should be formulated with a suitable ointment containing the active components suspended or dissolved in a carrier. Carriers for topical administration of the a particle described herein include, but are not limited to, mineral oil, liquid petroleum, white petroleum, propylene glycol, polyoxyethylene polyoxypropylene compound, emulsifying wax and water. Alternatively, the pharmaceutical composition can be formulated with a suitable lotion or cream containing the active particle suspended or dissolved in a carrier with suitable emulsifying agents. Suitable carriers include, but are not limited to, mineral oil, sorbitan monostearate, polysorbate 60, cetyl esters wax, cetearyl alcohol, 2-octyldodecanol, benzyl alcohol and water. The pharmaceutical compositions described herein may also be topically applied to the lower intestinal tract by rectal suppository formulation or in a suitable enema formulation. Topically-transdermal patches are also included herein.

The pharmaceutical compositions described herein may be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art.

The pharmaceutical compositions described herein may also be administered in the form of suppositories for rectal or vaginal administration. Suppositories may be prepared by mixing one or more CDP-therapeutic agent conjugate, particle or composition described herein with one or more suitable non-irritating excipients which is solid at room temperature, but liquid at body temperature. The composition will therefore melt in the rectum or vaginal cavity and release the CDP-therapeutic agent conjugate, particle or composition. Such materials include, for example, cocoa butter, polyethylene glycol, a suppository wax or a salicylate. Compositions of the present invention which are suitable for vaginal administration also include pessaries, tampons, creams, gels, pastes, foams or spray formulations containing such carriers as are known in the art to be appropriate.

Ophthalmic formulations, eye ointments, powders, solutions and the like, are also contemplated as being within the scope of the invention.

### Dosages and Dosing Regimens

The CDP-therapeutic agent conjugate, particle or composition can be formulated into pharmaceutically acceptable dosage forms by conventional methods known to those of skill in the art.

Actual dosage levels of the active ingredients in the pharmaceutical compositions of this invention may be varied so as to obtain an amount of the active ingredient which is effective to achieve the desired therapeutic response for a particular subject, composition, and mode of administration, without being toxic to the subject.

In one embodiment, the CDP-therapeutic agent conjugate, particle or composition is administered to a subject at a dosage described herein of the therapeutic agent. Administration can be at regular intervals, such as daily, weekly, or every 2, 3, 4, 5 or 6 weeks. The administration can be over a period of from about 10 minutes to about 6 hours, e.g., from about 30 minutes to about 2 hours, from about 45 minutes to 90 minutes, e.g., about 30 minutes, 45 minutes, 1 hour, 2 hours, 3 hours, 4 hours, 5 hours or more. The CDP-therapeutic agent conjugate, particle or composition can be administered, e.g., by intravenous or intraperitoneal administration.

In one embodiment, the CDP-therapeutic agent conjugate, particle or composition is administered as a bolus infusion or intravenous push, e.g., over a period of 15 minutes, 10 minutes, 5 minutes or less. In one embodiment, the CDP-therapeutic agent conjugate, particle or composition is administered in an amount such the desired dose of the agent is administered. Preferably the dose of the CDP-therapeutic agent conjugate, particle or composition is a dose described herein.

In one embodiment, the subject receives 1, 2, 3, up to 10 treatments, or more, or until the disorder or a symptom of the disorder is cured, healed, alleviated, relieved, altered, remedied, ameliorated, palliated, improved or affected. For example, the subject receives an infusion once every 1, 2, 3 or 4 weeks until the disorder or a symptom of the disorder is cured, healed, alleviated, relieved, altered, remedied, ameliorated, palliated, improved or affected. Preferably, the dosing schedule is a dosing schedule described herein.

The CDP-therapeutic agent conjugate, particle or composition can be administered as a first line therapy, e.g., alone or in combination with an additional or second agent or agents as described herein. The CDP-therapeutic agent conjugate, particle or composition can be administered as a second line therapy, e.g., alone or in combination with an additional or second agent or agents as described herein.

### Kits

A CDP-therapeutic agent conjugate, particle or composition described herein may be provided in a kit. The kit includes a CDP-therapeutic agent conjugate, particle or composition described herein and, optionally, a container, a pharmaceutically acceptable carrier and/or informational material. The informational material can be descriptive, instructional, marketing or other material that relates to the methods described herein and/or the use of the CDP-therapeutic agent conjugate, particle or composition for the methods described herein.

The informational material of the kits is not limited in its form. In one embodiment, the informational material can include information about production of the CDP-therapeutic agent conjugate, particle or composition, physical properties of the CDP-therapeutic agent conjugate, particle or composition, concentration, date of expiration, batch or production site information, and so forth. In one embodiment, the informational material relates to methods for administering the CDP-therapeutic agent conjugate, particle or composition, e.g., by a route of administration described herein and/or at a dose and/or dosing schedule described herein.

In one embodiment, the informational material can include instructions to administer a CDP-therapeutic agent conjugate, particle or composition described herein in a suitable manner to perform the methods described herein, e.g., in a suitable dose, dosage form, or mode of administration (e.g., a dose, dosage form, or mode of administration described herein). In another embodiment, the informational material can include instructions to administer a CDP-therapeutic agent conjugate, particle or composition described herein to a suitable subject, e.g., a human, e.g., a human having or at risk for a disorder described herein. In another embodiment, the informational material can include instructions to reconstitute a CDP-therapeutic agent conjugate, particle or composition described herein into a pharmaceutically acceptable composition.

In one embodiment, the kit includes instructions to use the CDP-therapeutic agent conjugate, particle or composition, such as for treatment of a subject. The instructions can include methods for reconstituting or diluting the CDP-therapeutic agent conjugate, particle or composition for use with a particular subject or in combination with a particular second therapeutic agent. The instructions can also include methods for reconstituting or diluting the CDP-therapeutic agent conjugate, particle or composition for use with a particular means of administration, such as by intravenous infusion.

In another embodiment, the kit includes instructions for treating a subject with a particular indication, such as a particular autoimmune disease. For example, the instructions can be for treatment of an autoimmune disease described herein at a dosing schedule described herein.

The informational material of the kits is not limited in its form. In many cases, the informational material, e.g., instructions, is provided in printed matter, e.g., a printed text, drawing, and/or photograph, e.g., a label or printed sheet. However, the informational material can also be provided in other formats, such as Braille, computer readable material, video recording, or audio recording. In another embodiment, the informational material of the kit is contact information, e.g., a physical address, email address, website, or telephone number, where a user of the kit can obtain substantive information about a CDP-therapeutic agent conjugate, particle or composition described herein and/or its use in the methods described herein. The informational material can also be provided in any combination of formats.

In addition to a CDP-therapeutic agent conjugate, particle or composition described herein, the composition of the kit can include other ingredients, such as a surfactant, a lyoprotectant or stabilizer, an antioxidant, an antibacterial agent, a bulking agent, a chelating agent, an inert gas, a tonicity agent and/or a viscosity agent, a solvent or buffer, a stabilizer, a preservative, a flavoring agent (e.g., a bitter antagonist or a sweetener), a fragrance, a dye or coloring agent, for example, to tint or color one or more components in the kit, or other cosmetic ingredient, a pharmaceutically acceptable carrier and/or a second agent for treating a condition or disorder described herein. Alternatively, the other ingredients can be included in the kit, but in different compositions or containers than a CDP-therapeutic agent conjugate, particle or composition described herein. In such embodiments, the kit can include instructions for admixing a CDP-therapeutic agent conjugate, particle or composition described herein and the other ingredients, or for using a CDP-therapeutic agent conjugate, particle or composition described herein together with the other ingredients. For example, the kit can include any of the second therapeutic agents described herein, e.g., for the treatment of lupus or rheumatoid arthritis. In one embodiment, the CDP-therapeutic agent conjugate, particle or composition and the second therapeutic agent are in separate containers, and in another embodiment, the CDP-therapeutic agent conjugate, particle or composition and the second therapeutic agent are packaged in the same container.

In some embodiments, a component of the kit is stored in a sealed vial, e.g., with a rubber or silicone closure (e.g., a polybutadiene or polyisoprene closure). In some embodiments, a component of the kit is stored under inert conditions (e.g., under Nitrogen or another inert gas such as Argon). In some embodiments, a component of the kit is stored under anhydrous conditions (e.g., with a desiccant). In some embodiments, a component of the kit is stored in a light blocking container such as an amber vial.

A CDP-therapeutica agent conjugate, particle or composition described herein can be provided in any form, e.g., liquid, frozen, dried or lyophilized form. It is preferred that a composition including the conjugate, particle or composition, e.g., a composition comprising a particle or particles that include a conjugate described herein be substantially pure and/or sterile. When a CDP-therapeutic agent conjugate, particle or composition described herein is provided in a liquid solution, the liquid solution preferably is an aqueous solution, with a sterile aqueous solution being preferred. In one embodiment, the CDP-therapeutic agent conjugate, particle or composition is provided in lyophilized form and, optionally, a diluent solution is provided for reconstituting the lyophilized agent. The diluent can include for example, a salt or saline solution, e.g., a sodium chloride solution having a pH between 6 and 9, lactated Ringer's injection solution, D5W, or PLASMA-LYTE A Injection pH 7.4® (Baxter, Deerfield, IL).

The kit can include one or more containers for the composition containing a CDP-therapeutic agent conjugate, particle or composition described herein. In some embodiments, the kit contains separate containers, dividers or compartments for the composition and informational material. For example, the composition can be contained in a bottle, vial, IV admixture bag, IV infusion set, piggyback set or syringe, and the informational material can be contained in a plastic sleeve or packet. In other embodiments, the separate elements of the kit are contained within a single, undivided container. For example, the composition is contained in a bottle, vial or syringe that has attached thereto the informational material in the form of a label. In some embodiments, the kit includes a plurality (e.g., a pack) of individual containers, each containing one or more unit dosage forms (e.g., a dosage form described herein) of a CDP-therapeutic agent conjugate, particle or composition described herein. For example, the kit includes a plurality of syringes, ampules, foil packets, or blister packs, each containing a single unit dose of a particle described herein. The containers of the kits can be air tight, waterproof (e.g., impermeable to changes in moisture or evaporation), and/or light-tight.

The kit optionally includes a device suitable for administration of the composition, e.g., a syringe, inhalant, pipette, forceps, measured spoon, dropper (e.g., eye dropper), swab (e.g., a cotton swab or wooden swab), or any such delivery device. In one embodiment, the device is a medical implant device, e.g., packaged for surgical insertion.

### Combination therapy

The CDP-therapeutic agent conjugate, particle or composition may be used in combination with other known therapies. Administered "in combination", as used herein, means that two (or more) different treatments are delivered to the subject during the course of the subject's affliction with the disorder, e.g., the two or more treatments are delivered after the subject has been diagnosed with the disorder and before the disorder has been cured or eliminated or treatment has ceased for other reasons. In some embodiments, the delivery of one treatment is still occurring when the delivery of the second begins, so that there is overlap in terms of administration. This is sometimes referred to herein as "simultaneous" or "concurrent delivery". In other embodiments, the delivery of one treatment ends before the delivery of the other treatment begins. In some embodiments of either case, the treatment is more effective because of combined administration. For example, the second treatment is more effective, e.g., an equivalent effect is seen with less of the second treatment, or the second treatment reduces symptoms to a greater extent, than would be seen if the second treatment were administered in the absence of the first treatment, or the analogous situation is seen with the first treatment. In some embodiments, delivery is such that the reduction in a symptom, or other parameter related to the disorder is greater than what would be observed with one treatment delivered in the absence of the other. The effect of the two treatments can be partially additive, wholly additive, or greater than additive. The delivery can be such that an effect of the first treatment delivered is still detectable when the second is delivered.

The CDP-therapeutic agent conjugate, particle or composition and the at least one additional therapeutic agent can be administered simultaneously, in the same or in separate compositions, or sequentially. For sequential administration, the CDP-therapeutic agent conjugate, particle or composition can be administered first, and the additional agent can be administered second, or the order of administration can be reversed.

### Indications

### Inflammation and Autoimmune Disease

The disclosed CDP-therapeutic agent conjugates, particles, compositions and methods described herein may be used to treat or prevent a disease or disorder associated with an immune response, e.g. an inflammatory disease or an autoimmune disease. For example, a CDP-therapeutic agent conjugate, particle, or composition described herein may be administered prior to the onset of, at, or after the initiation of inflammation.

When used prophylactically, the CDP-therapeutic agent conjugate, particle, or composition is preferably provided in advance of any inflammatory response or symptom. Administration of the CDP-therapeutic agent conjugate, particle, or composition may prevent or attenuate inflammatory responses or symptoms. Exemplary inflammatory conditions include, for example, degenerative joint disease, spondouloarthropathies, osteoporosis, menstrual cramps, cystic fibrosis, irritable bowel syndrome, gastritis, esophagitis, pancreatitis, peritonitis, Alzheimer's disease, shock, conjunctivitis, pancreatis (acute or chronic), multiple organ injury syndrome (e.g., secondary to septicemia or trauma), myocardial infarction, atherosclerosis, stroke, reperfusion injury (e.g., due to cardiopulmonary bypass or kidney dialysis), acute glomerulonephritis, vasculitis, thermal injury (i.e., sunburn), or necrotizing enterocolitis. Exemplary inflammatory conditions of the skin include, for example, eczema, atopic dermatitis, contact dermatitis, urticaria, and dermatosis with acute inflammatory components.

In another embodiment, a CDP-therapeutic agent conjugate, particle, composition or method described herein may be used to treat or prevent allergies and respiratory conditions, including asthma, bronchitis, allergic rhinitis, oxygen toxicity, emphysema, chronic bronchitis, and acute respiratory distress syndrome. The CDP-therapeutic agent conjugate, particle or composition may be used to treat chronic hepatitis infection, including hepatitis B and hepatitis C.

Additionally, a CDP-therapeutic agent conjugate, particle, composition or method described herein may be used to treat autoimmune diseases and/or inflammation associated with autoimmune diseases such as organ-tissue autoimmune diseases (e.g., Raynaud's syndrome), Addison's disease, ankylosing spondylitis, arthritis (e.g., rheumatoid arthritis, osteoarthritis, gout), autoimmune polyglandular disease (also known as autoimmune polyglandular syndrome), Chagas disease, chronic obstructive pulmonary disease (COPD), dermatomyositis, diabetes mellitus type 1, endometriosis, endotoxin shock, Goodpasture's syndrome, Graves' disease, Guillain-Barre syndrome (GBS), Hashiomoto's disease, Hidradenitis suppurativa, Kawasaki disease, IgA nephropathy, Idiopathic thrombocytopenic purpura, inflammatory bowel disease (e.g., Crohn's disease, ulcerative colitis, collagenous colitis, lymphocytic colitis, ischemic colitis, diversion colitis, Behcet's syndrome, infective colitis, indeterminate colitisinterstitial cystitis), lupus (e.g., systemic lupus erythematosus, discoid lupus, drug-induced lupus, neonatal lupus), mixed connective tissue disease, morphea, multiple sclerosis, myasthenia gravis, narcolepsy, neuromyotonia, pemphigus vulgaris, pernicious anemia, psoriasis, psoriatic arthritis, polymyositis, primary biliary cirrhosis, pulmonary fibrosis, relapsing polychondritis, schizophrenia, scleroderma, sepsis, systemic lupus erythematosus, Sjögren's syndrome, Stiff person syndrome, temporal arteritis (also known as giant cell arteritis), autoimmune thyroiditis, transplant rejection, uveitis, vasculitis, vitiligo, or Wegener's granulomatosis.

In an embodiment, the autoimmune disease is arthritis, e.g., rheumatoid arthritis, osteoarthritis, gout; lupus, e.g., systemic lupus erythematosus, discoid lupus, drug-induced lupus, neonatal lupus; inflammatory bowel disease, e.g., Crohn's disease, ulcerative colitis, collagenous colitis, lymphocytic colitis, ischemic colitis, diversion colitis, Behcet's syndrome, infective colitis, indeterminate colitis psoriasis, or multiple sclerosis.

In an embodiment, CDP-therapeutic agent conjugates, particles and compositions can be tested for activity against lupus, for example, in an animal model of lupus. Examples of such models include the flaky skin (fsn) mutant mouse model described in Withington et al. (2002) Autoimmunity 35(3):175-181 and the New Zealand Black x New Zealand White mouse model described in Frese-Schaper et al. (2010) The Journal of Immunology 184:2175-2182. The contents of these references are incorporated herein by this reference.

### Inflammatory and Autoimmune Combination therapy

In certain embodiments, a CDP-therapeutic agent conjugate, particle, or composition described herein may be administered alone or in combination with other compounds useful for treating or preventing inflammation. Exemplary anti-inflammatory agents include, for example, steroids (e.g., Cortisol, cortisone, fludrocortisone, prednisone, 6[alpha]-methylprednisone, triamcinolone, betamethasone or dexamethasone), nonsteroidal anti-inflammatory drugs (NSAIDS (e.g., aspirin, acetaminophen, tolmetin, ibuprofen, mefenamic acid, piroxicam, nabumetone, rofecoxib, celecoxib, etodolac or nimesulide). In another embodiment, the other therapeutic agent is an antibiotic (e.g., vancomycin, penicillin, amoxicillin, ampicillin, cefotaxime, ceftriaxone, cefixime, rifampinmetronidazole, doxycycline or streptomycin). In another embodiment, the other therapeutic agent is a PDE4 inhibitor (e.g., roflumilast or rolipram). In another embodiment, the other therapeutic agent is an antihistamine (e.g., cyclizine, hydroxyzine, promethazine or diphenhydramine). In another embodiment, the other therapeutic agent is an anti-malarial (e.g., artemisinin, artemether, artsunate, chloroquine phosphate, mefloquine hydrochloride, doxycycline hyclate, proguanil hydrochloride, atovaquone or halofantrine). In one embodiment, the other therapeutic agent is drotrecogin alfa.

Further examples of anti-inflammatory agents include, for example, aceclofenac, acemetacin, e-acetamidocaproic acid, acetaminophen, acetaminosalol, acetanilide, acetylsalicylic acid, S-adenosylmethionine, alclofenac, alclometasone, alfentanil, algestone, allylprodine, alminoprofen, aloxiprin, alphaprodine, aluminum bis(acetylsalicylate), amcinonide, amfenac, aminochlorthenoxazin, 3-amino-4-hydroxybutyric acid, 2-amino-4-picoline, aminopropylon, aminopyrine, amixetrine, ammonium salicylate, ampiroxicam, amtolmetin guacil, anileridine, antipyrine, antrafenine, apazone, beclomethasone, bendazac, benorylate, benoxaprofen, benzpiperylon, benzydamine, benzylmorphine, bermoprofen, betamethasone, betamethasone- 17-valerate, bezitramide, [alpha]-bisabolol, bromfenac, p-bromoacetanilide, 5-bromosalicylic acid acetate, bromosaligenin, bucetin, bucloxic acid, bucolome, budesonide, bufexamac, bumadizon, buprenorphine, butacetin, butibufen, butorphanol, carbamazepine, carbiphene, caiprofen, carsalam, chlorobutanol, chloroprednisone, chlorthenoxazin, choline salicylate, cinchophen, cinmetacin, ciramadol, clidanac, clobetasol, clocortolone, clometacin, clonitazene, clonixin, clopirac, cloprednol, clove, codeine, codeine methyl bromide, codeine phosphate, codeine sulfate, cortisone, cortivazol, cropropamide, crotethamide and cyclazocine.

Further examples of anti-inflammatory agents include deflazacort, dehydrotestosterone, desomorphine, desonide, desoximetasone, dexamethasone, dexamethasone-21- isonicotinate, dexoxadrol, dextromoramide, dextropropoxyphene, deoxycorticosterone, dezocine, diampromide, diamorphone, diclofenac, difenamizole, difenpiramide, diflorasone, diflucortolone, diflunisal, difluprednate, dihydrocodeine, dihydrocodeinone enol acetate, dihydromorphine, dihydroxyaluminum acetylsalicylate, dimenoxadol, dimepheptanol, dimethylthiambutene, dioxaphetyl butyrate, dipipanone, diprocetyl, dipyrone, ditazol, droxicam, emorfazone, enfenamic acid, enoxolone, epirizole, eptazocine, etersalate, ethenzamide, ethoheptazine, ethoxazene, ethylmethylthiambutene, ethylmorphine, etodolac, etofenamate, etonitazene, eugenol, felbinac, fenbufen, fenclozic acid, fendosal, fenoprofen, fentanyl, fentiazac, fepradinol, feprazone, floctafenine, fluazacort, flucloronide, flufenamic acid, flumethasone, flunisolide, flunixin, flunoxaprofen, fluocinolone acetonide, fluocinonide, fluocinolone acetonide, fluocortin butyl, fluocoitolone, fluoresone, fluorometholone, fluperolone, flupirtine, fluprednidene, fluprednisolone, fluproquazone, flurandrenolide, flurbiprofen, fluticasone, formocortal and fosfosal.

Further examples of anti-inflammatory agents include gentisic acid, glafenine, glucametacin, glycol salicylate, guaiazulene, halcinonide, halobetasol, halometasone, haloprednone, heroin, hydrocodone, hydro cortamate, hydrocortisone, hydrocortisone acetate, hydrocortisone succinate, hydrocortisone hemisuccinate, hydrocortisone 21-lysinate, hydrocortisone cypionate, hydromorphone, hydroxypethidine, ibufenac, ibuprofen, ibuproxam, imidazole salicylate, indomethacin, indoprofen, isofezolac, isoflupredone, isoflupredone acetate, isoladol, isomethadone, isonixin, isoxepac, isoxicam, ketobemidone, ketoprofen, ketorolac, p- lactophenetide, lefetamine, levallorphan, levorphanol, levophenacyl-morphan, lofentanil, lonazolac, lornoxicam, loxoprofen, lysine acetylsalicylate, mazipredone, meclofenamic acid, medrysone, mefenamic acid, meloxicam, meperidine, meprednisone, meptazinol, mesalamine, metazocine, methadone, methotrimeprazine, methylprednisolone, methylprednisolone acetate, methylprednisolone sodium succinate, methylprednisolone suleptnate, metiazinic acid, metofoline, metopon, mofebutazone, mofezolac, mometasone, morazone, morphine, morphine hydrochloride, morphine sulfate, morpholine salicylate and myrophine.

Further examples of anti-inflammatory agents include nabumetone, nalbuphine, nalorphine, 1-naphthyl salicylate, naproxen, narceine, nefopam, nicomorphine, nifenazone, niflumic acid, nimesulide, 5'-nitro-2'-propoxyacetanilide,norlevorphanol, normethadone, normorphine, norpipanone, olsalazine, opium, oxaceprol, oxametacine, oxaprozin, oxycodone, oxymorphone, oxyphenbutazone, papaveretum, paramethasone, paranyline, parsalmide, pentazocine, perisoxal, phenacetin, phenadoxone, phenazocine, phenazopyridine hydrochloride, phenocoll, phenoperidine, phenopyrazone, phenomorphan, phenyl acetylsalicylate, phenylbutazone, phenyl salicylate, phenyramidol, piketoprofen, piminodine, pipebuzone, piperylone, pirazolac, piritramide, piroxicam, pirprofen, pranoprofen, prednicarbate, prednisolone, prednisone, prednival, prednylidene, proglumetacin, proheptazine, promedol, propacetamol, properidine, propiram, propoxyphene, propyphenazone, proquazone, protizinic acid, proxazole, ramifenazone, remifentanil, rimazolium metilsulfate, salacetamide, salicin, salicylamide, salicylamide o-acetic acid, salicylic acid, salicylsulfuric acid, salsalate, salverine, simetride, sufentanil, sulfasalazine, sulindac, superoxide dismutase, suprofen, suxibuzone, talniflumate, tenidap, tenoxicam, terofenamate, tetrandrine, thiazolinobutazone, tiaprofenic acid, tiaramide, tilidine, tinoridine, tixocortol, tolfenamic acid, tolmetin, tramadol, triamcinolone, triamcinolone acetonide, tropesin, viminol, xenbucin, ximoprofen, zaltoprofen and zomepirac.

In one embodiment, a CDP-therapeutic agent conjugate, particle or composition described herein may be administered with a selective COX-2 inhibitor for treating or preventing inflammation. Exemplary selective COX-2 inhibitors include, for example, deracoxib, parecoxib, celecoxib, valdecoxib, rofecoxib, etoricoxib, and lumiracoxib.

### Cancer

The disclosed CDP-therapeutic agent conjugates, particles, compositions and methods described herein are useful in treating proliferative disorders, e.g., treating a tumor and metastases, e.g., a tumor or metastases of a cancer described herein.

The methods described herein can be used to treat a solid tumor, a soft tissue tumor or a liquid tumor. Exemplary solid tumors include malignancies (e.g., sarcomas and carcinomas (e.g., adenocarcinoma or squamous cell carcinoma)) of the various organ systems, such as those of brain, lung, breast, lymphoid, gastrointestinal (e.g., colon), and genitourinary (e.g., renal, urothelial, or testicular tumors) tracts, pharynx, prostate, and ovary. Exemplary adenocarcinomas include colorectal cancers, renal-cell carcinoma, liver cancer, non-small cell carcinoma of the lung, and cancer of the small intestine. The disclosed methods are also useful in evaluating or treating soft tissue tumors such as those of the tendons, muscles or fat, and liquid tumors.

The methods described herein can be used with any cancer, for example those described by the National Cancer Institute. The cancer can be a carcinoma, a sarcoma, a myeloma, a leukemia, a lymphoma or a mixed type. Exemplary cancers described by the National Cancer Institute include:
Digestive/gastrointestinal cancers such as anal cancer; bile duct cancer; extrahepatic bile duct cancer; appendix cancer; carcinoid tumor, gastrointestinal cancer; colon cancer; colorectal cancer including childhood colorectal cancer; esophageal cancer including childhood esophageal cancer; gallbladder cancer; gastric (stomach) cancer including childhood gastric (stomach) cancer; hepatocellular (liver) cancer including adult (primary) hepatocellular (liver) cancer and childhood (primary) hepatocellular (liver) cancer; pancreatic cancer including childhood pancreatic cancer; sarcoma, rhabdomyosarcoma; islet cell pancreatic cancer; rectal cancer; and small intestine cancer;
Endocrine cancers such as islet cell carcinoma (endocrine pancreas); adrenocortical carcinoma including childhood adrenocortical carcinoma; gastrointestinal carcinoid tumor; parathyroid cancer; pheochromocytoma; pituitary tumor; thyroid cancer including childhood thyroid cancer; childhood multiple endocrine neoplasia syndrome; and childhood carcinoid tumor;
Eye cancers such as intraocular melanoma; and retinoblastoma;
Musculoskeletal cancers such as Ewing's family of tumors; osteosarcoma/malignant fibrous histiocytoma of the bone; childhood rhabdomyosarcoma; soft tissue sarcoma including adult and childhood soft tissue sarcoma; clear cell sarcoma of tendon sheaths; and uterine sarcoma;
Breast cancer such as breast cancer including childhood and male breast cancer and pregnancy;
Neurologic cancers such as childhood brain stem glioma; brain tumor; childhood cerebellar astrocytoma; childhood cerebral astrocytoma/malignant glioma; childhood ependymoma; childhood medulloblastoma; childhood pineal and supratentorial primitive neuroectodermal tumors; childhood visual pathway and hypothalamic glioma; other childhood brain cancers; adrenocortical carcinoma; central nervous system lymphoma, primary; childhood cerebellar astrocytoma; neuroblastoma; craniopharyngioma; spinal cord tumors; central nervous system atypical teratoid/rhabdoid tumor; central nervous system embryonal tumors; and childhood supratentorial primitive neuroectodermal tumors and pituitary tumor;
Genitourinary cancers such as bladder cancer including childhood bladder cancer; renal cell (kidney) cancer; ovarian cancer including childhood ovarian cancer; ovarian epithelial cancer; ovarian low malignant potential tumor; penile cancer; prostate cancer; renal cell cancer including childhood renal cell cancer; renal pelvis and ureter, transitional cell cancer; testicular cancer; urethral cancer; vaginal cancer; vulvar cancer; cervical cancer; Wilms tumor and other childhood kidney tumors; endometrial cancer; and gestational trophoblastic tumor;
Germ cell cancers such as childhood extracranial germ cell tumor; extragonadal germ cell tumor; ovarian germ cell tumor; and testicular cancer;
Head and neck cancers such as lip and oral cavity cancer; oral cancer including childhood oral cancer; hypopharyngeal cancer; laryngeal cancer including childhood laryngeal cancer; metastatic squamous neck cancer with occult primary; mouth cancer; nasal cavity and paranasal sinus cancer; nasopharyngeal cancer including childhood nasopharyngeal cancer; oropharyngeal cancer; parathyroid cancer; pharyngeal cancer; salivary gland cancer including childhood salivary gland cancer; throat cancer; and thyroid cancer;
Hematologic/blood cell cancers such as a leukemia (e.g., acute lymphoblastic leukemia including adult and childhood acute lymphoblastic leukemia; acute myeloid leukemia including adult and childhood acute myeloid leukemia; chronic lymphocytic leukemia; chronic myelogenous leukemia; and hairy cell leukemia); a lymphoma (e.g., AIDS-related lymphoma; cutaneous T-cell lymphoma; Hodgkin's lymphoma including adult and childhood Hodgkin's lymphoma and Hodgkin's lymphoma during pregnancy; non-Hodgkin's lymphoma including adult and childhood non- Hodgkin's lymphoma and non-Hodgkin's lymphoma during pregnancy; mycosis fungoides; Sezary syndrome; Waldenstrom's macroglobulinemia; and primary central nervous system lymphoma); and other hematologic cancers (e.g., chronic myeloproliferative disorders; multiple myeloma/plasma cell neoplasm; myelodysplastic syndromes; and myelodysplastic/myeloproliferative disorders);
Lung cancer such as non-small cell lung cancer; and small cell lung cancer;
Respiratory cancers such as malignant mesothelioma, adult; malignant mesothelioma, childhood; malignant thymoma; childhood thymoma; thymic carcinoma; bronchial adenomas/carcinoids including childhood bronchial adenomas/carcinoids; pleuropulmonary blastoma; non-small cell lung cancer; and small cell lung cancer;
Skin cancers such as Kaposi's sarcoma; Merkel cell carcinoma; melanoma; and childhood skin cancer;
AIDS-related malignancies;
Other childhood cancers, unusual cancers of childhood and cancers of unknown primary site;
and metastases of the aforementioned cancers can also be treated or prevented in accordance with the methods described herein.

The CDP-therapeutic agent conjugates, particles, compositions and methods described herein are particularly suited to treat accelerated or metastatic cancers of the bladder cancer, pancreatic cancer, prostate cancer, renal cancer, non-small cell lung cancer, ovarian cancer, melanoma, colorectal cancer, and breast cancer.

In one embodiment, a method is provided for a combination treatment of a cancer, such as by treatment with a CDP-therapeutic agent conjugate, particle, or composition and a second therapeutic agent. Various combinations are described herein. The combination can reduce the development of tumors, reduces tumor burden, or produce tumor regression in a mammalian host.

### Cancer combination therapy

The CDP-therapeutic agent conjugates, particles, compositions and methods described herein may be used in combination with other known therapies. Administered "in combination", as used herein, means that two (or more) different treatments are delivered to the subject during the course of the subject's affliction with the disorder, e.g., the two or more treatments are delivered after the subject has been diagnosed with the disorder and before the disorder has been cured or eliminated or treatment has ceased for other reasons. In some embodiments, the delivery of one treatment is still occurring when the delivery of the second begins, so that there is overlap in terms of administration. This is sometimes referred to herein as "simultaneous" or "concurrent delivery". In other embodiments, the delivery of one treatment ends before the delivery of the other treatment begins. In some embodiments of either case, the treatment is more effective because of combined administration. For example, the second treatment is more effective, e.g., an equivalent effect is seen with less of the second treatment, or the second treatment reduces symptoms to a greater extent, than would be seen if the second treatment were administered in the absence of the first treatment, or the analogous situation is seen with the first treatment. In some embodiments, delivery is such that the reduction in a symptom, or other parameter related to the disorder is greater than what would be observed with one treatment delivered in the absence of the other. The effect of the two treatments can be partially additive, wholly additive, or greater than additive. The delivery can be such that an effect of the first treatment delivered is still detectable when the second is delivered.

The CDP-therapeutic agent conjugate, particle, or composition and the at least one additional therapeutic agent can be administered simultaneously, in the same or in separate compositions, or sequentially. For sequential administration, the CDP-therapeutic agent conjugate, particle, or composition can be administered first, and the additional agent can be administered second, or the order of administration can be reversed.

In some embodiments, the CDP-therapeutic agent conjugate, particle, or composition is administered in combination with other therapeutic treatment modalities, including surgery, radiation, cryosurgery, and/or thermotherapy. Such combination therapies may advantageously utilize lower dosages of the administered agent and/or other chemotherapeutic agent, thus avoiding possible toxicities or complications associated with the various monotherapies. The phrase "radiation" includes, but is not limited to, external-beam therapy which involves three dimensional, conformal radiation therapy where the field of radiation is designed to conform to the volume of tissue treated; interstitial-radiation therapy where seeds of radioactive compounds are implanted using ultrasound guidance; and a combination of external-beam therapy and interstitial-radiation therapy.

In some embodiments, the CDP-therapeutic agent conjugate, particle, or composition is administered with at least one additional therapeutic agent, such as a chemotherapeutic agent. In certain embodiments, the CDP-therapeutic agent conjugate, particle, or composition is administered in combination with one or more additional chemotherapeutic agent, e.g., with one or more chemotherapeutic agents described herein.

When employing the methods or compositions, other agents used in the modulation of tumor growth or metastasis in a clinical setting, such as antiemetics, can also be administered with CDP-therapeutic agent conjugates, particles, or compositions as desired.

When formulating the pharmaceutical compositions featured in the invention the clinician may utilize preferred dosages as warranted by the condition of the subject being treated. For example, in one embodiment, a CDP-therapeutic agent conjugate, particle, or composition may be administered at a dosing schedule described herein, e.g., once every one, two three four, five, or six weeks.

Also, in general, a CDP-therapeutic agent conjugate, particle, or composition, and an additional chemotherapeutic agent(s) do not have to be administered in the same pharmaceutical composition, and may, because of different physical and chemical characteristics, have to be administered by different routes. For example, the CDP-therapeutic agent conjugate, particle, or composition may be administered intravenously while the chemotherapeutic agent(s) may be administered orally. The determination of the mode of administration and the advisability of administration, where possible, in the same pharmaceutical composition, is well within the knowledge of the skilled clinician. The initial administration can be made according to established protocols known in the ait, and then, based upon the observed effects, the dosage, modes of administration and times of administration can be modified by the skilled clinician.

In one embodiment, a CDP-therapeutic agent conjugate, particle, or composition is administered once every three weeks and an additional therapeutic agent (or additional therapeutic agents) may also be administered every three weeks for as long as treatment is required. In another embodiment, the CDP-therapeutic agent conjugate, particle, or composition is administered once every two weeks in combination with one or more additional chemotherapeutic agent that is administered orally.

The actual dosage of the CDP-therapeutic agent conjugate, particle, or composition and/or any additional chemotherapeutic agent employed may be varied depending upon the requirements of the subject and the severity of the condition being treated. Determination of the proper dosage for a particular situation is within the skill of the art. Generally, treatment is initiated with smaller dosages which are less than the optimum dose of the compound. Thereafter, the dosage is increased by small amounts until the optimum effect under the circumstances is reached.

The disclosure also encompasses a method for the synergistic treatment of cancer wherein a CDP-therapeutic agent conjugate, particle, or composition is administered in combination with an additional chemotherapeutic agent or agents.

The particular choice of polymer conjugate and anti-proliferative cytotoxic agent(s) or radiation will depend upon the diagnosis of the attending physicians and their judgment of the condition of the subject and the appropriate treatment protocol.

If the CDP-therapeutic agent conjugate, particle, or composition and the chemotherapeutic agent(s) and/or radiation are not administered simultaneously or essentially simultaneously, then the initial order of administration of the CDP-therapeutic agent conjugate, particle, or composition, and the chemotherapeutic agent(s) and/or radiation, may be varied. Thus, for example, the CDP-therapeutic agent conjugate, particle, or composition may be administered first followed by the administration of the chemotherapeutic agent(s) and/or radiation; or the chemotherapeutic agent(s) and/or radiation may be administered first followed by the administration of the CDP-therapeutic agent conjugate, particle, or composition. This alternate administration may be repeated during a single treatment protocol. The determination of the order of administration, and the number of repetitions of administration of each therapeutic agent during a treatment protocol, is well within the knowledge of the skilled physician after evaluation of the disease being treated and the condition of the subject.

Thus, in accordance with experience and knowledge, the practicing physician can modify each protocol for the administration of a component (CDP-therapeutic agent conjugate, particle, composition, anti-neoplastic agent(s), or radiation) of the treatment according to the individual subject's needs, as the treatment proceeds.

The attending clinician, in judging whether treatment is effective at the dosage administered, will consider the general well-being of the subject as well as more definite signs such as relief of disease-related symptoms, inhibition of tumor growth, actual shrinkage of the tumor, or inhibition of metastasis. Size of the tumor can be measured by standard methods such as radiological studies, e.g., CAT or MRI scan, and successive measurements can be used to judge whether or not growth of the tumor has been retarded or even reversed. Relief of disease-related symptoms such as pain, and improvement in overall condition can also be used to help judge effectiveness of treatment.

Unless otherwise defined, all technical and scientific terms used herein have the same meaning as commonly understood by one of ordinary skill in the art to which this invention belongs. All publications, patent applications, patents, and other references mentioned herein are incorporated by reference in their entirety. In case of conflict, the present specification, including definitions, will control. In addition, the materials, methods, and examples are illustrative only and not intended to be limiting.

### EXAMPLES

### Example 1: Synthesis of 6^{A},6^{D}-Bis-(2-amino-2-carboxylethylthio)-6^{A},6^{D}-dideoxy-β-cyclodextrin, 4 (CD-BisCys)

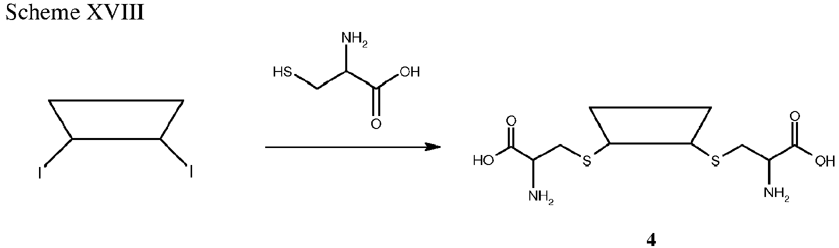

167 mL of 0.1 M sodium carbonate buffer were degassed for 45 minutes in a 500 mL 2-neck round bottom flask equipped with a magnetic stir bar, a condenser and septum. To this solution were added 1.96 g (16.2 mmol) of L-cysteine and 10.0 g (73.8 mmol) of diiodo, deoxy-β-cyclodextrin **2.** The resulting suspension was heated at a reflux temperature for 4.5 h until the solution turned clear (colorless). The solution was then cooled to room temperature and acidified to pH 3 using IN HCl. The product was precipitated by slow addition of acetone (3 times weight ratio of the solution). This afforded 9.0 g crude material containing CD-biscysteine (90.0%), unreacted cyclodextrin, CD-mono-cysteine and cystine. The resulting solid was subjected to anionic exchange column chromatography (SuperQ650M, Tosoh Bioscience) using a gradient elution of 0-0.4M ammonium bicarbonate. All fractions were analyzed by HPLC. The desired fractions were combined and the solvent was reduced to 100 mL under vacuum. The final product was either precipitated by adding acetone or by adding methanol (3 times weight ratio of the solution). **4** was obtained in 60-90% yield. ¹H NMR (D₂O) δ 5.08 (m, 7H, CD-2-CH), 3.79-3.94 (m, 30H, CD-3,4-CH, CD-CH₂, Cys-CH), 3.49-3.62 (m, 14H, CD-5, 6-CH), 2.92-3.30(m, 4H, Cys-CH₂). ¹³C NMR (D₂O) δ 172.3, 101.9, 83.9, 81.6, 81.5, 73.3, 72.2, 72.0, 60.7, 54.0, 34.0, 30.6. ESI/MS (m/z): 1342 [M]⁺, 1364 [M + Na]⁺. Purity of **4** was confirmed by HPLC.

### Example 2: Synthesis of Gly-CPT (Structure 11) (Greenwald et al., Bioorg. Med. Chem., 1998, 6, 551-562)

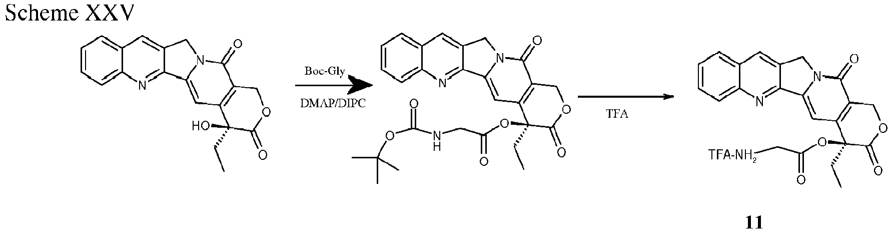

*t*-Boc-glycine (0.9 g, 4.7 mmol) was dissolved in 350 mL of anhydrous methylene chloride at room temperature, and to this solution were added DIPC (0.75 mL, 4.7 mmol), DMAP (382 mg, 3.13 mmol) and camptothecin (0.55g, 1.57 mmol) at 0 °C. The reaction mixture was allowed to warm to room temperature and left for 16 h. The solution was washed with 0.1 N HCl, dried and evaporated under reduced pressure to yield a white solid, which was recrystallized from methanol to give camptothecin-20-ester of *t*-Boc-glycine: ¹H NMR(DMSO-*d*₆) 7.5-8.8 (m), 7.3 (s),5.5 (s), 5.3 (s), 4 (m), 2.1 (m), 1.6 (s), 1.3 (d), 0.9 (t). Camptothecin-20-ester of *t*-Boc-glycine (0.595 g, 1.06 mmol) was dissolved in a mixture of methylene chloride (7.5 mL) and TFA (7.5 mL) and stirred at room temperature for 1 h. Solvent was removed and the residue was recrystallized from methylene chloride and ether to give 0.45 g of **11.** ¹H NMR (DMSO-*d*₆) δ7.7-8.5 (m); 7.2 (s), 5.6 (s), 5.4 (s), 4.4 (m), 2.2 (m), 1.6 (d), 1.0 (t), ¹³C NMR (DMSO-*d*₆) δ168.6, 166.6, 156.5, 152.2, 147.9, 146.2, 144.3, 131.9, 130.6, 129.7, 128.8, 128.6, 128.0, 127.8, 119.0, 95.0, 77.6, 66.6, 50.5, 47.9, 30.2, 15.9, 7.9. ESI/MS (m/z) expected 405; Found 406 (M+H).

### Example 3: Synthesis and Characterization of CD-BisCys-Pes3400 Copolymers 36 and their CPT Conjugates 37.

### A. Synthesis and Characterization of CD-BisCys-Peg3400 Copolymers 36

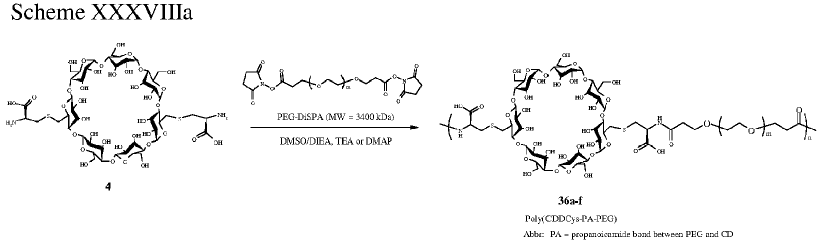

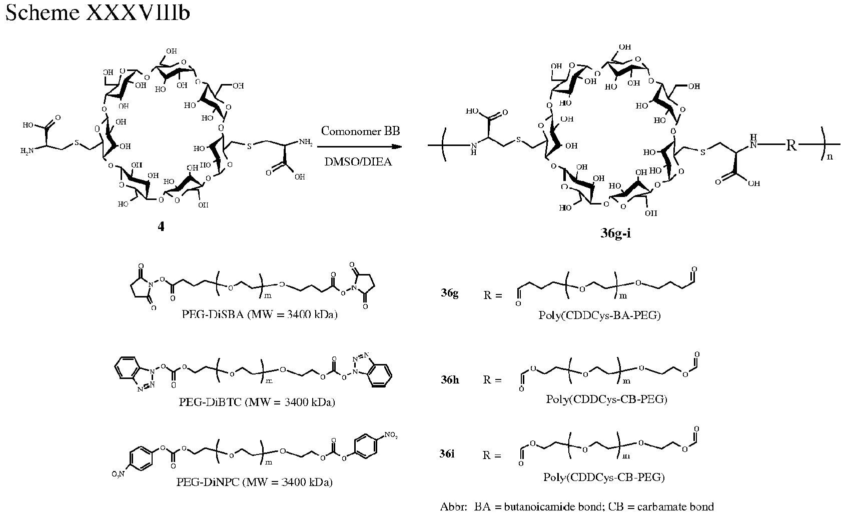

**Synthesis of Poly(CDDCys-PA-PEG), 36a 4** (after precipitation with acetone, 63 mg, 0.047 mmol) and PEG-DiSPA (MW 3400, 160 mg, 0.047 mmol) were dried under vacuum for 8 hours. Anhydrous DMSO (1.26 mL) was added to the mixture under argon. After 10 minutes of stirring, anhydrous diisopropylethylamine (DIEA, 19 µL, 2.3 eq.) was added under argon. The reaction mixture was stirred under argon for 120 h. The polymer containing solution was dialyzed using a 10,000 MWCO membrane (Spectra/Por 7) against water for 48 h and lyophilized to yield 196 mg **36a.** *M_{w}* = 57400 Da, *Mₙ* = 41700 Da, *M_{w}*/*Mₙ* = 1.38. ¹H NMR (D₂O) δ 5.08 (m, CD-2-H), 4.27 (m, Cys-CH), 2.72-3.76 (m, CD-3,4,5,6-CH, CD-CH₂, PEG-CH₂), 2.44 (m, Cys-CH₂).

Synthesis of other poly(CDDCys-PA-PEG) (**36b-f**), Poly(CDDCys-BA-PEG) (**36g**) Poly(CDDCys-CB-PEG) (**36h-i**) were achieved under polymerization condition similar to that of **36a.** Details for the polymerization conditions, monomer selection, polymer molecular weight, polydispersity and yields are listed in Table 2. **36g:** ¹H NMR (D₂O) δ 5.10 (m, CD-2-H), 4.25-4.37 (m, Cys-CH), 2.72-3.86 (m, CD-3,4,5,6-CH, CD-CH₂, PEG-CH₂), 2.21 (m, Cys-CH₂). **36h-i:** ¹H NMR (D₂O) δ 5.05 (m, CD-2-H), 4.56 (m, Cys-CH), 2.70-3.93 (m, CD-3,4,5,6-CH, CD-CH₂, PEG-CH₂), 2.38 (m, - OC*H*₂CH₂CH₂C(O)-NH-), 2.34 (m, Cys-CH₂), 1.90 (m, -OCH₂C*H*₂CH₂C(O)-NH-).

Addition of a non-nucleophilic organic base (such as DIEA) was essential for this polymerization as no viscosity changes of the polymerization solutions were observed after 48 hours if no base was added. When 2.3 eq. of DIEA were added, the viscosity of the polymerization solution increased dramatically after 4-6 hours of reaction. DIEA deprotonates the amino groups of **4** to render them more nucleophilic for coupling with PEG-DiSPA. There were essentially no differences in the polymerizations if other bases, such as TEA or DMAP, were used (**36b-c,** Table 2). Polymerization using **4** recovered by the two different precipitation methods (acetone and methanol) produced polymers with different MWs. **4** that was purified by the methanol-precipitation method (contains no free cystine) gave higher MW polymer (**36d-e**) as compared to the less pure **4** that was obtained from the acetone-precipitation method (**36a**). Polymerization of **4** with PEG-DiSPA typically produced polymer yields greater than 90%.

**4** was polymerized with other activated monomers such as PEG-DiSBA, PEG-DiBTC, and PEG-DiNPC. Reaction of **4** with PEG-DiSBA gave polymer **36g** with similar linkages as **36a-f** (amide bond, but one more -CH₂ group than **36a-f** at the linker) with *M_{w}* over 100,000 Da, while reaction of **4** with PEG-DiBTC and PEG-DiNPC generated polymers **36h** and **36i,** respectively, with connecting carbamate moiety and *M_{w}*'s over 50,000 Da (Table 2).

**Table 2. Polymerization of 4 with difunctionalized PEG**

| CDP | PEG Comonomer | Base | Polymerization time (h) | *M_{w}* (kDa) | *Mₙ* (kDa) | *M_{w}*/*Mₙ* | Yield (%) |
|---|---|---|---|---|---|---|---|
| **36a^{a}** | PEG-DiSPA | DIEA | 120 | 57.4 | 41.7 | 1.38 | 90 |
| **36b^{a}** | PEG-DiSPA | DMAP | 120 | 54.2 | 38.1 | 1.42 | 91 |
| **36c^{a}** | PEG-DiSPA | TEA | 120 | 57.4 | 42.6 | 1.35 | 91 |
| **36d^{b}** | PEG-DiSPA | DIEA | 120 | 93.6 | 58.0 | 1.48 | 96 |
| **36e^{b}** | PEG-DiSPA | DIEA | 144 | 97.3 | 58.0 | 1.67 | 94 |
| **36f^{b}** | PEG-DiSPA | DIEA | 2 | 35.3 | 25.6 | 1.38 | 95 |
| **36g** | PEG-DiSBA | DIEA | 120 | 114.7 | 77.9 | 1.47 | 96 |
| **36h** | PEG-DiBTC | DIEA | 120 | 67.6 | 39.4 | 1.47 | 95 |
| **36i** | PEG-DiNPC | DIEA | 120 | 86.5 | 57.2 | 1.51 | 96 |

| | | | | | | | |
|---|---|---|---|---|---|---|---|
| ^{a} **4** was washed with acetone before polymerization. ^{b} **4** was washed with methanol before polymerization. | | | | | | | |

Polymers **36a-i** are highly soluble in aqueous solution. They can be easily dissolved in water or phosphate buffered saline (PBS) solution at concentrations of at least 200 mg/mL. Solubility of these polymers in aqueous solution at concentrations higher than 200 mg/mL was not attempted due to the high viscosity. These polymers were also soluble in DMF, DMSO and methanol, slightly soluble in CH₃CN and CHCl₃, but insoluble in THF and ethyl ether.

**Molecular Weight Control of CD Polymers 4** (after precipitation with methanol) (56.2 mg, 0.0419 mmol) and PEG-DiSPA (147 mg, 0.0419 mmol) were dried under vacuum for 4-8 hours. To the mixture was added dry DMSO (1.1 mL) under argon. After 10 minutes stirring, DIEA (16 *µ*L, 2.2 eq) was added under argon. A portion of polymerization solution (150 *µ*L) was removed and precipitated with ether at selected times (2 h, 18 h, 43 h, 70 h, 168 h and 288 h). MWs of the precipitated polymers were determined as described above.

### B. Synthesis of Poly(CDDCys-PA-PEG)-CPTConjugates (HGGG6, LGGG10, HG6, HGGG10).

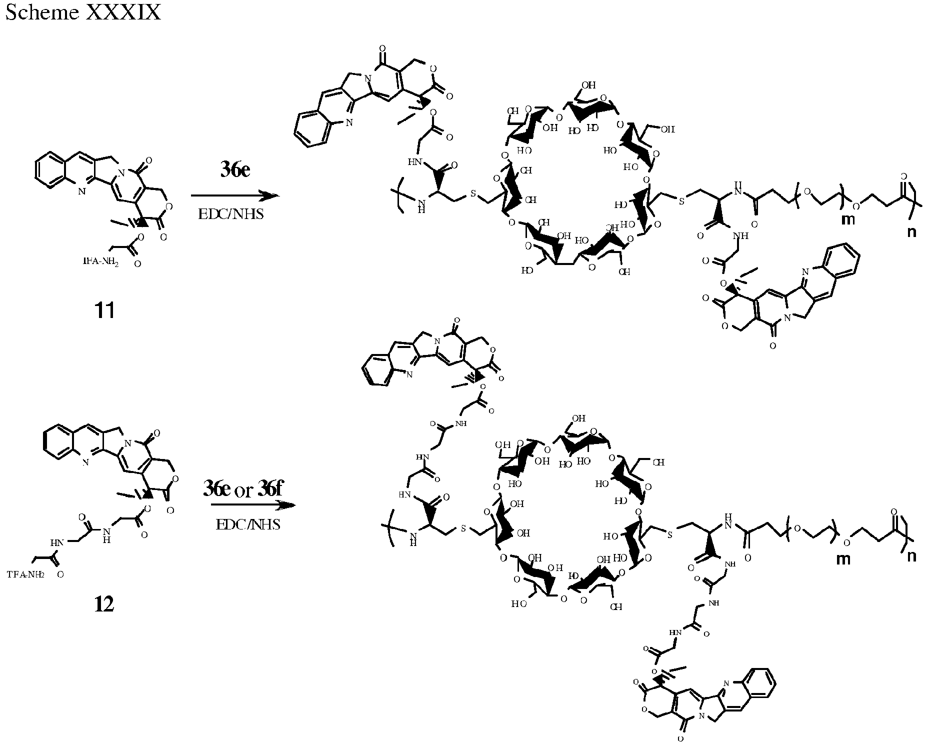

Synthesis of Poly(CDDCys-PA-PEG)-GlyGlyGly-CPT (HGGG6) 36e (1.37 g, 0.30 mmol of repeat unit) was dissolved in dry DMSO (136 mL). The mixture was stirred for 10 minutes. 12 (419 mg, 0.712 mmol, 2.36 eq), DIEA (0.092 mL, 0.712 mmol, 2.36 eq), EDC (172 mg, 0.903 mmol, 3 eq), and NHS (76 mg, 0.662 mmol, 2.2 eq) were added to the polymer solution and stirred for ca. 15 hours. The polymer was precipitated with ethyl ether (1 L). The ether was poured out and the precipitate was washed with CH₃CN (3 × 100 mL). The precipitate was dissolved in water 600 mL. Some insoluble solid was filtered through 0.2 *µ*m filters. The solution was dialyzed using 25,000 MWCO membrane (Spectra/Por 7) for 10 h at 10-15 °C in DI water. Dialysis water was changed every 60 minutes. The polymer-drug conjugate solution was sterilized by passing it through 0.2 µM filters. The solution was lyophilized to yield a yellow solid **HGGG6** (1.42 g, 85% yield).

Synthesis of Poly(CDDCys-PA-PEG)-GlyGlyGly-CPT (**LGGG10**) Conjugation of **12** to **36f** was performed in a manner similar to that used to produce **HGGG6** except that this conjugate was dialyzed with 10,000 MWCO membrane (Spectra/Por 7) instead of with 25,000 MWCO membrane. The yield of **LGGG10** was 83%. Synthesis of Poly(CDDCys-PA-PEG)-Gly-CPT (**HG6**) Conjugation of **11** to **36e** was performed in a manner similar to that used to produce **HGGG6.** The yield of **HG6** was 83%.

Synthesis of Poly(CDDCys-PA-PEG)-GlyGlyGly-CPT (**HGGG10**) **36e** (1.5 g, 0.33 mmol of repeat unit) was dissolved in dry DMSO (150 mL). The mixture was stirred for 10 minutes. **12** (941 mg, 1.49 mmol, 4.5 eq), DIEA (0.258 mL, 1.49 mmol, 4.5 eq), EDC (283 mg, 1.49 mmol, 4.5 eq), and NHS (113 mg, 0.99 mmol, 3 eq) was added to the polymer solution and stirred for ca. 24 hours. Another portion of EDC (142 mg, 0.75 mmol, 2.3 eq) and NHS (56 mg, 0.5 mmol, 1.5 eq) were added to the conjugation solution. The polymer was stirred for an additional 22 hours. The workup procedure was the same as that for the synthesis of **HGGG6.** The yield of **HGGG10** was 77%.

**Determination of wt% CPT on the Conjugates** Stock solutions of **HGGG6, LGGG10, HG6** and **HGGG10** were prepared at a concentration of 10 mg/mL in DMSO. An aliquot of corresponding stock solution was diluted to 100 µg/mL using 1 N NaOH. CPT was completely hydrolyzed in this basic solution and transformed to its carboxylate form within 2 h at room temperature. An aliquot of this solution was diluted to 10 µg/mL using 8.5% H₃PO₄, and the CPT carboxylate form was transformed to its lactone form. 30 *µ*L of this solution was injected into the HPLC. The peak area from the CPT lactone form was integrated and compared to a standard curve.

**11** and **12** were conjugated to **36e** or **36f** (Table 2) using conventional coupling methods. Due to the instability of the ester linker of **11** and **12** in aqueous solution, the conjugation was conducted in anhydrous DMSO under argon. An organic base was required to deprotonate the TFA salts of **11** and **12** to facilitate the coupling. For polymer conjugation with **12,** the weight percent (wt%) drug loading was around 6-10%. The theoretical maximum drug loading is around 13% using PEG with MW of 3400 Da; maximum values can be increased by decreasing the MW of the PEG segments. Solubilities of all conjugates in water or PBS were more than 200 mg/mL (equivalent to a 12-20 mg CPT/mL for 6-10 wt% drug loading, respectively). Details for the **HGGG6, LGGG10, HG6,** and **HGGG10** are summarized in Table 3.

**Table 3. Properties of polymer-CPT conjugates.**

| Conjugate^{a} | *M_{w}* of parent polymer (x 10⁻³) | *M_{w}*/*Mₙ*^{b} | Linker | CPT (wt%) |
|---|---|---|---|---|
| **HGGG6** | 97 | 1.7 | triglycine | 6.1 |
| **LGGG10** | 35 | 1.6 | triglycine | 10.2 |
| **HG6** | 97 | 1.7 | glycine | 6.8 |
| **HGGG10** | 97 | 1.7 | triglycine | 9.6 |

| | | | | |
|---|---|---|---|---|
| ^{a}Abbreviations: **H** = High *M_{w}* polymer (97 kDa), **L** = Low *M_{w}* polymer (35 kDa), **GGG** = triglycine linker, **G** = glycine linker, **6** = drug loading around 6 wt%, **10** = drug loading around 10 wt%. ^{b} Polymer polydispersity as measured by light scattering techniques(26) | | | | |

### C. Release of CPT from HGGG6 and HG6

*Release of CPT in PBS* **HGGG6** and **HG6** were prepared at 1 mg/mL in PBS (1×, pH 7.4). A 100 µL aliquot of the solution was transferred to a 1.5 mL Eppendorf tube and incubated at 37 °C. The incubated samples were quenched at selected time intervals and stored at -80 °C until the analysis. Each solution was diluted with 8.5% H₃PO₄ to a 5 mL total volume in a volumetric flask. 30 *µ*L of such solution was injected into the HPLC. The peak area from the CPT lactone form was integrated and compared to a standard curve.

Analysis for the release of CPT from **HGGG6** and **HG6** in PBS containing acetyl cholinesterase (an esterase, 100 units/mL), in KH₂PO₄ buffer (pH 6.1, 0.1 M) and in the KH₂PO₄ buffer (pH 6.1, 0.1 M) containing cathepsin B (a cysteine proteinase, 200 *µ*M, preactivated on ice for 30 minutes in this buffer containing 2 mM DTT and 1 mM EDTA) were performed in a manner similar to that described above for PBS alone. *Release of CPT in Human Plasma* An aliquot of **HGGG6** and **HG6** stock solution were diluted to give final concentration of 0.5 mg/mL in PBS (1×, pH 7.4). This solution was added to a lyophilized powder of human plasma to reconstitute 100% human plasma by the recommended amount. The solution was divided into equal volume (250 *µ*L) to 1.5 mL Eppendorf tubes, incubated at 37 °C, and stopped at selected time point. Samples were stored at -80 °C until the analysis. Samples were separated from plasma by solid phase extraction columns. The solid phase extraction cartridge (Oasis HLB 1cc cartridge from Waters) was pre-conditioned with 1 mL of acetonitrile and then with 1 mL of 8.5% H₃PO₄ before loading. Samples were acidified with equal volume of 8.5% H₃PO₄ prior to loading. After the acidified solution was loaded on the cartridge, the bed was washed with 3 × 1mL of water. Released CPT and polymer conjugate were eluted with 3 × 1 mL of a solution mixture of acetonitrile and potassium phosphate buffer (pH 4.1) (60/40 v/v). The eluted solution was diluted to 5 mL total volume in a 5 mL volumetric flask. 30 *µ*L of such solution was injected into the HPLC. The peak area from the CPT lactone form was integrated and compared to a standard curve.

Release of CPT from **HGGG6** and **HG6** in PBS containing 4% human plasma (PBS/reconstituted human plasma solution = 96/4 (v/v)), in mouse plasma and in reconstituted human albumin (PBS solution) were performed in a manner similar to that described above for pure human plasma.

In PBS (1×, pH 7.4), the half-lives (*t*_{1/2}) for releasing CPT from **HG6** and **HGGG6** were 59h and 32h, respectively. The half-lives decreased to 25h and 22h, respectively, in the presence of 4% human plasma, and to 1.7h and 1.6h, respectively, in 100% human plasma ("HP") and 2.6h and 2.2h, respectively, in 100% mouse plasma ("MP"). CPT release rates for both **HG6** and **HGGG6** in the presence of albumin ("Alb") or acetyl cholinesterase ("Ac Cho") were on the same order of magnitude as in PBS. In a buffer solution at a pH lower than PBS (pH 6.1) with or without the enzyme cathepsin B (active at pH 6.1), less than 50% of total conjugated CPT was released from both **HG6** and **HGGG6** for times up to 144 h (Table 4).

**Table 4. Half-life (t_{1/2}, in hour) of the release of CPT from HG6 and HGGG6^{a}**

| Conjugate | PBS^{b} | 4% HP^{c} | HP^{d} | MP^{e} | Alb^{f} | Ac Cho^{g} | pH 6.1 buffer^{h} | Cath B (pH 6.1)ⁱ |
|---|---|---|---|---|---|---|---|---|
| **HG6** | 59 | 25 | 1.7 | 2.6 | 62 | 33 | >144 | >144 |
| **HGGG6** | 32 | 22 | 1.6 | 2.2 | 73 | 43 | >144 | >144 |

| | | | | | | | | |
|---|---|---|---|---|---|---|---|---|
| ^{a} *t*_{1/2} is defined as time (hours) for the release of half of the total conjugated CPT. Abbreviations: HP means human plasma, MP means mouse plasma. ^{b} pH 7.4 PBS 1x buffer. ^{c} Reconstituted human plasma mixed with PBS (v/v = 4/96). ^{d} Reconstituted human plasma ^{e} Fresh mouse plasma ^{f} In reconstituted human albumin PBS buffer ^{g} In the presence of acetyl cholinesterase PBS solution (100 units/mL). ^{h} pH 6.1 phosphate buffer (0.1M) ⁱ pH 6.1 phosphate buffer in the presence of Cathepsin B | | | | | | | | |

### Release of CPT in Solution at Different pH.

**HGGG6** and **HG6** were prepared at 1 mg/mL in buffer solution with pHs ranging from acidic (pH = 1.2) to basic (pH = 13.1) and incubated at 37 °C for 24h. An aliquot of each solution was diluted with 8.5% H₃PO₄ to about 100 *µ*g/mL. 30 *µ*L of such solution was injected into HPLC. The peak area from the CPT lactone form was integrated and compared to a standard curve.

The pH of aqueous solution has a significant effect on the CPT release rates from both **HG6** and **HGGG6.** The amounts of CPT released from **HG6** and **HGGG6** at 37 °C after 24 h in buffer solutions with pHs ranging from 1.1 to 13.1 are depicted in US Patent No. 7,270,808. The glycinyl-CPT ester bonds of both **HG6** and **HGGG6** were very stable in acidic pH (1.1 to 6.4) as less than 7% of CPT were released in 24 h.

### Methods for increasing drug weight percent loading

### Method I. Synthesis of CD-BisCys-Peg Copolymer with a short Peg linkage and its GlyCPT conjugate

### Example 4: Synthesis of CD-BisCys-Peg (short PEG, e.g., Peg200-Peg2000) and its CPT Conjugate 42

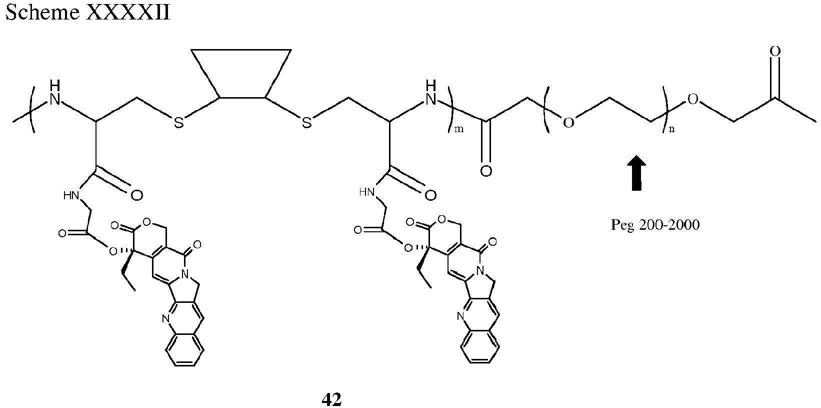

Synthesis of polymer and drug conjugate **42** are same as **36, 37,** and **38**

While Scheme XXXXII shows that the drug is attached at all available positions, not all positions may be reacted. Therefore, a particle comprising conjugates described above may include a conjugate reacted at all positions available for attachment and particles that have less than all of the positions available for attachment containing the drug, e.g., the particle can include CPD reacted at one or none of the positions available for attachment. Thus, while Scheme XXXXII depicts CPT at every point of attachment of each polymer subunit, the CDP-CPT conjugate can have less than 2 CPT molecules attached to any given polymer subunit of the CDP. For example, in one embodiment, the CDP-CPT conjugate includes several polymer subunits and each of the polymer subunits can independently include two, one or no CPT attached at each point of attachment of the polymer subunit. In addition, the particles and compositions can include CDP-CPT conjugates having two, one or no CPT attached to each polymer subunit of the CDP-CPT conjugate and the conjugates can also include a mixture of CDP-CPT conjugates that can vary as to the number of CPTs attached at each point of attachment of the polymer subunits of the conjugates in the particle or composition.

### Method II. Synthesis of CD-BisCys-Peg Copolymer with multiple drug molecules on each loading site.

### Example 5: Synthesis of CD-BisCys-Peg and its GluBis(GlyCPT) Conjugate 43.

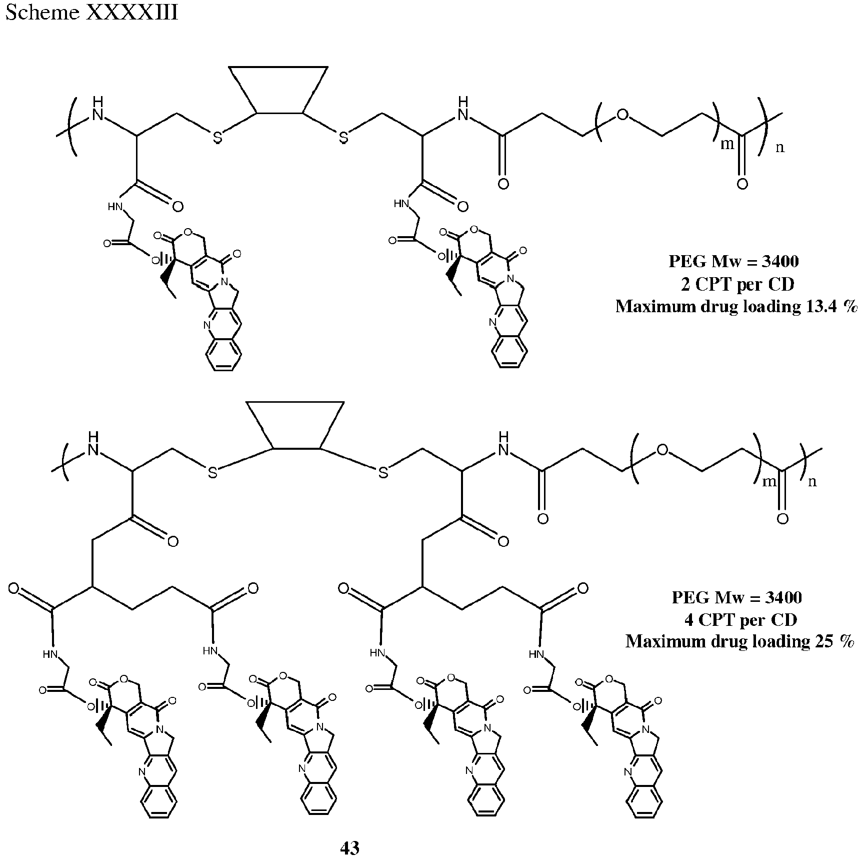

**36** and Glu-Bis(Gly-CPT) 17 are dissolved in DMSO. EDC (3 eq), NHS (2.2 eq), and DIEA (2.2 eq) are added to the solution. CD-BisCys-Peg-GluBis(GlyCPT) **43** is precipitated with CH₃CN and washed with the same solvent until no free drug is detected using UV or TLC. **43** is dried under high vacuum. While Scheme XXXXIII shows that the drug is attached at all available positions, not all positions may be reacted. Therefore, a particle comprising conjugates described above may include a conjugate reacted at all positions available for attachment and particles that have less than all of the positions available for attachment containing the drug, e.g., the particle can include CDP reacted at three, two, one or none of the positions available for attachment. Thus, while Scheme XXXXIII depicts CPT at every point of attachment of each polymer subunit, the CDP-CPT conjugate can have less than 4 CPT molecules attached to any given polymer subunit of the CDP. For example, in one embodiment, the CDP-CPT conjugate includes several polymer subunits and each of the polymer subunits can independently include four, three, two, one or no CPT attached at each point of attachment of the polymer subunit. In addition, the particles and compositions can include CDP-CPT conjugates having four, three, two, one or no CPT attached to each polymer subunit of the CDP-CPT conjugate and the conjugates can also include a mixture of CDP-CPT conjugates that can vary as to the number of CPTs attached at each point of attachment of the polymer subunits of the conjugates in the particle or composition.

### Example 6: Synthesis of CDP-Glv-SN-38 Conjugate

SN-38 was derivatized with the amino acid glycine at the 20-OH position as shown in Scheme 1. Briefly, 20(S)-7-ethyl-10-hydroxycamptothecin (SN-38, 1.0g, 2.5 mmol) was dissolved in a mixture of 70 mL dimethylformamide (DMF) and 30 mL pyridine. A solution of di-tert-butyl-dicarbonate (0.83 g, 3.8 mmol) in 10 mL DMF was added and the mixture stirred at room temperature overnight (12 hours). The solvent was removed under vacuum to yield a yellow solid and re-crystallized from boiling 2-propanol (75 mL) to yield 20(s)-10-tert-butoxycarbonyloxy-7-ethyl-camptothecin (Boc-SN-38) as a yellow solid (0.6 g, 48% yield).

Boc-SN-38 (0.73g, 1.5 mmol), N-(tertbutoxycarbonyl)glycine (0.26g, 1.5 mmol) and 4- dimethylaminopyridine (DMAP, 0.18g, 1.5 mmol) were dissolved in anhydrous methylene chloride (30 mL) and chilled to 0°C. 1,3-Diisopropyl-carbodiimide (DIPC, 0.19g, 1.5 mmol) was added, the mixture stirred at 0°C for 30 minutes followed by stirring for 4 hours at room temperature. The mixture was diluted with methylene chloride to 100 mL, washed twice with an aqueous solution of 0.1N hydrochloric acid (25 mL), dried over magnesium sulfate and the solvent removed under vacuum. The resulting yellow solid was purified by flash chromatography in methylene chloride:acetone (9:1) followed by solvent removal under vacuum to yield 20-O-(N-(tert-butoxycarbonyl) glycyl)-10-tert-butyoxycarbonyloxy-7-ethylcamptothecin (diBoc-Gly-SN-38, 640 mg, 67% yield).

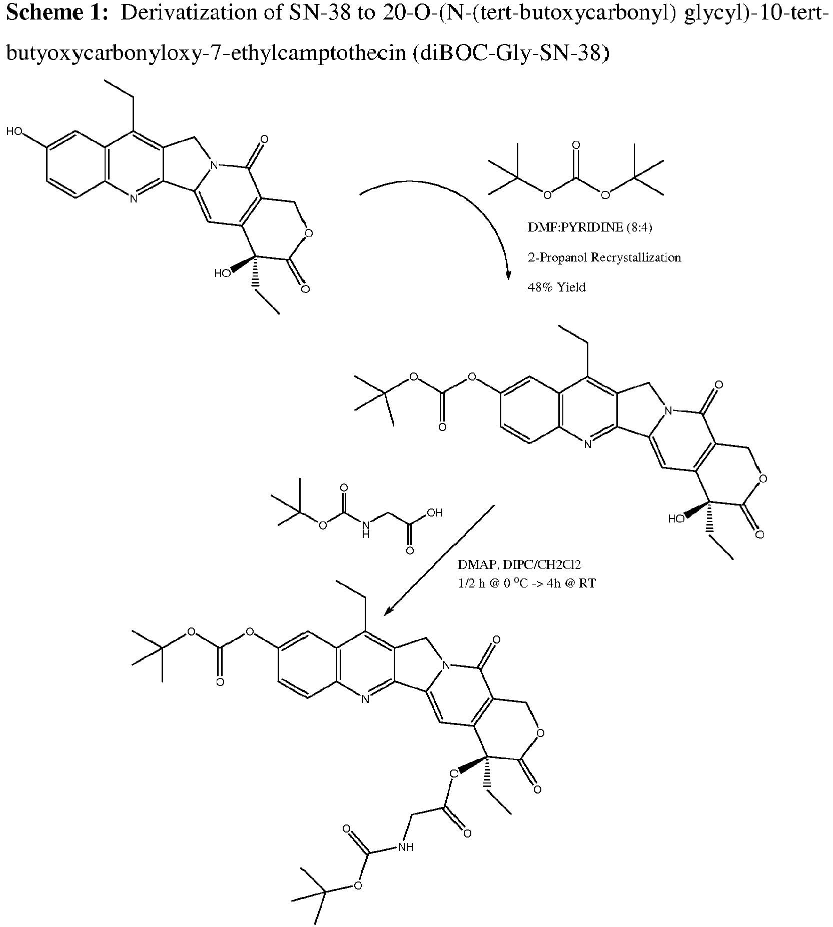

CDP was synthesized as previously described (Cheng et al. (2003) Bioconjugate Chemistry 14(5):1007-1017). diBOC-Gly-SN-38 (0.62g, 0.77 mmol) was deprotected in 15 mL of a 1:1 mixture of methylene chloride:trifluoroacetic acid (TFA) at room temperature for 1 hour. 20-O-trifluoroglycine-10-hydroxy-7-ethylcamptothecin (TFA-Gly-SN-38, 0.57g, 97% yield) was isolated as a yellow solid by precipitation with ethanol (100 mL), followed by two washes with ethanol (30 mL each), dissolution in methylene chloride and removal of solvent under vacuum. ESI/MS expected 449.4; Found 471.66 (M + Na).

CDP-Gly-SN-38 (Poly-CD-PEG-Gly-SN-38, scheme 2) was synthesized as follows: CDP (270 mg, 0.056 mmol), TFA-Gly-SN-38 (70 mg, 0.12 mmol), N-hydroxysuccinimide (14 mg, 0.12 mmol), and 1-ethyl-3-(3-dimethylaminopropyl) carbodiimide (EDCI, 32 mg, 0.17 mmol) were dissolved in dimethylformamide (10 mL) and stirred for 4 hours at room temperature. The polymer was precipitated by addition of 50 mL acetone followed by 50 mL diethyl ether. Precipitate was centrifuged, washed twice with 20 mL acetone each, and dissolved in water acidified to pH 3.0 with hydrochloric acid. Polymer solution was dialized for 24 hours against pH 3.0 water using a 25,000 Da MWCO dialysis membrane. The resulting solution was lyophilized to yield CDP-Gly-SN-38 (180 mg, 67% yield). The polymer was analyzed for total and free SN-38 content by HPLC using SN-38 as a standard curve as previously described (Cheng et al. (2003) Bioconjugate Chemistry 14(5):1007-1017). Total SN-38 content was 7.66% w/w of which 97.4% was polymer bound. Average particle size was determined by dynamic light scattering to be 27.9 nm.

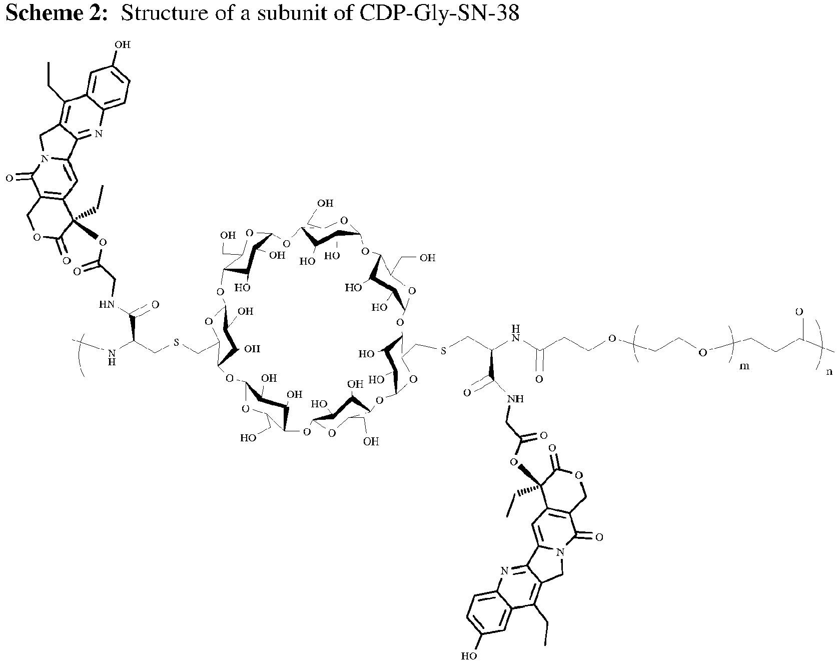

### Example 7: Synthesis of Various CDP-Etoposide Conjugates

In Table 5, various linkers that can be used to link an etoposide to CDP as well as the proposed mechanism of release are listed.

**Table 5: Various linkers that can be used to link an etoposide to CDP**

| # | Structure | Code | Release mechanism |
|---|---|---|---|
| 1 | 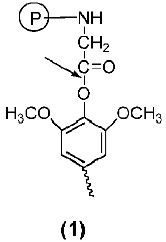 | Gly ester | Enzyme, Base |
| 2 | 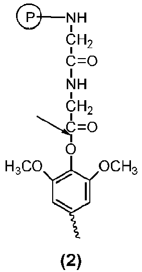 | GlyGly ester | Enzyme, Base |
| 3 | 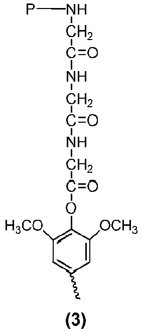 | GlyGlyGly ester | Enzyme, Base |
| 4 | 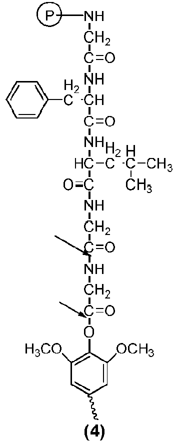 | GFLG-Gly-ester | Enzyme (Cathepsin) - base |
| 5 | 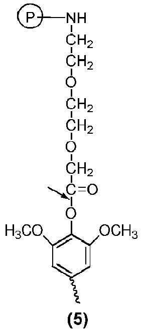 | Mini-PEG ester | Enzyme, Base |
| 6 | 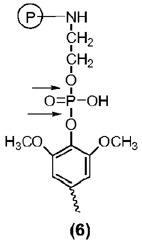 | Phospho-ester | Enzyme, Base |
| 7 | 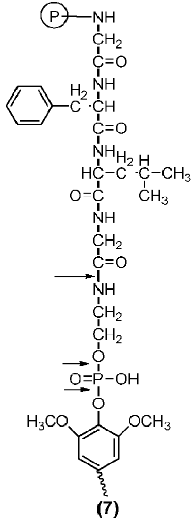 | GFLG Phospho-ester | Enzyme (Cathepsin), base |
| 8 | 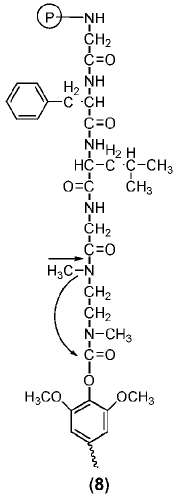 | GFLG-dmedacarbamate | Enzyme |
| 9 | 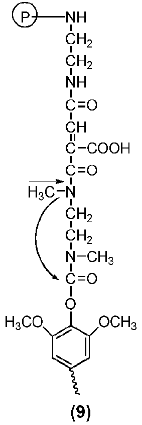 | Cis-aconityl-medacarbamate | Acid |
| 10 | 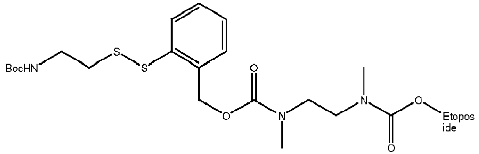 | Disulfidedmedacarbamate | Oxido-reductive with remote release (1,6 elimination followed by cyclization) |
| 11 | 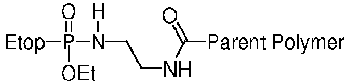 | Phosphoroamide (FY23) | Base, Enzyme (posphatase) |
| 12 | 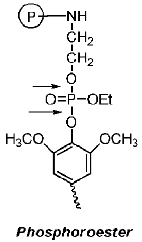 | Phosphoroester | Enzyme, Base |
| 13 | 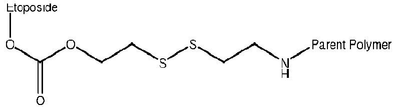 | Disulfidecarbonate | Oxido-reductive with remote release (cyclization) |
| 14 | 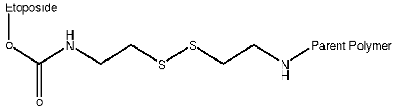 | Disulfidecarbamate | Oxido-reductive with remote release (cyclization) |
| 15 | 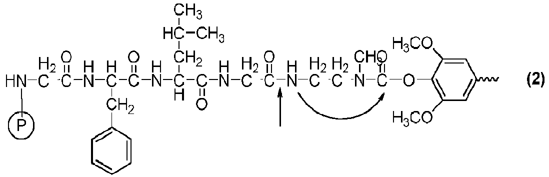 | GFLG-medacarbamate (FY24) | Enzyme (Cathepsin) w. remote release (cyclizing) |
| 16 | 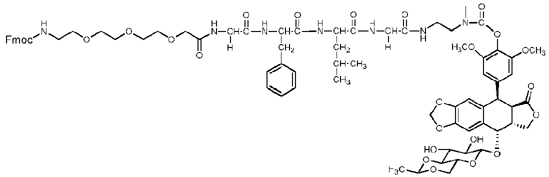 | Mini-PEG-GFLG-medacarbamate (FY25) | Enzyme (Cathepsin) w. remote release (cyclizing) |
| 17 | 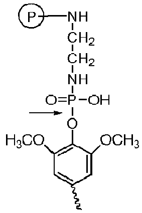 | EDA-etopophos (FY21, FY22) | Base, Enzyme (phosphatase) |
| 18 | 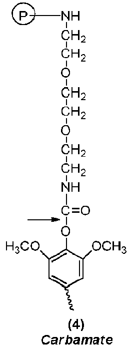 | Mini-PEG-carbamate (FY20) | Base, enzymatic |

### Synthesis of CDP-PEG-GFLG-MEDA-ETOP (Table 5, no. 16)

### Synthesis of FMOC-PEG-GFLG-MEDA

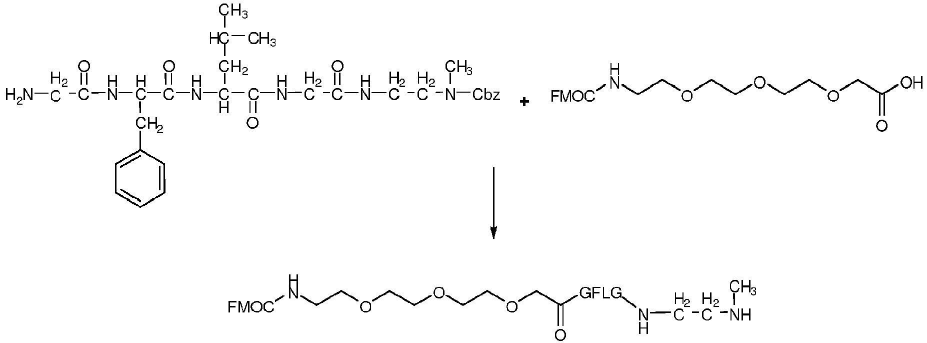

Fmoc-PEG-acetic acid (5.7 g, 13 mmol), HBTU (4.9 g, 13 mmol), HOBT (2.0 g, 13 mmol), and DIPEA (3.4 g, 26 mmol) were dissolved in DMF (25 mL). GFLG-MEDA-Z (5.1 g, 8.8 mmol) was dissolved in DMF (13 mL) and DIPEA (3.7 g, 29 mmol) and added to the previous solution prepared. The reaction mixture was stirred for 1.5 h at room temperature. DMF was removed under reduced pressure and the obtained residue was dissolved in 200 mL CH₂Cl₂, the solution was washed twice with 0.1 N HCl (200 mL) and followed by washing with water (200 mL). It was then dried over MgSO₄ and CH₂Cl₂ was removed under vacuum to yield crude product. It was then purified by flash column chromatography to yield white solid product, FMOC-PEG-GFLG-MEDA-Z (6.2 g, 72 %).

FMOC-PEG-GFLG-MEDA-Z (3.0 g, 3.0 mmol) was dissolved in CH₂Cl₂ (60 mL) of 0.2 M 2-Bromo-1,3,2-benzodioxaborole (2.4 g, 12 mmol). The reaction mixture was stirred overnight at room temperature. The reaction was stopped by the addition of MeOH (10 mL). Solvents were removed under vacuum. The obtained residue was dissolved in a small volume of methanol and precipitated in cool diethyl ether to yield the product (2.6 g, >99%). ESI/MS (m/z) expected 860.01; found 882.76 [M + Na].

### Synthesis of PEG-GFLG-MEDA-ETOP

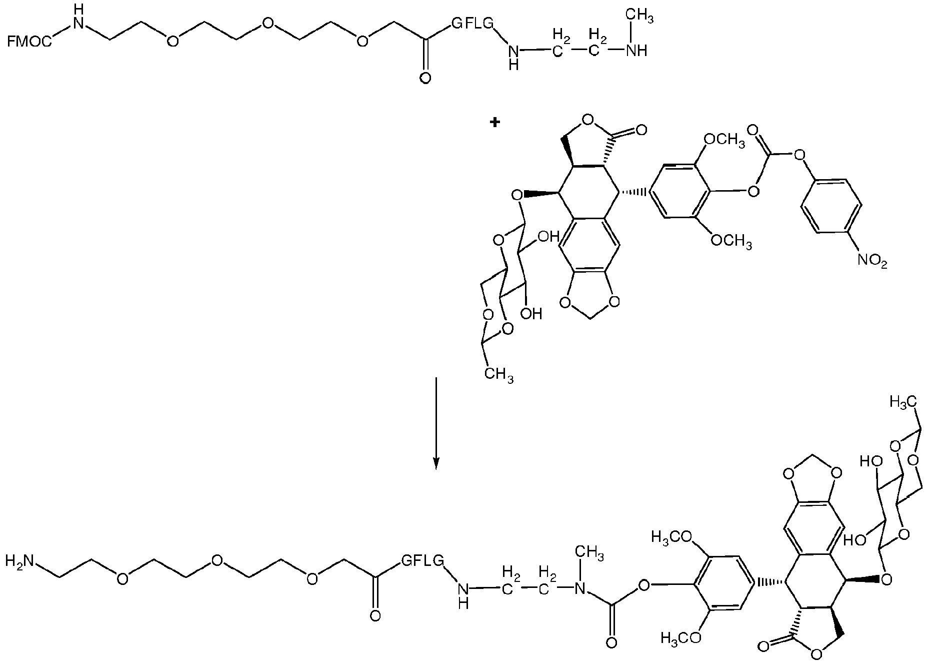

FMOC-PEG-GFLG-MEDA (2.6 g, 2.8 mmol), Etop-NP (2.7 g, 3.6 mmol), DIPEA (0.70 g, 5.5 mmol) and DMAP (34 mg, 0.28 mmol) were dissolved in DMF (60 mL) and stirred for 1.5 h at 60° C. DMF was removed under vacuum. The obtained residue was dissolved in CH₂Cl₂ (150 mL). It was then washed twice with 0.1 N HCl (150 mL) and followed by washing with water (150 mL). It was dried over MgSO₄ and reduced under vacuum to yield the crude product. The crude product was purified by flash column chromatography to yield the product, FMOC-PEG-GFLG-MEDA-ETOP (3.2 g, 80%). ESI/MS (m/z) expected 1474.6; found 1497.16 [M + Na].

FMOC-PEG-GFLG-MEDA-ETOP (100 mg, 0.068 mmol) was dissolved in 1.2 mL of 20% piperidine in DMF. The reaction mixture was stirred for 3 min at room temperature. The product was precipitated in diethyl ether (50 mL) and washed with to yield the product (60 mg, 70%). ESI/MS (m/z) expected 1252.32; found 1274.87 [M + Na].

### Synthesis of CDP-PEG-GFLG-MEDA-ETOP

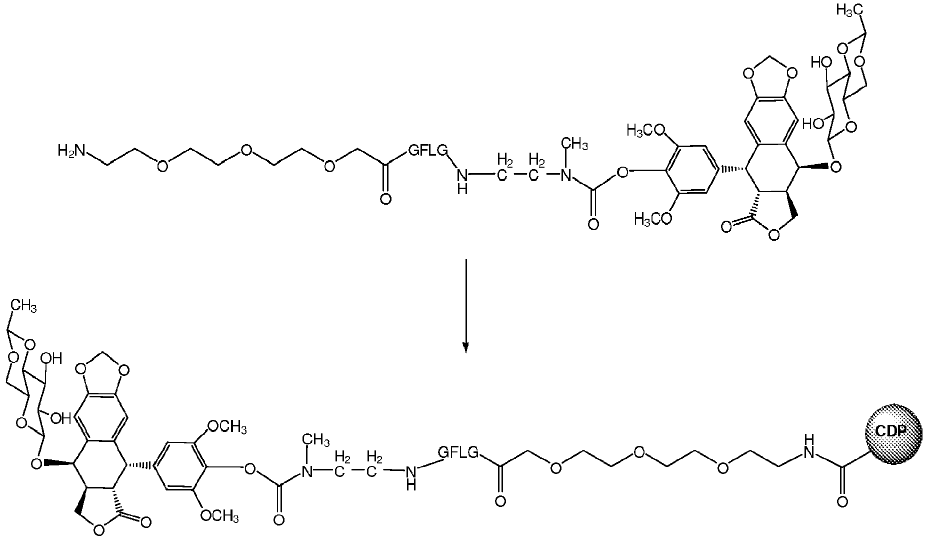

Cyclodextrin-based polymer (CDP) (1.8 g, 0.36 mmol) was dissolved in dry DMF (35 mL). The mixture was stirred until completely dissolved. DIPEA (0.94 g, 7.3 mmol), EDC (0.70 g, 3.6mmol), and NHS (420 mg, 3.6 mmol) were added into the above solution. PEG-GFLG-MEDA-ETOP (1.4 g, 1.1 mmol) was dissolved in DMF (10 mL) and added to the polymer solution. The solution was stirred for 4 h, and then the polymer was precipitated in ethylacetate (150 mL). The precipitate was dissolved in DMF (15 mL) and precipitated in acetone (75 mL). The precipitated product was dissolved in pH 4 water (80 mL). The solution was dialyzed using 25K MWCO membrane (Spectra/Por 7) for 24 h. It was filtered through 0.2 µm filters (Nalgene) and lyophilized to yield white solid (1.1 g, 61%). Loading of etoposide was determined to be 10% w/w by UV-Vis Spectroscopy at 283 nm.

### Synthesis of CDP-Carbamate-S-S-Etoposide (Table 5, no. 14)

### Synthesis of 4-nitrophenyl carbonate ester of etoposide

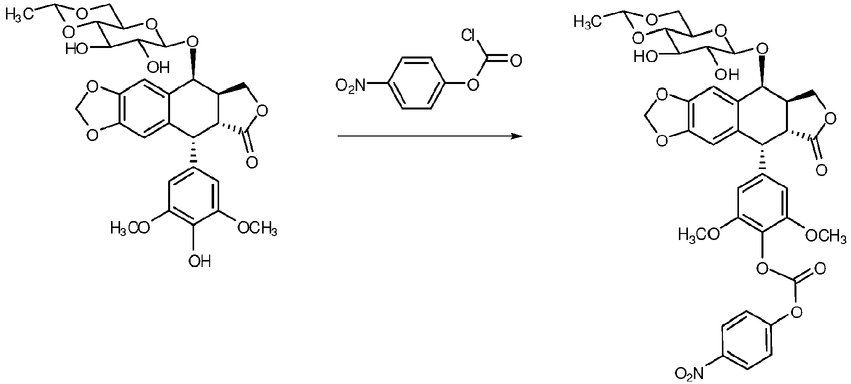

In a dry 100 mL round bottom flask, etoposide (1.0 g, 1.7 mmol) and TEA (2.5g, 25 mmol) were dissolved in anhydrous THF (35 mL) under argon. To that solution, 4-nitrophenyl chloroformate (0.39 g, 1.95 mmol) in anhydrous THF (15 mL) was added dropwise over 30 min. The reaction mixture was stirred for additional 2 h at RT. The mixture was filtered and concentrated under reduced pressure to yield yellow solid. The solid was purified by flash column chromatography to yield light yellow solid (0.75 g, 59%).

### Synthesis of 4-pyridylthiol cysteamine carbamate of etoposide

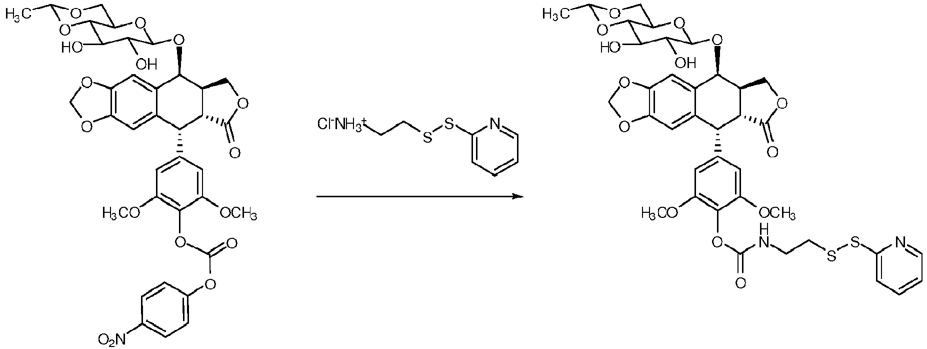

In a dry 25 mL round bottom flask, 4-nitrophenyl carbonate ester of etoposide (100 mg, 0.13 mmol), 4-pyridylthiol cysteamine hydrochloride (35 mg, 0.16 mmol), DIPEA (34 mg, 0.27 mmol) were dissolved in DMF (5 mL). The reaction mixture was stirred at room temperature for 15 h. DMF was removed under reduced pressure to yield a light yellow solid. CH₂Cl₂ (25 mL) was added and it was washed with 0.1 N HCl (10 mL) twice. It was then dried over MgSO₄ and concentrated to yield a light yellow solid. The solid was purified by flash column chromatography to yield yellow solid (51 mg, 48%).

### Synthesis of cystamine carbamate of etoposide

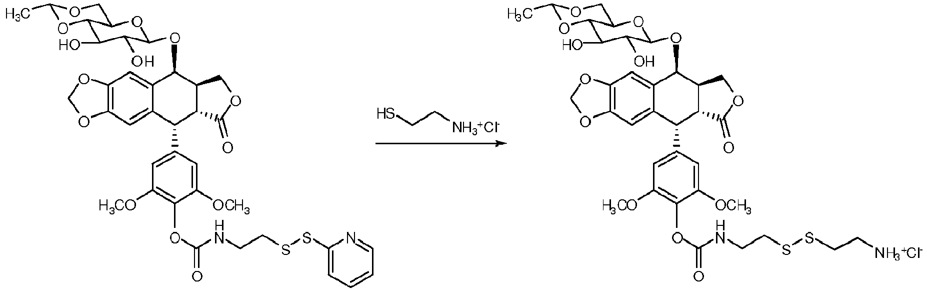

In a 10 mL round bottom flask, 4-pyridylthiol cysteamine carbamate of etoposide (50 mg, 0.0625 mmol) and cysteamine hydrochloride (6.4 mg, 0.057 mmol) were dissolved in MeOH (2 mL). The mixture was stirred for 1 h at room temperature. The solution was concentrated under vacuum and diethyl ether (5 mL) was added to precipitate out white solid. The solid was filtered and redissolved in MeOH (0.5 mL) and precipitated in CH₂Cl₂ (15 mL). The solid was filtered and dried under vacuum to yield a white solid. It was then purified by Prep HPLC to yield white solid (19 mg, 38%). ESI/MS (m/z) expected 767.84; found 767.29 [M]⁺.

### Synthesis of CDP-Carbamate-S-S-Etoposide

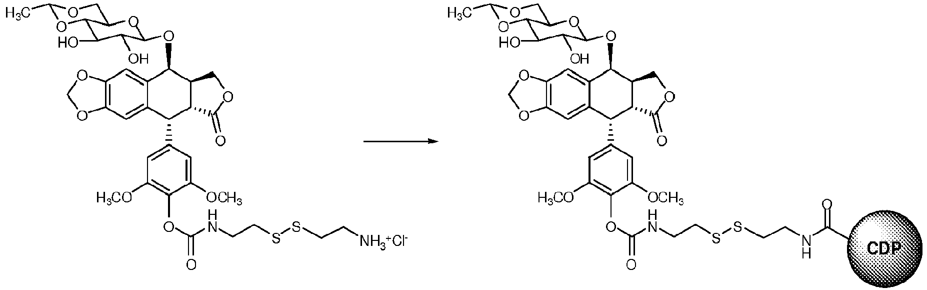

CDP (96 mg, 0.020 mmol) was dissolved in dry *N*,*N*-dimethylformamide (2 mL). The mixture was stirred for 20 min. Cystamine carbamate of etoposide (35 mg, 0.044 mmol), *N*,*N*-Diisopropylethylamine (5.6 mg, 0.044 mmol), *N*-(3-Dimethylaminopropyl)-*N*'-ethylcarbodiimide hydrochloride (11 mg, 0.059 mmol), and *N*-Hydroxysuccinimide (5.0 mg, 0.044 mmol) were added to the polymer solution and stirred for 4 h. The polymer was precipitated with ethylacetate (50 mL). The precipitate was dissolved in deionized water (10 mL). The solution was dialyzed using 25K MWCO membrane (Spectra/Por 7) for 27 h. It was filtered through 0.2 µm filters (Nalgene) and lyophilized to yield white solid (57 mg, 59%). Loading of etoposide was determined to be 12.5% w/w by UV-Vis Spectroscopy at 283 nm.

### Synthesis of CDP-EDA-Phosphoester-Etoposide (Table 5, no. 17)

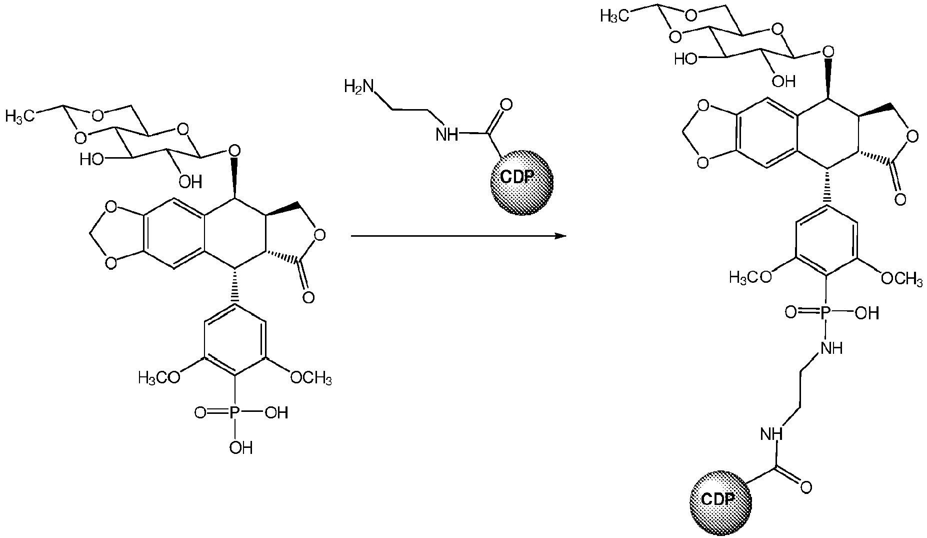

In a 100 mL round bottom flask, etopophosphate (720 mg, 1.1 mmol), *N,N'-*diisopropylcarbodiimide (96 mg, 0.72 mmol), N-hydroxysuccinimide (83 mg, 0.72 mmol) and *N*,*N*-Diisopropylethylamine (140 mg, 2.3 mmol) were dissolved in anhydrous DMF (10 mL). The solution was stirred for 45 min at room temperature. EDA functionalized CDP (1.5 g, 0.60 mmol) and N,N-Diisopropylethylamine (160 mg, 2.3 mmol) were dissolved in anhydrous DMF (10 mL) on a separate 100 mL round bottom flask. This reaction mixture was added to the previous mixture at room temperature and stirred for 4 h at room temperature. The mixture was concentrated to 10 mL and precipitated out in ethyl acetate (500 mL). The polymer was dissolved in deionized water (150 mL) and it was dialyzed using 25K MWCO membrane (Spectra/Por 7) for 26 h. It was then filtered through 0.2 µm filters (Nalgene) and lyophilized to yield white solid (1.1 g, 73%). Loading of etoposide was determined to be 8.3% w/w by UV-Vis Spectroscopy at 283 nm.

### Synthesis of BOC-S-S-DMEDA-Etoposide (Table 5, no. 10)

### Synthesis of cysteamine-S-S-mercaptobenzyl 4-nitrophenyl carbonate ester linker (3)

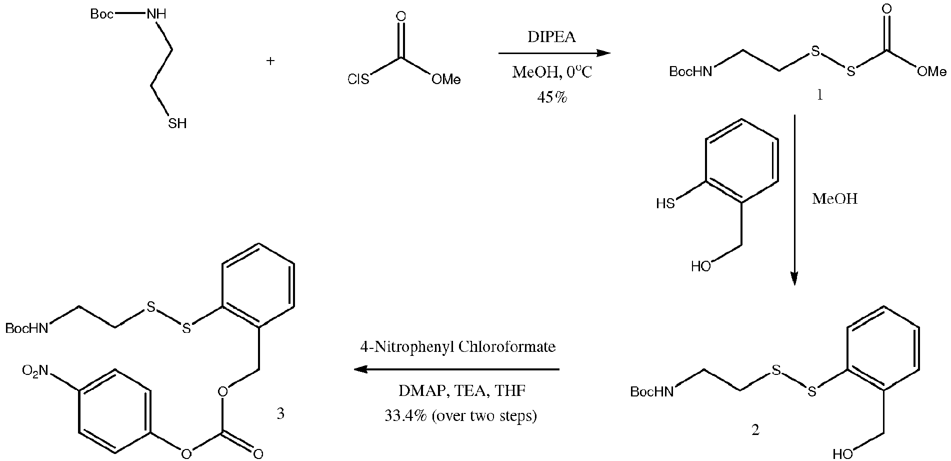

Methoxycarbonylsulfenyl chloride (2.8g, 15.8 mmol) was dissolved in 20 mL anhydrous methanol under inert atmosphere and chilled to 0°C. To this solution TBOC-cysteamine (2.0 g, 15.8 mmol) and *N*,*N*-diisopropylethylamine (DIPEA, 2.75 mL, 15.8 mmol) dissolved in 20 mL methanol were added dropwise while stirring at 0°C. The reaction mixture was stirred at 0°C for 2 hours. Solvent was removed under vacuum and the resulting liquid was purified by flash column chromatography in methylene chloride to yield intermediate **1** (2.2 g, 52% yield). ESI MS expected: 267.06 Found: 290.28 (m + Na).

To a solution of **1** (1.2 g, 4.5 mmol) dissolved in 5 mL anhydrous methanol was added a solution of 2-mercaptobenzyl alcohol (519.5 µL, 4.5 mmol) in 5 mL methanol and stirred for 2 hours at room temperature to yield a yellow liquid containing crude intermediate **2** (ESI MS expected: 315.1, found: 337.9 (M + Na).

Crude intermediate 1 (1.36 g, approx. 4.3 mmol), dimethyl-aminopyridine (DMAP, 50 mg), and triethylamine (TEA, 1.2 mL, 8.6 mmol) were dissolved in 30 mL anhydrous tetrahydrofuran (THF). 4-nitrophenyl chloroformate (1.75g, 8.6 mmol) dissolved in 20 mL THF was added dropwise while stirring under inert atmosphere, and the reaction mixture stirred over night at room temperature. The reaction mixture was concentrated and to yield a yellow solid. The solid was taken up in 25 mL methylene chloride and washed with 2 x 15 mL 0.1N hydrochloric acid in water followed by 2 x 15 mL saturated sodium bicarbonate in water. The organic layer was dried over magnesium sulfate and the solvent evaporated under vacuum and the resulting yellow liquid purified by flash chromatography in ethyl acetate:hexanes 4:1.5 to yield the BOC protected cysteamine-S-S-mercaptobenzyl 4-nitrophenyl carbonate ester linker **3** as a yellow liquid (721 mg, 33.4% yield). ESI MS expected: 480; found: (could not find the MS spectra although it is mentioned in NB).

### Synthesis of BOC-S-S-DMEDA-Etoposide (Table 5, #10)

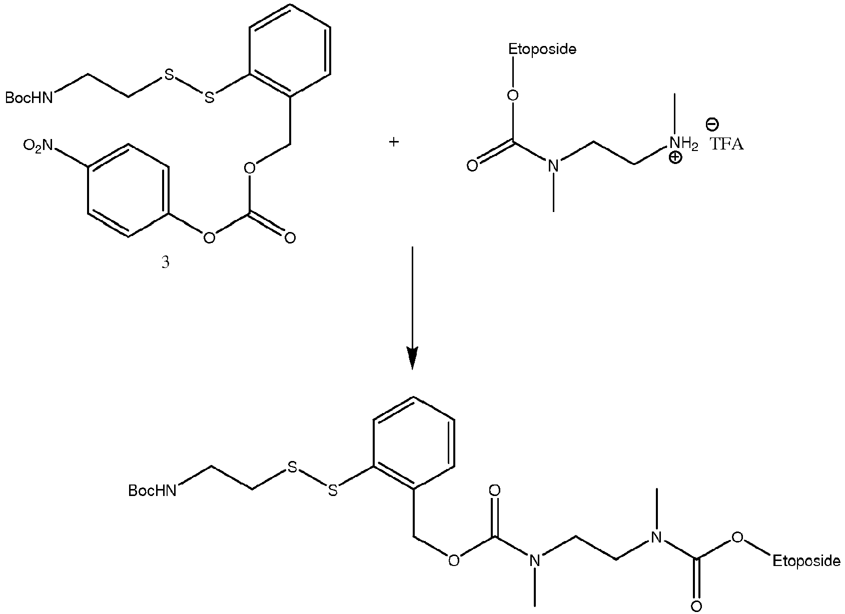

BOC protected dimethyl-ethylenediamine (DMEDA-BOC, 1.12 g, 5.97 mmol), etoposide carbonate (3.0 g, 3.98 mmol), DIPEA (1.36 mL, 7.96 mmol), and DMAP (486 mg, 3.98 mmol) in 60 mL anhydrous DMF were stirred under inert atmosphere for 110 minutes at 60 °C. The solvent was evaporated under vacuum and the oily residue taken up in 25 mL methylene chloride. The organic phase was washed with 2 x 15 mL 0.1 N hydrochloric acid in water and dried over magnesium sulfate. The solvent was evaporated under vacuum to yield a slightly yellow solid. The solid was further purified by flash chromatography in methylene chloride : acetone to yield pure (single peak in HPLC) BOC-DMEDA-Etoposide as a white solid (2.53 g, 79% yield). ESI MS expected: 802.32; found: 825.15 (M + Na).

BOC-DMEDA-etoposide was deprotected by reaction with 1 M trifluoroacetic acid (TFA) in methylene chloride for 6 hours at -15 °C. The solution was concentrated under vacuum and the TFA salt of DMEDA-etoposide precipitated by addition of ethylether.

TFA-DMEDA-etoposide (100 mg, 0.12 mmol), DIPEA (43 µL, 0.24 mmol), and DMAP (14.9 mg, 1.2 mmol) were dissolved in 3 mL anhydrous DMF. To this solution was added intermediate **3** (117 mg, 0.24 mmol) in 2 mL anhydrous DMF and the reaction stirred for 2 hours at 55 °C. Solvent was removed under vacuum and the product dissolved in 20 mL methylene chloride. The organic phase was washed with 3 x 10 mL 0.1 N hydrochloric acid in water, dried over magnesium sulfate and the solvent removed under vacuum. The product was purified by flash chromatography in methylene chloride:acetone 1:1 and the solvent removed under vacuum to yield BOC-S-S-DMEDA-etoposide as a white solid (51 mg). ESI MS expected: 1043.34; found: 1066.6 (M + Na). Single peak in HPLC.

### Example 8: Synthesis of CDP-5-FU

To 6-(Boc-amino)caproic acid (2g, 8.6 mmol) dissolved in 30 mL 1 molar sodium carbonate in water was added 40 mL of a solution of chloromethyl chlorosulfate (1.85 g, 11.2 mmol) and tetrabutylammonium bisulfate (0.58 g, 1.7 mmol) in dichloromethane. The reaction was stirred overnight at room temperature. The reaction mixture was filtered and the aqueous phase was separated and washed with dichloromethane. Water was evaporated under vacuum at 40-60°C to yield 6-(Boc-amino)caproic acid chloromethyl ester (yield not reported, expected yield 2.4 g, 8.6 mmol) as a yellow oil.

6-(Boc-amino)caproic acid chloromethyl ester (approx. 2.4 g, 8.6 mmol) was added dropwise to a solution of 5-fluoro uracil (2.24 g, 17.2 mmol) and triethylamine (TEA, 2.39 mL) in 50 mL dimethylformamide (DMF). The reaction was stirred at room temperature overnight. The reaction mixture was diluted wit 250 mL water vigorously mixed with 250 mL ethylacetate. The organic layer was separated and evaporated under vacuum. The resulting yellow oil was purified by flash chromatography in dichloromethane:methanol 9:1. Fractions containing the product were pooled (approx. 50 mL) and washed with a saturated aqueous solution of sodium chloride (3 x 250 mL). The organic phase was separated and solvent removed under vacuum to yield t-Boc protected 5-fluoro-1N-(methyl-6-amino-caprolate)uracil as a yellow oil.

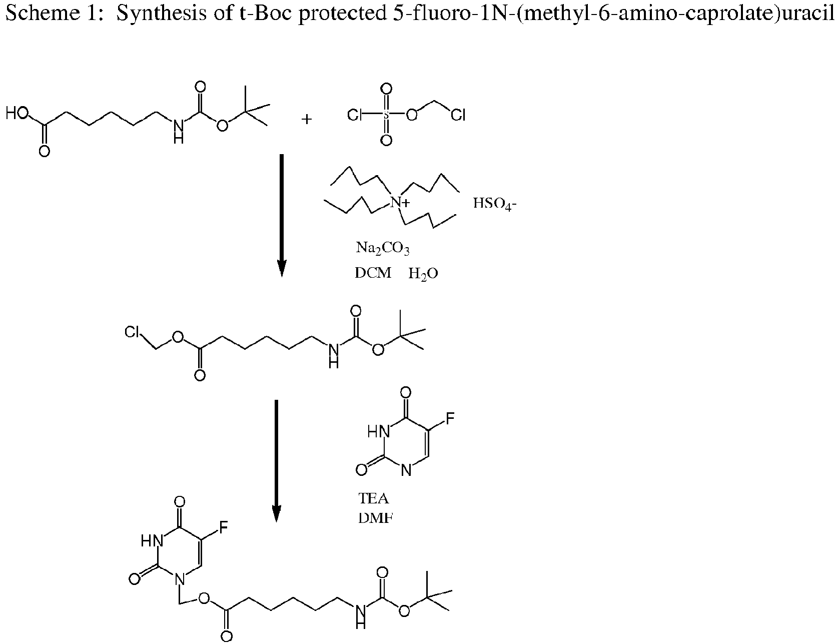

T-Boc protected 5-fluoro-1N-(methyl-6-amino-caprolate)uracil (195 mg) was deprotected by incubation with 5 mL of a 1:1 mixture of 4N HCl:dioxane at room temperature for 1 hour. The solvent was removed under vacuum to yield 5-fluoro-1N-(methyl-6-amino-caprolate)uracil as a white powder.

CDP was synthesized as previously described (Cheng et al. (2003) Bioconjugate Chemistry 14(5):1007-1017). CDP (0.5g, 0.104 mmol) was reacted with 5-fluoro-1N-(methyl-6-amino-caprolate)uracil (85 mg, 0.23 mmol) in the presence of N-hydroxysuccinimide (NHS, 2.62 mg, 0.23 mmol) (Scheme 2) 1-ethyl-3-(3-dimethylaminopropyl) carbodiimide (EDCI, 59.3 mg, 0.309 mmol) and N,N-diisopropyl-ethylamine (DIEA, 39.8 µL, 0.23 mmol) in 5 mL dimethylformamide (DMF) at room temperature for 4 hours. The polymer was precipitated by addition of 25 mL acetone. Precipitate was centrifuged, washed twice with 20 mL acetone each, and dissolved in water acidified to pH 3.0 with hydrochloric acid. Polymer solution was dialized for 24 hours against pH 3.0 water using a 25,000 Da MWCO dialysis membrane. The resulting solution was lyophilized to yield CDP-5-FU (250 mg, approx. 50% yield). The polymer was analyzed for total and free 5-FU content by HPLC using 5-FU as a standard curve as previously described (Cheng, Khin et al. 2003). Total 5-FU content was 3.7% w/w of which 99.2% was polymer bound. Average particle size was determined by dynamic light scattering to be 43.7 nm. 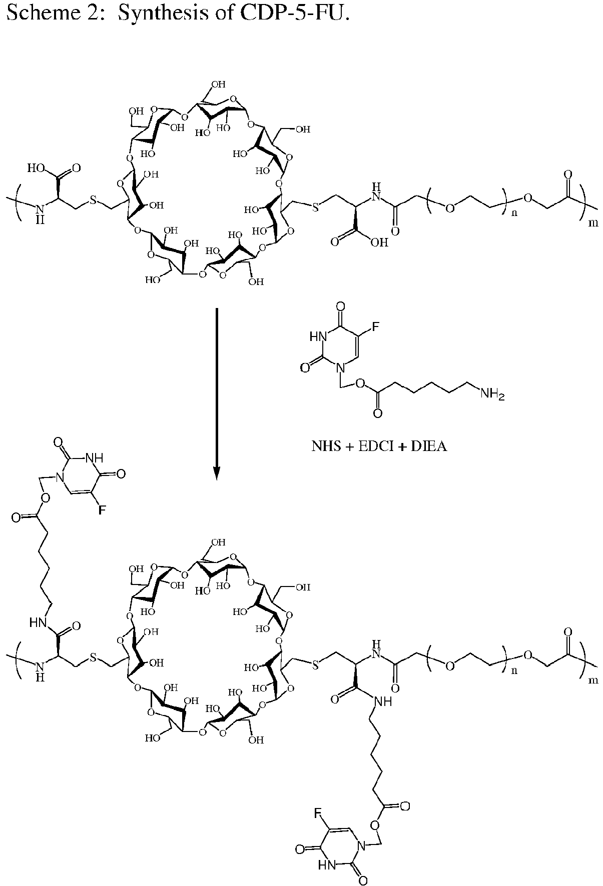 wherein n is an integer resulting in a PEG having a MW of 3400 or less; and m islto 100 (e.g., 4 to 20).

### Example 9: Synthesis of Various CDP-Epothilone Conjugates

**General Experimental Procedures Used in this Example.** All of the anhydrous solvents, HPLC grade solvents and other common organic solvents will be purchased from commercial suppliers (e.g., Sigma-Aldrich) and used without further purification. Parent polymer, Poly-CD-PEG, will be synthesized as previously described (Cheng, Khin et al. (2003) Bioconjug. Chem. 14(5):1007-17). Ixabepilone, KOS-1584, sagopilone and BMS310705 will be purchased from a commercial supplier: Hangzhou onicon corporation, China; ACC corporation, CA, USA; Tocric Biosciences, MO, USA; or Molocon Corporation, ON, Canada. De-ionized water (18-MΩ-cm) will be obtained by passing in-house de-ionized water through a Milli-Q Bioicel Water System (Millipore) or a Barnstead E-pure purification system (Thermo Fisher Scientific, Waltham, MA). NMR spectra will be recorded on a Varian Inova 400 MHz spectrometer (Palo Alto, CA). Mass spectral (MS) analysis will be performed on Bruker FT-MS 4.7 T electrospray mass spectrometer. MWs of the polymer samples will be analyzed on a Agilent 1200 RI coupled with Viscotek 270 LALS-RALS system. Ixabepilone, ixabepilone derivatives, polymer-ixabepilone conjugates, KOS-1584, KOS-1584 derivatives, polymer-KOS-1584 conjugates, sagopilone, sagopilone derivatives, polymer-sagopilone conjugates, epothilone, epothilone derivatives and polymer-epothilone conjugates will be analyzed with a C-18 reverse phase column (Zorbax eclipse) on a Agilent 1100 HPLC system using ammonium bicarbonate buffer (pH 8) and acetonitrile. Particle size measurement will be carried out on a Zetasizer nano-zs (Serial # mal1017190 Malvern Instruments, Worcestershire, UK).

### SYNTHESIS OF A C-3 DERIVATIVE OF CDP-C(O)-O-IXABEPILONE

### Method A : Directly attach linker to epothilone, separate mixture, deprotect and then couple to CDP

### Step 1: Synthesis of Ixabepilone-ε-TROC-aminohexanoate (Scheme 1):

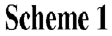

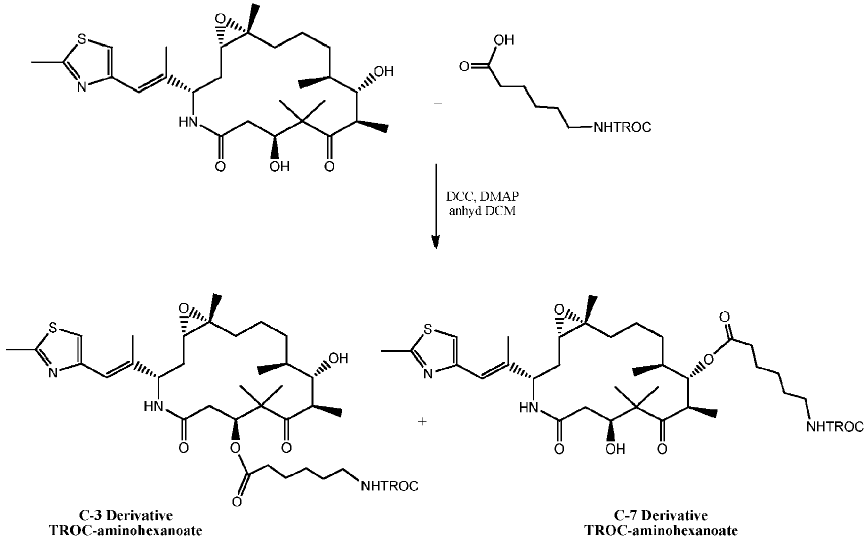

Ixabepilone (20 mg, 0.039 mmol) and ε-TROC-aminohexanoic acid (16.3 mg, 0.0585 mmol) will be dissolved in anhydrous DCM (10 mL) under N₂. To the resulting clear solution, DCC (13.4 mg, 0.065 mmol) and DMAP (7.9 mg, 0.065 mmol) will be added (**Scheme 1**). The reaction mixture will then be stirred for 12 h at room temperature. The solvent will subsequently be evaporated and the resulting residue dissolved in a minimum amount of chloroform. The desired C-3 and C-7 derivatives can be isolated via purification using flash column chromatography with chloroform/methanol as the mobile phase. The derivatives are to be analyzed by electron spray mass spectroscopy (m/z), HPLC and ¹H-NMR. The C-3 derivative of Ixabepilone-ε-TROC-aminohexanoate is used as an example in the following synthetic steps.

### Step 2: Synthesis of Ixabepilone-ε-aminohexanoate (Scheme 2):

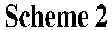

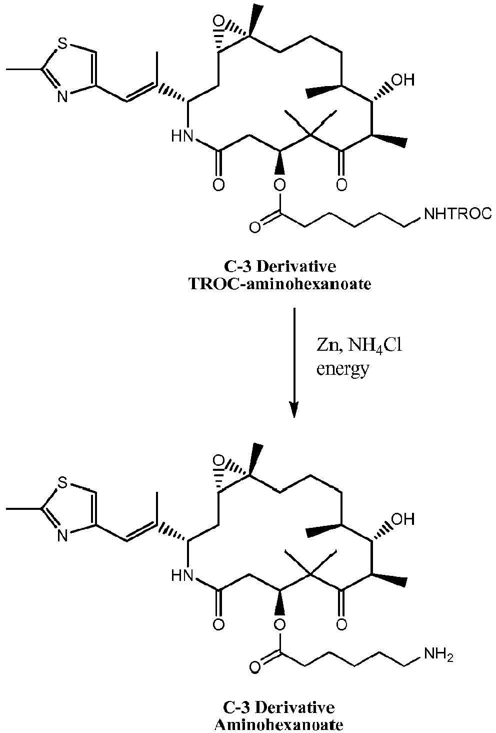

The C-3 derivative of Ixabepilone-ε-TROC-aminohexanoate (15 mg, 0.019 mmol) and ammonium chloride (100 mg, 1.88 mmol) will be combined and mixed in 3 ml of water. While stirring vigorously, Zn powder (98 mg, 1.51 mmol) will be added with the input of energy (e.g., heat, sonication, microwave or ultraviolet irradiation) (Martin et al. (2000) Angewandte Chemie International Edition 39 (3), 581-583) and stirred for an additional 20 min. The resulting solution will be filtered to remove zinc oxide and then washed with hot water. The product will be extracted in dichloromethane and dried over MgSO₄. Evaporation of the organic solvent will be followed by purification of the crude product via flash chromatography. The purified product will then be analyzed by electron spray mass spectroscopy (m/z), HPLC and ¹H-NMR.

### Step 3: Synthesis of CDP-C(O)-O-Ixabepilone (Scheme 3):

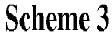

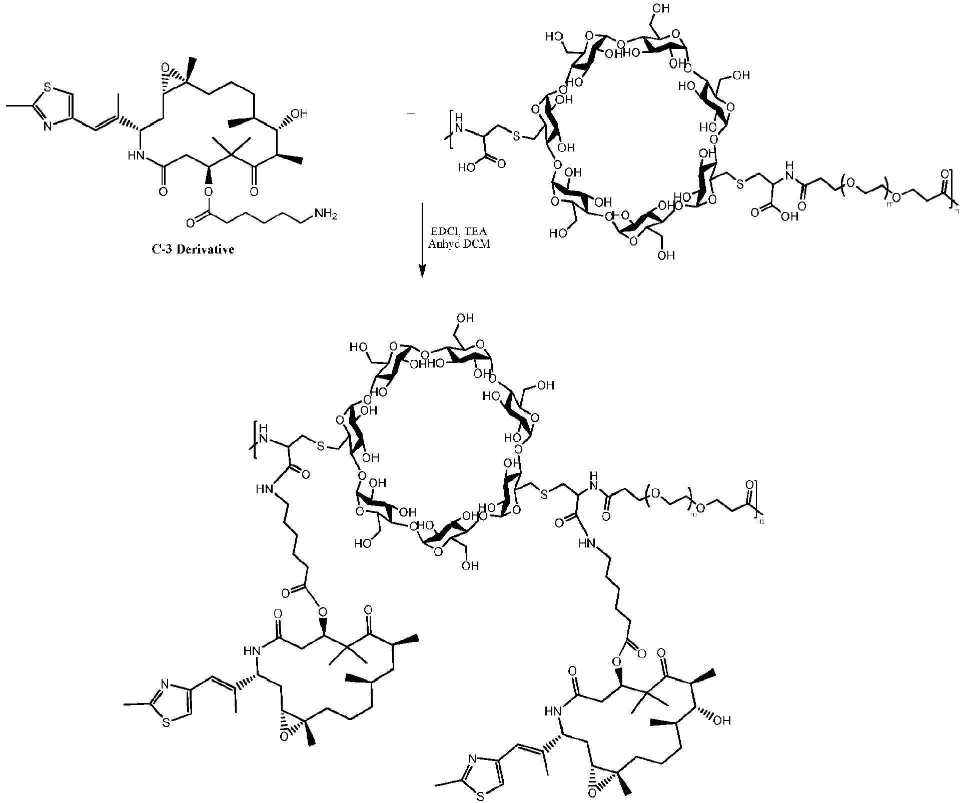

wherein n is an integer resulting in a PEG having a MW of 3400 or less; and m islto 100 (e.g., 4 to 20).

CDP-COOH (50 mg, 0.011 mmol) will be dissolved in MeOH (2.0 mL). The C-3 derivative of Ixabepilone-ε-aminohexanoate (14.7 mg, 0.024 mmol) will subsequently be added to the mixture and stirred for a few minutes to obtain a clear solution. EDCI (6.1 mg, 0.032 mmol) and TEA (3.8 mg, 0.038 mmol) will be added and the reaction stirred at ambient temperature for 3 h (**Scheme 3**). The resulting reaction mixture will be reduced to 0.1 mL of solution and precipitated in Et₂O (1.5 mL). The polymer conjugate will be redissolved in DMF (0.1 mL) and added to acetone (1.5 mL) to precipitate out the polymer conjugate. The polymer conjugate will then be washed with acetone (1 mL) twice, dissolved in nanopure water (3 mL) and then filtered through a 0.2 µm filter membrane and lyophilized to afford CDP-C(O)-O-Ixabepilone. Loading will be determined by UV/Vis spectrometry with a standard curve. The particle size will be determined by Zetasizer.

### Method B: Selectively protect with Silyl protecting group, addition of linker, followed by deprotection and then conjugation with CDP

### Step 1: Synthesis of 3-tert-butyldimethylsilyl Ixabepilone or 7-tert-butyldimethylsilyl Ixabepilone (Scheme 4):

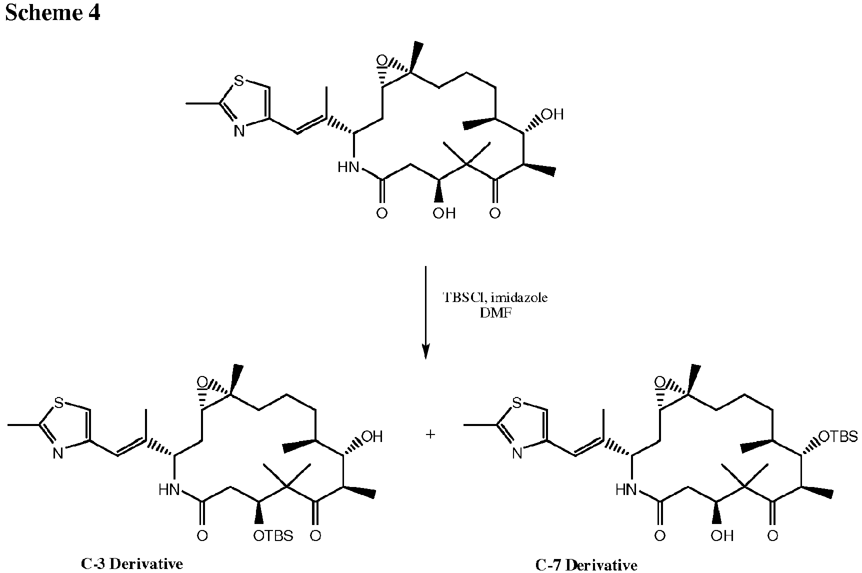

Ixabepilone (20 mg, 0.039 mmol) and tert-butyldimethylsilyl chloride (8.3 mg, 0.055 mmol) will be mixed in anhydrous DMF (5 mL) under N₂ atm. To the resulting clear solution, imidazole (10.7 mg, 0.158 mmol) will be added (**Scheme 4**) and the reaction will be allowed to stir at ambient temperature for 24 h. The solvent will be evaporated and the residue dissolved in a minimum amount of chloroform. The desired C-3 and C-7 derivatives will be isolated following purification of the crude product via flash column chromatography with chloroform/methanol as the mobile phase. The derivatives will be analyzed by electron spray mass spectroscopy (m/z), HPLC and ¹H-NMR. The C-3 derivative of TBS-Ixabepilone is used as an example in the following synthetic steps.

### Step 2: Synthesis of 3-(tert-butyldimethylsilyl)-7-(TROC-aminohexan)-Ixabepilone-oate (Scheme 5):

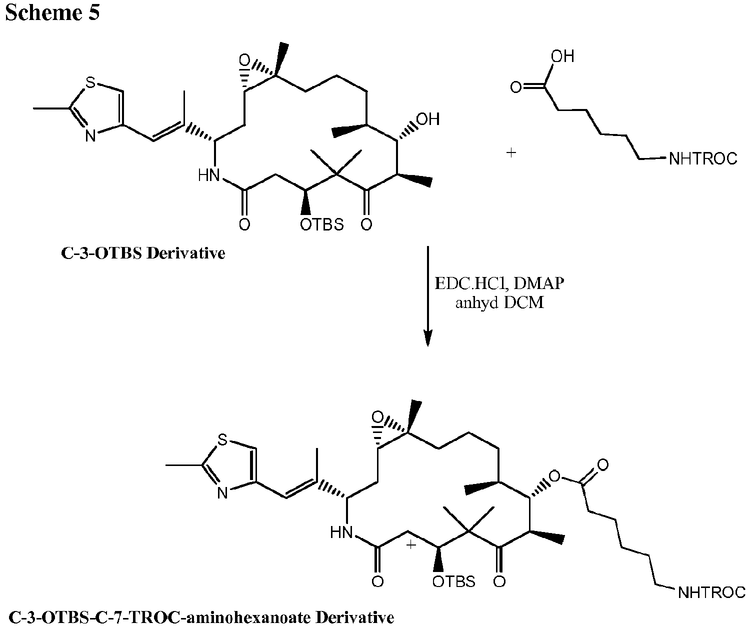

**7-*tert*-butyldimethylsilyl** Ixabepilone (20 mg, 0.032 mmol) and ε-TROC-aminohexanoic acid (12.0 mg, 0.039 mmol) will be stirred together in anhydrous DCM (2 mL) under N₂. To the resulting clear solution, EDC.HCl (11.1 mg, 0.058 mmol) and DMAP (7.08 mg, 0.058 mmol) will be added (**Scheme 5**). The reaction mixture is then stirred for 12 h at 22 °C. The solvent is subsequently evaporated and the resulting residue dissolved in a minimum amount of chloroform. The crude product will be purified via flash column chromatography with chloroform/methanol as the mobile phase. The product will be analyzed by electron spray mass spectroscopy (m/z), HPLC and ¹H-NMR.

### Step 3: Synthesis of 7-(aminohexan)-Ixabepilone-oate (Scheme 6):

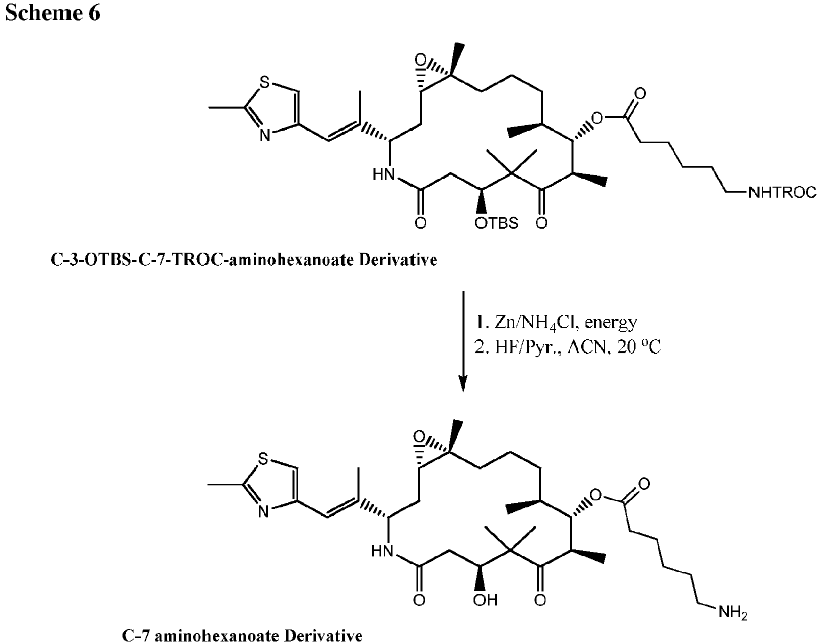

3-(*tert*-butyldimethylsilyl)-7-(TROC-aminohexan)-Ixabepilone-oate will be deprotected using Zn/NH₄Cl with the input of energy (e.g., heat, sonication, microwave or ultraviolet irradiation), followed by a solution of acetonitrile and HF/pyridine. The final product will be purified via flash column chromatography with chloroform/methanol as the mobile phase. 3-(aminohexan)-Ixabepilone-oate will be analyzed by electron spray mass spectroscopy (m/z), HPLC and ¹H-NMR.

### Step 4: Synthesis of CDP-C(O)-O-Ixabepilone (Scheme 7):

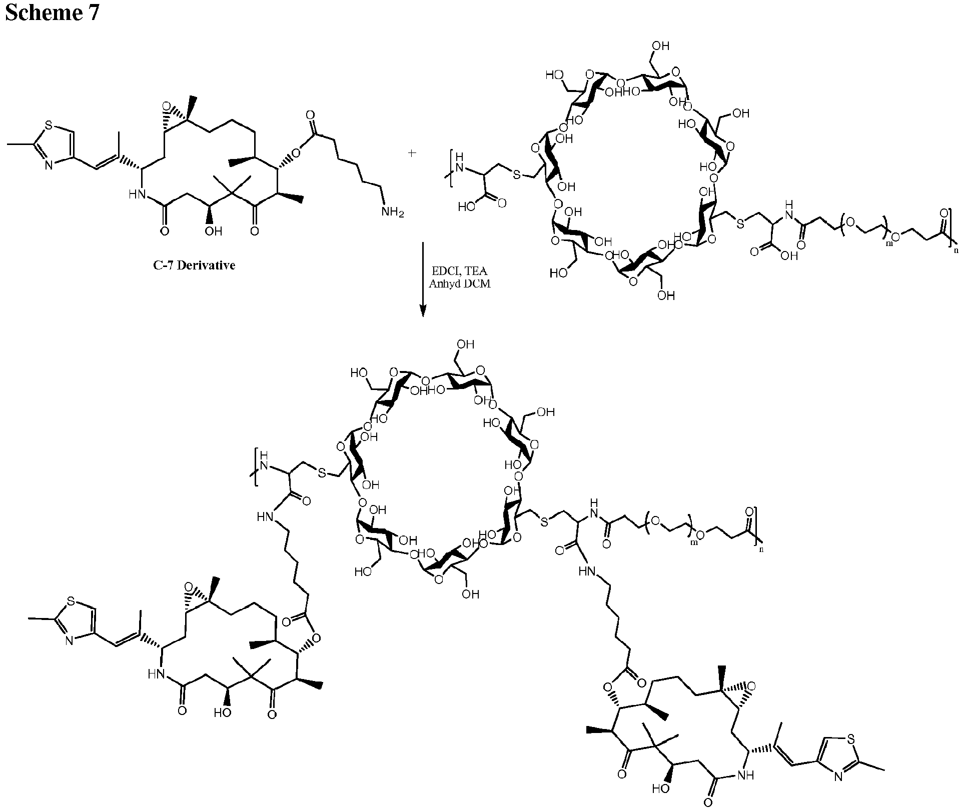

wherein n is an integer resulting in a PEG having a MW of 3400 or less; and m islto 100 (e.g., 4 to 20).

CDP-COOH (50 mg, 0.011 mmol) will be dissolved in MeOH (2.0 mL). C-7 derivative of Ixabepilone-ε-aminohexanoate (14.7 mg, 0.024 mmol) will subsequently be added to the mixture and stirred for a few minutes to obtain a clear solution. EDCI (6.1 mg, 0.032 mmol) and TEA (3.8 mg, 0.038 mmol) will then be added and the reaction stirred at ambient temperature for 3 h (**Scheme 7**). The reaction mixture will be reduced to 0.1 mL of solution and precipitated in Et₂O (1.5 mL). The polymer conjugate will be redissolved in DMF (0.1 mL) and added to acetone (1.5 mL) to precipitate out the polymer conjugate. The polymer conjugate will be washed with acetone (1 mL) twice, dissolved in nanopure water (3 mL) and then filtered through a 0.2 µm filter membrane and lyophilized to afford CDP-C(O)-O-Ixabepilone.

### SYNTHESIS OF A C-7 DERIVATIVE OF CDP-C(O)-O-IXABEPILONE

### Method A : Directly attach linker to epothilone, separate mixture, deprotect and then couple to CDP

### Step 1: Synthesis of Ixabepilone-ε-TROC-aminohexanoate (Scheme 8):

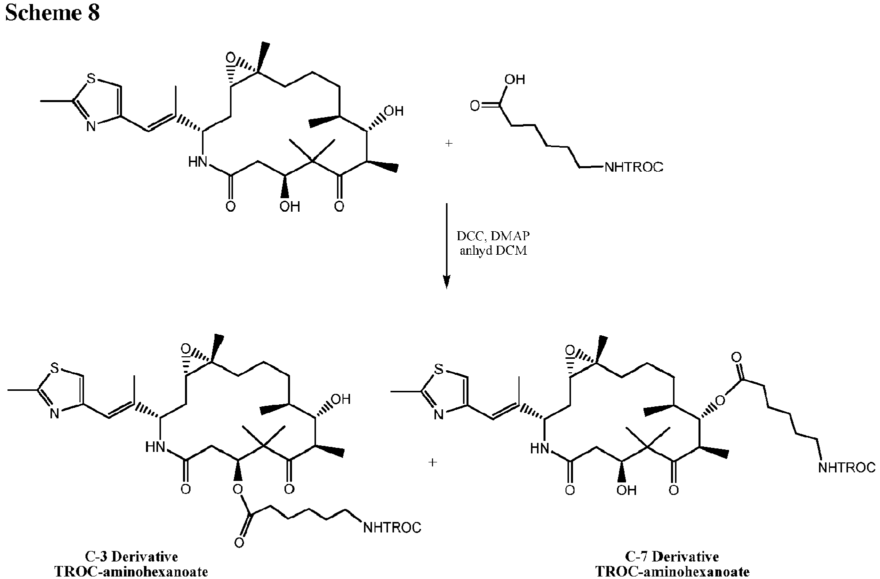

Ixabepilone (20 mg, 0.039 mmol) and ε-TROC-aminohexanoic acid (16.3 mg, 0.0585 mmol) will be dissolved in anhydrous DCM (10 mL) under N₂. To the resulting clear solution, DCC (13.4 mg, 0.065 mmol) and DMAP (7.9 mg, 0.065 mmol) will be added (**Scheme 8**). The reaction mixture will then be stirred for 12 h at room temperature. The solvent will subsequently be evaporated and the resulting residue dissolved in a minimum amount of chloroform. The desired C-3 and C-7 derivatives can be isolated via purification using flash column chromatography with chloroform/methanol as the mobile phase. The derivatives are to be analyzed by electron spray mass spectroscopy (m/z), HPLC and ¹H-NMR. The C-7 derivative of Ixabepilone-ε-TROC-aminohexanoate is used as an example in the following synthetic steps.

### Step 2: Synthesis of Ixabepilone-ε-aminohexanoate (Scheme 9):

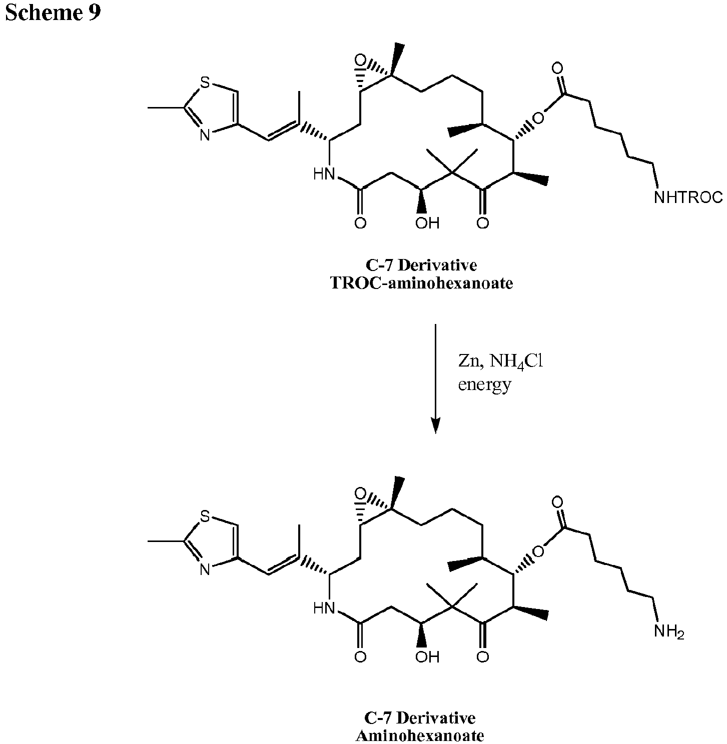

The C-7 derivative of Ixabepilone-ε-TROC-aminohexanoate (15 mg, 0.019 mmol) and ammonium chloride (100 mg, 1.88 mmol) will be combined and mixed in 3 ml of water. While stirring vigorously, Zn powder (98 mg, 1.51 mmol) will be added with the input of energy (e.g., heat, sonication, microwave or ultraviolet irradiation (Martin et al. (2000) Angewandte Chemie International Edition, 39 (3):581-583) and stirred for an additional 20 min. The resulting solution will be filtered to remove zinc oxide and then washed with hot water. The product will be extracted in dichloromethane and dried over MgSO₄. Evaporation of the organic solvent will be followed by purification of the crude product via flash chromatography. The purified product will then be analyzed by electron spray mass spectroscopy (m/z), HPLC and ¹H-NMR.

### Step 3: Synthesis of CDP-C(O)-O-Ixabepilone (Scheme 10):

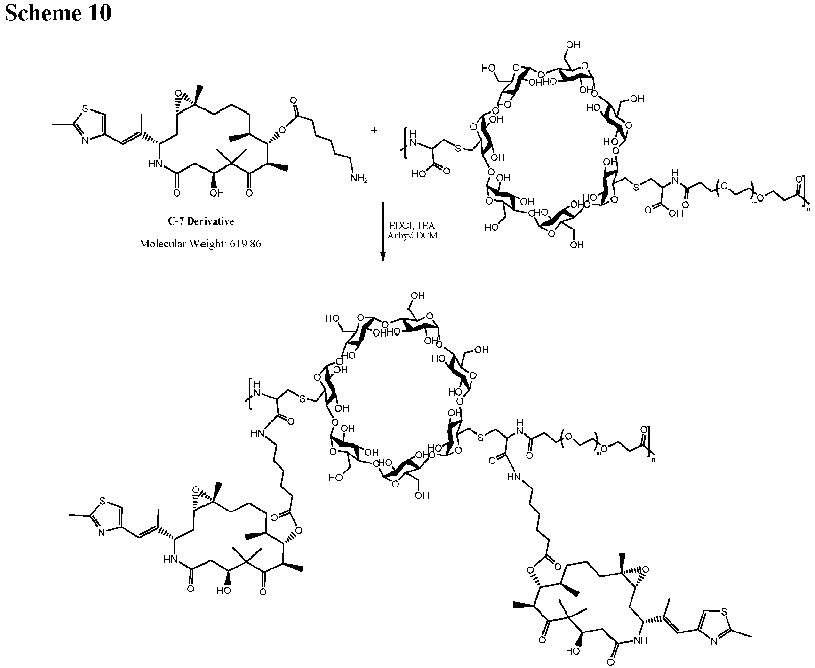

wherein n is an integer resulting in a PEG having a MW of 3400 or less; and m is1 to 100 (e.g., 4 to 20).

CDP-COOH (50 mg, 0.011 mmol) will be dissolved in MeOH (2.0 mL). The C-7 derivative of Ixabepilone-ε-aminohexanoate (14.7 mg, 0.024 mmol) will subsequently be added to the mixture and stirred for a few minutes to obtain a clear solution. EDCI (6.1 mg, 0.032 mmol) and TEA (3.8 mg, 0.038 mmol) will be added and the reaction stirred at ambient temperature for 3 h (**Scheme 10**). The resulting reaction mixture will be reduced to 0.1 mL of solution and precipitated in Et₂O (1.5 mL). The polymer conjugate will be redissolved in DMF (0.1 mL) and added to acetone (1.5 mL) to precipitate out the polymer conjugate. The polymer conjugate will then be washed with acetone (1 mL) twice, dissolved in nanopure water (3 mL) and then filtered through a 0.2 µm filter membrane and lyophilized to afford CDP-C(O)-O-Ixabepilone. Loading will be determined by UV/Vis spectrometry with a standard curve. The particle size will be determined by Zetasizer.

### Method B: Selectively protect with Silyl protecting group, addition of linker, followed by deprotection and then conjugation with CDP

### Step 1: Synthesis of 3-tert-butyldimethylsilyl Ixabepilone or 7-tert-butyldimethylsilyl Ixabepilone (Scheme 11):

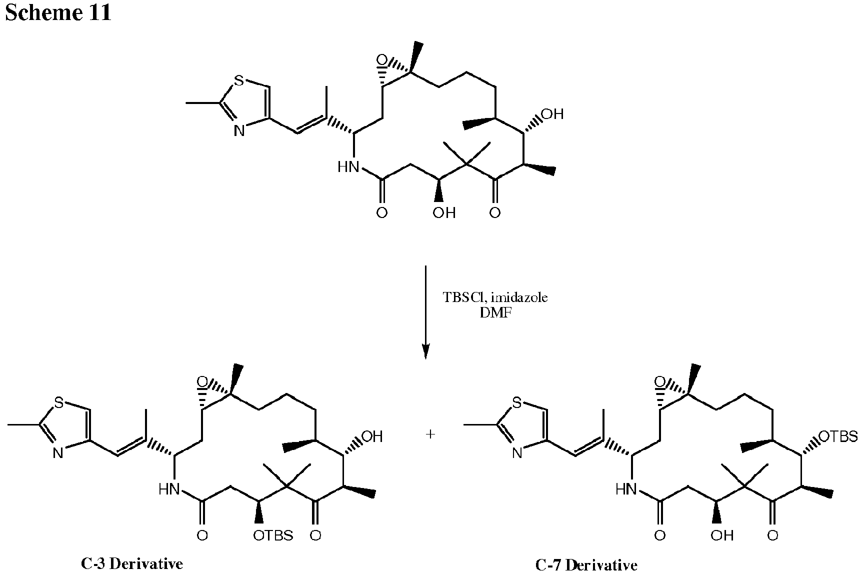

Ixabepilone (20 mg, 0.039 mmol) and tert-butyldimethylsilyl chloride (8.3 mg, 0.055 mmol) will be mixed in anhydrous DMF (5 mL) under N₂ atm. To the resulting clear solution, imidazole (10.7 mg, 0.158 mmol) will be added (**Scheme 11**) and the reaction will be allowed to stir at ambient temperature for 24 h. The solvent will be evaporated and the residue dissolved in a minimum amount of chloroform. The desired C-3 and C-7 derivatives will be isolated following purification of the crude product via flash column chromatography with chloroform/methanol as the mobile phase. The derivatives will be analyzed by electron spray mass spectroscopy (m/z), HPLC and ¹H-NMR. The C-7 derivative of TBS-Ixabepilone is used as an example in the following synthetic steps.

### Step 2: Synthesis of 7-(tert-butyldimethylsilyl)-3-(TROC-aminohexan)-Ixabepilone-oate (Scheme 12):

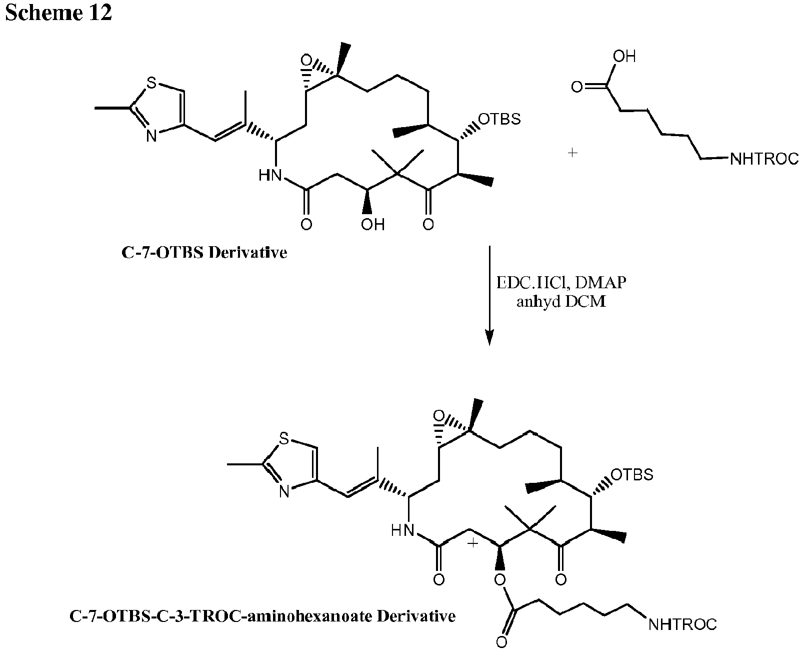

**7-*tert*-butyldimethylsilyl** Ixabepilone (20 mg, 0.032 mmol) and ε-TROC-aminohexanoic acid (12.0 mg, 0.039 mmol) will be stirred together in anhydrous DCM (2 mL) under N₂. To the resulting clear solution, EDC.HCl (11.1 mg, 0.058 mmol) and DMAP (7.08 mg, 0.058 mmol) will be added (Scheme 12). The reaction mixture will then be stirred for 12 h at 22 °C. The solvent is subsequently evaporated and the resulting residue dissolved in a minimum amount of chloroform. The crude product will be purified via flash column chromatography with chloroform/methanol as the mobile phase. The product will be analyzed by electron spray mass spectroscopy (m/z), HPLC and ¹H-NMR.

### Step 3: Synthesis of 3-(aminohexan)-Ixabepilone-oate (Scheme 13):

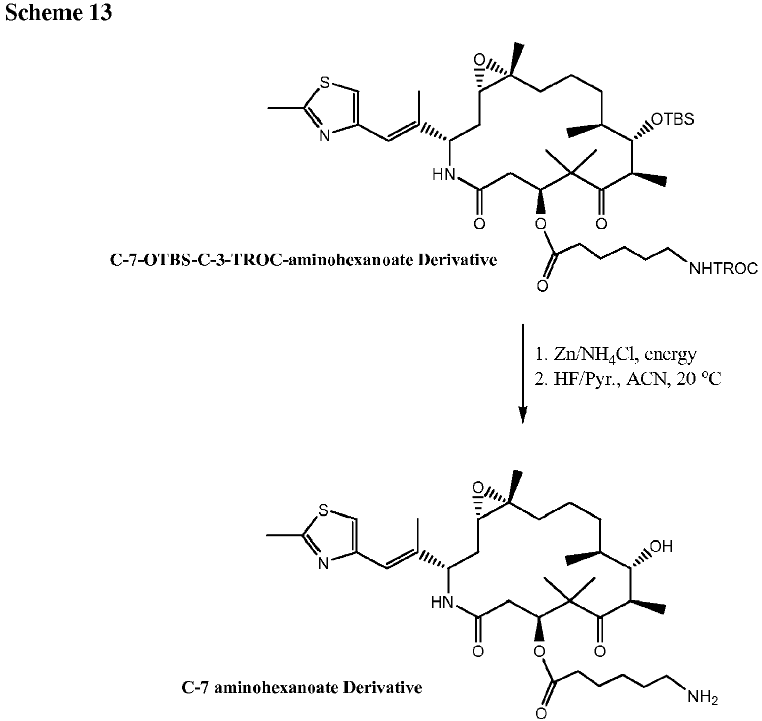

7-(*tert*-butyldimethylsilyl)-3-(TROC-aminohexan)-Ixabepilone-oate will be deprotected using Zn/NH₄Cl with the input of energy (e.g., heat, sonication, microwave or ultraviolet irradiation), followed by a solution of acetonitrile and HF/Pyridine. The final product will be purified via flash column chromatography with chloroform/methanol as the mobile phase. 3-(aminohexan)-Ixabepilone-oate will be analyzed by electron spray mass spectroscopy (m/z), HPLC and ¹H-NMR.

### Step 4: Synthesis of CDP-C(O)-O-Ixabepilone (Scheme 14):

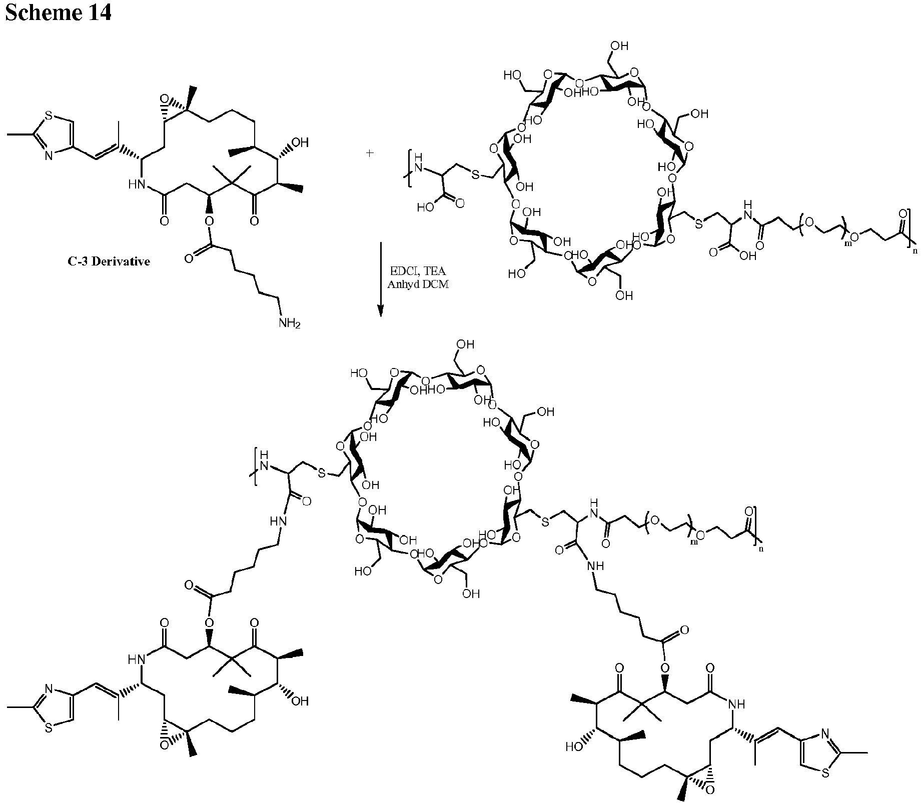

wherein n is an integer resulting in a PEG having a MW of 3400 or less; and m islto 100 (e.g., 4 to 20).

CDP-COOH (50 mg, 0.011 mmol) will be dissolved in MeOH (2.0 mL). C-3 derivative of Ixabepilone-ε-aminohexanoate (14.7 mg, 0.024 mmol) will subsequently be added to the mixture and stirred for a few minutes to obtain a clear solution. EDCI (6.1 mg, 0.032 mmol) and TEA (3.8 mg, 0.038 mmol) will then be added and the reaction stirred at ambient temperature for 3 h (**Scheme 14**). The reaction mixture will be reduced to 0.1 mL of solution and precipitated in Et₂O (1.5 mL). The polymer conjugate will be redissolved in DMF (0.1 mL) and added to acetone (1.5 mL) to precipitate out the polymer conjugate. The polymer conjugate will be washed with acetone (1 mL) twice, dissolved in nanopure water (3 mL) and then filtered through a 0.2 µm filter membrane and lyophilized to afford CDP-C(O)-O-Ixabepilone.

### SYNTHESIS OF CDP-PHOSPHONAMIDE-EPOTHILONE B

### Synthesis of Fmoc-NH-(CH₂)₂-PO(OH)₂

2-Aminoethylphosphonic acid (5.0 g, 0.040 mol) will be dissolved in a tetrahydrofuran/water mixture (1:1) (40 mL). To the mixture, Fmoc N-hydroxysuccinimide ester (16 g, 0.048 mmol) in THF (10 mL) will be added slowly in an ice bath and stirred for ½ h. It will be stirred at ambient temperature for an additional 2 h. The solvent will be removed under vacuum (**Scheme 15**).

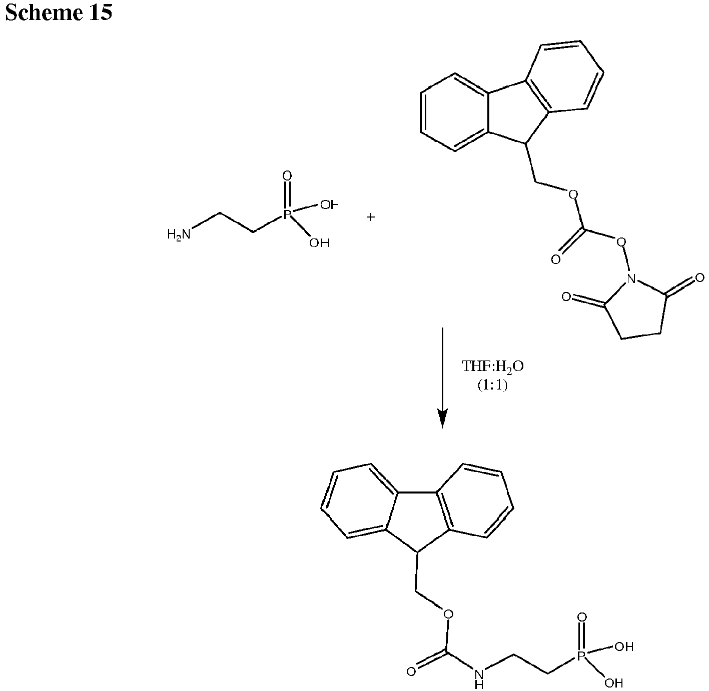

### Synthesis of NH₂-(CH₂)₂-PO(OH)-NH-Epothilone

Fmoc-NH-(CH₂)₂-PO(OH)₂ (3.0 g, 8.6 mmol) will be dissolved in methylene chloride (100 mL). N,N'-Dicyclohexylcarbodiimide (2.1 g, 10 mmol) and N-hydroxysuccinimide (1.2 g, 10 mmol) will be added to the solution in an ice bath. The mixture will be stirred for ½ h in an ice bath and it will be stirred at ambient temperature for additional 1 h. Epothilone B analog (5.4 g, 10 mmol) will be added to the mixture and stirred for an additional 3 h. White precipitate will be filtered off. The organic layer will be washed with brine and dried over MgSO₄. The organic layer will be removed under vacuum to yield solid product. The solid will be purified by flash column chromatography. The product will be deprotected using a piperidine in methanol mixture. The organic layer will be pumped down and used without further purification. (**Scheme 16**).

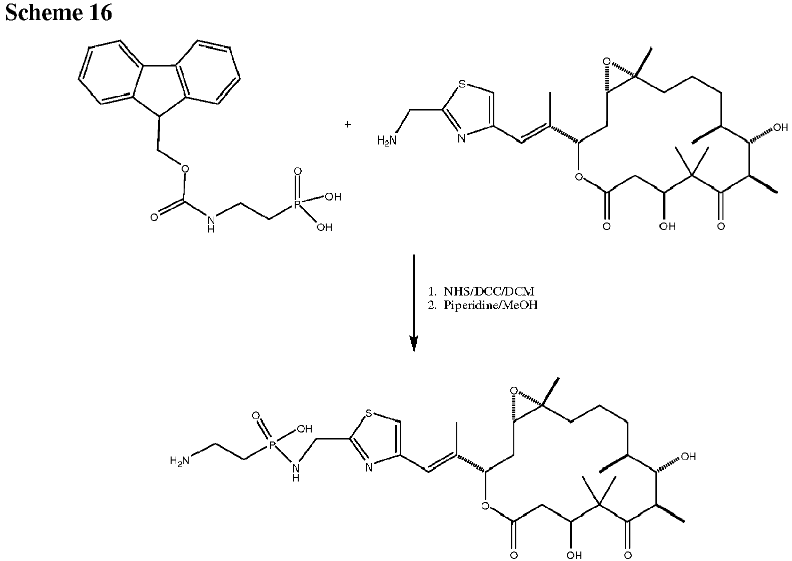

### Synthesis of CDP-NH₂-(CH₂)₂-PO(OH)-NH-Epothilone B

CDP (1.0 g, 0.21 mmol) will be dissolved in dry N,N-dimethylformamide (DMF, 20 mL). NH₂-(CH₂)₂-PO(OH)-NH-Epothilone (300 mg, 0.46 mmol), N,N-diisopropylethylamine (0.080 mL, 0.46 mmol), N-(3-Dimethylaminopropyl)-N'-ethylcarbodiimide hydrochloride (120 mg, 0.62 mmol), and N-Hydroxysuccinimide (52 mg, 0.46 mmol) will be added to the polymer solution and stirred for 4 h. The polymer will be precipitated with ethylacetate (100 mL) and rinsed with acetone (50 mL). The precipitate will be dissolved in water at pH 8 (100 mL). The solution will be dialyzed using 25,000 MWCO membrane (Spectra/Por 7) for 24 h in water. It will be filtered through 0.2 µm filters (Nalgene) and lyophilized to yield a white solid (**Scheme 17**). 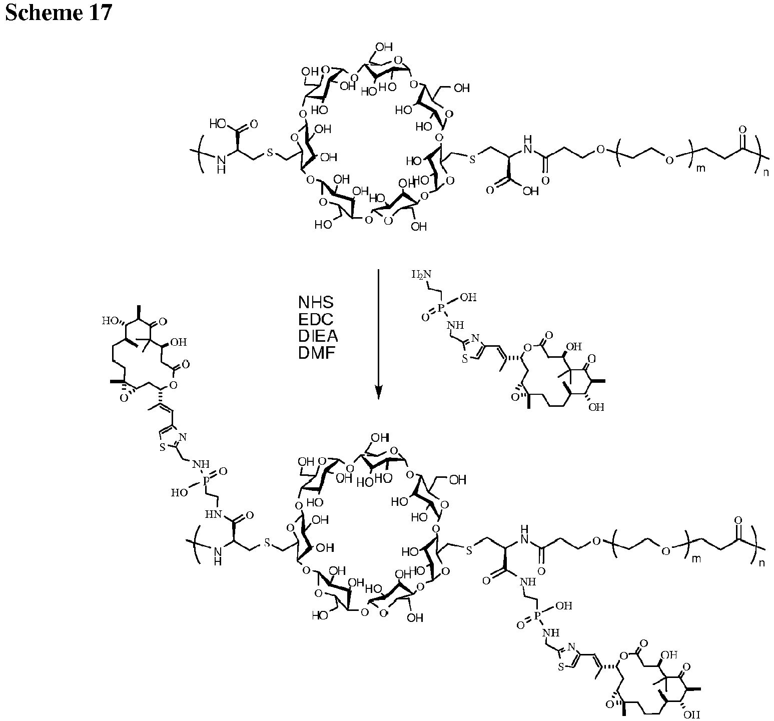 wherein n is an integer resulting in a PEG having a MW of 3400 or less; and m islto 100 (e.g., 4 to 20).

### SYNTHESIS OF CDP-C(O)-O-KOS-1584

### Method A : Directly attach linker to KOS-1584, separate mixture, deprotect and then couple to CDP

### Step 1: Synthesis of KOS-1584-ε-TROC-aminohexanoate (Scheme 18):

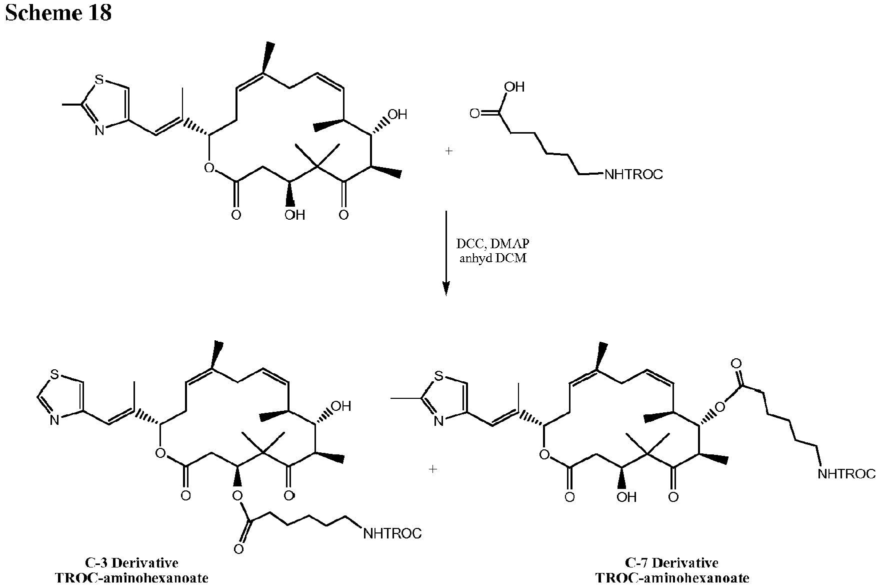

KOS-1584 (20 mg, 0.041 mmol) and ε-TROC-aminohexanoic acid (16.3 mg, 0.0585 mmol) will be dissolved in anhydrous DCM (10 mL) under N₂. To the resulting clear solution, DCC (13.4 mg, 0.065 mmol) and DMAP (7.9 mg, 0.065 mmol) will be added (**Scheme 18**). The reaction mixture will then be stirred for 12 h at room temperature. The solvent will subsequently be evaporated and the resulting residue dissolved in a minimum amount of chloroform. The desired C-3 and C-7 derivatives can be isolated via purification using flash column chromatography with chloroform/methanol as the mobile phase. The derivatives are to be analyzed by electron spray mass spectroscopy (m/z), HPLC and ¹H-NMR. The C-7 derivative of KOS-1584-ε-TROC-aminohexanoate is used as an example in the following synthetic steps.

### Step 2: Synthesis of KOS-1584-ε-aminohexanoate (Scheme 19):

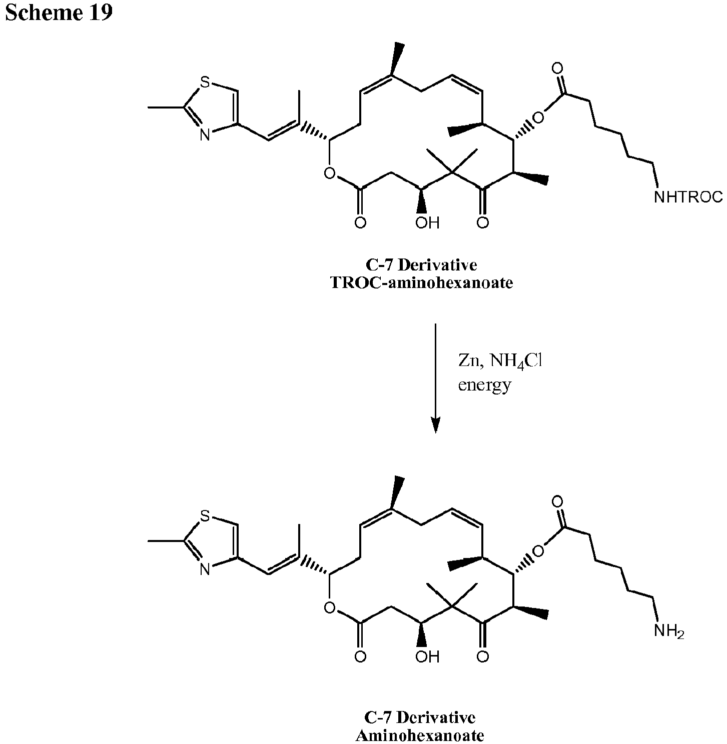

The C-7 derivative of KOS-1584-ε-TROC-aminohexanoate (15 mg, 0.019 mmol) and ammonium chloride (103 mg, 1.93 mmol) will be combined and mixed in 3 ml of water. While stirring vigorously, Zn powder (101 mg, 1.54 mmol) will be added with the input of energy (e.g., heat, sonication, microwave or ultraviolet irradiation) (Martin et al. (2000) Angewandte Chemie International Edition, 39 (3), 581-583) and stirred for an additional 20 min. The resulting solution will be filtered to remove zinc oxide and washed with hot water. The product will be extracted in dichloromethane and dried over MgSO₄. Evaporation of the organic solvent will be followed by purification of the resulting product via flash chromatography. The purified product will then be analyzed by electron spray mass spectroscopy (m/z), HPLC and ¹H-NMR.

### Step 3: Synthesis of CDP-C(O)-O-KOS-1584 (Scheme 20):

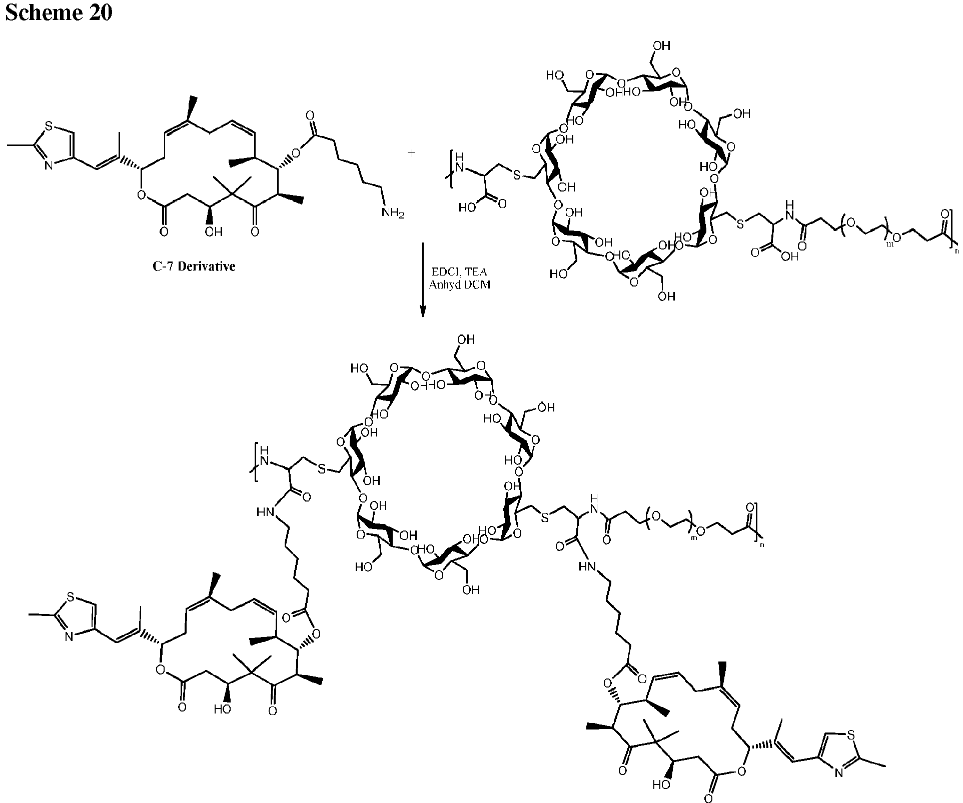

wherein n is an integer resulting in a PEG having a MW of 3400 or less; and m is 1 to 100 (e.g., 4 to 20).

CDP-COOH (50 mg, 0.011 mmol) will be dissolved in MeOH (2.0 mL). The C-7 derivative of KOS-1584-ε-aminohexanoate (14.3 mg, 0.024 mmol) will subsequently be added to the mixture and stirred for a few minutes to obtain a clear solution. EDCI (6.1 mg, 0.032 mmol) and TEA (3.8 mg, 0.038 mmol) will be added and the reaction stirred at ambient temperature for 3 h (**Scheme 20**). The resulting reaction mixture will be reduced to 0.1 mL of solution and precipitated in Et₂O (1.5 mL). The polymer conjugate will be redissolved in DMF (0.1 mL) and added to acetone (1.5 mL) to precipitate out the polymer conjugate. The polymer conjugate will then be washed with acetone (1 mL) twice, dissolved in nanopure water (3 mL) and then filtered through a 0.2 µm filter membrane and lyophilized to afford CDP-C(O)-O-KOS-1584. Loading will be determined by UV/Vis spectrometry with a standard curve and the particle size will be determined by zetasizer.

### Method B: Selectively protect with Silyl protecting group, addition of linker, followed by deprotection and then conjugation with CDP

### Step 1: Synthesis of 3-tert-butyldimethylsilyl KOS-1584 or 7-tert-butyldimethylsilyl KOS-1584 (Scheme 21):

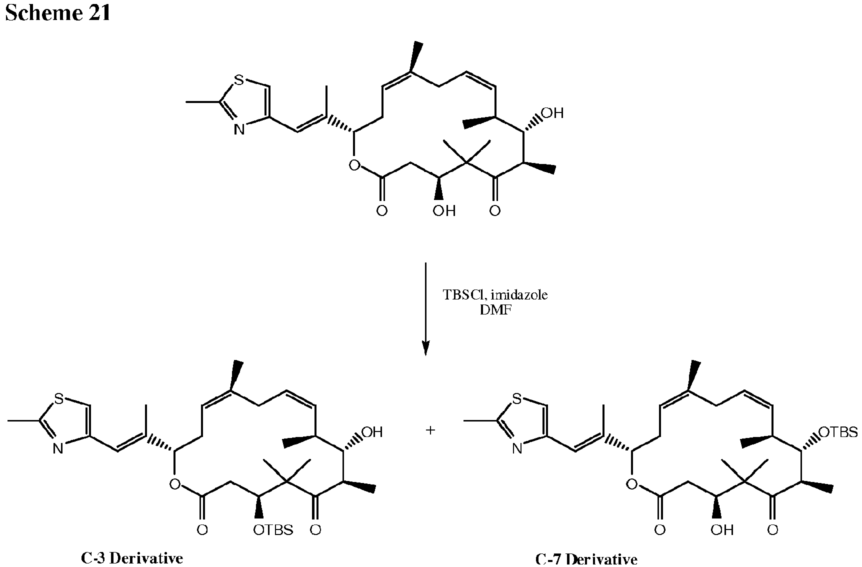

KOS-1584 (20 mg, 0.041 mmol) and tert-butyldimethylsilyl chloride (8.3 mg, 0.055 mmol) will be mixed in anhydrous DMF (5 mL) under N₂ atm (Trichloroethoxy chloroformate, TROC or any other bulky protecting group can be used instead to provide selective protection of OH group). To the resulting clear solution, imidazole (10.7 mg, 0.158 mmol) will be added (**Scheme 21**) and the reaction will be allowed to stir at ambient temperature for 24 h. The solvent will be evaporated and the residue dissolved in a minimum amount of chloroform. The desired C-3 and C-7 derivatives will be isolated following purification of the crude product via flash column chromatography with chloroform/methanol as the mobile phase. The derivatives will be analyzed by electron spray mass spectroscopy (m/z), HPLC and ¹H-NMR. C-7 derivative of TBS-KOS-1584 is used as an example in the following synthetic steps.

### Step 2: Synthesis of 7-(tert-butyldimethylsilyl)-3-(TROC-aminohexanoate)-KOS-1584 (Scheme 22):

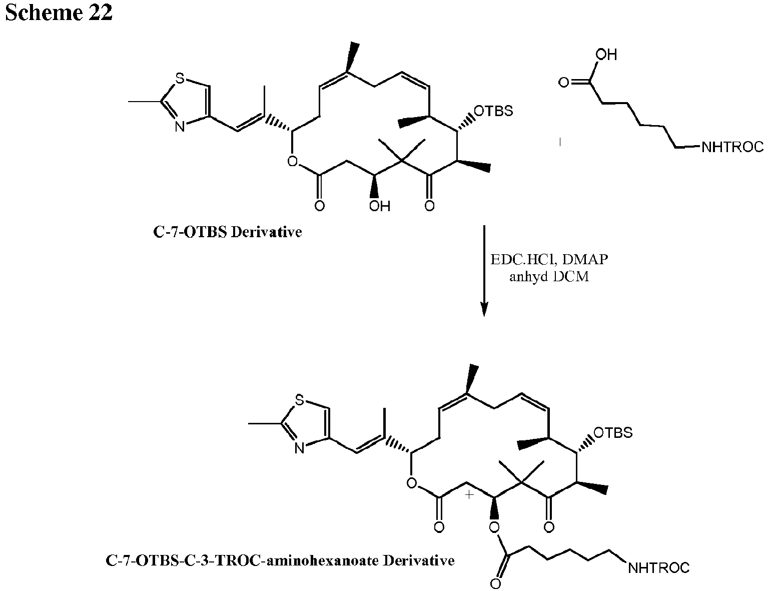

7-*tert*-butyidimethylsilyl KOS-1584 (20 mg, 0.032 mmol) and ε-TROC-aminohexanoic acid (12.0 mg, 0.039 mmol) will be stirred together in anhydrous DCM (2 mL) under N₂. To the resulting clear solution, EDC.HCl (11.1 mg, 0.058 mmol) and DMAP (7.08 mg, 0.058 mmol) will be added (**Scheme 22**). The reaction mixture will then be stirred for 12 h at 22 °C. The solvent is subsequently evaporated and the resulting residue dissolved in a minimum amount of chloroform. The crude product will be purified via flash column chromatography with chloroform/methanol as the mobile phase. The product will be analyzed by electron spray mass spectroscopy (m/z), HPLC and ¹H-NMR.

### Step 3: Synthesis of 3-(aminohexanoate)-KOS-1584 (Scheme 23):

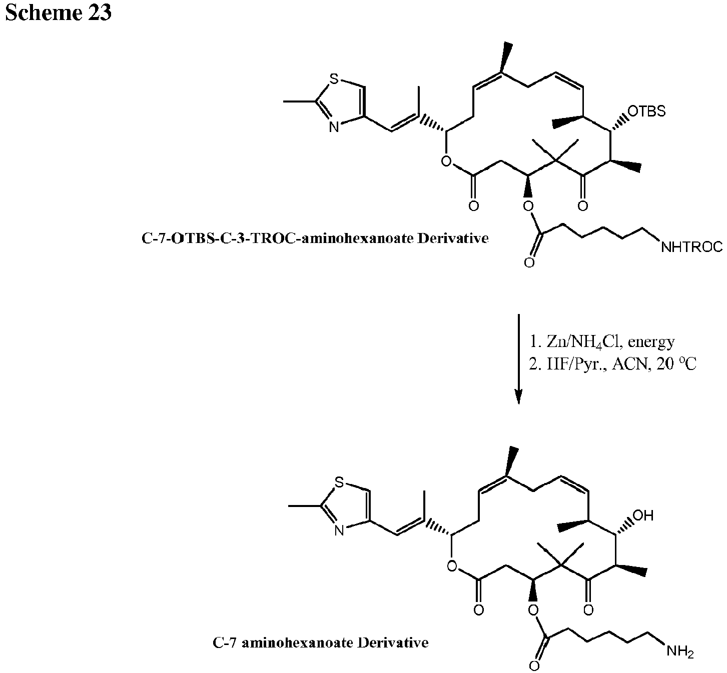

7-(*tert*-butyldimethylsilyl)-3-(TROC-aminohexanoate)-KOS-1584 will be deprotected using Zn/NH₄Cl with the input of energy (e.g., heat, sonication, microwave or ultraviolet irradiation), followed by a solution of acetonitrile and HF/Pyridine. The final product will be purified via flash column chromatography with chloroform/methanol as the mobile phase. 3-(aminohexanoate)-KOS-1584 will be analyzed by electron spray mass spectroscopy (m/z), HPLC and ¹H-NMR.

### Step 4: Synthesis of CDP-C(O)-O-KOS-1584 (Scheme 24):

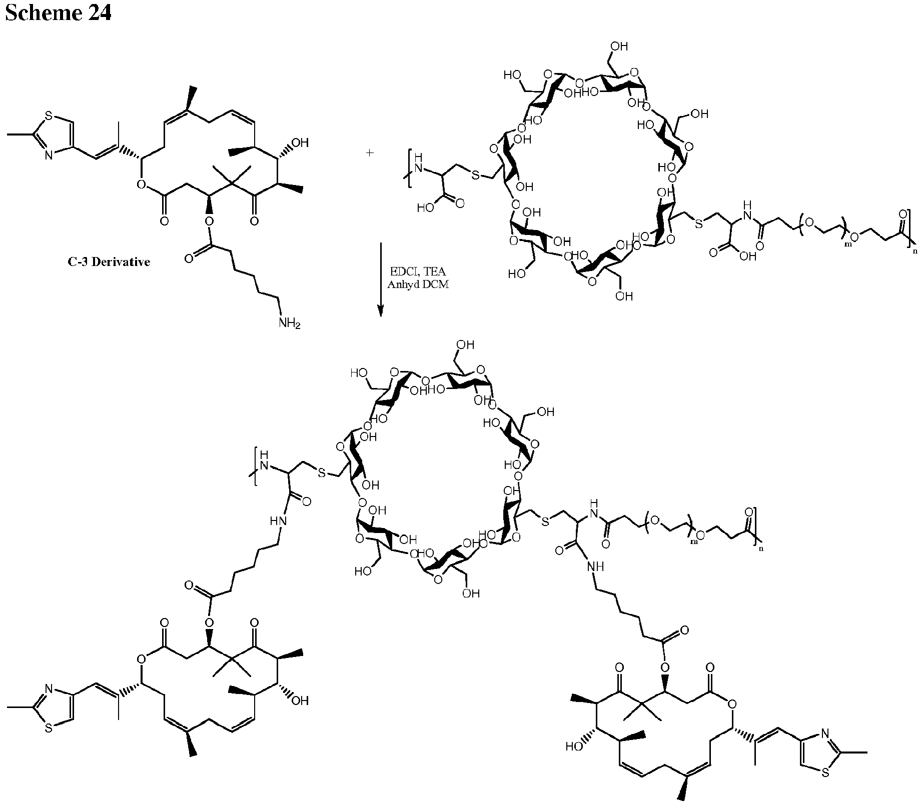

wherein n is an integer resulting in a PEG having a MW of 3400 or less; and m islto 100 (e.g., 4 to 20).

CDP-COOH (50 mg, 0.011 mmol) will be dissolved in MeOH (2.0 mL). A C-3 derivative of KOS-1584-ε-aminohexanoate (14.3 mg, 0.024 mmol) will subsequently be added to the mixture and stirred for a few minutes to obtain a clear solution. EDCI (6.1 mg, 0.032 mmol) and TEA (3.8 mg, 0.038 mmol) will then be added and the reaction stirred at ambient temperature for 3 h (**Scheme 24**). The reaction mixture will be reduced to 0.1 mL of solution and precipitated in Et₂O (1.5 mL). The polymer conjugate will be redissolved in DMF (0.1 mL) and added to acetone (1.5 mL) to precipitate out the polymer conjugate. The polymer conjugate will be washed with acetone (1 mL) twice, dissolved in nanopure water (3 mL) and then filtered through a 0.2 µm filter membrane and lyophilized to afford CDP-C(O)-O-KOS-1584. Loading will be determined by UV/Vis spectrometry with a standard curve and the particle size will be determined by zetasizer.

### SYNTHESIS OF CDP-AMIDE-EPOTHILONE B

### Method of Synthesizing CDP-amide-Epothilone B

CDP (1.0 g, 0.21 mmol) will be dissolved in dry N,N-dimethylformamide (DMF, 20 mL). Epothilone B analog (250 mg, 0.46 mmol), N,N-Diisopropylethylamine (0.080 mL, 0.46 mmol), N-(3-Dimethylaminopropyl)-N'-ethylcarbodiimide hydrochloride (120 mg, 0.62 mmol), and N-Hydroxysuccinimide (52 mg, 0.46 mmol) will then be added to the polymer solution and stirred for 4 h. The polymer will be precipitated with ethylacetate (100 mL) and then rinsed with acetone (50 mL). The precipitate will be dissolved in pH3 water (100 mL) which is prepared by acidification with HCl. The solution will be dialyzed using 25,000 MWCO membrane (Spectra/Por 7) for 24 h at pH3 water and filtered through 0.2 µm filters (Nalgene) and lyophilized to yield a white solid (**Scheme 25**) 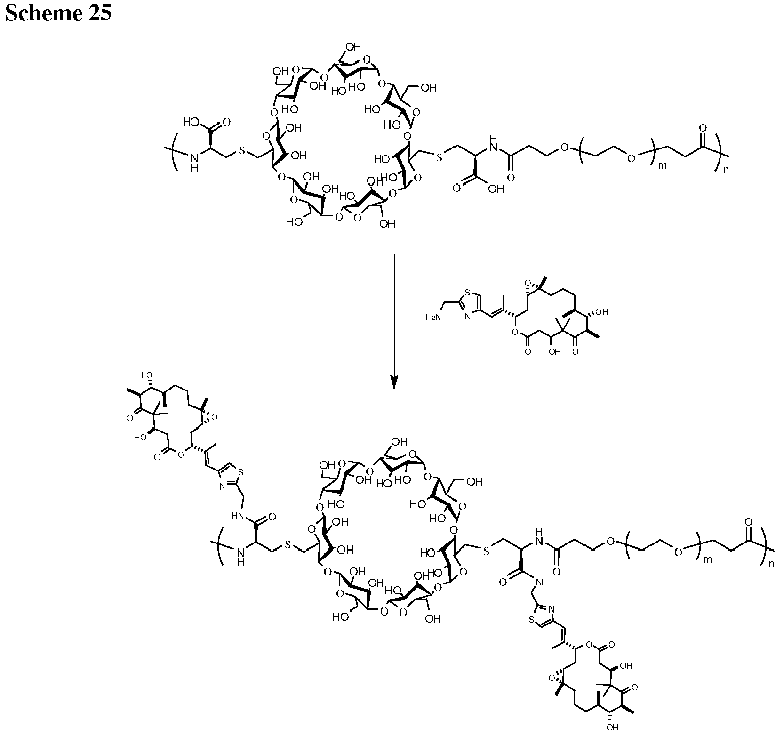 wherein n is an integer resulting in a PEG having a MW of 3400 or less; and m islto 100 (e.g., 4 to 20).

### SYNTHESIS OF CDP-C(O)-O-SAGOPILONE

### Method A : Directly attach linker to Sagopilone, separate mixture, deprotect and then couple to CDP

### Step 1: Synthesis of Sagopilone-ε-TROC-aminohexanoate (Scheme 26):

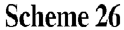

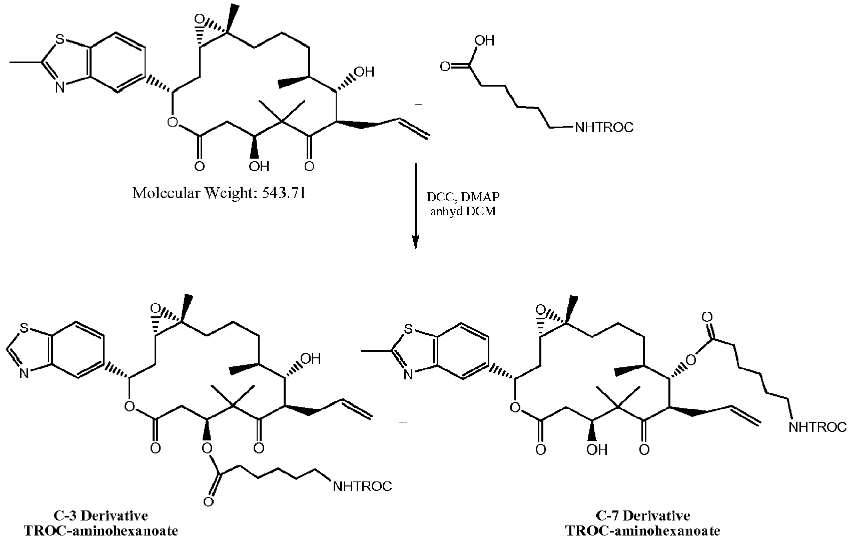

Sagopilone (20 mg, 0.037 mmol) and ε-TROC-aminohexanoic acid (16.3 mg, 0.0585 mmol) will be dissolved in anhydrous DCM (10 mL) under N₂. To the resulting clear solution, DCC (13.4 mg, 0.065 mmol) and DMAP (7.9 mg, 0.065 mmol) will be added (**Scheme 26**). The reaction mixture will then be stirred for 12 h at room temperature. The solvent will subsequently be evaporated and the resulting residue dissolved in a minimum amount of chloroform. The desired C-3 and C-7 derivatives can be isolated via purification using flash column chromatography with chloroform/methanol as the mobile phase. The derivatives are to be analyzed by electron spray mass spectroscopy (m/z), HPLC and ¹H-NMR. The C-7 derivative of Sagopilone-e-TROC-aminohexanoate is used as an example in the following synthetic steps.

### Step 2: Synthesis of Sagopilone-ε-aminohexanoate (Scheme 27):

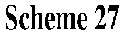

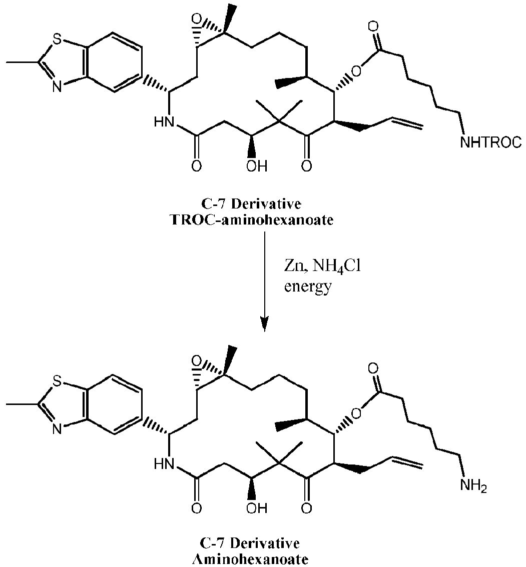

The C-7 derivative of Sagopilone-ε-TROC-aminohexanoate (15 mg, 0.018 mmol) and ammonium chloride (100 mg, 1.88 mmol) will be combined and mixed in 3 ml of water. While stirring vigorously, Zn powder (98 mg, 1.51 mmol) will be added with the input of energy (e.g., heat, sonication, microwave or ultraviolet irradiation (Martin et al. (2000) Angewandte Chemie International Edition, 39 (3), 581-583) and stirred for an additional 20 min. The resulting solution will be filtered to remove zinc oxide and washed with hot water. The product will be extracted in dichloromethane and dried over MgSO₄. Evaporation of the organic solvent will be followed by purification of the resulting product via flash chromatography. The purified product will then be analyzed by electron spray mass spectroscopy (m/z), HPLC and ¹H-NMR.

### Step 3: Synthesis of CDP-C(O)-O-Sagopilone (Scheme 28):

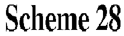

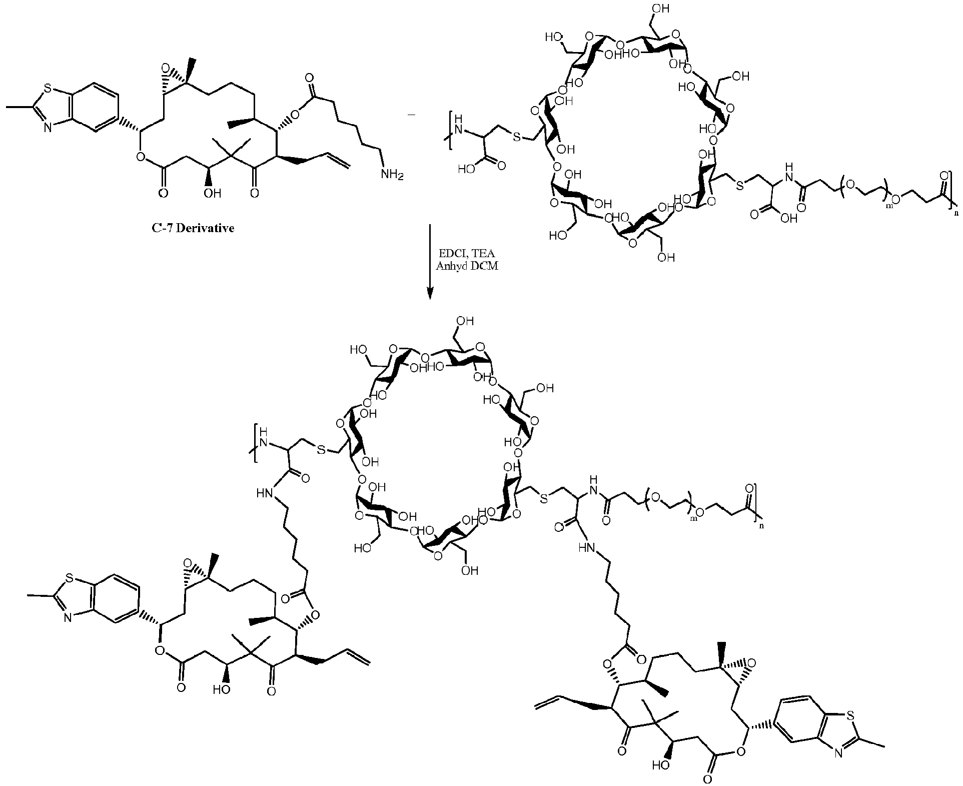

wherein n is an integer resulting in a PEG having a MW of 3400 or less; and m islto 100 (e.g., 4 to 20).

CDP-COOH (50 mg, 0.011 mmol) will be dissolved in MeOH (2.0 mL). The C-7 derivative of Sagopilone-ε-aminohexanoate (15.6 mg, 0.024 mmol) will subsequently be added to the mixture and stirred for a few minutes to obtain a clear solution. EDCI (6.1 mg, 0.032 mmol) and TEA (3.8 mg, 0.038 mmol) will be added and the reaction stirred at ambient temperature for 3 h (**Scheme 28**). The resulting reaction mixture will be reduced to 0.1 mL of solution and precipitated in Et₂O (1.5 mL). The polymer conjugate will be redissolved in DMF (0.1 mL) and added to acetone (1.5 mL) to precipitate out the polymer conjugate. The polymer conjugate will then be washed with acetone (1 mL) twice, dissolved in nanopure water (3 mL) and then filtered through a 0.2 µm filter membrane and lyophilized to afford CDP-C(O)-O-Sagopilone. Loading will be determined by UV/Vis spectrometry with a standard curve. The particle size is determined by zetasizer.

### Method B: Selectively protect with Silyl protecting group, addition of linker, followed by deprotection and then conjugation with CDP

### Step 1: Synthesis of 3-tert-butyldimethylsilyl Sagopilone or 7-tert-butyldimethylsilyl Sagopilone (Scheme 29):

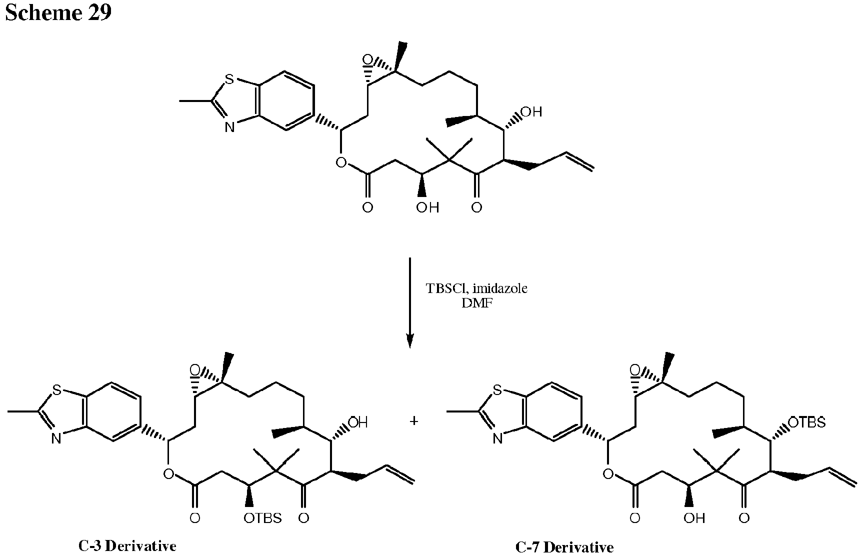

Sagopilone (20 mg, 0.037 mmol) and tert-butyldimethylsilyl chloride (8.3 mg, 0.055 mmol) will be mixed in anhydrous DMF (5 mL) under N₂ atm (Trichloroethoxy chloroformate, TROC, or any other bulky protecting group can be used instead to provide selective protection of OH group). To the resulting clear solution, imidazole (10.7 mg, 0.158 mmol) will be added (Scheme 4) and the reaction will be allowed to stir at ambient temperature for 24 h. The solvent will be evaporated and the residue dissolved in a minimum amount of chloroform. The desired C-3 and C-7 derivatives will be isolated following purification of the crude product via flash column chromatography with chloroform/methanol as the mobile phase. The derivatives will be analyzed by electron spray mass spectroscopy (m/z), HPLC and ¹H-NMR. The C-7 derivative of TBS-Sagopilone is used as an example in the following synthetic steps.

### Step 2: Synthesis of 7-(tert-butyldimethylsilyl)-3-(TROC-aminohexanoante)-Sagopilone (Scheme 30):

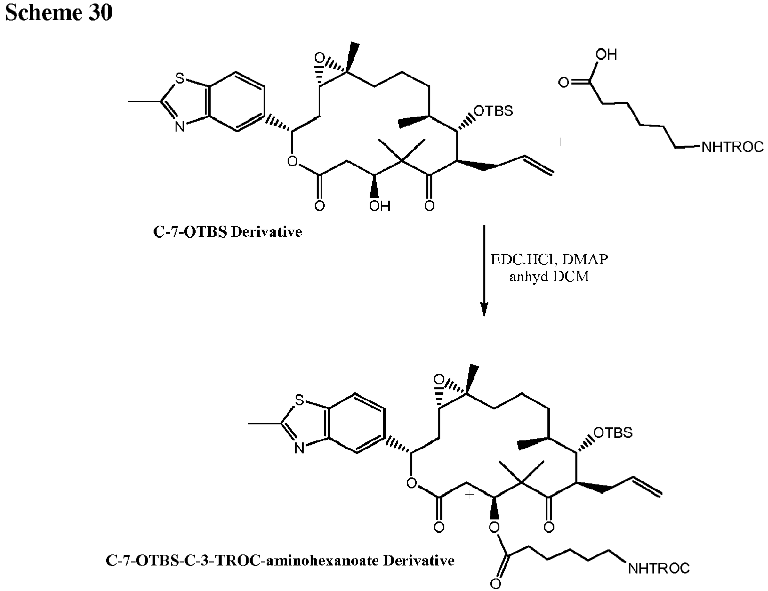

**7-*tert*-butyldimethylsilyl** Sagopilone (20 mg, 0.030 mmol) and ε-TROC-aminohexanoic acid (12.0 mg, 0.039 mmol) will be stirred together in anhydrous DCM (2 mL) under N₂. To the resulting clear solution, EDC.HCl (11.1 mg, 0.058 mmol) and DMAP (7.08 mg, 0.058 mmol) will be added (**Scheme 30**). The reaction mixture is then stirred for 12 h at 22 °C. The solvent is subsequently evaporated and the resulting residue dissolved in a minimum amount of chloroform. The crude product will be purified via flash column chromatography with chloroform/methanol as the mobile phase. The product will be analyzed by electron spray mass spectroscopy (m/z), HPLC and ¹H-NMR.

### Step 3: Synthesis of 3-(aminohexanoate)-Sagopilone (Scheme 31):

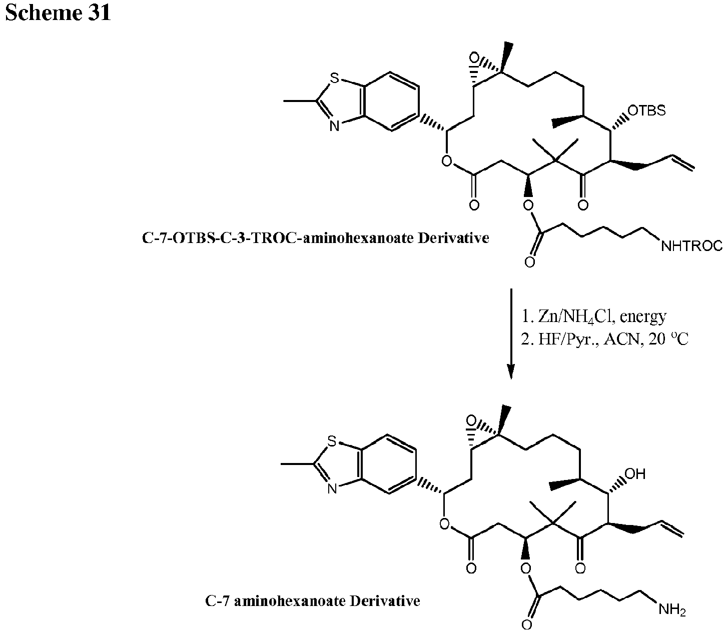

7-(*tert*-butyldimethylsilyl)-3-(TROC-aminohexan)-Sagopilone-oate will be deprotected using Zn/NH₄Cl with the input of energy (e.g., heat, sonication, microwave or ultraviolet irradiation), followed by a solution of acetonitrile and HF/Pyridine. The final product will be purified via flash column chromatography with chloroform/methanol as the mobile phase. 3-(aminohexan)-Sagopilone-oate will be analyzed by electron spray mass spectroscopy (m/z), HPLC and ¹H-NMR.

### Step 4: Synthesis of Poly-CD-Hex-C(O)-O-Sagopilone (CDP-C(O)-O-Sagopilone) (Scheme 32):

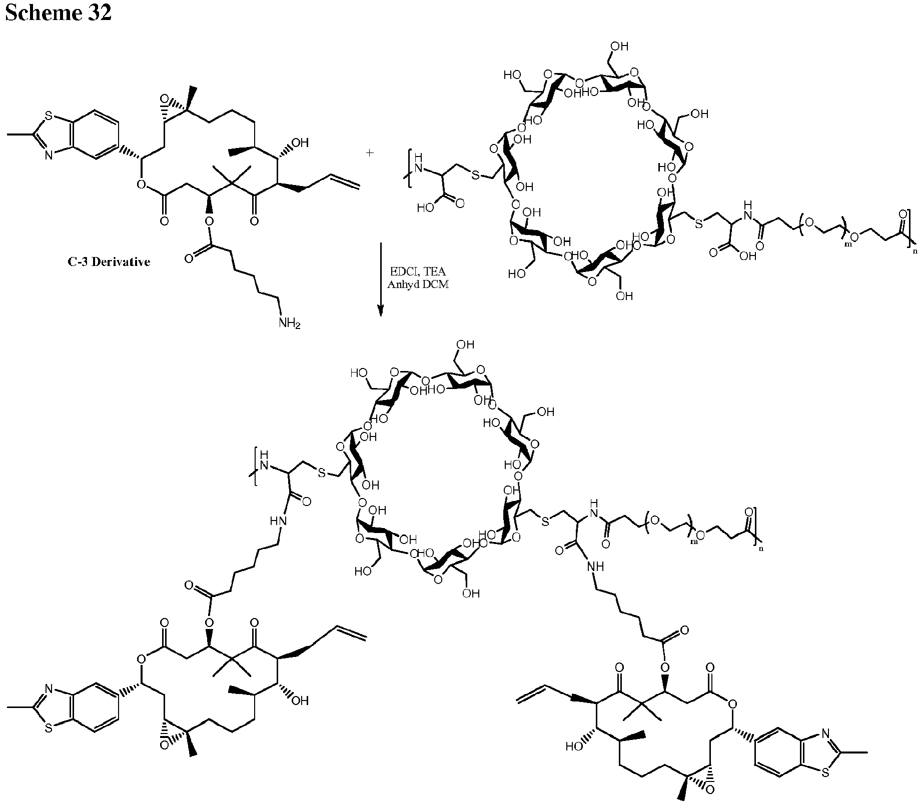

wherein n is an integer resulting in a PEG having a MW of 3400 or less; and m islto 100 (e.g., 4 to 20).

CDP-COOH (50 mg, 0.011 mmol) will be dissolved in MeOH (2.0 mL). A C-3 derivative of Sagopilone-ε-aminohexanoate (15.5 mg, 0.024 mmol) will subsequently be added to the mixture and stirred for a few minutes to obtain a clear solution. EDCI (6.1 mg, 0.032 mmol) and TEA (3.8 mg, 0.038 mmol) are then added and the reaction stirred at ambient temperature for 3 h (**Scheme 32**). The reaction mixture will be reduced to 0.1 mL of solution and precipitated in Et₂O (1.5 mL). The polymer conjugate will be redissolved in DMF (0.1 mL) and added to acetone (1.5 mL) to precipitate out the polymer conjugate. The polymer conjugate will be washed with acetone (1 mL) twice, dissolved in nanopure water (3 mL) and then filtered through a 0.2 µm filter membrane and lyophilized to afford CDP-C(O)-O-Sagopilone. Loading will be determined by UV/Vis spectrometry with a standard curve. The particle size will be determined by zetasizer.

### SYNTHESIS OF CDP-SS-IXABEPILONE (CARBONATE)

**Synthesis of CDP-SS-Py** A mixture of CDP, (67 kD, 2.0 g, 0.41 mmole), pyridine dithioethylamine hydrochloric salt (180 mg, 0.83 mmole), N-(3-dimethylaminopropyl)-N'-ethylcarbodiimide hydrochloride (EDCI, 240 mg, 1.2 mmole), and N-hydroxysuccinimide (NHS, 95 mg, 0.83 mmole) will be dissolved in anhydrous N,N-dimethylformamide (DMF, 20 mL) and followed by addition of anhydrous N,N-diisopropylethylamine (DIEA, 0.14 mL, 0.83 mmole). The reaction mixture will be stirred under argon at room temperature for 4 h. The mixture will then be added to ethyl acetate (EtOAc, 100 mL) to precipitate the polymer. In order to clean up the polymer further without dialysis, multiple crashouts will be carried out to purify the polymer. The Polymer will be dissolved back in methanol (MeOH, 20 mL) and precipitated in diethyl ether (Et₂O, 100 mL). Purification by reprecipitation will be carried out twice. The polymer will then be dried under vacuum to yield a white solid (**Scheme 33**).

### Synthesis of CDP-SH

CDP-SS-Py (200 mg, 0.042 mmol) will be redissolved in MeOH (2 mL). Dithiothreitol (DTT, 130 mg, 0.83 mmol) will be added to the reaction mixture and stirred for 1 h (**Scheme 33**). It will then be precipitated in Et₂O (20 mL). The polymer will be purified by multiple reprecipitation. It will be dissolved in MeOH (2 mL) and precipitated in Et₂O (20 mL). This process will be repeated twice. The polymer will be dried under vacuum to yield a white solid.

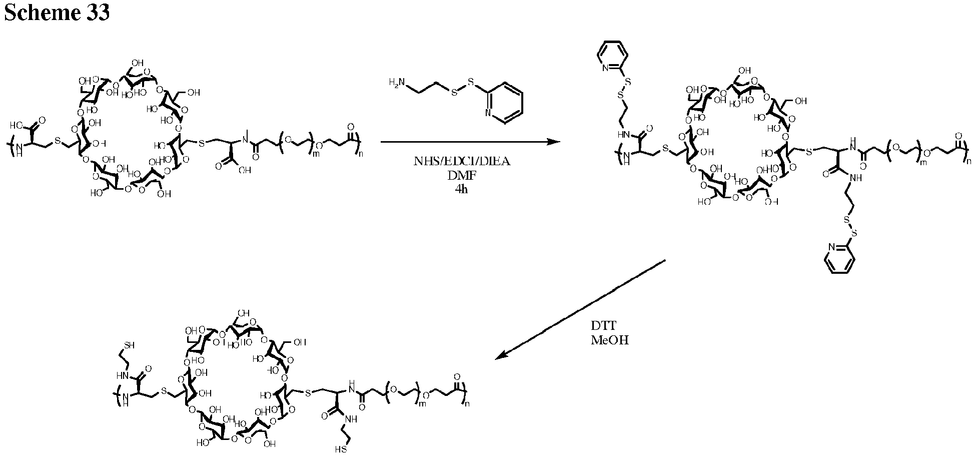

### Synthesis of pyridin-2-yldisulfanyl ethyl ester derivative of Ixabepilone

Ixabepilone (5 mg, 0.0099 mmol) will be in dichloromethane (CH₂Cl₂, 1.5 mL). Triethylamine (TEA, 5.6 µL, 0.040 mmol) and 20% phosgene in toluene (9.8 µL, 0.020 mmol) will be added to the mixture and stirred for ½ h. The mixture will be purged with Arto remove any excess phosgene. Pyridine dithioethanol (3.7 mg, 0.020 mmole), 4-dimethylaminopyridine (DMAP, 1.2 mg, 0.0099 mmol) and TEA (2.8 µL, 0.020 mmol) will be added and stirred for an additional one hour (**Scheme 34**). It will then be pumped down to dryness and purified by flash column chromatography with dichloromethane and methanol (9:1) ratio to yield a white solid.

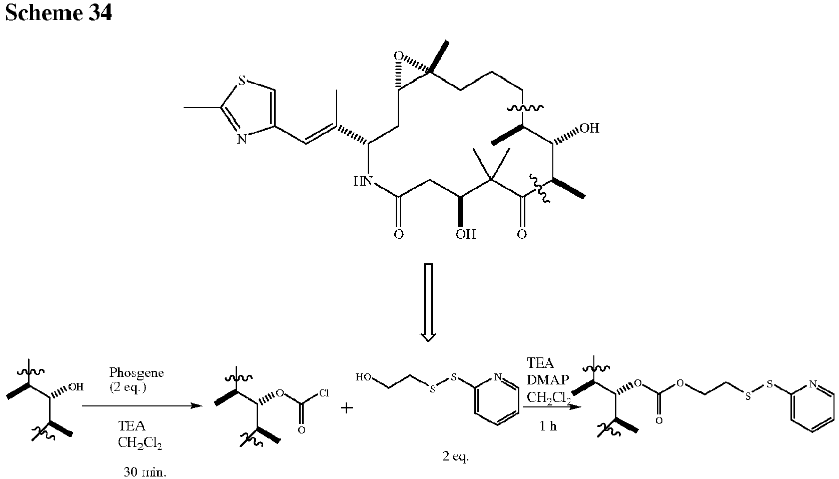

**Synthesis of CDP-SS-Ixabepilone.** CDP-SH (32 mg, 0.0070 mmole) will be dissolved in MeOH (1.0 mL). Pyridin-2-yldisulfanyl ethyl ester derivative of Ixabepilone (5 mg, 0.070 mmol) will be added to the mixture and stirred for 1 h. N-ethyl maleimide (NEM, 8.7 mg, 0.070 mmole) will then be added to quench the reaction and stirred for an additional hour (**Scheme 35**). The reaction mixture will be reduced to 0.1 mL of solution and subsequently precipitated in Et₂O (1 mL). The polymer conjugate will be redissolved in DMF (0.1 mL) and added to acetone (1 mL) to precipitate out the polymer conjugate. The polymer conjugate will be washed with acetone (1 mL) twice. It will be dissolved in nanopure water (3 mL) and then filtered through 0.2 µm filter membrane and lyophilized to afford CDP-Ixabepilone. In instances where a mixture of isomers is formed (e.g., acylation at the 3- and/or 7-position), the isomeric products can be separated (e.g., using flash chromatography). 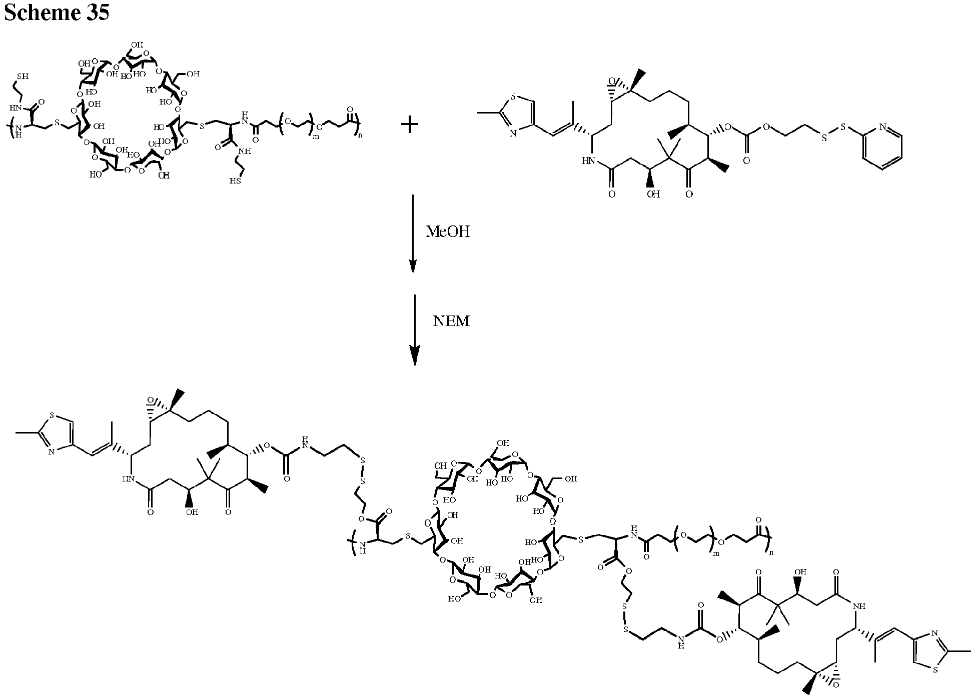 wherein n is an integer resulting in a PEG having a MW of 3400 or less; and m islto 100 (e.g., 4 to 20).

### SYNTHESIS OF CDP-SS-IXABEPILONE (CARBAMATE)

**Synthesis of CDP-SS-Py** A mixture of CDP, (67 kD, 2.0 g, 0.41 mmole), pyridine dithioethylamine hydrochloric salt (180 mg, 0.83 mmole), N-(3-dimethylaminopropyl)-N'-ethylcarbodiimide hydrochloride (EDCI, 240 mg, 1.2 mmole), and N-hydroxysuccinimide (NHS, 95 mg, 0.83 mmole) were dissolved in anhydrous N,N-dimethylformamide (DMF, 20 mL) and followed by addition of anhydrous N,N-diisopropylethylamine (DIEA, 0.14 mL, 0.83 mmole). The reaction mixture was stirred under argon at room temperature for 4 h. The mixture was then added to ethyl acetate (EtOAc, 100 mL) to precipitate the polymer. In order to clean up the polymer further without dialysis, multiple crashouts were carried out. The polymer was dissolved back in methanol (MeOH, 20 mL) and precipitated in diethyl ether (Et₂O, 100 mL). Purification by reprecipitation was carried out twice. The polymer was then dried under vacuum to yield a white solid (**Scheme 36**).

### Synthesis of CDP-SH

CDP-SS-Py (200 mg, 0.042 mmol) was redissolved in MeOH (2 mL). Dithiothreitol (DTT, 130 mg, 0.83 mmol) was added to the reaction mixture and stirred for 1 h (**Scheme 36**). It was then precipitated in Et₂O (20 mL). The polymer was purified by multiple reprecipitation. It was dissolved in MeOH (2 mL) and precipitated in Et₂O (20 mL) twice. The polymer was dried under vacuum to yield a white solid. 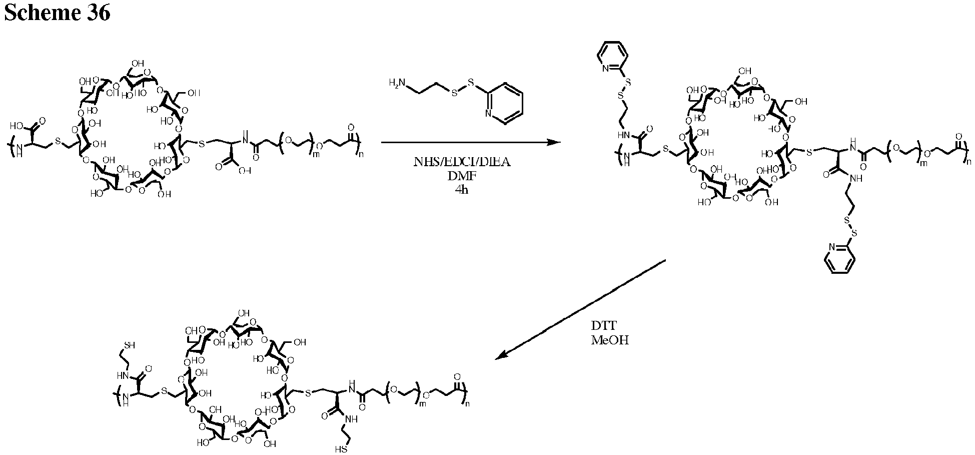 wherein n is an integer resulting in a PEG having a MW of 3400 or less; and m islto 100 (e.g., 4 to 20).

### Synthesis of pyridin-2-yldisulfanyl ethyl amide derivative of Ixabepilone

Ixabepilone (5 mg, 0.0099 mmol) was dissolved in dichloromethane (CH₂Cl₂, 1.5 mL). Triethylamine (TEA, 5.6 µL, 0.040 mmol) and 20% phosgene in toluene (9.8 µL, 0.020 mmol) were added to the mixture and stirred for ½ h. The mixture was purged with Ar to remove any excess phosgene. Pyridine dithioethylamine hydrochloric salt (3.7 mg, 0.020 mmole) and DIEA (2.8 µ, 0.020 mmole) were added and stirred for an additional hour (**Scheme 37**). It was then pumped down to dryness and purified by flash column chromatography with dichloromethane and methanol (9:1) to yield a white solid (5.2 mg, 49% Yield). It was confirmed by electron spray mass spectrometry (m/z expected 718.99; Found 741.48 M + Na).

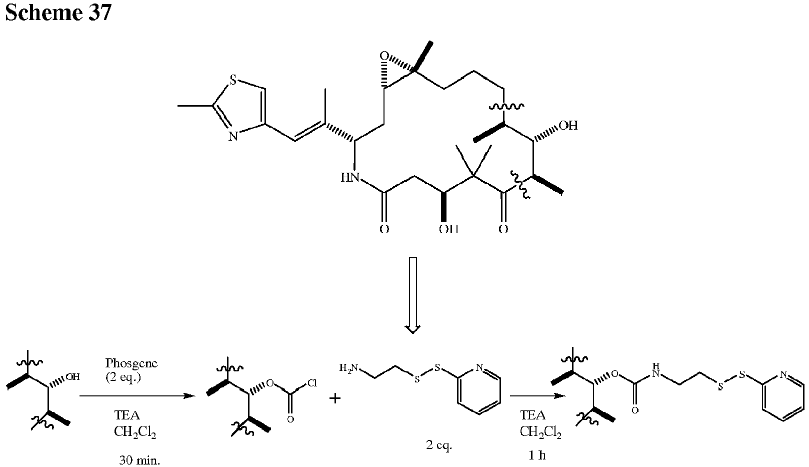

**Synthesis of CDP-SS-Ixabepilone.** CDP-SH (32 mg, 0.0070 mmole) was dissolved in MeOH (1.0 mL). Pyridin-2-yldisulfanyl ethyl amide derivative of Ixabepilone (5 mg, 0.070 mmol) was added to the mixture and stirred for 1 h. N-ethyl maleimide (NEM, 8.7 mg, 0.070 mmole) was then added to quench the reaction and stirred for an additional hour (**Scheme 38**). The reaction mixture was reduced to 0.1 mL of solution and precipitated in Et₂O (1 mL). The polymer conjugate was redissolved in DMF (0.1 mL) and added to acetone (1 mL) to precipitate out the polymer conjugate. The polymer conjugate was washed with acetone (1 mL) twice. It was dissolved in nanopure water (3 mL) and then filtered through a 0.2 µm filter membrane and lyophilized to afford CDP-Ixabepilone (19 mg, 58% Yield). Loading was determined to be 11.2% w/w by UV/Vis spectrometry with standard curve. The particle size is determined to be 49.0 nm. In instances where a mixture of isomers is formed (e.g., acylation at the 3- and/or 7-position), the isomeric products are separated (e.g., using flash chromatography). 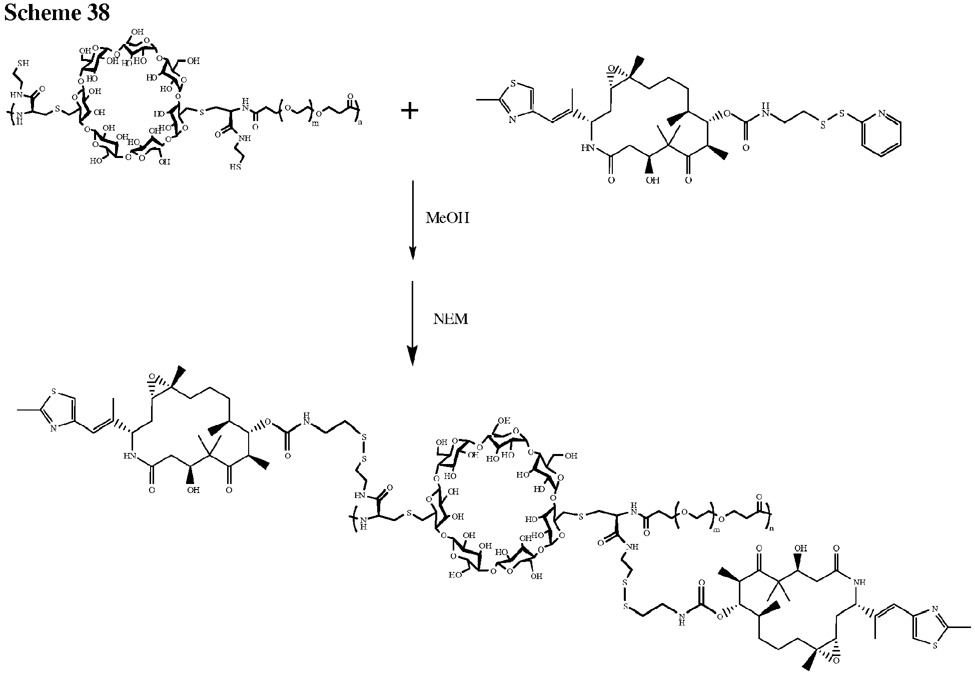 wherein n is an integer resulting in a PEG having a MW of 3400 or less; and m islto 100 (e.g., 4 to 20).

### Example 10: Synthesis of Various CDP-Proteasome Inhibitors

In all the relevant names and structures below, the terminology CDP_{0.5} indicates that up to 2 molecules of linker and/or linker conjugated to drug may be attached to each cyclodextrin unit of the CDP polymer with the number of cyclodextrin units determined by the overall MW of the CDP polymer.

Synthesis of CDP conjugate with (aminoethyl)(hydroxyethyl)amine based boronic acid - Conjugate of bortezomib with [(6-(CDP_{0.5}-carboxamidohexyl)-(2-methylaminoethyl)-(2-hydroxyethyl)]amine

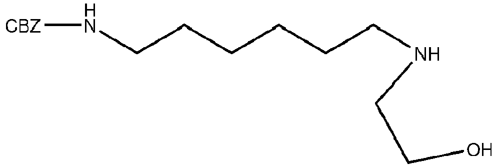

*Step 1: (6-Benzyloxycarbonylaminohexyl)(2-hydroxyethyl)amine:* In a manner similar to that described by Pellacini et al. (US Patent 6455576) the title compound will be prepared from 6-benzyloxycarbonylaminohexanol.

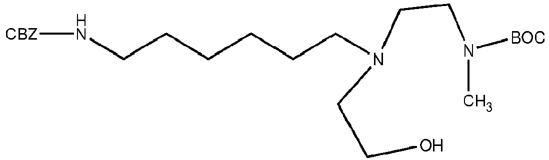

*Step 2: (6-Benzyloxycarbonylaminohexyl)-((2-t-butloxycarbonyl)methylaminoethyl)-(2-hydroxyethyl)amine:* In a manner similar to that described by Ackerman et al. (US Patent Appl. 2005065210) the title compound will be prepared from ((2-t-butoxycarbonyl)methylaminoethanol and (6-benzyloxycarbonylaminohexyl)(2-hydroxyethyl)amine (from Step 1).

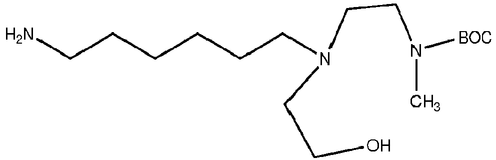

Step 3: *(6-Aminohexyl)-((2-benzyloxycarbonyl)methylaminoethyl)-(2-hydroxyethyl)amine:* (6-Benzyloxycarbonylaminohexyl)-((2-t-butoxycarbonyl)methylaminoethyl)-(2-hydroxyethyl)amine will be dissolved in MeOH (10 volumes). The mixture will stirred for 5 min to afford a clear solution. 5% Pd/C (200 mg, 50% moisture) will be charged. The flask will be evacuated for 1 min and then filled with H2 with a balloon. The reaction will be stirred at ambient temperature for 3 h or until the reaction is complete. The structure will be confirmed with LC/MS and 1H-NMR.

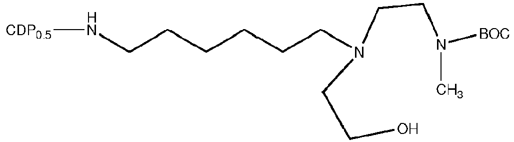

*Step 4: (6-(CDP_{0.5}-carboxamidohexyl)-((2-t-but oxycarbonyl)methylaminoethyl)-(2-hydroxyethyl)amine:* A 100-mL round-bottom flask will be charged with (6-aminohexyl)-((2-t-butoxycarbonyl)methylaminoethyl)-(2-hydroxyethyl)amine (2.0 mmol per estimated number of cyclodextrin units in the CDP polymer) and DMF (5 mL). The mixture will be stirred for 15 min to afford a clear solution. CDP (1 g) in DMF (20 mL) will be added and the mixture stirred for 10 min. EDC•HCl (2.3 mmol per estimated number of cyclodextrin units in the CDP polymer), DMAP (1.0 mmol per estimated number of cyclodextrin units in the CDP polymer), and TEA (5.0 mmol per estimated number of cyclodextrin units in the CDP polymer) will be added and the reaction stirred at ambient temperature for 6 h or until completion of the reaction. The reaction will be added into acetone or a mixture of acetone and diethylether or MTBE. The resulting precipitate will be isolated by filtration or decantation of the supernatant. The precipitate will then be dissolved in water and dialyzed for 3 days with a 25,000 Da MWCO. The lyophilized solution will be filtered through a 2 µM filter and the filtrate lyophilized to give the title product. The structure will be confirmed with 1H-NMR, HPLC and GPC.

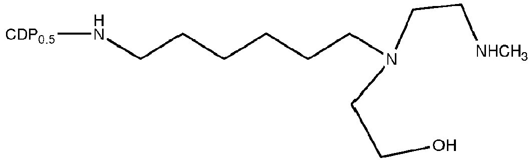

*Step 5: (6-(CDP_{0.5}-carboxamidohexyl)-(methylaminoethyl)-(2-hydroxyethyl)amine:* A round-bottom flask equipped with a magnetic stirrer will be charged with (6-(CDP_{0.5} - carboxamidohexyl)-((2-t-butoxycarbonyl)methylaminoethyl)-(2-hydroxyethyl)amine in CH2C12 (5 volumes). To this will be added TFA (5 volumes). The reaction will be stirred at ambient temperature for 3 h or until the reaction is complete. The reaction will be added into acetone or a mixture of acetone and diethylether or MTBE. The resulting precipitate will be isolated by filtration or decantation of the supernatant. The precipitate will then be dissolved in water and dialyzed for 3 days with a 25,000 Da MWCO. The lyophilized solution will be filtered through a 2 µM filter and the filtrate lyophilized to give the title product. The structure will be confirmed with 1H-NMR, HPLC and GPC.

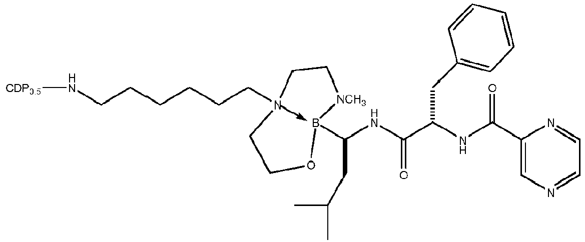

*Step 6: Conjugate of bortezomib with (6-(CDP_{0.5}-carboxamidohexyl)-(methylaminoethyl)-(2-hydroxyethyl)amine:* In a manner similar to that described by Hebel et al. (J. Org. Chem. 2002, 67, 9452) bortezomib (2.0 mmol per estimated number of cyclodextrin units in the CDP polymer) will be dissolved in DMF and treated with a solution of (6-(CDP_{0.5}-carboxamidohexyl)-(methylaminoethyl)-(2-hydroxyethyl)amine (1 g) in DMF and 4 Å MS. After 6 h at room temperature, the reaction mixture will be added into acetone or a mixture of acetone and diethylether or MTBE. The resulting precipitate will be isolated by filtration or decantation of the supernatant. The precipitate will then be dissolved in water and dialyzed for 3 days with a 25,000 Da MWCO. The lyophilized solution will be filtered through a 2 µM filter and the filtrate lyophilized to give the title product. The structure will be confirmed with 1H-NMR, HPLC and GPC.

Synthesis of CDP conjugate with 1,2-amino alcohol based boronic acid - Conjugate of bortezomib with (8-(CDP_{0.5}-carboxamido)-2-hydroxy-2-methyl-1-methylaminooctane

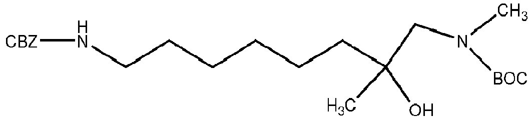

*Step 1:(8-(benzylocycarbonylamino)-2-hydrocy-2-methyl-1-((t-butoxycarbonyl)methylamino)octane:* In the manner described by Ortiz et al. (Tetrahedron 1999, 55, 4831) the title compound will be prepared from 8-benzyloxycarbonylamino-2-octanone. The structure will be confirmed with 1H-NMR and LC/MS.

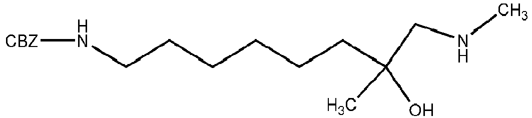

*Step 2: (8-(Benzyloxycarbonylamino)-2-hydroxy-2-methyl-1-(methylamino)octane:* (8-(benzyloxycarbonylamino)-2-hydroxy-2-methyl-1-((t-butoxycarbonyl)methylamino)octane will be dissolved 4N HCl in dioxane. After approximately 1 h, the solvents will be evaporated to dryness to give the product as its hydrochloride salt. The structure will be confirmed with LC/MS and 1H-NMR.

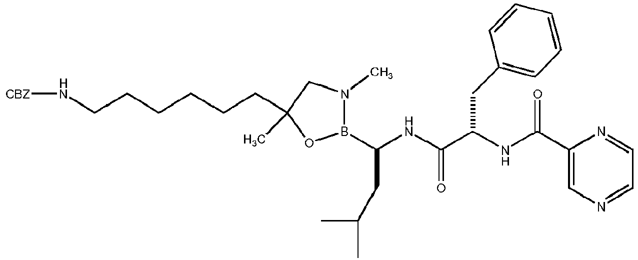

*Step 3: Conjugate of bortezomib (8-(benzyloxycarbonylamino)-2-hydroxy-2-methyl-1-(methylamino)octane:* In a manner similar to that described by Hebel et al. (J. Org. Chem. 2002, 67, 9452) bortezomib (1.0 mmol) will be dissolved in DMF and treated with a solution of (8-(benzyloxycarbonylamino)-2-hydroxy-2-methyl-1-(methylamino)octane (1.0 mmol) in DMF and 4 Å MS. After 6 h at room temperature, the reaction mixture will be added into in MTBE (30 mL) over 0.5 h with overhead stirring. The suspension will be stirred for another 0.5 h and filtered through a PP filter. The filter cake will be dried under vacuum for 24 h to afford product. The structure will be confirmed with 1H-NMR and LC/MS.

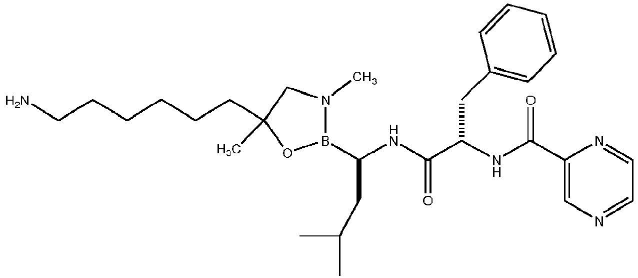

Step 4: *Conjugate of bortezomib with (8-amino-2-hydroxy-2-methyl-1-(methylamino)octane:* A 100-mL, round-bottom flask equipped with a magnetic stirrer will be charged with the conjugate of bortezomib (8-(benzyloxycarbonylamino)-2-hydroxy-2-methyl-1-(methylamino)octane [1 mmol], EtOAc (36 mL), and MeOH (0.5 mL). The mixture will be stirred for 5 min to afford a clear solution. 5% Pd/C (200 mg, 50% moisture) will be charged. The mixture will be evacuated for 1 min and then filled with H2 with a balloon. The reaction will be stirred at ambient temperature for 3 h or until the reaction is complete. The mixture will be filtered through a Celite® pad to remove the catalyst; the combined filtrate concentrated and added into a suspension of Celite (10 g) in MTBE (300 mL) over 0.5 h with overhead stirring. The suspension will be filtered through a PP filter and the Celite®/product complex air-dried at ambient temperature for 16 h. It will be suspended in acetone (30 mL) with overhead stirring for 0.5 h and filtered. The filter cake will be washed with acetone (3 × 10 mL). The filtrate will be concentrated and added into cold water (300 mL) over 0.5 h with overhead stirring. The suspension will be stirred for another 0.5 h and filtered through a PP filter. The filter cake will be dried under vacuum for 24 h to afford product. The structure will be confirmed with 1H-NMR, HPLC and GPC.

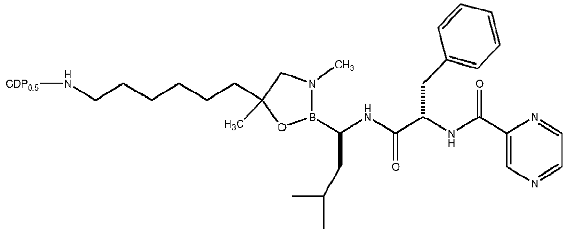

*Step 5: Conjugate of bortezomib with (8-(CDP_{0.5}-carboxamido)-2-hydroxy-2-methyl-1-(methylamino)octane:* A 100-mL round-bottom flask will be charged with the conjugate of bortezomib with (8-amino-2-hydroxy-2-methyl-1-(methylamino)octane (2.0 mmol per estimated number of cyclodextrin units in the CDP polymer) and DMF (5 mL). The mixture will be stirred for 15 min to afford a clear solution. CDP (1 g) and DMF (20 mL) will be added and the mixture stirred for 10 min. EDC·HCl (2.3 mmol per estimated number of cyclodextrin units in the CDP polymer), DMAP (1.0 mmol per estimated number of cyclodextrin units in the CDP polymer), and TEA (5.0 mmol per estimated number of cyclodextrin units in the CDP polymer) will be added and the reaction stirred at ambient temperature for 6 h or until completion of the reaction. The reaction will be added into acetone or a mixture of acetone and diethylether or MTBE. The resulting precipitate will be isolated by filtration or decantation of the supernatant. The precipitate will then be dissolved in water and dialyzed for 3 days with a 25,000 Da MWCO. The lyophilized solution will be filtered through a 2 µM filter and the filtrate lyophilized to give the title product. The structure will be confirmed with 1H-NMR, HPLC and GPC. Example 3. Synthesis of CDP conjugate with 1,2-Diol based boronic acid- Conjugate of bortezomib with (9-(CDP_{0.5}-carboxamido)-2,3-dihydroxy-2,3-dimethylnonane

### Method A:

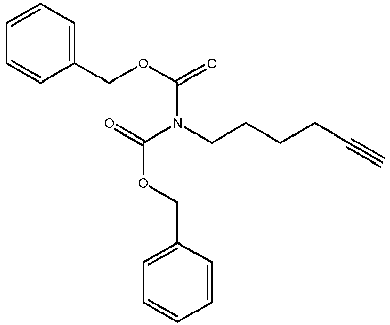

*Step 1: 6-Bis-(benzyloxycarbonyl)amino-1-hexyne:* 6-Chloro-1-hexyne (1.0 mmol) in THF will be treated with bis(benzyloxycarbonyl)amine (1.0 mmol) and potassium carbonate (1.2 mmol) in DMF (10 mL). After 16 h the reaction will be diluted with diethyl ether and washed successively with water, IN hydrochloric acid and saturated sodium bicarbonate. After drying with sodium sulfate, the extract will be filtered and concentrated to give the crude product. This will be purified by chromatography. The structure will be confirmed with 1H-NMR and LC/MS.

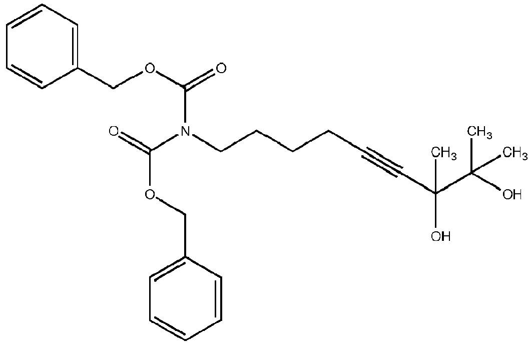

*Step 2: 9-Bis-(benzyloxycarbonyl)amino-2,3-dihydroxy-2,3-dimethyl-4-nonyne:* 6-Bis-(benzyloxycarbonyl)amino-1-hexyne (1.0 mmol) will be treated with lithium diisopropylamide in THF at -78 °C. After 15 minutes, 3-hydroxy-3-methyl-2-butanone in THF will be added. After 1 hour at -78 °C the reaction will be quenched with saturated ammonium chloride solution and allowed to warm to room temperature. The reaction mixture will then be diluted with diethyl ether and successively washed with water, IN hydrochloric acid, and saturated sodium bicarbonate. After drying with sodium sulfate, the extract will be filtered and the solvent evaporated to give the crude product. This will be purified by chromatography. The structure will be verified by 1H-NMR and LC/MS.

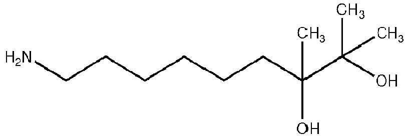

*Step 3: 9-amino-2,3-dihydroxy-2,3-dimethylnonane:* To a suspension of 10% Pd/C in methanol (~1 g of catalyst per 1 g of substrate) in an appropriately sized flask will be added a solution of 9-bis-(benzyloxycarbonyl)amino-2,3-dihydroxy-2,3-dimethyl-4-nonyne in methanol. The flask will be evacuated and after 1 minute filled with hydrogen gas. After the reaction is complete the mixture will be filtered to remove the catalyst and the solvent evaporated to yield the title product. The structure will be verified by 1H-NMR and LC/MS.

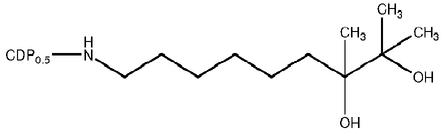

*Step 4: 9-(CDP_{0.5}-carboxamido)-2,3-dihydroxy-2,3-dimethylnonane:* A 100-mL round-bottom flask will be charged with 9-amino-2,3-dihydroxy-2,3-dimethylnonane (2.0 mmol per estimated number of cyclodextrin units in the CDP polymer) and DMF (5 mL). The mixture will be stirred for 15 min to afford a clear solution. CDP (1 g) and DMF (20 mL) will be added and the mixture stirred for 10 min. EDC•HCl (2.3 mmol per estimated number of cyclodextrin units in the CDP polymer), DMAP (1.0 mmol per estimated number of cyclodextrin units in the CDP polymer), and TEA (5.0 mmol per estimated number of cyclodextrin units in the CDP polymer) will be added and the reaction stirred at ambient temperature for 6 h or until completion of the reaction. The reaction will be added into acetone or a mixture of acetone and diethylether or MTBE. The resulting precipitate will be isolated by filtration or decantation of the supernatant. The precipitate will then be dissolved in water and dialyzed for 3 days with a 25,000 Da MWCO. The lyophilized solution will be filtered through a 2 µM filter and the filtrate lyophilized to give the title product. The structure will be confirmed with 1H-NMR, HPLC and GPC. 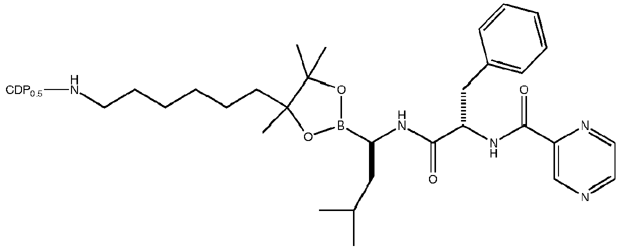 *Step 5: Conjugate of bortezomib with 9-(CDP_{0.5}-carboxamido)-2,3-dihydroxy-2,3-dimelhylnonane:* In a manner similar to that described by Hebel et al. (J. Org. Chem. 2002, 67, 9452) bortezomib (2.0 mmol per estimated number of cyclodextrin units in the CDP polymer) will be dissolved in DMF and treated with a solution of 9-(CDP_{0.5}-carboxamido)-2,3-dihydroxy-2,3-dimethylnonane (1 g) in DMF and 4 Å MS. After 6 h at room temperature, the reaction mixture will be added into acetone or a mixture of acetone and diethylether or MTBE. The resulting precipitate will be isolated by filtration or decantation of the supernatant. The precipitate will then be dissolved in water and dialyzed for 3 days with a 25,000 Da MWCO. The lyophilized solution will be filtered through a 2 µM filter and the filtrate lyophilized to give the title product. The structure will be confirmed with 1H-NMR, HPLC and GPC.

### Method B:

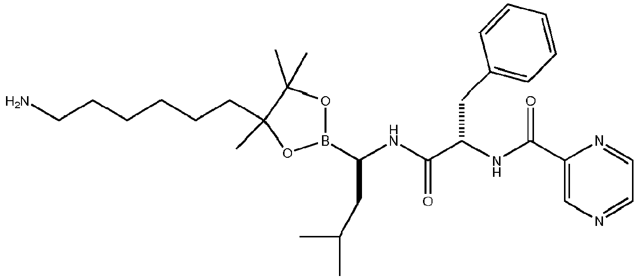

*Step 1: Conjugate of bortezomib with 9-amino-2,3-dihydroxy-2,3-dimethylnonane:* In a manner similar to that described by Hebel et al. (J. Org. Chem. 2002, 67, 9452) bortezomib (1.0 mmol) will be dissolved in DMF and treated with a solution of 9-amino-2,3-dihydroxy-2,3-dimethylnonane (from Method A, Step 3) (1.0 mmol) in DMF and 4 Å MS. After 6 h at room temperature, the reaction mixture will be added into in MTBE (30 mL) over 0.5 h with overhead stirring. The suspension will be stirred for another 0.5 h and filtered through a PP filter. The filter cake will be dried under vacuum for 24 h to afford product. The structure will be confirmed with 1H-NMR and LC/MS.

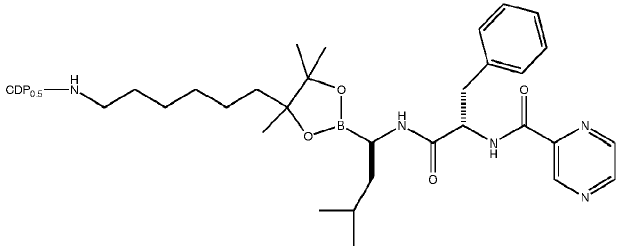

*Step 2: Conjugate of bortezomib with 9-(CDP_{0.5}-carboxamido)-2,3-dihydroxy-2,3-dimethylnonane:* A 100-mL round-bottom flask will be charged with the conjugate of bortezomib with 9-amino-2,3-dihydroxy-2,3-dimethylnonane (2.0 mmol per estimated number of cyclodextrin units in the CDP polymer) and DMF (5 mL). The mixture will be stirred for 15 min to afford a clear solution. CDP (1 g) and DMF (20 mL) will be added and the mixture stirred for 10 min. EDC·HCl (2.3 mmol per estimated number of cyclodextrin units in the CDP polymer), DMAP (1.0 mmol per estimated number of cyclodextrin units in the CDP polymer), and TEA (5.0 mmol per estimated number of cyclodextrin units in the CDP polymer) will be added and the reaction stirred at ambient temperature for 6 h or until completion of the reaction. The reaction will be added into acetone or a mixture of acetone and diethylether or MTBE. The resulting precipitate will be isolated by filtration or decantation of the supernatant. The precipitate will then be dissolved in water and dialyzed for 3 days with a 25,000 Da MWCO. The lyophilized solution will be filtered through a 2 µM filter and the filtrate lyophilized to give the title product. The structure will be confirmed with 1H-NMR, HPLC and GPC.

Example 4. Synthesis of CDP conjugate with 1,3-Diol based boronic acid - Conjugate of bortezomib with (6-(CDP_{0.5}-carboxamido)-1-hydroxy-2-(hydroxymethyl)hexane

### Method A:

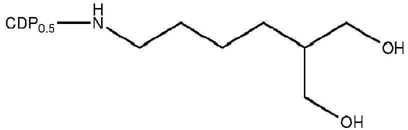

*Step 1:6-(CDP_{0.5}-carboxamido-1-hydroxy-2-(hydroxymethyl)hexane:* A 100-mL round-bottom flask will be charged with 6-amino-1-hydroxy-2-(hydroxymethyl)hexane (2.0 mmol per estimated number of cyclodextrin units in the CDP polymer) and DMF (5 mL). The mixture will be stirred for 15 min to afford a clear solution. CDP (1 g) and DMF (20 mL) will be added and the mixture stirred for 10 min. EDC•HCl (2.3 mmol per estimated number of cyclodextrin units in the CDP polymer), DMAP (1.0 mmol per estimated number of cyclodextrin units in the CDP polymer), and TEA (5.0 mmol per estimated number of cyclodextrin units in the CDP polymer) will be added and the reaction stirred at ambient temperature for 6 h or until completion of the reaction. The reaction will be added into acetone or a mixture of acetone and diethylether or MTBE. The resulting precipitate will be isolated by filtration or decantation of the supernatant. The precipitate will then be dissolved in water and dialyzed for 3 days with a 25,000 Da MWCO. The lyophilized solution will be filtered through a 2 µM filter and the filtrate lyophilized to give the title product. The structure will be confirmed with 1H-NMR, HPLC and GPC.

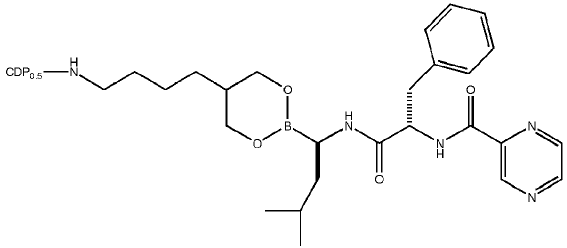

*Step 2: Conjugate of bortezomib with (6-(CDP-carboxamido)-1-hydroxy-2-(hydroxymethyl)hexane:* In a manner similar to that described by Hebel et al. (J. Org. Chem. 2002, 67, 9452) bortezomib (2.0 mmol per estimated number of cyclodextrin units in the CDP polymer) will be dissolved in DMF and treated with a solution of 6-(CDP_{0.5}-carboxamido)-l-hydroxy-2-(hydroxymethyl)hexane(1 g) in DMF and 4 Å MS. After 6 h at room temperature, the reaction mixture will be added into acetone or a mixture of acetone and diethylether or MTBE. The resulting precipitate will be isolated by filtration or decantation of the supernatant. The precipitate will then be dissolved in water and dialyzed for 3 days with a 25,000 Da MWCO. The lyophilized solution will be filtered through a 2 µM filter and the filtrate lyophilized to give the title product. The structure will be confirmed with 1H-NMR, HPLC and GPC.

### Method B:

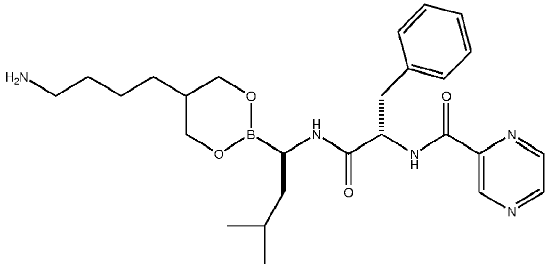

*Step 1: Conjugate of bortezomib with 6-amino-1-hydroxy-2-(hydroxymethyl)hexane:* In a manner similar to that described by Hebel et al. (J. Org. Chem. 2002, 67, 9452) bortezomib (1.0 mmol) will be dissolved in DMF and treated with a solution of 6-amino-1-hydroxy-2-(hydroxymethyl)hexane (1.0 mmol) in DMF and 4 Å MS. After 6 h at room temperature, the reaction mixture will be added into in MTBE (30 mL) over 0.5 h with overhead stirring. The suspension will be stirred for another 0.5 h and filtered through a PP filter. The filter cake will be dried under vacuum for 24 h to afford product. The structure will be confirmed with 1H-NMR and LC/MS.

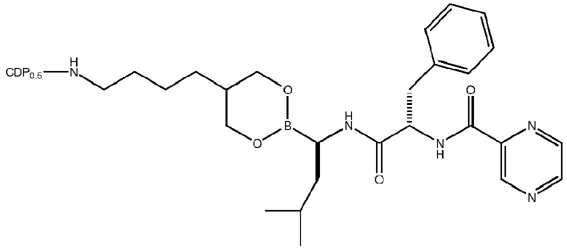

*Step 2: Conjugate of bortezomib with 6-(CDP_{0.5}-carboxamido)-1-hydroxy-2-(hydroxymethyl)hexane:* A 100-mL round-bottom flask will be charged with the conjugate of bortezomib with 6-amino-1-hydroxy-2-(hydroxymethyl)hexane (2.0 mmol per estimated number of cyclodextrin units in the CDP polymer) and DMF (5 mL). The mixture will be stirred for 15 min to afford a clear solution. CDP (1 g) and DMF (20 mL) will be added and the mixture stirred for 10 min. EDC•HCl (2.3 mmol per estimated number of cyclodextrin units in the CDP polymer), DMAP (1.0 mmol per estimated number of cyclodextrin units in the CDP polymer), and TEA (5.0 mmol per estimated number of cyclodextrin units in the CDP polymer) will be added and the reaction stirred at ambient temperature for 6 h or until completion of the reaction. The reaction will be added into acetone or a mixture of acetone and diethylether or MTBE. The resulting precipitate will be isolated by filtration or decantation of the supernatant. The precipitate will then be dissolved in water and dialyzed for 3 days with a 25,000 Da MWCO. The lyophilized solution will be filtered through a 2 µM filter and the filtrate lyophilized to give the title product. The structure will be confirmed with 1 H-NMR, HPLC and GPC. Example 5. Synthesis of CDP conjugate with diethanolamine based boronic acid - Conjugate of bortezomib with [(6-(CDP_{0.5}-carboxamidohexyl)-bis-(2-hydroxyethyl]amine

### Method A:

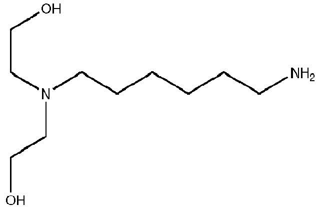

*Step 1: Bis-(2-hydroxyethyl)hexylamine:* In the manner described by R. M. Peck et al. (J. Am. Chem. Soc. 1959, 81, 3984) the title compound will be prepared.

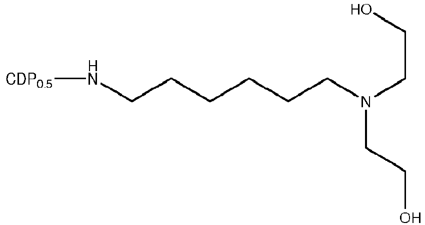

*Step 2: Bis-(2-hydroxyethyl)-[(6-(CDP_{0.5}-carboxamidohexyl)amine:* A 100-mL round-bottom flask will be charged with bis-(2-hydroxyethyl)hexylamine (2.0 mmol per estimated number of cyclodextrin units in the CDP polymer) and DMF (5 mL). The mixture will be stirred for 15 min to afford a clear solution. CDP (1 g) and DMF (20 mL) will be added and the mixture stirred for 10 min. EDC•HCl (2.3 mmol per estimated number of cyclodextrin units in the CDP polymer), DMAP (1.0 mmol per estimated number of cyclodextrin units in the CDP polymer), and TEA (5.0 mmol per estimated number of cyclodextrin units in the CDP polymer) will be added and the reaction stirred at ambient temperature for 6 h or until completion of the reaction. The reaction will be added into acetone or a mixture of acetone and diethylether or MTBE. The resulting precipitate will be isolated by filtration or decantation of the supernatant. The precipitate will then be dissolved in water and dialyzed for 3 days with a 25,000 Da MWCO. The lyophilized solution will be filtered through a 2 µM filter and the filtrate lyophilized to give the title product. The structure will be confirmed with 1H-NMR, HPLC and GPC.

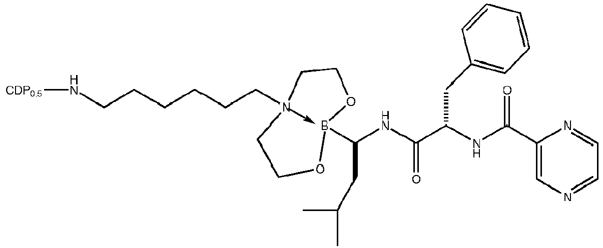

*Step 3: Conjugate of bortezomib with bis-(2-hydroxyethyl)-[(6-(CDP_{0.5}*-*carboxamadohexyl)amine:* In a manner similar to that described by Hebel et al. (J. Org. Chem. 2002, 67, 9452) bortezomib (2.0 mmol per estimated number of cyclodextrin units in the CDP polymer) will be dissolved in DMF and treated with a solution of bis-(2-hydroxyethyl)-[(6-(CDP_{0.5}-carboxamidohexyl)amine (1 g) in DMF and 4 Å MS. After 6 h at room temperature, the reaction mixture will be added into acetone or a mixture of acetone and diethylether or MTBE. The resulting precipitate will be isolated by filtration or decantation of the supernatant. The precipitate will then be dissolved in water and dialyzed for 3 days with a 25,000 Da MWCO. The lyophilized solution will be filtered through a 2 µM filter and the filtrate lyophilized to give the title product. The structure will be confirmed with 1H-NMR, HPLC and GPC.

### Method B:

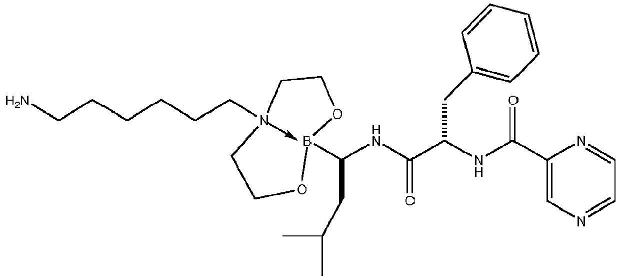

*Step 1: Conjugate of bortezomib with bis-(2-hydroxyethyl)hexylamine:* In a manner similar to that described by Hebel et al. (J. Org. Chem. 2002, 67, 9452) bortezomib (1.0 mmol) will be dissolved in DMF and treated with a solution of bis-(2-hydroxyethyl)hexylamine (from Method A, Step 1) (1.0 mmol) in DMF and 4 Å MS. After 6 h at room temperature, the reaction mixture will be added into in MTBE (30 mL) over 0.5 h with overhead stirring. The suspension will be stirred for another 0.5 h and filtered through a PP filter. The filter cake will be dried under vacuum for 24 h to afford product. The structure will be confirmed with 1H-NMR and LC/MS.

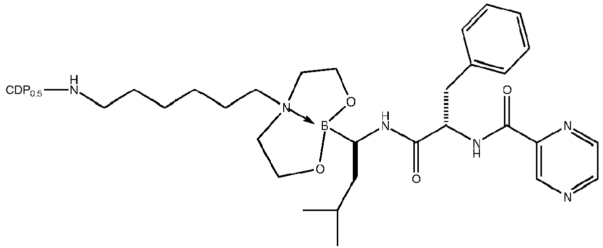

*Step 2: Conjugate of bortezomib with bis-(2-hydroxyethyl)-[(6-(CDP_{0.5}*-*carboxamidohexyl)amine:* A 100-mL round-bottom flask will be charged with the conjugate of bortezomib with bis-(2-hydroxyethyl)hexylamine (2.0 mmol) and DMF (5 mL). The mixture will be stirred for 15 min to afford a clear solution. CDP (1 g) and DMF (20 mL) will be added and the mixture stirred for 10 min. EDC·HCl (2.3 mmol per estimated number of cyclodextrin units in the CDP polymer), DMAP (1.0 mmol per estimated number of cyclodextrin units in the CDP polymer), and TEA (5.0 mmol per estimated number of cyclodextrin units in the CDP polymer) will be added and the reaction stirred at ambient temperature for 6 h or until completion of the reaction. The reaction will be added into acetone or a mixture of acetone and diethylether or MTBE. The resulting precipitate will be isolated by filtration or decantation of the supernatant. The precipitate will then be dissolved in water and dialyzed for 3 days with a 25,000 Da MWCO. The lyophilized solution will be filtered through a 2 µM filter and the filtrate lyophilized to give the title product. The structure will be confirmed with 1H-NMR, HPLC and GPC.

Example 6. Synthesis of CDP conjugate of iminodiacetic acid based boronic acid - Conjugate of bortezomib with [(6-(CDP_{0.5}-carboxamidohexyl)-carboxymethylamino]-acetate

### Method A:

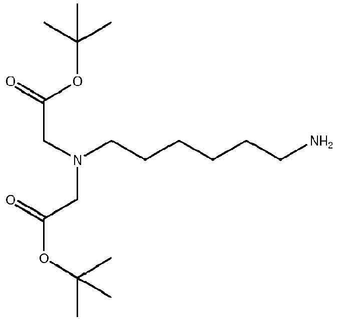

*Step 1: t-Butyl-[(6-aminohexyl)-t-butoxycarbonylmethylamino]-acetate hydrochloride:* In a manner similar to that described by M. Kruppa et al. (J. Am. Chem. Soc. 2005, 127, 3362) N-CBZ-1,6-diamino- hexane (4.9 mmol) will be dissolved in MeCN (20 ml) and mixed with t-butyl bromoacetate (10.6 mmol), potassium carbonate (2.92 g, 21.1 mmol) and a spatula tip of potassium iodide. The suspension will be stirred 2 days at 60 °C and monitored by TLC (ethyl acetate). The mixture will be filtrated, diluted with water and extracted with ethyl acetate. After drying over sodium sulfate the organic solvents will be evaporated to yield the crude product. Purification using column chromatography will give the CBZ-protected iminodiacetic acid-intermediate.

To deprotect the *CBZ*-group, the purified product will be hydrogentated over 10% Pd on carbon (50 wt. %) in methanol for 3 h. After completion of the reaction, the catalyst will be removed by filtration and the filtrate evaporated to dryness to give the title product. The structure will be confirmed with LC/MS and 1H-NMR.

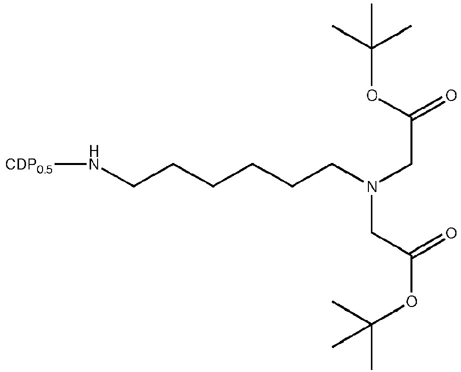

*Step 2: t-Butyl-[(6-(CDP_{0.5}-carboxamidohexyl)-t-butoxycarbonylmethylamino]-acetate:* A 100-mL round-bottom flask will be charged with t-butyl-[(6-aminohexyl)-t-butoxycarbonylmethylamino]-acetate hydrochloride (2.0 mmol per estimated number of cyclodextrin units in the CDP polymer) and DMF (5 mL). The mixture will be stirred for 15 min to afford a clear solution. CDP (1 g) and DMF (20 mL) will be added and the mixture stirred for 10 min. EDC·HCl (2.3 mmol per estimated number of cyclodextrin units in the CDP polymer), DMAP (1.0 mmol per estimated number of cyclodextrin units in the CDP polymer), and TEA (5.0 mmol per estimated number of cyclodextrin units in the CDP polymer) will be added and the reaction stirred at ambient temperature for 6 h or until completion of the reaction. The reaction will be added into acetone or a mixture of acetone and diethylether or MTBE. The resulting precipitate will be isolated by filtration or decantation of the supernatant. The precipitate will then be dissolved in water and dialyzed for 3 days with a 25,000 Da MWCO. The lyophilized solution will be filtered through a 2 µM filter and the filtrate lyophilized to give the title product. The structure will be confirmed with 1H-NMR, HPLC and GPC.

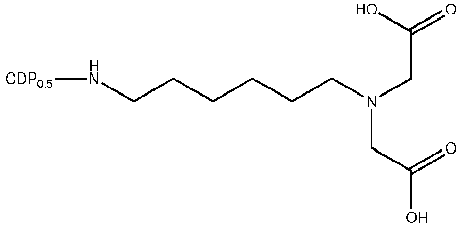

*Step 3: [(6-(CDP_{0.5}-carboxamidohexyl)-carboxymethylamino]-acetate:* A round-bottom flask equipped with a magnetic stirrer will be charged with t-butyl-[(6-(CDP_{0.5}-carboxamidohexyl)-t-butoxycarbonylmethylamino]-acetate, CH2C12 (5 volumes), and TFA (5 volumes). The reaction will be stirred at ambient temperature for 1 h or until the reaction is complete. The reaction will be concentrated and added into acetone or a mixture of acetone and diethylether or MTBE. The resulting precipitate will be isolated by filtration or decantation of the supernatant. The precipitate will then be dissolved in water and dialyzed for 3 days with a 25,000 Da MWCO. The lyophilized solution will be filtered through a 2 µM filter and the filtrate lyophilized to give the title product. The structure will be confirmed with 1H-NMR, HPLC and GPC.

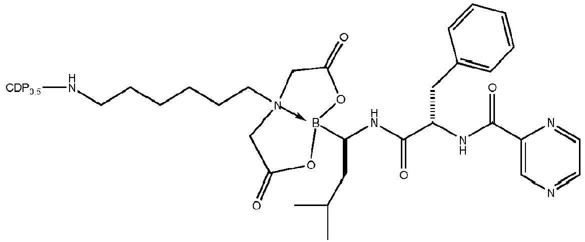

*Step 4: Conjugate of bortezomib with [(6-(CDP_{0.5}-carboxamidohexyl)-carboxymethylamino]-acetate:* In a manner similar to that described by Hebel et al. (J. Org. Chem. 2002, 67, 9452) bortezomib (2.0 mmol per estimated number of cyclodextrin units in the CDP polymer) will be dissolved in DMF and treated with a solution of [(6-(CDP_{0.5}-carboxamidohexyl)-carboxymethylamino]-acetate (1 g) in DMF and 4 Å MS. After 6 h at room temperature, the reaction will be added into acetone or a mixture of acetone and diethylether or MTBE. The resulting precipitate will be isolated by filtration or decantation of the supernatant. The precipitate will then be dissolved in water and dialyzed for 3 days with a 25,000 Da MWCO. The lyophilized solution will be filtered through a 2 µM filter and the filtrate lyophilized to give the title product. The structure will be confirmed with 1H-NMR, HPLC and GPC.

### Method B:

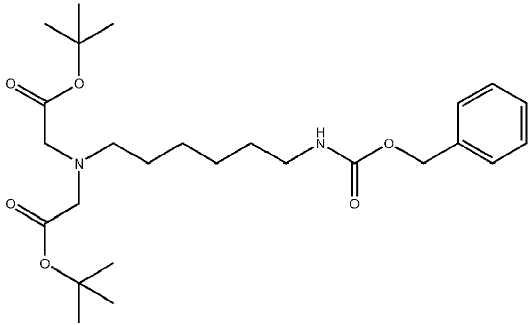

*Step 1: tert-Butyl-[(6-benzyloxycarbonylaminohexyl)-tert-butoxycarbonylmethylamino]-acetate:* In the manner described by M. Kruppa et al. (J. Am. Chem. Soc. 2005, 127, 3362) the title compound will be produced.

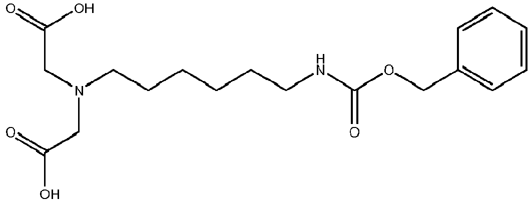

*Step 2: [(6-Benzyloxycarbonylaminohexyl)-carboxymethylamino]-acetate:* To a solution of *tert*-butyl-[(6-benzyloxycarbonylaminohexyl)-*tert*-butoxycarbonylmethylamino]-acetate in dichloromethane will be added at 0 °C trifluoroacetic acid. After 1 hour the solvent will be evaporated to yield the title product. The structure will be confirmed with 1H-NMR and LC/MS.

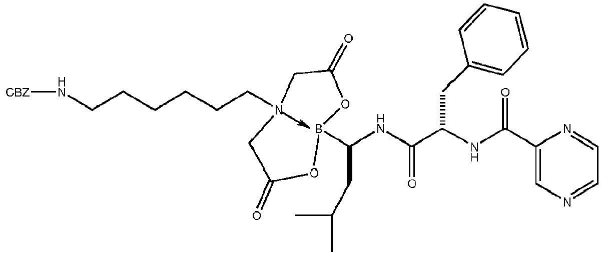

*Step 3: Conjugate of bortezomib with [(6-(benzyloxycarbonylaminohexyl)-carboxymethylamino]-acetate:* In a manner similar to that described by Hebel et al. (J. Org. Chem. 2002, 67, 9452) bortezomib (1.0 mmol) will be dissolved in DMF and treated with a solution of [(6-benzyloxycarbonylaminohexyl)-carboxymethylamino]-acetate (1.0 mmol) in DMF and 4 Å MS. After 6 h at room temperature, the reaction mixture will be added into in MTBE (30 mL) over 0.5 h with overhead stirring. The suspension will be stirred for another 0.5 h and filtered through a PP filter. The filter cake will be dried under vacuum for 24 h to afford product. The structure will be confirmed with 1H-NMR and LC/MS.

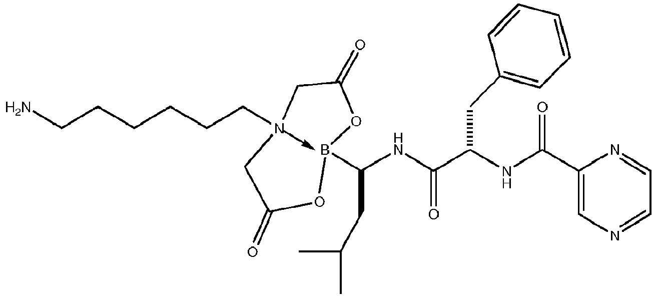

*Step 4: Conjugate of bortezomib with [(6-(aminohevyl)-carboxymethylamino]-acelate:* A 100-mL, round-bottom flask equipped with a magnetic stirrer will be charged with the conjugate of bortezomib with [(6-(benzyloxycarbonylaminohexyl)-carboxymethylamino]-acetate [1.06 mmol], EtOAc (36 mL), and MeOH (0.5 mL). The mixture will stirred for 5 min to afford a clear solution. 5% Pd/C (200 mg, 50% moisture) will be charged. The mixture will be evacuated for 1 min and then filled with H2 with a balloon. The reaction will be stirred at ambient temperature for 3 h or until the reaction is complete. The mixture will be added to MTBE (30 mL) over 0.5 h with overhead stirring. The suspension will be stirred for another 0.5 h and filtered through a PP filter. The filter cake will be dried under vacuum for 24 h to afford product. The structure will be confirmed with 1H-NMR and LC/MS.

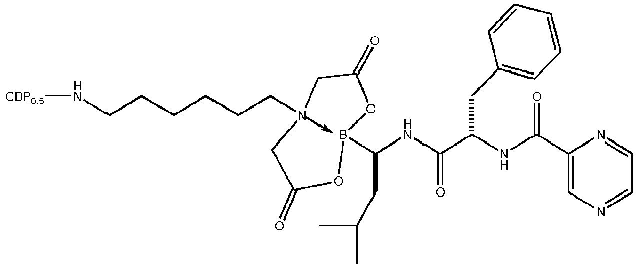

*Step 5: Conjugate of bortezomib with [(6-(CDP_{0.5}-carboxamidohexyl)-carboxymethylamino]-acetate:* A 100-mL round-bottom flask will be charged with the conjugate of bortezomib with [(6-(aminohexyl)-carboxymethylamino]-acetate (2.0 mmol per estimated number of cyclodextrin units in the CDP polymer) and DMF (5 mL). The mixture will be stirred for 15 min to afford a clear solution. CDP (1 g) in DMF (20 mL) will be added and the mixture stirred for 10 min. EDC•HCl (2.3 mmol per estimated number of cyclodextrin units in the CDP polymer), DMAP (1.0 mmol per estimated number of cyclodextrin units in the CDP polymer), and TEA (5.0 mmol per estimated number of cyclodextrin units in the CDP polymer) will be added and the reaction stirred at ambient temperature for 6 h or until completion of the reaction. The reaction will be added into acetone or a mixture of acetone and diethylether or MTBE. The resulting precipitate will be isolated by filtration or decantation of the supernatant. The precipitate will then be dissolved in water and dialyzed for 3 days with a 25,000 Da MWCO. The lyophilized solution will be filtered through a 2 µM filter and the filtrate lyophilized to give the title product. The structure will be confirmed with 1H-NMR, HPLC and GPC. The CDP polymer used in Examples 1-6 can be any CDP polymer described herein that has two functional groups, such as -COOH, that would react with an amino group. In one embodiment, the CDP polymer is represented by the following structural formula: 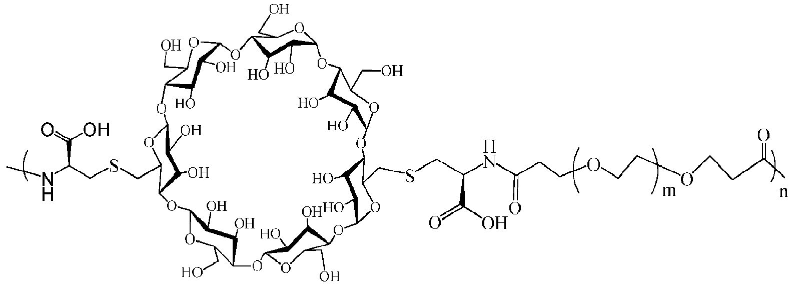 wherein n is an integer resulting in a PEG having a MW of 3400 or less; and m islto 100 (e.g., 4 to 20). A CDP-proteasome inhibitor conjugate comprising a boronic acid containing proteasome inhibitor described herein other than bortezomib can be prepared in similar manners as described in Example 1-6 with suitable starting materials.

### Example 11: Synthesis of CDP-Pemetrexed

### Materials and Methods

**General.** All of the anhydrous solvents, HPLC grade solvents and other common organic solvents will be purchased from commercial suppliers and used without further purification. Parent polymer, Poly-CD-PEG, will be synthesized as previously described (Cheng et al., Bioconjug Chem 2003, 14 (5), 1007-17). De-ionized water (18-MΩ-cm) will be obtained by passing in-house de-ionized water through a Milli-Q Biocel Water system (Millipore). NMR spectra will be recorded on a Varian Inova 400 MHz spectrometer (Palo Alto, CA). Mass spectral (MS) analysis will be performed on Bruker FT-MS 4.7 T electrospray mass spectrometer. MWs of the polymer samples will be analyzed on a Agilent 1200 RI coupled with Viscotek 270 LALS-RALS system. Gemcitabine, Gemcitabine derivatives and polymer-Gemcitabine conjugates will be analyzed with a C-18 reverse phase column on a Agilent 1100 HPLC system. Particle size measurement will be carried out on a Zetasizer nano-zs (Serial # mal1017190 Malvern Instruments, Worcestershire, UK).

### Synthesis of CDP-NH-EG₂-α-O-Glutamate-LY231514

CDP (1.0 g, 0.21 mmol) will be dissolved in dry N,N-dimethylformamide (DMF, 10 mL). NH₂-EG₂-α-O-Glutamate-LY231514 (240 mg, 0.46 mmol), N,N-diisopropylethylamine (0.080 mL, 0.46 mmol), N-(3-Dimethylaminopropyl)-N'-ethylcarbodiimide hydrochloride (120 mg, 0.62 mmol), and N-Hydroxysuccinimide (52 mg, 0.46 mmol) will be added to the polymer solution and stirred for 4 h. The polymer will be precipitated with acetone (100 mL). It will be then rinsed with acetone (50 mL). The precipitate will be dissolved in water (100 mL). The solution will be purified by TFF (30k MWCO) with water. It will be filtered through 0.2 µm filters (Nalgene) and will be kept frozen (**Scheme 39**). 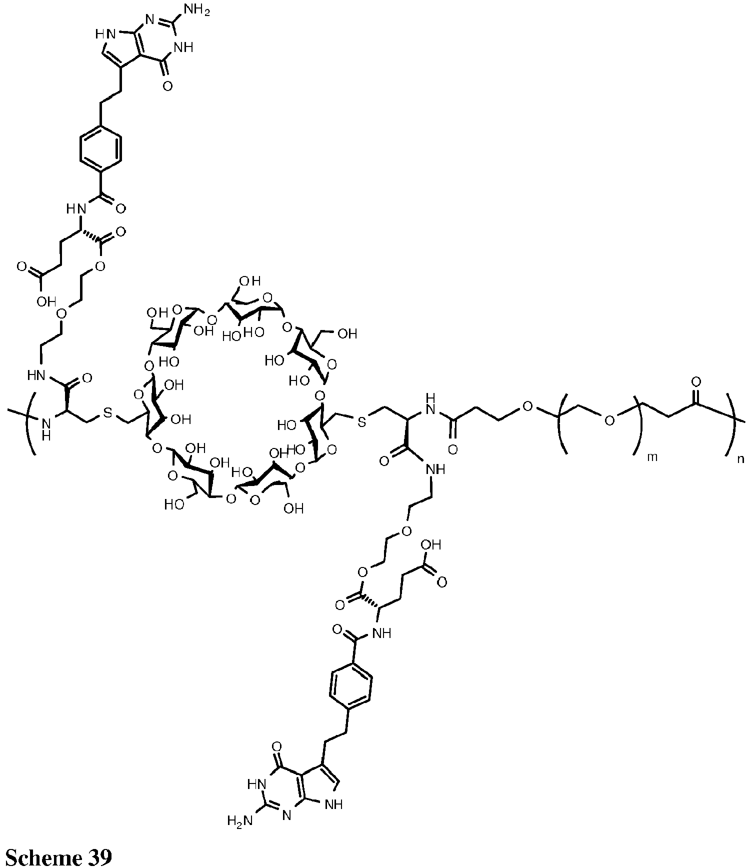 wherein n is an integer resulting in a PEG having a MW of 3400 or less; and m islto 100 (e.g., 4 to 20).

### Synthesis of CDP-NH-EG₂-γ-0-Glutamate-LY231514

CDP (1.0 g, 0.21 mmol) will be dissolved in dry N,N-dimethylformamide (DMF, 10 mL). NH₂-EG₂-γ-O-Glutamate- LY231514 (240 mg, 0.46 mmol), N,N-diisopropylethylamine (0.080 mL, 0.46 mmol), N-(3-Dimethylaminopropyl)-N'-ethylcarbodiimide hydrochloride (120 mg, 0.62 mmol), and N-Hydroxysuccinimide (52 mg, 0.46 mmol) will be added to the polymer solution and stirred for 4 h. The polymer will be precipitated with acetone (100 mL). It will be then rinsed with acetone (50 mL). The precipitate will be dissolved in water (100 mL). The solution will be purified by TFF (30k MWCO) with water. It will be filtered through 0.2 µm filters (Nalgene) and will be kept frozen (**Scheme 40**). 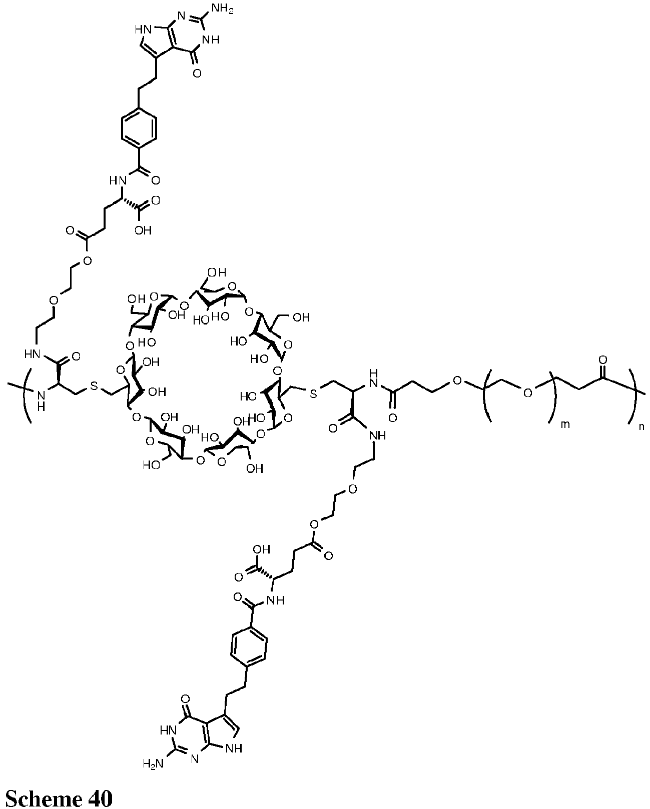 wherein n is an integer resulting in a PEG having a MW of 3400 or less; and m islto 100 (e.g., 4 to 20).

### Example 12: Synthesis of CDP-Gemcitabine and CDP-Gemcitabine Derivatives

### Materials and Methods

**General.** All of the anhydrous solvents, HPLC grade solvents and other common organic solvents will be purchased from commercial suppliers and used without further purification. Parent polymer, Poly-CD-PEG, will be synthesized as previously described (Cheng et al., Bioconjug Chem 2003, 14 (5), 1007-17). De-ionized water (18-MΩ-cm) will be obtained by passing in-house de-ionized water through a Milli-Q Biocel Water system (Millipore). NMR spectra will be recorded on a Varian Inova 400 MHz spectrometer (Palo Alto, CA). Mass spectral (MS) analysis will be performed on Bruker FT-MS 4.7 T electrospray mass spectrometer. MWs of the polymer samples will be analyzed on a Agilent 1200 RI coupled with Viscotek 270 LALS-RALS system. Gemcitabine, Gemcitabine derivatives and polymer-Gemcitabine conjugates will be analyzed with a C-18 reverse phase column on a Agilent 1100 HPLC system. Particle size measurement will be carried out on a Zetasizer nano-zs (Serial # mal1017190 Malvern Instruments, Worcestershire, UK).

### Synthesis of CDP-β-Ala-Glycolate-O-Gemcitabine

CDP (1.0 g, 0.21 mmol) will be dissolved in dry N,N-dimethylformamide (DMF, 10 mL). β-Ala-Glycolate-O-Gemcitabine (180 mg, 0.46 mmol), N,N-diisopropylethylamine (0.080 mL, 0.46 mmol), N-(3-Dimethylaminopropyl)-N'-ethylcarbodiimide hydrochloride (120 mg, 0.62 mmol), and N-Hydroxysuccinimide (52 mg, 0.46 mmol) will be added to the polymer solution and stirred for 4 h. The polymer will be precipitated with acetone (100 mL). It will be then rinsed with acetone (50 mL). The precipitate will be dissolved in water (100 mL). The solution will be purified by TFF (30k MWCO) with water. It will be filtered through 0.2 µm filters (Nalgene) and will be kept frozen (**Scheme 41**). 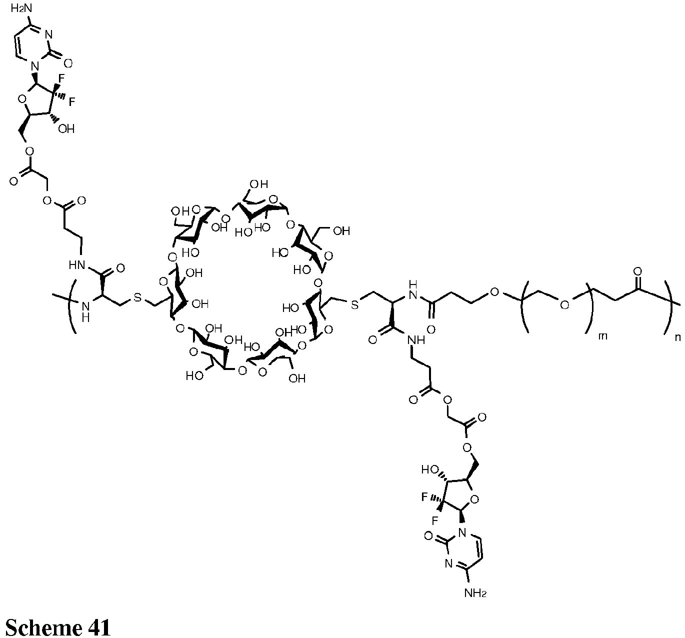 wherein n is an integer resulting in a PEG having a MW of 3400 or less; and m islto 100 (e.g., 4 to 20).

### Synthesis of CDP-β-Ala-Glycolate-NH-Gemcitabine

CDP (1.0 g, 0.21 mmol) will be dissolved in dry N,N-dimethylformamide (DMF, 10 mL). β-Ala-Glycolate-NH-Gemcitabine (180 mg, 0.46 mmol), N,N-diisopropylethylamine (0.080 mL, 0.46 mmol), N-(3-Dimethylaminopropyl)-N'-ethylcarbodiimide hydrochloride (120 mg, 0.62 mmol), and N-Hydroxysuccinimide (52 mg, 0.46 mmol) will be added to the polymer solution and stirred for 4 h. The polymer will be precipitated with acetone (100 mL). It will be then rinsed with acetone (50 mL). The precipitate will be dissolved in water (100 mL). The solution will be purified by TFF (30k MWCO) with water. It will be filtered through 0.2 µm filters (Nalgene) and will be kept frozen (**Scheme 42**). 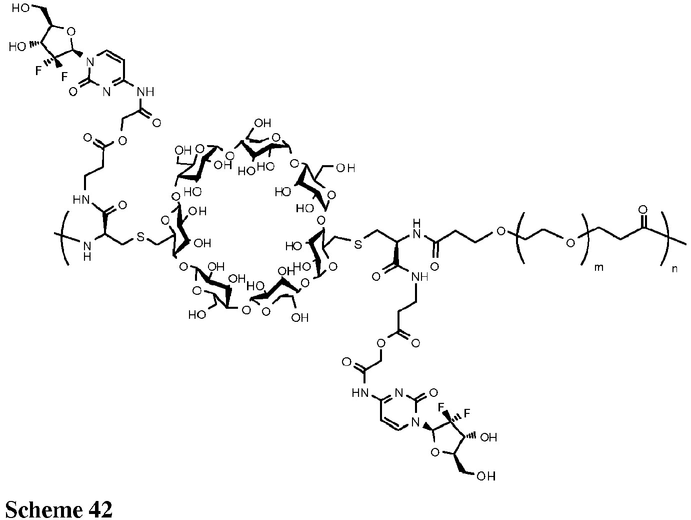 wherein n is an integer resulting in a PEG having a MW of 3400 or less; and m islto 100 (e.g., 4 to 20).

### Synthesis of CDP-β-Ala-Glycolate-Methyl-PO₃-O-Gemcitabine

CDP (1.0 g, 0.21 mmol) will be dissolved in dry N,N-dimethylformamide (DMF, 10 mL). β-Ala-Glycolate-Methyl-PO₃-O-Gemcitabine (230 mg, 0.46 mmol), N,N-diisopropylethylamine (0.080 mL, 0.46 mmol), N-(3-Dimethylaminopropyl)-N'-ethylcarbodiimide hydrochloride (120 mg, 0.62 mmol), and N-Hydroxysuccinimide (52 mg, 0.46 mmol) will be added to the polymer solution and stirred for 4 h. The polymer will be precipitated with acetone (100 mL). It will be then rinsed with acetone (50 mL). The precipitate will be dissolved in water (100 mL). The solution will be purified by TFF (30k MWCO) with water. It will be filtered through 0.2 µm filters (Nalgene) and will be kept frozen (**Scheme 43**). 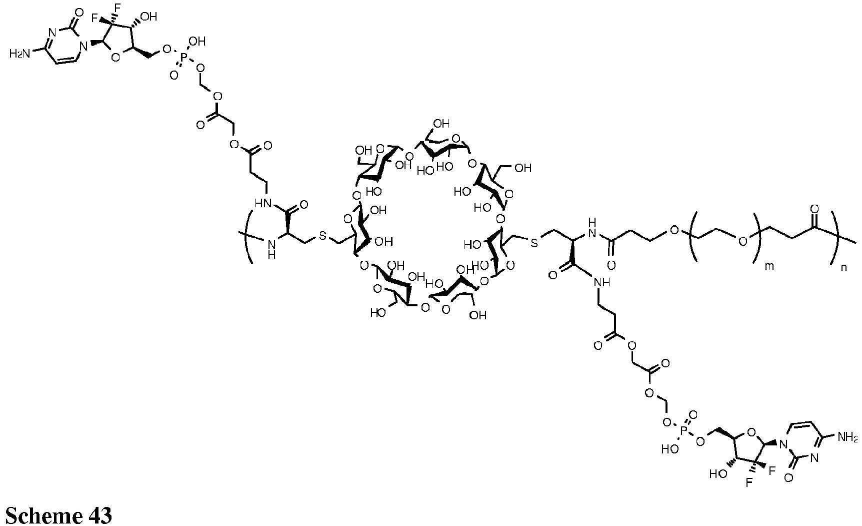 wherein n is an integer resulting in a PEG having a MW of 3400 or less; and m islto 100 (e.g., 4 to 20).

### Synthesis of CDP-β-Ala-Glycolate-NH-Gemeitabine-PO₃H

CDP (1.0 g, 0.21 mmol) will be dissolved in dry N,N-dimethylformamide (DMF, 10 mL). β-Ala-Glycolate-NH-Gemcitabine-PO₃H (220 mg, 0.46 mmol), N,N-diisopropylethylamine (0.080 mL, 0.46 mmol), N-(3-Dimethylaminopropyl)-N'-ethylcarbodiimide hydrochloride (120 mg, 0.62 mmol), and N-Hydroxysuccinimide (52 mg, 0.46 mmol) will be added to the polymer solution and stirred for 4 h. The polymer will be precipitated with acetone (100 mL). It will be then rinsed with acetone (50 mL). The precipitate will be dissolved in water (100 mL). The solution will be purified by TFF (30k MWCO) with water. It will be filtered through 0.2 µm filters (Nalgene) and will be kept frozen (**Scheme 44**). 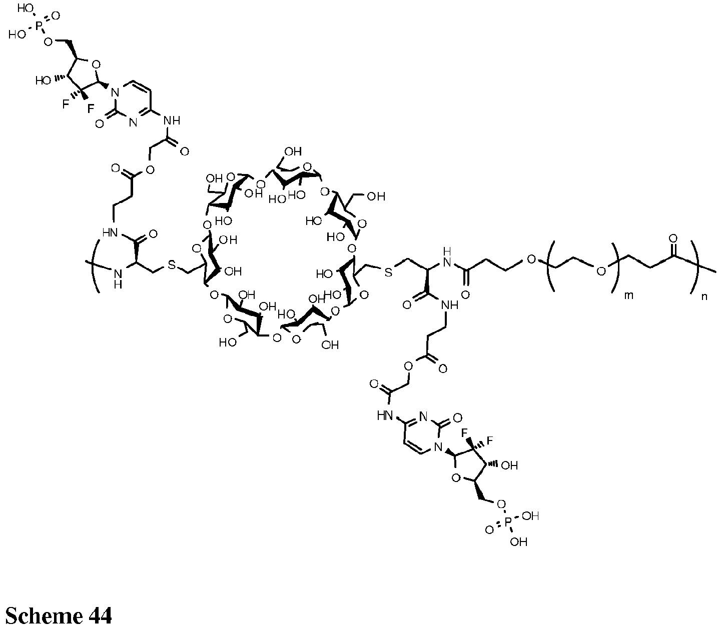 wherein n is an integer resulting in a PEG having a MW of 3400 or less; and m islto 100 (e.g., 4 to 20).

Other embodiments are in the claims.

Preferred embodiments of the present invention are described below and are referred to as embodiments E1 to E36:
E1. A method of treating an autoimmune disease in a subject comprising administering a CDP-therapeutic agent conjugate to the subject in an amount effective to treat the disease.
E2. A method of treating lupus in a subject comprising administering a CDP-therapeutic agent conjugate to the subject in an amount effective to treat the lupus.
E3. The method of E1 or E2, wherein the CDP-therapeutic agent conjugate is a CDP-cytotoxic agent conjugate.
E4. The method of E3, wherein the CDP-cytotoxic agent conjugate is selected from the group consisting of a CDP-topoisomerase I inhibitor conjugate, a CDP-topoisomerase II inhibitor conjugate, a CDP-anti-metabolic agent conjugate, a CDP-anti-folate agent conjugate, a CDP-alkylating agent conjugate, a CDP-anthracycline conjugate, a CDP-anti-tumor antibiotic conjugate, and a CDP-microtubule inhibitor conjugate.
E5. The method of E4, wherein the CDP-microtubule inhibitor conjugate is a CDP-taxane conjugate.
E6. The method of E4, wherein the CDP-microtubule inhibitor conjugate is a CDP-epothilone conjugate.
E7. The method of E1 or E2, wherein the CDP-therapeutic agent conjugate is a CDP-immunomodulator conjugate.
E8. The method of E7, wherein the CDP-immunomodulator conjugate is selected from the group consisting of a CDP-corticosteroid conjugate and a CDP-rapamycin analog conjugate.
E9. The method of E1 or E2, wherein the CDP-therapeutic agent conjugate is a CDP-topoisomerase I inhibitor conjugate.
E10. The method of E9, wherein the CDP-topoisomerase I inhibitor is a CDP-camptothecin conjugate.
E11. The method of E10, wherein the CDP-camptothecin conjugate is CRLX101.
E12. The method of E9, wherein the CDP-topoisomerase I inhibitor conjugate is a CDP-camptothecin derivative conjugate.
E13. The method of any one of E9-E12, wherein the CDP-topoisomerase I inhibitor conjugate is administered to the subject at 30 mg/m² per month or less.
E14. A method of treating lupus in a subject comprising administering a CDP-therapeutic agent conjugate to the subject in combination with a second therapeutic agent.
E15. The method of E14, wherein the second therapeutic agent is one or more of the following agents: an anti-inflammatory agent, an anti-malarial agent, an immunomodulator, an anti-coagulant, and a hormone.
E16. The method of E15, wherein the anti-inflammatory agent is one or more of the following agents: aspirin, acetaminophen, a non-steroidal anti-inflammatory drug, and a corticosteroid.
E17. The method of E15, wherein the anti-malarial agent is one or more of the following agents: hydroxychloroquine and chloroquine.
E18. The method of E15, wherein the immunomodulator is one or more of the following agents: an immunomodulator with an intracellular target, an immunomodulator with a cellular receptor target, an immunomodulator with a serum target, and a biologic that intereferes with immune cell function.
E19. The method of E18, wherein the immunomodulator with an intracellular target is one or more of the following agents: an anti-metabolite (e.g., purine synthesis inhibitor, a pyrimidine synthesis inhibitor, an anti-folate), an IL-2 inhibitor, an mTor inhibitor, a TNF inhibitor, and a macrolide.
E20. The method of E18, wherein the immunomodulator with a cellular receptor target is one or more of the following agents: an IL-1 receptor inhibitor and an antibody which inhibits the function of the cellular receptor target.
E21. The method of E18, wherein the immunomodulator with a serum target is an antibody which inhibits the function of the serum target.
E22. The method of E15, wherein the anti-coagulant is selected from the group consisting of aspirin, heparin, and warfarin.
E23. The method of E20, wherein the antibody which inhibits the function of a cellular receptor target is one of more of the following agents: an anti-CD3 antibody, an anti-CD4 antibody, an anti-CD5 antibody, an anti-CD 11a antibody, anti-BLyS antibody, an anti-CD20 antibody, an anti-CD22 antibody, an anti-CD23 antibody, an anti-CD40 antibody, an anti-CD62L antibody, an anti-CD80 antibody, an anti-CD147 antibody, an anti-CD 154 antibody, an anti-CAT antibody, an anti-integrin antibody, an CTLA4 antibody, an anti-IL6 receptor antibody, an anti-LFAl antibody, an anti-IL2 antibody, an anti-human T cell antibody.
E24. The method of E21,wherein the antibody which inhibits the function of a serum target is selected from the group consisting of an anti-BLyS antibody, an anti-IL5 antibody, anti-IL6 antibody, and anti-interferon alpha antibody, an anti-IgE antibody, an anti-C5a antibody, an anti-TNF antibody, anti-IL10 antibody, anti-IL12 antibody, an anti-IL13 antibody.
E25. The method of E24, wherein the anti-BLyS antibody is belimumab.
E26. The method of E15, wherein the hormone is one or more of the following agents: an androgen and a gonadotropin-hormone releasing agonist,
E27. The method of E15, wherein the immunomodulator is a macrolide.
E28. The method of E1, wherein the autoimmune disease is other than rheumatoid arthritis.
E29. The method of E1, wherein the autoimmune disease is rheumatoid arthritis and the CDP-therapeutic agent conjugate is other than a CDP-methylprednisolone conjugate.
E30. The method of E1, wherein the autoimmune disease is other than rheumatoid arthritis and the CDP-therapeutic agent conjugate is a CDP-corticosteroid conjugate.
E31. The method of E1, wherein the autoimmune disease is rheumatoid arthritis and the CDP-therapeutic agent conjugate is a CDP-corticosteroid conjugate wherein the corticosteroid is a Group B corticosteroid, a Group C corticosteroid, or a Group D corticosteroid.
E32. The method of E1, wherein the autoimmune disease is rheumatoid arthritis and the CDP-therapeutic agent conjugate is a CDP-corticosteroid conjugate wherein the corticosteroid is hydrocortisone, hydrocortisone acetate, cortisone acetate, tixocortol pivalate, prednisolone, methylprednisolone, or prednisone.
E33. The method of E1, wherein the autoimmune disease is rheumatoid arthritis and the CDP-therapeutic agent conjugate is a CDP-corticosteroid conjugate wherein the corticosteroid is a Group B corticosteroid, a Group C corticosteroid, a Group D corticosteroid, hydrocortisone, hydrocortisone acetate, cortisone acetate, tixocortol pivalate, prednisolone, methylprednisolone, or prednisone.
E34. The method of E1, wherein the autoimmune disease is rheumatoid arthritis and the CDP-therapeutic agent conjugate is administered to the subject in combination with a second therapeutic agent.
E35. The method of E34, wherein the second therapeutic agent is one or more of the following agents: an anti-inflammatory agent, a corticosteroid, a disease modifying antirheumatic drug (DMARD), an immunomodulator, a statin, and a bisphosphonate.
E36. The method of any of the preceding embodiments, wherein the CDP-drug conjugate is in the form of a nanoparticle.

## Claims

1. A cyclodextrin-containing polymer (CDP)-therapeutic agent conjugate comprising a subunit of the following formula: 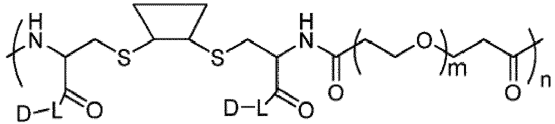 wherein
each L is independently a linker or is absent;
each D is independently a therapeutic agent selected from 5-fluorouracil, gemcitabine, pemetrexed, etoposide, and KOS-1584;
the group 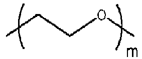 has a Mw of 5,000 Da or less; and
n is at least 4, 5, 6, 7, 8, 9, 10, 11, 12, 13, 14, 15, 16, 17, 18, 19 or 20.

2. The CDP-therapeutic agent conjugate of claim 1, wherein the group 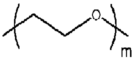 has a Mw of 3,400 Da.

3. The CDP-therapeutic agent conjugate of claim 1 or claim 2, wherein L is a linker comprising an amide bond, an ester bond, a disulfide bond, or a triazole.

4. The CDP-therapeutic agent conjugate of any one of the preceding claims, wherein L is an amino acid derivative (e.g., glycine, glutamate, or cysteine).

5. The CDP-therapeutic agent conjugate of any one of the preceding claims, wherein the therapeutic agents are from 1 to about 80% weight of the conjugate.

6. The CDP-therapeutic agent conjugate of any one of the preceding claims, wherein D is 5-fluorouracil.

7. The CDP-therapeutic agent conjugate of any one of claims 1-5, wherein D is 5-gemcitabine.

8. The CDP-therapeutic agent conjugate of any one of the preceding claims, wherein the CDP-therapeutic agent conjugate is in the form of a nanoparticle.

9. A CDP-therapeutic agent conjugate of any one of the preceding claims for use in a method of treating a subject having an autoimmune disease, an inflammatory disease, or a cancer.

10. The CDP-therapeutic agent conjugate for use of claim 9, wherein the subject has a cancer selected from bladder cancer, breast cancer, colorectal cancer, kidney cancer, liver cancer, lung cancer, skin cancer, ovarian cancer, cervical cancer, prostate cancer, testicular cancer, lymphoma, rectal cancer, gastrointestinal cancer, stomach cancer, pancreatic cancer, leukemia, Ewing's sarcoma, nasoesophageal cancer, nasopharyngeal cancer, a neural or glial cell cancer, and a cancer of the head or neck.

11. The CDP-therapeutic agent conjugate for use of claim 9, wherein the subject has an autoimmune disease selected from ankylosing spondylitis, arthritis, Chagas disease, chronic obstructive pulmonary disease (COPD), dermatomyositis, diabetes mellitus type 1, endometriosis, Goodpasture's syndrome, Graves' disease, Guillain-Barre syndrome (GBS), Hashiomoto's disease, Hidradenitis suppurativa, Kawasaki disease, IgA nephropathy, Idiopathic thrombocytopenic purpura, inflammatory bowel disease, lupus, mixed connective tissue disease, morphea, multiple sclerosis, myasthenia gravis, narcolepsy, neuromyotonia, pemphigus vulgaris, pernicious anemia, psoriasis, psoriatic arthritis, polymyositis, primary biliary cirrhosis, relapsing polychondritis, schizophrenia, scleroderma, Sjogren's syndrome, Stiff person syndrome, temporal arteritis, vasculitis, vitiligo, or Wegener's granulomatosis.

12. The CDP-therapeutic agent conjugate for use of claim 9, wherein the subject has lupus.

13. The CDP-therapeutic agent conjugate for use of claim 9, wherein the subject has an inflammatory disease selected from degenerative joint disease, a spondouloarthropathy, osteoporosis, menstrual cramps, cystic fibrosis, irritable bowel syndrome, gastritis, esophagitis, pancreatitis, peritonitis, Alzheimer's disease, shock, conjunctivitis, pancreatitis, multiple organ injury syndrome, myocardial infarction, atherosclerosis, stroke, reperfusion injury, acute glomerulonephritis, vasculitis, thermal injury, necrotizing enterocolitis, and an inflammatory condition of the skin.

14. The CDP-therapeutic agent conjugate for use of claim 13, wherein the subject has an inflammatory condition of the skin selected from eczema, atopic dermatitis, contact dermatitis, urticaria, and dermatosis with acute inflammatory components.

15. The CDP-therapeutic agent conjugate for use of any one of claims 9-14, wherein the CDP-therapeutic agent conjugate is administered to the subject in combination with a second therapeutic agent.
